(19) Europäisches Patentamt
European Patent Office
Office européen des brevets

(11) EP 2 527 449 A2

(12) **EUROPEAN PATENT APPLICATION**

(43) Date of publication:
**28.11.2012 Bulletin 2012/48**

(21) Application number: **12174205.0**

(22) Date of filing: **29.08.2008**

(51) Int Cl.:
*C12N 15/82* (2006.01) *C12N 9/12* (2006.01)
*A01H 5/00* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.08.2007 US 968754 P**

(62) Document number(s) of the earlier application(s) in accordance with Art. 76 EPC:
**08828465.8 / 2 180 780**

(71) Applicants:
- **E. I. du Pont de Nemours and Company Wilmington, DE 19898 (US)**
- **Pioneer Hi-Bred International Inc. Des Moines, IA 50309 (US)**

(72) Inventors:
- **Taramino, Graziana Wilmington Delaware 19805 (US)**
- **Tingey, Scott V. Wilmington Delaware 19803 (US)**
- **Sakai, Hajime Newark Delaware 19711 (US)**
- **Allen, Stephen M. Wilmington Delaware 19810 (US)**
- **Tomes, Dwight Grimes Iowa 50111 (US)**
- **Luck, Stanley Wilmington Delaware 19810-1449 (US)**
- **Niu, Xiaomu Johnston Iowa 50131 (US)**

(74) Representative: **Owen, Deborah Jane Dehns St Bride's House 10 Salisbury Square London EC4Y 8JD (GB)**

Remarks:
•This application was filed on 28-06-2012 as a divisional application to the application mentioned under INID code 62.
•Claims filed after the date of filing of the application/ after the date of receipt of the divisional application (Rule 68(4) EPC).

# (54) Methods involving genes encoding nucleoside diphosphatase kinase (NDK) polypeptides and homologs thereof for modifying the plant's root architecture

(57) Isolated polynucleotides and potypeptides and recombinant DNA constructs particularly useful for altering root structure of plants, compositions (such as plants or seeds) comprising these recombinant DNA constructs, and methods utilizing these recombinant DNA constructs. The recombinant DNA construct comprises a polynucleotide operably linked to a promoter functional in a plant, wherein said polynucleotide encodes a polypeptide useful for altering plant root architecture.

Fig.1

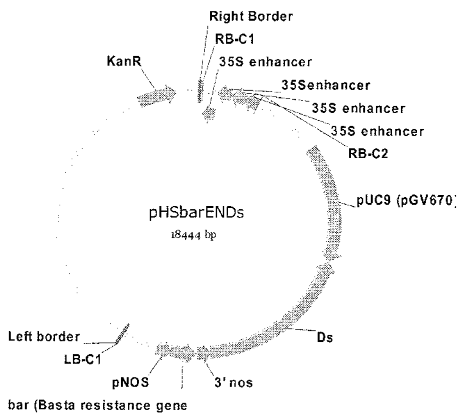


EP 2 527 449 A2

SEQUENCE LISTING

<110> DuPont de Nemours
Taramino, Graziana
Tingey, Scott
Sakai, Hajime
Allen, Steve
Tomes, Dwight
Luck, Stanley
Niu, Xiaomu

<120> PLANTS WITH ALTERED ROOT ARCHITECTURE, RELATED CONSTRUCTS AND METHODS INVOLVING GENES ENCODING NUCLEOSIDE DIPHOSPHATASE KINASE (NDK) POLYPEPTIDES AND HOMOLOGS THEREOF

<130> BB1573 USNA

<150> 60/968754
<151> 2007-08-29

<160> 61

<170> PatentIn version 3.3

<210> 1
<211> 18444
<212> DNA
<213> artificial sequence

<220>
<223> vector

<400> 1
```
catgaatcaa acaaacatac acagcgactt attcacacga gctcaaatta caacggtata      60

tatcctgccg tcgacaacca tggtctagac aggatccccg ggtaccgagc tcgaatttgc     120

aggtcgactg cgtcatccct tacgtcagtg gagatatcac atcaatccac ttgctttgaa     180

gacgtggttg gaacgtcttc tttttccacg atgctcctcg tgggtggggg tccatctttg     240

ggaccactgt cggcagaggc atcttgaacg atagcctttc ctttatcgca atgatggcat     300

ttgtaggtgc caccttcctt ttctactgtc cttttgatga agtgacagat agctgggcaa     360

tggaatccga ggaggtttcc cgatattacc ctttgttgaa aagtctcaat tgccctttgg     420

tcttctgaga ctgttgcgtc atcccttacg tcagtggaga tatcacatca atccacttgc     480

tttgaagacg tggttggaac gtcttctttt tccacgatgc tcctcgtggg tgggggtcca     540

tctttgggac cactgtcggc agaggcatct tgaacgatag cctttccttt atcgcaatga     600

tggcatttgt aggtgccacc ttccttttct actgtccttt tgatgaagtg acagatagct     660

gggcaatgga atccgaggag gtttcccgat attacccttt gttgaaaagt ctcagttaac     720

ccgcgatcct gcgtcatccc ttacgtcagt ggagatatca catcaatcca cttgctttga     780

agacgtggtt ggaacgtctt ctttttccac gatgctcctc gtgggtgggg gtccatcttt     840

gggaccactg tcggcagagg catcttgaac gatagccttt cctttatcgc aatgatggca     900

tttgtaggtg ccaccttcct tttctactgt ccttttgatg aagtgacaga tagctgggca     960
```

atggaatccg aggaggtttc ccgatattac cctttgttga aaagtctcaa ttgccctttg 1020
gtcttctgag actgttgcgt catcccttac gtcagtggag atatcacatc aatccacttg 1080
ctttgaagac gtggttggaa cgtcttcttt ttccacgatg ctcctcgtgg gtgggggtcc 1140
atctttggga ccactgtcgg cagaggcatc ttgaacgata gcctttcctt tatcgcaatg 1200
atggcatttg taggtgccac cttccttttc tactgtcctt ttgatgaagt gacagatagc 1260
tgggcaatgg aatccgagga ggtttcccga tattaccctt tgttgaaaag tctcagttaa 1320
cccgcaattc actggccgtc gttttacaac gtcgtgactg ggaaaaccct ggcgttaccc 1380
aacttaatcg ccttgcagca catcccctt tcgccagctg gcgtaatagc gaagaggccc 1440
gcaccgatcg cccttcccaa cagttgcgca gcctgaatgg cgaatggatc gatccgtcga 1500
tcgaccaaag cggccatcgt gcctccccac tcctgcagtt cgggggcatg gatgcgcgga 1560
tagccgctgc tggtttcctg gatgccgacg gatttgcact gccggtagaa ctccgcgagg 1620
tcgtccagcc tcaggcagca gctgaaccaa ctcgcgaggg gatcgagccc ctgctgagcc 1680
tcgacatgtt gtcgcaaaat tcgccctgga cccgcccaac gatttgtcgt cactgtcaag 1740
gtttgacctg cacttcattt ggggcccaca tacaccaaaa aaatgctgca taattctcgg 1800
ggcagcaagt cggttacccg gccgccgtgc tggaccgggt tgaatggtgc ccgtaacttt 1860
cggtagagcg gacggccaat actcaacttc aaggaatctc acccatgcgc gccggcgggg 1920
aaccggagtt cccttcagtg aacgttatta gttcgccgct cggtgtgtcg tagatactag 1980
cccctggggc cttttgaaat ttgaataaga tttatgtaat cagtctttta ggtttgaccg 2040
gttctgccgc tttttttaaa attggatttg taataataaa acgcaattgt ttgttattgt 2100
ggcgctctat catagatgtc gctataaacc tattcagcac aatatattgt tttcatttta 2160
atattgtaca tataagtagt agggtacaat cagtaaattg aacggagaat attattcata 2220
aaaatacgat agtaacgggt gatatattca ttagaatgaa ccgaaaccgg cggtaaggat 2280
ctgagctaca catgctcagg tttttacaa cgtgcacaac agaattgaaa gcaaatatca 2340
tgcgatcata ggcgtctcgc atatctcatt aaagcagggg gtgggcgaag aactccagca 2400
tgagatcccc gcgctggagg atcatccagc cggcgtcccg gaaaacgatt ccgaagccca 2460
acctttcata gaaggcggcg gtggaatcga aatctcgtga tggcaggttg ggcgtcgctt 2520
ggtcggtcat ttcgaacccc agagtcccgc tcagaagaac tcgtcaagaa ggcgatagaa 2580
ggcgatgcgc tgcgaatcgg gagcggcgat accgtaaagc acgaggaagc ggtcagccca 2640
ttcgccgcca agctcttcag caatatcacg ggtagccaac gctatgtcct gatagcggtc 2700
cgccacaccc agccggccac agtcgatgaa tccagaaaag cggccatttt ccaccatgat 2760
attcggcaag caggcatcgc catgggtcac gacgagatcc tcgccgtcgg gcatgccccc 2820
caattcactg gccgtcgttt tacaacgtcg tgactgggaa aaccctggcg ttacccaact 2880

taatcgcctt gcagcacatc cccctttcgc cagctggcgt aatagcgaag aggcccgcac 2940
cgatcgccct tcccaacagt tgcgcagcct gaatggcgaa tggcgcctga tgcggtattt 3000
tctccttacg catctgtgcg gtatttcaca ccgcatatgg tgcactctca gtacaatctg 3060
ctctgatgcc gcatagttaa gccagccccg acacccgcca acacccgctg acgcgccctg 3120
acgggcttgt ctgctcccgg catccgctta cagacaagct gtgaccgtct ccgggagctg 3180
catgtgtcag aggttttcac cgtcatcacc gaaacgcgcg agacgaaagg gcctcgtgat 3240
acgcctattt ttataggtta atgtcatgat aataatggtt tcttagacgt caggtggcac 3300
ttttcgggga aatgtgcgcg gaacccctat ttgtttattt ttctaaatac attcaaatat 3360
gtatccgctc atgagacaat aaccctgata aatgcttcaa taatattgaa aaaggaagag 3420
tatgagtatt caacatttcc gtgtcgccct tattcccttt tttgcggcat tttgccttcc 3480
tgtttttgct cacccagaaa cgctggtgaa agtaaaagat gctgaagatc agttgggtgc 3540
acgagtgggt tacatcgaac tggatctcaa cagcggtaag atccttgaga gttttcgccc 3600
cgaagaacgt tttccaatga tgagcacttt taaagttctg ctatgtggcg cggtattatc 3660
ccgtattgac gccgggcaag agcaactcgg tcgccgcata cactattctc agaatgactt 3720
ggttgagtac tcaccagtca cagaaaagca tcttacggat ggcatgacag taagagaatt 3780
atgcagtgct gccataacca tgagtgataa cactgcggcc aacttacttc tgacaacgat 3840
cggaggaccg aaggagctaa ccgctttttt gcacaacatg ggggatcatg taactcgcct 3900
tgatcgttgg gaaccggagc tgaatgaagc cataccaaac gacgagcgtg acaccacgat 3960
gcctgtagca atggcaacaa cgttgcgcaa actattaact ggcgaactac ttactctagc 4020
ttcccggcaa caattaatag actggatgga ggcggataaa gttgcaggac cacttctgcg 4080
ctcggccctt ccggctggct ggtttattgc tgataaatct ggagccggtg agcgtgggtc 4140
tcgcggtatc attgcagcac tggggccaga tggtaagccc tcccgtatcg tagttatcta 4200
cacgacgggg agtcaggcaa ctatggatga acgaaataga cagatcgctg agataggtgc 4260
ctcactgatt aagcattggt aactgtcaga ccaagtttac tcatatatac tttagattga 4320
tttaaaactt catttttaat ttaaaaggat ctaggtgaag atcctttttg ataatctcat 4380
gaccaaaatc ccttaacgtg agttttcgtt ccactgagcg tcagaccccg tagaaaagat 4440
caaaggatct tcttgagatc ctttttttct gcgcgtaatc tgctgcttgc aaacaaaaaa 4500
accaccgcta ccagcggtgg tttgtttgcc ggatcaagag ctaccaactc tttttccgaa 4560
ggtaactggc ttcagcagag cgcagatacc aaatactgtc cttctagtgt agccgtagtt 4620
aggccaccac ttcaagaact ctgtagcacc gcctacatac ctcgctctgc taatcctgtt 4680
accagtggct gctgccagtg gcgataagtc gtgtcttacc gggttggact caagacgata 4740
gttaccggat aaggcgcagc ggtcgggctg aacggggggt tcgtgcacac agcccagctt 4800
ggagcgaacg acctacaccg aactgagata cctacagcgt gagcattgag aaagcgccac 4860

gcttcccgaa gggagaaagg cggacaggta tccggtaagc ggcagggtcg gaacaggaga 4920
gcgcacgagg gagcttccag ggggaaacgc ctggtatctt tatagtcctg tcgggtttcg 4980
ccacctctga cttgagcgtc gatttttgtg atgctcgtca ggggggcgga gcctatggaa 5040
aaacgccagc aacgcggcct ttttacggtt cctggccttt tgctggcctt ttgctcacat 5100
gttctttcct gcgttatccc ctgattctgt ggataaccgt attaccgcct ttgagtgagc 5160
tgataccgct cgccgcagcc gaacgaccga gcgcagcgag tcagtgagcg aggaagcgga 5220
agagcgccca atacgcaaac cgcctctccc cgcgcgttgg ccgattcatt aatgcagctg 5280
gcacgacagg tttcccgact ggaaagcggg cagtgagcgc aacgcaatta atgtgagtta 5340
gctcactcat taggcacccc aggctttaca ctttatgctt ccggctcgta tgttgtgtgg 5400
aattgtgagc ggataacaat ttcacacagg aaacagctat gaccatgatt acgccaagct 5460
ttctaggggg ggggtaccga tctgagatcg gtaacgaaaa cgaacgggta gggatgaaaa 5520
cggtcggtaa cggtcggtaa aatacctcta ccgttttcat tttcatattt aacttgcggg 5580
acggaaacga aaacgggata taccggtaac gaaaacgaac gggataaata cggtaatcga 5640
aaaccgatac gatccggtcg ggttaaagtc gaaatcggac gggaaccggt atttttgttc 5700
ggtaaaatca cacatgaaaa catatattca aaacttaaaa acaaatataa aaaattgtaa 5760
acacaagtct taattaaaca tagataaaat ccatataaat ctggagcaca catagtttaa 5820
tgtagcacat aagtgataag tcttgggctc ttggctaaca taagaagcca tataagtcta 5880
ctagcacaca tgacacaata taaagtttaa aacacatatt cataatcact tgctcacatc 5940
tggatcactt agcatgctac agctagtgca atattagaca ctttccaata tttctcaaac 6000
ttttcactca ttgcaacggc cattctccta atgacaaatt tttcatgaac acaccattgg 6060
tcaatcaaat cctttatctc acagaaacct ttgtaaaata aatttgcagt ggaatattga 6120
gtaccagata ggagttcagt gagatcaaaa aacttcttca aacacttaaa aagagttaat 6180
gccatcttcc actcctcggc tttaggacaa attgcatcgt acctacaata attgacattt 6240
gattaattga gaatttataa tgatgacatg tacaacaatt gagacaaaca tacctgcgag 6300
gatcacttgt tttaagccgt gttagtgcag gcttataata taaggcatcc ctcaacatca 6360
aataggttga attccatcta gttgagacat catatgagat ccctttagat ttatccaagt 6420
cacattcact agcacacttc attagttctt cccactgcaa aggagaagat tttacagcaa 6480
gaacaatcgc tttgattttc tcaattgttc ctgcaattac agccaagcca tcctttgcaa 6540
ccaagttcag tatgtgacaa gcacacctca catgaaagaa agcaccatca caaactagat 6600
ttgaatcagt gtcctgcaaa tcctcaatta tatcgtgcac agctacttca tttgcactag 6660
cattatccaa agacaaggca aacaattttt tctcaatgtt ccacttaacc atgattgcag 6720
tgaaggtttg tgataacctt tggccagtgt ggcgcccttc aacatgaaaa aagccaacaa 6780

ttcttttttg gagacaccaa tcatcatcaa tccaatggat ggtgacacac atgtatgact 6840
tattttgaca agatgtccac atatccatag ttgtactgaa gcgagactga acatctttta 6900
gttttccata caacttttct ttttcttcca aatacaaatc catgatatat tttctagcag 6960
tgacacggga ctttattgga aagtgagggc gcagagactt aacaaactca acaaagtact 7020
catgttctac aatattgaaa ggatattcat gcatgattat tgccaaatga agcttcttta 7080
ggctaaccac ttcatcgtac ttataaggct caatgagatt tatgtctttg ccatgatcct 7140
tttcactttt tagacacaac tgacctttaa ctaaactatg tgatgttctc aagtgatttc 7200
gaaatccgct tgttccatga tgaccctcag ccctatactt agccttgcaa ttaggaaagt 7260
tgcaatgtcc ccatacctga acgtatttct ttccatcgac ctccacttca atttccttct 7320
tggtgaaatg ctgccataca tccgatgtgc acttctttgc cctcttctgt ggtgcttctt 7380
cttcgggttc aggttgtggc tgtggttgtg gttctggttg tggttgtggt tgtggttgtg 7440
gttcatgaac aatagccata tcatcttgac tcggatctgt agctgtacca tttgcattac 7500
tactgcttac actctgaata aaatgcctct cggcctcagc tgttgatgat gatggtgatg 7560
tgcggccaca tccatgccca cgcgcacgtg cacgtacatt ctgaatccga ctagaagagg 7620
cttcagcttt tcttttcaac cctgttataa acagattttt cgtattattc tacagtcaat 7680
atgatgcttc ccaatctaca accaattagt aatgctaatg ctattgctac tgtttttcta 7740
atatatacct tgagcatatg cagagaatac ggaatttgtt ttgcgagtag aaggcgctct 7800
tgtggtagac atcaacttgg ccaatcttat ggctgagcct gagggaggat tatttccaac 7860
cggaggcgtc atctgaggaa tggagtcgta gccggctagc cgaagtggag agcagagccc 7920
tggacagcag gtgttcagca atcagcttgg tgctgtactg ctgtgacttg tgagcacctg 7980
gacggctgga cagcaatcag caggtgttgc agagcccctg gacagcacac aaatgacaca 8040
acagcttggt gcaatggtgc tgacgtgctg tactgctaag tgctgtgagc ctgtgagcag 8100
ccgtggagac agggagaccg cggatggccg gatgggcgag cgccgagcag tggaggtctg 8160
gaggaccgct gaccgcagat ggcggatggc ggatgggcgg accgcggatg ggcgagcagt 8220
ggagtggagg tctgggcgga tgggcggacc gcggcgcgga tgggcgagtc gcgagcagtg 8280
gagtggaggg cggaccgtgg atggcggcgt ctgcgtccgg cgtgccgcgt cacggccgtc 8340
accgcgtgtg gtgcctggtg cagcccagcg gccggccggc tgggagacag ggagagtcgg 8400
agagagcagg cgagagcgag acgcgtcgcc ggcgtcggcg tgcggctggc ggcgtccgga 8460
ctccggcgtg ggcgcgtggc ggcgtgtgaa tgtgtgatgc tgttactcgt gtggtgcctg 8520
gccgcctggg agagaggcag agcagcgttc gctaggtatt tcttacatgg gctgggcctc 8580
agtggttatg gatgggagtt ggagctggcc atattgcagt catcccgaat tagaaaatac 8640
ggtaacgaaa cgggatcatc ccgattaaaa acgggatccc ggtgaaacgg tcgggaaact 8700
agctctaccg tttccgtttc cgtttaccgt tttgtatatc ccgtttccgt tccgttttcg 8760

tttttttacct cgggttcgaa atcgatcggg ataaaactaa caaaatcggt tatacgataa 8820
cggtcggtac gggattttcc catcctactt tcatccctga gattattgtc gtttctttcg 8880
cagatcggta ccccccccct agagtcgaca tcgatctagt aacatagatg acaccgcgcg 8940
cgataattta tcctagtttg cgcgctatat tttgttttct atcgcgtatt aaatgtataa 9000
ttgcgggact ctaatcataa aaacccatct cataaataac gtcatgcatt acatgttaat 9060
tattacatgc ttaacgtaat tcaacagaaa ttatatgata atcatcgcaa gaccggcaac 9120
aggattcaat cttaagaaac tttattgcca aatgtttgaa cgatctgctt cgacgcactc 9180
cttctttagg tacggactag atctcggtga cgggcaggac cggacggggc ggtaccggca 9240
ggctgaagtc cagctgccag aaacccacgt catgccagtt cccgtgcttg aagccggccg 9300
cccgcagcat gccgcggggg gcatatccga gcgcctcgtg catgcgcacg ctcgggtcgt 9360
tgggcagccc gatgacagcg accacgctct tgaagccctg tgcctccagg gacttcagca 9420
ggtgggtgta gagcgtggag cccagtcccg tccgctggtg gcggggggag acgtacacgg 9480
tcgactcggc cgtccagtcg taggcgttgc gtgccttcca ggggcccgcg taggcgatgc 9540
cggcgacctc gccgtccacc tcggcgacga gccagggata gcgctcccgc agacggacga 9600
ggtcgtccgt ccactcctgc ggttcctgcg gctcggtacg gaagttgacc gtgcttgtct 9660
cgatgtagtg gttgacgatg gtgcagaccg ccggcatgtc cgcctcggtg gcacggcgga 9720
tgtcggccgg gcgtcgttct gggctcatgg atctggattg agagtgaata tgagactcta 9780
attggatacc gaggggaatt tatggaacgt cagtggagca tttttgacaa gaaatatttg 9840
ctagctgata gtgaccttag gcgacttttg aacgcgcaat aatggtttct gacgtatgtg 9900
cttagctcat taaactccag aaacccgcgg ctgagtggct ccttcaatcg ttgcggttct 9960
gtcagttcca aacgtaaaac ggcttgtccc gcgtcatcgg cgggggtcat aacgtgactc 10020
ccttaattct ccgctcatga tccccgggta ccgagctcga attgcggctg agtggctcct 10080
tcaatcgttg cggttctgtc agttccaaac gtaaaacggc ttgtcccgcg tcatcggcgg 10140
gggtcataac gtgactccct taattctccg ctcatgatct tgatcccctg cgccatcaga 10200
tccttggcgg caagaaagcc atccagttta ctttgcaggg cttcccaacc ttaccagagg 10260
gcgccccagc tggcaattcc ggttcgcttg ctgtatcgat atggtggatt tatcacaaat 10320
gggacccgcc gccgacagag gtgtgatgtt aggccaggac tttgaaaatt tgcgcaacta 10380
tcgtatagtg gccgacaaat tgacgccgag ttgacagact gcctagcatt tgagtgaatt 10440
atgtgaggta atgggctaca ctgaattggt agctcaaact gtcagtattt atgtatatga 10500
gtgtatattt tcgcataatc tcagaccaat ctgaagatga aatgggtatc tgggaatggc 10560
gaaatcaagg catcgatcgt gaagtttctc atctaagccc ccatttggac gtgaatgtag 10620
acacgtcgaa ataaagattt ccgaattaga ataatttgtt tattgctttc gcctataaat 10680

acgacggatc gtaatttgtc gttttatcaa aatgtacttt cattttataa taacgctgcg 10740
gacatctaca tttttgaatt gaaaaaaaat tggtaattac tctttctttt tctccatatt 10800
gaccatcata ctcattgctg atccatgtag atttcccgga catgaagcca tttacaattg 10860
aatatatcct gccgccgctg ccgctttgca cccggtggag cttgcatgtt ggtttctacg 10920
cagaactgag ccggttaggc agataatttc cattgagaac tgagccatgt gcaccttccc 10980
cccaacacgg tgagcgacgg ggcaacggag tgatccacat gggactttta aacatcatcc 11040
gtcggatggc gttgcgagag aagcagtcga tccgtgagat cagccgacgc accgggcagg 11100
cgcgcaacac gatcgcaaag tatttgaacg caggtacaat cgagccgacg ttcaccgtca 11160
ccctggatgc tgtaggcata ggcttggtta tgccggtact gccgggcctc ttgcgggata 11220
tcgtccattc cgacagcatc gccagtcact atggcgtgct gctagcgcta tatgcgttga 11280
tgcaatttct atgcgcaccc gttctcggag cactgtccga ccgctttggc cgccgcccag 11340
tcctgctcgc ttcgctactt ggagccacta tcgactacgc gatcatggcg accacacccg 11400
tcctgtggtc caacccctcc gctgctatag tgcagtcggc ttctgacgtt cagtgcagcc 11460
gtcttctgaa aacgacatgt cgcacaagtc ctaagttacg cgacaggctg ccgccctgcc 11520
cttttcctgg cgttttcttg tcgcgtgttt tagtcgcata aagtagaata cttgcgacta 11580
gaaccggaga cattacgcca tgaacaagag cgccgccgct ggcctgctgg gctatgcccg 11640
cgtcagcacc gacgaccagg acttgaccaa ccaacgggcc gaactgcacg cggccggctg 11700
caccaagctg ttttccgaga agatcaccgg caccaggcgc gaccgcccgg agctggccag 11760
gatgcttgac cacctacgcc ctggcgacgt tgtgacagtg accaggctag accgcctggc 11820
ccgcagcacc cgcgacctac tggacattgc cgagcgcatc caggaggccg gcgcgggcct 11880
gcgtagcctg gcagagccgt gggccgacac caccacgccg gccggccgca tggtgttgac 11940
cgtgttcgcc ggcattgccg agttcgagcg ttccctaatc atcgaccgca cccggagcgg 12000
gcgcgaggcc gccaaggccc gaggcgtgaa gtttggcccc cgccctaccc tcaccccggc 12060
acagatcgcg cacgcccgcg agctgatcga ccaggaaggc cgcaccgtga aagaggcggc 12120
tgcactgctt ggcgtgcatc gctcgaccct gtaccgcgca cttgagcgca gcgaggaagt 12180
gacgcccacc gaggccaggc ggcgcggtgc cttccgtgag gacgcattga ccgaggccga 12240
cgccctggcg gccgccgaga atgaacgcca agaggaacaa gcatgaaacc gcaccaggac 12300
ggccaggacg aaccgttttt cattaccgaa gagatcgagg cggagatgat cgcggccggg 12360
tacgtgttcg agccgcccgc gcacgtctca accgtgcggc tgcatgaaat cctggccggt 12420
ttgtctgatg ccaagctggc ggcctggccg gccagcttgg ccgctgaaga aaccgagcgc 12480
cgccgtctaa aaaggtgatg tgtatttgag taaaacagct tgcgtcatgc ggtcgctgcg 12540
tatatgatgc gatgagtaaa taaacaaata cgcaagggaa cgcatgaagt tatcgctgta 12600
cttaaccaga aaggcgggtc aggcaagacg accatcgcaa cccatctagc ccgcgccctg 12660

caactcgccg gggccgatgt tctgttagtc gattccgatc cccagggcag tgcccgcgat 12720
tgggcggccg tgcgggaaga tcaaccgcta accgttgtcg gcatcgaccg cccgacgatt 12780
gaccgcgacg tgaaggccat cggccggcgc gacttcgtag tgatcgacgg agcgccccag 12840
gcggcggact tggctgtgtc cgcgatcaag gcagccgact tcgtgctgat tccggtgcag 12900
ccaagccctt acgacatatg ggccaccgcc gacctggtgg agctggttaa gcagcgcatt 12960
gaggtcacgg atggaaggct acaagcggcc tttgtcgtgt cgcgggcgat caaaggcacg 13020
cgcatcggcg gtgaggttgc cgaggcgctg gccgggtacg agctgcccat tcttgagtcc 13080
cgtatcacgc agcgcgtgag ctacccaggc actgccgccg ccggcacaac cgttcttgaa 13140
tcagaacccg agggcgacgc tgcccgcgag gtccaggcgc tggccgctga aattaaatca 13200
aaactcattt gagttaatga ggtaaagaga aaatgagcaa aagcacaaac acgctaagtg 13260
ccggccgtcc gagcgcacgc agcagcaagg ctgcaacgtt ggccagcctg gcagacacgc 13320
cagccatgaa gcgggtcaac tttcagttgc cggcggagga tcacaccaag ctgaagatgt 13380
acgcggtacg ccaaggcaag accattaccg agctgctatc tgaatacatc gcgcagctac 13440
cagagtaaat gagcaaatga ataaatgagt agatgaattt tagcggctaa aggaggcggc 13500
atggaaaatc aagaacaacc aggcaccgac gccgtggaat gccccatgtg tggaggaacg 13560
ggcggttggc caggcgtaag cggctgggtt gtctgccggc cctgcaatgg cactggaacc 13620
cccaagcccg aggaatcggc gtgagcggtc gcaaaccatc cggcccggta caaatcggcg 13680
cggcgctggg tgatgacctg gtggagaagt tgaaggccgc gcaggccgcc cagcggcaac 13740
gcatcgaggc agaagcacgc cccggtgaat cgtggcaagc ggccgctgat cgaatccgca 13800
aagaatcccg gcaaccgccg gcagccggtg cgccgtcgat taggaagccg cccaagggcg 13860
acgagcaacc agattttttc gttccgatgc tctatgacgt gggcacccgc gatagtcgca 13920
gcatcatgga cgtggccgtt ttccgtctgt cgaagcgtga ccgacgagct ggcgaggtga 13980
tccgctacga gcttccagac gggcacgtag aggtttccgc agggccggcc ggcatggcca 14040
gtgtgtggga ttacgacctg gtactgatgg cggtttccca tctaaccgaa tccatgaacc 14100
gataccggga agggaaggga gacaagcccg gccgcgtgtt ccgtccacac gttgcggacg 14160
tactcaagtt ctgccggcga gccgatggcg gaaagcagaa agacgacctg gtagaaacct 14220
gcattcggtt aaacaccacg cacgttgcca tgcagcgtac gaagaaggcc aagaacggcc 14280
gcctggtgac ggtatccgag ggtgaagcct tgattagccg ctacaagatc gtaaagagcg 14340
aaaccgggcg gccggagtac atcgagatcg agctagctga ttggatgtac cgcgagatca 14400
cagaaggcaa gaacccggac gtgctgacgg ttcaccccga ttactttttg atcgatcccg 14460
gcatcggccg ttttctctac cgcctggcac gccgcgccgc aggcaaggca gaagccagat 14520
ggttgttcaa gacgatctac gaacgcagtg gcagcgccgg agagttcaag aagttctgtt 14580

tcaccgtgcg caagctgatc gggtcaaatg acctgccgga gtacgatttg aaggaggagg 14640
cggggcaggc tggcccgatc ctagtcatgc gctaccgcaa cctgatcgag ggcgaagcat 14700
ccgccggttc ctaatgtacg gagcagatgc tagggcaaat tgccctagca ggggaaaaag 14760
gtcgaaaagg tctctttcct gtggatagca cgtacattgg gaacccaaag ccgtacattg 14820
ggaaccggaa cccgtacatt gggaacccaa agccgtacat tgggaaccgg tcacacatgt 14880
aagtgactga tataaaagag aaaaaaggcg atttttccgc ctaaaactct ttaaaactta 14940
ttaaaactct taaaacccgc ctggcctgtg cataactgtc tggccagcgc acagccgaag 15000
agctgcaaaa agcgcctacc cttcggtcgc tgcgctccct acgccccgcc gcttcgcgtc 15060
ggcctatcgc ggccgctggc cgctcaaaaa tggctggcct acggccaggc aatctaccag 15120
ggcgcggaca agccgcgccg tcgccactcg accgccggcg cccacatcaa ggcaccctgc 15180
ctcgcgcgtt tcggtgatga cggtgaaaac ctctgacaca tgcagctccc ggagacggtc 15240
acagcttgtc tgtaagcgga tgccgggagc agacaagccc gtcagggcgc gtcagcgggt 15300
gttggcgggt gtcggggcgc agccatgacc cagtcacgta gcgatagcgg agtgtatact 15360
ggcttaacta tgcggcatca gagcagattg tactgagagt gcaccatatg cggtgtgaaa 15420
taccgcacag atgcgtaagg agaaaatacc gcatcaggcg ctcttccgct tcctcgctca 15480
ctgactcgct gcgctcggtc gttcggctgc ggcgagcggt atcagctcac tcaaaggcgg 15540
taatacggtt atccacagaa tcaggggata acgcaggaaa gaacatgtga gcaaaaggcc 15600
agcaaaaggc caggaaccgt aaaaaggccg cgttgctggc gtttttccat aggctccgcc 15660
cccctgacga gcatcacaaa aatcgacgct caagtcagag gtggcgaaac ccgacaggac 15720
tataaagata ccaggcgttt ccccctggaa gctccctcgt gcgctctcct gttccgaccc 15780
tgccgcttac cggatacctg tccgcctttc tcccttcggg aagcgtggcg ctttctcata 15840
gctcacgctg taggtatctc agttcggtgt aggtcgttcg ctccaagctg ggctgtgtgc 15900
acgaaccccc cgttcagccc gaccgctgcg ccttatccgg taactatcgt cttgagtcca 15960
acccggtaag acacgactta tcgccactgg cagcagccac tggtaacagg attagcagag 16020
cgaggtatgt aggcggtgct acagagttct tgaagtggtg gcctaactac ggctacacta 16080
gaaggacagt atttggtatc tgcgctctgc tgaagccagt taccttcgga aaaagagttg 16140
gtagctcttg atccggcaaa caaaccaccg ctggtagcgg tggttttttt gtttgcaagc 16200
agcagattac gcgcagaaaa aaaggatctc aagaagatcc tttgatcttt tctacggggt 16260
ctgacgctca gtggaacgaa aactcacgtt aagggatttt ggtcatgaga ttatcaaaaa 16320
ggatcttcac ctagatcctt ttaaattaaa aatgaagttt taaatcaatc taaagtatat 16380
atgagtaaac ttggtctgac agttaccaat gcttaatcag tgaggcacct atctcagcga 16440
tctgtctatt tcgttcatcc atagttgcct gactccccgt cgtgtagata actacgatac 16500
gggagggctt accatctggc cccagtgctg caatgatacc gcgagaccca cgctcaccgg 16560

ctccagattt atcagcaata aaccagccag ccggaagggc cgagcgcaga agtggtcctg 16620
caactttatc cgcctccatc cagtctatta attgttgccg ggaagctaga gtaagtagtt 16680
cgccagttaa tagtttgcgc aacgttgttg ccattgctac aggcatcgtg gtgtcacgct 16740
cgtcgtttgg tatggcttca ttcagctccg gttcccaacg atcaaggcga gttacatgat 16800
cccccatgtt gtgcaaaaaa gcggttagct ccttcggtcc tccgatcgtt gtcagaagta 16860
agttggccgc agtgttatca ctcatggtta tggcagcact gcataattct cttactgtca 16920
tgccatccgt aagatgcttt tctgtgactg gtgagtactc aaccaagtca ttctgagaat 16980
agtgtatgcg gcgaccgagt tgctcttgcc cggcgtcaac acgggataat accgcgccac 17040
atagcagaac tttaaaagtg ctcatcattg gaaaagacct gcaggggggg gggggaaagc 17100
cacgttgtgt ctcaaaatct ctgatgttac attgcacaag ataaaaatat atcatcatga 17160
acaataaaac tgtctgctta cataaacagt aatacaaggg gtgttatgag ccatattcaa 17220
cgggaaacgt cttgctcgag gccgcgatta aattccaaca tggatgctga tttatatggg 17280
tataaatggg ctcgcgataa tgtcgggcaa tcaggtgcga caatctatcg attgtatggg 17340
aagcccgatg cgccagagtt gtttctgaaa catggcaaag gtagcgttgc caatgatgtt 17400
acagatgaga tggtcagact aaactggctg acggaattta tgcctcttcc gaccatcaag 17460
cattttatcc gtactcctga tgatgcatgg ttactcacca ctgcgatccc cgggaaaaca 17520
gcattccagg tattagaaga atatcctgat tcaggtgaaa atattgttga tgcgctggca 17580
gtgttcctgc gccggttgca ttcgattcct gtttgtaatt gtccttttaa cagcgatcgc 17640
gtatttcgtc tcgctcaggc gcaatcacga atgaataacg gtttggttga tgcgagtgat 17700
tttgatgacg agcgtaatgg ctggcctgtt gaacaagtct ggaaagaaat gcataagctt 17760
ttgccattct caccggattc agtcgtcact catggtgatt tctcacttga taaccttatt 17820
tttgacgagg ggaaattaat aggttgtatt gatgttggac gagtcggaat cgcagaccga 17880
taccaggatc ttgccatcct atggaactgc ctcggtgagt tttctccttc attacagaaa 17940
cggctttttc aaaaatatgg tattgataat cctgatatga ataaattgca gtttcatttg 18000
atgctcgatg agtttttcta atcagaattg gttaattggt tgtaacactg gcagagcatt 18060
acgctgactt gacgggacgg cggctttgtt gaataaatcg aacttttgct gagttgaagg 18120
atcagatcac gcatcttccc gacaacgcag accgttccgt ggcaaagcaa aagttcaaaa 18180
tcaccaactg gtccacctac aacaaagctc tcatcaaccg tggctccctc actttctggc 18240
tggatgatgg ggcgattcag gcctggtatg agtcagcaac accttcttca cgaggcagac 18300
ctcagcgccc ccccccccct gcaggtcaat tcggtcgata tggctattac gaagaaggct 18360
cgtgcgcgga gtcccgtgaa ctttcccacg caacaagtga accgcaccgg gtttgccgga 18420
ggccatttcg ttaaaatgcg cagc 18444

<210> 2
<211> 4291
<212> DNA
<213> artificial sequence

<220>
<223> vector

<400> 2
ctttcctgcg ttatcccctg attctgtgga taaccgtatt accgcctttg agtgagctga 60

taccgctcgc cgcagccgaa cgaccgagcg cagcgagtca gtgagcgagg aagcggaaga 120

gcgcccaata cgcaaaccgc ctctccccgc gcgttggccg attcattaat gcagctggca 180

cgacaggttt cccgactgga aagcgggcag tgagcgcaac gcaattaata cgcgtaccgc 240

tagccaggaa gagtttgtag aaacgcaaaa aggccatccg tcaggatggc cttctgctta 300

gtttgatgcc tggcagttta tggcgggcgt cctgcccgcc accctccggg ccgttgcttc 360

acaacgttca aatccgctcc cggcggattt gtcctactca ggagagcgtt caccgacaaa 420

caacagataa aacgaaaggc ccagtcttcc gactgagcct ttcgttttat ttgatgcctg 480

gcagttccct actctcgcgt taacgctagc atggatgttt tcccagtcac gacgttgtaa 540

aacgacggcc agtcttaagc tcgggcccca aataatgatt ttattttgac tgatagtgac 600

ctgttcgttg caacacattg atgagcaatg cttttttata atgccaactt tgtacaaaaa 660

agctgaacga gaaacgtaaa atgatataaa tatcaatata ttaaattaga ttttgcataa 720

aaaacagact acataatact gtaaaacaca acatatccag tcactatgaa tcaactactt 780

agatggtatt agtgacctgt agtcgaccga cagccttcca aatgttcttc gggtgatgct 840

gccaacttag tcgaccgaca gccttccaaa tgttcttctc aaacggaatc gtcgtatcca 900

gcctactcgc tattgtcctc aatgccgtat taaatcataa aaagaaataa gaaaaagagg 960

tgcgagcctc ttttttgtgt gacaaaataa aaacatctac ctattcatat acgctagtgt 1020

catagtcctg aaaatcatct gcatcaagaa caatttcaca actcttatac ttttctctta 1080

caagtcgttc ggcttcatct ggattttcag cctctatact tactaaacgt gataaagttt 1140

ctgtaatttc tactgtatcg acctgcagac tggctgtgta taagggagcc tgacatttat 1200

attccccaga acatcaggtt aatggcgttt ttgatgtcat tttcgcggtg gctgagatca 1260

gccacttctt ccccgataac ggagaccggc acactggcca tatcggtggt catcatgcgc 1320

cagctttcat ccccgatatg caccaccggg taaagttcac gggagacttt atctgacagc 1380

agacgtgcac tggccagggg gatcaccatc cgtcgcccgg gcgtgtcaat aatatcactc 1440

tgtacatcca caaacagacg ataacggctc tctcttttat aggtgtaaac cttaaactgc 1500

atttcaccag cccctgttct cgtcagcaaa agagccgttc atttcaataa accgggcgac 1560

ctcagccatc ccttcctgat tttccgcttt ccagcgttcg gcacgcagac gacgggcttc 1620

attctgcatg gttgtgctta ccagaccgga gatattgaca tcatatatgc cttgagcaac 1680

tgatagctgt cgctgtcaac tgtcactgta atacgctgct tcatagcata cctcttttttg 1740
acatacttcg ggtatacata tcagtatata ttcttatacc gcaaaaatca gcgcgcaaat 1800
acgcatactg ttatctggct tttagtaagc cggatccacg cggcgtttac gccccgccct 1860
gccactcatc gcagtactgt tgtaattcat taagcattct gccgacatgg aagccatcac 1920
agacggcatg atgaacctga atcgccagcg gcatcagcac cttgtcgcct tgcgtataat 1980
atttgcccat ggtgaaaacg ggggcgaaga agttgtccat attggccacg tttaaatcaa 2040
aactggtgaa actcacccag ggattggctg agacgaaaaa catattctca ataaaccctt 2100
tagggaaata ggccaggttt tcaccgtaac acgccacatc ttgcgaatat atgtgtagaa 2160
actgccggaa atcgtcgtgg tattcactcc agagcgatga aaacgtttca gtttgctcat 2220
ggaaaacggt gtaacaaggg tgaacactat cccatatcac cagctcaccg tctttcattg 2280
ccatacggaa ttccggatga gcattcatca ggcgggcaag aatgtgaata aaggccggat 2340
aaaacttgtg cttatttttc tttacggtct ttaaaaaggc cgtaatatcc agctgaacgg 2400
tctggttata ggtacattga gcaactgact gaaatgcctc aaaatgttct ttacgatgcc 2460
attgggatat atcaacggtg gtatatccag tgattttttt ctccatttta gcttccttag 2520
ctcctgaaaa tctcgataac tcaaaaaata cgcccggtag tgatcttatt tcattatggt 2580
gaaagttgga acctcttacg tgccgatcaa cgtctcattt tcgccaaaag ttggcccagg 2640
gcttcccggt atcaacaggg acaccaggat ttatttattc tgcgaagtga tcttccgtca 2700
caggtattta ttcggcgcaa agtgcgtcgg gtgatgctgc caacttagtc gactacaggt 2760
cactaatacc atctaagtag ttgattcata gtgactggat atgttgtgtt ttacagtatt 2820
atgtagtctg ttttttatgc aaaatctaat ttaatatatt gatatttata tcattttacg 2880
tttctcgttc agctttcttg tacaaagttg gcattataag aaagcattgc ttatcaattt 2940
gttgcaacga acaggtcact atcagtcaaa ataaaatcat tatttgccat ccagctgata 3000
tcccctatag tgagtcgtat tacatggtca tagctgtttc ctggcagctc tggcccgtgt 3060
ctcaaaatct ctgatgttac attgcacaag ataaaaataat atcatcatga tcagtcctgc 3120
tcctcggcca cgaagtgcac gcagttgccg gccgggtcgc gcagggcgaa ctcccgcccc 3180
cacggctgct cgccgatctc ggtcatggcc ggcccggagg cgtcccggaa gttcgtggac 3240
acgacctccg accactcggc gtacagctcg tccaggccgc gcacccacac ccaggccagg 3300
gtgttgtccg gcaccacctg gtcctggacc gcgctgatga acagggtcac gtcgtcccgg 3360
accacaccgg cgaagtcgtc ctccacgaag tcccgggaga acccgagccg gtcggtccag 3420
aactcgaccg ctccggcgac gtcgcgcgcg gtgagcaccg gaacggcact ggtcaacttg 3480
gccatggttt agttcctcac cttgtcgtat tatactatgc cgatatacta tgccgatgat 3540
taattgtcaa cacgtgctga tcatgaccaa aatcccttaa cgtgagttac gcgtcgttcc 3600

actgagcgtc agaccccgta gaaaagatca aaggatcttc ttgagatcct ttttttctgc 3660

gcgtaatctg ctgcttgcaa acaaaaaaac caccgctacc agcggtggtt tgtttgccgg 3720

atcaagagct accaactctt tttccgaagg taactggctt cagcagagcg cagataccaa 3780

atactgttct tctagtgtag ccgtagttag gccaccactt caagaactct gtagcaccgc 3840

ctacatacct cgctctgcta atcctgttac cagtggctgc tgccagtggc gataagtcgt 3900

gtcttaccgg gttggactca agacgatagt taccggataa ggcgcagcgg tcgggctgaa 3960

cggggggttc gtgcacacag cccagcttgg agcgaacgac ctacaccgaa ctgagatacc 4020

tacagcgtga gctatgagaa agcgccacgc ttcccgaagg gagaaaggcg gacaggtatc 4080

cggtaagcgg cagggtcgga acaggagagc gcacgaggga gcttccaggg ggaaacgcct 4140

ggtatcttta tagtcctgtc gggtttcgcc acctctgact tgagcgtcga tttttgtgat 4200

gctcgtcagg ggggcggagc ctatggaaaa acgccagcaa cgcggccttt ttacggttcc 4260

tggccttttg ctggcctttt gctcacatgt t 4291

<210> 3
<211> 4762
<212> DNA
<213> artificial sequence

<220>
<223> vector

<400> 3
ctttcctgcg ttatcccctg attctgtgga taaccgtatt accgcctttg agtgagctga 60

taccgctcgc cgcagccgaa cgaccgagcg cagcgagtca gtgagcgagg aagcggaaga 120

gcgcccaata cgcaaaccgc ctctccccgc gcgttggccg attcattaat gcagctggca 180

cgacaggttt cccgactgga aagcgggcag tgagcgcaac gcaattaata cgcgtaccgc 240

tagccaggaa gagtttgtag aaacgcaaaa aggccatccg tcaggatggc cttctgctta 300

gtttgatgcc tggcagttta tggcgggcgt cctgcccgcc accctccggg ccgttgcttc 360

acaacgttca aatccgctcc cggcggattt gtcctactca ggagagcgtt caccgacaaa 420

caacagataa aacgaaaggc ccagtcttcc gactgagcct ttcgttttat ttgatgcctg 480

gcagttccct actctcgcgt taacgctagc atggatgttt tcccagtcac gacgttgtaa 540

aacgacggcc agtcttaagc tcgggcccca aataatgatt ttattttgac tgatagtgac 600

ctgttcgttg caacacattg atgagcaatg cttttttata atgccaactt tgtacaaaaa 660

agctgaacga gaaacgtaaa atgatataaa tatcaatata ttaaattaga ttttgcataa 720

aaaacagact acataatact gtaaaacaca acatatccag tcactatgaa tcaactactt 780

agatggtatt agtgacctgt agtcgaccga cagccttcca aatgttcttc gggtgatgct 840

gccaacttag tcgaccgaca gccttccaaa tgttcttctc aaacggaatc gtcgtatcca 900

gcctactcgc tattgtcctc aatgccgtat taaatcataa aaagaaataa gaaaaagagg 960

tgcgagcctc ttttttgtgt gacaaaataa aaacatctac ctattcatat acgctagtgt 1020
catagtcctg aaaatcatct gcatcaagaa caatttcaca actcttatac ttttctctta 1080
caagtcgttc ggcttcatct ggattttcag cctctatact tactaaacgt gataaagttt 1140
ctgtaatttc tactgtatcg acctgcagac tggctgtgta taagggagcc tgacatttat 1200
attccccaga acatcaggtt aatggcgttt ttgatgtcat tttcgcggtg gctgagatca 1260
gccacttctt ccccgataac ggagaccggc acactggcca tatcggtggt catcatgcgc 1320
cagctttcat ccccgatatg caccaccggg taaagttcac gggagacttt atctgacagc 1380
agacgtgcac tggccagggg gatcaccatc cgtcgcccgg gcgtgtcaat aatatcactc 1440
tgtacatcca caaacagacg ataacggctc tctcttttat aggtgtaaac cttaaactgc 1500
atttcaccag cccctgttct cgtcagcaaa agagccgttc atttcaataa accgggcgac 1560
ctcagccatc ccttcctgat tttccgcttt ccagcgttcg gcacgcagac gacgggcttc 1620
attctgcatg gttgtgctta ccagaccgga gatattgaca tcatatatgc cttgagcaac 1680
tgatagctgt cgctgtcaac tgtcactgta atacgctgct tcatagcata cctctttttg 1740
acatacttcg ggtatacata tcagtatata ttcttatacc gcaaaaatca gcgcgcaaat 1800
acgcatactg ttatctggct tttagtaagc cggatccacg cggcgtttac gccccgccct 1860
gccactcatc gcagtactgt tgtaattcat taagcattct gccgacatgg aagccatcac 1920
agacggcatg atgaacctga atcgccagcg gcatcagcac cttgtcgcct tgcgtataat 1980
atttgcccat ggtgaaaacg ggggcgaaga agttgtccat attggccacg tttaaatcaa 2040
aactggtgaa actcacccag ggattggctg agacgaaaaa catattctca ataaaccctt 2100
tagggaaata ggccaggttt tcaccgtaac acgccacatc ttgcgaatat atgtgtagaa 2160
actgccggaa atcgtcgtgg tattcactcc agagcgatga aaacgtttca gtttgctcat 2220
ggaaaacggt gtaacaaggg tgaacactat cccatatcac cagctcaccg tctttcattg 2280
ccatacggaa ttccggatga gcattcatca ggcgggcaag aatgtgaata aaggccggat 2340
aaaacttgtg cttatttttc tttacggtct ttaaaaaggc cgtaatatcc agctgaacgg 2400
tctggttata ggtacattga gcaactgact gaaatgcctc aaaatgttct ttacgatgcc 2460
attgggatat atcaacggtg gtatatccag tgattttttt ctccatttta gcttccttag 2520
ctcctgaaaa tctcgataac tcaaaaaata cgcccggtag tgatcttatt tcattatggt 2580
gaaagttgga acctcttacg tgccgatcaa cgtctcattt tcgccaaaag ttggcccagg 2640
gcttcccggt atcaacaggg acaccaggat ttatttattc tgcgaagtga tcttccgtca 2700
caggtattta ttcggcgcaa agtgcgtcgg gtgatgctgc caacttagtc gactacaggt 2760
cactaatacc atctaagtag ttgattcata gtgactggat atgttgtgtt ttacagtatt 2820
atgtagtctg ttttttatgc aaaatctaat ttaatatatt gatatttata tcattttacg 2880

tttctcgttc agctttcttg tacaaagttg gcattataag aaagcattgc ttatcaattt 2940
gttgcaacga acaggtcact atcagtcaaa ataaaatcat tatttgccat ccagctgata 3000
tcccctatag tgagtcgtat tacatggtca tagctgtttc ctggcagctc tggcccgtgt 3060
ctcaaaatct ctgatgttac attgcacaag ataaaaataat atcatcatga acaataaaac 3120
tgtctgctta cataaacagt aatacaaggg gtgttatgag ccatattcaa cgggaaacgt 3180
cgaggccgcg attaaattcc aacatggatg ctgatttata tgggtataaa tgggctcgcg 3240
ataatgtcgg gcaatcaggt gcgacaatct atcgcttgta tgggaagccc gatgcgccag 3300
agttgtttct gaaacatggc aaaggtagcg ttgccaatga tgttacagat gagatggtca 3360
gactaaactg gctgacggaa tttatgcctc ttccgaccat caagcatttt atccgtactc 3420
ctgatgatgc atggttactc accactgcga tccccggaaa aacagcattc caggtattag 3480
aagaatatcc tgattcaggt gaaaatattg ttgatgcgct ggcagtgttc ctgcgccggt 3540
tgcattcgat tcctgtttgt aattgtcctt ttaacagcga tcgcgtattt cgtctcgctc 3600
aggcgcaatc acgaatgaat aacggtttgg ttgatgcgag tgattttgat gacgagcgta 3660
atggctggcc tgttgaacaa gtctggaaag aaatgcataa acttttgcca ttctcaccgg 3720
attcagtcgt cactcatggt gatttctcac ttgataacct tatttttgac gaggggaaat 3780
taataggttg tattgatgtt ggacgagtcg gaatcgcaga ccgataccag gatcttgcca 3840
tcctatggaa ctgcctcggt gagttttctc cttcattaca gaaacggctt tttcaaaaat 3900
atggtattga taatcctgat atgaataaat tgcagtttca tttgatgctc gatgagtttt 3960
tctaatcaga attggttaat tggttgtaac actggcagag cattacgctg acttgacggg 4020
acggcgcaag ctcatgacca aaatccctta acgtgagtta cgcgtcgttc cactgagcgt 4080
cagaccccgt agaaaagatc aaaggatctt cttgagatcc tttttttctg cgcgtaatct 4140
gctgcttgca aacaaaaaaa ccaccgctac cagcggtggt ttgtttgccg gatcaagagc 4200
taccaactct ttttccgaag gtaactggct tcagcagagc gcagatacca aatactgttc 4260
ttctagtgta gccgtagtta ggccaccact tcaagaactc tgtagcaccg cctacatacc 4320
tcgctctgct aatcctgtta ccagtggctg ctgccagtgg cgataagtcg tgtcttaccg 4380
ggttggactc aagacgatag ttaccggata aggcgcagcg gtcgggctga acggggggtt 4440
cgtgcacaca gcccagcttg gagcgaacga cctacaccga actgagatac ctacagcgtg 4500
agctatgaga aagcgccacg cttcccgaag ggagaaaggc ggacaggtat ccggtaagcg 4560
gcagggtcgg aacaggagag cgcacgaggg agcttccagg gggaaacgcc tggtatcttt 4620
atagtcctgt cgggtttcgc cacctctgac ttgagcgtcg atttttgtga tgctcgtcag 4680
gggggcggag cctatggaaa aacgccagca acgcggcctt tttacggttc ctggcctttt 4740
gctggccttt tgctcacatg tt 4762

<210> 4
<211> 16843
<212> DNA
<213> artificial sequence

<220>
<223> vector

<400> 4
ccgggctggt tgccctcgcc gctgggctgg cggccgtcta tggccctgca aacgcgccag 60
aaacgccgtc gaagccgtgt gcgagacacc gcggccgccg gcgttgtgga tacctcgcgg 120
aaaacttggc cctcactgac agatgagggg cggacgttga cacttgaggg gccgactcac 180
ccggcgcggc gttgacagat gaggggcagg ctcgatttcg gccggcgacg tggagctggc 240
cagcctcgca aatcggcgaa aacgcctgat tttacgcgag tttcccacag atgatgtgga 300
caagcctggg gataagtgcc ctgcggtatt gacacttgag ggcgcgact actgacagat 360
gaggggcgcg atccttgaca cttgaggggc agagtgctga cagatgaggg gcgcacctat 420
tgacatttga ggggctgtcc acaggcagaa aatccagcat ttgcaagggt ttccgcccgt 480
ttttcggcca ccgctaacct gtcttttaac ctgcttttaa accaatattt ataaaccttg 540
tttttaacca gggctgcgcc ctgtgcgcgt gaccgcgcac gccgaagggg ggtgcccccc 600
cttctcgaac cctcccggcc cgctaacgcg ggcctcccat ccccccaggg gctgcgcccc 660
tcggccgcga acggcctcac cccaaaaatg gcagcgctgg cagtccttgc cattgccggg 720
atcggggcag taacgggatg ggcgatcagc ccgagcgcga cgcccggaag cattgacgtg 780
ccgcaggtgc tggcatcgac attcagcgac caggtgccgg gcagtgaggg cggcggcctg 840
ggtggcggcc tgcccttcac ttcggccgtc ggggcattca cggacttcat ggcggggccg 900
gcaattttta ccttgggcat tcttggcata gtggtcgcgg gtgccgtgct cgtgttcggg 960
ggtgcgataa acccagcgaa ccatttgagg tgataggtaa gattataccg aggtatgaaa 1020
acgagaattg gacctttaca gaattactct atgaagcgcc atatttaaaa agctaccaag 1080
acgaagagga tgaagaggat gaggaggcag attgccttga atatattgac aatactgata 1140
agataatata tcttttatat agaagatatc gccgtatgta aggatttcag ggggcaaggc 1200
ataggcagcg cgcttatcaa tatatctata gaatgggcaa agcataaaaa cttgcatgga 1260
ctaatgcttg aaacccagga caataacctt atagcttgta aattctatca taattgggta 1320
atgactccaa cttattgata gtgttttatg ttcagataat gcccgatgac tttgtcatgc 1380
agctccaccg attttgagaa cgacagcgac ttccgtccca gccgtgccag gtgctgcctc 1440
agattcaggt tatgccgctc aattcgctgc gtatatcgct tgctgattac gtgcagcttt 1500
cccttcaggc gggattcata cagcggccag ccatccgtca tccatatcac cacgtcaaag 1560
ggtgacagca ggctcataag acgccccagc gtcgccatag tgcgttcacc gaatacgtgc 1620
gcaacaaccg tcttccggag actgtcatac gcgtaaaaca gccagcgctg gcgcgattta 1680

gccccgacat agccccactg ttcgtccatt tccgcgcaga cgatgacgtc actgcccggc 1740
tgtatgcgcg aggttaccga ctgcggcctg agttttttaa gtgacgtaaa atcgtgttga 1800
ggccaacgcc cataatgcgg gctgttgccc ggcatccaac gccattcatg gccatatcaa 1860
tgattttctg gtgcgtaccg ggttgagaag cggtgtaagt gaactgcagt tgccatgttt 1920
tacggcagtg agagcagaga tagcgctgat gtccggcggt gcttttgccg ttacgcacca 1980
ccccgtcagt agctgaacag gagggacagc tgatagacac agaagccact ggagcacctc 2040
aaaaacacca tcatacacta aatcagtaag ttggcagcat cacccataat tgtggtttca 2100
aaatcggctc cgtcgatact atgttatacg ccaactttga aaacaacttt gaaaaagctg 2160
ttttctggta tttaaggttt tagaatgcaa ggaacagtga attggagttc gtcttgttat 2220
aattagcttc ttggggtatc tttaaatact gtagaaaaga ggaaggaaat aataaatggc 2280
taaaatgaga atatcaccgg aattgaaaaa actgatcgaa aaataccgct gcgtaaaaga 2340
tacggaagga atgtctcctg ctaaggtata taagctggtg ggagaaaatg aaaacctata 2400
tttaaaaatg acggacagcc ggtataaagg gaccacctat gatgtggaac gggaaaagga 2460
catgatgcta tggctggaag gaaagctgcc tgttccaaag gtcctgcact ttgaacggca 2520
tgatggctgg agcaatctgc tcatgagtga ggccgatggc gtcctttgct cggaagagta 2580
tgaagatgaa caaagccctg aaaagattat cgagctgtat gcggagtgca tcaggctctt 2640
tcactccatc gacatatcgg attgtcccta tacgaatagc ttagacagcc gcttagccga 2700
attggattac ttactgaata acgatctggc cgatgtggat tgcgaaaact gggaagaaga 2760
cactccattt aaagatccgc gcgagctgta tgatttttta aagacggaaa agcccgaaga 2820
ggaacttgtc ttttcccacg gcgacctggg agacagcaac atctttgtga aagatggcaa 2880
agtaagtggc tttattgatc ttgggagaag cggcagggcg gacaagtggt atgacattgc 2940
cttctgcgtc cggtcgatca gggaggatat cggggaagaa cagtatgtcg agctattttt 3000
tgacttactg gggatcaagc ctgattggga gaaaataaaa tattatattt tactggatga 3060
attgttttag tacctagatg tggcgcaacg atgccggcga caagcaggag cgcaccgact 3120
tcttccgcat caagtgtttt ggctctcagg ccgaggccca cggcaagtat ttgggcaagg 3180
ggtcgctggt attcgtgcag ggcaagattc ggaataccaa gtacgagaag gacggccaga 3240
cggtctacgg gaccgacttc attgccgata aggtggatta tctggacacc aaggcaccag 3300
gcgggtcaaa tcaggaataa gggcacattg ccccggcgtg agtcggggca atcccgcaag 3360
gagggtgaat gaatcggacg tttgaccgga aggcatacag gcaagaactg atcgacgcgg 3420
ggttttccgc cgaggatgcc gaaaccatcg caagccgcac cgtcatgcgt gcgccccgcg 3480
aaaccttcca gtccgtcggc tcgatggtcc agcaagctac ggccaagatc gagcgcgaca 3540
gcgtgcaact ggctccccct gccctgcccg cgccatcggc cgccgtggag cgttcgcgtc 3600
gtctcgaaca ggaggcggca ggtttggcga agtcgatgac catcgacacg cgaggaacta 3660

tgacgaccaa gaagcgaaaa accgccggcg aggacctggc aaaacaggtc agcgaggcca 3720
agcaggccgc gttgctgaaa cacacgaagc agcagatcaa ggaaatgcag ctttccttgt 3780
tcgatattgc gccgtggccg gacacgatgc gagcgatgcc aaacgacacg gcccgctctg 3840
ccctgttcac cacgcgcaac aagaaaatcc cgcgcgaggc gctgcaaaac aaggtcattt 3900
tccacgtcaa caaggacgtg aagatcacct acaccggcgt cgagctgcgg gccgacgatg 3960
acgaactggt gtggcagcag gtgttggagt acgcgaagcg cacccctatc ggcgagccga 4020
tcaccttcac gttctacgag ctttgccagg acctgggctg gtcgatcaat ggccggtatt 4080
acacgaaggc cgaggaatgc ctgtcgcgcc tacaggcgac ggcgatgggc ttcacgtccg 4140
accgcgttgg gcacctggaa tcggtgtcgc tgctgcaccg cttccgcgtc ctggaccgtg 4200
gcaagaaaac gtcccgttgc caggtcctga tcgacgagga aatcgtcgtg ctgtttgctg 4260
gcgaccacta cacgaaattc atatgggaga agtaccgcaa gctgtcgccg acggcccgac 4320
ggatgttcga ctatttcagc tcgcaccggg agccgtaccc gctcaagctg gaaaccttcc 4380
gcctcatgtg cggatcggat tccacccgcg tgaagaagtg gcgcgagcag gtcggcgaag 4440
cctgcgaaga gttgcgaggc agcggcctgg tggaacacgc ctgggtcaat gatgacctgg 4500
tgcattgcaa acgctagggc cttgtggggt cagttccggc tgggggttca gcagccagcg 4560
ctttactggc atttcaggaa caagcgggca ctgctcgacg cacttgcttc gctcagtatc 4620
gctcgggacg cacggcgcgc tctacgaact gccgataaac agaggattaa aattgacaat 4680
tgtgattaag gctcagattc gacggcttgg agcggccgac gtgcaggatt tccgcgagat 4740
ccgattgtcg gccctgaaga aagctccaga gatgttcggg tccgtttacg agcacgagga 4800
gaaaaagccc atggaggcgt tcgctgaacg gttgcgagat gccgtggcat tcggcgccta 4860
catcgacggc gagatcattg ggctgtcggt cttcaaacag gaggacggcc ccaaggacgc 4920
tcacaaggcg catctgtccg gcgttttcgt ggagcccgaa cagcgaggcc gaggggtcgc 4980
cggtatgctg ctgcgggcgt tgccggcggg tttattgctc gtgatgatcg tccgacagat 5040
tccaacggga atctggtgga tgcgcatctt catcctcggc gcacttaata tttcgctatt 5100
ctggagcttg ttgtttattt cggtctaccg cctgccgggc ggggtcgcgg cgacggtagg 5160
cgctgtgcag ccgctgatgg tcgtgttcat ctctgccgct ctgctaggta gcccgatacg 5220
attgatggcg gtcctggggg ctatttgcgg aactgcgggc gtggcgctgt tggtgttgac 5280
accaaacgca gcgctagatc ctgtcggcgt cgcagcgggc ctggcggggg cggtttccat 5340
ggcgttcgga accgtgctga cccgcaagtg gcaacctccc gtgcctctgc tcacctttac 5400
cgcctggcaa ctggcggccg gaggacttct gctcgttcca gtagctttag tgtttgatcc 5460
gccaatcccg atgcctacag gaaccaatgt tctcggcctg gcgtggctcg gcctgatcgg 5520
agcgggttta acctacttcc tttggttccg gggatctcg cgactcgaac ctacagttgt 5580

ttccttactg ggctttctca gccccagatc tggggtcgat cagccgggga tgcatcaggc 5640
cgacagtcgg aacttcgggt ccccgacctg taccattcgg tgagcaatgg ataggggagt 5700
tgatatcgtc aacgttcact tctaaagaaa tagcgccact cagcttcctc agcggcttta 5760
tccagcgatt tcctattatg tcggcatagt tctcaagatc gacagcctgt cacggttaag 5820
cgagaaatga ataagaaggc tgataattcg gatctctgcg agggagatga tatttgatca 5880
caggcagcaa cgctctgtca tcgttacaat caacatgcta ccctccgcga gatcatccgt 5940
gtttcaaacc cggcagctta gttgccgttc ttccgaatag catcggtaac atgagcaaag 6000
tctgccgcct tacaacggct ctcccgctga cgccgtcccg gactgatggg ctgcctgtat 6060
cgagtggtga ttttgtgccg agctgccggt cggggagctg ttggctggct ggtggcagga 6120
tatattgtgg tgtaaacaaa ttgacgctta gacaacttaa taacacattg cggacgtttt 6180
taatgtactg gggtggtttt tcttttcacc agtgagacgg gcaacagctg attgcccttc 6240
accgcctggc cctgagagag ttgcagcaag cggtccacgc tggtttgccc cagcaggcga 6300
aaatcctgtt tgatggtggt tccgaaatcg gcaaaatccc ttataaatca aaagaatagc 6360
ccgagatagg gttgagtgtt gttccagttt ggaacaagag tccactatta aagaacgtgg 6420
actccaacgt caaagggcga aaaaccgtct atcagggcga tggcccacta cctgtatggc 6480
cgcattcgca aaacacacct agactagatt tgttttgcta acccaattga tattaattat 6540
atatgattaa tatttatatg tatatggatt tggttaatga aatgcatctg gttcatcaaa 6600
gaattataaa gacacgtgac attcatttag gataagaaat atggatgatc tctttctctt 6660
ttattcagat aactagtaat tacacataac acacaacttt gatgcccaca ttatagtgat 6720
tagcatgtca ctatgtgtgc atccttttat ttcatacatt aattaagttg gccaatccag 6780
aagatggaca agtctaggtt aaccatgtgg tacctacgcg ttcgaatatc catgggccgc 6840
ttcaggccag ggcgctgggg aaggcgatgg cgtgctcggt cagctgccac ttctggttct 6900
tggcgtcgct ccggtcctcc cgcagcagct tgtgctggat gaagtgccac tcgggcatct 6960
tgctgggcac gctcttggcc ttgtacacgg tgtcgaactg gcaccggtac cggccgccgt 7020
ccttcagcag caggtacatg ctcacgtcgc ccttcaggat gccctgctta ggcacgggca 7080
tgatcttctc gcagctggcc tcccagttgg tggtcatctt cttcatcacg gggccgtcgg 7140
cggggaagtt cacgccgttg aagatgctct tgtggtagat gcagttctcc ttcacgctca 7200
cggtgatgtc cacgttacag atgcacacgg cgccgtcctc gaacaggaag ctccggcccc 7260
aggtgtagcc ggcggggcag ctgttcttga agtagtccac gatgtcctgg gggtactcgg 7320
tgaagatccg gtcgccgtac ttgaagccgg cgctcaggat gtcctcgctg aagggcaggg 7380
ggccgccctc gatcacgcac aggttgatgg tctgcttgcc cttgaagggg tagccgatgc 7440
cctcgccggt gatcacgaac ttgtggccgt tcacgcagcc ctccatgtgg tacttcatgg 7500
tcatctcctc cttcaggccg tgcttgctgt gggccatggt ggcgaccggt gaattcgagc 7560

tcggtacccg gggatcctga gtaaaacaga ggagggtctc actaagttta tagagagact 7620
gagagagata aagggacacg tatgaagcgt ctgttttcgt ggtgtgacgt caaagtcatt 7680
ttgctctcta cgcgtgtctg tgtcggcttg atcttttttt ttgcttttg gaactcatgt 7740
cggtagtata tcttttattt attttttctt tttttccctt ttctttcaaa ctgatgtcgg 7800
tatgatattt attccatcct aaaatgtaac ttactattat tagtagtcgg tccatgtcta 7860
ttggcccatc atgtggtcat tttacgttta cgtcgtgtgg ctgtttatta taacaaacgg 7920
cacatccttc tcattcgaat tgtatttctc cttaatcgtt ctaataggta tgatctttta 7980
ttttatacgt aaaattaaaa ttgaatgatg tcaagaacga aaattaattt gtatttacaa 8040
aggagctaaa tattgtttat tcctctactg gtagaagata aaagaagtag atgaaataat 8100
gatcttacta gagaatattc ctcatttaca ctagtcaaat ggaaatcttg taaactttta 8160
caataattta tcctgaaaat atgaaaaaat agaagaaaat gtttacctcc tctctcctct 8220
taattcacct acgatcggtg cgggcctctt cgctattacg ccagctggcg aaagggggat 8280
gtgctgcaag gcgattaagt tgggtaacgc cagggttttc ccagtcacga cgttgtaaaa 8340
cgacggccag tgaattcgag ctcggtaccc ggggatcctc tagagtcgac ctgcaggcat 8400
gcaagcttgt tgaaacatcc ctgaagtgtc tcattttatt ttatttattc tttgctgata 8460
aaaaaataaa ataaaagaag ctaagcacac ggtcaaccat tgctctactg ctaaaagggt 8520
tatgtgtagt gttttactgc ataaattatg cagcaaacaa gacaactcaa attaaaaaat 8580
ttcctttgct tgtttttttg ttgtctctga cttgactttc ttgtggaagt tggttgtata 8640
aggattggga cacaccattg tccttcttaa tttaatttta tttctttgct gataaaaaaa 8700
aaaaatttca tatagtgtta aataataatt tgttaaataa ccaaaaagtc aaatatgttt 8760
actctcgttt aaataattga gagtcgtcca gcaaggctaa acgattgtat agatttatga 8820
caatatttac ttttttatag ataaatgtta tattataata aatttatata catatattat 8880
atgttattta ttatttatta ttattttaaa tccttcaata ttttatcaaa ccaactcata 8940
attttttttt tatctgtaag aagcaataaa attaaataga cccactttaa ggatgatcca 9000
acctttatac agagtaagag agttcaaata gtaccctttc atatacatat caactaaaat 9060
attagaaata tcatggatca aaccttataa agacattaaa taagtggata agtataatat 9120
ataaatgggt agtatataat atataaatgg atacaaactt ctctctttat aattgttatg 9180
tctccttaac atcctaatat aatacataag tgggtaatat ataatatata aatggagaca 9240
aacttcttcc attataattg ttatgtcttc ttaacactta tgtctcgttc acaatgctaa 9300
agttagaatt gtttagaaag tcttatagta cacatttgtt tttgtactat ttgaagcatt 9360
ccataagccg tcacgattca gatgatttat aataataaga ggaaatttat catagaacaa 9420
taaggtgcat agatagagtg ttaatatatc ataacatcct ttgtttattc atagaagaag 9480

tgagatggag ctcagttatt atactgttac atggtcggat acaatattcc atgctctcca 9540
tgagctctta cacctacatg cattttagtt catacttcat gcacgtggcc atcacagcta 9600
gctgcagcta catatttaca ttttacaaca ccaggagaac tgccctgtta gtgcataaca 9660
atcagaagat ggccgtggct actcgagtta tcgaaccact ttgtacaaga aagctgaacg 9720
agaaacgtaa aatgatataa atatcaatat attaaattag attttgcata aaaaacagac 9780
tacataatac tgtaaaacac aacatatcca gtcactatgg tcgacctgca gactggctgt 9840
gtataaggga gcctgacatt tatattcccc agaacatcag gttaatggcg tttttgatgt 9900
cattttcgcg gtggctgaga tcagccactt cttccccgat aacggagacc ggcacactgg 9960
ccatatcggt ggtcatcatg cgccagcttt catccccgat atgcaccacc gggtaaagtt 10020
cacgggagac tttatctgac agcagacgtg cactggccag ggggatcacc atccgtcgcc 10080
cgggcgtgtc aataatatca ctctgtacat ccacaaacag acgataacgg ctctctcttt 10140
tataggtgta aaccttaaac tgcatttcac cagtccctgt tctcgtcagc aaaagagccg 10200
ttcatttcaa taaaccgggc gacctcagcc atcccttcct gattttccgc tttccagcgt 10260
tcggcacgca gacgacgggc ttcattctgc atggttgtgc ttaccagacc ggagatattg 10320
acatcatata tgccttgagc aactgatagc tgtcgctgtc aactgtcact gtaatacgct 10380
gcttcatagc acacctcttt ttgacatact tcgggtatac atatcagtat atattcttat 10440
accgcaaaaa tcagcgcgca aatacgcata ctgttatctg gcttttagta agccggatcc 10500
tctagattac gccccgccct gccactcatc gcagtactgt tgtaattcat taagcattct 10560
gccgacatgg aagccatcac agacggcatg atgaacctga atcgccagcg gcatcagcac 10620
cttgtcgcct tgcgtataat atttgcccat ggtgaaaacg ggggcgaaga agttgtccat 10680
attggccacg tttaaatcaa aactggtgaa actcacccag ggattggctg agacgaaaaa 10740
catattctca ataaaccctt tagggaaata ggccaggttt tcaccgtaac acgccacatc 10800
ttgcgaatat atgtgtagaa actgccggaa atcgtcgtgg tattcactcc agagcgatga 10860
aaacgtttca gtttgctcat ggaaaacggt gtaacaaggg tgaacactat cccatatcac 10920
cagctcaccg tctttcattg ccatacggaa ttccggatga gcattcatca ggcgggcaag 10980
aatgtgaata aaggccggat aaaacttgtg cttatttttc tttacggtct ttaaaaaggc 11040
cgtaatatcc agctgaacgg tctggttata ggtacattga gcaactgact gaaatgcctc 11100
aaaatgttct ttacgatgcc attgggatat atcaacggtg gtatatccag tgattttttt 11160
ctccatttta gcttccttag ctcctgaaaa tctcgccgga tcctaactca aaatccacac 11220
attatacgag ccggaagcat aaagtgtaaa gcctggggtg cctaatgcgg ccgccatagt 11280
gactggatat gttgtgtttt acagtattat gtagtctgtt ttttatgcaa aatctaattt 11340
aatatattga tatttatatc attttacgtt tctcgttcag ctttttttgta caaacttgtt 11400
tgataaccgg tactagtgtg cacgtcgagc gtgtcctctc caaatgaaat gaacttcctt 11460

atatagagga agggtcttgc gaaggatagt gggattgtgc gtcatccctt acgtcagtgg 11520
agatgtcaca tcaatccact tgctttgaag acgtggttgg aacgtcttct ttttccacga 11580
tgctcctcgt gggtgggggt ccatctttgg gaccactgtc ggcagaggca tcttgaatga 11640
tagcctttcc tttatcgcaa tgatggcatt tgtaggagcc accttccttt tctactgtcc 11700
tttcgatgaa gtgacagata gctgggcaat ggaatccgag gaggtttccc gaaattatcc 11760
tttgttgaaa agtctcaata gccctttggt cttctgagac tgtatctttg acatttttgg 11820
agtagaccag agtgtcgtgc tccaccatgt tgacgaagat tttcttcttg tcattgagtc 11880
gtaaaagact ctgtatgaac tgttcgccag tcttcacggc gagttctgtt agatcctcga 11940
tttgaatctt agactccatg catggcctta gattcagtag gaactacctt tttagagact 12000
ccaatctcta ttacttgcct tggtttatga agcaagcctt gaatcgtcca tactggaata 12060
gtacttctga tcttgagaaa tatgtctttc tctgtgttct tgatgcaatt agtcctgaat 12120
cttttgactg catctttaac cttcttggga aggtatttga tctcctggag attgttactc 12180
gggtagatcg tcttgatgag acctgctgcg taggcctctc taaccatctg tgggtcagca 12240
ttctttctga aattgaagag gctaaccttc tcattatcag tggtgaacat agtgtcgtca 12300
ccttcacctt cgaacttcct tcctagatcg taaagataga ggaaatcgtc cattgtaatc 12360
tccggggcaa aggagatctc ttttggggct ggatcactgc tgggcctttt ggttcctagc 12420
gtgagccagt gggctttttg ctttggtggg cttgttaggg ccttagcaaa gctcttgggc 12480
ttgagttgag cttctccttt ggggatgaag ttcaacctgt ctgtttgctg acttgttgtg 12540
tacgcgtcag ctgctgctct tgcctctgta atagtggcaa atttcttgtg tgcaactccg 12600
ggaacgccgt ttgttgccgc ctttgtacaa ccccagtcat cgtatatacc ggcatgtgga 12660
ccgttataca caacgtagta gttgatatga gggtgttgaa tacccgattc tgctctgaga 12720
ggagcaactg tgctgttaag ctcagatttt tgtgggattg gaattggatc ctctagagca 12780
aagcttggcg taatcatggt catagctgtt tcctgtgtga aattgttatc cgctcacaat 12840
tccacacaac atacgagccg gaagcataaa gtgtaaagcc tggggtgcct aatgagtgag 12900
ctaactcaca ttaattgcgt tgcgctcact gcccgctttc cagtcgggaa acctgtcgtg 12960
ccagctgcat taatgaatcg gccaacgcgc ggggagaggc ggtttgcgta ttgggccaaa 13020
gacaaaaggg cgacattcaa ccgattgagg gagggaaggt aaatattgac ggaaattatt 13080
cattaaaggt gaattatcac cgtcaccgac ttgagccatt tgggaattag agccagcaaa 13140
atcaccagta gcaccattac cattagcaag gccggaaacg tcaccaatga aaccatcatc 13200
tagtaacata gatgacaccg cgcgcgataa tttatcctag tttgcgcgct atattttgtt 13260
ttctatcgcg tattaaatgt ataattgcgg gactctaatc ataaaaaccc atctcataaa 13320
taacgtcatg cattacatgt taattattac atgcttaacg taattcaaca gaaattatat 13380

gataatcatc gcaagaccgg caacaggatt caatcttaag aaactttatt gccaaatgtt 13440
tgaacgatct gcttcgacgc actccttctt taggtacgga ctagatctcg gtgacgggca 13500
ggaccggacg gggcggtacc ggcaggctga agtccagctg ccagaaaccc acgtcatgcc 13560
agttcccgtg cttgaagccg gccgcccgca gcatgccgcg gggggcatat ccgagcgcct 13620
cgtgcatgcg cacgctcggg tcgttgggca gcccgatgac agcgaccacg ctcttgaagc 13680
cctgtgcctc cagggacttc agcaggtggg tgtagagcgt ggagcccagt cccgtccgct 13740
ggtggcgggg ggagacgtac acggtcgact cggccgtcca gtcgtaggcg ttgcgtgcct 13800
tccaggggcc cgcgtaggcg atgccggcga cctcgccgtc cacctcggcg acgagccagg 13860
gatagcgctc ccgcagacgg acgaggtcgt ccgtccactc ctgcggttcc tgcggctcgg 13920
tacggaagtt gaccgtgctt gtctcgatgt agtggttgac gatggtgcag accgccggca 13980
tgtccgcctc ggtggcacgg cggatgtcgg ccgggcgtcg ttctgggctc atggatctgg 14040
attgagagtg aatatgagac tctaattgga taccgagggg aatttatgga acgtcagtgg 14100
agcatttttg acaagaaata tttgctagct gatagtgacc ttaggcgact tttgaacgcg 14160
caataatggt ttctgacgta tgtgcttagc tcattaaact ccagaaaccc gcggctgagt 14220
ggctccttca acgttgcggt tctgtcagtt ccaaacgtaa aacggcttgt cccgcgtcat 14280
cggcgggggt cataacgtga ctcccttaat tctccgctca tgatcagatt gtcgtttccc 14340
gccttcagtt taaactatca gtgtttgaca ggatatattg gcgggtaaac ctaagagaaa 14400
agagcgttta ttagaataat cggatattta aaagggcgtg aaaaggttta tccgttcgtc 14460
catttgtatg tgcatgccaa ccacagggtt ccccagatct ggcgccggcc agcgagacga 14520
gcaagattgg ccgccgcccg aaacgatccg acagcgcgcc cagcacaggt gcgcaggcaa 14580
attgcaccaa cgcatacagc gccagcagaa tgccatagtg ggcggtgacg tcgttcgagt 14640
gaaccagatc gcgcaggagg cccggcagca ccggcataat caggccgatg ccgacagcgt 14700
cgagcgcgac agtgctcaga attacgatca ggggtatgtt gggtttcacg tctggcctcc 14760
ggaccagcct ccgctggtcc gattgaacgc gcggattctt tatcactgat aagttggtgg 14820
acatattatg tttatcagtg ataaagtgtc aagcatgaca aagttgcagc cgaatacagt 14880
gatccgtgcc gccctggacc tgttgaacga ggtcggcgta gacggtctga cgacacgcaa 14940
actggcggaa cggttggggg ttcagcagcc ggcgctttac tggcacttca ggaacaagcg 15000
ggcgctgctc gacgcactgg ccgaagccat gctggcggag aatcatacgc attcggtgcc 15060
gagagccgac gacgactggc gctcatttct gatcgggaat gcccgcagct tcaggcaggc 15120
gctgctcgcc taccgcgatg gcgcgcgcat ccatgccggc acgcgaccgg gcgcaccgca 15180
gatggaaacg gccgacgcgc agcttcgctt cctctgcgag gcgggttttt cggccgggga 15240
cgccgtcaat gcgctgatga caatcagcta cttcactgtt ggggccgtgc ttgaggagca 15300
ggccggcgac agcgatgccg gcgagcgcgg cggcaccgtt gaacaggctc cgctctcgcc 15360

gctgttgcgg gccgcgatag acgccttcga cgaagccggt ccggacgcag cgttcgagca 15420

gggactcgcg gtgattgtcg atggattggc gaaaaggagg ctcgttgtca ggaacgttga 15480

aggaccgaga aagggtgacg attgatcagg accgctgccg gagcgcaacc cactcactac 15540

agcagagcca tgtagacaac atcccctccc cctttccacc gcgtcagacg cccgtagcag 15600

cccgctacgg gctttttcat gccctgccct agcgtccaag cctcacggcc gcgctcggcc 15660

tctctggcgg ccttctggcg ctcttccgct tcctcgctca ctgactcgct gcgctcggtc 15720

gttcggctgc ggcgagcggt atcagctcac tcaaaggcgg taatacggtt atccacagaa 15780

tcaggggata acgcaggaaa gaacatgtga gcaaaaggcc agcaaaaggc caggaaccgt 15840

aaaaaggccg cgttgctggc gtttttccat aggctccgcc cccctgacga gcatcacaaa 15900

aatcgacgct caagtcagag gtggcgaaac ccgacaggac tataaagata ccaggcgttt 15960

ccccctggaa gctccctcgt gcgctctcct gttccgaccc tgccgcttac cggatacctg 16020

tccgcctttc tcccttcggg aagcgtggcg cttttccgct gcataaccct gcttcggggt 16080

cattatagcg atttttttcgg tatatccatc ctttttcgca cgatatacag gattttgcca 16140

aagggttcgt gtagactttc cttggtgtat ccaacggcgt cagccgggca ggataggtga 16200

agtaggccca cccgcgagcg ggtgttcctt cttcactgtc ccttattcgc acctggcggt 16260

gctcaacggg aatcctgctc tgcgaggctg gccggctacc gccggcgtaa cagatgaggg 16320

caagcggatg gctgatgaaa ccaagccaac caggaagggc agcccaccta tcaaggtgta 16380

ctgccttcca gacgaacgaa gagcgattga ggaaaaggcg gcggcggccg gcatgagcct 16440

gtcggcctac ctgctggccg tcggccaggg ctacaaaatc acgggcgtcg tggactatga 16500

gcacgtccgc gagctggccc gcatcaatgg cgacctgggc cgcctgggcg gcctgctgaa 16560

actctggctc accgacgacc cgcgcacggc gcggttcggt gatgccacga tcctcgccct 16620

gctggcgaag atcgaagaga agcaggacga gcttggcaag gtcatgatgg gcgtggtccg 16680

cccgagggca gagccatgac tttttttagcc gctaaaacgg ccggggggtg cgcgtgattg 16740

ccaagcacgt ccccatgcgc tccatcaaga agagcgactt cgcggagctg gtgaagtaca 16800

tcaccgacga gcaaggcaag accgagcgcc tttgcgacgc tca 16843

<210> 5
<211> 9142
<212> DNA
<213> artificial sequence

<220>
<223> vector

<400> 5
ctagttatct gaataaaaga gaaagagatc atccatattt cttatcctaa atgaatgtca 60

cgtgtcttta taattctttg atgaaccaga tgcatttcat taaccaaatc catatacata 120

taaatattaa tcatatataa ttaatatcaa ttgggttagc aaaacaaatc tagtctaggt 180
gtgttttgcg aattcgatat caagcttgat gggtaccggc gcgcccgatc atccggatat 240
agttcctcct ttcagcaaaa aacccctcaa gacccgttta gaggccccaa ggggttatgc 300
tagttattgc tcagcggtgg cagcagccaa ctcagcttcc tttcgggctt tgttagcagc 360
cggatcgatc caagctgtac ctcactattc ctttgccctc ggacgagtgc tggggcgtcg 420
gtttccacta tcggcgagta cttctacaca gccatcggtc cagacggccg cgcttctgcg 480
ggcgatttgt gtacgcccga cagtcccggc tccggatcgg acgattgcgt cgcatcgacc 540
ctgcgcccaa gctgcatcat cgaaattgcc gtcaaccaag ctctgataga gttggtcaag 600
accaatgcgg agcatatacg cccggagccg cggcgatcct gcaagctccg gatgcctccg 660
ctcgaagtag cgcgtctgct gctccataca agccaaccac ggcctccaga agaagatgtt 720
ggcgacctcg tattgggaat ccccgaacat cgcctcgctc cagtcaatga ccgctgttat 780
gcggccattg tccgtcagga cattgttgga gccgaaatcc gcgtgcacga ggtgccggac 840
ttcggggcag tcctcggccc aaagcatcag ctcatcgaga gcctgcgcga cggacgcact 900
gacggtgtcg tccatcacag tttgccagtg atacacatgg ggatcagcaa tcgcgcatat 960
gaaatcacgc catgtagtgt attgaccgat tccttgcggt ccgaatgggc cgaacccgct 1020
cgtctggcta agatcggccg cagcgatcgc atccatagcc tccgcgaccg gctgcagaac 1080
agcgggcagt tcggtttcag gcaggtcttg caacgtgaca ccctgtgcac ggcgggagat 1140
gcaataggtc aggctctcgc tgaattcccc aatgtcaagc acttccggaa tcgggagcgc 1200
ggccgatgca aagtgccgat aaacataacg atctttgtag aaaccatcgg cgcagctatt 1260
tacccgcagg acatatccac gccctcctac atcgaagctg aaagcacgag attcttcgcc 1320
ctccgagagc tgcatcaggt cggagacgct gtcgaacttt tcgatcagaa acttctcgac 1380
agacgtcgcg gtgagttcag gcttttccat gggtatatct ccttcttaaa gttaaacaaa 1440
attatttcta gagggaaacc gttgtggtct ccctatagtg agtcgtatta atttcgcggg 1500
atcgagatct gatcaacctg cattaatgaa tcggccaacg cgcggggaga ggcggtttgc 1560
gtattgggcg ctcttccgct tcctcgctca ctgactcgct gcgctcggtc gttcggctgc 1620
ggcgagcggt atcagctcac tcaaaggcgg taatacggtt atccacagaa tcaggggata 1680
acgcaggaaa gaacatgtga gcaaaaggcc agcaaaaggc caggaaccgt aaaaaggccg 1740
cgttgctggc gtttttccat aggctccgcc cccctgacga gcatcacaaa aatcgacgct 1800
caagtcagag gtggcgaaac ccgacaggac tataaagata ccaggcgttt ccccctggaa 1860
gctccctcgt gcgctctcct gttccgaccc tgccgcttac cggatacctg tccgcctttc 1920
tcccttcggg aagcgtggcg ctttctcaat gctcacgctg taggtatctc agttcggtgt 1980
aggtcgttcg ctccaagctg ggctgtgtgc acgaaccccc cgttcagccc gaccgctgcg 2040
ccttatccgg taactatcgt cttgagtcca acccggtaag acacgactta tcgccactgg 2100

cagcagccac tggtaacagg attagcagag cgaggtatgt aggcggtgct acagagttct 2160

tgaagtggtg gcctaactac ggctacacta gaaggacagt atttggtatc tgcgctctgc 2220

tgaagccagt taccttcgga aaaagagttg gtagctcttg atccggcaaa caaaccaccg 2280

ctggtagcgg tggttttttt gtttgcaagc agcagattac gcgcagaaaa aaaggatctc 2340

aagaagatcc tttgatcttt tctacggggt ctgacgctca gtggaacgaa aactcacgtt 2400

aagggatttt ggtcatgaca ttaacctata aaaataggcg tatcacgagg ccctttcgtc 2460

tcgcgcgttt cggtgatgac ggtgaaaacc tctgacacat gcagctcccg gagacggtca 2520

cagcttgtct gtaagcggat gccgggagca gacaagcccg tcagggcgcg tcagcgggtg 2580

ttggcgggtg tcggggctgg cttaactatg cggcatcaga gcagattgta ctgagagtgc 2640

accatatgga catattgtcg ttagaacgcg gctacaatta atacataacc ttatgtatca 2700

tacacatacg atttaggtga cactatagaa cggcgcgcca agctgggtct agaactagaa 2760

acgtgatgcc acttgttatt gaagtcgatt acagcatcta ttctgtttta ctatttataa 2820

ctttgccatt tctgactttt gaaaactatc tctggatttc ggtatcgctt tgtgaagatc 2880

gagcaaaaga gacgttttgt ggacgcaatg gtccaaatcc gttctacatg aacaaattgg 2940

tcacaatttc cactaaaagt aaataaatgg caagttaaaa aaggaatatg cattttactg 3000

attgcctagg tgagctccaa gagaagttga atctacacgt ctaccaaccg ctaaaaaaag 3060

aaaaacattg aatatgtaac ctgattccat tagcttttga cttcttcaac agattctcta 3120

cttagatttc taacagaaat attattacta gcacatcatt ttcagtctca ctacagcaaa 3180

aaatccaacg gcacaataca gacaacagga gatatcagac tacagagata gatagatgct 3240

actgcatgta gtaagttaaa taaaaggaaa ataaaatgtc ttgctaccaa aactactaca 3300

gactatgatg ctcaccacag gccaaatcct gcaactagga cagcattatc ttatatatat 3360

tgtacaaaac aagcatcaag gaacatttgg tctaggcaat cagtacctcg ttctaccatc 3420

accctcagtt atcacatcct tgaaggatcc attactggga atcatcggca acacatgctc 3480

ctgatggggc acaatgacat caagaaggta ggggccaggg gtgtccaaca ttctctgaat 3540

tgccgctcta agctcttcct tcttcgtcac tcgcgctgcc ggtatcccac aagcatcagc 3600

aaacttgagc atgtttggga atatctcgct ctcgctagac ggatctccaa gataggtgtg 3660

agctctattg gacttgtaga acctatcctc caactgaacc accataccca aatgctgatt 3720

gttcaacaac aatatcttaa ctgggagatt ctccactctt atagtggcca actcctgaac 3780

attcatgatg aaactaccat ccccatcaat gtcaaccaca acagccccag ggttagcaac 3840

agcagcacca atagccgcag gcaatccaaa acccatggct ccaagacccc ctgaggtcaa 3900

ccactgcctc ggtctcttgt acttgtaaaa ctgcgcagcc cacatttgat gctgcccaac 3960

cccagtacta acaatagcat ctccattagt caactcatca agaacctcga tagcatgctg 4020

cggagaaatc gcgtcctgga atgtcttgta acccaatgga aacttgtgtt tctgcacatt 4080
aatctcttct ctccaacctc caagatcaaa cttaccctcc actcctttct cctccaaaat 4140
catattaatt cccttcaagg ccaacttcaa atccgcgcaa accgacacgt gcgcctgctt 4200
gttcttccca atctcggcag aatcaatatc aatgtgaaca atcttagccc tactagcaaa 4260
agcctcaagc ttcccagtaa cacggtcatc aaaccttacc ccaaaggcaa gcaacaaatc 4320
actattgtca acagcatagt tagcataaac agtaccatgc atacccagca tctgaaggga 4380
atattcatca ccaataggaa aagttccaag acccattaaa gtgctagcaa cgggaatacc 4440
agtgagttca acaaagcgcc tcaattcagc actggaattc aaactgccac cgccgacgta 4500
gagaacgggc ttttgggcct ccatgatgag tctgacaatg tgttccaatt gggcctcggc 4560
ggggggcctg ggcagcctgg cgaggtaacc ggggaggtta acgggctcgt cccaattagg 4620
cacggcgagt tgctgctgaa cgtctttggg aatgtcgatg aggaccggac cggggcggcc 4680
ggaggtggcg acgaagaaag cctcggcgac gacgcggggg atgtcgtcga cgtcgaggat 4740
gaggtagttg tgcttcgtga tggatctgct cacctccacg atcggggttt cttggaaggc 4800
gtcggtgccg atcatccggc gggcgacctg gccggtgatg gcgacgactg ggacgctgtc 4860
cattaaagcg tcggcgaggc cgctcacgag gttggtggcg ccggggccgg aggtggcaat 4920
gcagacgccg gggaggccgg aggaacgcgc gtagccttcg gcggcgaaga cgccgccctg 4980
ctcgtggcgc gggagcacgt tgcggatggc ggcggagcgc gtgagcgcct ggtggatctc 5040
catcgacgca ccgccggggt acgcgaacac cgtcgtcacg ccctgcctct ccagcgcctc 5100
cacaaggatg tccgcgccct tgcgaggttc gccggaggcg aaccgtgaca cgaagggctc 5160
cgtggtcggc gcttccttgg tgaagggcgc cgccgtgggg ggtttggaga tggaacattt 5220
gattttgaga gcgtggttgg gtttggtgag ggtttgatga gagagaggga gggtggatct 5280
agtaatgcgt ttggggaagg tggggtgtga agaggaagaa gagaatcggg tggttctgga 5340
agcggtggcc gccattgtgt tgtgtggcat ggttatactt caaaaactgc acaacaagcc 5400
tagagttagt acctaaacag taaatttaca acagagagca aagacacatg caaaaatttc 5460
agccataaaa aaagttataa tagaatttaa agcaaaagtt tcattttta aacatatata 5520
caaacaaact ggatttgaag gaagggatta attcccctgc tcaaagtttg aattcctatt 5580
gtgacctata ctcgaataaa attgaagcct aaggaatgta tgagaaacaa gaaaacaaaa 5640
caaaactaca gacaaacaag tacaattaca aaattcgcta aaattctgta atcaccaaac 5700
cccatctcag tcagcacaag gcccaaggtt tattttgaaa taaaaaaaaa gtgattttat 5760
ttctcataag ctaaaagaaa gaaaggcaat tatgaaatga tttcgactag atctgaaagt 5820
caaacgcgta ttccgcagat attaaagaaa gagtagagtt tcacatggat cctagatgga 5880
cccagttgag gaaaaagcaa ggcaaagcaa accagaagtg caagatccga aattgaacca 5940
cggaatctag gatttggtag agggagaaga aaagtacctt gagaggtaga agagaagaga 6000

agagcagaga gatatatgaa cgagtgtgtc ttggtctcaa ctctgaagcg atacgagttt 6060
agaggggagc attgagttcc aatttatagg gaaaccgggt ggcaggggtg agttaatgac 6120
ggaaaagccc ctaagtaacg agattggatt gtgggttaga ttcaaccgtt tgcatccgcg 6180
gcttagattg gggaagtcag agtgaatctc aaccgttgac tgagttgaaa attgaatgta 6240
gcaaccaatt gagccaaccc cagcctttgc cctttgattt tgatttgttt gttgcatact 6300
ttttatttgt cttctggttc tgactctctt tctctcgttt caatgccagg ttgcctactc 6360
ccacaccact cacaagaaga ttctactgtt agtattaaat atttttaat gtattaaatg 6420
atgaatgctt ttgtaaacag aacaagacta tgtctaataa gtgtcttgca acattttta 6480
agaaattaaa aaaaatatat ttattatcaa aatcaaatgt atgaaaaatc atgaataata 6540
taattttata catttttta aaaaatcttt taatttctta attaatatct taaaaataat 6600
gattaatatt taacccaaaa taattagtat gattggtaag gaagatatcc atgttatgtt 6660
tggatgtgag tttgatctag agcaaagctt actagagtcg acctgcagcc cctccaccgc 6720
ggtggcggcc gctctagaga tccgtcaaca tggtggagca cgacactctc gtctactcca 6780
agaatatcaa agatacagtc tcagaagacc aaagggctat tgagactttt caacaaaggg 6840
taatatcggg aaacctcctc ggattccatt gcccagctat ctgtcacttc atcaaaagga 6900
cagtagaaaa ggaaggtggc acctacaaat gccatcattg cgataaagga aaggctatcg 6960
ttcaagatgc ctctgccgac agtggtccca aagatggacc cccacccacg aggagcatcg 7020
tggaaaaaga agacgttcca accacgtctt caaagcaagt ggattgatgt gatgatccta 7080
tgcgtatggt atgacgtgtg ttcaagatga tgacttcaaa cctacctatg acgtatggta 7140
tgacgtgtgt cgactgatga cttagatcca ctcgagcggc tataaatacg tacctacgca 7200
ccctgcgcta ccatccctag agctgcagct tatttttaca acaattacca acaacaacaa 7260
acaacaaaca acattacaat tactatttac aattacagtc gacccatcaa caagtttgta 7320
caaaaaagct gaacgagaaa cgtaaaatga tataaatatc aatatattaa attagatttt 7380
gcataaaaaa cagactacat aatactgtaa aacacaacat atccagtcat attggcggcc 7440
gcattaggca ccccaggctt tacactttat gcttccggct cgtataatgt gtggattttg 7500
agttaggatc cgtcgagatt ttcaggagct aaggaagcta aaatggagaa aaaaatcact 7560
ggatatacca ccgttgatat atcccaatgg catcgtaaag aacattttga ggcatttcag 7620
tcagttgctc aatgtaccta taaccagacc gttcagctgg atattacggc ctttttaaag 7680
accgtaaaga aaaataagca caagttttat ccggccttta ttcacattct tgcccgcctg 7740
atgaatgctc atccggaatt ccgtatggca atgaaagacg gtgagctggt gatatgggat 7800
agtgttcacc cttgttacac cgttttccat gagcaaactg aaacgttttc atcgctctgg 7860
agtgaatacc acgacgattt ccggcagttt ctacacatat attcgcaaga tgtggcgtgt 7920

tacggtgaaa acctggccta tttccctaaa gggtttattg agaatatgtt tttcgtctca 7980

gccaatccct gggtgagttt caccagtttt gatttaaacg tggccaatat ggacaacttc 8040

ttcgcccccg ttttcaccat gggcaaatat tatacgcaag gcgacaaggt gctgatgccg 8100

ctggcgattc aggttcatca tgccgtttgt gatggcttcc atgtcggcag aatgcttaat 8160

gaattacaac agtactgcga tgagtggcag ggcggggcgt aaagatctgg atccggctta 8220

ctaaaagcca gataacagta tgcgtatttg cgcgctgatt tttgcggtat aagaatatat 8280

actgatatgt atacccgaag tatgtcaaaa agaggtatgc tatgaagcag cgtattacag 8340

tgacagttga cagcgacagc tatcagttgc tcaaggcata tatgatgtca atatctccgg 8400

tctggtaagc acaaccatgc agaatgaagc ccgtcgtctg cgtgccgaac gctggaaagc 8460

ggaaaatcag gaagggatgg ctgaggtcgc ccggtttatt gaaatgaacg gctcttttgc 8520

tgacgagaac aggggctggt gaaatgcagt ttaaggttta cacctataaa agagagagcc 8580

gttatcgtct gtttgtggat gtacagagtg atattattga cacgcccggg cgacggatgg 8640

tgatccccct ggccagtgca cgtctgctgt cagataaagt ctcccgtgaa ctttacccgg 8700

tggtgcatat cggggatgaa agctggcgca tgatgaccac cgatatggcc agtgtgccgg 8760

tctccgttat cggggaagaa gtggctgatc tcagccaccg cgaaaatgac atcaaaaacg 8820

ccattaacct gatgttctgg ggaatataaa tgtcaggctc ccttatacac agccagtctg 8880

caggtcgacc atagtgactg gatatgttgt gttttacagt attatgtagt ctgtttttta 8940

tgcaaaatct aatttaatat attgatattt atatcatttt acgtttctcg ttcagctttc 9000

ttgtacaaag tggttgataa cctagacttg tccatcttct ggattggcca acttaattaa 9060

tgtatgaaat aaaaggatgc acacatagtg acatgctaat cactataatg tgggcatcaa 9120

agttgtgtgt tatgtgtaat ta 9142

<210> 6
<211> 49911
<212> DNA
<213> artificial sequence

<220>
<223> vector

<400> 6
gtgcagcgtg acccggtcgt gcccctctct agagataatg agcattgcat gtctaagtta 60

taaaaaatta ccacatattt tttttgtcac acttgtttga agtgcagttt atctatcttt 120

atacatatat ttaaacttta ctctacgaat aatataatct atagtactac aataatatca 180

gtgttttaga gaatcatata aatgaacagt tagacatggt ctaaaggaca attgagtatt 240

ttgacaacag gactctacag ttttatcttt ttagtgtgca tgtgttctcc tttttttttg 300

caaatagctt cacctatata atacttcatc cattttatta gtacatccat ttagggttta 360

gggttaatgg tttttataga ctaatttttt tagtacatct attttattct attttagcct 420

```
ctaaattaag aaaactaaaa ctctatttta gtttttttat ttaataattt agatataaaa    480
tagaataaaa taaagtgact aaaaattaaa caaataccct ttaagaaatt aaaaaaacta    540
aggaaacatt tttcttgttt cgagtagata atgccagcct gttaaacgcc gtcgacgagt    600
ctaacggaca ccaaccagcg aaccagcagc gtcgcgtcgg gccaagcgaa gcagacggca    660
cggcatctct gtcgctgcct ctggacccct ctcgagagtt ccgctccacc gttggacttg    720
ctccgctgtc ggcatccaga aattgcgtgg cggagcggca gacgtgagcc ggcacggcag    780
gcggcctcct cctcctctca cggcacggca gctacggggg attcctttcc caccgctcct    840
tcgctttccc ttcctcgccc gccgtaataa atagacaccc cctccacacc ctctttcccc    900
aacctcgtgt tgttcggagc gcacacacac acaaccagat ctcccccaaa tccacccgtc    960
ggcacctccg cttcaaggta cgccgctcgt cctccccccc cccccctctc taccttctct   1020
agatcggcgt tccggtccat ggttagggcc cggtagttct acttctgttc atgtttgtgt   1080
tagatccgtg tttgtgttag atccgtgctg ctagcgttcg tacacggatg cgacctgtac   1140
gtcagacacg ttctgattgc taacttgcca gtgtttctct ttggggaatc ctgggatggc   1200
tctagccgtt ccgcagacgg gatcgatttc atgatttttt ttgtttcgtt gcatagggtt   1260
tggtttgccc ttttccttta tttcaatata tgccgtgcac ttgtttgtcg ggtcatcttt   1320
tcatgctttt ttttgtcttg gttgtgatga tgtggtctgg ttgggcggtc gttctagatc   1380
ggagtagaat tctgtttcaa actacctggt ggatttatta attttggatc tgtatgtgtg   1440
tgccatacat attcatagtt acgaattgaa gatgatggat ggaaatatcg atctaggata   1500
ggtatacatg ttgatgcggg ttttactgat gcatatacag agatgctttt tgttcgcttg   1560
gttgtgatga tgtggtgtgg ttgggcggtc gttcattcgt tctagatcgg agtagaatac   1620
tgtttcaaac tacctggtgt atttattaat tttggaactg tatgtgtgtg tcatacatct   1680
tcatagttac gagtttaaga tggatggaaa tatcgatcta ggataggtat acatgttgat   1740
gtgggtttta ctgatgcata tacatgatgg catatgcagc atctattcat atgctctaac   1800
cttgagtacc tatctattat aataaacaag tatgttttat aattattttg atcttgatat   1860
acttggatga tggcatatgc agcagctata tgtggatttt tttagccctg ccttcatacg   1920
ctatttattt gcttggtact gtttcttttg tcgatgctca ccctgttgtt tggtgttact   1980
tctgcaggtc gactctagag gatccacaag tttgtacaaa aaagctgaac gagaaacgta   2040
aaatgatata aatatcaata tattaaatta gattttgcat aaaaaacaga ctacataata   2100
ctgtaaaaca caacatatcc agtcactatg gcggccgcat taggcacccc aggctttaca   2160
ctttatgctt ccggctcgta taatgtgtgg attttgagtt aggatttaaa tacgcgttga   2220
tccggcttac taaaagccag ataacagtat gcgtatttgc gcgctgattt ttgcggtata   2280
agaatatata ctgatatgta tacccgaagt atgtcaaaaa gaggtatgct atgaagcagc   2340
```

gtattacagt gacagttgac agcgacagct atcagttgct caaggcatat atgatgtcaa 2400
tatctccggt ctggtaagca caaccatgca gaatgaagcc cgtcgtctgc gtgccgaacg 2460
ctggaaagcg gaaaatcagg aagggatggc tgaggtcgcc cggtttattg aaatgaacgg 2520
ctcttttgct gacgagaaca ggggctggtg aaatgcagtt taaggtttac acctataaaa 2580
gagagagccg ttatcgtctg tttgtggatg tacagagtga tatcattgac acgcccggtc 2640
gacggatggt gatccccctg gccagtgcac gtctgctgtc agataaagtc tcccgtgaac 2700
tttacccggt ggtgcatatc ggggatgaaa gctggcgcat gatgaccacc gatatggcca 2760
gtgtgccggt ctccgttatc ggggaagaag tggctgatct cagccaccgc gaaaatgaca 2820
tcaaaaacgc cattaacctg atgttctggg gaatataaat gtcaggctcc cttatacaca 2880
gccagtctgc aggtcgacca tagtgactgg atatgttgtg ttttacagta ttatgtagtc 2940
tgtttttat gcaaaatcta atttaatata ttgatattta tatcatttta cgtttctcgt 3000
tcagctttct tgtacaaagt ggtgttaacc tagacttgtc catcttctgg attggccaac 3060
ttaattaatg tatgaaataa aaggatgcac acatagtgac atgctaatca ctataatgtg 3120
ggcatcaaag ttgtgtgtta tgtgtaatta ctagttatct gaataaaaga gaaagagatc 3180
atccatattt cttatcctaa atgaatgtca cgtgtcttta taattctttg atgaaccaga 3240
tgcatttcat taaccaaatc catatacata taaatattaa tcatatataa ttaatatcaa 3300
ttgggttagc aaaacaaatc tagtctaggt gtgttttgcg aattgcggcc gccaccgcgg 3360
tggagctcga attccggtcc gggtcacctt tgtccaccaa gatggaactg cggccgctca 3420
ttaattaagt caggcgcgcc tctagttgaa gacacgttca tgtcttcatc gtaagaagac 3480
actcagtagt cttcggccag aatggccatc tggattcagc aggcctagaa ggccatttaa 3540
atcctgagga tctggtcttc ctaaggaccc gggatatcgg accgattaaa ctttaattcg 3600
gtccgaagct tgcatgcctg cagtgcagcg tgacccggtc gtgcccctct ctagagataa 3660
tgagcattgc atgtctaagt tataaaaaat taccacatat tttttttgtc acacttgttt 3720
gaagtgcagt ttatctatct ttatacatat atttaaactt tactctacga ataatataat 3780
ctatagtact acaataatat cagtgtttta gagaatcata taaatgaaca gttagacatg 3840
gtctaaagga caattgagta ttttgacaac aggactctac agttttatct ttttagtgtg 3900
catgtgttct cctttttttt tgcaaatagc ttcacctata taatacttca tccattttat 3960
tagtacatcc atttagggtt tagggttaat ggtttttata gactaatttt tttagtacat 4020
ctattttatt ctattttagc ctctaaatta agaaaactaa aactctattt tagtttttt 4080
atttaataat ttagatataa aatagaataa aataaagtga ctaaaaatta aacaaatacc 4140
ctttaagaaa ttaaaaaaac taaggaaaca tttttcttgt ttcgagtaga taatgccagc 4200
ctgttaaacg ccgtcgacga gtctaacgga caccaaccag cgaaccagca gcgtcgcgtc 4260
gggccaagcg aagcagacgg cacggcatct ctgtcgctgc ctctggaccc ctctcgagag 4320

ttccgctcca ccgttggact tgctccgctg tcggcatcca gaaattgcgt ggcggagcgg 4380
cagacgtgag ccggcacggc aggcggcctc ctcctcctct cacggcaccg gcagctacgg 4440
gggattcctt tcccaccgct ccttcgcttt cccttcctcg cccgccgtaa taaatagaca 4500
ccccctccac accctctttc cccaacctcg tgttgttcgg agcgcacaca cacacaacca 4560
gatctccccc aaatccaccc gtcggcacct ccgcttcaag gtacgccgct cgtcctcccc 4620
cccccccctc tctaccttct ctagatcggc gttccggtcc atgcatggtt agggcccggt 4680
agttctactt ctgttcatgt ttgtgttaga tccgtgtttg tgttagatcc gtgctgctag 4740
cgttcgtaca cggatgcgac ctgtacgtca gacacgttct gattgctaac ttgccagtgt 4800
ttctctttgg ggaatcctgg gatggctcta gccgttccgc agacgggatc gatttcatga 4860
ttttttttgt ttcgttgcat agggtttggt ttgccctttt cctttatttc aatatatgcc 4920
gtgcacttgt ttgtcgggtc atcttttcat gctttttttt gtcttggttg tgatgatgtg 4980
gtctggttgg gcggtcgttc tagatcggag tagaattctg tttcaaacta cctggtggat 5040
ttattaattt tggatctgta tgtgtgtgcc atacatattc atagttacga attgaagatg 5100
atggatggaa atatcgatct aggataggta tacatgttga tgcgggtttt actgatgcat 5160
atacagagat gctttttgtt cgcttggttg tgatgatgtg gtgtggttgg gcggtcgttc 5220
attcgttcta gatcggagta gaatactgtt tcaaactacc tggtgtattt attaattttg 5280
gaactgtatg tgtgtgtcat acatcttcat agttacgagt ttaagatgga tggaaatatc 5340
gatctaggat aggtatacat gttgatgtgg gttttactga tgcatataca tgatggcata 5400
tgcagcatct attcatatgc tctaaccttg agtacctatc tattataata aacaagtatg 5460
ttttataatt attttgatct tgatatactt ggatgatggc atatgcagca gctatatgtg 5520
gattttttta gccctgcctt catacgctat ttatttgctt ggtactgttt cttttgtcga 5580
tgctcaccct gttgtttggt gttacttctg caggtcgact ttaacttagc ctaggatcca 5640
cacgacacca tgtccccccga gcgccgcccc gtcgagatcc gcccggccac cgccgccgac 5700
atggccgccg tgtgcgacat cgtgaaccac tacatcgaga cctccaccgt gaacttccgc 5760
accgagccgc agaccccgca ggagtggatc gacgacctgg agcgcctcca ggaccgctac 5820
ccgtggctcg tggccgaggt ggagggcgtg gtggccggca tcgcctacgc cggcccgtgg 5880
aaggcccgca acgcctacga ctggaccgtg gagtccaccg tgtacgtgtc ccaccgccac 5940
cagcgcctcg gcctcggctc caccctctac acccacctcc tcaagagcat ggaggcccag 6000
ggcttcaagt ccgtggtggc cgtgatcggc ctcccgaacg acccgtccgt gcgcctccac 6060
gaggccctcg gctacaccgc ccgcggcacc ctccgcgccg ccggctacaa gcacggcggc 6120
tggcacgacg tcggcttctg gcagcgcgac ttcgagctgc cggccccgcc gcgcccggtg 6180
cgcccggtga cgcagatctg agtcgaaacc tagacttgtc catcttctgg attggccaac 6240

ttaattaatg tatgaaataa aaggatgcac acatagtgac atgctaatca ctataatgtg 6300
ggcatcaaag ttgtgtgtta tgtgtaatta ctagttatct gaataaaaga gaaagagatc 6360
atccatattt cttatcctaa atgaatgtca cgtgtcttta taattctttg atgaaccaga 6420
tgcatttcat taaccaaatc catatacata taaatattaa tcatatataa ttaatatcaa 6480
ttgggttagc aaaacaaatc tagtctaggt gtgttttgcg aattgcggcc gccaccgcgg 6540
tggagctcga attcattccg attaatcgtg gcctcttgct cttcaggatg aagagctatg 6600
tttaaacgtg caagcgctac tagacaattc agtacattaa aaacgtccgc aatgtgttat 6660
taagttgtct aagcgtcaat ttggtttaca ccacaatata tcctgccacc agccagccaa 6720
cagctccccg accggcagct cggcacaaaa tcaccactcg atacaggcag cccatcagtc 6780
cgggacggcg tcagcgggag agccgttgta aggcggcaga ctttgctcat gttaccgatg 6840
ctattcggaa gaacggcaac taagctgccg ggtttgaaac acggatgatc tcgcggaggg 6900
tagcatgttg attgtaacga tgacagagcg ttgctgcctg tgatcaaata tcatctccct 6960
cgcagagatc cgaattatca gccttcttat tcatttctcg cttaaccgtg acaggctgtc 7020
gatcttgaga actatgccga cataatagga aatcgctgga taaagccgct gaggaagctg 7080
agtggcgcta tttctttaga agtgaacgtt gacgatcgtc gaccgtaccc cgatgaatta 7140
attcggacgt acgttctgaa cacagctgga tacttacttg ggcgattgtc atacatgaca 7200
tcaacaatgt acccgtttgt gtaaccgtct cttggaggtt cgtatgacac tagtggttcc 7260
cctcagcttg cgactagatg ttgaggccta acattttatt agagagcagg ctagttgctt 7320
agatacatga tcttcaggcc gttatctgtc agggcaagcg aaaattggcc atttatgacg 7380
accaatgccc cgcagaagct cccatctttg ccgccataga cgccgcgccc ccttttgggg 7440
gtgtagaaca tccttttgcc agatgtggaa aagaagttcg ttgtcccatt gttggcaatg 7500
acgtagtagc cggcgaaagt gcgagaccca tttgcgctat atataagcct acgatttccg 7560
ttgcgactat tgtcgtaatt ggatgaacta ttatcgtagt tgctctcaga gttgtcgtaa 7620
tttgatggac tattgtcgta attgcttatg gagttgtcgt agttgcttgg agaaatgtcg 7680
tagttggatg gggagtagtc atagggaaga cgagcttcat ccactaaaac aattggcagg 7740
tcagcaagtg cctgccccga tgccatcgca agtacgaggc ttagaaccac cttcaacaga 7800
tcgcgcatag tcttccccag ctctctaacg cttgagttaa gccgcgccgc gaagcggcgt 7860
cggcttgaac gaattgttag acattatttg ccgactacct tggtgatctc gcctttcacg 7920
tagtgaacaa attcttccaa ctgatctgcg cgcgaggcca agcgatcttc ttgtccaaga 7980
taagcctgcc tagcttcaag tatgacgggc tgatactggg ccggcaggcg ctccattgcc 8040
cagtcggcag cgacatcctt cggcgcgatt ttgccggtta ctgcgctgta ccaaatgcgg 8100
gacaacgtaa gcactacatt tcgctcatcg ccagcccagt cgggcggcga gttccatagc 8160
gttaaggttt catttagcgc ctcaaataga tcctgttcag gaaccggatc aaagagttcc 8220

tccgccgctg gacctaccaa ggcaacgcta tgttctcttg cttttgtcag caagatagcc 8280
agatcaatgt cgatcgtggc tggctcgaag atacctgcaa gaatgtcatt gcgctgccat 8340
tctccaaatt gcagttcgcg cttagctgga taacgccacg gaatgatgtc gtcgtgcaca 8400
acaatggtga cttctacagc gcggagaatc tcgctctctc caggggaagc cgaagtttcc 8460
aaaaggtcgt tgatcaaagc tcgccgcgtt gtttcatcaa gccttacagt caccgtaacc 8520
agcaaatcaa tatcactgtg tggcttcagg ccgccatcca ctgcggagcc gtacaaatgt 8580
acggccagca acgtcggttc gagatggcgc tcgatgacgc caactacctc tgatagttga 8640
gtcgatactt cggcgatcac cgcttccctc atgatgttta actcctgaat taagccgcgc 8700
cgcgaagcgg tgtcggcttg aatgaattgt taggcgtcat cctgtgctcc cgagaaccag 8760
taccagtaca tcgctgtttc gttcgagact tgaggtctag ttttatacgt gaacaggtca 8820
atgccgccga gagtaaagcc acattttgcg tacaaattgc aggcaggtac attgttcgtt 8880
tgtgtctcta atcgtatgcc aaggagctgt ctgcttagtg cccacttttt cgcaaattcg 8940
atgagactgt gcgcgactcc tttgcctcgg tgcgtgtgcg acacaacaat gtgttcgata 9000
gaggctagat cgttccatgt tgagttgagt tcaatcttcc cgacaagctc ttggtcgatg 9060
aatgcgccat agcaagcaga gtcttcatca gagtcatcat ccgagatgta atccttccgg 9120
taggggctca cacttctggt agatagttca aagccttggt cggataggtg cacatcgaac 9180
acttcacgaa caatgaaatg gttctcagca tccaatgttt ccgccacctg ctcagggatc 9240
accgaaatct tcatatgacg cctaacgcct ggcacagcgg atcgcaaacc tggcgcggct 9300
tttggcacaa aaggcgtgac aggtttgcga atccgttgct gccacttgtt aacccttttg 9360
ccagatttgg taactataat ttatgttaga ggcgaagtct tgggtaaaaa ctggcctaaa 9420
attgctgggg atttcaggaa agtaaacatc accttccggc tcgatgtcta ttgtagatat 9480
atgtagtgta tctacttgat cgggggatct gctgcctcgc gcgtttcggt gatgacggtg 9540
aaaacctctg acacatgcag ctcccggaga cggtcacagc ttgtctgtaa gcggatgccg 9600
ggagcagaca agcccgtcag ggcgcgtcag cgggtgttgg cgggtgtcgg ggcgcagcca 9660
tgacccagtc acgtagcgat agcggagtgt atactggctt aactatgcgg catcagagca 9720
gattgtactg agagtgcacc atatgcggtg tgaaataccg cacagatgcg taaggagaaa 9780
ataccgcatc aggcgctctt ccgcttcctc gctcactgac tcgctgcgct cggtcgttcg 9840
gctgcggcga gcggtatcag ctcactcaaa ggcggtaata cggttatcca cagaatcagg 9900
ggataacgca ggaaagaaca tgtgagcaaa aggccagcaa aaggccagga accgtaaaaa 9960
ggccgcgttg ctggcgtttt tccataggct ccgcccccct gacgagcatc acaaaaatcg 10020
acgctcaagt cagaggtggc gaaacccgac aggactataa agataccagg cgtttccccc 10080
tggaagctcc ctcgtgcgct ctcctgttcc gaccctgccg cttaccggat acctgtccgc 10140

ctttctccct tcgggaagcg tggcgctttc tcatagctca cgctgtaggt atctcagttc 10200
ggtgtaggtc gttcgctcca agctgggctg tgtgcacgaa ccccccgttc agcccgaccg 10260
ctgcgcctta tccggtaact atcgtcttga gtccaacccg gtaagacacg acttatcgcc 10320
actggcagca gccactggta acaggattag cagagcgagg tatgtaggcg gtgctacaga 10380
gttcttgaag tggtggccta actacggcta cactagaagg acagtatttg gtatctgcgc 10440
tctgctgaag ccagttacct tcggaaaaag agttggtagc tcttgatccg gcaaacaaac 10500
caccgctggt agcggtggtt tttttgtttg caagcagcag attacgcgca gaaaaaaagg 10560
atctcaagaa gatcctttga tcttttctac ggggtctgac gctcagtgga acgaaaactc 10620
acgttaaggg attttggtca tgagattatc aaaaaggatc ttcacctaga tccttttaaa 10680
ttaaaaatga agttttaaat caatctaaag tatatatgag taaacttggt ctgacagtta 10740
ccaatgctta atcagtgagg cacctatctc agcgatctgt ctatttcgtt catccatagt 10800
tgcctgactc cccgtcgtgt agataactac gatacgggag ggcttaccat ctggccccag 10860
tgctgcaatg ataccgcgag acccacgctc accggctcca gatttatcag caataaacca 10920
gccagccgga agggccgagc gcagaagtgg tcctgcaact ttatccgcct ccatccagtc 10980
tattaattgt tgccgggaag ctagagtaag tagttcgcca gttaatagtt tgcgcaacgt 11040
tgttgccatt gctgcagggg gggggggggg gggggacttc cattgttcat tccacggaca 11100
aaaacagaga aaggaaacga cagaggccaa aaagcctcgc tttcagcacc tgtcgtttcc 11160
tttcttttca gagggtattt taaataaaaa cattaagtta tgacgaagaa gaacggaaac 11220
gccttaaacc ggaaaatttt cataaatagc gaaaacccgc gaggtcgccg ccccgtaacc 11280
tacctgtcgg atcaccggaa aggacccgta aagtgataat gattatcatc tacatatcac 11340
aacgtgcgtg gaggccatca aaccacgtca aataatcaat tatgacgcag gtatcgtatt 11400
aattgatctg catcaactta acgtaaaaac aacttcagac aatacaaatc agcgacactg 11460
aatacggggc aacctcatgt cccccccccc cccccccctg caggcatcgt ggtgtcacgc 11520
tcgtcgtttg gtatggcttc attcagctcc ggttcccaac gatcaaggcg agttacatga 11580
tcccccatgt tgtgcaaaaa agcggttagc tccttcggtc ctccgatcgt tgtcagaagt 11640
aagttggccg cagtgttatc actcatggtt atggcagcac tgcataattc tcttactgtc 11700
atgccatccg taagatgctt ttctgtgact ggtgagtact caaccaagtc attctgagaa 11760
tagtgtatgc ggcgaccgag ttgctcttgc ccggcgtcaa cacgggataa taccgcgcca 11820
catagcagaa ctttaaaagt gctcatcatt ggaaaacgtt cttcggggcg aaaactctca 11880
aggatcttac cgctgttgag atccagttcg atgtaaccca ctcgtgcacc caactgatct 11940
tcagcatctt ttactttcac cagcgtttct gggtgagcaa aaacaggaag gcaaaatgcc 12000
gcaaaaaagg gaataagggc gacacggaaa tgttgaatac tcatactctt cctttttcaa 12060
tattattgaa gcatttatca gggttattgt ctcatgagcg gatacatatt tgaatgtatt 12120

tagaaaaata aacaaatagg ggttccgcgc acatttcccc gaaaagtgcc acctgacgtc 12180

taagaaacca ttattatcat gacattaacc tataaaaata ggcgtatcac gaggcccttt 12240

cgtcttcaag aattcggagc ttttgccatt ctcaccggat tcagtcgtca ctcatggtga 12300

tttctcactt gataacctta tttttgacga ggggaaatta ataggttgta ttgatgttgg 12360

acgagtcgga atcgcagacc gataccagga tcttgccatc ctatggaact gcctcggtga 12420

gttttctcct tcattacaga aacggctttt tcaaaaatat ggtattgata atcctgatat 12480

gaataaattg cagtttcatt tgatgctcga tgagtttttc taatcagaat tggttaattg 12540

gttgtaacac tggcagagca ttacgctgac ttgacgggac ggcggctttg ttgaataaat 12600

cgaacttttg ctgagttgaa ggatcagatc acgcatcttc ccgacaacgc agaccgttcc 12660

gtggcaaagc aaaagttcaa aatcaccaac tggtccacct acaacaaagc tctcatcaac 12720

cgtggctccc tcactttctg gctggatgat ggggcgattc aggcctggta tgagtcagca 12780

acaccttctt cacgaggcag acctcagcgc cagaaggccg ccagagaggc cgagcgcggc 12840

cgtgaggctt ggacgctagg gcagggcatg aaaaagcccg tagcgggctg ctacgggcgt 12900

ctgacgcggt ggaaaggggg aggggatgtt gtctacatgg ctctgctgta gtgagtgggt 12960

tgcgctccgg cagcggtcct gatcaatcgt caccctttct cggtccttca acgttcctga 13020

caacgagcct ccttttcgcc aatccatcga caatcaccgc gagtccctgc tcgaacgctg 13080

cgtccggacc ggcttcgtcg aaggcgtcta tcgcggcccg caacagcggc gagagcggag 13140

cctgttcaac ggtgccgccg cgctcgccgg catcgctgtc gccggcctgc tcctcaagca 13200

cggccccaac agtgaagtag ctgattgtca tcagcgcatt gacggcgtcc ccggccgaaa 13260

aacccgcctc gcagaggaag cgaagctgcg cgtcggccgt ttccatctgc ggtgcgcccg 13320

gtcgcgtgcc ggcatggatg cgcgcgccat cgcggtaggc gagcagcgcc tgcctgaagc 13380

tgcgggcatt cccgatcaga aatgagcgcc agtcgtcgtc ggctctcggc accgaatgcg 13440

tatgattctc cgccagcatg gcttcggcca gtgcgtcgag cagcgcccgc ttgttcctga 13500

agtgccagta aagcgccggc tgctgaaccc ccaaccgttc cgccagtttg cgtgtcgtca 13560

gaccgtctac gccgacctcg ttcaacaggt ccagggcggc acggatcact gtattcggct 13620

gcaactttgt catgcttgac actttatcac tgataaacat aatatgtcca ccaacttatc 13680

agtgataaag aatccgcgcg ttcaatcgga ccagcggagg ctggtccgga ggccagacgt 13740

gaaacccaac atacccctga tcgtaattct gagcactgtc gcgctcgacg ctgtcggcat 13800

cggcctgatt atgccggtgc tgccgggcct cctgcgcgat ctggttcact cgaacgacgt 13860

caccgcccac tatggcattc tgctggcgct gtatgcgttg gtgcaatttg cctgcgcacc 13920

tgtgctgggc gcgctgtcgg atcgtttcgg gcggcggcca atcttgctcg tctcgctggc 13980

cggcgccact gtcgactacg ccatcatggc gacagcgcct ttcctttggg ttctctatat 14040

cgggcggatc gtggccggca tcaccggggc gactggggcg gtagccggcg cttatattgc 14100
cgatatcact gatggcgatg agcgcgcgcg gcacttcggc ttcatgagcg cctgtttcgg 14160
gttcgggatg gtcgcgggac ctgtgctcgg tgggctgatg ggcggtttct ccccccacgc 14220
tccgttcttc gccgcggcag ccttgaacgg cctcaatttc ctgacgggct gtttcctttt 14280
gccggagtcg cacaaaggcg aacgccggcc gttacgccgg gaggctctca acccgctcgc 14340
ttcgttccgg tgggcccggg gcatgaccgt cgtcgccgcc ctgatggcgg tcttcttcat 14400
catgcaactt gtcggacagg tgccggccgc gctttgggtc attttcggcg aggatcgctt 14460
tcactgggac gcgaccacga tcggcatttc gcttgccgca tttggcattc tgcattcact 14520
cgcccaggca atgatcaccg gccctgtagc cgcccggctc ggcgaaaggc gggcactcat 14580
gctcggaatg attgccgacg gcacaggcta catcctgctt gccttcgcga cacggggatg 14640
gatggcgttc ccgatcatgg tcctgcttgc ttcgggtggc atcggaatgc cggcgctgca 14700
agcaatgttg tccaggcagg tggatgagga acgtcagggg cagctgcaag gctcactggc 14760
ggcgctcacc agcctgacct cgatcgtcgg acccctcctc ttcacggcga tctatgcggc 14820
ttctataaca acgtggaacg ggtgggcatg gattgcaggc gctgccctct acttgctctg 14880
cctgccggcg ctgcgtcgcg ggctttggag cggcgcaggg caacgagccg atcgctgatc 14940
gtggaaacga taggcctatg ccatgcgggt caaggcgact tccggcaagc tatacgcgcc 15000
ctaggagtgc ggttggaacg ttggcccagc cagatactcc cgatcacgag caggacgccg 15060
atgatttgaa gcgcactcag cgtctgatcc aagaacaacc atcctagcaa cacggcggtc 15120
cccgggctga gaaagcccag taaggaaaca actgtaggtt cgagtcgcga gatcccccgg 15180
aaccaaagga agtaggttaa acccgctccg atcaggccga gccacgccag gccgagaaca 15240
ttggttcctg taggcatcgg gattggcgga tcaaacacta aagctactgg aacgagcaga 15300
agtcctccgg ccgccagttg ccaggcggta aaggtgagca gaggcacggg aggttgccac 15360
ttgcgggtca gcacggttcc gaacgccatg gaaaccgccc ccgccaggcc cgctgcgacg 15420
ccgacaggat ctagcgctgc gtttggtgtc aacaccaaca gcgccacgcc cgcagttccg 15480
caaatagccc ccaggaccgc catcaatcgt atcgggctac ctagcagagc ggcagagatg 15540
aacacgacca tcagcggctg cacagcgcct accgtcgccg cgaccccgcc cggcaggcgg 15600
tagaccgaaa taaacaacaa gctccagaat agcgaaatat taagtgcgcc gaggatgaag 15660
atgcgcatcc accagattcc cgttggaatc tgtcggacga tcatcacgag caataaaccc 15720
gccggcaacg cccgcagcag cataccggcg acccctcggc ctcgctgttc gggctccacg 15780
aaaacgccgg acagatgcgc cttgtgagcg tccttggggc cgtcctcctg tttgaagacc 15840
gacagcccaa tgatctcgcc gtcgatgtag gcgccgaatg ccacggcatc tcgcaaccgt 15900
tcagcgaacg cctccatggg ctttttctcc tcgtgctcgt aaacggaccc gaacatctct 15960
ggagctttct tcagggccga caatcggatc tcgcggaaat cctgcacgtc ggccgctcca 16020

agccgtcgaa tctgagcctt aatcacaatt gtcaatttta atcctctgtt tatcggcagt 16080
tcgtagagcg cgccgtgcgt cccgagcgat actgagcgaa gcaagtgcgt cgagcagtgc 16140
ccgcttgttc ctgaaatgcc agtaaagcgc tggctgctga acccccagcc ggaactgacc 16200
ccacaaggcc ctagcgtttg caatgcacca ggtcatcatt gacccaggcg tgttccacca 16260
ggccgctgcc tcgcaactct tcgcaggctt cgccgacctg ctcgcgccac ttcttcacgc 16320
gggtggaatc cgatccgcac atgaggcgga aggtttccag cttgagcggg tacggctccc 16380
ggtgcgagct gaaatagtcg aacatccgtc gggccgtcgg cgacagcttg cggtacttct 16440
cccatatgaa tttcgtgtag tggtcgccag caaacagcac gacgatttcc tcgtcgatca 16500
ggacctggca acgggacgtt ttcttgccac ggtccaggac gcggaagcgg tgcagcagcg 16560
acaccgattc caggtgccca acgcggtcgg acgtgaagcc catcgccgtc gcctgtaggc 16620
gcgacaggca ttcctcggcc ttcgtgtaat accggccatt gatcgaccag cccaggtcct 16680
ggcaaagctc gtagaacgtg aaggtgatcg gctcgccgat aggggtgcgc ttcgcgtact 16740
ccaacacctg ctgccacacc agttcgtcat cgtcggcccg cagctcgacg ccggtgtagg 16800
tgatcttcac gtccttgttg acgtggaaaa tgaccttgtt ttgcagcgcc tcgcgcggga 16860
ttttcttgtt gcgcgtggtg aacagggcag agcgggccgt gtcgtttggc atcgctcgca 16920
tcgtgtccgg ccacggcgca atatcgaaca aggaaagctg catttccttg atctgctgct 16980
tcgtgtgttt cagcaacgcg gcctgcttgg cctcgctgac ctgttttgcc aggtcctcgc 17040
cggcggtttt tcgcttcttg gtcgtcatag ttcctcgcgt gtcgatggtc atcgacttcg 17100
ccaaacctgc cgcctcctgt tcgagacgac gcgaacgctc cacggcggcc gatggcgcgg 17160
gcagggcagg gggagccagt tgcacgctgt cgcgctcgat cttggccgta gcttgctgga 17220
ccatcgagcc gacggactgg aaggtttcgc ggggcgcacg catgacggtg cggcttgcga 17280
tggtttcggc atcctcggcg gaaaaccccg cgtcgatcag ttcttgcctg tatgccttcc 17340
ggtcaaacgt ccgattcatt caccctcctt gcgggattgc cccgactcac gccggggcaa 17400
tgtgccctta ttcctgattt gacccgcctg gtgccttggt gtccagataa tccaccttat 17460
cggcaatgaa gtcggtcccg tagaccgtct ggccgtcctt ctcgtacttg gtattccgaa 17520
tcttgccctg cacgaatacc agcgacccct tgcccaaata cttgccgtgg gcctcggcct 17580
gagagccaaa acacttgatg cggaagaagt cggtgcgctc ctgcttgtcg ccggcatcgt 17640
tgcgccactc ttcattaacc gctatatcga aaattgcttg cggcttgtta gaattgccat 17700
gacgtacctc ggtgtcacgg gtaagattac cgataaactg gaactgatta tggctcatat 17760
cgaaagtctc cttgagaaag gagactctag tttagctaaa cattggttcc gctgtcaaga 17820
actttagcgg ctaaaatttt gcgggccgcg accaaaggtg cgaggggcgg cttccgctgt 17880
gtacaaccag atatttttca ccaacatcct tcgtctgctc gatgagcggg gcatgacgaa 17940

acatgagctg tcggagaggg caggggtttc aatttcgttt ttatcagact taaccaacgg 18000
taaggccaac ccctcgttga aggtgatgga ggccattgcc gacgccctgg aaactcccct 18060
acctcttctc ctggagtcca ccgaccttga ccgcgaggca ctcgcggaga ttgcgggtca 18120
tcctttcaag agcagcgtgc cgcccggata cgaacgcatc agtgtggttt tgccgtcaca 18180
taaggcgttt atcgtaaaga aatggggcga cgacacccga aaaaagctgc gtggaaggct 18240
ctgacgccaa gggttagggc ttgcacttcc ttctttagcc gctaaaacgg ccccttctct 18300
gcgggccgtc ggctcgcgca tcatatcgac atcctcaacg gaagccgtgc cgcgaatggc 18360
atcgggcggg tgcgctttga cagttgtttt ctatcagaac ccctacgtcg tgcggttcga 18420
ttagctgttt gtcttgcagg ctaaacactt tcggtatatc gtttgcctgt gcgataatgt 18480
tgctaatgat ttgttgcgta ggggttactg aaaagtgagc gggaaagaag agtttcagac 18540
catcaaggag cgggccaagc gcaagctgga acgcgacatg ggtgcggacc tgttggccgc 18600
gctcaacgac ccgaaaaccg ttgaagtcat gctcaacgcg gacggcaagg tgtggcacga 18660
acgccttggc gagccgatgc ggtacatctg cgacatgcgg cccagccagt cgcaggcgat 18720
tatagaaacg gtggccggat tccacggcaa agaggtcacg cggcattcgc ccatcctgga 18780
aggcgagttc cccttggatg gcagccgctt tgccggccaa ttgccgccgg tcgtggccgc 18840
gccaaccttt gcgatccgca agcgcgcggt cgccatcttc acgctggaac agtacgtcga 18900
ggcgggcatc atgacccgcg agcaatacga ggtcattaaa agcgccgtcg cggcgcatcg 18960
aaacatcctc gtcattggcg gtactggctc gggcaagacc acgctcgtca acgcgatcat 19020
caatgaaatg gtcgccttca acccgtctga gcgcgtcgtc atcatcgagg acaccggcga 19080
aatccagtgc gccgcagaga acgccgtcca ataccacacc agcatcgacg tctcgatgac 19140
gctgctgctc aagacaacgc tgcgtatgcg ccccgaccgc atcctggtcg gtgaggtacg 19200
tggccccgaa gcccttgatc tgttgatggc ctggaacacc gggcatgaag gaggtgccgc 19260
caccctgcac gcaaacaacc ccaaagcggg cctgagccgg ctcgccatgc ttatcagcat 19320
gcacccggat tcaccgaaac ccattgagcc gctgattggc gaggcggttc atgtggtcgt 19380
ccatatcgcc aggaccccta gcggccgtcg agtgcaagaa attctcgaag ttcttggtta 19440
cgagaacggc cagtacatca ccaaaaccct gtaaggagta tttccaatga caacggctgt 19500
tccgttccgt ctgaccatga atcgcggcat tttgttctac cttgccgtgt tcttcgttct 19560
cgctctcgcg ttatccgcgc atccggcgat ggcctcggaa ggcaccggcg gcagcttgcc 19620
atatgagagc tggctgacga acctgcgcaa ctccgtaacc ggcccggtgg ccttcgcgct 19680
gtccatcatc ggcatcgtcg tcgccggcgg cgtgctgatc ttcggcggcg aactcaacgc 19740
cttcttccga accctgatct tcctggttct ggtgatggcg ctgctggtcg gcgcgcagaa 19800
cgtgatgagc accttcttcg gtcgtggtgc cgaaatcgcg gccctcggca acggggcgct 19860
gcaccaggtg caagtcgcgg cggcggatgc cgtgcgtgcg gtagcggctg gacggctcgc 19920

ctaatcatgg ctctgcgcac gatccccatc cgtcgcgcag gcaaccgaga aaacctgttc 19980
atgggtggtg atcgtgaact ggtgatgttc tcgggcctga tggcgtttgc gctgattttc 20040
agcgcccaag agctgcgggc caccgtggtc ggtctgatcc tgtggttcgg ggcgctctat 20100
gcgttccgaa tcatggcgaa ggccgatccg aagatgcggt tcgtgtacct gcgtcaccgc 20160
cggtacaagc cgtattaccc ggcccgctcg accccgttcc gcgagaacac caatagccaa 20220
gggaagcaat accgatgatc caagcaattg cgattgcaat cgcgggcctc ggcgcgcttc 20280
tgttgttcat cctctttgcc cgcatccgcg cggtcgatgc cgaactgaaa ctgaaaaagc 20340
atcgttccaa ggacgccggc ctggccgatc tgctcaacta cgccgctgtc gtcgatgacg 20400
gcgtaatcgt gggcaagaac ggcagcttta tggctgcctg gctgtacaag ggcgatgaca 20460
acgcaagcag caccgaccag cagcgcgaag tagtgtccgc ccgcatcaac caggccctcg 20520
cgggcctggg aagtgggtgg atgatccatg tggacgccgt gcggcgtcct gctccgaact 20580
acgcggagcg gggcctgtcg gcgttccctg accgtctgac ggcagcgatt gaagaagagc 20640
gctcggtctt gccttgctcg tcggtgatgt acttcaccag ctccgcgaag tcgctcttct 20700
tgatggagcg catggggacg tgcttggcaa tcacgcgcac cccccggccg ttttagcggc 20760
taaaaaagtc atggctctgc cctcgggcgg accacgccca tcatgacctt gccaagctcg 20820
tcctgcttct cttcgatctt cgccagcagg gcgaggatcg tggcatcacc gaaccgcgcc 20880
gtgcgcgggt cgtcggtgag ccagagtttc agcaggccgc ccaggcggcc caggtcgcca 20940
ttgatgcggg ccagctcgcg gacgtgctca tagtccacga cgcccgtgat tttgtagccc 21000
tggccgacgg ccagcaggta ggccgacagg ctcatgccgg ccgccgccgc cttttcctca 21060
atcgctcttc gttcgtctgg aaggcagtac accttgatag gtgggctgcc cttcctggtt 21120
ggcttggttt catcagccat ccgcttgccc tcatctgtta cgccggcggt agccggccag 21180
cctcgcagag caggattccc gttgagcacc gccaggtgcg aataagggac agtgaagaag 21240
gaacacccgc tcgcgggtgg gcctacttca cctatcctgc ccggctgacg ccgttggata 21300
caccaaggaa agtctacacg aaccctttgg caaaatcctg tatatcgtgc gaaaaaggat 21360
ggatataccg aaaaaatcgc tataatgacc ccgaagcagg gttatgcagc ggaaaagcgc 21420
tgcttccctg ctgttttgtg gaatatctac cgactggaaa caggcaaatg caggaaatta 21480
ctgaactgag gggacaggcg agagacgatg ccaaagagct acaccgacga gctggccgag 21540
tgggttgaat cccgcgcggc caagaagcgc cggcgtgatg aggctgcggt tgcgttcctg 21600
gcggtgaggg cggatgtcga ggcggcgtta gcgtccggct atgcgctcgt caccatttgg 21660
gagcacatgc gggaaacggg gaaggtcaag ttctcctacg agacgttccg ctcgcacgcc 21720
aggcggcaca tcaaggccaa gcccgccgat gtgcccgcac cgcaggccaa ggctgcggaa 21780
cccgcgccgg cacccaagac gccggagcca cggcggccga agcagggggg caaggctgaa 21840

aagccggccc ccgctgcggc cccgaccggc ttcaccttca acccaacacc ggacaaaaag 21900
gatctactgt aatggcgaaa attcacatgg ttttgcaggg caagggcggg gtcggcaagt 21960
cggccatcgc cgcgatcatt gcgcagtaca agatggacaa ggggcagaca cccttgtgca 22020
tcgacaccga cccggtgaac gcgacgttcg agggctacaa ggccctgaac gtccgccggc 22080
tgaacatcat ggccggcgac gaaattaact cgcgcaactt cgacaccctg gtcgagctga 22140
ttgcgccgac caaggatgac gtggtgatcg acaacggtgc cagctcgttc gtgcctctgt 22200
cgcattacct catcagcaac caggtgccgg ctctgctgca agaaatgggg catgagctgg 22260
tcatccatac cgtcgtcacc ggcggccagg ctctcctgga cacggtgagc ggcttcgccc 22320
agctcgccag ccagttcccg gccgaagcgc ttttcgtggt ctggctgaac ccgtattggg 22380
ggcctatcga gcatgagggc aagagctttg agcagatgaa ggcgtacacg gccaacaagg 22440
cccgcgtgtc gtccatcatc cagattccgg ccctcaagga agaaacctac ggccgcgatt 22500
tcagcgacat gctgcaagag cggctgacgt tcgaccaggc gctggccgat gaatcgctca 22560
cgatcatgac gcggcaacgc ctcaagatcg tgcggcgcgg cctgtttgaa cagctcgacg 22620
cggcggccgt gctatgagcg accagattga agagctgatc cgggagattg cggccaagca 22680
cggcatcgcc gtcggccgcg acgacccggt gctgatcctg cataccatca acgcccggct 22740
catggccgac agtgcggcca agcaagagga aatccttgcc gcgttcaagg aagagctgga 22800
agggatcgcc catcgttggg gcgaggacgc caaggccaaa gcggagcgga tgctgaacgc 22860
ggccctggcg gccagcaagg acgcaatggc gaaggtaatg aaggacagcg ccgcgcaggc 22920
ggccgaagcg atccgcaggg aaatcgacga cggccttggc cgccagctcg cggccaaggt 22980
cgcggacgcg cggcgcgtgg cgatgatgaa catgatcgcc ggcggcatgg tgttgttcgc 23040
ggccgccctg gtggtgtggg cctcgttatg aatcgcagag gcgcagatga aaaagcccgg 23100
cgttgccggg ctttgttttt gcgttagctg ggcttgtttg acaggcccaa gctctgactg 23160
cgcccgcgct cgcgctcctg ggcctgtttc ttctcctgct cctgcttgcg catcagggcc 23220
tggtgccgtc gggctgcttc acgcatcgaa tcccagtcgc cggccagctc gggatgctcc 23280
gcgcgcatct tgcgcgtcgc cagttcctcg atcttgggcg cgtgaatgcc catgccttcc 23340
ttgatttcgc gcaccatgtc cagccgcgtg tgcagggtct gcaagcgggc ttgctgttgg 23400
gcctgctgct gctgccaggc ggcctttgta cgcggcaggg acagcaagcc gggggcattg 23460
gactgtagct gctgcaaacg cgcctgctga cggtctacga gctgttctag gcggtcctcg 23520
atgcgctcca cctggtcatg ctttgcctgc acgtagagcg caagggtctg ctggtaggtc 23580
tgctcgatgg gcgcggattc taagagggcc tgctgttccg tctcggcctc ctgggccgcc 23640
tgtagcaaat cctcgccgct gttgccgctg gactgcttta ctgccgggga ctgctgttgc 23700
cctgctcgcg ccgtcgtcgc agttcggctt gcccccactc gattgactgc ttcatttcga 23760
gccgcagcga tgcgatctcg gattgcgtca acggacgggg cagcgcggag gtgtccggct 23820

tctccttggg tgagtcggtc gatgccatag ccaaaggttt ccttccaaaa tgcgtccatt 23880
gctggaccgt gtttctcatt gatgcccgca agcatcttcg gcttgaccgc caggtcaagc 23940
gcgccttcat gggcggtcat gacggacgcc gccatgacct tgccgccgtt gttctcgatg 24000
tagccgcgta atgaggcaat ggtgccgccc atcgtcagcg tgtcatcgac aacgatgtac 24060
ttctggccgg ggatcacctc cccctcgaaa gtcgggttga acgccaggcg atgatctgaa 24120
ccggctccgg ttcgggcgac cttctcccgc tgcacaatgt ccgtttcgac ctcaaggcca 24180
aggcggtcgg ccagaacgac cgccatcatg gccggaatct tgttgttccc cgccgcctcg 24240
acggcgagga ctggaacgat gcggggcttg tcgtcgccga tcagcgtctt gagctgggca 24300
acagtgtcgt ccgaaatcag gcgctcgacc aaattaagcg ccgcttccgc gtcgccctgc 24360
ttcgcagcct ggtattcagg ctcgttggtc aaagaaccaa ggtcgccgtt gcgaaccacc 24420
ttcgggaagt ctccccacgg tgcgcgctcg gctctgctgt agctgctcaa gacgcctccc 24480
tttttagccg ctaaaactct aacgagtgcg cccgcgactc aacttgacgc tttcggcact 24540
tacctgtgcc ttgccacttg cgtcataggt gatgcttttc gcactcccga tttcaggtac 24600
tttatcgaaa tctgaccggg cgtgcattac aaagttcttc cccacctgtt ggtaaatgct 24660
gccgctatct gcgtggacga tgctgccgtc gtggcgctgc gacttatcgg ccttttgggc 24720
catatagatg ttgtaaatgc caggtttcag ggccccggct ttatctacct tctggttcgt 24780
ccatgcgcct tggttctcgg tctggacaat tctttgccca ttcatgacca ggaggcggtg 24840
tttcattggg tgactcctga cggttgcctc tggtgttaaa cgtgtcctgg tcgcttgccg 24900
gctaaaaaaa agccgacctc ggcagttcga ggccggcttt ccctagagcc gggcgcgtca 24960
aggttgttcc atctatttta gtgaactgcg ttcgatttat cagttacttt cctcccgctt 25020
tgtgtttcct cccactcgtt tccgcgtcta gccgacccct caacatagcg gcctcttctt 25080
gggctgcctt tgcctcttgc cgcgcttcgt cacgctcggc ttgcaccgtc gtaaagcgct 25140
cggcctgcct ggccgcctct tgcgccgcca acttcctttg ctcctggtgg gcctcggcgt 25200
cggcctgcgc cttcgctttc accgctgcca actccgtgcg caaactctcc gcttcgcgcc 25260
tggtggcgtc gcgctcgccg cgaagcgcct gcatttcctg gttggccgcg tccagggtct 25320
tgcggctctc ttctttgaat gcgcgggcgt cctggtgagc gtagtccagc tcggcgcgca 25380
gctcctgcgc tcgacgctcc acctcgtcgg cccgctgcgt cgccagcgcg gcccgctgct 25440
cggctcctgc cagggcggtg cgtgcttcgg ccagggcttg ccgctggcgt gcggccagct 25500
cggccgcctc ggcggcctgc tgctctagca atgtaacgcg cgcctgggct tcttccagct 25560
cgcgggcctg cgcctcgaag gcgtcggcca gctccccgcg cacggcttcc aactcgttgc 25620
gctcacgatc ccagccggct tgcgctgcct gcaacgattc attggcaagg gcctgggcgg 25680
cttgccagag ggcggccacg gcctggttgc cggcctgctg caccgcgtcc ggcacctgga 25740

ctgccagcgg ggcggcctgc gccgtgcgct ggcgtcgcca ttcgcgcatg ccggcgctgg 25800

cgtcgttcat gttgacgcgg gcggccttac gcactgcatc cacggtcggg aagttctccc 25860

ggtcgccttg ctcgaacagc tcgtccgcag ccgcaaaaat gcggtcgcgc gtctctttgt 25920

tcagttccat gttggctccg gtaattggta agaataataa tactcttacc taccttatca 25980

gcgcaagagt ttagctgaac agttctcgac ttaacggcag gttttttagc ggctgaaggg 26040

caggcaaaaa aagccccgca cggtcggcgg gggcaaaggg tcagcgggaa ggggattagc 26100

gggcgtcggg cttcttcatg cgtcggggcc gcgcttcttg ggatggagca cgacgaagcg 26160

cgcacgcgca tcgtcctcgg ccctatcggc ccgcgtcgcg gtcaggaact tgtcgcgcgc 26220

taggtcctcc ctggtgggca ccaggggcat gaactcggcc tgctcgatgt aggtccactc 26280

catgaccgca tcgcagtcga ggccgcgttc cttcaccgtc tcttgcaggt cgcggtacgc 26340

ccgctcgttg agcggctggt aacgggccaa ttggtcgtaa atggctgtcg gccatgagcg 26400

gcctttcctg ttgagccagc agccgacgac gaagccggca atgcaggccc ctggcacaac 26460

caggccgacg ccgggggcag gggatggcag cagctcgcca accaggaacc ccgccgcgat 26520

gatgccgatg ccggtcaacc agcccttgaa actatccggc cccgaaacac ccctgcgcat 26580

tgcctggatg ctgcgccgga tagcttgcaa catcaggagc cgtttctttt gttcgtcagt 26640

catggtccgc cctcaccagt tgttcgtatc ggtgtcggac gaactgaaat cgcaagagct 26700

gccggtatcg gtccagccgc tgtccgtgtc gctgctgccg aagcacggcg aggggtccgc 26760

gaacgccgca gacggcgtat ccggccgcag cgcatcgccc agcatggccc cggtcagcga 26820

gccgccggcc aggtagccca gcatggtgct gttggtcgcc ccggccacca gggccgacgt 26880

gacgaaatcg ccgtcattcc ctctggattg ttcgctgctc ggcggggcag tgcgccgcgc 26940

cggcggcgtc gtggatggct cgggttggct ggcctgcgac ggccggcgaa aggtgcgcag 27000

cagctcgtta tcgaccggct gcggcgtcgg ggccgccgcc ttgcgctgcg gtcggtgttc 27060

cttcttcggc tcgcgcagct tgaacagcat gatcgcggaa accagcagca acgccgcgcc 27120

tacgcctccc gcgatgtaga acagcatcgg attcattctt cggtcctcct tgtagcggaa 27180

ccgttgtctg tgcggcgcgg gtggcccgcg ccgctgtctt tggggatcag ccctcgatga 27240

gcgcgaccag tttcacgtcg gcaaggttcg cctcgaactc ctggccgtcg tcctcgtact 27300

tcaaccaggc atagccttcc gccggcggcc gacggttgag gataaggcgg gcagggcgct 27360

cgtcgtgctc gacctggacg atggcctttt tcagcttgtc cgggtccggc tccttcgcgc 27420

ccttttcctt ggcgtcctta ccgtcctggt cgccgtcctc gccgtcctgg ccgtcgccgg 27480

cctccgcgtc acgctcggca tcagtctggc cgttgaaggc atcgacggtg ttgggatcgc 27540

ggcccttctc gtccaggaac tcgcgcagca gcttgaccgt gccgcgcgtg atttcctggg 27600

tgtcgtcgtc aagccacgcc tcgacttcct ccgggcgctt cttgaaggcc gtcaccagct 27660

cgttcaccac ggtcacgtcg cgcacgcggc cggtgttgaa cgcatcggcg atcttctccg 27720

gcaggtccag cagcgtgacg tgctgggtga tgaacgccgg cgacttgccg atttccttgg 27780

cgatatcgcc tttcttcttg cccttcgcca gctcgcggcc aatgaagtcg gcaatttcgc 27840

gcggggtcag ctcgttgcgt tgcaggttct cgataacctg gtcggcttcg ttgtagtcgt 27900

tgtcgatgaa cgccgggatg gacttcttgc cggcccactt cgagccacgg tagcggcggg 27960

cgccgtgatt gatgatatag cggcccggct gctcctggtt ctcgcgcacc gaaatgggtg 28020

acttcacccc gcgctctttg atcgtggcac cgatttccgc gatgctctcc ggggaaaagc 28080

cggggttgtc ggccgtccgc ggctgatgcg gatcttcgtc gatcaggtcc aggtccagct 28140

cgatagggcc ggaaccgccc tgagacgccg caggagcgtc caggaggctc gacaggtcgc 28200

cgatgctatc caaccccagg ccggacggct gcgccgcgcc tgcggcttcc tgagcggccg 28260

cagcggtgtt tttcttggtg gtcttggctt gagccgcagt cattgggaaa tctccatctt 28320

cgtgaacacg taatcagcca gggcgcgaac ctctttcgat gccttgcgcg cggccgtttt 28380

cttgatcttc cagaccggca caccggatgc gagggcatcg gcgatgctgc tgcgcaggcc 28440

aacggtggcc ggaatcatca tcttggggta cgcggccagc agctcggctt ggtggcgcgc 28500

gtggcgcgga ttccgcgcat cgaccttgct gggcaccatg ccaaggaatt gcagcttggc 28560

gttcttctgg cgcacgttcg caatggtcgt gaccatcttc ttgatgccct ggatgctgta 28620

cgcctcaagc tcgatggggg acagcacata gtcggccgcg aagagggcgg ccgccaggcc 28680

gacgccaagg gtcggggccg tgtcgatcag gcacacgtcg aagccttggt tcgccagggc 28740

cttgatgttc gccccgaaca gctcgcgggc gtcgtccagc gacagccgtt cggcgttcgc 28800

cagtaccggg ttggactcga tgagggcgag gcgcgcggcc tggccgtcgc cggctgcggg 28860

tgcggtttcg gtccagccgc cggcagggac agcgccgaac agcttgcttg catgcaggcc 28920

ggtagcaaag tccttgagcg tgtaggacgc attgccctgg gggtccaggt cgatcacggc 28980

aacccgcaag ccgcgctcga aaaagtcgaa ggcaagatgc acaagggtcg aagtcttgcc 29040

gacgccgcct ttctggttgg ccgtgaccaa agttttcatc gtttggtttc ctgtttttc 29100

ttggcgtccg cttcccactt ccggacgatg tacgcctgat gttccggcag aaccgccgtt 29160

acccgcgcgt accccctcggg caagttcttg tcctcgaacg cggcccacac gcgatgcacc 29220

gcttgcgaca ctgcgcccct ggtcagtccc agcgacgttg cgaacgtcgc ctgtggcttc 29280

ccatcgacta agacgccccg cgctatctcg atggtctgct gccccacttc cagcccctgg 29340

atcgcctcct ggaactggct ttcggtaagc cgtttcttca tggataacac ccataatttg 29400

ctccgcgcct tggttgaaca tagcggtgac agccgccagc acatgagaga agtttagcta 29460

aacatttctc gcacgtcaac acctttagcc gctaaaactc gtccttggcg taacaaaaca 29520

aaagcccgga aaccgggctt tcgtctcttg ccgcttatgg ctctgcaccc ggctccatca 29580

ccaacaggtc gcgcacgcgc ttcactcggt tgcggatcga cactgccagc ccaacaaagc 29640

cggttgccgc cgccgccagg atcgcgccga tgatgccggc cacaccggcc atcgcccacc 29700
aggtcgccgc cttccggttc cattcctgct ggtactgctt cgcaatgctg gacctcggct 29760
caccataggc tgaccgctcg atggcgtatg ccgcttctcc ccttggcgta aaacccagcg 29820
ccgcaggcgg cattgccatg ctgcccgccg ctttcccgac cacgacgcgc gcaccaggct 29880
tgcggtccag accttcggcc acggcgagct gcgcaaggac ataatcagcc gccgacttgg 29940
ctccacgcgc ctcgatcagc tcttgcactc gcgcgaaatc cttggcctcc acggccgcca 30000
tgaatcgcgc acgcggcgaa ggctccgcag ggccggcgtc gtgatcgccg ccgagaatgc 30060
ccttcaccaa gttcgacgac acgaaaatca tgctgacggc tatcaccatc atgcagacgg 30120
atcgcacgaa cccgctgaat tgaacacgag cacggcaccc gcgaccacta tgccaagaat 30180
gcccaaggta aaaattgccg gccccgccat gaagtccgtg aatgccccga cggccgaagt 30240
gaagggcagg ccgccaccca ggccgccgcc ctcactgccc ggcacctggt cgctgaatgt 30300
cgatgccagc acctgcggca cgtcaatgct tccgggcgtc gcgctcgggc tgatcgccca 30360
tcccgttact gccccgatcc cggcaatggc aaggactgcc agcgctgcca tttttggggt 30420
gaggccgttc gcggccgagg ggcgcagccc ctgggggat gggaggcccg cgttagcggg 30480
ccgggagggt tcgagaaggg ggggcacccc ccttcggcgt gcgcggtcac gcgcacaggg 30540
cgcagccctg gttaaaaaca aggtttataa atattggttt aaaagcaggt taaaagacag 30600
gttagcggtg gccgaaaaac gggcggaaac ccttgcaaat gctggatttt ctgcctgtgg 30660
acagcccctc aaatgtcaat aggtgcgccc ctcatctgtc agcactctgc ccctcaagtg 30720
tcaaggatcg cgcccctcat ctgtcagtag tcgcgcccct caagtgtcaa taccgcaggg 30780
cacttatccc caggcttgtc cacatcatct gtgggaaact cgcgtaaaat caggcgtttt 30840
cgccgatttg cgaggctggc cagctccacg tcgccggccg aaatcgagcc tgcccctcat 30900
ctgtcaacgc cgcgccgggt gagtcggccc ctcaagtgtc aacgtccgcc cctcatctgt 30960
cagtgagggc caagttttcc gcgaggtatc cacaacgccg gcggccgcgg tgtctcgcac 31020
acggcttcga cggcgtttct ggcgcgtttg cagggccata gacggccgcc agcccagcgg 31080
cgagggcaac cagcccggtg agcgtcggaa aggcgctgga agccccgtag cgacgcggag 31140
aggggcgaga caagccaagg gcgcaggctc gatgcgcagc acgacatagc cggttctcgc 31200
aaggacgaga atttccctgc ggtgcccctc aagtgtcaat gaaagtttcc aacgcgagcc 31260
attcgcgaga gccttgagtc cacgctagat gagagctttg ttgtaggtgg accagttggt 31320
gattttgaac ttttgctttg ccacggaacg gtctgcgttg tcgggaagat gcgtgatctg 31380
atccttcaac tcagcaaaag ttcgatttat tcaacaaagc cacgttgtgt ctcaaaatct 31440
ctgatgttac attgcacaag ataaaaatat atcatcatga acaataaaac tgtctgctta 31500
cataaacagt aatacaaggg gtgttatgag ccatattcaa cgggaaacgt cttgctcgac 31560
tctagagctc gttcctcgag gaacggtacc tgcggggaag cttacaataa tgtgtgttgt 31620

taagtcttgt tgcctgtcat cgtctgactg actttcgtca taaatcccgg cctccgtaac 31680
ccagctttgg gcaagctcac ggatttgatc cggcggaacg ggaatatcga gatgccgggc 31740
tgaacgctgc agttccagct ttccctttcg ggacaggtac tccagctgat tgattatctg 31800
ctgaagggtc ttggttccac ctcctggcac aatgcgaatg attacttgag cgcgatcggg 31860
catccaattt tctcccgtca ggtgcgtggt caagtgctac aaggcacctt tcagtaacga 31920
gcgaccgtcg atccgtcgcc gggatacgga caaaatggag cgcagtagtc catcgagggc 31980
ggcgaaagcc tcgccaaaag caatacgttc atctcgcaca gcctccagat ccgatcgagg 32040
gtcttcggcg taggcagata gaagcatgga tacattgctt gagagtattc cgatggactg 32100
aagtatggct tccatctttt ctcgtgtgtc tgcatctatt tcgagaaagc cccgatgcg 32160
gcgcaccgca acgcgaattg ccatactatc cgaaagtccc agcaggcgcg cttgatagga 32220
aaaggtttca tactcggccg atcgcagacg ggcactcacg accttgaacc cttcaacttt 32280
cagggatcga tgctggttga tggtagtctc actcgacgtg gctctggtgt gttttgacat 32340
agcttcctcc aaagaaagcg gaaggtctgg atactccagc acgaaatgtg cccgggtaga 32400
cggatggaag tctagccctg ctcaatatga aatcaacagt acatttacag tcaatactga 32460
atatacttgc tacatttgca attgtcttat aacgaatgtg aaataaaaat agtgtaacaa 32520
cgcttttact catcgataat cacaaaaaca tttatacgaa caaaaataca aatgcactcc 32580
ggtttcacag gataggcggg atcagaatat gcaacttttg acgttttgtt ctttcaaagg 32640
gggtgctggc aaaaccaccg cactcatggg cctttgcgct gctttggcaa atgacggtaa 32700
acgagtggcc ctctttgatg ccgacgaaaa ccggcctctg acgcgatgga gagaaaacgc 32760
cttacaaagc agtactggga tcctcgctgt gaagtctatt ccgccgacga aatgcccctt 32820
cttgaagcag cctatgaaaa tgccgagctc gaaggatttg attatgcgtt ggccgatacg 32880
cgtggcggct cgagcgagct caacaacaca atcatcgcta gctcaaacct gcttctgatc 32940
cccaccatgc taacgccgct cgacatcgat gaggcactat ctacctaccg ctacgtcatc 33000
gagctgctgt tgagtgaaaa tttggcaatt cctacagctg ttttgcgcca acgcgtcccg 33060
gtcggccgat tgacaacatc gcaacgcagg atgtcagaga cgctagagag ccttccagtt 33120
gtaccgtctc ccatgcatga aagagatgca tttgccgcga tgaaagaacg cggcatgttg 33180
catcttacat tactaaacac gggaactgat ccgacgatgc gcctcataga gaggaatctt 33240
cggattgcga tggaggaagt cgtggtcatt tcgaaactga tcagcaaaat cttggaggct 33300
tgaagatggc aattcgcaag cccgcattgt cggtcggcga agcacggcgg cttgctggtg 33360
ctcgacccga gatccaccat cccaacccga cacttgttcc ccagaagctg gacctccagc 33420
acttgcctga aaaagccgac gagaaagacc agcaacgtga gcctctcgtc gccgatcaca 33480
tttacagtcc cgatcgacaa cttaagctaa ctgtggatgc ccttagtcca cctccgtccc 33540

cgaaaaagct ccaggttttt ctttcagcgc gaccgcccgc gcctcaagtg tcgaaaacat 33600
atgacaacct cgttcggcaa tacagtccct cgaagtcgct acaaatgatt ttaaggcgcg 33660
cgttggacga tttcgaaagc atgctggcag atggatcatt tcgcgtggcc ccgaaaagtt 33720
atccgatccc ttcaactaca gaaaaatccg ttctcgttca gacctcacgc atgttcccgg 33780
ttgcgttgct cgaggtcgct cgaagtcatt ttgatccgtt ggggttggag accgctcgag 33840
ctttcggcca caagctggct accgccgcgc tcgcgtcatt ctttgctgga gagaagccat 33900
cgagcaattg gtgaagaggg acctatcgga acccctcacc aaatattgag tgtaggtttg 33960
aggccgctgg ccgcgtcctc agtcaccttt tgagccagat aattaagagc caaatgcaat 34020
tggctcaggc tgccatcgtc cccccgtgcg aaacctgcac gtccgcgtca aagaaataac 34080
cggcacctct tgctgttttt atcagttgag ggcttgacgg atccgcctca agtttgcggc 34140
gcagccgcaa aatgagaaca tctatactcc tgtcgtaaac ctcctcgtcg cgtactcgac 34200
tggcaatgag aagttgctcg cgcgatagaa cgtcgcgggg tttctctaaa aacgcgagga 34260
gaagattgaa ctcacctgcc gtaagtttca cctcaccgcc agcttcggac atcaagcgac 34320
gttgcctgag attaagtgtc cagtcagtaa aacaaaaaga ccgtcggtct ttggagcgga 34380
caacgttggg gcgcacgcgc aaggcaaccc gaatgcgtgc aagaaactct ctcgtactaa 34440
acggcttagc gataaaatca cttgctccta gctcgagtgc aacaacttta tccgtctcct 34500
caaggcggtc gccactgata attatgattg gaatatcaga ctttgccgcc agatttcgaa 34560
cgatctcaag cccatcttca cgacctaaat ttagatcaac aaccacgaca tcgaccgtcg 34620
cggaagagag tactctagtg aactgggtgc tgtcggctac cgcggtcact ttgaaggcgt 34680
ggatcgtaag gtattcgata ataagatgcc gcatagcgac atcgtcatcg ataagaagaa 34740
cgtgtttcaa cggctcacct ttcaatctaa aatctgaacc cttgttcaca gcgcttgaga 34800
aattttcacg tgaaggatgt acaatcatct ccagctaaat gggcagttcg tcagaattgc 34860
ggctgaccgc ggatgacgaa aatgcgaacc aagtatttca attttatgac aaaagttctc 34920
aatcgttgtt acaagtgaaa cgcttcgagg ttacagctac tattgattaa ggagatcgcc 34980
tatggtctcg ccccggcgtc gtgcgtccgc cgcgagccag atctcgccta cttcataaac 35040
gtcctcatag gcacggaatg gaatgatgac atcgatcgcc gtagagagca tgtcaatcag 35100
tgtgcgatct tccaagctag caccttgggc gctacttttg acaagggaaa acagtttctt 35160
gaatccttgg attggattcg cgccgtgtat tgttgaaatc gatcccggat gtcccgagac 35220
gacttcactc agataagccc atgctgcatc gtcgcgcatc tcgccaagca atatccggtc 35280
cggccgcata cgcagacttg cttggagcaa gtgctcggcg ctcacagcac ccagcccagc 35340
accgttcttg gagtagagta gtctaacatg attatcgtgt ggaatgacga gttcgagcgt 35400
atcttctatg gtgattagcc tttcctgggg ggggatggcg ctgatcaagg tcttgctcat 35460
tgttgtcttg ccgcttccgg tagggccaca tagcaacatc gtcagtcggc tgacgacgca 35520

tgcgtgcaga aacgcttcca aatccccgtt gtcaaaatgc tgaaggatag cttcatcatc 35580
ctgattttgg cgtttccttc gtgtctgcca ctggttccac ctcgaagcat cataacggga 35640
ggagacttct ttaagaccag aaacacgcga gcttggccgt cgaatggtca agctgacggt 35700
gcccgaggga acggtcggcg gcagacagat ttgtagtcgt tcaccaccag gaagttcagt 35760
ggcgcagagg gggttacgtg gtccgacatc ctgctttctc agcgcgcccg ctaaaatagc 35820
gatatcttca agatcatcat aagagacggg caaaggcatc ttggtaaaaa tgccggcttg 35880
gcgcacaaat gcctctccag gtcgattgat cgcaatttct tcagtcttcg ggtcatcgag 35940
ccattccaaa atcggcttca gaagaaagcg tagttgcgga tccacttcca tttacaatgt 36000
atcctatctc taagcggaaa tttgaattca ttaagagcgg cggttcctcc cccgcgtggc 36060
gccgccagtc aggcggagct ggtaaacacc aaagaaatcg aggtcccgtg ctacgaaaat 36120
ggaaacggtg tcaccctgat tcttcttcag ggttggcggt atgttgatgg ttgccttaag 36180
ggctgtctca gttgtctgct caccgttatt ttgaaagctg ttgaagctca tcccgccacc 36240
cgagctgccg gcgtaggtgc tagctgcctg gaaggcgcct tgaacaacac tcaagagcat 36300
agctccgcta aaacgctgcc agaagtggct gtcgaccgag cccggcaatc ctgagcgacc 36360
gagttcgtcc gcgcttggcg atgttaacga gatcatcgca tggtcaggtg tctcggcgcg 36420
atcccacaac acaaaaacgc gcccatctcc ctgttgcaag ccacgctgta tttcgccaac 36480
aacggtggtg ccacgatcaa gaagcacgat attgttcgtt gttccacgaa tatcctgagg 36540
caagacacac tttacatagc ctgccaaatt tgtgtcgatt gcggtttgca agatgcacgg 36600
aattattgtc ccttgcgtta ccataaaatc ggggtgcggc aagagcgtgg cgctgctggg 36660
ctgcagctcg gtgggtttca tacgtatcga caaatcgttc tcgccggaca cttcgccatt 36720
cggcaaggag ttgtcgtcac gcttgccttc ttgtcttcgg cccgtgtcgc cctgaatggc 36780
gcgtttgctg acccccttgat cgccgctgct atatgcaaaa atcggtgttt cttccggccg 36840
tggctcatgc cgctccggtt cgcccctcgg cggtagagga gcagcaggct gaacagcctc 36900
ttgaaccgct ggaggatccg gcggcacctc aatcggagct ggatgaaatg gcttggtgtt 36960
tgttgcgatc aaagttgacg gcgatgcgtt ctcattcacc ttcttttggc gcccacctag 37020
ccaaatgagg cttaatgata acgcgagaac gacacctccg acgatcaatt tctgagaccc 37080
cgaaagacgc cggcgatgtt tgtcggagac cagggatcca gatgcatcaa cctcatgtgc 37140
cgcttgctga ctatcgttat tcatcccttc gccccccttca ggacgcgttt cacatcgggc 37200
ctcaccgtgc ccgtttgcgg cctttggcca acgggatcgt aagcggtgtt ccagatacat 37260
agtactgtgt ggccatccct cagacgccaa cctcgggaaa ccgaagaaat ctcgacatcg 37320
ctccctttaa ctgaatagtt ggcaacagct tccttgccat caggattgat ggtgtagatg 37380
gagggtatgc gtacattgcc cggaaagtgg aataccgtcg taaatccatt gtcgaagact 37440

tcgagtggca acagcgaacg atcgccttgg gcgacgtagt gccaattact gtccgccgca 37500
ccaagggctg tgacaggctg atccaataaa ttctcagctt tccgttgata ttgtgcttcc 37560
gcgtgtagtc tgtccacaac agccttctgt tgtgcctccc ttcgccgagc cgccgcatcg 37620
tcggcggggt aggcgaattg gacgctgtaa tagagatcgg gctgctcttt atcgaggtgg 37680
gacagagtct tggaacttat actgaaaaca taacggcgca tcccggagtc gcttgcggtt 37740
agcacgatta ctggctgagg cgtgaggacc tggcttgcct tgaaaaatag ataatttccc 37800
cgcggtaggg ctgctagatc tttgctattt gaaacggcaa ccgctgtcac cgtttcgttc 37860
gtggcgaatg ttacgaccaa agtagctcca accgccgtcg agaggcgcac cacttgatcg 37920
ggattgtaag ccaaataacg catgcgcgga tctagcttgc ccgccattgg agtgtcttca 37980
gcctccgcac cagtcgcagc ggcaaataaa catgctaaaa tgaaaagtgc ttttctgatc 38040
atggttcgct gtggcctacg tttgaaacgg tatcttccga tgtctgatag gaggtgacaa 38100
ccagacctgc cgggttggtt agtctcaatc tgccgggcaa gctggtcacc ttttcgtagc 38160
gaactgtcgc ggtccacgta ctcaccacag gcattttgcc gtcaacgacg agggtccttt 38220
tatagcgaat ttgctgcgtg cttggagtta catcatttga agcgatgtgc tcgacctcca 38280
ccctgccgcg tttgccaaga atgacttgag gcgaactggg attgggatag ttgaagaatt 38340
gctggtaatc ctggcgcact gttggggcac tgaagttcga taccaggtcg taggcgtact 38400
gagcggtgtc ggcatcataa ctctcgcgca ggcgaacgta ctcccacaat gaggcgttaa 38460
cgacggcctc ctcttgagtt gcaggcaatc gcgagacaga cacctcgctg tcaacggtgc 38520
cgtccggccg tatccataga tatacgggca caagcctgct caacggcacc attgtggcta 38580
tagcgaacgc ttgagcaaca tttcccaaaa tcgcgatagc tgcgacagct gcaatgagtt 38640
tggagagacg tcgcgccgat ttcgctcgcg cggtttgaaa ggcttctact tccttatagt 38700
gctcggcaag gctttcgcgc gccactagca tggcatattc aggccccgtc atagcgtcca 38760
cccgaattgc cgagctgaag atctgacgga gtaggctgcc atcgccccac attcagcggg 38820
aagatcgggc ctttgcagct cgctaatgtg tcgtttgtct ggcagccgct caaagcgaca 38880
actaggcaca gcaggcaata cttcatagaa ttctccattg aggcgaattt ttgcgcgacc 38940
tagcctcgct caacctgagc gaagcgacgg tacaagctgc tggcagattg ggttgcgccg 39000
ctccagtaac tgcctccaat gttgccggcg atcgccggca aagcgacaat gagcgcatcc 39060
cctgtcagaa aaaacatatc gagttcgtaa agaccaatga tcttggccgc ggtcgtaccg 39120
gcgaaggtga ttacaccaag cataagggtg agcgcagtcg cttcggttag gatgacgatc 39180
gttgccacga ggtttaagag gagaagcaag agaccgtagg tgataagttg cccgatccac 39240
ttagctgcga tgtcccgcgt gcgatcaaaa atatatccga cgaggatcag aggcccgatc 39300
gcgagaagca ctttcgtgag aattccaacg gcgtcgtaaa ctccgaaggc agaccagagc 39360
gtgccgtaaa ggacccactg tgccccttgg aaagcaagga tgtcctggtc gttcatcgga 39420

ccgatttcgg atgcgatttt ctgaaaaacg gcctgggtca cggcgaacat tgtatccaac 39480
tgtgccggaa cagtctgcag aggcaagccg gttacactaa actgctgaac aaagtttggg 39540
accgtctttt cgaagatgga aaccacatag tcttggtagt tagcctgccc aacaattaga 39600
gcaacaacga tggtgaccgt gatcacccga gtgataccgc tacgggtatc gacttcgccg 39660
cgtatgacta aaataccctg aacaataatc caaagagtga cacaggcgat caatggcgca 39720
ctcaccgcct cctggatagt ctcaagcatc gagtccaagc ctgtcgtgaa ggctacatcg 39780
aagatcgtat gaatggccgt aaacggcgcc ggaatcgtga aattcatcga ttggacctga 39840
acttgactgg tttgtcgcat aatgttggat aaaatgagct cgcattcggc gaggatgcgg 39900
gcggatgaac aaatcgccca gccttagggg agggcaccaa agatgacagc ggtcttttga 39960
tgctccttgc gttgagcggc cgcctcttcc gcctcgtgaa ggccggcctg cgcggtagtc 40020
atcgttaata ggcttgtcgc ctgtacattt tgaatcattg cgtcatggat ctgcttgaga 40080
agcaaaccat tggtcacggt tgcctgcatg atattgcgag atcgggaaag ctgagcagac 40140
gtatcagcat tcgccgtcaa gcgtttgtcc atcgtttcca gattgtcagc cgcaatgcca 40200
gcgctgtttg cggaaccggt gatctgcgat cgcaacaggt ccgcttcagc atcactaccc 40260
acgactgcac gatctgtatc gctggtgatc gcacgtgccg tggtcgacat tggcattcgc 40320
ggcgaaaaca tttcattgtc taggtccttc gtcgaaggat actgattttt ctggttgagc 40380
gaagtcagta gtccagtaac gccgtaggcc gacgtcaaca tcgtaaccat cgctatagtc 40440
tgagtgagat tctccgcagt cgcgagcgca gtcgcgagcg tctcagcctc cgttgccggg 40500
tcgctaacaa caaactgcgc ccgcgcgggc tgaatatata gaaagctgca ggtcaaaact 40560
gttgcaataa gttgcgtcgt cttcatcgtt tcctacctta tcaatcttct gcctcgtggt 40620
gacgggccat gaattcgctg agccagccag atgagttgcc ttcttgtgcc tcgcgtagtc 40680
gagttgcaaa gcgcaccgtg ttggcacgcc ccgaaagcac ggcgacatat tcacgcatat 40740
cccgcagatc aaattcgcag atgacgcttc cactttctcg tttaagaaga aacttacggc 40800
tgccgaccgt catgtcttca cggatcgcct gaaattcctt ttcggtacat ttcagtccat 40860
cgacataagc cgatcgatct gcggttggtg atggatagaa aatcttcgtc atacattgcg 40920
caaccaagct ggctcctagc ggcgattcca gaacatgctc tggttgctgc gttgccagta 40980
ttagcatccc gttgtttttt cgaacggtca ggaggaattt gtcgacgaca gtcgaaaatt 41040
tagggtttaa caaataggcg cgaaactcat cgcagctcat cacaaaacgg cggccgtcga 41100
tcatggctcc aatccgatgc aggagatatg ctgcagcggg agcgcatact tcctcgtatt 41160
cgagaagatg cgtcatgtcg aagccggtaa tcgacggatc taactttact tcgtcaactt 41220
cgccgtcaaa tgcccagcca agcgcatggc cccggcacca gcgttggagc cgcgctcctg 41280
cgccttcggc gggcccatgc aacaaaaatt cacgtaaccc cgcgattgaa cgcatttgtg 41340

gatcaaacga gagctgacga tggataccac ggaccagacg gcggttctct tccggagaaa 41400
tcccaccccg accatcactc tcgatgagag ccacgatcca ttcgcgcaga aaatcgtgtg 41460
aggctgctgt gttttctagg ccacgcaacg gcgccaaccc gctgggtgtg cctctgtgaa 41520
gtgccaaata tgttcctcct gtggcgcgaa ccagcaattc gccaccccgg tccttgtcaa 41580
agaacacgac cgtacctgca cggtcgacca tgctctgttc gagcatggct agaacaaaca 41640
tcatgagcgt cgtcttaccc ctcccgatag gcccgaatat tgccgtcatg ccaacatcgt 41700
gctcatgcgg gatatagtcg aaaggcgttc cgccattggt acgaaatcgg gcaatcgcgt 41760
tgccccagtg gcctgagctg gcgccctctg gaaagttttc gaaagagaca aaccctgcga 41820
aattgcgtga agtgattgcg ccagggcgtg tgcgccactt aaaattcccc ggcaattggg 41880
accaataggc cgcttccata ccaatacctt cttggacaac cacggcacct gcatccgcca 41940
ttcgtgtccg agcccgcgcg cccctgtccc caagactatt gagatcgtct gcatagacgc 42000
aaaggctcaa atgatgtgag cccataacga attcgttgct cgcaagtgcg tcctcagcct 42060
cggataattt gccgatttga gtcacggctt tatcgccgga actcagcatc tggctcgatt 42120
tgaggctaag tttcgcgtgc gcttgcgggc gagtcaggaa cgaaaaactc tgcgtgagaa 42180
caagtggaaa atcgagggat agcagcgcgt tgagcatgcc cggccgtgtt tttgcagggt 42240
attcgcgaaa cgaatagatg gatccaacgt aactgtcttt tggcgttctg atctcgagtc 42300
ctcgcttgcc gcaaatgact ctgtcggtat aaatcgaagc gccgagtgag ccgctgacga 42360
ccggaaccgg tgtgaaccga ccagtcatga tcaaccgtag cgcttcgcca atttcggtga 42420
agagcacacc ctgcttctcg cggatgccaa gacgatgcag gccatacgct ttaagagagc 42480
cagcgacaac atgccaaaga tcttccatgt tcctgatctg gcccgtgaga tcgttttccc 42540
tttttccgct tagcttggtg aacctcctct ttaccttccc taaagccgcc tgtgggtaga 42600
caatcaacgt aaggaagtgt tcattgcgga ggagttggcc ggagagcacg cgctgttcaa 42660
aagcttcgtt caggctagcg gcgaaaacac tacggaagtg tcgcggcgcc gatgatggca 42720
cgtcggcatg acgtacgagg tgagcatata ttgacacatg atcatcagcg atattgcgca 42780
acagcgtgtt gaacgcacga caacgcgcat tgcgcatttc agtttcctca agctcgaatg 42840
caacgccatc aattctcgca atggtcatga tcgatccgtc ttcaagaagg acgatatggt 42900
cgctgaggtg gccaatataa gggagataga tctcaccgga tctttcggtc gttccactcg 42960
cgccgagcat cacaccattc ctctccctcg tgggggaacc ctaattggat ttgggctaac 43020
agtagcgccc ccccaaactg cactatcaat gcttcttccc gcggtccgca aaaatagcag 43080
gacgacgctc gccgcattgt agtctcgctc cacgatgagc cgggctgcaa accataacgg 43140
cacgagaacg acttcgtaga gcgggttctg aacgataacg atgacaaagc cggcgaacat 43200
catgaataac cctgccaatg tcagtggcac cccaagaaac aatgcgggcc gtgtggctgc 43260
gaggtaaagg gtcgattctt ccaaacgatc agccatcaac taccgccagt gagcgtttgg 43320

ccgaggaagc tcgccccaaa catgataaca atgccgccga cgacgccggc aaccagccca 43380
agcgaagccc gcccgaacat ccaggagatc ccgatagcga caatgccgag aacagcgagt 43440
gactggccga acggaccaag gataaacgtg catatattgt taaccattgt ggcggggtca 43500
gtgccgccac ccgcagattg cgctgcggcg ggtccggatg aggaaatgct ccatgcaatt 43560
gcaccgcaca agcttggggc gcagctcgat atcacgcgca tcatcgcatt cgagagcgag 43620
aggcgattta gatgtaaacg gtatctctca aagcatcgca tcaatgcgca cctccttagt 43680
ataagtcgaa taagacttga ttgtcgtctg cggatttgcc gttgtcctgg tgtggcggtg 43740
gcggagcgat taaaccgcca gcgccatcct cctgcgagcg gcgctgatat gacccccaaa 43800
catcccacgt ctcttcggat tttagcgcct cgtgatcgtc ttttggaggc tcgattaacg 43860
cgggcaccag cgattgagca gctgtttcaa cttttcgcac gtagccgttt gcaaaaccgc 43920
cgatgaaatt accggtgttg taagcggaga tcgcccgacg aagcgcaaat tgcttctcgt 43980
caatcgtttc gccgcctgca taacgacttt tcagcatgtt tgcagcggca gataatgatg 44040
tgcacgcctg gagcgcaccg tcaggtgtca gaccgagcat agaaaaattt cgagagttta 44100
tttgcatgag gccaacatcc agcgaatgcc gtgcatcgag acggtgcctg acgacttggg 44160
ttgcttggct gtgatcttgc cagtgaagcg tttcgccggt cgtgttgtca tgaatcgcta 44220
aaggatcaaa gcgactctcc accttagcta tcgccgcaag cgtagatgtc gcaactgatg 44280
gggcacactt gcgagcaaca tggtcaaact cagcagatga gagtggcgtg gcaaggctcg 44340
acgaacagaa ggagaccatc aaggcaagag aaagcgaccc cgatctctta agcatacctt 44400
atctccttag ctcgcaacta acaccgcctc tcccgttgga agaagtgcgt tgttttatgt 44460
tgaagattat cgggagggtc ggttactcga aaattttcaa ttgcttcttt atgatttcaa 44520
ttgaagcgag aaacctcgcc cggcgtcttg gaacgcaaca tggaccgaga accgcgcatc 44580
catgactaag caaccggatc gacctattca ggccgcagtt ggtcaggtca ggctcagaac 44640
gaaaatgctc ggcgaggtta cgctgtctgt aaacccattc gatgaacggg aagcttcctt 44700
ccgattgctc ttggcaggaa tattggccca tgcctgcttg cgctttgcaa atgctcttat 44760
cgcgttggta tcatatgcct tgtccgccag cagaaacgca ctctaagcga ttatttgtaa 44820
aaatgtttcg gtcatgcggc ggtcatgggc ttgacccgct gtcagcgcaa gacggatcgg 44880
tcaaccgtcg gcatcgacaa cagcgtgaat cttggtggtc aaaccgccac gggaacgtcc 44940
catacagcca tcgtcttgat cccgctgttt cccgtcgccg catgttggtg gacgcggaca 45000
caggaactgt caatcatgac gacattctat cgaaagcctt ggaaatcaca ctcagaatat 45060
gatcccagac gtctgcctca cgccatcgta caaagcgatt gtagcaggtt gtacaggaac 45120
cgtatcgatc aggaacgtct gcccagggcg ggcccgtccg gaagcgccac aagatgacat 45180
tgatcacccg cgtcaacgcg cggcacgcga cgcggcttat ttgggaacaa aggactgaac 45240

aacagtccat tcgaaatcgg tgacatcaaa gcggggacgg gttatcagtg gcctccaagt 45300
caagcctcaa tgaatcaaaa tcagaccgat ttgcaaacct gatttatgag tgtgcggcct 45360
aaatgatgaa atcgtccttc tagatcgcct ccgtggtgta gcaacacctc gcagtatcgc 45420
cgtgctgacc ttggccaggg aattgactgg caagggtgct ttcacatgac cgctcttttg 45480
gccgcgatag atgatttcgt tgctgctttg ggcacgtaga aggagagaag tcatatcgga 45540
gaaattcctc ctggcgcgag agcctgctct atcgcgacgg catcccactg tcgggaacag 45600
accggatcat tcacgaggcg aaagtcgtca acacatgcgt tataggcatc ttcccttgaa 45660
ggatgatctt gttgctgcca atctggaggt gcggcagccg caggcagatg cgatctcagc 45720
gcaacttgcg gcaaaacatc tcactcacct gaaaaccact agcgagtctc gcgatcagac 45780
gaaggccttt tacttaacga cacaatatcc gatgtctgca tcacaggcgt cgctatccca 45840
gtcaatacta aagcggtgca ggaactaaag attactgatg acttaggcgt gccacgaggc 45900
ctgagacgac gcgcgtagac agtttttga aatcattatc aaagtgatgg cctccgctga 45960
agcctatcac ctctgcgccg gtctgtcgga gagatgggca agcattatta cggtcttcgc 46020
gcccgtacat gcattggacg attgcagggt caatggatct gagatcatcc agaggattgc 46080
cgcccttacc ttccgtttcg agttggagcc agcccctaaa tgagacgaca tagtcgactt 46140
gatgtgacaa tgccaagaga gagatttgct taacccgatt tttttgctca agcgtaagcc 46200
tattgaagct tgccggcatg acgtccgcgc cgaaagaata tcctacaagt aaaacattct 46260
gcacaccgaa atgcttggtg tagacatcga ttatgtgacc aagatcctta gcagtttcgc 46320
ttggggaccg ctccgaccag aaataccgaa gtgaactgac gccaatgaca ggaatccctt 46380
ccgtctgcag ataggtacca tcgatagatc tgctgcctcg cgcgtttcgg tgatgacggt 46440
gaaaacctct gacacatgca gctcccggag acggtcacag cttgtctgta agcggatgcc 46500
gggagcagac aagcccgtca gggcgcgtca gcgggtgttg gcgggtgtcg gggcgcagcc 46560
atgacccagt cacgtagcga tagcggagtg tatactggct taactatgcg gcatcagagc 46620
agattgtact gagagtgcac catatgcggt gtgaaatacc gcacagatgc gtaaggagaa 46680
aataccgcat caggcgctct tccgcttcct cgctcactga ctcgctgcgc tcggtcgttc 46740
ggctgcggcg agcggtatca gctcactcaa aggcggtaat acggttatcc acagaatcag 46800
gggataacgc aggaaagaac atgtgagcaa aaggccagca aaaggccagg aaccgtaaaa 46860
aggccgcgtt gctggcgttt ttccataggc tccgcccccc tgacgagcat cacaaaaatc 46920
gacgctcaag tcagaggtgg cgaaacccga caggactata aagataccag gcgtttcccc 46980
ctggaagctc cctcgtgcgc tctcctgttc cgaccctgcc gcttaccgga tacctgtccg 47040
cctttctccc ttcgggaagc gtggcgcttt ctcatagctc acgctgtagg tatctcagtt 47100
cggtgtaggt cgttcgctcc aagctgggct gtgtgcacga accccccgtt cagcccgacc 47160
gctgcgcctt atccggtaac tatcgtcttg agtccaaccc ggtaagacac gacttatcgc 47220

cactggcagc agccactggt aacaggatta gcagagcgag gtatgtaggc ggtgctacag 47280

agttcttgaa gtggtggcct aactacggct acactagaag gacagtattt ggtatctgcg 47340

ctctgctgaa gccagttacc ttcggaaaaa gagttggtag ctcttgatcc ggcaaacaaa 47400

ccaccgctgg tagcggtggt ttttttgttt gcaagcagca gattacgcgc agaaaaaaag 47460

gatctcaaga agatcctttg atcttttcta cggggtctga cgctcagtgg aacgaaaact 47520

cacgttaagg gattttggtc atgagattat caaaaaggat cttcacctag atccttttaa 47580

attaaaaatg aagttttaaa tcaatctaaa gtatatatga gtaaacttgg tctgacagtt 47640

accaatgctt aatcagtgag gcacctatct cagcgatctg tctatttcgt tcatccatag 47700

ttgcctgact ccccgtcgtg tagataacta cgatacggga gggcttacca tctggcccca 47760

gtgctgcaat gataccgcga gacccacgct caccggctcc agatttatca gcaataaacc 47820

agccagccgg aagggccgag cgcagaagtg gtcctgcaac tttatccgcc tccatccagt 47880

ctattaattg ttgccgggaa gctagagtaa gtagttcgcc agttaatagt ttgcgcaacg 47940

ttgttgccat tgctgcaggg gggggggggg ggggggactt ccattgttca ttccacggac 48000

aaaaacagag aaaggaaacg acagaggcca aaaagcctcg ctttcagcac ctgtcgtttc 48060

ctttcttttc agagggtatt ttaaataaaa acattaagtt atgacgaaga agaacggaaa 48120

cgccttaaac cggaaaattt tcataaatag cgaaaacccg cgaggtcgcc gccccgtagt 48180

cggatcaccg gaaaggaccc gtaaagtgat aatgattatc atctacatat cacaacgtgc 48240

gtggaggcca tcaaaccacg tcaaataatc aattatgacg caggtatcgt attaattgat 48300

ctgcatcaac ttaacgtaaa aacaacttca gacaatacaa atcagcgaca ctgaatacgg 48360

ggcaacctca tgtccccccc cccccccccc ctgcaggcat cgtggtgtca cgctcgtcgt 48420

ttggtatggc ttcattcagc tccggttccc aacgatcaag gcgagttaca tgatccccca 48480

tgttgtgcaa aaaagcggtt agctccttcg gtcctccgat cgttgtcaga agtaagttgg 48540

ccgcagtgtt atcactcatg gttatggcag cactgcataa ttctcttact gtcatgccat 48600

ccgtaagatg cttttctgtg actggtgagt actcaaccaa gtcattctga gaatagtgta 48660

tgcggcgacc gagttgctct tgcccggcgt caacacggga taataccgcg ccacatagca 48720

gaactttaaa agtgctcatc attggaaaac gttcttcggg gcgaaaactc tcaaggatct 48780

taccgctgtt gagatccagt tcgatgtaac ccactcgtgc acccaactga tcttcagcat 48840

cttttacttt caccagcgtt tctgggtgag caaaaacagg aaggcaaaat gccgcaaaaa 48900

agggaataag ggcgacacgg aaatgttgaa tactcatact cttccttttt caatattatt 48960

gaagcattta tcagggttat tgtctcatga gcggatacat atttgaatgt atttagaaaa 49020

ataaacaaat aggggttccg cgcacatttc cccgaaaagt gccacctgac gtctaagaaa 49080

ccattattat catgacatta acctataaaa ataggcgtat cacgaggccc tttcgtcttc 49140

aagaattggt cgacgatctt gctgcgttcg gatattttcg tggagttccc gccacagacc 49200

cggattgaag gcgagatcca gcaactcgcg ccagatcatc ctgtgacgga actttggcgc 49260

gtgatgactg gccaggacgt cggccgaaag agcgacaagc agatcacgct tttcgacagc 49320

gtcggatttg cgatcgagga tttttcggcg ctgcgctacg tccgcgaccg cgttgaggga 49380

tcaagccaca gcagcccact cgaccttcta gccgacccag acgagccaag ggatcttttt 49440

ggaatgctgc tccgtcgtca ggctttccga cgtttgggtg gttgaacaga agtcattatc 49500

gtacggaatg ccaagcactc ccgaggggaa ccctgtggtt ggcatgcaca tacaaatgga 49560

cgaacggata aaccttttca cgccctttta aatatccgtt attctaataa acgctctttt 49620

ctcttaggtt tacccgccaa tatatcctgt caaacactga tagtttaaac tgaaggcggg 49680

aaacgacaat ctgatcatga gcggagaatt aagggagtca cgttatgacc cccgccgatg 49740

acgcgggaca agccgtttta cgtttggaac tgacagaacc gcaacgttga aggagccact 49800

cagcaagctg gtacgattgt aatacgactc actatagggc gaattgagcg ctgtttaaac 49860

gctcttcaac tggaagagcg gttacccgga ccgaagcttg catgcctgca g 49911

<210> 7
<211> 36909
<212> DNA
<213> artificial sequence

<220>
<223> vector

<400> 7
tctagagctc gttcctcgag gcctcgaggc ctcgaggaac ggtacctgcg gggaagctta 60

caataatgtg tgttgttaag tcttgttgcc tgtcatcgtc tgactgactt tcgtcataaa 120

tcccggcctc cgtaacccag ctttgggcaa gctcacggat ttgatccggc ggaacgggaa 180

tatcgagatg ccgggctgaa cgctgcagtt ccagctttcc ctttcgggac aggtactcca 240

gctgattgat tatctgctga agggtcttgg ttccacctcc tggcacaatg cgaatgatta 300

cttgagcgcg atcgggcatc caattttctc ccgtcaggtg cgtggtcaag tgctacaagg 360

cacctttcag taacgagcga ccgtcgatcc gtcgccggga tacggacaaa atggagcgca 420

gtagtccatc gagggcggcg aaagcctcgc caaaagcaat acgttcatct cgcacagcct 480

ccagatccga tcgagggtct tcggcgtagg cagatagaag catggataca ttgcttgaga 540

gtattccgat ggactgaagt atggcttcca tcttttctcg tgtgtctgca tctatttcga 600

gaaagccccc gatgcggcgc accgcaacgc gaattgccat actatccgaa agtcccagca 660

ggcgcgcttg ataggaaaag gtttcatact cggccgatcg cagacgggca ctcacgacct 720

tgaacccttc aactttcagg gatcgatgct ggttgatggt agtctcactc gacgtggctc 780

tggtgtgttt tgacatagct tcctccaaag aaagcggaag gtctggatac tccagcacga 840

aatgtgcccg ggtagacgga tggaagtcta gccctgctca atatgaaatc aacagtacat 900

ttacagtcaa tactgaatat acttgctaca tttgcaattg tcttataacg aatgtgaaat 960
aaaaatagtg taacaacgct tttactcatc gataatcaca aaaacattta tacgaacaaa 1020
aatacaaatg cactccggtt tcacaggata ggcgggatca gaatatgcaa cttttgacgt 1080
tttgttcttt caaagggggt gctggcaaaa ccaccgcact catgggcctt tgcgctgctt 1140
tggcaaatga cggtaaacga gtggccctct ttgatgccga cgaaaaccgg cctctgacgc 1200
gatggagaga aaacgcctta caaagcagta ctgggatcct cgctgtgaag tctattccgc 1260
cgacgaaatg ccccttcttg aagcagccta tgaaaatgcc gagctcgaag gatttgatta 1320
tgcgttggcc gatacgcgtg gcggctcgag cgagctcaac aacacaatca tcgctagctc 1380
aaacctgctt ctgatcccca ccatgctaac gccgctcgac atcgatgagg cactatctac 1440
ctaccgctac gtcatcgagc tgctgttgag tgaaaatttg gcaattccta cagctgtttt 1500
gcgccaacgc gtcccggtcg gccgattgac aacatcgcaa cgcaggatgt cagagacgct 1560
agagagcctt ccagttgtac cgtctcccat gcatgaaaga gatgcatttg ccgcgatgaa 1620
agaacgcggc atgttgcatc ttacattact aaacacggga actgatccga cgatgcgcct 1680
catagagagg aatcttcgga ttgcgatgga ggaagtcgtg gtcatttcga aactgatcag 1740
caaaatcttg gaggcttgaa gatggcaatt cgcaagcccg cattgtcggt cggcgaagca 1800
cggcggcttg ctggtgctcg acccgagatc caccatccca acccgacact tgttccccag 1860
aagctggacc tccagcactt gcctgaaaaa gccgacgaga aagaccagca acgtgagcct 1920
ctcgtcgccg atcacattta cagtcccgat cgacaactta agctaactgt ggatgccctt 1980
agtccacctc cgtccccgaa aaagctccag gtttttcttt cagcgcgacc gcccgcgcct 2040
caagtgtcga aaacatatga caacctcgtt cggcaataca gtccctcgaa gtcgctacaa 2100
atgattttaa ggcgcgcgtt ggacgatttc gaaagcatgc tggcagatgg atcatttcgc 2160
gtggccccga aaagttatcc gatcccttca actacagaaa aatccgttct cgttcagacc 2220
tcacgcatgt tcccggttgc gttgctcgag gtcgctcgaa gtcattttga tccgttgggg 2280
ttggagaccg ctcgagcttt cggccacaag ctggctaccg ccgcgctcgc gtcattcttt 2340
gctggagaga agccatcgag caattggtga agagggacct atcggaaccc ctcaccaaat 2400
attgagtgta ggtttgaggc cgctggccgc gtcctcagtc acctttttgag ccagataatt 2460
aagagccaaa tgcaattggc tcaggctgcc atcgtccccc cgtgcgaaac ctgcacgtcc 2520
gcgtcaaaga aataaccggc acctcttgct gtttttatca gttgagggct tgacggatcc 2580
gcctcaagtt tgcggcgcag ccgcaaaatg agaacatcta tactcctgtc gtaaacctcc 2640
tcgtcgcgta ctcgactggc aatgagaagt tgctcgcgcg atagaacgtc gcggggtttc 2700
tctaaaaacg cgaggagaag attgaactca cctgccgtaa gtttcacctc accgccagct 2760
tcggacatca agcgacgttg cctgagatta agtgtccagt cagtaaaaca aaaagaccgt 2820

cggtctttgg agcggacaac gttggggcgc acgcgcaagg caaccgaat gcgtgcaaga 2880
aactctctcg tactaaacgg cttagcgata aaatcacttg ctcctagctc gagtgcaaca 2940
actttatccg tctcctcaag gcggtcgcca ctgataatta tgattggaat atcagacttt 3000
gccgccagat ttcgaacgat ctcaagccca tcttcacgac ctaaatttag atcaacaacc 3060
acgacatcga ccgtcgcgga agagagtact ctagtgaact gggtgctgtc ggctaccgcg 3120
gtcactttga aggcgtggat cgtaaggtat tcgataataa gatgccgcat agcgacatcg 3180
tcatcgataa gaagaacgtg tttcaacggc tcacctttca atctaaaatc tgaacccttg 3240
ttcacagcgc ttgagaaatt ttcacgtgaa ggatgtacaa tcatctccag ctaaatgggc 3300
agttcgtcag aattgcggct gaccgcggat gacgaaaatg cgaaccaagt atttcaattt 3360
tatgacaaaa gttctcaatc gttgttacaa gtgaaacgct tcgaggttac agctactatt 3420
gattaaggag atcgcctatg gtctcgcccc ggcgtcgtgc gtccgccgcg agccagatct 3480
cgcctacttc ataaacgtcc tcataggcac ggaatggaat gatgacatcg atcgccgtag 3540
agagcatgtc aatcagtgtg cgatcttcca agctagcacc ttgggcgcta cttttgacaa 3600
gggaaaacag tttcttgaat ccttggattg gattcgcgcc gtgtattgtt gaaatcgatc 3660
ccggatgtcc cgagacgact tcactcagat aagcccatgc tgcatcgtcg cgcatctcgc 3720
caagcaatat ccggtccggc cgcatacgca gacttgcttg gagcaagtgc tcggcgctca 3780
cagcacccag cccagcaccg ttcttggagt agagtagtct aacatgatta tcgtgtggaa 3840
tgacgagttc gagcgtatct tctatggtga ttagcctttc ctgggggggg atggcgctga 3900
tcaaggtctt gctcattgtt gtcttgccgc ttccggtagg gccacatagc aacatcgtca 3960
gtcggctgac gacgcatgcg tgcagaaacg cttccaaatc cccgttgtca aaatgctgaa 4020
ggatagcttc atcatcctga ttttggcgtt tccttcgtgt ctgccactgg ttccacctcg 4080
aagcatcata acgggaggag acttctttaa gaccagaaac acgcgagctt ggccgtcgaa 4140
tggtcaagct gacggtgccc gagggaacgg tcggcggcag acagatttgt agtcgttcac 4200
caccaggaag ttcagtggcg cagagggggt tacgtggtcc gacatcctgc tttctcagcg 4260
cgcccgctaa aatagcgata tcttcaagat catcataaga gacgggcaaa ggcatcttgg 4320
taaaaatgcc ggcttggcgc acaaatgcct ctccaggtcg attgatcgca atttcttcag 4380
tcttcgggtc atcgagccat tccaaaatcg gcttcagaag aaagcgtagt tgcggatcca 4440
cttccattta caatgtatcc tatctctaag cggaaatttg aattcattaa gagcggcggt 4500
tcctcccccg cgtggcgccg ccagtcaggc ggagctggta aacaccaaag aaatcgaggt 4560
cccgtgctac gaaaatggaa acggtgtcac cctgattctt cttcagggtt ggcggtatgt 4620
tgatggttgc cttaagggct gtctcagttg tctgctcacc gttattttga aagctgttga 4680
agctcatccc gccacccgag ctgccggcgt aggtgctagc tgcctggaag gcgccttgaa 4740
caacactcaa gagcatagct ccgctaaaac gctgccagaa gtggctgtcg accgagcccg 4800

gcaatcctga gcgaccgagt tcgtccgcgc ttggcgatgt taacgagatc atcgcatggt 4860
caggtgtctc ggcgcgatcc cacaacacaa aaacgcgccc atctccctgt tgcaagccac 4920
gctgtatttc gccaacaacg gtggtgccac gatcaagaag cacgatattg ttcgttgttc 4980
cacgaatatc ctgaggcaag acacacttta catagcctgc caaatttgtg tcgattgcgg 5040
tttgcaagat gcacggaatt attgtccctt gcgttaccat aaaatcgggg tgcggcaaga 5100
gcgtggcgct gctgggctgc agctcggtgg gtttcatacg tatcgacaaa tcgttctcgc 5160
cggacacttc gccattcggc aaggagttgt cgtcacgctt gccttcttgt cttcggcccg 5220
tgtcgccctg aatggcgcgt ttgctgaccc cttgatcgcc gctgctatat gcaaaaatcg 5280
gtgtttcttc cggccgtggc tcatgccgct ccggttcgcc cctcggcggt agaggagcag 5340
caggctgaac agcctcttga accgctggag gatccggcgg cacctcaatc ggagctggat 5400
gaaatggctt ggtgtttgtt gcgatcaaag ttgacggcga tgcgttctca ttcaccttct 5460
tttggcgccc acctagccaa atgaggctta atgataacgc gagaacgaca cctccgacga 5520
tcaatttctg agaccccgaa agacgccggc gatgtttgtc ggagaccagg gatccagatg 5580
catcaacctc atgtgccgct tgctgactat cgttattcat cccttcgccc ccttcaggac 5640
gcgtttcaca tcgggcctca ccgtgcccgt ttgcggcctt tggccaacgg gatcgtaagc 5700
ggtgttccag atacatagta ctgtgtggcc atccctcaga cgccaacctc gggaaaccga 5760
agaaatctcg acatcgctcc ctttaactga atagttggca acagcttcct tgccatcagg 5820
attgatggtg tagatggagg gtatgcgtac attgcccgga aagtggaata ccgtcgtaaa 5880
tccattgtcg aagacttcga gtggcaacag cgaacgatcg ccttgggcga cgtagtgcca 5940
attactgtcc gccgcaccaa gggctgtgac aggctgatcc aataaattct cagctttccg 6000
ttgatattgt gcttccgcgt gtagtctgtc cacaacagcc ttctgttgtg cctcccttcg 6060
ccgagccgcc gcatcgtcgg cggggtaggc gaattggacg ctgtaataga gatcgggctg 6120
ctctttatcg aggtgggaca gagtcttgga acttatactg aaaacataac ggcgcatccc 6180
ggagtcgctt gcggttagca cgattactgg ctgaggcgtg aggacctggc ttgccttgaa 6240
aaatagataa tttccccgcg gtagggctgc tagatctttg ctatttgaaa cggcaaccgc 6300
tgtcaccgtt tcgttcgtgg cgaatgttac gaccaaagta gctccaaccg ccgtcgagag 6360
gcgcaccact tgatcgggat tgtaagccaa ataacgcatg cgcggatcta gcttgcccgc 6420
cattggagtg tcttcagcct ccgcaccagt cgcagcggca aataaacatg ctaaaatgaa 6480
aagtgctttt ctgatcatgg ttcgctgtgg cctacgtttg aaacggtatc ttccgatgtc 6540
tgataggagg tgacaaccag acctgccggg ttggttagtc tcaatctgcc gggcaagctg 6600
gtcacctttt cgtagcgaac tgtcgcggtc cacgtactca ccacaggcat tttgccgtca 6660
acgacgaggg tccttttata gcgaatttgc tgcgtgcttg gagttacatc atttgaagcg 6720

atgtgctcga cctccaccct gccgcgtttg ccaagaatga cttgaggcga actgggattg 6780

ggatagttga agaattgctg gtaatcctgg cgcactgttg gggcactgaa gttcgatacc 6840

aggtcgtagg cgtactgagc ggtgtcggca tcataactct cgcgcaggcg aacgtactcc 6900

cacaatgagg cgttaacgac ggcctcctct tgagttgcag gcaatcgcga gacagacacc 6960

tcgctgtcaa cggtgccgtc cggccgtatc catagatata cgggcacaag cctgctcaac 7020

ggcaccattg tggctatagc gaacgcttga gcaacatttc ccaaaatcgc gatagctgcg 7080

acagctgcaa tgagtttgga gagacgtcgc gccgatttcg ctcgcgcggt ttgaaaggct 7140

tctacttcct tatagtgctc ggcaaggctt tcgcgcgcca ctagcatggc atattcaggc 7200

cccgtcatag cgtccacccg aattgccgag ctgaagatct gacggagtag gctgccatcg 7260

ccccacattc agcgggaaga tcgggccttt gcagctcgct aatgtgtcgt ttgtctggca 7320

gccgctcaaa gcgacaacta ggcacagcag gcaatacttc atagaattct ccattgaggc 7380

gaatttttgc gcgacctagc ctcgctcaac ctgagcgaag cgacggtaca agctgctggc 7440

agattgggtt gcgccgctcc agtaactgcc tccaatgttg ccggcgatcg ccggcaaagc 7500

gacaatgagc gcatcccctg tcagaaaaaa catatcgagt tcgtaaagac caatgatctt 7560

ggccgcggtc gtaccggcga aggtgattac accaagcata agggtgagcg cagtcgcttc 7620

ggttaggatg acgatcgttg ccacgaggtt taagaggaga agcaagagac cgtaggtgat 7680

aagttgcccg atccacttag ctgcgatgtc ccgcgtgcga tcaaaaatat atccgacgag 7740

gatcagaggc ccgatcgcga gaagcacttt cgtgagaatt ccaacggcgt cgtaaactcc 7800

gaaggcagac cagagcgtgc cgtaaaggac ccactgtgcc ccttggaaag caaggatgtc 7860

ctggtcgttc atcggaccga tttcggatgc gattttctga aaaacggcct gggtcacggc 7920

gaacattgta tccaactgtg ccggaacagt ctgcagaggc aagccggtta cactaaactg 7980

ctgaacaaag tttgggaccg tcttttcgaa gatggaaacc acatagtctt ggtagttagc 8040

ctgcccaaca attagagcaa caacgatggt gaccgtgatc acccgagtga taccgctacg 8100

ggtatcgact tcgccgcgta tgactaaaat accctgaaca ataatccaaa gagtgacaca 8160

ggcgatcaat ggcgcactca ccgcctcctg gatagtctca agcatcgagt ccaagcctgt 8220

cgtgaaggct acatcgaaga tcgtatgaat ggccgtaaac ggcgccggaa tcgtgaaatt 8280

catcgattgg acctgaactt gactggtttg tcgcataatg ttggataaaa tgagctcgca 8340

ttcggcgagg atgcgggcgg atgaacaaat cgcccagcct taggggaggg caccaaagat 8400

gacagcggtc ttttgatgct ccttgcgttg agcggccgcc tcttccgcct cgtgaaggcc 8460

ggcctgcgcg gtagtcatcg ttaataggct tgtcgcctgt acattttgaa tcattgcgtc 8520

atggatctgc ttgagaagca aaccattggt cacggttgcc tgcatgatat tgcgagatcg 8580

ggaaagctga gcagacgtat cagcattcgc cgtcaagcgt ttgtccatcg tttccagatt 8640

gtcagccgca atgccagcgc tgtttgcgga accggtgatc tgcgatcgca acaggtccgc 8700

ttcagcatca ctacccacga ctgcacgatc tgtatcgctg gtgatcgcac gtgccgtggt 8760
cgacattggc attcgcggcg aaaacatttc attgtctagg tccttcgtcg aaggatactg 8820
atttttctgg ttgagcgaag tcagtagtcc agtaacgccg taggccgacg tcaacatcgt 8880
aaccatcgct atagtctgag tgagattctc cgcagtcgcg agcgcagtcg cgagcgtctc 8940
agcctccgtt gccgggtcgc taacaacaaa ctgcgcccgc gcgggctgaa tatatagaaa 9000
gctgcaggtc aaaactgttg caataagttg cgtcgtcttc atcgtttcct accttatcaa 9060
tcttctgcct cgtggtgacg ggccatgaat tcgctgagcc agccagatga gttgccttct 9120
tgtgcctcgc gtagtcgagt tgcaaagcgc accgtgttgg cacgccccga aagcacggcg 9180
acatattcac gcatatcccg cagatcaaat tcgcagatga cgcttccact ttctcgttta 9240
agaagaaact tacggctgcc gaccgtcatg tcttcacgga tcgcctgaaa ttccttttcg 9300
gtacatttca gtccatcgac ataagccgat cgatctgcgg ttggtgatgg atagaaaatc 9360
ttcgtcatac attgcgcaac caagctggct cctagcggcg attccagaac atgctctggt 9420
tgctgcgttg ccagtattag catcccgttg ttttttcgaa cggtcaggag gaatttgtcg 9480
acgacagtcg aaaatttagg gtttaacaaa taggcgcgaa actcatcgca gctcatcaca 9540
aaacggcggc cgtcgatcat ggctccaatc cgatgcagga gatatgctgc agcgggagcg 9600
catacttcct cgtattcgag aagatgcgtc atgtcgaagc cggtaatcga cggatctaac 9660
tttacttcgt caacttcgcc gtcaaatgcc cagccaagcg catggccccg gcaccagcgt 9720
tggagccgcg ctcctgcgcc ttcggcgggc ccatgcaaca aaaattcacg taaccccgcg 9780
attgaacgca tttgtggatc aaacgagagc tgacgatgga taccacggac cagacggcgg 9840
ttctcttccg gagaaatccc accccgacca tcactctcga tgagagccac gatccattcg 9900
cgcagaaaat cgtgtgaggc tgctgtgttt tctaggccac gcaacggcgc caacccgctg 9960
ggtgtgcctc tgtgaagtgc caaatatgtt cctcctgtgg cgcgaaccag caattcgcca 10020
ccccggtcct tgtcaaagaa cacgaccgta cctgcacggt cgaccatgct ctgttcgagc 10080
atggctagaa caaacatcat gagcgtcgtc ttacccctcc cgataggccc gaatattgcc 10140
gtcatgccaa catcgtgctc atgcgggata tagtcgaaag gcgttccgcc attggtacga 10200
aatcgggcaa tcgcgttgcc ccagtggcct gagctggcgc cctctggaaa gttttcgaaa 10260
gagacaaacc ctgcgaaatt gcgtgaagtg attgcgccag ggcgtgtgcg ccacttaaaa 10320
ttccccggca attgggacca ataggccgct tccataccaa taccttcttg gacaaccacg 10380
gcacctgcat ccgccattcg tgtccgagcc cgcgcgcccc tgtccccaag actattgaga 10440
tcgtctgcat agacgcaaag gctcaaatga tgtgagccca taacgaattc gttgctcgca 10500
agtgcgtcct cagcctcgga taatttgccg atttgagtca cggctttatc gccggaactc 10560
agcatctggc tcgatttgag gctaagtttc gcgtgcgctt gcgggcgagt caggaacgaa 10620

aaactctgcg tgagaacaag tggaaaatcg agggatagca gcgcgttgag catgcccggc 10680
cgtgtttttg cagggtattc gcgaaacgaa tagatggatc caacgtaact gtcttttggc 10740
gttctgatct cgagtcctcg cttgccgcaa atgactctgt cggtataaat cgaagcgccg 10800
agtgagccgc tgacgaccgg aaccggtgtg aaccgaccag tcatgatcaa ccgtagcgct 10860
tcgccaattt cggtgaagag cacaccctgc ttctcgcgga tgccaagacg atgcaggcca 10920
tacgctttaa gagagccagc gacaacatgc caaagatctt ccatgttcct gatctggccc 10980
gtgagatcgt tttccctttt tccgcttagc ttggtgaacc tcctctttac cttccctaaa 11040
gccgcctgtg ggtagacaat caacgtaagg aagtgttcat tgcggaggag ttggccggag 11100
agcacgcgct gttcaaaagc ttcgttcagg ctagcggcga aaacactacg gaagtgtcgc 11160
ggcgccgatg atggcacgtc ggcatgacgt acgaggtgag catatattga cacatgatca 11220
tcagcgatat tgcgcaacag cgtgttgaac gcacgacaac gcgcattgcg catttcagtt 11280
tcctcaagct cgaatgcaac gccatcaatt ctcgcaatgg tcatgatcga tccgtcttca 11340
agaaggacga tatggtcgct gaggtggcca atataaggga gatagatctc accggatctt 11400
tcggtcgttc cactcgcgcc gagcatcaca ccattcctct ccctcgtggg ggaaccctaa 11460
ttggatttgg gctaacagta gcgccccccc aaactgcact atcaatgctt cttcccgcgg 11520
tccgcaaaaa tagcaggacg acgctcgccg cattgtagtc tcgctccacg atgagccggg 11580
ctgcaaacca taacggcacg agaacgactt cgtagagcgg gttctgaacg ataacgatga 11640
caaagccggc gaacatcatg aataaccctg ccaatgtcag tggcacccca agaaacaatg 11700
cgggccgtgt ggctgcgagg taaagggtcg attcttccaa acgatcagcc atcaactacc 11760
gccagtgagc gtttggccga ggaagctcgc cccaaacatg ataacaatgc cgccgacgac 11820
gccggcaacc agcccaagcg aagcccgccc gaacatccag gagatcccga tagcgacaat 11880
gccgagaaca gcgagtgact ggccgaacgg accaaggata aacgtgcata tattgttaac 11940
cattgtggcg gggtcagtgc cgccacccgc agattgcgct gcggcgggtc cggatgagga 12000
aatgctccat gcaattgcac cgcacaagct tggggcgcag ctcgatatca cgcgcatcat 12060
cgcattcgag agcgagaggc gatttagatg taaacggtat ctctcaaagc atcgcatcaa 12120
tgcgcacctc cttagtataa gtcgaataag acttgattgt cgtctgcgga tttgccgttg 12180
tcctggtgtg gcggtggcgg agcgattaaa ccgccagcgc catcctcctg cgagcggcgc 12240
tgatatgacc cccaaacatc ccacgtctct tcggatttta gcgcctcgtg atcgtctttt 12300
ggaggctcga ttaacgcggg caccagcgat tgagcagctg tttcaacttt tcgcacgtag 12360
ccgtttgcaa aaccgccgat gaaattaccg gtgttgtaag cggagatcgc ccgacgaagc 12420
gcaaattgct tctcgtcaat cgtttcgccg cctgcataac gacttttcag catgtttgca 12480
gcggcagata atgatgtgca cgcctggagc gcaccgtcag gtgtcagacc gagcatagaa 12540
aaatttcgag agtttatttg catgaggcca acatccagcg aatgccgtgc atcgagacgg 12600

tgcctgacga cttgggttgc ttggctgtga tcttgccagt gaagcgtttc gccggtcgtg 12660
ttgtcatgaa tcgctaaagg atcaaagcga ctctccacct tagctatcgc cgcaagcgta 12720
gatgtcgcaa ctgatggggc acacttgcga gcaacatggt caaactcagc agatgagagt 12780
ggcgtggcaa ggctcgacga acagaaggag accatcaagg caagagaaag cgaccccgat 12840
ctcttaagca taccttatct ccttagctcg caactaacac cgcctctccc gttggaagaa 12900
gtgcgttgtt ttatgttgaa gattatcggg agggtcggtt actcgaaaat tttcaattgc 12960
ttctttatga tttcaattga agcgagaaac ctcgcccggc gtcttggaac gcaacatgga 13020
ccgagaaccg cgcatccatg actaagcaac cggatcgacc tattcaggcc gcagttggtc 13080
aggtcaggct cagaacgaaa atgctcggcg aggttacgct gtctgtaaac ccattcgatg 13140
aacgggaagc ttccttccga ttgctcttgg caggaatatt ggcccatgcc tgcttgcgct 13200
ttgcaaatgc tcttatcgcg ttggtatcat atgccttgtc cgccagcaga aacgcactct 13260
aagcgattat ttgtaaaaat gtttcggtca tgcggcggtc atgggcttga cccgctgtca 13320
gcgcaagacg gatcggtcaa ccgtcggcat cgacaacagc gtgaatcttg gtggtcaaac 13380
cgccacggga acgtcccata cagccatcgt cttgatcccg ctgtttcccg tcgccgcatg 13440
ttggtggacg cggacacagg aactgtcaat catgacgaca ttctatcgaa agccttggaa 13500
atcacactca gaatatgatc ccagacgtct gcctcacgcc atcgtacaaa gcgattgtag 13560
caggttgtac aggaaccgta tcgatcagga acgtctgccc agggcgggcc cgtccggaag 13620
cgccacaaga tgacattgat cacccgcgtc aacgcgcggc acgcgacgcg gcttatttgg 13680
gaacaaagga ctgaacaaca gtccattcga aatcggtgac atcaaagcgg ggacgggtta 13740
tcagtggcct ccaagtcaag cctcaatgaa tcaaaatcag accgatttgc aaacctgatt 13800
tatgagtgtg cggcctaaat gatgaaatcg tccttctaga tcgcctccgt ggtgtagcaa 13860
cacctcgcag tatcgccgtg ctgaccttgg ccagggaatt gactggcaag ggtgctttca 13920
catgaccgct cttttggccg cgatagatga tttcgttgct gctttgggca cgtagaagga 13980
gagaagtcat atcggagaaa ttcctcctgg cgcgagagcc tgctctatcg cgacggcatc 14040
ccactgtcgg gaacagaccg gatcattcac gaggcgaaag tcgtcaacac atgcgttata 14100
ggcatcttcc cttgaaggat gatcttgttg ctgccaatct ggaggtgcgg cagccgcagg 14160
cagatgcgat ctcagcgcaa cttgcggcaa aacatctcac tcacctgaaa accactagcg 14220
agtctcgcga tcagacgaag gccttttact taacgacaca atatccgatg tctgcatcac 14280
aggcgtcgct atcccagtca atactaaagc ggtgcaggaa ctaaagatta ctgatgactt 14340
aggcgtgcca cgaggcctga gacgacgcgc gtagacagtt ttttgaaatc attatcaaag 14400
tgatggcctc cgctgaagcc tatcacctct gcgccggtct gtcggagaga tgggcaagca 14460
ttattacggt cttcgcgccc gtacatgcat tggacgattg cagggtcaat ggatctgaga 14520

tcatccagag gattgccgcc cttaccttcc gtttcgagtt ggagccagcc cctaaatgag 14580
acgacatagt cgacttgatg tgacaatgcc aagagagaga tttgcttaac ccgatttttt 14640
tgctcaagcg taagcctatt gaagcttgcc ggcatgacgt ccgcgccgaa agaatatcct 14700
acaagtaaaa cattctgcac accgaaatgc ttggtgtaga catcgattat gtgaccaaga 14760
tccttagcag tttcgcttgg ggaccgctcc gaccagaaat accgaagtga actgacgcca 14820
atgacaggaa tcccttccgt ctgcagatag gtaccatcga tagatctgct gcctcgcgcg 14880
tttcggtgat gacggtgaaa acctctgaca catgcagctc ccggagacgg tcacagcttg 14940
tctgtaagcg gatgccggga gcagacaagc ccgtcagggc gcgtcagcgg gtgttggcgg 15000
gtgtcggggc gcagccatga cccagtcacg tagcgatagc ggagtgtata ctggcttaac 15060
tatgcggcat cagagcagat tgtactgaga gtgcaccata tgcggtgtga aataccgcac 15120
agatgcgtaa ggagaaaata ccgcatcagg cgctcttccg cttcctcgct cactgactcg 15180
ctgcgctcgg tcgttcggct gcggcgagcg gtatcagctc actcaaaggc ggtaatacgg 15240
ttatccacag aatcagggga taacgcagga aagaacatgt gagcaaaagg ccagcaaaag 15300
gccaggaacc gtaaaaaggc cgcgttgctg gcgtttttcc ataggctccg cccccctgac 15360
gagcatcaca aaaatcgacg ctcaagtcag aggtggcgaa acccgacagg actataaaga 15420
taccaggcgt ttccccctgg aagctccctc gtgcgctctc ctgttccgac cctgccgctt 15480
accggatacc tgtccgcctt tctcccttcg ggaagcgtgg cgctttctca tagctcacgc 15540
tgtaggtatc tcagttcggt gtaggtcgtt cgctccaagc tgggctgtgt gcacgaaccc 15600
cccgttcagc ccgaccgctg cgccttatcc ggtaactatc gtcttgagtc caacccggta 15660
agacacgact tatcgccact ggcagcagcc actggtaaca ggattagcag agcgaggtat 15720
gtaggcggtg ctacagagtt cttgaagtgg tggcctaact acggctacac tagaaggaca 15780
gtatttggta tctgcgctct gctgaagcca gttaccttcg gaaaaagagt tggtagctct 15840
tgatccggca aacaaaccac cgctggtagc ggtggttttt ttgtttgcaa gcagcagatt 15900
acgcgcagaa aaaaaggatc tcaagaagat cctttgatct tttctacggg gtctgacgct 15960
cagtggaacg aaaactcacg ttaagggatt ttggtcatga gattatcaaa aaggatcttc 16020
acctagatcc ttttaaatta aaaatgaagt tttaaatcaa tctaaagtat atatgagtaa 16080
acttggtctg acagttacca atgcttaatc agtgaggcac ctatctcagc gatctgtcta 16140
tttcgttcat ccatagttgc ctgactcccc gtcgtgtaga taactacgat acgggagggc 16200
ttaccatctg gccccagtgc tgcaatgata ccgcgagacc cacgctcacc ggctccagat 16260
ttatcagcaa taaaccagcc agccggaagg gccgagcgca gaagtggtcc tgcaacttta 16320
tccgcctcca tccagtctat taattgttgc cgggaagcta gagtaagtag ttcgccagtt 16380
aatagtttgc gcaacgttgt tgccattgct gcaggggggg gggggggggg gttccattgt 16440
tcattccacg gacaaaaaca gagaaaggaa acgacagagg ccaaaaagct cgctttcagc 16500

acctgtcgtt tcctttcttt tcagagggta ttttaaataa aaacattaag ttatgacgaa 16560
gaagaacgga aacgccttaa accggaaaat tttcataaat agcgaaaacc cgcgaggtcg 16620
ccgccccgta acctgtcgga tcaccggaaa ggacccgtaa agtgataatg attatcatct 16680
acatatcaca acgtgcgtgg aggccatcaa accacgtcaa ataatcaatt atgacgcagg 16740
tatcgtatta attgatctgc atcaacttaa cgtaaaaaca acttcagaca atacaaatca 16800
gcgacactga atacggggca acctcatgtc cccccccccc cccccctgc aggcatcgtg 16860
gtgtcacgct cgtcgtttgg tatggcttca ttcagctccg gttcccaacg atcaaggcga 16920
gttacatgat cccccatgtt gtgcaaaaaa gcggttagct ccttcggtcc tccgatcgtt 16980
gtcagaagta agttggccgc agtgttatca ctcatggtta tggcagcact gcataattct 17040
cttactgtca tgccatccgt aagatgcttt tctgtgactg gtgagtactc aaccaagtca 17100
ttctgagaat agtgtatgcg gcgaccgagt tgctcttgcc cggcgtcaac acgggataat 17160
accgcgccac atagcagaac tttaaaagtg ctcatcattg gaaaacgttc ttcggggcga 17220
aaactctcaa ggatcttacc gctgttgaga tccagttcga tgtaacccac tcgtgcaccc 17280
aactgatctt cagcatcttt tactttcacc agcgtttctg ggtgagcaaa aacaggaagg 17340
caaaatgccg caaaaaaggg aataagggcg acacggaaat gttgaatact catactcttc 17400
ctttttcaat attattgaag catttatcag ggttattgtc tcatgagcgg atacatattt 17460
gaatgtattt agaaaaataa acaaataggg gttccgcgca catttccccg aaaagtgcca 17520
cctgacgtct aagaaaccat tattatcatg acattaacct ataaaaatag gcgtatcacg 17580
aggccctttc gtcttcaaga attcggagct tttgccattc tcaccggatt cagtcgtcac 17640
tcatggtgat ttctcacttg ataaccttat ttttgacgag gggaaattaa taggttgtat 17700
tgatgttgga cgagtcggaa tcgcagaccg ataccaggat cttgccatcc tatggaactg 17760
cctcggtgag ttttctcctt cattacagaa acggcttttt caaaaatatg gtattgataa 17820
tcctgatatg aataaattgc agtttcattt gatgctcgat gagtttttct aatcagaatt 17880
ggttaattgg ttgtaacact ggcagagcat tacgctgact tgacgggacg gcggctttgt 17940
tgaataaatc gaacttttgc tgagttgaag gatcagatca cgcatcttcc cgacaacgca 18000
gaccgttccg tggcaaagca aaagttcaaa atcaccaact ggtccaccta caacaaagct 18060
ctcatcaacc gtggctccct cactttctgg ctggatgatg gggcgattca ggcctggtat 18120
gagtcagcaa caccttcttc acgaggcaga cctcagcgcc agaaggccgc cagagaggcc 18180
gagcgcggcc gtgaggcttg gacgctaggg cagggcatga aaaagcccgt agcgggctgc 18240
tacgggcgtc tgacgcggtg gaaagggga ggggatgttg tctacatggc tctgctgtag 18300
tgagtgggtt gcgctccggc agcggtcctg atcaatcgtc accctttctc ggtccttcaa 18360
cgttcctgac aacgagcctc cttttcgcca atccatcgac aatcaccgcg agtccctgct 18420

cgaacgctgc gtccggaccg gcttcgtcga aggcgtctat cgcggcccgc aacagcggcg 18480
agagcggagc ctgttcaacg gtgccgccgc gctcgccggc atcgctgtcg ccggcctgct 18540
cctcaagcac ggccccaaca gtgaagtagc tgattgtcat cagcgcattg acggcgtccc 18600
cggccgaaaa acccgcctcg cagaggaagc gaagctgcgc gtcggccgtt tccatctgcg 18660
gtgcgcccgg tcgcgtgccg gcatggatgc gcgcgccatc gcggtaggcg agcagcgcct 18720
gcctgaagct gcgggcattc ccgatcagaa atgagcgcca gtcgtcgtcg gctctcggca 18780
ccgaatgcgt atgattctcc gccagcatgg cttcggccag tgcgtcgagc agcgcccgct 18840
tgttcctgaa gtgccagtaa agcgccggct gctgaacccc caaccgttcc gccagtttgc 18900
gtgtcgtcag accgtctacg ccgacctcgt tcaacaggtc cagggcggca cggatcactg 18960
tattcggctg caactttgtc atgcttgaca ctttatcact gataaacata atatgtccac 19020
caacttatca gtgataaaga atccgcgcgt tcaatcggac cagcggaggc tggtccggag 19080
gccagacgtg aaacccaaca tacccctgat cgtaattctg agcactgtcg cgctcgacgc 19140
tgtcggcatc ggcctgatta tgccggtgct gccgggcctc ctgcgcgatc tggttcactc 19200
gaacgacgtc accgcccact atggcattct gctggcgctg tatgcgttgg tgcaatttgc 19260
ctgcgcacct gtgctgggcg cgctgtcgga tcgtttcggg cggcggccaa tcttgctcgt 19320
ctcgctggcc ggcgccactg tcgactacgc catcatggcg acagcgcctt tcctttgggt 19380
tctctatatc gggcggatcg tggccggcat caccggggcg actggggcgg tagccggcgc 19440
ttatattgcc gatatcactg atggcgatga gcgcgcgcgg cacttcggct tcatgagcgc 19500
ctgtttcggg ttcgggatgg tcgcgggacc tgtgctcggt gggctgatgg gcggtttctc 19560
cccccacgct ccgttcttcg ccgcggcagc cttgaacggc ctcaatttcc tgacgggctg 19620
tttccttttg ccggagtcgc acaaaggcga acgccggccg ttacgccggg aggctctcaa 19680
cccgctcgct tcgttccggt gggcccgggg catgaccgtc gtcgccgccc tgatggcggt 19740
cttcttcatc atgcaacttg tcggacaggt gccggccgcg ctttgggtca ttttcggcga 19800
ggatcgcttt cactgggacg cgaccacgat cggcatttcg cttgccgcat ttggcattct 19860
gcattcactc gcccaggcaa tgatcaccgg ccctgtagcc gcccggctcg gcgaaaggcg 19920
ggcactcatg ctcggaatga ttgccgacgg cacaggctac atcctgcttg ccttcgcgac 19980
acggggatgg atggcgttcc cgatcatggt cctgcttgct tcgggtggca tcggaatgcc 20040
ggcgctgcaa gcaatgttgt ccaggcaggt ggatgaggaa cgtcaggggc agctgcaagg 20100
ctcactggcg gcgctcacca gcctgacctc gatcgtcgga cccctcctct tcacggcgat 20160
ctatgcggct tctataacaa cgtggaacgg gtgggcatgg attgcaggcg ctgccctcta 20220
cttgctctgc ctgccggcgc tgcgtcgcgg gctttggagc ggcgcagggc aacgagccga 20280
tcgctgatcg tggaaacgat aggcctatgc catgcgggtc aaggcgactt ccggcaagct 20340
atacgcgccc taggagtgcg gttggaacgt tggcccagcc agatactccc gatcacgagc 20400

aggacgccga tgatttgaag cgcactcagc gtctgatcca agaacaacca tcctagcaac 20460
acggcggtcc ccgggctgag aaagcccagt aaggaaacaa ctgtaggttc gagtcgcgag 20520
atcccccgga accaaaggaa gtaggttaaa cccgctccga tcaggccgag ccacgccagg 20580
ccgagaacat tggttcctgt aggcatcggg attggcggat caaacactaa agctactgga 20640
acgagcagaa gtcctccggc cgccagttgc caggcggtaa aggtgagcag aggcacggga 20700
ggttgccact tgcgggtcag cacggttccg aacgccatgg aaaccgcccc cgccaggccc 20760
gctgcgacgc cgacaggatc tagcgctgcg tttggtgtca acaccaacag cgccacgccc 20820
gcagttccgc aaatagcccc caggaccgcc atcaatcgta tcgggctacc tagcagagcg 20880
gcagagatga acacgaccat cagcggctgc acagcgccta ccgtcgccgc gaccccgccc 20940
ggcaggcggt agaccgaaat aaacaacaag ctccagaata gcgaaatatt aagtgcgccg 21000
aggatgaaga tgcgcatcca ccagattccc gttggaatct gtcggacgat catcacgagc 21060
aataaacccg ccggcaacgc ccgcagcagc ataccggcga cccctcggcc tcgctgttcg 21120
ggctccacga aaacgccgga cagatgcgcc ttgtgagcgt ccttggggcc gtcctcctgt 21180
ttgaagaccg acagcccaat gatctcgccg tcgatgtagg cgccgaatgc cacggcatct 21240
cgcaaccgtt cagcgaacgc ctccatgggc tttttctcct cgtgctcgta aacggacccg 21300
aacatctctg gagctttctt cagggccgac aatcggatct cgcggaaatc ctgcacgtcg 21360
gccgctccaa gccgtcgaat ctgagcctta atcacaattg tcaattttaa tcctctgttt 21420
atcggcagtt cgtagagcgc gccgtgcgtc ccgagcgata ctgagcgaag caagtgcgtc 21480
gagcagtgcc cgcttgttcc tgaaatgcca gtaaagcgct ggctgctgaa cccccagccg 21540
gaactgaccc cacaaggccc tagcgtttgc aatgcaccag gtcatcattg acccaggcgt 21600
gttccaccag gccgctgcct cgcaactctt cgcaggcttc gccgacctgc tcgcgccact 21660
tcttcacgcg ggtggaatcc gatccgcaca tgaggcggaa ggtttccagc ttgagcgggt 21720
acggctcccg gtgcgagctg aaatagtcga acatccgtcg ggccgtcggc gacagcttgc 21780
ggtacttctc ccatatgaat ttcgtgtagt ggtcgccagc aaacagcacg acgatttcct 21840
cgtcgatcag gacctggcaa cgggacgttt tcttgccacg gtccaggacg cggaagcggt 21900
gcagcagcga caccgattcc aggtgcccaa cgcggtcgga cgtgaagccc atcgccgtcg 21960
cctgtaggcg cgacaggcat tcctcggcct tcgtgtaata ccggccattg atcgaccagc 22020
ccaggtcctg gcaaagctcg tagaacgtga aggtgatcgg ctcgccgata ggggtgcgct 22080
tcgcgtactc caacacctgc tgccacacca gttcgtcatc gtcggcccgc agctcgacgc 22140
cggtgtaggt gatcttcacg tccttgttga cgtggaaaat gaccttgttt tgcagcgcct 22200
cgcgcgggat tttcttgttg cgcgtggtga acagggcaga gcgggccgtg tcgtttggca 22260
tcgctcgcat cgtgtccggc cacggcgcaa tatcgaacaa ggaaagctgc atttccttga 22320

tctgctgctt cgtgtgtttc agcaacgcgg cctgcttggc ctcgctgacc tgttttgcca 22380

ggtcctcgcc ggcggttttt cgcttcttgg tcgtcatagt tcctcgcgtg tcgatggtca 22440

tcgacttcgc caaacctgcc gcctcctgtt cgagacgacg cgaacgctcc acggcggccg 22500

atggcgcggg cagggcaggg ggagccagtt gcacgctgtc gcgctcgatc ttggccgtag 22560

cttgctggac catcgagccg acggactgga aggtttcgcg gggcgcacgc atgacggtgc 22620

ggcttgcgat ggtttcggca tcctcggcgg aaaaccccgc gtcgatcagt tcttgcctgt 22680

atgccttccg gtcaaacgtc cgattcattc accctccttg cgggattgcc ccgactcacg 22740

ccggggcaat gtgcccttat tcctgatttg acccgcctgg tgccttggtg tccagataat 22800

ccaccttatc ggcaatgaag tcggtcccgt agaccgtctg gccgtccttc tcgtacttgg 22860

tattccgaat cttgccctgc acgaatacca gcgacccctt gcccaaatac ttgccgtggg 22920

cctcggcctg agagccaaaa cacttgatgc ggaagaagtc ggtgcgctcc tgcttgtcgc 22980

cggcatcgtt gcgccactct tcattaaccg ctatatcgaa aattgcttgc ggcttgttag 23040

aattgccatg acgtacctcg gtgtcacggg taagattacc gataaactgg aactgattat 23100

ggctcatatc gaaagtctcc ttgagaaagg agactctagt ttagctaaac attggttccg 23160

ctgtcaagaa ctttagcggc taaaattttg cgggccgcga ccaaaggtgc gaggggcggc 23220

ttccgctgtg tacaaccaga tatttttcac caacatcctt cgtctgctcg atgagcgggg 23280

catgacgaaa catgagctgt cggagagggc aggggtttca atttcgtttt tatcagactt 23340

aaccaacggt aaggccaacc cctcgttgaa ggtgatggag gccattgccg acgccctgga 23400

aactccccta cctcttctcc tggagtccac cgaccttgac cgcgaggcac tcgcggagat 23460

tgcgggtcat cctttcaaga gcagcgtgcc gcccggatac gaacgcatca gtgtggtttt 23520

gccgtcacat aaggcgttta tcgtaaagaa atggggcgac gacacccgaa aaaagctgcg 23580

tggaaggctc tgacgccaag ggttagggct tgcacttcct tctttagccg ctaaaacggc 23640

cccttctctg cgggccgtcg gctcgcgcat catatcgaca tcctcaacgg aagccgtgcc 23700

gcgaatggca tcgggcgggt gcgctttgac agttgttttc tatcagaacc cctacgtcgt 23760

gcggttcgat tagctgtttg tcttgcaggc taaacacttt cggtatatcg tttgcctgtg 23820

cgataatgtt gctaatgatt tgttgcgtag gggttactga aaagtgagcg ggaaagaaga 23880

gtttcagacc atcaaggagc gggccaagcg caagctggaa cgcgacatgg gtgcggacct 23940

gttggccgcg ctcaacgacc cgaaaaccgt tgaagtcatg ctcaacgcgg acggcaaggt 24000

gtggcacgaa cgccttggcg agccgatgcg gtacatctgc gacatgcggc ccagccagtc 24060

gcaggcgatt atagaaacgg tggccggatt ccacggcaaa gaggtcacgc ggcattcgcc 24120

catcctggaa ggcgagttcc ccttggatgg cagccgcttt gccggccaat tgccgccggt 24180

cgtggccgcg ccaacctttg cgatccgcaa gcgcgcggtc gccatcttca cgctggaaca 24240

gtacgtcgag gcgggcatca tgacccgcga gcaatacgag gtcattaaaa gcgccgtcgc 24300

ggcgcatcga aacatcctcg tcattggcgg tactggctcg ggcaagacca cgctcgtcaa 24360
cgcgatcatc aatgaaatgg tcgccttcaa cccgtctgag cgcgtcgtca tcatcgagga 24420
caccggcgaa atccagtgcg ccgcagagaa cgccgtccaa taccacacca gcatcgacgt 24480
ctcgatgacg ctgctgctca agacaacgct gcgtatgcgc cccgaccgca tcctggtcgg 24540
tgaggtacgt ggccccgaag cccttgatct gttgatggcc tggaacaccg ggcatgaagg 24600
aggtgccgcc accctgcacg caaacaaccc caaagcgggc ctgagccggc tcgccatgct 24660
tatcagcatg cacccggatt caccgaaacc cattgagccg ctgattggcg aggcggttca 24720
tgtggtcgtc catatcgcca ggacccctag cggccgtcga gtgcaagaaa ttctcgaagt 24780
tcttggttac gagaacggcc agtacatcac caaaaccctg taaggagtat ttccaatgac 24840
aacggctgtt ccgttccgtc tgaccatgaa tcgcggcatt ttgttctacc ttgccgtgtt 24900
cttcgttctc gctctcgcgt tatccgcgca tccggcgatg gcctcggaag gcaccggcgg 24960
cagcttgcca tatgagagct ggctgacgaa cctgcgcaac tccgtaaccg gcccggtggc 25020
cttcgcgctg tccatcatcg gcatcgtcgt cgccggcggc gtgctgatct tcggcggcga 25080
actcaacgcc ttcttccgaa ccctgatctt cctggttctg gtgatggcgc tgctggtcgg 25140
cgcgcagaac gtgatgagca ccttcttcgg tcgtggtgcc gaaatcgcgg ccctcggcaa 25200
cggggcgctg caccaggtgc aagtcgcggc ggcggatgcc gtgcgtgcgg tagcggctgg 25260
acggctcgcc taatcatggc tctgcgcacg atccccatcc gtcgcgcagg caaccgagaa 25320
aacctgttca tgggtggtga tcgtgaactg gtgatgttct cgggcctgat ggcgtttgcg 25380
ctgattttca gcgcccaaga gctgcgggcc accgtggtcg gtctgatcct gtggttcggg 25440
gcgctctatg cgttccgaat catggcgaag gccgatccga agatgcggtt cgtgtacctg 25500
cgtcaccgcc ggtacaagcc gtattacccg gcccgctcga ccccgttccg cgagaacacc 25560
aatagccaag ggaagcaata ccgatgatcc aagcaattgc gattgcaatc gcgggcctcg 25620
gcgcgcttct gttgttcatc ctctttgccc gcatccgcgc ggtcgatgcc gaactgaaac 25680
tgaaaaagca tcgttccaag gacgccggcc tggccgatct gctcaactac gccgctgtcg 25740
tcgatgacgg cgtaatcgtg ggcaagaacg gcagctttat ggctgcctgg ctgtacaagg 25800
gcgatgacaa cgcaagcagc accgaccagc agcgcgaagt agtgtccgcc cgcatcaacc 25860
aggccctcgc gggcctggga agtgggtgga tgatccatgt ggacgccgtg cggcgtcctg 25920
ctccgaacta cgcggagcgg ggcctgtcgg cgttccctga ccgtctgacg gcagcgattg 25980
aagaagagcg ctcggtcttg ccttgctcgt cggtgatgta cttcaccagc tccgcgaagt 26040
cgctcttctt gatggagcgc atggggacgt gcttggcaat cacgcgcacc ccccggccgt 26100
tttagcggct aaaaaagtca tggctctgcc ctcgggcgga ccacgcccat catgaccttg 26160
ccaagctcgt cctgcttctc ttcgatcttc gccagcaggg cgaggatcgt ggcatcaccg 26220

aaccgcgccg tgcgcgggtc gtcggtgagc cagagtttca gcaggccgcc caggcggccc 26280

aggtcgccat tgatgcgggc cagctcgcgg acgtgctcat agtccacgac gcccgtgatt 26340

ttgtagccct ggccgacggc cagcaggtag gccgacaggc tcatgccggc cgccgccgcc 26400

ttttcctcaa tcgctcttcg ttcgtctgga aggcagtaca ccttgatagg tgggctgccc 26460

ttcctggttg gcttggtttc atcagccatc cgcttgccct catctgttac gccggcggta 26520

gccggccagc ctcgcagagc aggattcccg ttgagcaccg ccaggtgcga ataagggaca 26580

gtgaagaagg aacacccgct cgcgggtggg cctacttcac ctatcctgcc cggctgacgc 26640

cgttggatac accaaggaaa gtctacacga accctttggc aaaatcctgt atatcgtgcg 26700

aaaaaggatg gatataccga aaaaatcgct ataatgaccc cgaagcaggg ttatgcagcg 26760

gaaaagcgct gcttccctgc tgttttgtgg aatatctacc gactggaaac aggcaaatgc 26820

aggaaattac tgaactgagg ggacaggcga gagacgatgc caaagagcta caccgacgag 26880

ctggccgagt gggttgaatc ccgcgcggcc aagaagcgcc ggcgtgatga ggctgcggtt 26940

gcgttcctgg cggtgagggc ggatgtcgag gcggcgttag cgtccggcta tgcgctcgtc 27000

accatttggg agcacatgcg ggaaacgggg aaggtcaagt tctcctacga gacgttccgc 27060

tcgcacgcca ggcggcacat caaggccaag cccgccgatg tgcccgcacc gcaggccaag 27120

gctgcggaac ccgcgccggc acccaagacg ccggagccac ggcggccgaa gcaggggggc 27180

aaggctgaaa agccggcccc cgctgcggcc ccgaccggct tcaccttcaa cccaacaccg 27240

gacaaaaagg atctactgta atggcgaaaa ttcacatggt tttgcagggc aagggcgggg 27300

tcggcaagtc ggccatcgcc gcgatcattg cgcagtacaa gatggacaag gggcagacac 27360

ccttgtgcat cgacaccgac ccggtgaacg cgacgttcga gggctacaag gccctgaacg 27420

tccgccggct gaacatcatg gccggcgacg aaattaactc gcgcaacttc gacaccctgg 27480

tcgagctgat tgcgccgacc aaggatgacg tggtgatcga caacggtgcc agctcgttcg 27540

tgcctctgtc gcattacctc atcagcaacc aggtgccggc tctgctgcaa gaaatggggc 27600

atgagctggt catccatacc gtcgtcaccg gcggccaggc tctcctggac acggtgagcg 27660

gcttcgccca gctcgccagc cagttcccgg ccgaagcgct tttcgtggtc tggctgaacc 27720

cgtattgggg gcctatcgag catgagggca agagctttga gcagatgaag gcgtacacgg 27780

ccaacaaggc ccgcgtgtcg tccatcatcc agattccggc cctcaaggaa gaaacctacg 27840

gccgcgattt cagcgacatg ctgcaagagc ggctgacgtt cgaccaggcg ctggccgatg 27900

aatcgctcac gatcatgacg cggcaacgcc tcaagatcgt gcggcgcggc ctgtttgaac 27960

agctcgacgc ggcggccgtg ctatgagcga ccagattgaa gagctgatcc gggagattgc 28020

ggccaagcac ggcatcgccg tcggccgcga cgacccggtg ctgatcctgc ataccatcaa 28080

cgcccggctc atggccgaca gtgcggccaa gcaagaggaa atccttgccg cgttcaagga 28140

agagctggaa gggatcgccc atcgttgggg cgaggacgcc aaggccaaag cggagcggat 28200

gctgaacgcg gccctggcgg ccagcaagga cgcaatggcg aaggtaatga aggacagcgc 28260
cgcgcaggcg gccgaagcga tccgcaggga aatcgacgac ggccttggcc gccagctcgc 28320
ggccaaggtc gcggacgcgc ggcgcgtggc gatgatgaac atgatcgccg gcggcatggt 28380
gttgttcgcg gccgccctgg tggtgtgggc ctcgttatga atcgcagagg cgcagatgaa 28440
aaagcccggc gttgccgggc tttgtttttg cgttagctgg gcttgtttga caggcccaag 28500
ctctgactgc gcccgcgctc gcgctcctgg gcctgtttct tctcctgctc ctgcttgcgc 28560
atcagggcct ggtgccgtcg ggctgcttca cgcatcgaat cccagtcgcc ggccagctcg 28620
ggatgctccg cgcgcatctt gcgcgtcgcc agttcctcga tcttgggcgc gtgaatgccc 28680
atgccttcct tgatttcgcg caccatgtcc agccgcgtgt gcagggtctg caagcgggct 28740
tgctgttggg cctgctgctg ctgccaggcg gcctttgtac gcggcaggga cagcaagccg 28800
ggggcattgg actgtagctg ctgcaaacgc gcctgctgac ggtctacgag ctgttctagg 28860
cggtcctcga tgcgctccac ctggtcatgc tttgcctgca cgtagagcgc aagggtctgc 28920
tggtaggtct gctcgatggg cgcggattct aagagggcct gctgttccgt ctcggcctcc 28980
tgggccgcct gtagcaaatc ctcgccgctg ttgccgctgg actgctttac tgccggggac 29040
tgctgttgcc ctgctcgcgc cgtcgtcgca gttcggcttg cccccactcg attgactgct 29100
tcatttcgag ccgcagcgat gcgatctcgg attgcgtcaa cggacggggc agcgcggagg 29160
tgtccggctt ctccttgggt gagtcggtcg atgccatagc caaaggtttc cttccaaaat 29220
gcgtccattg ctggaccgtg tttctcattg atgcccgcaa gcatcttcgg cttgaccgcc 29280
aggtcaagcg cgccttcatg ggcggtcatg acggacgccg ccatgacctt gccgccgttg 29340
ttctcgatgt agccgcgtaa tgaggcaatg gtgccgccca tcgtcagcgt gtcatcgaca 29400
acgatgtact tctggccggg gatcacctcc ccctcgaaag tcgggttgaa cgccaggcga 29460
tgatctgaac cggctccggt tcgggcgacc ttctcccgct gcacaatgtc cgtttcgacc 29520
tcaaggccaa ggcggtcggc cagaacgacc gccatcatgg ccggaatctt gttgttcccc 29580
gccgcctcga cggcgaggac tggaacgatg cggggcttgt cgtcgccgat cagcgtcttg 29640
agctgggcaa cagtgtcgtc cgaaatcagg cgctcgacca aattaagcgc cgcttccgcg 29700
tcgccctgct tcgcagcctg gtattcaggc tcgttggtca aagaaccaag gtcgccgttg 29760
cgaaccacct tcgggaagtc tccccacggt gcgcgctcgg ctctgctgta gctgctcaag 29820
acgcctccct ttttagccgc taaaactcta acgagtgcgc ccgcgactca acttgacgct 29880
ttcggcactt acctgtgcct tgccacttgc gtcataggtg atgcttttcg cactcccgat 29940
ttcaggtact ttatcgaaat ctgaccgggc gtgcattaca aagttcttcc ccacctgttg 30000
gtaaatgctg ccgctatctg cgtggacgat gctgccgtcg tggcgctgcg acttatcggc 30060
cttttgggcc atatagatgt tgtaaatgcc aggtttcagg gccccggctt tatctacctt 30120

ctggttcgtc catgcgcctt ggttctcggt ctggacaatt ctttgcccat tcatgaccag 30180
gaggcggtgt ttcattgggt gactcctgac ggttgcctct ggtgttaaac gtgtcctggt 30240
cgcttgccgg ctaaaaaaaa gccgacctcg gcagttcgag gccggctttc cctagagccg 30300
ggcgcgtcaa ggttgttcca tctattttag tgaactgcgt tcgatttatc agttactttc 30360
ctcccgcttt gtgtttcctc ccactcgttt ccgcgtctag ccgacccctc aacatagcgg 30420
cctcttcttg ggctgccttt gcctcttgcc gcgcttcgtc acgctcggct tgcaccgtcg 30480
taaagcgctc ggcctgcctg gccgcctctt gcgccgccaa cttcctttgc tcctggtggg 30540
cctcggcgtc ggcctgcgcc ttcgctttca ccgctgccaa ctccgtgcgc aaactctccg 30600
cttcgcgcct ggtggcgtcg cgctcgccgc gaagcgcctg catttcctgg ttggccgcgt 30660
ccagggtctt gcggctctct tctttgaatg cgcgggcgtc ctggtgagcg tagtccagct 30720
cggcgcgcag ctcctgcgct cgacgctcca cctcgtcggc ccgctgcgtc gccagcgcgg 30780
cccgctgctc ggctcctgcc agggcggtgc gtgcttcggc cagggcttgc cgctggcgtg 30840
cggccagctc ggccgcctcg gcggcctgct gctctagcaa tgtaacgcgc gcctgggctt 30900
cttccagctc gcgggcctgc gcctcgaagg cgtcggccag ctccccgcgc acggcttcca 30960
actcgttgcg ctcacgatcc cagccggctt gcgctgcctg caacgattca ttggcaaggg 31020
cctgggcggc ttgccagagg gcggccacgg cctggttgcc ggcctgctgc accgcgtccg 31080
gcacctggac tgccagcggg gcggcctgcg ccgtgcgctg gcgtcgccat tcgcgcatgc 31140
cggcgctggc gtcgttcatg ttgacgcggg cggccttacg cactgcatcc acggtcggga 31200
agttctcccg gtcgccttgc tcgaacagct cgtccgcagc cgcaaaaatg cggtcgcgcg 31260
tctctttgtt cagttccatg ttggctccgg taattggtaa gaataataat actcttacct 31320
accttatcag cgcaagagtt tagctgaaca gttctcgact taacggcagg tttttttagcg 31380
gctgaagggc aggcaaaaaa agccccgcac ggtcggcggg ggcaaagggt cagcgggaag 31440
gggattagcg ggcgtcgggc ttcttcatgc gtcggggccg cgcttcttgg gatggagcac 31500
gacgaagcgc gcacgcgcat cgtcctcggc cctatcggcc cgcgtcgcgg tcaggaactt 31560
gtcgcgcgct aggtcctccc tggtgggcac caggggcatg aactcggcct gctcgatgta 31620
ggtccactcc atgaccgcat cgcagtcgag gccgcgttcc ttcaccgtct cttgcaggtc 31680
gcggtacgcc cgctcgttga gcggctggta acgggccaat tggtcgtaaa tggctgtcgg 31740
ccatgagcgg cctttcctgt tgagccagca gccgacgacg aagccggcaa tgcaggcccc 31800
tggcacaacc aggccgacgc cgggggcagg ggatggcagc agctcgccaa ccaggaaccc 31860
cgccgcgatg atgccgatgc cggtcaacca gcccttgaaa ctatccggcc ccgaaacacc 31920
cctgcgcatt gcctggatgc tgcgccggat agcttgcaac atcaggagcc gtttcttttg 31980
ttcgtcagtc atggtccgcc ctcaccagtt gttcgtatcg gtgtcggacg aactgaaatc 32040
gcaagagctg ccggtatcgg tccagccgct gtccgtgtcg ctgctgccga agcacggcga 32100

ggggtccgcg aacgccgcag acggcgtatc cggccgcagc gcatcgccca gcatggcccc 32160
ggtcagcgag ccgccggcca ggtagcccag catggtgctg ttggtcgccc cggccaccag 32220
ggccgacgtg acgaaatcgc cgtcattccc tctggattgt tcgctgctcg gcggggcagt 32280
gcgccgcgcc ggcggcgtcg tggatggctc gggttggctg gcctgcgacg gccggcgaaa 32340
ggtgcgcagc agctcgttat cgaccggctg cggcgtcggg gccgccgcct tgcgctgcgg 32400
tcggtgttcc ttcttcggct cgcgcagctt gaacagcatg atcgcggaaa ccagcagcaa 32460
cgccgcgcct acgcctcccg cgatgtagaa cagcatcgga ttcattcttc ggtcctcctt 32520
gtagcggaac cgttgtctgt gcggcgcggg tggcccgcgc cgctgtcttt ggggatcagc 32580
cctcgatgag cgcgaccagt ttcacgtcgg caaggttcgc ctcgaactcc tggccgtcgt 32640
cctcgtactt caaccaggca tagccttccg ccggcggccg acggttgagg ataaggcggg 32700
cagggcgctc gtcgtgctcg acctggacga tggccttttt cagcttgtcc gggtccggct 32760
ccttcgcgcc cttttccttg gcgtccttac cgtcctggtc gccgtcctcg ccgtcctggc 32820
cgtcgccggc ctccgcgtca cgctcggcat cagtctggcc gttgaaggca tcgacggtgt 32880
tgggatcgcg gcccttctcg tccaggaact cgcgcagcag cttgaccgtg ccgcgcgtga 32940
tttcctgggt gtcgtcgtca agccacgcct cgacttcctc cgggcgcttc ttgaaggccg 33000
tcaccagctc gttcaccacg gtcacgtcgc gcacgcggcc ggtgttgaac gcatcggcga 33060
tcttctccgg caggtccagc agcgtgacgt gctgggtgat gaacgccggc gacttgccga 33120
tttccttggc gatatcgcct ttcttcttgc ccttcgccag ctcgcggcca atgaagtcgg 33180
caatttcgcg cggggtcagc tcgttgcgtt gcaggttctc gataacctgg tcggcttcgt 33240
tgtagtcgtt gtcgatgaac gccgggatgg acttcttgcc ggcccacttc gagccacggt 33300
agcggcgggc gccgtgattg atgatatagc ggcccggctg ctcctggttc tcgcgcaccg 33360
aaatgggtga cttcaccccg cgctctttga tcgtggcacc gatttccgcg atgctctccg 33420
gggaaaagcc ggggttgtcg gccgtccgcg gctgatgcgg atcttcgtcg atcaggtcca 33480
ggtccagctc gatagggccg gaaccgccct gagacgccgc aggagcgtcc aggaggctcg 33540
acaggtcgcc gatgctatcc aaccccaggc cggacggctg cgccgcgcct gcggcttcct 33600
gagcggccgc agcggtgttt ttcttggtgg tcttggcttg agccgcagtc attgggaaat 33660
ctccatcttc gtgaacacgt aatcagccag ggcgcgaacc tctttcgatg ccttgcgcgc 33720
ggccgttttc ttgatcttcc agaccggcac accggatgcg agggcatcgg cgatgctgct 33780
gcgcaggcca acggtggccg gaatcatcat cttggggtac gcggccagca gctcggcttg 33840
gtggcgcgcg tggcgcggat tccgcgcatc gaccttgctg ggcaccatgc caaggaattg 33900
cagcttggcg ttcttctggc gcacgttcgc aatggtcgtg accatcttct tgatgccctg 33960
gatgctgtac gcctcaagct cgatggggga cagcacatag tcggccgcga agagggcggc 34020

cgccaggccg acgccaaggg tcggggccgt gtcgatcagg cacacgtcga agccttggtt 34080
cgccagggcc ttgatgttcg ccccgaacag ctcgcgggcg tcgtccagcg acagccgttc 34140
ggcgttcgcc agtaccgggt tggactcgat gagggcgagg cgcgcggcct ggccgtcgcc 34200
ggctgcgggt gcggtttcgg tccagccgcc ggcagggaca gcgccgaaca gcttgcttgc 34260
atgcaggccg gtagcaaagt ccttgagcgt gtaggacgca ttgccctggg ggtccaggtc 34320
gatcacggca acccgcaagc cgcgctcgaa aaagtcgaag gcaagatgca caagggtcga 34380
agtcttgccg acgccgcctt tctggttggc cgtgaccaaa gttttcatcg tttggtttcc 34440
tgttttttct tggcgtccgc ttcccacttc cggacgatgt acgcctgatg ttccggcaga 34500
accgccgtta cccgcgcgta cccctcgggc aagttcttgt cctcgaacgc ggcccacacg 34560
cgatgcaccg cttgcgacac tgcgcccctg gtcagtccca gcgacgttgc gaacgtcgcc 34620
tgtggcttcc catcgactaa gacgccccgc gctatctcga tggtctgctg ccccacttcc 34680
agcccctgga tcgcctcctg gaactggctt tcggtaagcc gtttcttcat ggataacacc 34740
cataatttgc tccgcgcctt ggttgaacat agcggtgaca gccgccagca catgagagaa 34800
gtttagctaa acatttctcg cacgtcaaca cctttagccg ctaaaactcg tccttggcgt 34860
aacaaaacaa aagcccggaa accgggcttt cgtctcttgc cgcttatggc tctgcacccg 34920
gctccatcac caacaggtcg cgcacgcgct tcactcggtt gcggatcgac actgccagcc 34980
caacaaagcc ggttgccgcc gccgccagga tcgcgccgat gatgccggcc acaccggcca 35040
tcgcccacca ggtcgccgcc ttccggttcc attcctgctg gtactgcttc gcaatgctgg 35100
acctcggctc accataggct gaccgctcga tggcgtatgc cgcttctccc cttggcgtaa 35160
aacccagcgc cgcaggcggc attgccatgc tgcccgccgc tttcccgacc acgacgcgcg 35220
caccaggctt gcggtccaga ccttcggcca cggcgagctg cgcaaggaca taatcagccg 35280
ccgacttggc tccacgcgcc tcgatcagct cttgcactcg cgcgaaatcc ttggcctcca 35340
cggccgccat gaatcgcgca cgcggcgaag gctccgcagg gccggcgtcg tgatcgccgc 35400
cgagaatgcc cttcaccaag ttcgacgaca cgaaaatcat gctgacggct atcaccatca 35460
tgcagacgga tcgcacgaac ccgctgaatt gaacacgagc acggcacccg cgaccactat 35520
gccaagaatg cccaaggtaa aaattgccgg ccccgccatg aagtccgtga atgccccgac 35580
ggccgaagtg aagggcaggc cgccacccag gccgccgccc tcactgcccg gcacctggtc 35640
gctgaatgtc gatgccagca cctgcggcac gtcaatgctt ccgggcgtcg cgctcgggct 35700
gatcgcccat cccgttactg ccccgatccc ggcaatggca aggactgcca gcgctgccat 35760
ttttggggtg aggccgttcg cggccgaggg gcgcagcccc tgggggggatg ggaggcccgc 35820
gttagcgggc cgggagggtt cgagaagggg gggcaccccc cttcggcgtg cgcggtcacg 35880
cgcacagggc gcagccctgg ttaaaaacaa ggtttataaa tattggttta aaagcaggtt 35940
aaaagacagg ttagcggtgg ccgaaaaacg ggcggaaacc cttgcaaatg ctggattttc 36000

tgcctgtgga cagcccctca aatgtcaata ggtgcgcccc tcatctgtca gcactctgcc 36060

cctcaagtgt caaggatcgc gcccctcatc tgtcagtagt cgcgcccctc aagtgtcaat 36120

accgcagggc acttatcccc aggcttgtcc acatcatctg tgggaaactc gcgtaaaatc 36180

aggcgttttc gccgatttgc gaggctggcc agctccacgt cgccggccga aatcgagcct 36240

gcccctcatc tgtcaacgcc gcgccgggtg agtcggcccc tcaagtgtca acgtccgccc 36300

ctcatctgtc agtgagggcc aagttttccg cgaggtatcc acaacgccgg cggccgcggt 36360

gtctcgcaca cggcttcgac ggcgtttctg gcgcgtttgc agggccatag acggccgcca 36420

gcccagcggc gagggcaacc agcccggtga gcgtcggaaa ggcgctggaa gccccgtagc 36480

gacgcggaga ggggcgagac aagccaaggg cgcaggctcg atgcgcagca cgacatagcc 36540

ggttctcgca aggacgagaa tttccctgcg gtgcccctca agtgtcaatg aaagtttcca 36600

acgcgagcca ttcgcgagag ccttgagtcc acgctagatg agagctttgt tgtaggtgga 36660

ccagttggtg attttgaact tttgctttgc cacggaacgg tctgcgttgt cgggaagatg 36720

cgtgatctga tccttcaact cagcaaaagt tcgatttatt caacaaagcc acgttgtgtc 36780

tcaaaatctc tgatgttaca ttgcacaaga taaaaatata tcatcatgaa caataaaact 36840

gtctgcttac ataaacagta atacaagggg tgttatgagc catattcaac gggaaacgtc 36900

ttgctcgac 36909

<210> 8
<211> 13019
<212> DNA
<213> artificial sequence

<220>
<223> vector

<400> 8
gttacccgga ccgaagctta gcccgggcat gcctgcagtg cagcgtgacc cggtcgtgcc 60

cctctctaga gataatgagc attgcatgtc taagttataa aaaattacca catatttttt 120

ttgtcacact tgtttgaagt gcagtttatc tatctttata catatattta aactttactc 180

tacgaataat ataatctata gtactacaat aatatcagtg ttttagagaa tcatataaat 240

gaacagttag acatggtcta aaggacaatt gagtattttg acaacaggac tctacagttt 300

tatcttttta gtgtgcatgt gttctccttt ttttttgcaa atagcttcac ctatataata 360

cttcatccat tttattagta catccattta gggtttaggg ttaatggttt ttatagacta 420

attttttag tacatctatt ttattctatt ttagcctcta aattaagaaa actaaaactc 480

tattttagtt tttttattta ataatttaga tataaaatag aataaaataa agtgactaaa 540

aattaaacaa ataccctta agaaattaaa aaaactaagg aaacattttt cttgtttcga 600

gtagataatg ccagcctgtt aaacgccgtc gacgagtcta acggacacca accagcgaac 660

cagcagcgtc gcgtcgggcc aagcgaagca gacggcacgg catctctgtc gctgcctctg 720
gacccctctc gagagttccg ctccaccgtt ggacttgctc cgctgtcggc atccagaaat 780
tgcgtggcgg agcggcagac gtgagccggc acggcaggcg gcctcctcct cctctcacgg 840
cacggcagct acgggggatt cctttcccac cgctccttcg ctttcccttc ctcgcccgcc 900
gtaataaata gacacccccct ccacaccctc tttccccaac ctcgtgttgt tcggagcgca 960
cacacacaca accagatctc ccccaaatcc acccgtcggc acctccgctt caaggtacgc 1020
cgctcgtcct cccccccccc ccctctctac cttctctaga tcggcgttcc ggtccatggt 1080
tagggcccgg tagttctact tctgttcatg tttgtgttag atccgtgttt gtgttagatc 1140
cgtgctgcta gcgttcgtac acggatgcga cctgtacgtc agacacgttc tgattgctaa 1200
cttgccagtg tttctctttg gggaatcctg ggatggctct agccgttccg cagacgggat 1260
cgatttcatg attttttttg tttcgttgca tagggtttgg tttgcccttt tcctttattt 1320
caatatatgc cgtgcacttg tttgtcgggt catcttttca tgcttttttt tgtcttggtt 1380
gtgatgatgt ggtctggttg ggcggtcgtt ctagatcgga gtagaattct gtttcaaact 1440
acctggtgga tttattaatt ttggatctgt atgtgtgtgc catacatatt catagttacg 1500
aattgaagat gatggatgga aatatcgatc taggataggt atacatgttg atgcgggttt 1560
tactgatgca tatacagaga tgctttttgt tcgcttggtt gtgatgatgt ggtgtggttg 1620
ggcggtcgtt cattcgttct agatcggagt agaatactgt ttcaaactac ctggtgtatt 1680
tattaatttt ggaactgtat gtgtgtgtca tacatcttca tagttacgag tttaagatgg 1740
atggaaatat cgatctagga taggtataca tgttgatgtg ggttttactg atgcatatac 1800
atgatggcat atgcagcatc tattcatatg ctctaacctt gagtacctat ctattataat 1860
aaacaagtat gttttataat tattttgatc ttgatatact tggatgatgg catatgcagc 1920
agctatatgt ggattttttt agccctgcct tcatacgcta tttatttgct tggtactgtt 1980
tcttttgtcg atgctcaccc tgttgtttgg tgttacttct gcaggtcgac tctagaggat 2040
ccacaagttt gtacaaaaaa gctgaacgag aaacgtaaaa tgatataaat atcaatatat 2100
taaattagat tttgcataaa aaacagacta cataatactg taaaacacaa catatccagt 2160
cactatggcg gccgcattag gcaccccagg ctttacactt tatgcttccg gctcgtataa 2220
tgtgtggatt ttgagttagg atttaaatac gcgttgatcc ggcttactaa aagccagata 2280
acagtatgcg tatttgcgcg ctgatttttg cggtataaga atatatactg atatgtatac 2340
ccgaagtatg tcaaaaagag gtatgctatg aagcagcgta ttacagtgac agttgacagc 2400
gacagctatc agttgctcaa ggcatatatg atgtcaatat ctccggtctg gtaagcacaa 2460
ccatgcagaa tgaagcccgt cgtctgcgtg ccgaacgctg gaaagcggaa aatcaggaag 2520
ggatggctga ggtcgcccgg tttattgaaa tgaacggctc ttttgctgac gagaacaggg 2580
gctggtgaaa tgcagtttaa ggtttacacc tataaaagag agagccgtta tcgtctgttt 2640

gtggatgtac agagtgatat cattgacacg cccggtcgac ggatggtgat ccccctggcc 2700
agtgcacgtc tgctgtcaga taaagtctcc cgtgaacttt acccggtggt gcatatcggg 2760
gatgaaagct ggcgcatgat gaccaccgat atggccagtg tgccggtctc cgttatcggg 2820
gaagaagtgg ctgatctcag ccaccgcgaa aatgacatca aaaacgccat taacctgatg 2880
ttctggggaa tataaatgtc aggctccctt atacacagcc agtctgcagg tcgaccatag 2940
tgactggata tgttgtgttt tacagtatta tgtagtctgt tttttatgca aaatctaatt 3000
taatatattg atatttatat cattttacgt ttctcgttca gctttcttgt acaaagtggt 3060
gttaacctag acttgtccat cttctggatt ggccaactta attaatgtat gaaataaaag 3120
gatgcacaca tagtgacatg ctaatcacta taatgtgggc atcaaagttg tgtgttatgt 3180
gtaattacta gttatctgaa taaaagagaa agagatcatc catatttctt atcctaaatg 3240
aatgtcacgt gtctttataa ttctttgatg aaccagatgc atttcattaa ccaaatccat 3300
atacatataa atattaatca tatataatta atatcaattg ggttagcaaa acaaatctag 3360
tctaggtgtg ttttgcgaat tgcggccgcc accgcggtgg agctcgaatt ccggtccggg 3420
tcacctttgt ccaccaagat ggaactgcgg ccgctcatta attaagtcag gcgcgcctct 3480
agttgaagac acgttcatgt cttcatcgta agaagacact cagtagtctt cggccagaat 3540
ggccatctgg attcagcagg cctagaaggc catttaaatc ctgaggatct ggtcttccta 3600
aggacccggg atatcggacc gattaaactt taattcggtc cgaagcttgc atgcctgcag 3660
tgcagcgtga cccggtcgtg cccctctcta gagataatga gcattgcatg tctaagttat 3720
aaaaaattac cacatatttt ttttgtcaca cttgtttgaa gtgcagttta tctatcttta 3780
tacatatatt taaactttac tctacgaata atataatcta tagtactaca ataatatcag 3840
tgttttagag aatcatataa atgaacagtt agacatggtc taaaggacaa ttgagtattt 3900
tgacaacagg actctacagt tttatctttt tagtgtgcat gtgttctcct ttttttttgc 3960
aaatagcttc acctatataa tacttcatcc attttattag tacatccatt tagggtttag 4020
ggttaatggt ttttatagac taattttttt agtacatcta ttttattcta ttttagcctc 4080
taaattaaga aaactaaaac tctattttag tttttttatt taataattta gatataaaat 4140
agaataaaat aaagtgacta aaaattaaac aaataccctt taagaaatta aaaaaactaa 4200
ggaaacattt ttcttgtttc gagtagataa tgccagcctg ttaaacgccg tcgacgagtc 4260
taacggacac caaccagcga accagcagcg tcgcgtcggg ccaagcgaag cagacggcac 4320
ggcatctctg tcgctgcctc tggacccctc tcgagagttc cgctccaccg ttggacttgc 4380
tccgctgtcg gcatccagaa attgcgtggc ggagcggcag acgtgagccg gcacggcagg 4440
cggcctcctc ctcctctcac ggcaccggca gctacggggg attcctttcc caccgctcct 4500
tcgctttccc ttcctcgccc gccgtaataa atagacaccc cctccacacc ctctttcccc 4560

aacctcgtgt tgttcggagc gcacacacac acaaccagat ctcccccaaa tccacccgtc 4620
ggcacctccg cttcaaggta cgccgctcgt cctccccccc ccccctctct accttctcta 4680
gatcggcgtt ccggtccatg catggttagg gcccggtagt tctacttctg ttcatgtttg 4740
tgttagatcc gtgtttgtgt tagatccgtg ctgctagcgt tcgtacacgg atgcgacctg 4800
tacgtcagac acgttctgat tgctaacttg ccagtgtttc tctttgggga atcctgggat 4860
ggctctagcc gttccgcaga cgggatcgat ttcatgattt tttttgtttc gttgcatagg 4920
gtttggtttg cccttttcct ttatttcaat atatgccgtg cacttgtttg tcgggtcatc 4980
ttttcatgct tttttttgtc ttggttgtga tgatgtggtc tggttgggcg gtcgttctag 5040
atcggagtag aattctgttt caaactacct ggtggattta ttaattttgg atctgtatgt 5100
gtgtgccata catattcata gttacgaatt gaagatgatg gatggaaata tcgatctagg 5160
ataggtatac atgttgatgc gggttttact gatgcatata cagagatgct ttttgttcgc 5220
ttggttgtga tgatgtggtg tggttgggcg gtcgttcatt cgttctagat cggagtagaa 5280
tactgtttca aactacctgg tgtatttatt aattttggaa ctgtatgtgt gtgtcataca 5340
tcttcatagt tacgagttta agatggatgg aaatatcgat ctaggatagg tatacatgtt 5400
gatgtgggtt ttactgatgc atatacatga tggcatatgc agcatctatt catatgctct 5460
aaccttgagt acctatctat tataataaac aagtatgttt tataattatt ttgatcttga 5520
tatacttgga tgatggcata tgcagcagct atatgtggat ttttttagcc ctgccttcat 5580
acgctattta tttgcttggt actgtttctt ttgtcgatgc tcaccctgtt gtttggtgtt 5640
acttctgcag gtcgacttta acttagccta ggatccacac gacaccatgt cccccgagcg 5700
ccgcccgtc gagatccgcc cggccaccgc cgccgacatg gccgccgtgt gcgacatcgt 5760
gaaccactac atcgagacct ccaccgtgaa cttccgcacc gagccgcaga ccccgcagga 5820
gtggatcgac gacctggagc gcctccagga ccgctacccg tggctcgtgg ccgaggtgga 5880
gggcgtggtg gccggcatcg cctacgccgg cccgtggaag gcccgcaacg cctacgactg 5940
gaccgtggag tccaccgtgt acgtgtccca ccgccaccag cgcctcggcc tcggctccac 6000
cctctacacc cacctcctca agagcatgga ggcccagggc ttcaagtccg tggtggccgt 6060
gatcggcctc ccgaacgacc cgtccgtgcg cctccacgag gccctcggct acaccgcccg 6120
cggcaccctc cgcgccgccg gctacaagca cggcggctgg cacgacgtcg gcttctggca 6180
gcgcgacttc gagctgccgg ccccgccgcg cccggtgcgc ccggtgacgc agatctgagt 6240
cgaaacctag acttgtccat cttctggatt ggccaactta attaatgtat gaaataaaag 6300
gatgcacaca tagtgacatg ctaatcacta taatgtgggc atcaaagttg tgtgttatgt 6360
gtaattacta gttatctgaa taaaagagaa agagatcatc catatttctt atcctaaatg 6420
aatgtcacgt gtctttataa ttctttgatg aaccagatgc atttcattaa ccaaatccat 6480
atacatataa atattaatca tatataatta atatcaattg ggttagcaaa acaaatctag 6540

tctaggtgtg ttttgcgaat tgcggccgcc accgcggtgg agctcgaatt cattccgatt 6600
aatcgtggcc tcttgctctt caggatgaag agctatgttt aaacgtgcaa gcgctactag 6660
acaattcagt acattaaaaa cgtccgcaat gtgttattaa gttgtctaag cgtcaatttg 6720
tttacaccac aatatatcct gccaccagcc agccaacagc tccccgaccg gcagctcggc 6780
acaaaatcac cactcgatac aggcagccca tcagtccggg acggcgtcag cgggagagcc 6840
gttgtaaggc ggcagacttt gctcatgtta ccgatgctat tcggaagaac ggcaactaag 6900
ctgccgggtt tgaaacacgg atgatctcgc ggagggtagc atgttgattg taacgatgac 6960
agagcgttgc tgcctgtgat caaatatcat ctccctcgca gagatccgaa ttatcagcct 7020
tcttattcat ttctcgctta accgtgacag gctgtcgatc ttgagaacta tgccgacata 7080
ataggaaatc gctggataaa gccgctgagg aagctgagtg gcgctatttc tttagaagtg 7140
aacgttgacg atcgtcgacc gtaccccgat gaattaattc ggacgtacgt tctgaacaca 7200
gctggatact tacttgggcg attgtcatac atgacatcaa caatgtaccc gtttgtgtaa 7260
ccgtctcttg gaggttcgta tgacactagt ggttcccctc agcttgcgac tagatgttga 7320
ggcctaacat tttattagag agcaggctag ttgcttagat acatgatctt caggccgtta 7380
tctgtcaggg caagcgaaaa ttggccattt atgacgacca atgccccgca gaagctccca 7440
tctttgccgc catagacgcc gcgccccct tttggggtgt agaacatcct tttgccagat 7500
gtggaaaaga agttcgttgt cccattgttg gcaatgacgt agtagccggc gaaagtgcga 7560
gacccatttg cgctatatat aagcctacga tttccgttgc gactattgtc gtaattggat 7620
gaactattat cgtagttgct ctcagagttg tcgtaatttg atggactatt gtcgtaattg 7680
cttatggagt tgtcgtagtt gcttggagaa atgtcgtagt tggatgggga gtagtcatag 7740
ggaagacgag cttcatccac taaaacaatt ggcaggtcag caagtgcctg ccccgatgcc 7800
atcgcaagta cgaggcttag aaccaccttc aacagatcgc gcatagtctt ccccagctct 7860
ctaacgcttg agttaagccg cgccgcgaag cggcgtcggc ttgaacgaat tgttagacat 7920
tatttgccga ctaccttggt gatctcgcct ttcacgtagt gaacaaattc ttccaactga 7980
tctgcgcgcg aggccaagcg atcttcttgt ccaagataag cctgcctagc ttcaagtatg 8040
acgggctgat actgggccgg caggcgctcc attgcccagt cggcagcgac atccttcggc 8100
gcgattttgc cggttactgc gctgtaccaa atgcgggaca acgtaagcac tacatttcgc 8160
tcatcgccag cccagtcggg cggcgagttc catagcgtta aggtttcatt tagcgcctca 8220
aatagatcct gttcaggaac cggatcaaag agttcctccg ccgctggacc taccaaggca 8280
acgctatgtt ctcttgcttt tgtcagcaag atagccagat caatgtcgat cgtggctggc 8340
tcgaagatac ctgcaagaat gtcattgcgc tgccattctc caaattgcag ttcgcgctta 8400
gctggataac gccacggaat gatgtcgtcg tgcacaacaa tggtgacttc tacagcgcgg 8460

agaatctcgc tctctccagg ggaagccgaa gtttccaaaa ggtcgttgat caaagctcgc 8520
cgcgttgttt catcaagcct tacagtcacc gtaaccagca aatcaatatc actgtgtggc 8580
ttcaggccgc catccactgc ggagccgtac aaatgtacgg ccagcaacgt cggttcgaga 8640
tggcgctcga tgacgccaac tacctctgat agttgagtcg atacttcggc gatcaccgct 8700
tccctcatga tgtttaactc ctgaattaag ccgcgccgcg aagcggtgtc ggcttgaatg 8760
aattgttagg cgtcatcctg tgctcccgag aaccagtacc agtacatcgc tgtttcgttc 8820
gagacttgag gtctagtttt atacgtgaac aggtcaatgc cgccgagagt aaagccacat 8880
tttgcgtaca aattgcaggc aggtacattg ttcgtttgtg tctctaatcg tatgccaagg 8940
agctgtctgc ttagtgccca ctttttcgca aattcgatga gactgtgcgc gactcctttg 9000
cctcggtgcg tgtgcgacac aacaatgtgt tcgatagagg ctagatcgtt ccatgttgag 9060
ttgagttcaa tcttcccgac aagctcttgg tcgatgaatg cgccatagca agcagagtct 9120
tcatcagagt catcatccga gatgtaatcc ttccggtagg ggctcacact tctggtagat 9180
agttcaaagc cttggtcgga taggtgcaca tcgaacactt cacgaacaat gaaatggttc 9240
tcagcatcca atgtttccgc cacctgctca gggatcaccg aaatcttcat atgacgccta 9300
acgcctggca cagcggatcg caaacctggc gcggcttttg gcacaaaagg cgtgacaggt 9360
ttgcgaatcc gttgctgcca cttgttaacc cttttgccag atttggtaac tataatttat 9420
gttagaggcg aagtcttggg taaaaactgg cctaaaattg ctggggattt caggaaagta 9480
aacatcacct tccggctcga tgtctattgt agatatatgt agtgtatcta cttgatcggg 9540
ggatctgctg cctcgcgcgt ttcggtgatg acggtgaaaa cctctgacac atgcagctcc 9600
cggagacggt cacagcttgt ctgtaagcgg atgccgggag cagacaagcc cgtcagggcg 9660
cgtcagcggg tgttggcggg tgtcggggcg cagccatgac ccagtcacgt agcgatagcg 9720
gagtgtatac tggcttaact atgcggcatc agagcagatt gtactgagag tgcaccatat 9780
gcggtgtgaa ataccgcaca gatgcgtaag gagaaaatac cgcatcaggc gctcttccgc 9840
ttcctcgctc actgactcgc tgcgctcggt cgttcggctg cggcgagcgg tatcagctca 9900
ctcaaaggcg gtaatacggt tatccacaga atcaggggat aacgcaggaa agaacatgtg 9960
agcaaaaggc cagcaaaagg ccaggaaccg taaaaaggcc gcgttgctgg cgtttttcca 10020
taggctccgc ccccctgacg agcatcacaa aaatcgacgc tcaagtcaga ggtggcgaaa 10080
cccgacagga ctataaagat accaggcgtt tccccctgga agctccctcg tgcgctctcc 10140
tgttccgacc ctgccgctta ccggatacct gtccgccttt ctcccttcgg gaagcgtggc 10200
gctttctcat agctcacgct gtaggtatct cagttcggtg taggtcgttc gctccaagct 10260
gggctgtgtg cacgaacccc ccgttcagcc cgaccgctgc gccttatccg gtaactatcg 10320
tcttgagtcc aacccggtaa gacacgactt atcgccactg gcagcagcca ctggtaacag 10380
gattagcaga gcgaggtatg taggcggtgc tacagagttc ttgaagtggt ggcctaacta 10440

cggctacact agaaggacag tatttggtat ctgcgctctg ctgaagccag ttaccttcgg 10500
aaaaagagtt ggtagctctt gatccggcaa acaaaccacc gctggtagcg gtggtttttt 10560
tgtttgcaag cagcagatta cgcgcagaaa aaaaggatct caagaagatc ctttgatctt 10620
ttctacgggg tctgacgctc agtggaacga aaactcacgt taagggattt tggtcatgag 10680
attatcaaaa aggatcttca cctagatcct tttaaattaa aaatgaagtt ttaaatcaat 10740
ctaaagtata tatgagtaaa cttggtctga cagttaccaa tgcttaatca gtgaggcacc 10800
tatctcagcg atctgtctat ttcgttcatc catagttgcc tgactccccg tcgtgtagat 10860
aactacgata cgggagggct taccatctgg ccccagtgct gcaatgatac cgcgagaccc 10920
acgctcaccg gctccagatt tatcagcaat aaaccagcca gccggaaggg ccgagcgcag 10980
aagtggtcct gcaactttat ccgcctccat ccagtctatt aattgttgcc gggaagctag 11040
agtaagtagt tcgccagtta atagtttgcg caacgttgtt gccattgctg cagggggggg 11100
gggggggggg gacttccatt gttcattcca cggacaaaaa cagagaaagg aaacgacaga 11160
ggccaaaaag cctcgctttc agcacctgtc gtttcctttc ttttcagagg gtattttaaa 11220
taaaaacatt aagttatgac gaagaagaac ggaaacgcct taaaccggaa aattttcata 11280
aatagcgaaa acccgcgagg tcgccgcccc gtaacctgtc ggatcaccgg aaaggacccg 11340
taaagtgata atgattatca tctacatatc acaacgtgcg tggaggccat caaaccacgt 11400
caaataatca attatgacgc aggtatcgta ttaattgatc tgcatcaact taacgtaaaa 11460
acaacttcag acaatacaaa tcagcgacac tgaatacggg gcaacctcat gtcccccccc 11520
cccccccccc tgcaggcatc gtggtgtcac gctcgtcgtt tggtatggct tcattcagct 11580
ccggttccca acgatcaagg cgagttacat gatcccccat gttgtgcaaa aaagcggtta 11640
gctccttcgg tcctccgatc gttgtcagaa gtaagttggc cgcagtgtta tcactcatgg 11700
ttatggcagc actgcataat tctcttactg tcatgccatc cgtaagatgc ttttctgtga 11760
ctggtgagta ctcaaccaag tcattctgag aatagtgtat gcggcgaccg agttgctctt 11820
gcccggcgtc aacacgggat aataccgcgc cacatagcag aactttaaaa gtgctcatca 11880
ttggaaaacg ttcttcgggg cgaaaactct caaggatctt accgctgttg agatccagtt 11940
cgatgtaacc cactcgtgca cccaactgat cttcagcatc ttttactttc accagcgttt 12000
ctgggtgagc aaaaacagga aggcaaaatg ccgcaaaaaa gggaataagg gcgacacgga 12060
aatgttgaat actcatactc ttcctttttc aatattattg aagcatttat cagggttatt 12120
gtctcatgag cggatacata tttgaatgta tttagaaaaa taaacaaata ggggttccgc 12180
gcacatttcc ccgaaaagtg ccacctgacg tctaagaaac cattattatc atgacattaa 12240
cctataaaaa taggcgtatc acgaggccct ttcgtcttca agaattggtc gacgatcttg 12300
ctgcgttcgg atattttcgt ggagttcccg ccacagaccc ggattgaagg cgagatccag 12360

caactcgcgc cagatcatcc tgtgacggaa ctttggcgcg tgatgactgg ccaggacgtc 12420

ggccgaaaga gcgacaagca gatcacgctt ttcgacagcg tcggatttgc gatcgaggat 12480

ttttcggcgc tgcgctacgt ccgcgaccgc gttgagggat caagccacag cagcccactc 12540

gaccttctag ccgacccaga cgagccaagg gatcttttg gaatgctgct ccgtcgtcag 12600

gctttccgac gtttgggtgg ttgaacagaa gtcattatcg tacggaatgc caagcactcc 12660

cgaggggaac cctgtggttg gcatgcacat acaaatggac gaacggataa acctttcac 12720

gcccttttaa atatccgtta ttctaataaa cgctcttttc tcttaggttt acccgccaat 12780

atatcctgtc aaacactgat agtttaaact gaaggcggga aacgacaatc tgatcatgag 12840

cggagaatta agggagtcac gttatgaccc ccgccgatga cgcgggacaa gccgttttac 12900

gtttggaact gacagaaccg caacgttgaa ggagccactc agcaagctgg tacgattgta 12960

atacgactca ctatagggcg aattgagcgc tgtttaaacg ctcttcaact ggaagagcg 13019

<210> 9
<211> 2991
<212> DNA
<213> artificial sequence

<220>
<223> vector

<400> 9
ctttcctgcg ttatcccctg attctgtgga taaccgtatt accgcctttg agtgagctga 60

taccgctcgc cgcagccgaa cgaccgagcg cagcgagtca gtgagcgagg aagcggaaga 120

gcgcccaata cgcaaaccgc ctctccccgc gcgttggccg attcattaat gcagctggca 180

cgacaggttt cccgactgga aagcgggcag tgagcgcaac gcaattaata cgcgtaccgc 240

tagccaggaa gagtttgtag aaacgcaaaa aggccatccg tcaggatggc cttctgctta 300

gtttgatgcc tggcagttta tggcgggcgt cctgcccgcc accctccggg ccgttgcttc 360

acaacgttca aatccgctcc cggcggattt gtcctactca ggagagcgtt caccgacaaa 420

caacagataa aacgaaaggc ccagtcttcc gactgagcct ttcgttttat ttgatgcctg 480

gcagttccct actctcgcgt taacgctagc atggatgttt tcccagtcac gacgttgtaa 540

aacgacggcc agtcttaagc tcgggccctg cagctctaga gctcgaattc tacaggtcac 600

taataccatc taagtagttg gttcatagtg actgcatatg ttgtgtttta cagtattatg 660

tagtctgttt tttatgcaaa atctaattta atatattgat atttatatca ttttacgttt 720

ctcgttcaac tttcttgtac aaagtggccg ttaacggatc cagacttgtc catcttctgg 780

attggccaac ttaattaatg tatgaaataa aaggatgcac acatagtgac atgctaatca 840

ctataatgtg ggcatcaaag ttgtgtgtta tgtgtaatta ctagttatct gaataaaaga 900

gaaagagatc atccatattt cttatcctaa atgaatgtca cgtgtcttta taattctttg 960

atgaaccaga tgcatttcat taaccaaatc catatacata taaatattaa tcatatataa 1020

ttaatatcaa ttgggttagc aaaacaaatc tagtctaggt gtgttttgcg aattgcggca 1080
agcttgcggc cgccccgggc aactttatta tacaaagttg gcattataaa aaagcattgc 1140
ttatcaattt gttgcaacga acaggtcact atcagtcaaa ataaaatcat tatttggagc 1200
tccatggtag cgttaacgcg gccgcgatat cccctatagt gagtcgtatt acatggtcat 1260
agctgtttcc tggcagctct ggcccgtgtc tcaaaatctc tgatgttaca ttgcacaaga 1320
taaaaatata tcatcatgaa caataaaact gtctgcttac ataaacagta atacaagggg 1380
tgttatgagc catattcaac gggaaacgtc gaggccgcga ttaaattcca acatggatgc 1440
tgatttatat gggtataaat gggctcgcga taatgtcggg caatcaggtg cgacaatcta 1500
tcgcttgtat gggaagcccg atgcgccaga gttgtttctg aaacatggca aaggtagcgt 1560
tgccaatgat gttacagatg agatggtcag actaaactgg ctgacggaat ttatgcctct 1620
tccgaccatc aagcatttta tccgtactcc tgatgatgca tggttactca ccactgcgat 1680
ccccggaaaa acagcattcc aggtattaga agaatatcct gattcaggtg aaaatattgt 1740
tgatgcgctg gcagtgttcc tgcgccggtt gcattcgatt cctgtttgta attgtccttt 1800
taacagcgat cgcgtatttc gtctcgctca ggcgcaatca cgaatgaata acggtttggt 1860
tgatgcgagt gattttgatg acgagcgtaa tggctggcct gttgaacaag tctggaaaga 1920
aatgcataaa cttttgccat tctcaccgga ttcagtcgtc actcatggtg atttctcact 1980
tgataacctt atttttgacg aggggaaatt aataggttgt attgatgttg gacgagtcgg 2040
aatcgcagac cgataccagg atcttgccat cctatggaac tgcctcggtg agttttctcc 2100
ttcattacag aaacggcttt ttcaaaaata tggtattgat aatcctgata tgaataaatt 2160
gcagtttcat ttgatgctcg atgagttttt ctaatcagaa ttggttaatt ggttgtaaca 2220
ctggcagagc attacgctga cttgacggga cggcgcaagc tcatgaccaa aatcccttaa 2280
cgtgagttac gcgtcgttcc actgagcgtc agaccccgta gaaaagatca aaggatcttc 2340
ttgagatcct ttttttctgc gcgtaatctg ctgcttgcaa acaaaaaaac caccgctacc 2400
agcggtggtt tgtttgccgg atcaagagct accaactctt tttccgaagg taactggctt 2460
cagcagagcg cagataccaa atactgtcct tctagtgtag ccgtagttag gccaccactt 2520
caagaactct gtagcaccgc ctacatacct cgctctgcta atcctgttac cagtggctgc 2580
tgccagtggc gataagtcgt gtcttaccgg gttggactca agacgatagt taccggataa 2640
ggcgcagcgg tcgggctgaa cggggggttc gtgcacacag cccagcttgg agcgaacgac 2700
ctacaccgaa ctgagatacc tacagcgtga gcattgagaa agcgccacgc ttcccgaagg 2760
gagaaaggcg gacaggtatc cggtaagcgg cagggtcgga acaggagagc gcacgaggga 2820
gcttccaggg ggaaacgcct ggtatcttta tagtcctgtc gggtttcgcc acctctgact 2880
tgagcgtcga tttttgtgat gctcgtcagg ggggcggagc ctatggaaaa acgccagcaa 2940

cgcggccttt ttacggttcc tggccttttg ctggcctttt gctcacatgt t 2991

<210> 10
<211> 13807
<212> DNA
<213> artificial sequence

<220>
<223> vector

<400> 10
aagctggtac gattgtaata cgactcacta tagggcgaat tgagcgctgt ttaaacgctc 60

ttcaactgga agagcggtta ccagagctgg tcacctttgt ccaccaagat ggaactgcgg 120

ccgctcatta attaagtcag gcgcgcctct agttgaagac acgttcatgt cttcatcgta 180

agaagacact cagtagtctt cggccagaat ggccgtaggt gaattaagag gagagaggag 240

gtaaacattt tcttctattt tttcatattt tcaggataaa ttattgtaaa agtttacaag 300

atttccattt gactagtgta aatgaggaat attctctagt aagatcatta tttcatctac 360

ttcttttatc ttctaccagt agaggaataa acaatattta gctcctttgt aaatacaaat 420

taattttcgt tcttgacatc attcaatttt aattttacgt ataaaataaa agatcatacc 480

tattagaacg attaaggaga aatacaattc gaatgagaag gatgtgccgt ttgttataat 540

aaacagccac acgacgtaaa cgtaaaatga ccacatgatg ggccaataga catggaccga 600

ctactaataa tagtaagtta cattttagga tggaataaat atcataccga catcagtttg 660

aaagaaaagg gaaaaaaaga aaaaataaat aaaagatata ctaccgacat gagttccaaa 720

aagcaaaaaa aaagatcaag ccgacacaga cacgcgtaga gagcaaaatg actttgacgt 780

cacaccacga aaacagacgc ttcatacgtg tccctttatc tctctcagtc tctctataaa 840

cttagtgaga ccctcctctg ttttactcag gatccccggg taccgagctc gaattcaccg 900

gtcgccacca tggcccacag caagcacggc ctgaaggagg agatgaccat gaagtaccac 960

atggagggct gcgtgaacgg ccacaagttc gtgatcaccg gcgagggcat cggctacccc 1020

ttcaagggca agcagaccat caacctgtgc gtgatcgagg gcggccccct gcccttcagc 1080

gaggacatcc tgagcgccgg cttcaagtac ggcgaccgga tcttcaccga gtacccccag 1140

gacatcgtgg actacttcaa gaacagctgc cccgccggct acacctgggg ccggagcttc 1200

ctgttcgagg acggcgccgt gtgcatctgt aacgtggaca tcaccgtgag cgtgaaggag 1260

aactgcatct accacaagag catcttcaac ggcgtgaact tccccgccga cggccccgtg 1320

atgaagaaga tgaccaccaa ctgggaggcc agctgcgaga agatcatgcc cgtgcctaag 1380

cagggcatcc tgaagggcga cgtgagcatg tacctgctgc tgaaggacgg cggccggtac 1440

cggtgccagt tcgacaccgt gtacaaggcc aagagcgtgc ccagcaagat gcccgagtgg 1500

cacttcatcc agcacaagct gctgcgggag gaccggagcg acgccaagaa ccagaagtgg 1560

cagctgaccg agcacgccat cgccttcccc agcgccctgg cctgaagcgg cccatggata 1620

ttcgaacgcg taggtaccac atggttaacc tagacttgtc catcttctgg attggccaac 1680
ttaattaatg tatgaaataa aaggatgcac acatagtgac atgctaatca ctataatgtg 1740
ggcatcaaag ttgtgtgtta tgtgtaatta ctagttatct gaataaaaga gaaagagatc 1800
atccatattt cttatcctaa atgaatgtca cgtgtcttta taattctttg atgaaccaga 1860
tgcatttcat taaccaaatc catatacata taaatattaa tcatatataa ttaatatcaa 1920
ttgggttagc aaaacaaatc tagtctaggt gtgttttgcg aatgcggcca ttggcctaga 1980
aggccattta aatcctgagg atctggtctt cctaaggacc cgggatatcg ctatcaactt 2040
tgtatagaaa agttgaacga gaaacgtaaa atgatataaa tatcaatata ttaaattaga 2100
ttttgcataa aaaacagact acataatact gtaaaacaca acatatccag tcactatggt 2160
cgacctgcag actggctgtg tataagggag cctgacattt atattcccca gaacatcagg 2220
ttaatggcgt ttttgatgtc attttcgcgg tggctgagat cagccacttc ttccccgata 2280
acggagaccg gcacactggc catatcggtg gtcatcatgc gccagctttc atccccgata 2340
tgcaccaccg ggtaaagttc acgggggact ttatctgaca gcagacgtgc actggccagg 2400
gggatcacca tccgtcgccc gggcgtgtca ataatatcac tctgtacatc cacaaacaga 2460
cgataacggc tctctctttt ataggtgtaa accttaaact gcatttcacc agcccctgtt 2520
ctcgtcggca aaagagccgt tcatttcaat aaaccgggcg acctcagcca tcccttcctg 2580
attttccgct ttccagcgtt cggcacgcag acgacgggct tcattctgca tggttgtgct 2640
taccgaaccg gagatattga catcatatat gccttgagca actgatagct gtcgctgtca 2700
actgtcactg taatacgctg cttcatagca tacctctttt tgacatactt cgggtataca 2760
tatcagtata tattcttata ccgcaaaaat cagcgcgcaa atacgcatac tgttatctgg 2820
cttttagtaa gccggatcct ctagattacg ccccgcctgc cactcatcgc agtactgttg 2880
taattcatta agcattctgc cgacatggaa gccatcacaa acggcatgat gaacctgaat 2940
cgccagcggc atcagcacct tgtcgccttg cgtataatat ttgcccatgg tgaaaacggg 3000
ggcgaagaag ttgtccatat tggccacgtt taaatcaaaa ctggtgaaac tcacccaggg 3060
attggctgag acgaaaaaca tattctcaat aaacccttta gggaaatagg ccaggttttc 3120
accgtaacac gccacatctt gcgaatatat gtgtagaaac tgccggaaat cgtcgtggta 3180
ttcactccag agcgatgaaa acgtttcagt ttgctcatgg aaaacggtgt aacaagggtg 3240
aacactatcc catatcacca gctcaccgtc tttcattgcc atacggaatt ccggatgagc 3300
attcatcagg cgggcaagaa tgtgaataaa ggccggataa aacttgtgct tatttttctt 3360
tacggtcttt aaaaaggccg taatatccag ctgaacggtc tggttatagg tacattgagc 3420
aactgactga aatgcctcaa aatgttcttt acgatgccat tgggatatat caacggtggt 3480
atatccagtg atttttttct ccatttagc ttccttagct cctgaaaatc tcgacggatc 3540

ctaactcaaa atccacacat tatacgagcc ggaagcataa agtgtaaagc ctggggtgcc 3600
ctaatgcggc cgccatagtg actggatatg ttgtgtttta cagtattatg tagtctgttt 3660
tttatgcaaa atctaattta atatattgat atttatatca ttttacgttt ctcgttcaac 3720
tttattatac aaagttgata gatatcggac cgattaaact ttaattcggt ccgaagcttg 3780
catgcctgca gtgcagcgtg acccggtcgt gcccctctct agagataatg agcattgcat 3840
gtctaagtta taaaaaatta ccacatattt tttttgtcac acttgtttga agtgcagttt 3900
atctatcttt atacatatat ttaaacttta ctctacgaat aatataatct atagtactac 3960
aataatatca gtgttttaga gaatcatata aatgaacagt tagacatggt ctaaaggaca 4020
attgagtatt ttgacaacag gactctacag ttttatcttt ttagtgtgca tgtgttctcc 4080
tttttttttg caaatagctt cacctatata atacttcatc cattttatta gtacatccat 4140
ttagggttta gggttaatgg tttttataga ctaatttttt tagtacatct attttattct 4200
attttagcct ctaaattaag aaaactaaaa ctctatttta gtttttttat ttaataattt 4260
agatataaaa tagaataaaa taaagtgact aaaaattaaa caaataccct ttaagaaatt 4320
aaaaaaacta aggaaacatt tttcttgttt cgagtagata atgccagcct gttaaacgcc 4380
gtcgacgagt ctaacggaca ccaaccagcg aaccagcagc gtcgcgtcgg gccaagcgaa 4440
gcagacggca cggcatctct gtcgctgcct ctggacccct ctcgagagtt ccgctccacc 4500
gttggacttg ctccgctgtc ggcatccaga aattgcgtgg cggagcggca gacgtgagcc 4560
ggcacggcag gcggcctcct cctcctctca cggcaccggc agctacgggg gattcctttc 4620
ccaccgctcc ttcgctttcc cttcctcgcc cgccgtaata aatagacacc ccctccacac 4680
cctctttccc caacctcgtg ttgttcggag cgcacacaca cacaaccaga tctcccccaa 4740
atccacccgt cggcacctcc gcttcaaggt acgccgctcg tcctcccccc ccccccctctc 4800
taccttctct agatcggcgt tccggtccat gcatggttag ggcccggtag ttctacttct 4860
gttcatgttt gtgttagatc cgtgtttgtg ttagatccgt gctgctagcg ttcgtacacg 4920
gatgcgacct gtacgtcaga cacgttctga ttgctaactt gccagtgttt ctctttgggg 4980
aatcctggga tggctctagc cgttccgcag acgggatcga tttcatgatt ttttttgttt 5040
cgttgcatag ggtttggttt gcccttttcc tttatttcaa tatatgccgt gcacttgttt 5100
gtcgggtcat cttttcatgc ttttttttgt cttggttgtg atgatgtggt ctggttgggc 5160
ggtcgttcta gatcggagta gaattctgtt tcaaactacc tggtggattt attaattttg 5220
gatctgtatg tgtgtgccat acatattcat agttacgaat tgaagatgat ggatggaaat 5280
atcgatctag gataggtata catgttgatg cgggttttac tgatgcatat acagagatgc 5340
tttttgttcg cttggttgtg atgatgtggt gtggttgggc ggtcgttcat tcgttctaga 5400
tcggagtaga atactgtttc aaactacctg gtgtatttat taattttgga actgtatgtg 5460
tgtgtcatac atcttcatag ttacgagttt aagatggatg gaaatatcga tctaggatag 5520

gtatacatgt tgatgtgggt tttactgatg catatacatg atggcatatg cagcatctat 5580
tcatatgctc taaccttgag tacctatcta ttataataaa caagtatgtt ttataattat 5640
tttgatcttg atatacttgg atgatggcat atgcagcagc tatatgtgga ttttttttagc 5700
cctgccttca tacgctattt atttgcttgg tactgtttct tttgtcgatg ctcaccctgt 5760
tgtttggtgt tacttctgca ggtcgacttt aacttagcct aggatccaca cgacaccatg 5820
tcccccgagc gccgccccgt cgagatccgc ccggccaccg ccgccgacat ggccgccgtg 5880
tgcgacatcg tgaaccacta catcgagacc tccaccgtga acttccgcac cgagccgcag 5940
accccgcagg agtggatcga cgacctggag cgcctccagg accgctaccc gtggctcgtg 6000
gccgaggtgg agggcgtggt ggccggcatc gcctacgccg gcccgtggaa ggcccgcaac 6060
gcctacgact ggaccgtgga gtccaccgtg tacgtgtccc accgccacca gcgcctcggc 6120
ctcggctcca ccctctacac ccacctcctc aagagcatgg aggcccaggg cttcaagtcc 6180
gtggtggccg tgatcggcct cccgaacgac ccgtccgtgc gcctccacga ggccctcggc 6240
tacaccgccc gcggcaccct ccgcgccgcc ggctacaagc acggcggctg gcacgacgtc 6300
ggcttctggc agcgcgactt cgagctgccg gccccgccgc gcccggtgcg cccggtgacg 6360
cagatctccg gtggaggcgg cagcggtggc ggaggctccg gaggcggtgg ctccatggcc 6420
tcctccgagg acgtcatcaa ggagttcatg cgcttcaagg tgcgcatgga gggctccgtg 6480
aacggccacg agttcgagat cgagggcgag ggcgagggcc gcccctacga gggcacccag 6540
accgccaagc tgaaggtgac caagggcggc cccctgccct tcgcctggga catcctgtcc 6600
ccccagttcc agtacggctc caaggtgtac gtgaagcacc ccgccgacat ccccgactac 6660
aagaagctgt ccttccccga gggcttcaag tgggagcgcg tgatgaactt cgaggacggc 6720
ggcgtggtga ccgtgaccca ggactcctcc ctgcaggacg gctccttcat ctacaaggtg 6780
aagttcatcg gcgtgaactt cccctccgac ggccccgtaa tgcagaagaa gactatgggc 6840
tgggaggcct ccaccgagcg cctgtacccc cgcgacggcg tgctgaaggg cgagatccac 6900
aaggccctga agctgaagga cggcggccac tacctggtgg agttcaagtc catctacatg 6960
gccaagaagc ccgtgcagct gcccggctac tactacgtgg actccaagct ggacatcacc 7020
tcccacaacg aggactacac catcgtggag cagtacgagc gcgccgaggg ccgccaccac 7080
ctgttcctgt agtcaggatc tgagtcgaaa cctagacttg tccatcttct ggattggcca 7140
acttaattaa tgtatgaaat aaaaggatgc acacatagtg acatgctaat cactataatg 7200
tgggcatcaa agttgtgtgt tatgtgtaat tactagttat ctgaataaaa gagaaagaga 7260
tcatccatat ttcttatcct aaatgaatgt cacgtgtctt tataattctt tgatgaacca 7320
gatgcatttc attaaccaaa tccatataca tataaatatt aatcatatat aattaatatc 7380
aattgggtta gcaaaacaaa tctagtctag gtgtgttttg cgaatgcggc cgccaccgcg 7440

gtggagctcg aattcattcc gattaatcgt ggcctcttgc tcttcaggat gaagagctat 7500
gtttaaacgt gcaagcgcta ctagacaatt cagtacatta aaaacgtccg caatgtgtta 7560
ttaagttgtc taagcgtcaa tttgtttaca ccacaatata tcctgccacc agccagccaa 7620
cagctccccg accggcagct cggcacaaaa tcaccactcg atacaggcag cccatcagtc 7680
cgggacggcg tcagcgggag agccgttgta aggcggcaga ctttgctcat gttaccgatg 7740
ctattcggaa gaacggcaac taagctgccg ggtttgaaac acggatgatc tcgcggaggg 7800
tagcatgttg attgtaacga tgacagagcg ttgctgcctg tgatcaaata tcatctccct 7860
cgcagagatc cgaattatca gccttcttat tcatttctcg cttaaccgtg acaggctgtc 7920
gatcttgaga actatgccga cataatagga aatcgctgga taaagccgct gaggaagctg 7980
agtggcgcta tttctttaga agtgaacgtt gacgatcgtc gaccgtaccc cgatgaatta 8040
attcggacgt acgttctgaa cacagctgga tacttacttg ggcgattgtc atacatgaca 8100
tcaacaatgt acccgtttgt gtaaccgtct cttggaggtt cgtatgacac tagtggttcc 8160
cctcagcttg cgactagatg ttgaggccta acattttatt agagagcagg ctagttgctt 8220
agatacatga tcttcaggcc gttatctgtc agggcaagcg aaaattggcc atttatgacg 8280
accaatgccc cgcagaagct cccatctttg ccgccataga cgccgcgccc cccttttggg 8340
gtgtagaaca tccttttgcc agatgtggaa aagaagttcg ttgtcccatt gttggcaatg 8400
acgtagtagc cggcgaaagt gcgagaccca tttgcgctat atataagcct acgatttccg 8460
ttgcgactat tgtcgtaatt ggatgaacta ttatcgtagt tgctctcaga gttgtcgtaa 8520
tttgatggac tattgtcgta attgcttatg gagttgtcgt agttgcttgg agaaatgtcg 8580
tagttggatg gggagtagtc atagggaaga cgagcttcat ccactaaaac aattggcagg 8640
tcagcaagtg cctgccccga tgccatcgca agtacgaggc ttagaaccac cttcaacaga 8700
tcgcgcatag tcttccccag ctctctaacg cttgagttaa gccgcgccgc gaagcggcgt 8760
cggcttgaac gaattgttag acattatttg ccgactacct tggtgatctc gcctttcacg 8820
tagtgaacaa attcttccaa ctgatctgcg cgcgaggcca agcgatcttc ttgtccaaga 8880
taagcctgcc tagcttcaag tatgacgggc tgatactggg ccggcaggcg ctccattgcc 8940
cagtcggcag cgacatcctt cggcgcgatt ttgccggtta ctgcgctgta ccaaatgcgg 9000
gacaacgtaa gcactacatt tcgctcatcg ccagcccagt cgggcggcga gttccatagc 9060
gttaaggttt catttagcgc ctcaaataga tcctgttcag gaaccggatc aaagagttcc 9120
tccgccgctg gacctaccaa ggcaacgcta tgttctcttg cttttgtcag caagatagcc 9180
agatcaatgt cgatcgtggc tggctcgaag atacctgcaa gaatgtcatt gcgctgccat 9240
tctccaaatt gcagttcgcg cttagctgga taacgccacg gaatgatgtc gtcgtgcaca 9300
acaatggtga cttctacagc gcggagaatc tcgctctctc caggggaagc cgaagtttcc 9360
aaaaggtcgt tgatcaaagc tcgccgcgtt gtttcatcaa gccttacagt caccgtaacc 9420

agcaaatcaa tatcactgtg tggcttcagg ccgccatcca ctgcggagcc gtacaaatgt 9480
acggccagca acgtcggttc gagatggcgc tcgatgacgc caactacctc tgatagttga 9540
gtcgatactt cggcgatcac cgcttccctc atgatgttta actcctgaat taagccgcgc 9600
cgcgaagcgg tgtcggcttg aatgaattgt taggcgtcat cctgtgctcc cgagaaccag 9660
taccagtaca tcgctgtttc gttcgagact tgaggtctag ttttatacgt gaacaggtca 9720
atgccgccga gagtaaagcc acattttgcg tacaaattgc aggcaggtac attgttcgtt 9780
tgtgtctcta atcgtatgcc aaggagctgt ctgcttagtg cccacttttt cgcaaattcg 9840
atgagactgt gcgcgactcc tttgcctcgg tgcgtgtgcg acacaacaat gtgttcgata 9900
gaggctagat cgttccatgt tgagttgagt tcaatcttcc cgacaagctc ttggtcgatg 9960
aatgcgccat agcaagcaga gtcttcatca gagtcatcat ccgagatgta atccttccgg 10020
taggggctca cacttctggt agatagttca aagccttggt cggataggtg cacatcgaac 10080
acttcacgaa caatgaaatg gttctcagca tccaatgttt ccgccacctg ctcagggatc 10140
accgaaatct tcatatgacg cctaacgcct ggcacagcgg atcgcaaacc tggcgcggct 10200
tttggcacaa aaggcgtgac aggtttgcga atccgttgct gccacttgtt aaccctttg 10260
ccagatttgg taactataat ttatgttaga ggcgaagtct tgggtaaaaa ctggcctaaa 10320
attgctgggg atttcaggaa agtaaacatc accttccggc tcgatgtcta ttgtagatat 10380
atgtagtgta tctacttgat cgggggatct gctgcctcgc gcgtttcggt gatgacggtg 10440
aaaacctctg acacatgcag ctcccggaga cggtcacagc ttgtctgtaa gcggatgccg 10500
ggagcagaca agcccgtcag ggcgcgtcag cgggtgttgg cgggtgtcgg ggcgcagcca 10560
tgacccagtc acgtagcgat agcggagtgt atactggctt aactatgcgg catcagagca 10620
gattgtactg agagtgcacc atatgcggtg tgaaataccg cacagatgcg taaggagaaa 10680
ataccgcatc aggcgctctt ccgcttcctc gctcactgac tcgctgcgct cggtcgttcg 10740
gctgcggcga gcggtatcag ctcactcaaa ggcggtaata cggttatcca cagaatcagg 10800
ggataacgca ggaaagaaca tgtgagcaaa aggccagcaa aaggccagga accgtaaaaa 10860
ggccgcgttg ctggcgtttt tccataggct ccgcccccct gacgagcatc acaaaaatcg 10920
acgctcaagt cagaggtggc gaaacccgac aggactataa agataccagg cgtttccccc 10980
tggaagctcc ctcgtgcgct ctcctgttcc gaccctgccg cttaccggat acctgtccgc 11040
ctttctccct tcgggaagcg tggcgctttc tcatagctca cgctgtaggt atctcagttc 11100
ggtgtaggtc gttcgctcca agctgggctg tgtgcacgaa ccccccgttc agcccgaccg 11160
ctgcgcctta tccggtaact atcgtcttga gtccaacccg gtaagacacg acttatcgcc 11220
actggcagca gccactggta acaggattag cagagcgagg tatgtaggcg gtgctacaga 11280
gttcttgaag tggtggccta actacggcta cactagaagg acagtatttg gtatctgcgc 11340

tctgctgaag ccagttacct tcggaaaaag agttggtagc tcttgatccg gcaaacaaac 11400
caccgctggt agcggtggtt tttttgtttg caagcagcag attacgcgca gaaaaaaagg 11460
atctcaagaa gatcctttga tcttttctac ggggtctgac gctcagtgga acgaaaactc 11520
acgttaaggg attttggtca tgagattatc aaaaaggatc ttcacctaga tccttttaaa 11580
ttaaaaatga agttttaaat caatctaaag tatatatgag taaacttggt ctgacagtta 11640
ccaatgctta atcagtgagg cacctatctc agcgatctgt ctatttcgtt catccatagt 11700
tgcctgactc cccgtcgtgt agataactac gatacgggag ggcttaccat ctggccccag 11760
tgctgcaatg ataccgcgag acccacgctc accggctcca gatttatcag caataaacca 11820
gccagccgga agggccgagc gcagaagtgg tcctgcaact ttatccgcct ccatccagtc 11880
tattaattgt tgccgggaag ctagagtaag tagttcgcca gttaatagtt tgcgcaacgt 11940
tgttgccatt gctgcagggg gggggggggg gggggacttc cattgttcat tccacggaca 12000
aaaacagaga aaggaaacga cagaggccaa aaagcctcgc tttcagcacc tgtcgtttcc 12060
tttcttttca gagggtattt taaataaaaa cattaagtta tgacgaagaa gaacggaaac 12120
gccttaaacc ggaaaatttt cataaatagc gaaaacccgc gaggtcgccg ccccgtaacc 12180
tgtcggatca ccggaaagga cccgtaaagt gataatgatt atcatctaca tatcacaacg 12240
tgcgtggagg ccatcaaacc acgtcaaata atcaattatg acgcaggtat cgtattaatt 12300
gatctgcatc aacttaacgt aaaaacaact tcagacaata caaatcagcg acactgaata 12360
cggggcaacc tcatgtcccc cccccccccc cccctgcagg catcgtggtg tcacgctcgt 12420
cgtttggtat ggcttcattc agctccggtt cccaacgatc aaggcgagtt acatgatccc 12480
ccatgttgtg caaaaaagcg gttagctcct tcggtcctcc gatcgttgtc agaagtaagt 12540
tggccgcagt gttatcactc atggttatgg cagcactgca taattctctt actgtcatgc 12600
catccgtaag atgcttttct gtgactggtg agtactcaac caagtcattc tgagaatagt 12660
gtatgcggcg accgagttgc tcttgcccgg cgtcaacacg ggataatacc gcgccacata 12720
gcagaacttt aaaagtgctc atcattggaa aacgttcttc ggggcgaaaa ctctcaagga 12780
tcttaccgct gttgagatcc agttcgatgt aacccactcg tgcacccaac tgatcttcag 12840
catcttttac tttcaccagc gtttctgggt gagcaaaaac aggaaggcaa aatgccgcaa 12900
aaaagggaat aagggcgaca cggaaatgtt gaatactcat actcttcctt tttcaatatt 12960
attgaagcat ttatcagggt tattgtctca tgagcggata catatttgaa tgtatttaga 13020
aaaataaaca aataggggtt ccgcgcacat ttccccgaaa agtgccacct gacgtctaag 13080
aaaccattat tatcatgaca ttaacctata aaaataggcg tatcacgagg ccctttcgtc 13140
ttcaagaatt ggtcgacgat cttgctgcgt tcggatattt tcgtggagtt cccgccacag 13200
acccggattg aaggcgagat ccagcaactc gcgccagatc atcctgtgac ggaactttgg 13260
cgcgtgatga ctggccagga cgtcggccga aagagcgaca agcagatcac gcttttcgac 13320

agcgtcggat ttgcgatcga ggatttttcg gcgctgcgct acgtccgcga ccgcgttgag 13380

ggatcaagcc acagcagccc actcgacctt ctagccgacc cagacgagcc aagggatctt 13440

tttggaatgc tgctccgtcg tcaggctttc cgacgtttgg gtggttgaac agaagtcatt 13500

atcgtacgga atgccaagca ctcccgaggg gaaccctgtg gttggcatgc acatacaaat 13560

ggacgaacgg ataaaccttt tcacgccctt ttaaatatcc gttattctaa taaacgctct 13620

tttctcttag gtttacccgc caatatatcc tgtcaaacac tgatagttta aactgaaggc 13680

gggaaacgac aatctgatca tgagcggaga attaagggag tcacgttatg acccccgccg 13740

atgacgcggg acaagccgtt ttacgtttgg aactgacaga accgcaacgt tgaaggagcc 13800

actcagc 13807

<210> 11
<211> 4678
<212> DNA
<213> artificial sequence

<220>
<223> vector

<400> 11
gaaaggccca gtcttccgac tgagcctttc gttttatttg atgcctggca gttccctact 60

ctcgcgttaa cgctagcatg gatgttttcc cagtcacgac gttgtaaaac gacggccagt 120

cttaagctcg ggcccgcgtt aacgctacca tggagctcca aataatgatt ttattttgac 180

tgatagtgac ctgttcgttg caacaaattg ataagcaatg ctttttttata atgccaactt 240

tgtatagaaa agttgggccg aattcgagct cggtacggcc agaatggccc ggaccgggtt 300

accgaattcg agctcggtac cctgggatcc ctggtaatta ttggctgtag gattctaaac 360

agagcctaaa tagctggaat agctctagcc ctcaatccaa actaatgata tctatactta 420

tgcaactcta aatttttatt ctaaaagtaa tatttcattt ttgtcaacga gattctctac 480

tctattccac aatcttttga agcaatattt accttaaatc tgtactctat accaataatc 540

atatattcta ttatttattt ttatctctct cctaaggagc atccccctat gtctgcatgg 600

cccccgcctc gggtcccaat ctcttgctct gctagtagca cagaagaaaa cactagaaat 660

gacttgcttg acttagagta tcagataaac atcatgttta cttaacttta atttgtatcg 720

gtttctacta tttttataat atttttgtct ctatagatac tacgtgcaac agtataatca 780

acctagttta atccagagcg aaggattttt tactaagtac gtgactccat atgcacagcg 840

ttccttttat ggttcctcac tgggcacagc ataaacgaac cctgtccaat gttttcagcg 900

cgaacaaaca gaaattccat cagcgaacaa acaacataca tgcgagatga aaataaataa 960

taaaaaaagc tccgtctcga taggccggca cgaatcgaga gcctccatag ccagtttttt 1020

ccatcggaac ggcggttcgc gcacctaatt atatgcacca cacgcctata aagccaacca 1080

acccgtcgga ggggcgcaag ccagacagaa gacagcccgt cagcccctct cgtttttcat 1140
ccgccttcgc ctccaaccgc gtgcgctcca cgcctcctcc aggaaagcga ggatctcccc 1200
caaatccacc cgtcggcacc tccgcttcaa ggtacgccgc tcgtcctccc cccccccccc 1260
tctctacctt ctctagatcg gcgttccggt ccatggttag ggcccggtag ttctacttct 1320
gttcatgttt gtgttagatc cgtgtttgtg ttagatccgt gctgctagcg ttcgtacacg 1380
gatgcgacct gtacgtcaga cacgttctga ttgctaactt gccagtgttt ctctttgggg 1440
aatcctggga tggctctagc cgttccgcag acgggatcga tttcatgatt ttttttgttt 1500
cgttgcatag ggtttggttt gcccttttcc tttatttcaa tatatgccgt gcacttgttt 1560
gtcgggtcat cttttcatgc ttttttttgt cttggttgtg atgatgtggt ctggttgggc 1620
ggtcgttcta gatcggagta gaattctgtt tcaaactacc tggtggattt attaattttg 1680
gatctgtatg tgtgtgccat acatattcat agttacgaat tgaagatgat ggatggaaat 1740
atcgatctag gataggtata catgttgatg cgggttttac tgatgcatat acagagatgc 1800
tttttgttcg cttggttgtg atgatgtggt gtggttgggc ggtcgttcat tcgttctaga 1860
tcggagtaga atactgtttc aaactacctg gtgtatttat taattttgga actgtatgtg 1920
tgtgtcatac atcttcatag ttacgagttt aagatggatg gaaatatcga tctaggatag 1980
gtatacatgt tgatgtgggt tttactgatg catatacatg atggcatatg cagcatctat 2040
tcatatgctc taaccttgag tacctatcta ttataataaa caagtatgtt ttataattat 2100
tttgatcttg atatacttgg atgatggcat atgcagcagc tatatgtgga tttttttagc 2160
cctgccttca tacgctattt atttgcttgg tactgtttct tttgtcgatg ctcaccctgt 2220
tgtttggtgt tacttctgca ggtcgactct agaagcttgg tcacccggtc cgggcctaga 2280
aggccagctt caagtttgta caaaaaagtt gaacgagaaa cgtaaaatga tataaatatc 2340
aatatattaa attagatttt gcataaaaaa cagactacat aatactgtaa aacacaacat 2400
atgcagtcac tatgaatcaa ctacttagat ggtattagtg acctgtagaa ttcgagctct 2460
agagctgcag ggcggccgcg atatccccta tagtgagtcg tattacatgg tcatagctgt 2520
ttcctggcag ctctggcccg tgtctcaaaa tctctgatgt tacattgcac aagataaaaa 2580
tatatcatca tgaacaataa aactgtctgc ttacataaac agtaatacaa ggggtgttat 2640
gagccatatt caacgggaaa cgtcgaggcc gcgattaaat tccaacatgg atgctgattt 2700
atatgggtat aaatgggctc gcgataatgt cgggcaatca ggtgcgacaa tctatcgctt 2760
gtatgggaag cccgatgcgc cagagttgtt tctgaaacat ggcaaaggta gcgttgccaa 2820
tgatgttaca gatgagatgg tcagactaaa ctggctgacg gaatttatgc ctcttccgac 2880
catcaagcat tttatccgta ctcctgatga tgcatggtta ctcaccactg cgatccccgg 2940
aaaaacagca ttccaggtat tagaagaata tcctgattca ggtgaaaata ttgttgatgc 3000
gctggcagtg ttcctgcgcc ggttgcattc gattcctgtt tgtaattgtc cttttaacag 3060

cgatcgcgta tttcgtctcg ctcaggcgca atcacgaatg aataacggtt tggttgatgc 3120

gagtgatttt gatgacgagc gtaatggctg gcctgttgaa caagtctgga aagaaatgca 3180

taaacttttg ccattctcac cggattcagt cgtcactcat ggtgatttct cacttgataa 3240

ccttattttt gacgagggga aattaatagg ttgtattgat gttggacgag tcggaatcgc 3300

agaccgatac caggatcttg ccatcctatg gaactgcctc ggtgagtttt ctccttcatt 3360

acagaaacgg ctttttcaaa aatatggtat tgataatcct gatatgaata aattgcagtt 3420

tcatttgatg ctcgatgagt ttttctaatc agaattggtt aattggttgt aacactggca 3480

gagcattacg ctgacttgac gggacggcgc aagctcatga ccaaaatccc ttaacgtgag 3540

ttacgcgtcg ttccactgag cgtcagaccc cgtagaaaag atcaaaggat cttcttgaga 3600

tcctttttt ctgcgcgtaa tctgctgctt gcaaacaaaa aaaccaccgc taccagcggt 3660

ggtttgtttg ccggatcaag agctaccaac tctttttccg aaggtaactg gcttcagcag 3720

agcgcagata ccaaatactg tccttctagt gtagccgtag ttaggccacc acttcaagaa 3780

ctctgtagca ccgcctacat acctcgctct gctaatcctg ttaccagtgg ctgctgccag 3840

tggcgataag tcgtgtctta ccgggttgga ctcaagacga tagttaccgg ataaggcgca 3900

gcggtcgggc tgaacggggg gttcgtgcac acagcccagc ttggagcgaa cgacctacac 3960

cgaactgaga tacctacagc gtgagcattg agaaagcgcc acgcttcccg aagggagaaa 4020

ggcggacagg tatccggtaa gcggcagggt cggaacagga gagcgcacga gggagcttcc 4080

agggggaaac gcctggtatc tttatagtcc tgtcgggttt cgccacctct gacttgagcg 4140

tcgatttttg tgatgctcgt caggggggcg gagcctatgg aaaaacgcca gcaacgcggc 4200

ctttttacgg ttcctggcct tttgctggcc ttttgctcac atgttctttc ctgcgttatc 4260

ccctgattct gtggataacc gtattaccgc ctttgagtga gctgataccg ctcgccgcag 4320

ccgaacgacc gagcgcagcg agtcagtgag cgaggaagcg gaagagcgcc caatacgcaa 4380

accgcctctc cccgcgcgtt ggccgattca ttaatgcagc tggcacgaca ggtttcccga 4440

ctggaaagcg ggcagtgagc gcaacgcaat taatacgcgt accgctagcc aggaagagtt 4500

tgtagaaacg caaaaaggcc atccgtcagg atggccttct gcttagtttg atgcctggca 4560

gtttatggcg ggcgtcctgc ccgccaccct ccgggccgtt gcttcacaac gttcaaatcc 4620

gctcccggcg gatttgtcct actcaggaga gcgttcaccg acaaacaaca gataaaac 4678

<210> 12
<211> 3505
<212> DNA
<213> artificial sequence

<220>
<223> vector

<400> 12

gatccccggg taccgagctc gaattcggcc caagtttgta caaaaaagtt gaacgagaaa 60
cgtaaaatga tataaatatc aatatattaa attagatttt gcataaaaaa cagactacat 120
aatactgtaa aacacaacat atgcagtcac tatgaatcaa ctacttagat ggtattagtg 180
acctgtagaa ttcgagctct agagctgcag ggcggccgcg atatccccta tagtgagtcg 240
tattacatgg tcatagctgt ttcctggcag ctctggcccg tgtctcaaaa tctctgatgt 300
tacattgcac aagataaaaa tatatcatca tgaacaataa aactgtctgc ttacataaac 360
agtaatacaa ggggtgttat gagccatatt caacgggaaa cgtcgaggcc gcgattaaat 420
tccaacatgg atgctgattt atatgggtat aaatgggctc gcgataatgt cgggcaatca 480
ggtgcgacaa tctatcgctt gtatgggaag cccgatgcgc cagagttgtt tctgaaacat 540
ggcaaaggta gcgttgccaa tgatgttaca gatgagatgg tcagactaaa ctggctgacg 600
gaatttatgc ctcttccgac catcaagcat tttatccgta ctcctgatga tgcatggtta 660
ctcaccactg cgatccccgg aaaaacagca ttccaggtat tagaagaata tcctgattca 720
ggtgaaaata ttgttgatgc gctggcagtg ttcctgcgcc ggttgcattc gattcctgtt 780
tgtaattgtc cttttaacag cgatcgcgta tttcgtctcg ctcaggcgca atcacgaatg 840
aataacggtt tggttgatgc gagtgatttt gatgacgagc gtaatggctg gcctgttgaa 900
caagtctgga aagaaatgca taaacttttg ccattctcac cggattcagt cgtcactcat 960
ggtgatttct cacttgataa ccttattttt gacgagggga aattaatagg ttgtattgat 1020
gttggacgag tcggaatcgc agaccgatac caggatcttg ccatcctatg gaactgcctc 1080
ggtgagtttt ctccttcatt acagaaacgg ctttttcaaa aatatggtat tgataatcct 1140
gatatgaata aattgcagtt tcatttgatg ctcgatgagt ttttctaatc agaattggtt 1200
aattggttgt aacactggca gagcattacg ctgacttgac gggacggcgc aagctcatga 1260
ccaaaatccc ttaacgtgag ttacgcgtcg ttccactgag cgtcagaccc cgtagaaaag 1320
atcaaaggat cttcttgaga tccttttttt ctgcgcgtaa tctgctgctt gcaaacaaaa 1380
aaaccaccgc taccagcggt ggtttgtttg ccggatcaag agctaccaac tctttttccg 1440
aaggtaactg gcttcagcag agcgcagata ccaaatactg tccttctagt gtagccgtag 1500
ttaggccacc acttcaagaa ctctgtagca ccgcctacat acctcgctct gctaatcctg 1560
ttaccagtgg ctgctgccag tggcgataag tcgtgtctta ccgggttgga ctcaagacga 1620
tagttaccgg ataaggcgca gcggtcgggc tgaacggggg gttcgtgcac acagcccagc 1680
ttggagcgaa cgacctacac cgaactgaga tacctacagc gtgagcattg agaaagcgcc 1740
acgcttcccg aagggagaaa ggcggacagg tatccggtaa gcggcagggt cggaacagga 1800
gagcgcacga gggagcttcc agggggaaac gcctggtatc tttatagtcc tgtcgggttt 1860
cgccacctct gacttgagcg tcgatttttg tgatgctcgt caggggggcg gagcctatgg 1920
aaaaacgcca gcaacgcggc ctttttacgg ttcctggcct tttgctggcc ttttgctcac 1980

atgttctttc ctgcgttatc ccctgattct gtggataacc gtattaccgc ctttgagtga 2040

gctgataccg ctcgccgcag ccgaacgacc gagcgcagcg agtcagtgag cgaggaagcg 2100

gaagagcgcc caatacgcaa accgcctctc cccgcgcgtt ggccgattca ttaatgcagc 2160

tggcacgaca ggtttcccga ctggaaagcg ggcagtgagc gcaacgcaat taatacgcgt 2220

accgctagcc aggaagagtt tgtagaaacg caaaaaggcc atccgtcagg atggccttct 2280

gcttagtttg atgcctggca gtttatggcg ggcgtcctgc ccgccaccct ccgggccgtt 2340

gcttcacaac gttcaaatcc gctcccggcg gatttgtcct actcaggaga gcgttcaccg 2400

acaaacaaca gataaaacga aaggcccagt cttccgactg agcctttcgt tttatttgat 2460

gcctggcagt tccctactct cgcgttaacg ctagcatgga tgttttccca gtcacgacgt 2520

tgtaaaacga cggccagtct taagctcggg cccgcgttaa cgctaccatg gagctccaaa 2580

taatgatttt attttgactg atagtgacct gttcgttgca acaaattgat aagcaatgct 2640

tttttataat gccaactttg tatagaaaag ttgaagctta aatccttaca gaattgctgt 2700

agtttcatag tgctagatgt ggacagcaaa gcgccgctgt atgcttctgc ttttcttttt 2760

tggtgtgtgt agccacatcc tttgttcctg cccggcgcca tcccacttgg ttgttttttt 2820

ttatgattga aagccttcat gcttcctcgg tcaatcaccg gtgcgcactg ggagcatcgc 2880

cggaaaaaaa attcttcggc taagagtaac ttctttctcc ttttcttctc tgatctcgcg 2940

agcagtgctg ataacgtgtt gtaatctact tagcggtaac gagattgaga gagacaaaat 3000

gacagaacta ttgtctttat tgcagagtgt catgtattta tacaggggat acaaagtctc 3060

ccaaggggtg tgtcccttgg gagtaactgc cagttgatca caggacaata ttttgtaaca 3120

aaacgtacac atcgtcaaaa tagcgaggca tgaaactggc cttggccatg gacgcgtgaa 3180

gcgcgccatg cgttggatat gtggtcaata agtatataca atacaatgtt taacagagct 3240

gatagtactg ctttggcaca tttttgtcca cgcttcatga gagataaaac acctgcacgt 3300

aaattcacat gctgcactga aggcccgatc actgaggagc gaactgccgt aactcccttc 3360

tatatatacc cccagtccct gtttcagttt tcgtcaagct agcagcacca agttgtcgat 3420

cacttgcctg ctcttgagct cgattaagct atcatcagct acagcatccg atcccaaact 3480

gcaactgtag cagcgacaac tgccg 3505

<210> 13
<211> 49765
<212> DNA
<213> artificial sequence

<220>
<223> vector

<400> 13
gggggggggg ggggggggtt ccattgttca ttccacggac aaaaacagag aaaggaaacg 60

acagaggcca aaaagctcgc tttcagcacc tgtcgtttcc tttcttttca gagggtattt 120
taaataaaaa cattaagtta tgacgaagaa gaacggaaac gccttaaacc ggaaaatttt 180
cataaatagc gaaaacccgc gaggtcgccg ccccgtaacc tgtcggatca ccggaaagga 240
cccgtaaagt gataatgatt atcatctaca tatcacaacg tgcgtggagg ccatcaaacc 300
acgtcaaata atcaattatg acgcaggtat cgtattaatt gatctgcatc aacttaacgt 360
aaaaacaact tcagacaata caaatcagcg acactgaata cggggcaacc tcatgtcccc 420
cccccccccc cccctgcagg catcgtggtg tcacgctcgt cgtttggtat ggcttcattc 480
agctccggtt cccaacgatc aaggcgagtt acatgatccc ccatgttgtg caaaaaagcg 540
gttagctcct tcggtcctcc gatcgttgtc agaagtaagt tggccgcagt gttatcactc 600
atggttatgg cagcactgca taattctctt actgtcatgc catccgtaag atgcttttct 660
gtgactggtg agtactcaac caagtcattc tgagaatagt gtatgcggcg accgagttgc 720
tcttgcccgg cgtcaacacg ggataatacc gcgccacata gcagaacttt aaaagtgctc 780
atcattggaa aacgttcttc ggggcgaaaa ctctcaagga tcttaccgct gttgagatcc 840
agttcgatgt aacccactcg tgcacccaac tgatcttcag catcttttac tttcaccagc 900
gtttctgggt gagcaaaaac aggaaggcaa aatgccgcaa aaaagggaat aagggcgaca 960
cggaaatgtt gaatactcat actcttcctt tttcaatatt attgaagcat ttatcagggt 1020
tattgtctca tgagcggata catatttgaa tgtatttaga aaaataaaca aataggggtt 1080
ccgcgcacat ttccccgaaa agtgccacct gacgtctaag aaaccattat tatcatgaca 1140
ttaacctata aaaataggcg tatcacgagg ccctttcgtc ttcaagaatt cggagctttt 1200
gccattctca ccggattcag tcgtcactca tggtgatttc tcacttgata accttatttt 1260
tgacgagggg aaattaatag gttgtattga tgttggacga gtcggaatcg cagaccgata 1320
ccaggatctt gccatcctat ggaactgcct cggtgagttt tctccttcat tacagaaacg 1380
gctttttcaa aaatatggta ttgataatcc tgatatgaat aaattgcagt ttcatttgat 1440
gctcgatgag tttttctaat cagaattggt taattggttg taacactggc agagcattac 1500
gctgacttga cgggacggcg gctttgttga ataaatcgaa cttttgctga gttgaaggat 1560
cagatcacgc atcttcccga caacgcagac cgttccgtgg caaagcaaaa gttcaaaatc 1620
accaactggt ccacctacaa caaagctctc atcaaccgtg gctccctcac tttctggctg 1680
gatgatgggg cgattcaggc ctggtatgag tcagcaacac cttcttcacg aggcagacct 1740
cagcgccaga aggccgccag agaggccgag cgcggccgtg aggcttggac gctagggcag 1800
ggcatgaaaa agcccgtagc gggctgctac gggcgtctga cgcggtggaa agggggaggg 1860
gatgttgtct acatggctct gctgtagtga gtgggttgcg ctccggcagc ggtcctgatc 1920
aatcgtcacc ctttctcggt ccttcaacgt tcctgacaac gagcctcctt ttcgccaatc 1980
catcgacaat caccgcgagt ccctgctcga acgctgcgtc cggaccggct tcgtcgaagg 2040

cgtctatcgc ggcccgcaac agcggcgaga gcggagcctg ttcaacggtg ccgccgcgct 2100
cgccggcatc gctgtcgccg gcctgctcct caagcacggc cccaacagtg aagtagctga 2160
ttgtcatcag cgcattgacg gcgtccccgg ccgaaaaacc cgcctcgcag aggaagcgaa 2220
gctgcgcgtc ggccgtttcc atctgcggtg cgcccggtcg cgtgccggca tggatgcgcg 2280
cgccatcgcg gtaggcgagc agcgcctgcc tgaagctgcg ggcattcccg atcagaaatg 2340
agcgccagtc gtcgtcggct ctcggcaccg aatgcgtatg attctccgcc agcatggctt 2400
cggccagtgc gtcgagcagc gcccgcttgt tcctgaagtg ccagtaaagc gccggctgct 2460
gaacccccaa ccgttccgcc agtttgcgtg tcgtcagacc gtctacgccg acctcgttca 2520
acaggtccag ggcggcacgg atcactgtat tcggctgcaa ctttgtcatg cttgacactt 2580
tatcactgat aaacataata tgtccaccaa cttatcagtg ataaagaatc cgcgcgttca 2640
atcggaccag cggaggctgg tccggaggcc agacgtgaaa cccaacatac ccctgatcgt 2700
aattctgagc actgtcgcgc tcgacgctgt cggcatcggc ctgattatgc cggtgctgcc 2760
gggcctcctg cgcgatctgg ttcactcgaa cgacgtcacc gcccactatg gcattctgct 2820
ggcgctgtat gcgttggtgc aatttgcctg cgcacctgtg ctgggcgcgc tgtcggatcg 2880
tttcgggcgg cggccaatct tgctcgtctc gctggccggc gccactgtcg actacgccat 2940
catggcgaca gcgcctttcc tttgggttct ctatatcggg cggatcgtgg ccggcatcac 3000
cggggcgact ggggcggtag ccggcgctta tattgccgat atcactgatg gcgatgagcg 3060
cgcgcggcac ttcggcttca tgagcgcctg tttcgggttc gggatggtcg cgggacctgt 3120
gctcggtggg ctgatgggcg gtttctcccc ccacgctccg ttcttcgccg cggcagcctt 3180
gaacggcctc aatttcctga cgggctgttt ccttttgccg gagtcgcaca aaggcgaacg 3240
ccggccgtta cgccgggagg ctctcaaccc gctcgcttcg ttccggtggg cccggggcat 3300
gaccgtcgtc gccgccctga tggcggtctt cttcatcatg caacttgtcg gacaggtgcc 3360
ggccgcgctt tgggtcattt tcggcgagga tcgctttcac tgggacgcga ccacgatcgg 3420
catttcgctt gccgcatttg gcattctgca ttcactcgcc caggcaatga tcaccggccc 3480
tgtagccgcc cggctcggcg aaaggcgggc actcatgctc ggaatgattg ccgacggcac 3540
aggctacatc ctgcttgcct tcgcgacacg ggatggatg gcgttcccga tcatggtcct 3600
gcttgcttcg ggtggcatcg gaatgccggc gctgcaagca atgttgtcca ggcaggtgga 3660
tgaggaacgt caggggcagc tgcaaggctc actggcggcg ctcaccagcc tgacctcgat 3720
cgtcggaccc ctcctcttca cggcgatcta tgcggcttct ataacaacgt ggaacgggtg 3780
ggcatggatt gcaggcgctg ccctctactt gctctgcctg ccggcgctgc gtcgcgggct 3840
ttggagcggc gcagggcaac gagccgatcg ctgatcgtgg aaacgatagg cctatgccat 3900
gcgggtcaag gcgacttccg gcaagctata cgcgccctag gagtgcggtt ggaacgttgg 3960

cccagccaga tactcccgat cacgagcagg acgccgatga tttgaagcgc actcagcgtc 4020
tgatccaaga acaaccatcc tagcaacacg gcggtccccg ggctgagaaa gcccagtaag 4080
gaaacaactg taggttcgag tcgcgagatc cccccggaacc aaaggaagta ggttaaaccc 4140
gctccgatca ggccgagcca cgccaggccg agaacattgg ttcctgtagg catcgggatt 4200
ggcggatcaa acactaaagc tactggaacg agcagaagtc ctccggccgc cagttgccag 4260
gcggtaaagg tgagcagagg cacgggaggt tgccacttgc gggtcagcac ggttccgaac 4320
gccatggaaa ccgcccccgc caggcccgct gcgacgccga caggatctag cgctgcgttt 4380
ggtgtcaaca ccaacagcgc cacgcccgca gttccgcaaa tagcccccag gaccgccatc 4440
aatcgtatcg ggctacctag cagagcggca gagatgaaca cgaccatcag cggctgcaca 4500
gcgcctaccg tcgccgcgac cccgcccggc aggcggtaga ccgaaataaa caacaagctc 4560
cagaatagcg aaatattaag tgcgccgagg atgaagatgc gcatccacca gattcccgtt 4620
ggaatctgtc ggacgatcat cacgagcaat aaacccgccg gcaacgcccg cagcagcata 4680
ccggcgaccc ctcggcctcg ctgttcgggc tccacgaaaa cgccggacag atgcgccttg 4740
tgagcgtcct tggggccgtc ctcctgtttg aagaccgaca gcccaatgat ctcgccgtcg 4800
atgtaggcgc cgaatgccac ggcatctcgc aaccgttcag cgaacgcctc catgggcttt 4860
ttctcctcgt gctcgtaaac ggacccgaac atctctggag ctttcttcag ggccgacaat 4920
cggatctcgc ggaaatcctg cacgtcggcc gctccaagcc gtcgaatctg agccttaatc 4980
acaattgtca attttaatcc tctgtttatc ggcagttcgt agagcgcgcc gtgcgtcccg 5040
agcgatactg agcgaagcaa gtgcgtcgag cagtgcccgc ttgttcctga aatgccagta 5100
aagcgctggc tgctgaaccc ccagccggaa ctgaccccac aaggccctag cgtttgcaat 5160
gcaccaggtc atcattgacc caggcgtgtt ccaccaggcc gctgcctcgc aactcttcgc 5220
aggcttcgcc gacctgctcg cgccacttct tcacgcgggt ggaatccgat ccgcacatga 5280
ggcggaaggt ttccagcttg agcgggtacg gctcccggtg cgagctgaaa tagtcgaaca 5340
tccgtcgggc cgtcggcgac agcttgcggt acttctccca tatgaatttc gtgtagtggt 5400
cgccagcaaa cagcacgacg atttcctcgt cgatcaggac ctggcaacgg gacgttttct 5460
tgccacggtc caggacgcgg aagcggtgca gcagcgacac cgattccagg tgcccaacgc 5520
ggtcggacgt gaagcccatc gccgtcgcct gtaggcgcga caggcattcc tcggccttcg 5580
tgtaataccg gccattgatc gaccagccca ggtcctggca aagctcgtag aacgtgaagg 5640
tgatcggctc gccgataggg gtgcgcttcg cgtactccaa cacctgctgc cacaccagtt 5700
cgtcatcgtc ggcccgcagc tcgacgccgg tgtaggtgat cttcacgtcc ttgttgacgt 5760
ggaaaatgac cttgttttgc agcgcctcgc gcgggatttt cttgttgcgc gtggtgaaca 5820
gggcagagcg ggccgtgtcg tttggcatcg ctcgcatcgt gtccggccac ggcgcaatat 5880
cgaacaagga aagctgcatt tccttgatct gctgcttcgt gtgtttcagc aacgcggcct 5940

gcttggcctc gctgacctgt tttgccaggt cctcgccggc ggtttttcgc ttcttggtcg 6000
tcatagttcc tcgcgtgtcg atggtcatcg acttcgccaa acctgccgcc tcctgttcga 6060
gacgacgcga acgctccacg gcggccgatg gcgcgggcag ggcaggggga gccagttgca 6120
cgctgtcgcg ctcgatcttg gccgtagctt gctggaccat cgagccgacg gactggaagg 6180
tttcgcgggg cgcacgcatg acggtgcggc ttgcgatggt ttcggcatcc tcggcggaaa 6240
accccgcgtc gatcagttct tgcctgtatg ccttccggtc aaacgtccga ttcattcacc 6300
ctccttgcgg gattgccccg actcacgccg gggcaatgtg cccttattcc tgatttgacc 6360
cgcctggtgc cttggtgtcc agataatcca ccttatcggc aatgaagtcg gtcccgtaga 6420
ccgtctggcc gtccttctcg tacttggtat tccgaatctt gccctgcacg aataccagcg 6480
acccccttgcc caaatacttg ccgtgggcct cggcctgaga gccaaaacac ttgatgcgga 6540
agaagtcggt gcgctcctgc ttgtcgccgg catcgttgcg ccactcttca ttaaccgcta 6600
tatcgaaaat tgcttgcggc ttgttagaat tgccatgacg tacctcggtg tcacgggtaa 6660
gattaccgat aaactggaac tgattatggc tcatatcgaa agtctccttg agaaaggaga 6720
ctctagttta gctaaacatt ggttccgctg tcaagaactt tagcggctaa aattttgcgg 6780
gccgcgacca aaggtgcgag gggcggcttc cgctgtgtac aaccagatat ttttcaccaa 6840
catccttcgt ctgctcgatg agcggggcat gacgaaacat gagctgtcgg agagggcagg 6900
ggtttcaatt tcgtttttat cagacttaac caacggtaag gccaacccct cgttgaaggt 6960
gatggaggcc attgccgacg ccctggaaac tcccctacct cttctcctgg agtccaccga 7020
ccttgaccgc gaggcactcg cggagattgc gggtcatcct ttcaagagca gcgtgccgcc 7080
cggatacgaa cgcatcagtg tggttttgcc gtcacataag gcgtttatcg taaagaaatg 7140
gggcgacgac acccgaaaaa agctgcgtgg aaggctctga cgccaagggt tagggcttgc 7200
acttccttct ttagccgcta aaacggcccc ttctctgcgg gccgtcggct cgcgcatcat 7260
atcgacatcc tcaacggaag ccgtgccgcg aatggcatcg ggcgggtgcg ctttgacagt 7320
tgttttctat cagaacccct acgtcgtgcg gttcgattag ctgtttgtct tgcaggctaa 7380
acactttcgg tatatcgttt gcctgtgcga taatgttgct aatgatttgt tgcgtagggg 7440
ttactgaaaa gtgagcggga aagaagagtt tcagaccatc aaggagcggg ccaagcgcaa 7500
gctggaacgc gacatgggtg cggacctgtt ggccgcgctc aacgacccga aaaccgttga 7560
agtcatgctc aacgcggacg gcaaggtgtg gcacgaacgc cttggcgagc cgatgcggta 7620
catctgcgac atgcggccca gccagtcgca ggcgattata gaaacggtgg ccggattcca 7680
cggcaaagag gtcacgcggc attcgcccat cctggaaggc gagttcccct tggatggcag 7740
ccgctttgcc ggccaattgc cgccggtcgt ggccgcgcca acctttgcga tccgcaagcg 7800
cgcggtcgcc atcttcacgc tggaacagta cgtcgaggcg ggcatcatga cccgcgagca 7860

atacgaggtc attaaaagcg ccgtcgcggc gcatcgaaac atcctcgtca ttggcggtac 7920
tggctcgggc aagaccacgc tcgtcaacgc gatcatcaat gaaatggtcg ccttcaaccc 7980
gtctgagcgc gtcgtcatca tcgaggacac cggcgaaatc cagtgcgccg cagagaacgc 8040
cgtccaatac cacaccagca tcgacgtctc gatgacgctg ctgctcaaga caacgctgcg 8100
tatgcgcccc gaccgcatcc tggtcggtga ggtacgtggc cccgaagccc ttgatctgtt 8160
gatggcctgg aacaccgggc atgaaggagg tgccgccacc ctgcacgcaa acaaccccaa 8220
agcgggcctg agccggctcg ccatgcttat cagcatgcac ccggattcac cgaaacccat 8280
tgagccgctg attggcgagg cggttcatgt ggtcgtccat atcgccagga cccctagcgg 8340
ccgtcgagtg caagaaattc tcgaagttct tggttacgag aacggccagt acatcaccaa 8400
aaccctgtaa ggagtatttc caatgacaac ggctgttccg ttccgtctga ccatgaatcg 8460
cggcattttg ttctaccttg ccgtgttctt cgttctcgct ctcgcgttat ccgcgcatcc 8520
ggcgatggcc tcggaaggca ccggcggcag cttgccatat gagagctggc tgacgaacct 8580
gcgcaactcc gtaaccggcc cggtggcctt cgcgctgtcc atcatcggca tcgtcgtcgc 8640
cggcggcgtg ctgatcttcg gcggcgaact caacgccttc ttccgaaccc tgatcttcct 8700
ggttctggtg atggcgctgc tggtcggcgc gcagaacgtg atgagcacct tcttcggtcg 8760
tggtgccgaa atcgcggccc tcggcaacgg ggcgctgcac caggtgcaag tcgcggcggc 8820
ggatgccgtg cgtgcggtag cggctggacg gctcgcctaa tcatggctct gcgcacgatc 8880
cccatccgtc gcgcaggcaa ccgagaaaac ctgttcatgg gtggtgatcg tgaactggtg 8940
atgttctcgg gcctgatggc gtttgcgctg attttcagcg cccaagagct gcgggccacc 9000
gtggtcggtc tgatcctgtg gttcggggcg ctctatgcgt tccgaatcat ggcgaaggcc 9060
gatccgaaga tgcggttcgt gtacctgcgt caccgccggt acaagccgta ttacccggcc 9120
cgctcgaccc cgttccgcga gaacaccaat agccaaggga agcaataccg atgatccaag 9180
caattgcgat tgcaatcgcg ggcctcggcg cgcttctgtt gttcatcctc tttgcccgca 9240
tccgcgcggt cgatgccgaa ctgaaactga aaaagcatcg ttccaaggac gccggcctgg 9300
ccgatctgct caactacgcc gctgtcgtcg atgacggcgt aatcgtgggc aagaacggca 9360
gctttatggc tgcctggctg tacaagggcg atgacaacgc aagcagcacc gaccagcagc 9420
gcgaagtagt gtccgcccgc atcaaccagg ccctcgcggg cctgggaagt gggtggatga 9480
tccatgtgga cgccgtgcgg cgtcctgctc cgaactacgc ggagcggggc ctgtcggcgt 9540
tccctgaccg tctgacggca gcgattgaag aagagcgctc ggtcttgcct tgctcgtcgg 9600
tgatgtactt caccagctcc gcgaagtcgc tcttcttgat ggagcgcatg gggacgtgct 9660
tggcaatcac gcgcaccccc cggccgtttt agcggctaaa aaagtcatgg ctctgccctc 9720
gggcggacca cgcccatcat gaccttgcca agctcgtcct gcttctcttc gatcttcgcc 9780
agcagggcga ggatcgtggc atcaccgaac cgcgccgtgc gcgggtcgtc ggtgagccag 9840

agtttcagca ggccgcccag gcggcccagg tcgccattga tgcgggccag ctcgcggacg 9900
tgctcatagt ccacgacgcc cgtgattttg tagccctggc cgacggccag caggtaggcc 9960
gacaggctca tgccggccgc cgccgccttt tcctcaatcg ctcttcgttc gtctggaagg 10020
cagtacacct tgataggtgg gctgcccttc ctggttggct tggtttcatc agccatccgc 10080
ttgccctcat ctgttacgcc ggcggtagcc ggccagcctc gcagagcagg attcccgttg 10140
agcaccgcca ggtgcgaata agggacagtg aagaaggaac acccgctcgc gggtgggcct 10200
acttcaccta tcctgcccgg ctgacgccgt tggatacacc aaggaaagtc tacacgaacc 10260
ctttggcaaa atcctgtata tcgtgcgaaa aaggatggat ataccgaaaa aatcgctata 10320
atgaccccga agcagggtta tgcagcggaa aagcgctgct tccctgctgt tttgtggaat 10380
atctaccgac tggaaacagg caaatgcagg aaattactga actgagggga caggcgagag 10440
acgatgccaa agagctacac cgacgagctg gccgagtggg ttgaatcccg cgcggccaag 10500
aagcgccggc gtgatgaggc tgcggttgcg ttcctggcgg tgagggcgga tgtcgaggcg 10560
gcgttagcgt ccggctatgc gctcgtcacc atttgggagc acatgcggga aacggggaag 10620
gtcaagttct cctacgagac gttccgctcg cacgccaggc ggcacatcaa ggccaagccc 10680
gccgatgtgc ccgcaccgca ggccaaggct gcggaacccg cgccggcacc caagacgccg 10740
gagccacggc ggccgaagca ggggggcaag gctgaaaagc cggcccccgc tgcggccccg 10800
accggcttca ccttcaaccc aacaccggac aaaaaggatc tactgtaatg gcgaaaattc 10860
acatggtttt gcagggcaag ggcggggtcg gcaagtcggc catcgccgcg atcattgcgc 10920
agtacaagat ggacaagggg cagacaccct tgtgcatcga caccgacccg gtgaacgcga 10980
cgttcgaggg ctacaaggcc ctgaacgtcc gccggctgaa catcatggcc ggcgacgaaa 11040
ttaactcgcg caacttcgac accctggtcg agctgattgc gccgaccaag gatgacgtgg 11100
tgatcgacaa cggtgccagc tcgttcgtgc ctctgtcgca ttacctcatc agcaaccagg 11160
tgccggctct gctgcaagaa atggggcatg agctggtcat ccataccgtc gtcaccggcg 11220
gccaggctct cctggacacg gtgagcggct tcgcccagct cgccagccag ttcccggccg 11280
aagcgctttt cgtggtctgg ctgaacccgt attgggggcc tatcgagcat gagggcaaga 11340
gctttgagca gatgaaggcg tacacggcca acaaggcccg cgtgtcgtcc atcatccaga 11400
ttccggccct caaggaagaa acctacggcc gcgatttcag cgacatgctg caagagcggc 11460
tgacgttcga ccaggcgctg gccgatgaat cgctcacgat catgacgcgg caacgcctca 11520
agatcgtgcg gcgcggcctg tttgaacagc tcgacgcggc ggccgtgcta tgagcgacca 11580
gattgaagag ctgatccggg agattgcggc caagcacggc atcgccgtcg gccgcgacga 11640
cccggtgctg atcctgcata ccatcaacgc ccggctcatg gccgacagtg cggccaagca 11700
agaggaaatc cttgccgcgt tcaaggaaga gctggaaggg atcgcccatc gttggggcga 11760

ggacgccaag gccaaagcgg agcggatgct gaacgcggcc ctggcggcca gcaaggacgc 11820
aatggcgaag gtaatgaagg acagcgccgc gcaggcggcc gaagcgatcc gcagggaaat 11880
cgacgacggc cttggccgcc agctcgcggc caaggtcgcg gacgcgcggc gcgtggcgat 11940
gatgaacatg atcgccggcg gcatggtgtt gttcgcggcc gccctggtgg tgtgggcctc 12000
gttatgaatc gcagaggcgc agatgaaaaa gcccggcgtt gccgggcttt gtttttgcgt 12060
tagctgggct tgtttgacag gcccaagctc tgactgcgcc cgcgctcgcg ctcctgggcc 12120
tgtttcttct cctgctcctg cttgcgcatc agggcctggt gccgtcgggc tgcttcacgc 12180
atcgaatccc agtcgccggc cagctcggga tgctccgcgc gcatcttgcg cgtcgccagt 12240
tcctcgatct tgggcgcgtg aatgcccatg ccttccttga tttcgcgcac catgtccagc 12300
cgcgtgtgca gggtctgcaa gcgggcttgc tgttgggcct gctgctgctg ccaggcggcc 12360
tttgtacgcg gcagggacag caagccgggg gcattggact gtagctgctg caaacgcgcc 12420
tgctgacggt ctacgagctg ttctaggcgg tcctcgatgc gctccacctg gtcatgcttt 12480
gcctgcacgt agagcgcaag ggtctgctgg taggtctgct cgatgggcgc ggattctaag 12540
agggcctgct gttccgtctc ggcctcctgg gccgcctgta gcaaatcctc gccgctgttg 12600
ccgctggact gctttactgc cggggactgc tgttgccctg ctcgcgccgt cgtcgcagtt 12660
cggcttgccc ccactcgatt gactgcttca tttcgagccg cagcgatgcg atctcggatt 12720
gcgtcaacgg acggggcagc gcggaggtgt ccggcttctc cttgggtgag tcggtcgatg 12780
ccatagccaa aggtttcctt ccaaaatgcg tccattgctg gaccgtgttt ctcattgatg 12840
cccgcaagca tcttcggctt gaccgccagg tcaagcgcgc cttcatgggc ggtcatgacg 12900
gacgccgcca tgaccttgcc gccgttgttc tcgatgtagc cgcgtaatga ggcaatggtg 12960
ccgcccatcg tcagcgtgtc atcgacaacg atgtacttct ggccggggat cacctccccc 13020
tcgaaagtcg ggttgaacgc caggcgatga tctgaaccgg ctccggttcg ggcgaccttc 13080
tcccgctgca caatgtccgt ttcgacctca aggccaaggc ggtcggccag aacgaccgcc 13140
atcatggccg gaatcttgtt gttccccgcc gcctcgacgg cgaggactgg aacgatgcgg 13200
ggcttgtcgt cgccgatcag cgtcttgagc tgggcaacag tgtcgtccga aatcaggcgc 13260
tcgaccaaat taagcgccgc ttccgcgtcg ccctgcttcg cagcctggta ttcaggctcg 13320
ttggtcaaag aaccaaggtc gccgttgcga accaccttcg ggaagtctcc ccacggtgcg 13380
cgctcggctc tgctgtagct gctcaagacg cctccctttt tagccgctaa aactctaacg 13440
agtgcgcccg cgactcaact tgacgctttc ggcacttacc tgtgccttgc cacttgcgtc 13500
ataggtgatg cttttcgcac tcccgatttc aggtacttta tcgaaatctg accgggcgtg 13560
cattacaaag ttcttcccca cctgttggta aatgctgccg ctatctgcgt ggacgatgct 13620
gccgtcgtgg cgctgcgact tatcggcctt ttgggccata tagatgttgt aaatgccagg 13680
tttcagggcc ccggctttat ctaccttctg gttcgtccat gcgccttggt tctcggtctg 13740

gacaattctt tgcccattca tgaccaggag gcggtgtttc attgggtgac tcctgacggt 13800
tgcctctggt gttaaacgtg tcctggtcgc ttgccggcta aaaaaaagcc gacctcggca 13860
gttcgaggcc ggctttccct agagccgggc gcgtcaaggt tgttccatct attttagtga 13920
actgcgttcg atttatcagt tactttcctc ccgctttgtg tttcctccca ctcgtttccg 13980
cgtctagccg accccctcaac atagcggcct cttcttgggc tgcctttgcc tcttgccgcg 14040
cttcgtcacg ctcggcttgc accgtcgtaa agcgctcggc ctgcctggcc gcctcttgcg 14100
ccgccaactt cctttgctcc tggtgggcct cggcgtcggc ctgcgccttc gctttcaccg 14160
ctgccaactc cgtgcgcaaa ctctccgctt cgcgcctggt ggcgtcgcgc tcgccgcgaa 14220
gcgcctgcat ttcctggttg gccgcgtcca gggtcttgcg gctctcttct ttgaatgcgc 14280
gggcgtcctg gtgagcgtag tccagctcgg cgcgcagctc ctgcgctcga cgctccacct 14340
cgtcggcccg ctgcgtcgcc agcgcggccc gctgctcggc tcctgccagg gcggtgcgtg 14400
cttcggccag ggcttgccgc tggcgtgcgg ccagctcggc cgcctcggcg gcctgctgct 14460
ctagcaatgt aacgcgcgcc tgggcttctt ccagctcgcg ggcctgcgcc tcgaaggcgt 14520
cggccagctc cccgcgcacg gcttccaact cgttgcgctc acgatcccag ccggcttgcg 14580
ctgcctgcaa cgattcattg gcaagggcct gggcggcttg ccagagggcg gccacggcct 14640
ggttgccggc ctgctgcacc gcgtccggca cctggactgc cagcggggcg gcctgcgccg 14700
tgcgctggcg tcgccattcg cgcatgccgg cgctggcgtc gttcatgttg acgcgggcgg 14760
ccttacgcac tgcatccacg gtcgggaagt tctcccggtc gccttgctcg aacagctcgt 14820
ccgcagccgc aaaaatgcgg tcgcgcgtct ctttgttcag ttccatgttg gctccggtaa 14880
ttggtaagaa taataatact cttacctacc ttatcagcgc aagagtttag ctgaacagtt 14940
ctcgacttaa cggcaggttt tttagcggct gaagggcagg caaaaaaagc cccgcacggt 15000
cggcgggggc aaagggtcag cgggaagggg attagcgggc gtcgggcttc ttcatgcgtc 15060
ggggccgcgc ttcttgggat ggagcacgac gaagcgcgca cgcgcatcgt cctcggccct 15120
atcggcccgc gtcgcggtca ggaacttgtc gcgcgctagg tcctccctgg tgggcaccag 15180
gggcatgaac tcggcctgct cgatgtaggt ccactccatg accgcatcgc agtcgaggcc 15240
gcgttccttc accgtctctt gcaggtcgcg gtacgcccgc tcgttgagcg gctggtaacg 15300
ggccaattgg tcgtaaatgg ctgtcggcca tgagcggcct ttcctgttga gccagcagcc 15360
gacgacgaag ccggcaatgc aggcccctgg cacaaccagg ccgacgccgg gggcagggga 15420
tggcagcagc tcgccaacca ggaaccccgc cgcgatgatg ccgatgccgg tcaaccagcc 15480
cttgaaacta tccggccccg aaacacccct gcgcattgcc tggatgctgc gccggatagc 15540
ttgcaacatc aggagccgtt tcttttgttc gtcagtcatg gtccgccctc accagttgtt 15600
cgtatcggtg tcggacgaac tgaaatcgca agagctgccg gtatcggtcc agccgctgtc 15660

cgtgtcgctg ctgccgaagc acggcgaggg gtccgcgaac gccgcagacg gcgtatccgg 15720
ccgcagcgca tcgcccagca tggccccggt cagcgagccg ccggccaggt agcccagcat 15780
ggtgctgttg gtcgccccgg ccaccagggc cgacgtgacg aaatcgccgt cattccctct 15840
ggattgttcg ctgctcggcg gggcagtgcg ccgcgccggc ggcgtcgtgg atggctcggg 15900
ttggctggcc tgcgacggcc ggcgaaaggt gcgcagcagc tcgttatcga ccggctgcgg 15960
cgtcggggcc gccgccttgc gctgcggtcg gtgttccttc ttcggctcgc gcagcttgaa 16020
cagcatgatc gcggaaacca gcagcaacgc cgcgcctacg cctcccgcga tgtagaacag 16080
catcggattc attcttcggt cctccttgta gcggaaccgt tgtctgtgcg gcgcgggtgg 16140
cccgcgccgc tgtctttggg gatcagccct cgatgagcgc gaccagtttc acgtcggcaa 16200
ggttcgcctc gaactcctgg ccgtcgtcct cgtacttcaa ccaggcatag ccttccgccg 16260
gcggccgacg gttgaggata aggcgggcag ggcgctcgtc gtgctcgacc tggacgatgg 16320
cctttttcag cttgtccggg tccggctcct tcgcgccctt ttccttggcg tccttaccgt 16380
cctggtcgcc gtcctcgccg tcctggccgt cgccggcctc cgcgtcacgc tcggcatcag 16440
tctggccgtt gaaggcatcg acggtgttgg gatcgcggcc cttctcgtcc aggaactcgc 16500
gcagcagctt gaccgtgccg cgcgtgattt cctgggtgtc gtcgtcaagc cacgcctcga 16560
cttcctccgg gcgcttcttg aaggccgtca ccagctcgtt caccacggtc acgtcgcgca 16620
cgcggccggt gttgaacgca tcggcgatct tctccggcag gtccagcagc gtgacgtgct 16680
gggtgatgaa cgccggcgac ttgccgattt ccttggcgat atcgcctttc ttcttgccct 16740
tcgccagctc gcggccaatg aagtcggcaa tttcgcgcgg ggtcagctcg ttgcgttgca 16800
ggttctcgat aacctggtcg gcttcgttgt agtcgttgtc gatgaacgcc gggatggact 16860
tcttgccggc ccacttcgag ccacggtagc ggcgggcgcc gtgattgatg atatagcggc 16920
ccggctgctc ctggttctcg cgcaccgaaa tgggtgactt caccccgcgc tctttgatcg 16980
tggcaccgat ttccgcgatg ctctccgggg aaaagccggg gttgtcggcc gtccgcggct 17040
gatgcggatc ttcgtcgatc aggtccaggt ccagctcgat agggccggaa ccgccctgag 17100
acgccgcagg agcgtccagg aggctcgaca ggtcgccgat gctatccaac cccaggccgg 17160
acggctgcgc cgcgcctgcg gcttcctgag cggccgcagc ggtgtttttc ttggtggtct 17220
tggcttgagc cgcagtcatt gggaaatctc catcttcgtg aacacgtaat cagccagggc 17280
gcgaacctct ttcgatgcct tgcgcgcggc cgttttcttg atcttccaga ccggcacacc 17340
ggatgcgagg gcatcggcga tgctgctgcg caggccaacg gtggccggaa tcatcatctt 17400
ggggtacgcg gccagcagct cggcttggtg gcgcgcgtgg cgcggattcc gcgcatcgac 17460
cttgctgggc accatgccaa ggaattgcag cttggcgttc ttctggcgca cgttcgcaat 17520
ggtcgtgacc atcttcttga tgccctggat gctgtacgcc tcaagctcga tgggggacag 17580
cacatagtcg gccgcgaaga gggcggccgc caggccgacg ccaagggtcg ggccgtgtc 17640

gatcaggcac acgtcgaagc cttggttcgc cagggccttg atgttcgccc cgaacagctc 17700
gcgggcgtcg tccagcgaca gccgttcggc gttcgccagt accgggttgg actcgatgag 17760
ggcgaggcgc gcggcctggc cgtcgccggc tgcgggtgcg gtttcggtcc agccgccggc 17820
agggacagcg ccgaacagct tgcttgcatg caggccggta gcaaagtcct tgagcgtgta 17880
ggacgcattg ccctgggggt ccaggtcgat cacggcaacc cgcaagccgc gctcgaaaaa 17940
gtcgaaggca agatgcacaa gggtcgaagt cttgccgacg ccgcctttct ggttggccgt 18000
gaccaaagtt ttcatcgttt ggtttcctgt tttttcttgg cgtccgcttc ccacttccgg 18060
acgatgtacg cctgatgttc cggcagaacc gccgttaccc gcgcgtaccc ctcgggcaag 18120
ttcttgtcct cgaacgcggc ccacacgcga tgcaccgctt gcgacactgc gcccctggtc 18180
agtcccagcg acgttgcgaa cgtcgcctgt ggcttcccat cgactaagac gccccgcgct 18240
atctcgatgg tctgctgccc cacttccagc ccctggatcg cctcctggaa ctggctttcg 18300
gtaagccgtt tcttcatgga taacacccat aatttgctcc gcgccttggt tgaacatagc 18360
ggtgacagcc gccagcacat gagagaagtt tagctaaaca tttctcgcac gtcaacacct 18420
ttagccgcta aaactcgtcc ttggcgtaac aaaacaaaag cccggaaacc gggctttcgt 18480
ctcttgccgc ttatggctct gcacccggct ccatcaccaa caggtcgcgc acgcgcttca 18540
ctcggttgcg gatcgacact gccagcccaa caaagccggt tgccgccgcc gccaggatcg 18600
cgccgatgat gccggccaca ccggccatcg cccaccaggt cgccgccttc cggttccatt 18660
cctgctggta ctgcttcgca atgctggacc tcggctcacc ataggctgac cgctcgatgg 18720
cgtatgccgc ttctcccctt ggcgtaaaac ccagcgccgc aggcggcatt gccatgctgc 18780
ccgccgcttt cccgaccacg acgcgcgcac caggcttgcg gtccagacct tcggccacgg 18840
cgagctgcgc aaggacataa tcagccgccg acttggctcc acgcgcctcg atcagctctt 18900
gcactcgcgc gaaatccttg gcctccacgg ccgccatgaa tcgcgcacgc ggcgaaggct 18960
ccgcagggcc ggcgtcgtga tcgccgccga gaatgccctt caccaagttc gacgacacga 19020
aaatcatgct gacggctatc accatcatgc agacggatcg cacgaacccg ctgaattgaa 19080
cacgagcacg gcacccgcga ccactatgcc aagaatgccc aaggtaaaaa ttgccggccc 19140
cgccatgaag tccgtgaatg ccccgacggc cgaagtgaag ggcaggccgc cacccaggcc 19200
gccgccctca ctgcccggca cctggtcgct gaatgtcgat gccagcacct gcggcacgtc 19260
aatgcttccg ggcgtcgcgc tcgggctgat cgcccatccc gttactgccc cgatcccggc 19320
aatggcaagg actgccagcg ctgccatttt tggggtgagg ccgttcgcgg ccgaggggcg 19380
cagcccctgg ggggatggga ggcccgcgtt agcgggccgg gagggttcga gaaggggggg 19440
cacccccctt cggcgtgcgc ggtcacgcgc acagggcgca gccctggtta aaaacaaggt 19500
ttataaatat tggtttaaaa gcaggttaaa agacaggtta gcggtggccg aaaaacgggc 19560

ggaaaccctt gcaaatgctg gattttctgc ctgtggacag cccctcaaat gtcaataggt 19620
gcgcccctca tctgtcagca ctctgcccct caagtgtcaa ggatcgcgcc cctcatctgt 19680
cagtagtcgc gcccctcaag tgtcaatacc gcagggcact tatccccagg cttgtccaca 19740
tcatctgtgg gaaactcgcg taaaatcagg cgttttcgcc gatttgcgag gctggccagc 19800
tccacgtcgc cggccgaaat cgagcctgcc cctcatctgt caacgccgcg ccgggtgagt 19860
cggcccctca agtgtcaacg tccgcccctc atctgtcagt gagggccaag ttttccgcga 19920
ggtatccaca acgccggcgg ccgcggtgtc tcgcacacgg cttcgacggc gtttctggcg 19980
cgtttgcagg gccatagacg gccgccagcc cagcggcgag ggcaaccagc ccggtgagcg 20040
tcggaaaggc gctggaagcc ccgtagcgac gcggagaggg gcgagacaag ccaagggcgc 20100
aggctcgatg cgcagcacga catagccggt tctcgcaagg acgagaattt ccctgcggtg 20160
cccctcaagt gtcaatgaaa gtttccaacg cgagccattc gcgagagcct tgagtccacg 20220
ctagatgaga gctttgttgt aggtggacca gttggtgatt ttgaactttt gctttgccac 20280
ggaacggtct gcgttgtcgg gaagatgcgt gatctgatcc ttcaactcag caaaagttcg 20340
atttattcaa caaagccacg ttgtgtctca aaatctctga tgttacattg cacaagataa 20400
aaatatatca tcatgaacaa taaaactgtc tgcttacata aacagtaata caaggggtgt 20460
tatgagccat attcaacggg aaacgtcttg ctcgactcta gagctcgttc ctcgaggcct 20520
cgaggcctcg aggaacggta cctgcgggga agcttacaat aatgtgtgtt gttaagtctt 20580
gttgcctgtc atcgtctgac tgactttcgt cataaatccc ggcctccgta acccagcttt 20640
gggcaagctc acggatttga tccggcggaa cgggaatatc gagatgccgg gctgaacgct 20700
gcagttccag ctttcccttt cgggacaggt actccagctg attgattatc tgctgaaggg 20760
tcttggttcc acctcctggc acaatgcgaa tgattacttg agcgcgatcg ggcatccaat 20820
tttctcccgt caggtgcgtg gtcaagtgct acaaggcacc tttcagtaac gagcgaccgt 20880
cgatccgtcg ccgggatacg gacaaaatgg agcgcagtag tccatcgagg gcggcgaaag 20940
cctcgccaaa agcaatacgt tcatctcgca cagcctccag atccgatcga gggtcttcgg 21000
cgtaggcaga tagaagcatg gatacattgc ttgagagtat tccgatggac tgaagtatgg 21060
cttccatctt ttctcgtgtg tctgcatcta tttcgagaaa gcccccgatg cggcgcaccg 21120
caacgcgaat tgccatacta tccgaaagtc ccagcaggcg cgcttgatag gaaaaggttt 21180
catactcggc cgatcgcaga cgggcactca cgaccttgaa cccttcaact ttcagggatc 21240
gatgctggtt gatggtagtc tcactcgacg tggctctggt gtgttttgac atagcttcct 21300
ccaaagaaag cggaaggtct ggatactcca gcacgaaatg tgcccgggta gacggatgga 21360
agtctagccc tgctcaatat gaaatcaaca gtacatttac agtcaatact gaatatactt 21420
gctacatttg caattgtctt ataacgaatg tgaaataaaa atagtgtaac aacgctttta 21480
ctcatcgata atcacaaaaa catttatacg aacaaaaata caaatgcact ccggtttcac 21540

aggataggcg ggatcagaat atgcaacttt tgacgttttg ttctttcaaa gggggtgctg 21600

gcaaaaccac cgcactcatg ggcctttgcg ctgctttggc aaatgacggt aaacgagtgg 21660

ccctctttga tgccgacgaa aaccggcctc tgacgcgatg gagagaaaac gccttacaaa 21720

gcagtactgg gatcctcgct gtgaagtcta ttccgccgac gaaatgcccc ttcttgaagc 21780

agcctatgaa aatgccgagc tcgaaggatt tgattatgcg ttggccgata cgcgtggcgg 21840

ctcgagcgag ctcaacaaca caatcatcgc tagctcaaac ctgcttctga tccccaccat 21900

gctaacgccg ctcgacatcg atgaggcact atctacctac cgctacgtca tcgagctgct 21960

gttgagtgaa aatttggcaa ttcctacagc tgttttgcgc caacgcgtcc cggtcggccg 22020

attgacaaca tcgcaacgca ggatgtcaga gacgctagag agccttccag ttgtaccgtc 22080

tcccatgcat gaaagagatg catttgccgc gatgaaagaa cgcggcatgt tgcatcttac 22140

attactaaac acgggaactg atccgacgat gcgcctcata gagaggaatc ttcggattgc 22200

gatggaggaa gtcgtggtca tttcgaaact gatcagcaaa atcttggagg cttgaagatg 22260

gcaattcgca agcccgcatt gtcggtcggc gaagcacggc ggcttgctgg tgctcgaccc 22320

gagatccacc atcccaaccc gacacttgtt ccccagaagc tggacctcca gcacttgcct 22380

gaaaaagccg acgagaaaga ccagcaacgt gagcctctcg tcgccgatca catttacagt 22440

cccgatcgac aacttaagct aactgtggat gcccttagtc cacctccgtc cccgaaaaag 22500

ctccaggttt ttctttcagc gcgaccgccc gcgcctcaag tgtcgaaaac atatgacaac 22560

ctcgttcggc aatacagtcc ctcgaagtcg ctacaaatga ttttaaggcg cgcgttggac 22620

gatttcgaaa gcatgctggc agatggatca tttcgcgtgg ccccgaaaag ttatccgatc 22680

ccttcaacta cagaaaaatc cgttctcgtt cagacctcac gcatgttccc ggttgcgttg 22740

ctcgaggtcg ctcgaagtca ttttgatccg ttggggttgg agaccgctcg agctttcggc 22800

cacaagctgg ctaccgccgc gctcgcgtca ttctttgctg gagagaagcc atcgagcaat 22860

tggtgaagag ggacctatcg gaacccctca ccaaatattg agtgtaggtt tgaggccgct 22920

ggccgcgtcc tcagtcacct tttgagccag ataattaaga gccaaatgca attggctcag 22980

gctgccatcg tccccccgtg cgaaacctgc acgtccgcgt caaagaaata accggcacct 23040

cttgctgttt ttatcagttg agggcttgac ggatccgcct caagtttgcg gcgcagccgc 23100

aaaatgagaa catctatact cctgtcgtaa acctcctcgt cgcgtactcg actggcaatg 23160

agaagttgct cgcgcgatag aacgtcgcgg ggtttctcta aaaacgcgag gagaagattg 23220

aactcacctg ccgtaagttt cacctcaccg ccagcttcgg acatcaagcg acgttgcctg 23280

agattaagtg tccagtcagt aaaacaaaaa gaccgtcggt ctttggagcg gacaacgttg 23340

gggcgcacgc gcaaggcaac ccgaatgcgt gcaagaaact ctctcgtact aaacggctta 23400

gcgataaaat cacttgctcc tagctcgagt gcaacaactt tatccgtctc ctcaaggcgg 23460

tcgccactga taattatgat tggaatatca gactttgccg ccagatttcg aacgatctca 23520

agcccatctt cacgacctaa atttagatca acaaccacga catcgaccgt cgcggaagag 23580

agtactctag tgaactgggt gctgtcggct accgcggtca ctttgaaggc gtggatcgta 23640

aggtattcga taataagatg ccgcatagcg acatcgtcat cgataagaag aacgtgtttc 23700

aacggctcac ctttcaatct aaaatctgaa cccttgttca cagcgcttga gaaattttca 23760

cgtgaaggat gtacaatcat ctccagctaa atgggcagtt cgtcagaatt gcggctgacc 23820

gcggatgacg aaaatgcgaa ccaagtattt caattttatg acaaaagttc tcaatcgttg 23880

ttacaagtga aacgcttcga ggttacagct actattgatt aaggagatcg cctatggtct 23940

cgccccggcg tcgtgcgtcc gccgcgagcc agatctcgcc tacttcataa acgtcctcat 24000

aggcacggaa tggaatgatg acatcgatcg ccgtagagag catgtcaatc agtgtgcgat 24060

cttccaagct agcaccttgg gcgctacttt tgacaaggga aaacagtttc ttgaatcctt 24120

ggattggatt cgcgccgtgt attgttgaaa tcgatcccgg atgtcccgag acgacttcac 24180

tcagataagc ccatgctgca tcgtcgcgca tctcgccaag caatatccgg tccggccgca 24240

tacgcagact tgcttggagc aagtgctcgg cgctcacagc acccagccca gcaccgttct 24300

tggagtagag tagtctaaca tgattatcgt gtggaatgac gagttcgagc gtatcttcta 24360

tggtgattag cctttcctgg ggggggatgg cgctgatcaa ggtcttgctc attgttgtct 24420

tgccgcttcc ggtagggcca catagcaaca tcgtcagtcg gctgacgacg catgcgtgca 24480

gaaacgcttc caaatccccg ttgtcaaaat gctgaaggat agcttcatca tcctgatttt 24540

ggcgtttcct tcgtgtctgc cactggttcc acctcgaagc atcataacgg gaggagactt 24600

ctttaagacc agaaacacgc gagcttggcc gtcgaatggt caagctgacg gtgcccgagg 24660

gaacggtcgg cggcagacag atttgtagtc gttcaccacc aggaagttca gtggcgcaga 24720

gggggttacg tggtccgaca tcctgctttc tcagcgcgcc cgctaaaata gcgatatctt 24780

caagatcatc ataagagacg ggcaaaggca tcttggtaaa aatgccggct tggcgcacaa 24840

atgcctctcc aggtcgattg atcgcaattt cttcagtctt cgggtcatcg agccattcca 24900

aaatcggctt cagaagaaag cgtagttgcg gatccacttc catttacaat gtatcctatc 24960

tctaagcgga aatttgaatt cattaagagc ggcggttcct cccccgcgtg gcgccgccag 25020

tcaggcggag ctggtaaaca ccaaagaaat cgaggtcccg tgctacgaaa atggaaacgg 25080

tgtcaccctg attcttcttc agggttggcg gtatgttgat ggttgcctta agggctgtct 25140

cagttgtctg ctcaccgtta ttttgaaagc tgttgaagct catcccgcca cccgagctgc 25200

cggcgtaggt gctagctgcc tggaaggcgc cttgaacaac actcaagagc atagctccgc 25260

taaaacgctg ccagaagtgg ctgtcgaccg agcccggcaa tcctgagcga ccgagttcgt 25320

ccgcgcttgg cgatgttaac gagatcatcg catggtcagg tgtctcggcg cgatcccaca 25380

acacaaaaac gcgcccatct ccctgttgca agccacgctg tatttcgcca acaacggtgg 25440

tgccacgatc aagaagcacg atattgttcg ttgttccacg aatatcctga ggcaagacac 25500
actttacata gcctgccaaa tttgtgtcga ttgcggtttg caagatgcac ggaattattg 25560
tcccttgcgt taccataaaa tcggggtgcg gcaagagcgt ggcgctgctg ggctgcagct 25620
cggtgggttt catacgtatc gacaaatcgt tctcgccgga cacttcgcca ttcggcaagg 25680
agttgtcgtc acgcttgcct tcttgtcttc ggcccgtgtc gccctgaatg gcgcgtttgc 25740
tgaccccttg atcgccgctg ctatatgcaa aaatcggtgt ttcttccggc cgtggctcat 25800
gccgctccgg ttcgcccctc ggcggtagag gagcagcagg ctgaacagcc tcttgaaccg 25860
ctggaggatc cggcggcacc tcaatcggag ctggatgaaa tggcttggtg tttgttgcga 25920
tcaaagttga cggcgatgcg ttctcattca ccttcttttg gcgcccacct agccaaatga 25980
ggcttaatga taacgcgaga acgacacctc cgacgatcaa tttctgagac cccgaaagac 26040
gccggcgatg tttgtcggag accagggatc cagatgcatc aacctcatgt gccgcttgct 26100
gactatcgtt attcatccct tcgccccctt caggacgcgt ttcacatcgg gcctcaccgt 26160
gcccgtttgc ggcctttggc caacgggatc gtaagcggtg ttccagatac atagtactgt 26220
gtggccatcc ctcagacgcc aacctcggga aaccgaagaa atctcgacat cgctcccttt 26280
aactgaatag ttggcaacag cttccttgcc atcaggattg atggtgtaga tggagggtat 26340
gcgtacattg cccggaaagt ggaataccgt cgtaaatcca ttgtcgaaga cttcgagtgg 26400
caacagcgaa cgatcgcctt gggcgacgta gtgccaatta ctgtccgccg caccaagggc 26460
tgtgacaggc tgatccaata aattctcagc tttccgttga tattgtgctt ccgcgtgtag 26520
tctgtccaca acagccttct gttgtgcctc ccttcgccga gccgccgcat cgtcggcggg 26580
gtaggcgaat tggacgctgt aatagagatc gggctgctct ttatcgaggt gggacagagt 26640
cttggaactt atactgaaaa cataacggcg catcccggag tcgcttgcgg ttagcacgat 26700
tactggctga ggcgtgagga cctggcttgc cttgaaaaat agataatttc cccgcggtag 26760
ggctgctaga tctttgctat ttgaaacggc aaccgctgtc accgtttcgt tcgtggcgaa 26820
tgttacgacc aaagtagctc caaccgccgt cgagaggcgc accacttgat cgggattgta 26880
agccaaataa cgcatgcgcg gatctagctt gcccgccatt ggagtgtctt cagcctccgc 26940
accagtcgca gcggcaaata aacatgctaa aatgaaaagt gcttttctga tcatggttcg 27000
ctgtggccta cgtttgaaac ggtatcttcc gatgtctgat aggaggtgac aaccagacct 27060
gccgggttgg ttagtctcaa tctgccgggc aagctggtca ccttttcgta gcgaactgtc 27120
gcggtccacg tactcaccac aggcattttg ccgtcaacga cgagggtcct tttatagcga 27180
atttgctgcg tgcttggagt tacatcattt gaagcgatgt gctcgacctc caccctgccg 27240
cgtttgccaa gaatgacttg aggcgaactg ggattgggat agttgaagaa ttgctggtaa 27300
tcctggcgca ctgttggggc actgaagttc gataccaggt cgtaggcgta ctgagcggtg 27360

tcggcatcat aactctcgcg caggcgaacg tactcccaca atgaggcgtt aacgacggcc 27420
tcctcttgag ttgcaggcaa tcgcgagaca gacacctcgc tgtcaacggt gccgtccggc 27480
cgtatccata gatatacggg cacaagcctg ctcaacggca ccattgtggc tatagcgaac 27540
gcttgagcaa catttcccaa aatcgcgata gctgcgacag ctgcaatgag tttggagaga 27600
cgtcgcgccg atttcgctcg cgcggtttga aaggcttcta cttccttata gtgctcggca 27660
aggctttcgc gcgccactag catggcatat tcaggccccg tcatagcgtc cacccgaatt 27720
gccgagctga agatctgacg gagtaggctg ccatcgcccc acattcagcg ggaagatcgg 27780
gcctttgcag ctcgctaatg tgtcgtttgt ctggcagccg ctcaaagcga caactaggca 27840
cagcaggcaa tacttcatag aattctccat tgaggcgaat ttttgcgcga cctagcctcg 27900
ctcaacctga gcgaagcgac ggtacaagct gctggcagat tgggttgcgc cgctccagta 27960
actgcctcca atgttgccgg cgatcgccgg caaagcgaca atgagcgcat cccctgtcag 28020
aaaaaacata tcgagttcgt aaagaccaat gatcttggcc gcggtcgtac cggcgaaggt 28080
gattacacca agcataaggg tgagcgcagt cgcttcggtt aggatgacga tcgttgccac 28140
gaggtttaag aggagaagca agagaccgta ggtgataagt tgcccgatcc acttagctgc 28200
gatgtcccgc gtgcgatcaa aaatatatcc gacgaggatc agaggcccga tcgcgagaag 28260
cactttcgtg agaattccaa cggcgtcgta aactccgaag gcagaccaga gcgtgccgta 28320
aaggacccac tgtgcccctt ggaaagcaag gatgtcctgg tcgttcatcg gaccgatttc 28380
ggatgcgatt ttctgaaaaa cggcctgggt cacggcgaac attgtatcca actgtgccgg 28440
aacagtctgc agaggcaagc cggttacact aaactgctga acaaagtttg ggaccgtctt 28500
ttcgaagatg gaaaccacat agtcttggta gttagcctgc ccaacaatta gagcaacaac 28560
gatggtgacc gtgatcaccc gagtgatacc gctacgggta tcgacttcgc cgcgtatgac 28620
taaaataccc tgaacaataa tccaaagagt gacacaggcg atcaatggcg cactcaccgc 28680
ctcctggata gtctcaagca tcgagtccaa gcctgtcgtg aaggctacat cgaagatcgt 28740
atgaatggcc gtaaacggcg ccggaatcgt gaaattcatc gattggacct gaacttgact 28800
ggtttgtcgc ataatgttgg ataaaatgag ctcgcattcg gcgaggatgc gggcggatga 28860
acaaatcgcc cagccttagg ggagggcacc aaagatgaca gcggtctttt gatgctcctt 28920
gcgttgagcg gccgcctctt ccgcctcgtg aaggccggcc tgcgcggtag tcatcgttaa 28980
taggcttgtc gcctgtacat tttgaatcat tgcgtcatgg atctgcttga gaagcaaacc 29040
attggtcacg gttgcctgca tgatattgcg agatcgggaa agctgagcag acgtatcagc 29100
attcgccgtc aagcgtttgt ccatcgtttc cagattgtca gccgcaatgc cagcgctgtt 29160
tgcggaaccg gtgatctgcg atcgcaacag gtccgcttca gcatcactac ccacgactgc 29220
acgatctgta tcgctggtga tcgcacgtgc cgtggtcgac attggcattc gcggcgaaaa 29280
catttcattg tctaggtcct tcgtcgaagg atactgattt ttctggttga gcgaagtcag 29340

tagtccagta acgccgtagg ccgacgtcaa catcgtaacc atcgctatag tctgagtgag 29400
attctccgca gtcgcgagcg cagtcgcgag cgtctcagcc tccgttgccg ggtcgctaac 29460
aacaaactgc gcccgcgcgg gctgaatata tagaaagctg caggtcaaaa ctgttgcaat 29520
aagttgcgtc gtcttcatcg tttcctacct tatcaatctt ctgcctcgtg gtgacgggcc 29580
atgaattcgc tgagccagcc agatgagttg ccttcttgtg cctcgcgtag tcgagttgca 29640
aagcgcaccg tgttggcacg ccccgaaagc acggcgacat attcacgcat atcccgcaga 29700
tcaaattcgc agatgacgct tccactttct cgtttaagaa gaaacttacg gctgccgacc 29760
gtcatgtctt cacggatcgc ctgaaattcc ttttcggtac atttcagtcc atcgacataa 29820
gccgatcgat ctgcggttgg tgatggatag aaaatcttcg tcatacattg cgcaaccaag 29880
ctggctccta gcggcgattc cagaacatgc tctggttgct gcgttgccag tattagcatc 29940
ccgttgtttt ttcgaacggt caggaggaat ttgtcgacga cagtcgaaaa tttagggttt 30000
aacaaatagg cgcgaaactc atcgcagctc atcacaaaac ggcggccgtc gatcatggct 30060
ccaatccgat gcaggagata tgctgcagcg ggagcgcata cttcctcgta ttcgagaaga 30120
tgcgtcatgt cgaagccggt aatcgacgga tctaacttta cttcgtcaac ttcgccgtca 30180
aatgcccagc caagcgcatg gccccggcac cagcgttgga gccgcgctcc tgcgccttcg 30240
gcgggcccat gcaacaaaaa ttcacgtaac cccgcgattg aacgcatttg tggatcaaac 30300
gagagctgac gatggatacc acggaccaga cggcggttct cttccggaga aatcccaccc 30360
cgaccatcac tctcgatgag agccacgatc cattcgcgca gaaaatcgtg tgaggctgct 30420
gtgttttcta ggccacgcaa cggcgccaac ccgctgggtg tgcctctgtg aagtgccaaa 30480
tatgttcctc ctgtggcgcg aaccagcaat tcgccacccc ggtccttgtc aaagaacacg 30540
accgtacctg cacggtcgac catgctctgt tcgagcatgg ctagaacaaa catcatgagc 30600
gtcgtcttac cctcccgat aggcccgaat attgccgtca tgccaacatc gtgctcatgc 30660
gggatatagt cgaaaggcgt tccgccattg gtacgaaatc gggcaatcgc gttgccccag 30720
tggcctgagc tggcgccctc tggaaagttt tcgaaagaga caaaccctgc gaaattgcgt 30780
gaagtgattg cgccagggcg tgtgcgccac ttaaaattcc ccggcaattg ggaccaatag 30840
gccgcttcca taccaatacc ttcttggaca accacggcac ctgcatccgc cattcgtgtc 30900
cgagcccgcg cgcccctgtc cccaagacta ttgagatcgt ctgcatagac gcaaaggctc 30960
aaatgatgtg agcccataac gaattcgttg ctcgcaagtg cgtcctcagc ctcggataat 31020
ttgccgattt gagtcacggc tttatcgccg gaactcagca tctggctcga tttgaggcta 31080
agtttcgcgt gcgcttgcgg gcgagtcagg aacgaaaaac tctgcgtgag aacaagtgga 31140
aaatcgaggg atagcagcgc gttgagcatg cccggccgtg tttttgcagg gtattcgcga 31200
aacgaataga tggatccaac gtaactgtct tttggcgttc tgatctcgag tcctcgcttg 31260

ccgcaaatga ctctgtcggt ataaatcgaa gcgccgagtg agccgctgac gaccggaacc 31320
ggtgtgaacc gaccagtcat gatcaaccgt agcgcttcgc caatttcggt gaagagcaca 31380
ccctgcttct cgcggatgcc aagacgatgc aggccatacg ctttaagaga gccagcgaca 31440
acatgccaaa gatcttccat gttcctgatc tggcccgtga gatcgttttc ccttttttccg 31500
cttagcttgg tgaacctcct ctttaccttc cctaaagccg cctgtgggta gacaatcaac 31560
gtaaggaagt gttcattgcg gaggagttgg ccggagagca cgcgctgttc aaaagcttcg 31620
ttcaggctag cggcgaaaac actacggaag tgtcgcggcg ccgatgatgg cacgtcggca 31680
tgacgtacga ggtgagcata tattgacaca tgatcatcag cgatattgcg caacagcgtg 31740
ttgaacgcac gacaacgcgc attgcgcatt tcagtttcct caagctcgaa tgcaacgcca 31800
tcaattctcg caatggtcat gatcgatccg tcttcaagaa ggacgatatg gtcgctgagg 31860
tggccaatat aagggagata gatctcaccg gatctttcgg tcgttccact cgcgccgagc 31920
atcacaccat tcctctccct cgtgggggaa ccctaattgg atttgggcta acagtagcgc 31980
ccccccaaac tgcactatca atgcttcttc ccgcggtccg caaaaatagc aggacgacgc 32040
tcgccgcatt gtagtctcgc tccacgatga gccgggctgc aaaccataac ggcacgagaa 32100
cgacttcgta gagcgggttc tgaacgataa cgatgacaaa gccggcgaac atcatgaata 32160
accctgccaa tgtcagtggc accccaagaa acaatgcggg ccgtgtggct gcgaggtaaa 32220
gggtcgattc ttccaaacga tcagccatca actaccgcca gtgagcgttt ggccgaggaa 32280
gctcgcccca aacatgataa caatgccgcc gacgacgccg gcaaccagcc caagcgaagc 32340
ccgcccgaac atccaggaga tcccgatagc gacaatgccg agaacagcga gtgactggcc 32400
gaacggacca aggataaacg tgcatatatt gttaaccatt gtggcggggt cagtgccgcc 32460
acccgcagat tgcgctgcgg cgggtccgga tgaggaaatg ctccatgcaa ttgcaccgca 32520
caagcttggg gcgcagctcg atatcacgcg catcatcgca ttcgagagcg agaggcgatt 32580
tagatgtaaa cggtatctct caaagcatcg catcaatgcg cacctcctta gtataagtcg 32640
aataagactt gattgtcgtc tgcggatttg ccgttgtcct ggtgtggcgg tggcggagcg 32700
attaaaccgc cagcgccatc ctcctgcgag cggcgctgat atgacccccca aacatcccac 32760
gtctcttcgg attttagcgc ctcgtgatcg tcttttggag gctcgattaa cgcgggcacc 32820
agcgattgag cagctgtttc aacttttcgc acgtagccgt ttgcaaaacc gccgatgaaa 32880
ttaccggtgt tgtaagcgga gatcgcccga cgaagcgcaa attgcttctc gtcaatcgtt 32940
tcgccgcctg cataacgact tttcagcatg tttgcagcgg cagataatga tgtgcacgcc 33000
tggagcgcac cgtcaggtgt cagaccgagc atagaaaaat ttcgagagtt tatttgcatg 33060
aggccaacat ccagcgaatg ccgtgcatcg agacggtgcc tgacgacttg ggttgcttgg 33120
ctgtgatctt gccagtgaag cgtttcgccg gtcgtgttgt catgaatcgc taaaggatca 33180
aagcgactct ccaccttagc tatcgccgca agcgtagatg tcgcaactga tggggcacac 33240

ttgcgagcaa catggtcaaa ctcagcagat gagagtggcg tggcaaggct cgacgaacag 33300
aaggagacca tcaaggcaag agaaagcgac cccgatctct taagcatacc ttatctcctt 33360
agctcgcaac taacaccgcc tctcccgttg gaagaagtgc gttgttttat gttgaagatt 33420
atcgggaggg tcggttactc gaaaattttc aattgcttct ttatgatttc aattgaagcg 33480
agaaacctcg cccggcgtct tggaacgcaa catggaccga gaaccgcgca tccatgacta 33540
agcaaccgga tcgacctatt caggccgcag ttggtcaggt caggctcaga acgaaaatgc 33600
tcggcgaggt tacgctgtct gtaaacccat tcgatgaacg ggaagcttcc ttccgattgc 33660
tcttggcagg aatattggcc catgcctgct tgcgctttgc aaatgctctt atcgcgttgg 33720
tatcatatgc cttgtccgcc agcagaaacg cactctaagc gattatttgt aaaaatgttt 33780
cggtcatgcg gcggtcatgg gcttgacccg ctgtcagcgc aagacggatc ggtcaaccgt 33840
cggcatcgac aacagcgtga atcttggtgg tcaaaccgcc acgggaacgt cccatacagc 33900
catcgtcttg atcccgctgt ttcccgtcgc cgcatgttgg tggacgcgga cacaggaact 33960
gtcaatcatg acgacattct atcgaaagcc ttggaaatca cactcagaat atgatcccag 34020
acgtctgcct cacgccatcg tacaaagcga ttgtagcagg ttgtacagga accgtatcga 34080
tcaggaacgt ctgcccaggg cgggcccgtc cggaagcgcc acaagatgac attgatcacc 34140
cgcgtcaacg cgcggcacgc gacgcggctt atttgggaac aaaggactga acaacagtcc 34200
attcgaaatc ggtgacatca aagcggggac gggttatcag tggcctccaa gtcaagcctc 34260
aatgaatcaa aatcagaccg atttgcaaac ctgatttatg agtgtgcggc ctaaatgatg 34320
aaatcgtcct tctagatcgc ctccgtggtg tagcaacacc tcgcagtatc gccgtgctga 34380
ccttggccag ggaattgact ggcaagggtg ctttcacatg accgctcttt tggccgcgat 34440
agatgatttc gttgctgctt tgggcacgta gaaggagaga agtcatatcg gagaaattcc 34500
tcctggcgcg agagcctgct ctatcgcgac ggcatcccac tgtcgggaac agaccggatc 34560
attcacgagg cgaaagtcgt caacacatgc gttataggca tcttcccttg aaggatgatc 34620
ttgttgctgc caatctggag gtgcggcagc cgcaggcaga tgcgatctca gcgcaacttg 34680
cggcaaaaca tctcactcac ctgaaaacca ctagcgagtc tcgcgatcag acgaaggcct 34740
tttacttaac gacacaatat ccgatgtctg catcacaggc gtcgctatcc cagtcaatac 34800
taaagcggtg caggaactaa agattactga tgacttaggc gtgccacgag gcctgagacg 34860
acgcgcgtag acagtttttt gaaatcatta tcaaagtgat ggcctccgct gaagcctatc 34920
acctctgcgc cggtctgtcg gagagatggg caagcattat tacggtcttc gcgcccgtac 34980
atgcattgga cgattgcagg gtcaatggat ctgagatcat ccagaggatt gccgccctta 35040
ccttccgttt cgagttggag ccagccccta aatgagacga catagtcgac ttgatgtgac 35100
aatgccaaga gagagatttg cttaacccga ttttttgct caagcgtaag cctattgaag 35160

cttgccggca tgacgtccgc gccgaaagaa tatcctacaa gtaaaacatt ctgcacaccg 35220

aaatgcttgg tgtagacatc gattatgtga ccaagatcct tagcagtttc gcttggggac 35280

cgctccgacc agaaataccg aagtgaactg acgccaatga caggaatccc ttccgtctgc 35340

agataggtac catcgataga tctgctgcct cgcgcgtttc ggtgatgacg gtgaaaacct 35400

ctgacacatg cagctcccgg agacggtcac agcttgtctg taagcggatg ccgggagcag 35460

acaagcccgt cagggcgcgt cagcgggtgt tggcgggtgt cggggcgcag ccatgaccca 35520

gtcacgtagc gatagcggag tgtatactgg cttaactatg cggcatcaga gcagattgta 35580

ctgagagtgc accatatgcg gtgtgaaata ccgcacagat gcgtaaggag aaaataccgc 35640

atcaggcgct cttccgcttc ctcgctcact gactcgctgc gctcggtcgt tcggctgcgg 35700

cgagcggtat cagctcactc aaaggcggta atacggttat ccacagaatc aggggataac 35760

gcaggaaaga acatgtgagc aaaaggccag caaaaggcca ggaaccgtaa aaaggccgcg 35820

ttgctggcgt ttttccatag gctccgcccc cctgacgagc atcacaaaaa tcgacgctca 35880

agtcagaggt ggcgaaaccc gacaggacta taaagatacc aggcgtttcc ccctggaagc 35940

tccctcgtgc gctctcctgt tccgaccctg ccgcttaccg gatacctgtc cgcctttctc 36000

ccttcgggaa gcgtggcgct ttctcatagc tcacgctgta ggtatctcag ttcggtgtag 36060

gtcgttcgct ccaagctggg ctgtgtgcac gaaccccccg ttcagcccga ccgctgcgcc 36120

ttatccggta actatcgtct tgagtccaac ccggtaagac acgacttatc gccactggca 36180

gcagccactg gtaacaggat tagcagagcg aggtatgtag gcggtgctac agagttcttg 36240

aagtggtggc ctaactacgg ctacactaga aggacagtat ttggtatctg cgctctgctg 36300

aagccagtta ccttcggaaa aagagttggt agctcttgat ccggcaaaca aaccaccgct 36360

ggtagcggtg gtttttttgt ttgcaagcag cagattacgc gcagaaaaaa aggatctcaa 36420

gaagatcctt tgatcttttc tacggggtct gacgctcagt ggaacgaaaa ctcacgttaa 36480

gggattttgg tcatgagatt atcaaaaagg atcttcacct agatcctttt aaattaaaaa 36540

tgaagtttta aatcaatcta aagtatatat gagtaaactt ggtctgacag ttaccaatgc 36600

ttaatcagtg aggcacctat ctcagcgatc tgtctatttc gttcatccat agttgcctga 36660

ctccccgtcg tgtagataac tacgatacgg gagggcttac catctggccc cagtgctgca 36720

atgataccgc gagacccacg ctcaccggct ccagatttat cagcaataaa ccagccagcc 36780

ggaagggccg agcgcagaag tggtcctgca actttatccg cctccatcca gtctattaat 36840

tgttgccggg aagctagagt aagtagttcg ccagttaata gtttgcgcaa cgttgttgcc 36900

attgctgcag gggggggggg ggggggggac ttccattgtt cattccacgg acaaaaacag 36960

agaaaggaaa cgacagaggc caaaaagcct cgctttcagc acctgtcgtt tcctttcttt 37020

tcagagggta ttttaaataa aaacattaag ttatgacgaa gaagaacgga aacgccttaa 37080

accggaaaat tttcataaat agcgaaaacc cgcgaggtcg ccgccccgta acctgtcgga 37140

tcaccggaaa ggacccgtaa agtgataatg attatcatct acatatcaca acgtgcgtgg 37200

aggccatcaa accacgtcaa ataatcaatt atgacgcagg tatcgtatta attgatctgc 37260

atcaacttaa cgtaaaaaca acttcagaca atacaaatca gcgacactga atacggggca 37320

acctcatgtc cccccccccc cccccctgc aggcatcgtg gtgtcacgct cgtcgtttgg 37380

tatggcttca ttcagctccg gttcccaacg atcaaggcga gttacatgat cccccatgtt 37440

gtgcaaaaaa gcggttagct ccttcggtcc tccgatcgtt gtcagaagta agttggccgc 37500

agtgttatca ctcatggtta tggcagcact gcataattct cttactgtca tgccatccgt 37560

aagatgcttt tctgtgactg gtgagtactc aaccaagtca ttctgagaat agtgtatgcg 37620

gcgaccgagt tgctcttgcc cggcgtcaac acgggataat accgcgccac atagcagaac 37680

tttaaaagtg ctcatcattg gaaaacgttc ttcggggcga aaactctcaa ggatcttacc 37740

gctgttgaga tccagttcga tgtaacccac tcgtgcaccc aactgatctt cagcatcttt 37800

tactttcacc agcgtttctg ggtgagcaaa aacaggaagg caaaatgccg caaaaaaggg 37860

aataagggcg acacggaaat gttgaatact catactcttc ctttttcaat attattgaag 37920

catttatcag ggttattgtc tcatgagcgg atacatattt gaatgtattt agaaaaataa 37980

acaaataggg gttccgcgca catttccccg aaaagtgcca cctgacgtct aagaaaccat 38040

tattatcatg acattaacct ataaaaatag gcgtatcacg aggccctttc gtcttcaaga 38100

attggtcgac gatcttgctg cgttcggata ttttcgtgga gttcccgcca cagacccgga 38160

ttgaaggcga gatccagcaa ctcgcgccag atcatcctgt gacggaactt tggcgcgtga 38220

tgactggcca ggacgtcggc cgaaagagcg acaagcagat cacgcttttc gacagcgtcg 38280

gatttgcgat cgaggatttt tcggcgctgc gctacgtccg cgaccgcgtt gagggatcaa 38340

gccacagcag cccactcgac cttctagccg acccagacga gccaagggat ctttttggaa 38400

tgctgctccg tcgtcaggct ttccgacgtt tgggtggttg aacagaagtc attatcgtac 38460

ggaatgccaa gcactcccga ggggaaccct gtggttggca tgcacataca aatggacgaa 38520

cggataaacc ttttcacgcc cttttaaata tccgttattc taataaacgc tcttttctct 38580

taggtttacc cgccaatata tcctgtcaaa cactgatagt ttaaactgaa ggcgggaaac 38640

gacaatctga tcatgagcgg agaattaagg gagtcacgtt atgacccccg ccgatgacgc 38700

gggacaagcc gttttacgtt tggaactgac agaaccgcaa cgttgaagga gccactcagc 38760

aagctggtac gattgtaata cgactcacta tagggcgaat tgagcgctgt ttaaacgctc 38820

ttcaactgga agagcggtta cccggaccga agcttgaagt tcctattccg aagttcctat 38880

tctctagaaa gtataggaac ttcagatctc gatgctcacc ctgttgtttg gtgttacttc 38940

tgcaggtcga ctctagagga tccaccatga gcccagaacg acgcccggcc gacatccgcc 39000

gtgccaccga ggcggacatg ccggcggtct gcaccatcgt caaccactac atcgagacaa 39060

gcacggtcaa cttccgtacc gagccgcagg aaccgcagga ctggacggac gacctcgtcc 39120

gtctgcggga gcgctatccc tggctcgtcg ccgaggtgga cggcgaggtc gccggcatcg 39180

cctacgcggg cccctggaag gcacgcaacg cctacgactg gacggccgag tcgaccgtgt 39240

acgtctcccc ccgccaccag cggacgggac tgggctccac gctctacacc cacctgctga 39300

agtccctgga ggcacagggc ttcaagagcg tggtcgctgt catcgggctg cccaacgacc 39360

cgagcgtgcg catgcacgag gcgctcggat atgccccccg cggcatgctg cgggcggccg 39420

gcttcaagca cgggaactgg catgacgtgg gtttctggca gctggacttc agcctgccgg 39480

taccgccccg tccggtcctg cccgtcaccg agatctgatc cgtcgaccaa cctagacttg 39540

tccatcttct ggattggcca acttaattaa tgtatgaaat aaaaggatgc acacatagtg 39600

acatgctaat cactataatg tgggcatcaa agttgtgtgt tatgtgtaat tactagttat 39660

ctgaataaaa gagaaagaga tcatccatat ttcttatcct aaatgaatgt cacgtgtctt 39720

tataattctt tgatgaacca gatgcatttc attaaccaaa tccatataca tataaatatt 39780

aatcatatat aattaatatc aattgggtta gcaaaacaaa tctagtctag gtgtgttttg 39840

cgaattgcgg ccgcgatctg gggaattccc atggacaccg gtaattccca tgatcttctc 39900

tccttcatca atggatgcca tgtttcataa caataacacc aaatgtttga tgagctacca 39960

acaattgcgc aaagactatg gctaagctcg agctcgctcg ctacaagttg ttgactttca 40020

aatacaagtt tgtttttgga acaccaaata ttctacatga tctttcacta agttgcgcac 40080

cactatcaaa agattatcta ggccattatt caagtaaaga gtgaacacgt ctaagaccca 40140

caaccacacc aaatagaata cgcatacatg caacatattg tgcaagaagt atccaactgg 40200

actcccatgt attctaaaac tattttcgta gagttaaagt tatgacaaac ttatcaaata 40260

aaaatttgaa cgctggacca aaactttcat ctttcaaatc caccatcgtc tatcctcata 40320

aattgttttg attataacac atctacgtaa atcatttgtt ttgaacaata ctaatttaat 40380

tttattaagt caaataacct gcttagaaaa taatccctcc acctcattta acaatttctt 40440

gtcaaacaca caccaagaaa aaaattaatg aaagagaaaa gaaatgaaaa ggacatggag 40500

ttgaatacta gcaaaattga ttgaaggaag attcacaatt gaaattgaaa ccatttaatt 40560

tattttcggg tccataataa taaattggta agaataaaaa cccgatcaag tccggtacag 40620

tacaattcca ctccaccaac tccttactta aacccctatt tatacccact ctcatcctca 40680

ctcttccttc acctctcaca ctctcttctc tctctcaaaa ccctcacaca aacgctgcgt 40740

ttagtgtaag aaattcaatc cggcgccttg gcgcgccgat catccacaag tttgtacaaa 40800

aaagctgaac gagaaacgta aaatgatata aatatcaata tattaaatta gattttgcat 40860

aaaaaacaga ctacataata ctgtaaaaca caacatatcc agtcactatg gcggccgcat 40920

taggcacccc aggctttaca ctttatgctt ccggctcgta taatgtgtgg attttgagtt 40980

aggatttaaa tacgcgttga tccggcttac taaaagccag ataacagtat gcgtatttgc 41040

gcgctgattt ttgcggtata agaatatata ctgatatgta tacccgaagt atgtcaaaaa 41100

gaggtatgct atgaagcagc gtattacagt gacagttgac agcgacagct atcagttgct 41160

caaggcatat atgatgtcaa tatctccggt ctggtaagca caaccatgca gaatgaagcc 41220

cgtcgtctgc gtgccgaacg ctggaaagcg gaaaatcagg aagggatggc tgaggtcgcc 41280

cggtttattg aaatgaacgg ctcttttgct gacgagaaca ggggctggtg aaatgcagtt 41340

taaggtttac acctataaaa gagagagccg ttatcgtctg tttgtggatg tacagagtga 41400

tatcattgac acgcccggtc gacggatggt gatccccctg gccagtgcac gtctgctgtc 41460

agataaagtc tcccgtgaac tttacccggt ggtgcatatc ggggatgaaa gctggcgcat 41520

gatgaccacc gatatggcca gtgtgccggt ctccgttatc ggggaagaag tggctgatct 41580

cagccaccgc gaaaatgaca tcaaaaacgc cattaacctg atgttctggg gaatataaat 41640

gtcaggctcc cttatacaca gccagtctgc aggtcgacca tagtgactgg atatgttgtg 41700

ttttacagta ttatgtagtc tgttttttat gcaaaatcta atttaatata ttgatattta 41760

tatcatttta cgtttctcgt tcagctttct tgtacaaagt ggtgttaacc tagacttgtc 41820

catcttctgg attggccaac ttaattaatg tatgaaataa aaggatgcac acatagtgac 41880

atgctaatca ctataatgtg ggcatcaaag ttgtgtgtta tgtgtaatta ctagttatct 41940

gaataaaaga gaaagagatc atccatattt cttatcctaa atgaatgtca cgtgtcttta 42000

taattctttg atgaaccaga tgcatttcat taaccaaatc catatacata taaatattaa 42060

tcatatataa ttaatatcaa ttgggttagc aaaacaaatc tagtctaggt gtgttttgcg 42120

aattgcggcc gccaccgcgg tggagctcga attccggtcc gggtcacctt tgtccaccaa 42180

gatggaactg cggccgctca ttaattaagt caggcgcgcc tctagttgaa gacacgttca 42240

tgtcttcatc gtaagaagac actcagtagt cttcggccag aatggccatc tggattcagc 42300

aggcctagaa ggccatttaa atcctgagga tctggtcttc ctaaggaccc gggatatcgg 42360

accgattaaa ctttaattcg gtccgaagct tgaagttcct attccgaagt tcctattctc 42420

cagaaagtat aggaacttcg catgcctgca gtgcagcgtg acccggtcgt gcccctctct 42480

agagataatg agcattgcat gtctaagtta taaaaaatta ccacatattt tttttgtcac 42540

acttgtttga agtgcagttt atctatcttt atacatatat ttaaacttta ctctacgaat 42600

aatataatct atagtactac aataatatca gtgttttaga gaatcatata aatgaacagt 42660

tagacatggt ctaaaggaca attgagtatt ttgacaacag gactctacag ttttatcttt 42720

ttagtgtgca tgtgttctcc tttttttttg caaatagctt cacctatata atacttcatc 42780

cattttatta gtacatccat ttagggttta gggttaatgg tttttataga ctaatttttt 42840

tagtacatct attttattct attttagcct ctaaattaag aaaactaaaa ctctatttta 42900

gtttttttat ttaataattt agatataaaa tagaataaaa taaagtgact aaaaattaaa 42960

caaataccct ttaagaaatt aaaaaaacta aggaaacatt tttcttgttt cgagtagata 43020
atgccagcct gttaaacgcc gtcgacgagt ctaacggaca ccaaccagcg aaccagcagc 43080
gtcgcgtcgg gccaagcgaa gcagacggca cggcatctct gtcgctgcct ctggacccct 43140
ctcgagagtt ccgctccacc gttggacttg ctccgctgtc ggcatccaga aattgcgtgg 43200
cggagcggca gacgtgagcc ggcacggcag gcggcctcct cctcctctca cggcaccggc 43260
agctacgggg gattcctttc ccaccgctcc ttcgctttcc cttcctcgcc cgccgtaata 43320
aatagacacc ccctccacac cctctttccc caacctcgtg ttgttcggag cgcacacaca 43380
cacaaccaga tctcccccaa atccacccgt cggcacctcc gcttcaaggt acgccgctcg 43440
tcctcccccc ccccctctc taccttctct agatcggcgt tccggtccat gcatggttag 43500
ggcccggtag ttctacttct gttcatgttt gtgttagatc cgtgtttgtg ttagatccgt 43560
gctgctagcg ttcgtacacg gatgcgacct gtacgtcaga cacgttctga ttgctaactt 43620
gccagtgttt ctctttgggg aatcctggga tggctctagc cgttccgcag acgggatcga 43680
tttcatgatt ttttttgttt cgttgcatag ggtttggttt gccctttcc tttatttcaa 43740
tatatgccgt gcacttgttt gtcgggtcat cttttcatgc ttttttttgt cttggttgtg 43800
atgatgtggt ctggttgggc ggtcgttcta gatcggagta gaattctgtt tcaaactacc 43860
tggtggattt attaattttg gatctgtatg tgtgtgccat acatattcat agttacgaat 43920
tgaagatgat ggatggaaat atcgatctag gataggtata catgttgatg cgggttttac 43980
tgatgcatat acagagatgc tttttgttcg cttggttgtg atgatgtggt gtggttgggc 44040
ggtcgttcat tcgttctaga tcggagtaga atactgtttc aaactacctg gtgtatttat 44100
taattttgga actgtatgtg tgtgtcatac atcttcatag ttacgagttt aagatggatg 44160
gaaatatcga tctaggatag gtatacatgt tgatgtgggt tttactgatg catatacatg 44220
atggcatatg cagcatctat tcatatgctc taaccttgag tacctatcta ttataataaa 44280
caagtatgtt ttataattat tttgatcttg atatacttgg atgatggcat atgcagcagc 44340
tatatgtgga tttttttagc cctgccttca tacgctattt atttgcttgg tactgtttct 44400
tttgtcgatg ctcaccctgt tgtttggtgt tacttctgca ggtcgacttt aacttagcct 44460
aggatccaca cgacaccatg atagaggtga aaccgattaa cgcagaggat acctatgaac 44520
taaggcatag aatactcaga ccaaaccagc cgatagaagc gtgtatgttt gaaagcgatt 44580
tacttcgtgg tgcatttcac ttaggcggct attacggggg caaactgatt tccatagctt 44640
cattccacca ggccgagcac tcagaactcc aaggccagaa acagtaccag ctccgaggta 44700
tggctacctt ggaaggttat cgtgagcaga aggcgggatc gagtctaatt aaacacgctg 44760
aagaaattct tcgtaagagg ggggcggact tgctttggtg taatgcgcgg acatccgcct 44820
caggctacta caaaaagtta ggcttcagcg agcagggaga ggtattcgac acgccgccag 44880
taggacctca catcctgatg tataaaagga tcacataact agctagtcag ttaacctaga 44940

cttgtccatc ttctggattg gccaacttaa ttaatgtatg aaataaaagg atgcacacat 45000
agtgacatgc taatcactat aatgtgggca tcaaagttgt gtgttatgtg taattactag 45060
ttatctgaat aaaagagaaa gagatcatcc atatttctta tcctaaatga atgtcacgtg 45120
tctttataat tctttgatga accagatgca tttcattaac caaatccata tacatataaa 45180
tattaatcat atataattaa tatcaattgg gttagcaaaa caaatctagt ctaggtgtgt 45240
tttgcgaatt cagagctcga attcattccg attaatcgtg gcctcttgct cttcaggatg 45300
aagagctatg tttaaacgtg caagcgctac tagacaattc agtacattaa aaacgtccgc 45360
aatgtgttat taagttgtct aagcgtcaat ttgtttacac cacaatatat cctgccacca 45420
gccagccaac agctccccga ccggcagctc ggcacaaaat caccactcga tacaggcagc 45480
ccatcagtcc gggacggcgt cagcgggaga gccgttgtaa ggcggcagac tttgctcatg 45540
ttaccgatgc tattcggaag aacggcaact aagctgccgg gtttgaaaca cggatgatct 45600
cgcggagggt agcatgttga ttgtaacgat gacagagcgt tgctgcctgt gatcaaatat 45660
catctccctc gcagagatcc gaattatcag ccttcttatt catttctcgc ttaaccgtga 45720
caggctgtcg atcttgagaa ctatgccgac ataataggaa atcgctggat aaagccgctg 45780
aggaagctga gtggcgctat ttctttagaa gtgaacgttg acgatcgtcg accgtacccc 45840
gatgaattaa ttcggacgta cgttctgaac acagctggat acttacttgg gcgattgtca 45900
tacatgacat caacaatgta cccgtttgtg taaccgtctc ttggaggttc gtatgacact 45960
agtggttccc ctcagcttgc gactagatgt tgaggcctaa cattttatta gagagcaggc 46020
tagttgctta gatacatgat cttcaggccg ttatctgtca gggcaagcga aaattggcca 46080
tttatgacga ccaatgcccc gcagaagctc ccatctttgc cgccatagac gccgcgcccc 46140
ccttttgggg tgtagaacat ccttttgcca gatgtggaaa agaagttcgt tgtcccattg 46200
ttggcaatga cgtagtagcc ggcgaaagtg cgagacccat ttgcgctata tataagccta 46260
cgatttccgt tgcgactatt gtcgtaattg gatgaactat tatcgtagtt gctctcagag 46320
ttgtcgtaat ttgatggact attgtcgtaa ttgcttatgg agttgtcgta gttgcttgga 46380
gaaatgtcgt agttggatgg ggagtagtca tagggaagac gagcttcatc cactaaaaca 46440
attggcaggt cagcaagtgc ctgccccgat gccatcgcaa gtacgaggct tagaaccacc 46500
ttcaacagat cgcgcatagt cttccccagc tctctaacgc ttgagttaag ccgcgccgcg 46560
aagcggcgtc ggcttgaacg aattgttaga cattatttgc cgactacctt ggtgatctcg 46620
cctttcacgt agtgaacaaa ttcttccaac tgatctgcgc gcgaggccaa gcgatcttct 46680
tgtccaagat aagcctgcct agcttcaagt atgacgggct gatactgggc cggcaggcgc 46740
tccattgccc agtcggcagc gacatccttc ggcgcgattt tgccggttac tgcgctgtac 46800
caaatgcggg acaacgtaag cactacattt cgctcatcgc cagcccagtc gggcggcgag 46860

ttccatagcg ttaaggtttc atttagcgcc tcaaatagat cctgttcagg aaccggatca 46920

aagagttcct ccgccgctgg acctaccaag gcaacgctat gttctcttgc ttttgtcagc 46980

aagatagcca gatcaatgtc gatcgtggct ggctcgaaga tacctgcaag aatgtcattg 47040

cgctgccatt ctccaaattg cagttcgcgc ttagctggat aacgccacgg aatgatgtcg 47100

tcgtgcacaa caatggtgac ttctacagcg cggagaatct cgctctctcc aggggaagcc 47160

gaagtttcca aaaggtcgtt gatcaaagct cgccgcgttg tttcatcaag ccttacagtc 47220

accgtaacca gcaaatcaat atcactgtgt ggcttcaggc cgccatccac tgcggagccg 47280

tacaaatgta cggccagcaa cgtcggttcg agatggcgct cgatgacgcc aactacctct 47340

gatagttgag tcgatacttc ggcgatcacc gcttccctca tgatgtttaa ctcctgaatt 47400

aagccgcgcc gcgaagcggt gtcggcttga atgaattgtt aggcgtcatc ctgtgctccc 47460

gagaaccagt accagtacat cgctgtttcg ttcgagactt gaggtctagt tttatacgtg 47520

aacaggtcaa tgccgccgag agtaaagcca cattttgcgt acaaattgca ggcaggtaca 47580

ttgttcgttt gtgtctctaa tcgtatgcca aggagctgtc tgcttagtgc ccactttttc 47640

gcaaattcga tgagactgtg cgcgactcct ttgcctcggt gcgtgtgcga cacaacaatg 47700

tgttcgatag aggctagatc gttccatgtt gagttgagtt caatcttccc gacaagctct 47760

tggtcgatga atgcgccata gcaagcagag tcttcatcag agtcatcatc cgagatgtaa 47820

tccttccggt aggggctcac acttctggta gatagttcaa agccttggtc ggataggtgc 47880

acatcgaaca cttcacgaac aatgaaatgg ttctcagcat ccaatgtttc cgccacctgc 47940

tcagggatca ccgaaatctt catatgacgc ctaacgcctg gcacagcgga tcgcaaacct 48000

ggcgcggctt ttggcacaaa aggcgtgaca ggtttgcgaa tccgttgctg ccacttgtta 48060

acccttttgc cagatttggt aactataatt tatgttagag gcgaagtctt gggtaaaaac 48120

tggcctaaaa ttgctgggga tttcaggaaa gtaaacatca ccttccggct cgatgtctat 48180

tgtagatata tgtagtgtat ctacttgatc gggggatctg ctgcctcgcg cgtttcggtg 48240

atgacggtga aaacctctga cacatgcagc tcccggagac ggtcacagct tgtctgtaag 48300

cggatgccgg gagcagacaa gcccgtcagg gcgcgtcagc gggtgttggc gggtgtcggg 48360

gcgcagccat gacccagtca cgtagcgata gcggagtgta tactggctta actatgcggc 48420

atcagagcag attgtactga gagtgcacca tatgcggtgt gaaataccgc acagatgcgt 48480

aaggagaaaa taccgcatca ggcgctcttc cgcttcctcg ctcactgact cgctgcgctc 48540

ggtcgttcgg ctgcggcgag cggtatcagc tcactcaaag gcggtaatac ggttatccac 48600

agaatcaggg gataacgcag gaaagaacat gtgagcaaaa ggccagcaaa aggccaggaa 48660

ccgtaaaaag gccgcgttgc tggcgttttt ccataggctc cgcccccctg acgagcatca 48720

caaaaatcga cgctcaagtc agaggtggcg aaacccgaca ggactataaa gataccaggc 48780

gtttccccct ggaagctccc tcgtgcgctc tcctgttccg accctgccgc ttaccggata 48840

cctgtccgcc tttctccctt cgggaagcgt ggcgctttct catagctcac gctgtaggta 48900

tctcagttcg gtgtaggtcg ttcgctccaa gctgggctgt gtgcacgaac cccccgttca 48960

gcccgaccgc tgcgccttat ccggtaacta tcgtcttgag tccaacccgg taagacacga 49020

cttatcgcca ctggcagcag ccactggtaa caggattagc agagcgaggt atgtaggcgg 49080

tgctacagag ttcttgaagt ggtggcctaa ctacggctac actagaagga cagtatttgg 49140

tatctgcgct ctgctgaagc cagttacctt cggaaaaaga gttggtagct cttgatccgg 49200

caaacaaacc accgctggta gcggtggttt ttttgtttgc aagcagcaga ttacgcgcag 49260

aaaaaaagga tctcaagaag atcctttgat cttttctacg gggtctgacg ctcagtggaa 49320

cgaaaactca cgttaaggga ttttggtcat gagattatca aaaaggatct tcacctagat 49380

ccttttaaat taaaaatgaa gttttaaatc aatctaaagt atatatgagt aaacttggtc 49440

tgacagttac caatgcttaa tcagtgaggc acctatctca gcgatctgtc tatttcgttc 49500

atccatagtt gcctgactcc ccgtcgtgta gataactacg atacgggagg gcttaccatc 49560

tggccccagt gctgcaatga taccgcgaga cccacgctca ccggctccag atttatcagc 49620

aataaaccag ccagccggaa gggccgagcg cagaagtggt cctgcaactt tatccgcctc 49680

catccagtct attaattgtt gccgggaagc tagagtaagt agttcgccag ttaatagttt 49740

gcgcaacgtt gttgccattg ctgca 49765

<210> 14
<211> 1100
<212> DNA
<213> Zea mays

<400> 14
catcgcatcg catcgcatcg cgcgctttgc ctgccgcagc ggctaacgaa cgcgtggatc 60

atcccttcta ttattaatta attgggagga gtgggagtga tagctgcagc acccagcgcc 120

catcactgag cccatccgga accctagcct cctcgtcgtt gaatccacca caccatgagc 180

gggagcgcct ccaggaagct cttccaggcc gccaggtcca tcgtcctctc cgcttctcaa 240

cgctcctcct tctccgtcct cgccgccgag ggccgcaccg ccgcgctcgc caactttggc 300

aggaagatcc tccccagcgc ctgctcctac cacaggcagg gatcccacgc cgcgtcggga 360

tggggagcca tcgctgccgc agtccccgcc gcagtttaca tgctccagga ccaggaggct 420

catgctgcag agatggagcg cactttcatc gccatcaagc ccgacggcgt ccaaagaggc 480

ctgatttctg agattgtgaa ccgattcgag agaaaaggct acaagcttgt tgccatcaag 540

ctgattgtcc catccaaagg attcgctgag aagcactacc atgatctcaa ggaaaggcct 600

ttcttcaacg ggttgtgtga cttcctcagc tctggccctg tacttgcaat ggtttgggaa 660

ggagagggtg tcatcaagta tgggagaaaa ctaattggtg ccacagaccc acagaaatct 720

gaaccaggaa ccatcagggg cgatcttggt gttgttgtgg gaagaaacat cattcatgga 780

```
agtgatggcc cggagacagc aaaggatgag atcgccttgt ggtttgaacc caaggagctg     840

gtctcttata ccagcaatgc ggagaagtgg gtctatgggg tgaattaacc agcgactaaa     900

tctcttgagg tttgcttttt tcttttcagt tttcacataa ttgccgacag accaaggcag     960

gatgtaataa agtagctacc gtcacctatt acttttgagt tggattgatt gtggtgcagt    1020

aaggcacaag gaaaggaagg aatacaccgt gaaatttgtt aaaaaaaaaa aaaaaaaaaa    1080

aaaaaaaaaa aaaaaaaaaa                                                1100
```

<210> 15
<211> 237
<212> PRT
<213> Zea mays

<400> 15

```
Met Ser Gly Ser Ala Ser Arg Lys Leu Phe Gln Ala Ala Arg Ser Ile
1               5                   10                  15

Val Leu Ser Ala Ser Gln Arg Ser Ser Phe Ser Val Leu Ala Ala Glu
            20                  25                  30

Gly Arg Thr Ala Ala Leu Ala Asn Phe Gly Arg Lys Ile Leu Pro Ser
        35                  40                  45

Ala Cys Ser Tyr His Arg Gln Gly Ser His Ala Ala Ser Gly Trp Gly
    50                  55                  60

Ala Ile Ala Ala Ala Val Pro Ala Ala Val Tyr Met Leu Gln Asp Gln
65                  70                  75                  80

Glu Ala His Ala Ala Glu Met Glu Arg Thr Phe Ile Ala Ile Lys Pro
                85                  90                  95

Asp Gly Val Gln Arg Gly Leu Ile Ser Glu Ile Val Asn Arg Phe Glu
            100                 105                 110

Arg Lys Gly Tyr Lys Leu Val Ala Ile Lys Leu Ile Val Pro Ser Lys
        115                 120                 125

Gly Phe Ala Glu Lys His Tyr His Asp Leu Lys Glu Arg Pro Phe Phe
    130                 135                 140

Asn Gly Leu Cys Asp Phe Leu Ser Ser Gly Pro Val Leu Ala Met Val
145                 150                 155                 160

Trp Glu Gly Glu Gly Val Ile Lys Tyr Gly Arg Lys Leu Ile Gly Ala
                165                 170                 175
```

```
Thr Asp Pro Gln Lys Ser Glu Pro Gly Thr Ile Arg Gly Asp Leu Gly
            180                 185                 190

Val Val Val Gly Arg Asn Ile Ile His Gly Ser Asp Gly Pro Glu Thr
        195                 200                 205

Ala Lys Asp Glu Ile Ala Leu Trp Phe Glu Pro Lys Glu Leu Val Ser
    210                 215                 220

Tyr Thr Ser Asn Ala Glu Lys Trp Val Tyr Gly Val Asn
225                 230                 235


<210>  16
<211>  1242
<212>  DNA
<213>  Zea mays

<400>  16
ggaaaccttt gcgtttttta agcgagccac gccaataacg gagaggcatg caccgacgat      60

cggcatttcc ttccagtggc ggtgagaagg gaaagggagg acagcagagc agaggtcgag     120

catcgcatcg catcgcatcg cgcgctttgc ctgccgcagc ggctaacgaa cgcgtggatc     180

atcccttcta ttattaatta attgggagga gtgggagtga tagctgcagc acccagcgcc     240

catcactgag cccatccgga accctagcct cctcgtcgtt gaatccacca caccatgagc     300

gggagcgcct ccaggaagct cttccaggcc gccaggtcca tcgtcctctc cgcttctcaa     360

cgctcctcct tctccgtcct cgccgccgag ggccgcaccg ccgcgctcgc caactttggc     420

aggaagatcc tccccagcgc ctgctcctac cacaggcagg gatcccacgc cgcgtcggga     480

tggggagcca tcgctgccgc agtccccgcc gcagtttaca tgctccagga ccaggaggct     540

catgctgcag agatggagcg cactttcatc gccatcaagc ccgacggcgt ccaaagaggc     600

ctgatttctg agattgtgaa ccgattcgag agaaaaggct acaagcttgt tgccatcaag     660

ctcattgtcc catccaaagg attcgctgag aagcactacc atgatctcaa ggaaaggcct     720

ttcttcaacg ggttgtgtga cttcctcagc tctggccctg tacttgcaat ggtttgggaa     780

ggagagggtg tcatcaagta tgggagaaaa ctaattggtg ccacagaccc acagaaatct     840

gaaccaggaa ccatcagggg cgatcttggt gttgttgtgg gaagaaacat cattcatgga     900

agtgatggcc cggagacagc aaaggatgag atcgccttgt ggtttgaacc caaggagctg     960

gtctcttata ccagcaatgc ggagaagtgg gtctatgggg tgaattaacc agcgactaaa    1020

tctcttgagg tttgcttttt tcttttcagt tttcacataa ttgccgacag accaaggcag    1080

gatgtaataa agtagctacc gtcacctatt acttttgagt tggattgatt gtggtgcagt    1140

aaggcacaag gaaaggaagg aatacaccgt gaaatttgtt aaaaaaaaaa aaaaaaaaaa    1200

aaaaaaaaaa aaaaaaaaaa aaaaaaaaaa aaaaaaaaaa aa                       1242
```

<210> 17
<211> 237
<212> PRT
<213> Zea mays

<400> 17

```
Met Ser Gly Ser Ala Ser Arg Lys Leu Phe Gln Ala Ala Arg Ser Ile
1               5                   10                  15

Val Leu Ser Ala Ser Gln Arg Ser Ser Phe Ser Val Leu Ala Ala Glu
            20                  25                  30

Gly Arg Thr Ala Ala Leu Ala Asn Phe Gly Arg Lys Ile Leu Pro Ser
        35                  40                  45

Ala Cys Ser Tyr His Arg Gln Gly Ser His Ala Ala Ser Gly Trp Gly
    50                  55                  60

Ala Ile Ala Ala Ala Val Pro Ala Ala Val Tyr Met Leu Gln Asp Gln
65                  70                  75                  80

Glu Ala His Ala Ala Glu Met Glu Arg Thr Phe Ile Ala Ile Lys Pro
                85                  90                  95

Asp Gly Val Gln Arg Gly Leu Ile Ser Glu Ile Val Asn Arg Phe Glu
            100                 105                 110

Arg Lys Gly Tyr Lys Leu Val Ala Ile Lys Leu Ile Val Pro Ser Lys
        115                 120                 125

Gly Phe Ala Glu Lys His Tyr His Asp Leu Lys Glu Arg Pro Phe Phe
    130                 135                 140

Asn Gly Leu Cys Asp Phe Leu Ser Ser Gly Pro Val Leu Ala Met Val
145                 150                 155                 160

Trp Glu Gly Glu Gly Val Ile Lys Tyr Gly Arg Lys Leu Ile Gly Ala
                165                 170                 175

Thr Asp Pro Gln Lys Ser Glu Pro Gly Thr Ile Arg Gly Asp Leu Gly
            180                 185                 190

Val Val Val Gly Arg Asn Ile Ile His Gly Ser Asp Gly Pro Glu Thr
        195                 200                 205

Ala Lys Asp Glu Ile Ala Leu Trp Phe Glu Pro Lys Glu Leu Val Ser
    210                 215                 220

Tyr Thr Ser Asn Ala Glu Lys Trp Val Tyr Gly Val Asn
225                 230                 235
```

<210> 18
<211> 1102
<212> DNA
<213> Zea mays

<400> 18
agcgaccgca ccggcacggc gcccatccat ccatcgccat cctctagccc agtccaaagg 60

aaccctagcc atgagcgccg cctccaagaa gctcttccag gccgccaggt ccctcgtcct 120

ctccgcttct caccgctccg tcctcgccgc cgagggccgc accgccgcgc tcgccacgct 180

caccaacttt ggcaggaaga ccctccccac cgcctactta tcctaccaca agcagggatc 240

ccaccatgcc gcgtcggggt ggggagccat cgccgccgca gtcccagctg cagtttacat 300

gctccaggac caggaggctc atgctgcaga gatggagcgc accttcattg ccatcaagcc 360

tgatggcgtc caaagaggcc tgatttctga gattatgagc cgatttgaga gaaaaggcta 420

taagcttgtt gccatcaagc tgattgttcc atccaaagaa tttgctgaga agcactacca 480

tgatctcaag gaaaggcctt tcttcagtgg gttgtgtgat tttctcagct ctggccctgt 540

gcttgcaatg gtttgggaag gagagggtgt catcaagtat gggagaaaac taattggtgc 600

cacagaccca cagaaatctg aaccaggaac catcaggggc gatcttgcca ttgttgttgg 660

aagaaacatt attcatggaa gtgatggccc agagacagcg aaggatgaga tcgctttatg 720

gtttgaaccc aaggagctgg tctcttacac cagcaatgcg gagaagtgga tctatggggt 780

gaattaacga gagagtcaat ctgttttttt tcccttcttt tgatctcggt tttcacataa 840

ttgccgacag acctaggcac aaggatgtaa taaagtcgct accgtcactt cgagttggat 900

tgtgaccttc aggtgtagta aggcacaagg aaggatggaa aggaaaggaa tataccgtga 960

aatatagagc gtgcactgag tagtcgcgat gttcaaatca aactatatat caccgtcact 1020

ggagtcatgt aatccaagat ggcgatgcag atgtttcttt ggatctaaaa aaaaaaaaaa 1080

aaaaaaaaaa aaaacctcgt gc 1102

<210> 19
<211> 238
<212> PRT
<213> Zea mays

<400> 19

Met Ser Ala Ala Ser Lys Lys Leu Phe Gln Ala Ala Arg Ser Leu Val
1 5 10 15

Leu Ser Ala Ser His Arg Ser Val Leu Ala Ala Glu Gly Arg Thr Ala
20 25 30

Ala Leu Ala Thr Leu Thr Asn Phe Gly Arg Lys Thr Leu Pro Thr Ala
35 40 45

Tyr Leu Ser Tyr His Lys Gln Gly Ser His His Ala Ala Ser Gly Trp
    50                  55                  60

Gly Ala Ile Ala Ala Ala Val Pro Ala Ala Val Tyr Met Leu Gln Asp
65                  70                  75                  80

Gln Glu Ala His Ala Ala Glu Met Glu Arg Thr Phe Ile Ala Ile Lys
                85                  90                  95

Pro Asp Gly Val Gln Arg Gly Leu Ile Ser Glu Ile Met Ser Arg Phe
            100                 105                 110

Glu Arg Lys Gly Tyr Lys Leu Val Ala Ile Lys Leu Ile Val Pro Ser
        115                 120                 125

Lys Glu Phe Ala Glu Lys His Tyr His Asp Leu Lys Glu Arg Pro Phe
    130                 135                 140

Phe Ser Gly Leu Cys Asp Phe Leu Ser Ser Gly Pro Val Leu Ala Met
145                 150                 155                 160

Val Trp Glu Gly Glu Gly Val Ile Lys Tyr Gly Arg Lys Leu Ile Gly
                165                 170                 175

Ala Thr Asp Pro Gln Lys Ser Glu Pro Gly Thr Ile Arg Gly Asp Leu
            180                 185                 190

Ala Ile Val Val Gly Arg Asn Ile Ile His Gly Ser Asp Gly Pro Glu
        195                 200                 205

Thr Ala Lys Asp Glu Ile Ala Leu Trp Phe Glu Pro Lys Glu Leu Val
    210                 215                 220

Ser Tyr Thr Ser Asn Ala Glu Lys Trp Ile Tyr Gly Val Asn
225                 230                 235

<210>  20
<211>  1066
<212>  DNA
<213>  Cyamopsis tetragonoloba

<400>  20
gcttctctgt agacgaagaa gatggcctca caggtttgca aatctgcttc cagagccgcg      60

aggtctcttc tctctgcttc caaggcctct cgtttctact cacaaggaag cgccgctgcg     120

gctgttacat taggccgtaa agtgcctttt tatgcttcca actatggaag gagcggttct     180

agaagtggat ccgccgcatg gattccagga gctctcgctc ttcctgctgc agcttacatg     240

ttccaagatc aggaagtgca cgctgctgag ctggagcgca ctttcattgc cattaagcct     300

gacggagttc agagagggct gattgcagag attctatcac gttttgagcg caaagggtac 360

aagcttgtgg ggattaaagt agtgattcct acaaaggagt ttgcccaaca acattatcat 420

gacctgaaag agagaccctt cttcaacggg ctttgtgaat tccttagctc tggccctgtc 480

attgcaatgg tgtgggaagg acaaggagtt attacttacg gacggaaact aattggagcc 540

acagatccac agaaatcaga gcctggaacc attaggggtg acctggctgt tgttgttgga 600

agaaatatca tccatgggag tgatggtcca gagactgcca aggaggagat taagttgtgg 660

tttaagccag aggagctggt tagtttcact agcaatgcag agaagtggat ttatggcgaa 720

aactgattct ttccctcacc tattcgattt tcagaataaa taaattattt gttagtaaag 780

ggtaatgctg aactctagaa gccacaaata agcgggattg agaatagtac cgtgagcact 840

atctatttag ccttttaggt gttaccgatg agtcaaaaat aggtcaacag tctttcttct 900

tcttcttctt cttttttta atagccaaaa aggattatgc atctatctat ctattctatt 960

ctctgaagtt agagaacaaa tggttaattt aagtggtaaa tgtttcattt ctcttaaata 1020

aataaagttg tggaaaaaaa aaaaaaaaaa aaaaaaaaaa aaaaaa 1066

<210> 21
<211> 234
<212> PRT
<213> Cyamopsis tetragonoloba

<400> 21

Met Ala Ser Gln Val Cys Lys Ser Ala Ser Arg Ala Ala Arg Ser Leu
1 5 10 15

Leu Ser Ala Ser Lys Ala Ser Arg Phe Tyr Ser Gln Gly Ser Ala Ala
20 25 30

Ala Ala Val Thr Leu Gly Arg Lys Val Pro Phe Tyr Ala Ser Asn Tyr
35 40 45

Gly Arg Ser Gly Ser Arg Ser Gly Ser Ala Ala Trp Ile Pro Gly Ala
50 55 60

Leu Ala Leu Pro Ala Ala Ala Tyr Met Phe Gln Asp Gln Glu Val His
65 70 75 80

Ala Ala Glu Leu Glu Arg Thr Phe Ile Ala Ile Lys Pro Asp Gly Val
85 90 95

Gln Arg Gly Leu Ile Ala Glu Ile Leu Ser Arg Phe Glu Arg Lys Gly
100 105 110

Tyr Lys Leu Val Gly Ile Lys Val Val Ile Pro Thr Lys Glu Phe Ala
115 120 125

Gln Gln His Tyr His Asp Leu Lys Glu Arg Pro Phe Phe Asn Gly Leu
    130                 135                 140

Cys Glu Phe Leu Ser Ser Gly Pro Val Ile Ala Met Val Trp Glu Gly
145                 150                 155                 160

Gln Gly Val Ile Thr Tyr Gly Arg Lys Leu Ile Gly Ala Thr Asp Pro
                165                 170                 175

Gln Lys Ser Glu Pro Gly Thr Ile Arg Gly Asp Leu Ala Val Val Val
            180                 185                 190

Gly Arg Asn Ile Ile His Gly Ser Asp Gly Pro Glu Thr Ala Lys Glu
        195                 200                 205

Glu Ile Lys Leu Trp Phe Lys Pro Glu Glu Leu Val Ser Phe Thr Ser
    210                 215                 220

Asn Ala Glu Lys Trp Ile Tyr Gly Glu Asn
225                 230

<210> 22
<211> 1014
<212> DNA
<213> psyllium

<400> 22
gggggcactt tgtcacaact aacgctctca ctcatcaatc tcagtaaatc tattcttcca 60

aaatgaggtc tcagatttgc agatccgcca ggtctcttct ctctgctgct tccaagcaat 120

cttctcgctc cttctcctcc ggagggcgag cagctgcagc ggctgcagtt tcaggtagag 180

gaagatggtc ttctctagct tcatatgctg catccggaaa tgcaaatagt ggctggatga 240

ctggactcct tgccctaccc acagcagctt tcatgcttca ggaacaagaa gcacatgctg 300

ccgagatgga gagaactttc attgctatca agccagatgg cgtgcaaagg ggattgatct 360

cagaaatcat ctctcgcttt gaacggaaag gattcaagct tgtggggatc aaggtcgtga 420

ttccttccaa ggactttgcc cagaggcatt accatgacct caaggaaaga cctttcttca 480

atgggttgtg caatttcctt agctctggac cagttattgc catggtatgg gaaggcaaag 540

gagtgatcaa gtatggccat aagctcattg gagcaacaga tccccagaaa tcagagcctg 600

gaaccatcag ggagacttta gctgttgttg taggaagaaa cattattcat ggtagtgacg 660

gacctgaaac tgccaaggat gaaatcagct tatggtttaa acctgaggaa ttggtcagtt 720

acacaagcaa tactgagaag tggttgtacg gtgacaattg atgacatacc tttttgttat 780

ttattcacac attgccggcg aactttgatc gtgaactaaa gaaagagtag ttatgagctc 840

aatgctcaga ataatatcca cctttcagct ggtggatacg agtaataatt tagttgcgta 900

tcaccaaatg aacttcattg gtacactgat gtattactga aatgtgagtt tgatcaattc    960

ttgcgcatct tttttatgga cttctctttg aaaaaaaaaa aaaaaaaaaa aaaa          1014

<210>  23
<211>  232
<212>  PRT
<213>  Psyllium

<400>  23

Met Arg Ser Gln Ile Cys Arg Ser Ala Arg Ser Leu Leu Ser Ala Ala
1               5                   10                  15

Ser Lys Gln Ser Ser Arg Ser Phe Ser Ser Gly Gly Arg Ala Ala Ala
            20                  25                  30

Ala Ala Ala Val Ser Gly Arg Gly Arg Trp Ser Ser Leu Ala Ser Tyr
        35                  40                  45

Ala Ala Ser Gly Asn Ala Asn Ser Gly Trp Met Thr Gly Leu Leu Ala
    50                  55                  60

Leu Pro Thr Ala Ala Phe Met Leu Gln Glu Gln Glu Ala His Ala Ala
65                  70                  75                  80

Glu Met Glu Arg Thr Phe Ile Ala Ile Lys Pro Asp Gly Val Gln Arg
                85                  90                  95

Gly Leu Ile Ser Glu Ile Ile Ser Arg Phe Glu Arg Lys Gly Phe Lys
            100                 105                 110

Leu Val Gly Ile Lys Val Val Ile Pro Ser Lys Asp Phe Ala Gln Arg
        115                 120                 125

His Tyr His Asp Leu Lys Glu Arg Pro Phe Phe Asn Gly Leu Cys Asn
    130                 135                 140

Phe Leu Ser Ser Gly Pro Val Ile Ala Met Val Trp Glu Gly Lys Gly
145                 150                 155                 160

Val Ile Lys Tyr Gly His Lys Leu Ile Gly Ala Thr Asp Pro Gln Lys
                165                 170                 175

Ser Glu Pro Gly Thr Ile Arg Gly Asp Leu Ala Val Val Val Gly Arg
            180                 185                 190

Asn Ile Ile His Gly Ser Asp Gly Pro Glu Thr Ala Lys Asp Glu Ile
        195                 200                 205

Ser Leu Trp Phe Lys Pro Glu Glu Leu Val Ser Tyr Thr Ser Asn Thr

210 215 220

Glu Lys Trp Leu Tyr Gly Asp Asn
225 230

<210> 24
<211> 1147
<212> DNA
<213> Avena strigosa

<400> 24
ggcacgagtc tactagtact ccatacagac cactcgctga ggcaggctca cggccattcc 60

tcctcctcct cctcctcctc cttgaagaaa ctgaaccaga cccacatctc gccctccctc 120

cacatctcgc cgccggccgc catgagctcc tccaagatgt accagtccgc gtgcaaggcc 180

gccaggtccc tcatcgcatc ctcctccgcc cgctccgccg tcctcgcaga cgggaggaac 240

gccgcgctcg ccacgctcac caacctcggc aggaccagga tccccgccgc ctactcctca 300

taccacacca acgccggcgc cgcccgcgga tacggctgga tcgcaggcat ccccgccgct 360

gcctacatgc tccaggatca ggaggtgcac gccgcagagc tggagcgcac cttcatcgcc 420

atcaagcccg acggcgtcca gagaggcctc atttctgaga tagtttcccg atttgagagg 480

aaaggattca agcttgttgc catcaagctg gtggttccat ccaaggagtt cgcccagaag 540

cactaccatg atctgaagga aagacctttc ttcagtggat tgtgcgactt ccttagctcc 600

gggcctgtcc ttgccatggt ctgggaagga gagggtgtca tcaagtacgg gaggaagatg 660

attggtgcca cggacccagc gaagtctgaa ccaggaacca tcaggggtga ccttgctgtt 720

gttgttggaa gaaacatcat tcacgggagc gacggcccag agacagccaa ggacgagatt 780

gctctctggt ttacgcccaa ggagctggtc tcttacacca gcaacgagga gaagtgggtc 840

tacggcgtga actaaccggc ggatcatctt ctctttgaaa agaaaaaagt caacatcatt 900

ttccataatt gccttgtaga agagcatggc agagtgtaat aacgctgggt gcaataaggc 960

ctgacgaaaa gagaagtgta actagcttgg aatgtagagc gtgcaccgag caattcgtga 1020

tgtttacatc gaactatcgc ggcctccagc cagcttgatc tggatggatg ctctttattt 1080

tgatctattc tccatttttg accaaacatt aattctatgc acttttgcta aaaaaaaaaa 1140

aaaaaaa 1147

<210> 25
<211> 237
<212> PRT
<213> Avena strigosa

<400> 25

Met Ser Ser Ser Lys Met Tyr Gln Ser Ala Cys Lys Ala Ala Arg Ser
1 5 10 15

Leu Ile Ala Ser Ser Ser Ala Arg Ser Ala Val Leu Ala Asp Gly Arg
20 25 30

Asn Ala Ala Leu Ala Thr Leu Thr Asn Leu Gly Arg Thr Arg Ile Pro
35 40 45

Ala Ala Tyr Ser Ser Tyr His Thr Asn Ala Gly Ala Ala Arg Gly Tyr
50 55 60

Gly Trp Ile Ala Gly Ile Pro Ala Ala Ala Tyr Met Leu Gln Asp Gln
65 70 75 80

Glu Val His Ala Ala Glu Leu Glu Arg Thr Phe Ile Ala Ile Lys Pro
85 90 95

Asp Gly Val Gln Arg Gly Leu Ile Ser Glu Ile Val Ser Arg Phe Glu
100 105 110

Arg Lys Gly Phe Lys Leu Val Ala Ile Lys Leu Val Val Pro Ser Lys
115 120 125

Glu Phe Ala Gln Lys His Tyr His Asp Leu Lys Glu Arg Pro Phe Phe
130 135 140

Ser Gly Leu Cys Asp Phe Leu Ser Ser Gly Pro Val Leu Ala Met Val
145 150 155 160

Trp Glu Gly Glu Gly Val Ile Lys Tyr Gly Arg Lys Met Ile Gly Ala
165 170 175

Thr Asp Pro Ala Lys Ser Glu Pro Gly Thr Ile Arg Gly Asp Leu Ala
180 185 190

Val Val Val Gly Arg Asn Ile Ile His Gly Ser Asp Gly Pro Glu Thr
195 200 205

Ala Lys Asp Glu Ile Ala Leu Trp Phe Thr Pro Lys Glu Leu Val Ser
210 215 220

Tyr Thr Ser Asn Glu Glu Lys Trp Val Tyr Gly Val Asn
225 230 235

<210> 26
<211> 1035
<212> DNA
<213> sunflower

<400> 26
cccaaactca ccattttcag taattttttc acaaatttgg agaatgagtt cacagatttt 60

cagatctgct tcacgagccg ctaggtcgat cgtctcatct gcttctaagc agaagcgtct 120

cttctccgaa ggacgatctg ttgctgctgc tacagctgtt tcggtcaaag gagtcttgcc 180

agctctagct tcttttgggc gtgatcattc tggaaactca tccagcacat ggattgccgg 240

agcactcgct ctccctgctg cagcatacat gcttcaagac caagaggcac atgcagcaca 300

gatggagcgc actttcattg ctatcaagcc agatggtgtg caaagaggac tgatttcaga 360

gatcatagct cggtttgaac gtaaaggctt taaactcgta gccattaaac ttgtgacccc 420

tactaagtcc tttgctcaga agcattacca tgatcttaag gaaagaccat tttttgatgg 480

gctgtgtgac ttccttagct ctggtcccgt tcttgccatg gtttgggaag gtgaaggtgt 540

tatcaagtat gggcgtaaac tcattggagc cacagatcca caaaaatcag aaccgggaac 600

tatcaggggt gatttggctg ttgttgtggg aaggaacatc atccatggaa gtgacggtcc 660

agaaaccgcc aaggatgaaa tcaacttatg gttcaaacca gaggagttga cgaattacac 720

aagcaaccaa gaaaagtggg tctatggagt caactaatgg catctgtttt ttgctttgtc 780

gactgcctag gcaaattagt tcttcggctg caagaataaa aaccaaagaa taaaatgtcc 840

tttttttcct tgaaaatgag caaaacagta tcagttttgt aacttaaaag atactcaaag 900

accatcttta tagttgttaa agactagtat ttggttaatg cagggctgtt ttaattttac 960

ttattttgat tttgcacttt atttagttgt tgaaaacaaa attggttagt tttttttaaa 1020

aaaaaaaaaa aaaaa 1035

<210> 27
<211> 237
<212> PRT
<213> sunflower

<400> 27

Met Ser Ser Gln Ile Phe Arg Ser Ala Ser Arg Ala Ala Arg Ser Ile
1               5                   10                  15

Val Ser Ser Ala Ser Lys Gln Lys Arg Leu Phe Ser Glu Gly Arg Ser
            20                  25                  30

Val Ala Ala Ala Thr Ala Val Ser Val Lys Gly Val Leu Pro Ala Leu
        35                  40                  45

Ala Ser Phe Gly Arg Asp His Ser Gly Asn Ser Ser Ser Thr Trp Ile
    50                  55                  60

Ala Gly Ala Leu Ala Leu Pro Ala Ala Ala Tyr Met Leu Gln Asp Gln
65                  70                  75                  80

Glu Ala His Ala Ala Gln Met Glu Arg Thr Phe Ile Ala Ile Lys Pro
                85                  90                  95

```
Asp Gly Val Gln Arg Gly Leu Ile Ser Glu Ile Ile Ala Arg Phe Glu
            100                 105                 110

Arg Lys Gly Phe Lys Leu Val Ala Ile Lys Leu Val Thr Pro Thr Lys
        115                 120                 125

Ser Phe Ala Gln Lys His Tyr His Asp Leu Lys Glu Arg Pro Phe Phe
    130                 135                 140

Asp Gly Leu Cys Asp Phe Leu Ser Ser Gly Pro Val Leu Ala Met Val
145                 150                 155                 160

Trp Glu Gly Glu Gly Val Ile Lys Tyr Gly Arg Lys Leu Ile Gly Ala
                165                 170                 175

Thr Asp Pro Gln Lys Ser Glu Pro Gly Thr Ile Arg Gly Asp Leu Ala
            180                 185                 190

Val Val Val Gly Arg Asn Ile Ile His Gly Ser Asp Gly Pro Glu Thr
        195                 200                 205

Ala Lys Asp Glu Ile Asn Leu Trp Phe Lys Pro Glu Glu Leu Thr Asn
    210                 215                 220

Tyr Thr Ser Asn Gln Glu Lys Trp Val Tyr Gly Val Asn
225                 230                 235


<210>  28
<211>  901
<212>  DNA
<213>  Glycine max

<400>  28
gcctcacagg tttgcaaatc tgcttccaga gccgcgaggt cccttctatc ctctcgtttt      60

cactctcaag gacgtgcgct tggggctgct gcggctgttt cattgagcag caaagtgcct     120

ctttttttacg gaaagaacgg ttctggaaat ggatccgctt cttcatcatc atcatcatca     180

tggatttcag gagctcttgc tcttcctgcc gcagcttaca tgttccaaga tcaggaggtg     240

caggcagctg agctggagcg cactttcatt gccattaagc ctgatggagt gcagagaggc     300

ctgatttctg agattatatc tcgttttgag cggaaagggt acaagcttgt gggaattaaa     360

gtagtgattc ctaaaaagga atttgcccaa aggcactatc acgacctgaa agaaagaccc     420

ttcttcgatg ggctgtgtga ttttctaagc tctggccctg ttattgcaat ggtgtgggaa     480

ggacagggag ttatttccta tggccgaaag ctaattggag ccacagatcc acagaaatca     540

gaacctggaa ccattagggg tgatcttgct gttgttgttg gaagaaacat catccatggg     600

agtgatggtc cagaaactgc caaggatgag attaagttgt ggtttaagcc agaggagttg     660

gttagtttca ctagcaatgc agagaagtgg atttatggtg ccaactgatt ctttccctca     720
```

tgccttgggt tttctataac aactgctcag tgagtttttg gaataaatta tttgtcagaa 780

tgaactctag aagccataaa taagcgcgat tgagaatttg agattggtac ggttaatata 840

ttttaatagg aattgcattg tttttatctt ttaaaaaaaa aaaaaaaaaa aaaaaaaaaa 900

a 901

<210> 29
<211> 235
<212> PRT
<213> Glycine max

<400> 29

Ala Ser Gln Val Cys Lys Ser Ala Ser Arg Ala Ala Arg Ser Leu Leu
1 5 10 15

Ser Ser Arg Phe His Ser Gln Gly Arg Ala Leu Gly Ala Ala Ala Ala
20 25 30

Val Ser Leu Ser Ser Lys Val Pro Leu Phe Tyr Gly Lys Asn Gly Ser
35 40 45

Gly Asn Gly Ser Ala Ser Ser Ser Ser Ser Ser Ser Trp Ile Ser Gly
50 55 60

Ala Leu Ala Leu Pro Ala Ala Ala Tyr Met Phe Gln Asp Gln Glu Val
65 70 75 80

Gln Ala Ala Glu Leu Glu Arg Thr Phe Ile Ala Ile Lys Pro Asp Gly
85 90 95

Val Gln Arg Gly Leu Ile Ser Glu Ile Ile Ser Arg Phe Glu Arg Lys
100 105 110

Gly Tyr Lys Leu Val Gly Ile Lys Val Val Ile Pro Lys Lys Glu Phe
115 120 125

Ala Gln Arg His Tyr His Asp Leu Lys Glu Arg Pro Phe Phe Asp Gly
130 135 140

Leu Cys Asp Phe Leu Ser Ser Gly Pro Val Ile Ala Met Val Trp Glu
145 150 155 160

Gly Gln Gly Val Ile Ser Tyr Gly Arg Lys Leu Ile Gly Ala Thr Asp
165 170 175

Pro Gln Lys Ser Glu Pro Gly Thr Ile Arg Gly Asp Leu Ala Val Val
180 185 190

Val Gly Arg Asn Ile Ile His Gly Ser Asp Gly Pro Glu Thr Ala Lys
195 200 205

Asp Glu Ile Lys Leu Trp Phe Lys Pro Glu Glu Leu Val Ser Phe Thr
210 215 220

Ser Asn Ala Glu Lys Trp Ile Tyr Gly Ala Asn
225 230 235

<210> 30
<211> 1105
<212> DNA
<213> Gossypium hirsutum

<400> 30
gaaaacacat cactgccctt gcactcggct cataccaatc aatcaaaatc cccaaaaaaa 60

tattaaaaaa aatgagctct cagattttca gatctgcttc tagagccgcc aggtctctcc 120

tttcagcatc caaggcttct cgcttttact ctgaagggcg agctgtagct gccgctgcag 180

cagtttcact tggcggtaaa gtgcctcttt tggcttcagc ttatggaagt actgcttctg 240

ccaatgcatc tagagcatgg ctttcaggag tttttgctct tccagtggca gcttacatgc 300

ttcaggagca ggaggttcat gctgcagaga tggagcgcac cttcattgct atcaagccag 360

atggagtgca aagagggctg atttcggaga tcatctctcg ttttgagcgc aaagggttca 420

agcttgtggc tatcaagttg gtagttcctt caaaggagtt tgcccagaaa cattatgatg 480

acttgaagga aagacccttt ttcaatggcc tgtgtgaatt cctcagctct ggccctgttc 540

ttgccatggt ctgggaagga gagggagtga tcaaatacgg ccggaaactc attggagcaa 600

cagatcctca aaaatcagag cctggaacca tcagaggtga tctagccgtg gttgtcggaa 660

ggaatataat tcatgggagt gatggtcctg agactgccaa gcatgaaatc aacttgtggt 720

tcaagccaca agagttggtt aactatacaa gcaacgctga gaaatgggtc tatggaaaca 780

actgatgagg gcatgttttt tcaagttaat cagtagtagt tcaggaaaat ttagtcccat 840

atcaggtgtg ggttagaggc ataatttttt agaataaatg cgttttatgg atgtgaggag 900

cattgagcca aaagctttga tttttgttat agttaaacta gtgctcaaaa aataatatct 960

ttttcttttc ttcagactca aaataataat ccctttcacc tgtttaatat agggtggaaa 1020

taaaaaaaaa aaaaaaaaaa aaaaaaaaaa aaaaaaaaaa aaaaaaaaaa aaaaaaaaaa 1080

aaaaaaaaaa aaaaaaaaaa aaaaa 1105

<210> 31
<211> 237
<212> PRT
<213> Gossypium hirsutum

<400> 31

Met Ser Ser Gln Ile Phe Arg Ser Ala Ser Arg Ala Ala Arg Ser Leu

```
1               5                   10                  15

Leu Ser Ala Ser Lys Ala Ser Arg Phe Tyr Ser Glu Gly Arg Ala Val
            20                  25                  30

Ala Ala Ala Ala Ala Val Ser Leu Gly Gly Lys Val Pro Leu Leu Ala
        35                  40                  45

Ser Ala Tyr Gly Ser Thr Ala Ser Ala Asn Ala Ser Arg Ala Trp Leu
    50                  55                  60

Ser Gly Val Phe Ala Leu Pro Val Ala Ala Tyr Met Leu Gln Glu Gln
65                  70                  75                  80

Glu Val His Ala Ala Glu Met Glu Arg Thr Phe Ile Ala Ile Lys Pro
                85                  90                  95

Asp Gly Val Gln Arg Gly Leu Ile Ser Glu Ile Ile Ser Arg Phe Glu
            100                 105                 110

Arg Lys Gly Phe Lys Leu Val Ala Ile Lys Leu Val Val Pro Ser Lys
        115                 120                 125

Glu Phe Ala Gln Lys His Tyr Asp Asp Leu Lys Glu Arg Pro Phe Phe
    130                 135                 140

Asn Gly Leu Cys Glu Phe Leu Ser Ser Gly Pro Val Leu Ala Met Val
145                 150                 155                 160

Trp Glu Gly Glu Gly Val Ile Lys Tyr Gly Arg Lys Leu Ile Gly Ala
                165                 170                 175

Thr Asp Pro Gln Lys Ser Glu Pro Gly Thr Ile Arg Gly Asp Leu Ala
            180                 185                 190

Val Val Val Gly Arg Asn Ile Ile His Gly Ser Asp Gly Pro Glu Thr
        195                 200                 205

Ala Lys His Glu Ile Asn Leu Trp Phe Lys Pro Gln Glu Leu Val Asn
    210                 215                 220

Tyr Thr Ser Asn Ala Glu Lys Trp Val Tyr Gly Asn Asn
225                 230                 235
```

<210> 32
<211> 1007
<212> DNA
<213> Glycine max

<400> 32

```
tatttctgtg ccaacaaaag aagatggcct cacaggtttg caaagctgct tccagagccg      60
cgaggtccct tctctccgct tccagagcct cccatggacg tgcggttggg gctgctacgg     120
ctgtttcatt gagctgcaaa gtgcctcttt tctacggaaa gaacggttct ggaagtggtt     180
ccgcttcatc atcatcatca tcatggattt caggagcact tgctcttcct gccgcagctt     240
acatgttcca agatcaggag gtgcgcgcag ctgagctgga gcgcactttc attgccatta     300
agcctgatgg agtgcagaga gggctgattt ctgagattat atctcgtttt gagcggaaag     360
ggtacaagct tgtggggatt aaagtagtga ttccttcaaa ggaatttgcc caaaagcact     420
atcacgacct gaaagaaaga cccttcttcg atgggctgtg tgatttcctt agctctggcc     480
ctgttattgc aatggtgtgg gaaggacagg gagttatttc ctatggccga aagctaattg     540
gagccacaga tcctcagaaa tcagaacctg gaaccattag gggtgatctt gctgttgttg     600
ttggaagaaa tatcattcat gggagtgatg gtcctgagac tgccaaggat gagattaagt     660
tgtggtttaa gccagaggag ttggttagtt tcactagcaa tgcagaaaag tgggtttatg     720
gtgtcaactg attctttccc tcatgccttg ggttttctat aacaactgct cagtgagttt     780
ttgaaataaa ttatttgtca gcggcaatac taatgaactc tggaagccac aaataagcgc     840
gattgagaat agtaccaata ggaattgcat tatttttata ttttattagc cgggtgaaga     900
caatgtgtga catttattat gccaatactt caaaatgggt taattggcct tttgaaagga     960
gttgaggatg atttgttcat cctataataa aaaaaaaaaa aaaaaaa                  1007
```

<210> 33
<211> 235
<212> PRT
<213> Glycine max

<400> 33

```
Met Ala Ser Gln Val Cys Lys Ala Ala Ser Arg Ala Ala Arg Ser Leu
1               5                   10                  15

Leu Ser Ala Ser Arg Ala Ser His Gly Arg Ala Val Gly Ala Ala Thr
            20                  25                  30

Ala Val Ser Leu Ser Cys Lys Val Pro Leu Phe Tyr Gly Lys Asn Gly
        35                  40                  45

Ser Gly Ser Gly Ser Ala Ser Ser Ser Ser Ser Ser Trp Ile Ser Gly
    50                  55                  60

Ala Leu Ala Leu Pro Ala Ala Ala Tyr Met Phe Gln Asp Gln Glu Val
65                  70                  75                  80

Arg Ala Ala Glu Leu Glu Arg Thr Phe Ile Ala Ile Lys Pro Asp Gly
                85                  90                  95
```

Val Gln Arg Gly Leu Ile Ser Glu Ile Ile Ser Arg Phe Glu Arg Lys
100 105 110

Gly Tyr Lys Leu Val Gly Ile Lys Val Val Ile Pro Ser Lys Glu Phe
115 120 125

Ala Gln Lys His Tyr His Asp Leu Lys Glu Arg Pro Phe Phe Asp Gly
130 135 140

Leu Cys Asp Phe Leu Ser Ser Gly Pro Val Ile Ala Met Val Trp Glu
145 150 155 160

Gly Gln Gly Val Ile Ser Tyr Gly Arg Lys Leu Ile Gly Ala Thr Asp
165 170 175

Pro Gln Lys Ser Glu Pro Gly Thr Ile Arg Gly Asp Leu Ala Val Val
180 185 190

Val Gly Arg Asn Ile Ile His Gly Ser Asp Gly Pro Glu Thr Ala Lys
195 200 205

Asp Glu Ile Lys Leu Trp Phe Lys Pro Glu Glu Leu Val Ser Phe Thr
210 215 220

Ser Asn Ala Glu Lys Trp Val Tyr Gly Val Asn
225 230 235

<210> 34
<211> 1089
<212> DNA
<213> Zea mays

<400> 34
cgagcgaccg caccggcacg gcgcccatcc atccatcgcc atcctctagc ccagtccaaa 60

ggaaccctag ccatgagcgc cgcctccaag aagctcttcc aggccgccag gtccctcgtc 120

ctctccgctt ctaaccgctc cgtcctcgcc gccgagggcc gcaccgccgc gctcgccacg 180

ctcaccaact ttggcaggaa gaccctcccc accgcctact tatcctacca caagcaggga 240

tcccaccatg ccgcgtcggg gtggggagcc atcgccgccg cagtcccagc tgcagtttac 300

atgctccagg accaggaggc tcatgctgca gagatggagc gcaccttcat tgccatcaag 360

cctgatggtg tccaaagagg cctgatttct gagattatga gccgatttga gagaaaaggc 420

tataagcttg ttgccatcaa gctgattgtt ccatccaaag aatttgctga gaagcactac 480

catgatctca aggaaaggcc tttcttcagt gggttgtgtg attttctcag ctctggccct 540

gtgcttgcaa tggtttggga aggagagggt gtcatcaagt atgggagaaa actaattggt 600

gccacagacc cacagaaatc tgaaccagga accatcaggg gtgatcttgc cattgttgtt 660

ggaagaaaca tcattcatgg aagtgatggc ccagagacag cgaaggatga gatcgcttta 720

tggtttgaac ccaaggagct ggtctcttac accagcaatg cggagaagtg gatctatggg 780

gtgaattaac gagagagtca atctgttttt tttccttctt ttgatctcgg ttttcacata 840

attgccgaca gacctaggca caaggatgta ataaagtcgc taccgtcact tctgagttgg 900

attgtgacct tcaggtgtag taaggcacaa ggaaggatgg aaaggaaagg aatataccgt 960

gaaatataga gcgtgcactg agtagtcgcg atgttcaaat caaactatat atcaccgtca 1020

ctggagtcat gtaatccaag atggtgatgc agatgtttct ttggatctat ttctctagat 1080

atccctacc 1089

<210> 35
<211> 238
<212> PRT
<213> Zea mays

<400> 35

```
Met Ser Ala Ala Ser Lys Lys Leu Phe Gln Ala Ala Arg Ser Leu Val
1               5                   10                  15

Leu Ser Ala Ser Asn Arg Ser Val Leu Ala Ala Glu Gly Arg Thr Ala
            20                  25                  30

Ala Leu Ala Thr Leu Thr Asn Phe Gly Arg Lys Thr Leu Pro Thr Ala
        35                  40                  45

Tyr Leu Ser Tyr His Lys Gln Gly Ser His His Ala Ala Ser Gly Trp
    50                  55                  60

Gly Ala Ile Ala Ala Ala Val Pro Ala Ala Val Tyr Met Leu Gln Asp
65                  70                  75                  80

Gln Glu Ala His Ala Ala Glu Met Glu Arg Thr Phe Ile Ala Ile Lys
                85                  90                  95

Pro Asp Gly Val Gln Arg Gly Leu Ile Ser Glu Ile Met Ser Arg Phe
            100                 105                 110

Glu Arg Lys Gly Tyr Lys Leu Val Ala Ile Lys Leu Ile Val Pro Ser
        115                 120                 125

Lys Glu Phe Ala Glu Lys His Tyr His Asp Leu Lys Glu Arg Pro Phe
    130                 135                 140

Phe Ser Gly Leu Cys Asp Phe Leu Ser Ser Gly Pro Val Leu Ala Met
145                 150                 155                 160

Val Trp Glu Gly Glu Gly Val Ile Lys Tyr Gly Arg Lys Leu Ile Gly
```

165 170 175

Ala Thr Asp Pro Gln Lys Ser Glu Pro Gly Thr Ile Arg Gly Asp Leu
180 185 190

Ala Ile Val Val Gly Arg Asn Ile Ile His Gly Ser Asp Gly Pro Glu
195 200 205

Thr Ala Lys Asp Glu Ile Ala Leu Trp Phe Glu Pro Lys Glu Leu Val
210 215 220

Ser Tyr Thr Ser Asn Ala Glu Lys Trp Ile Tyr Gly Val Asn
225 230 235

<210> 36
<211> 480
<212> DNA
<213> Zea mays

<400> 36
ctgcagagat ggagcgcact ttcatcgcca tcaagcccga cggcgtccaa agaggcctga 60

tttctgagat tgtgaaccga ttcgagagaa aaggctacaa gcttgttgcc atcaagctga 120

ttgtcccatc caaaggattc gctgagaagc actaccatga tctcaaggaa aggcctttct 180

tcaacgggtt gtgtgacttc ctcagctctg gccctgtact tgcaatggtt tgggaaggag 240

agggtgtcat caagtatggg agaaaactaa ttggtgccac agacccacag aaatctgaac 300

caggaaccat caggggcgat cttggtgttg ttgtgggaag aaacatcatt catggaagtg 360

atggcccgga gacagcaaag gatgagatcg ccttgtggtt tgaacccaag gagctggtct 420

cttataccag caatgcggag aagtgggtct atggggtgaa ttaaccagcg actaaatctc 480

<210> 37
<211> 151
<212> PRT
<213> Zea mays

<400> 37

Met Glu Arg Thr Phe Ile Ala Ile Lys Pro Asp Gly Val Gln Arg Gly
1 5 10 15

Leu Ile Ser Glu Ile Val Asn Arg Phe Glu Arg Lys Gly Tyr Lys Leu
20 25 30

Val Ala Ile Lys Leu Ile Val Pro Ser Lys Gly Phe Ala Glu Lys His
35 40 45

Tyr His Asp Leu Lys Glu Arg Pro Phe Phe Asn Gly Leu Cys Asp Phe
50 55 60

Leu Ser Ser Gly Pro Val Leu Ala Met Val Trp Glu Gly Glu Gly Val
65 70 75 80

Ile Lys Tyr Gly Arg Lys Leu Ile Gly Ala Thr Asp Pro Gln Lys Ser
85 90 95

Glu Pro Gly Thr Ile Arg Gly Asp Leu Gly Val Val Val Gly Arg Asn
100 105 110

Ile Ile His Gly Ser Asp Gly Pro Glu Thr Ala Lys Asp Glu Ile Ala
115 120 125

Leu Trp Phe Glu Pro Lys Glu Leu Val Ser Tyr Thr Ser Asn Ala Glu
130 135 140

Lys Trp Val Tyr Gly Val Asn
145 150

<210> 38
<211> 606
<212> DNA
<213> Glycine max

<400> 38
atattggagc tccaccgcgg tggcggacgc tctagaacta gtggatcccc cgggctgcag 60

gaattcggca cgagcaacaa aagaagatgg cctcacaggt ttgcaaagct gcttccagag 120

ccgcgaggtc ccttctctcc gcttccagag cctcccatgg acgtgcggtt ggggctgcta 180

cggctgtttc attgagctgc aaagtgcctc ttttctacgg aaagaacggt tctggaagtg 240

gttccgcttc atcatcatca tcatcatgga tttcaggagc acttgctctt cctgccgcag 300

cttacatgtt ccaagatcag gaggtgcgcg cagctgagct ggagcgcact ttcattgcca 360

ttaagcctga tggagtgcag agagggctga tttctgagat tatatctcgt tttgagcgga 420

aagggtacaa gcttgtgggg attaaagtag tgattccttc aaaggaattt gcccaaaagc 480

actatcacga cctgaaagaa agacccttct tcgatgggct gtgtgatttc cttagctctg 540

gccctgttat tgcaatgggt gtgggaagga caagggagtt atttcctatg gccgaaagct 600

aattgg 606

<210> 39
<211> 171
<212> PRT
<213> Glycine max

<400> 39

```
Met Ala Ser Gln Val Cys Lys Ala Ala Ser Arg Ala Ala Arg Ser Leu
1               5                   10                  15

Leu Ser Ala Ser Arg Ala Ser His Gly Arg Ala Val Gly Ala Ala Thr
            20                  25                  30

Ala Val Ser Leu Ser Cys Lys Val Pro Leu Phe Tyr Gly Lys Asn Gly
        35                  40                  45

Ser Gly Ser Gly Ser Ala Ser Ser Ser Ser Ser Ser Trp Ile Ser Gly
    50                  55                  60

Ala Leu Ala Leu Pro Ala Ala Ala Tyr Met Phe Gln Asp Gln Glu Val
65                  70                  75                  80

Arg Ala Ala Glu Leu Glu Arg Thr Phe Ile Ala Ile Lys Pro Asp Gly
                85                  90                  95

Val Gln Arg Gly Leu Ile Ser Glu Ile Ile Ser Arg Phe Glu Arg Lys
            100                 105                 110

Gly Tyr Lys Leu Val Gly Ile Lys Val Val Ile Pro Ser Lys Glu Phe
        115                 120                 125

Ala Gln Lys His Tyr His Asp Leu Lys Glu Arg Pro Phe Phe Asp Gly
    130                 135                 140

Leu Cys Asp Phe Leu Ser Ser Gly Pro Val Ile Ala Met Gly Val Gly
145                 150                 155                 160

Arg Thr Arg Glu Leu Phe Pro Met Ala Glu Ser
                165                 170


<210>  40
<211>  552
<212>  DNA
<213>  Oryza sativa


<220>
<221>  misc_feature
<222>  (436)..(436)
<223>  n is a, c, g, or t

<220>
<221>  misc_feature
<222>  (492)..(492)
<223>  n is a, c, g, or t

<220>
<221>  misc_feature
<222>  (516)..(516)
<223>  n is a, c, g, or t
```

<220>
<221> misc_feature
<222> (551)..(551)
<223> n is a, c, g, or t

<400> 40
gccactgaga aggagaagag gtcctccgga tcaaagcggc accgacgacg ccggcgaggc 60

cactactacc atatcatagt acatactagt acaagactgg agggctagtg gggtgggggt 120

acaatccaat ccaatccaat caaatcacat cgatcgacgc cgcaacccag cagcagcagc 180

ggaaccctag ccaagccacc atgagcaagc tctgccagtc ggcgtgcaag gccgccaagt 240

ctctcctctc cgccaccgcc gccgcctcct ccccgcgcac ctccctccta gccgagggga 300

ggaacgcggc gctggccacc ctcaccaacc tggggaggaa gacctcccca cagcttacgc 360

ctactcctac caccacaact cctccgccgc cgccgccgga tggctcgctg ccatcccgcc 420

gctgttaatg ctacangatc aggagcgcat gctgcagaga tggagcgcac tttatcgcat 480

caagcctgac gngtcaaagg ggctgattct gaatangtcc gattgaagaa aagatcaagc 540

tgttgcatca nc 552

<210> 41
<211> 75
<212> PRT
<213> Oryza sativa

<400> 41

Met Ser Lys Leu Cys Gln Ser Ala Cys Lys Ala Ala Lys Ser Leu Leu
1 5 10 15

Ser Ala Thr Ala Ala Ala Ser Ser Pro Arg Thr Ser Leu Leu Ala Glu
20 25 30

Gly Arg Asn Ala Ala Leu Ala Thr Leu Thr Asn Leu Gly Arg Lys Thr
35 40 45

Ser Pro Gln Leu Thr Pro Thr Pro Thr Thr Thr Thr Pro Pro Pro Pro
50 55 60

Pro Pro Asp Gly Ser Leu Pro Ser Arg Arg Cys
65 70 75

<210> 42
<211> 719
<212> DNA
<213> Zea mays

<220>
<221> misc_feature
<222> (1)..(11)
<223> n is a, c, g, or t

<220>
<221> misc_feature
<222> (13)..(19)
<223> n is a, c, g, or t

<220>
<221> misc_feature
<222> (22)..(22)
<223> n is a, c, g, or t

<220>
<221> misc_feature
<222> (24)..(27)
<223> n is a, c, g, or t

<220>
<221> misc_feature
<222> (80)..(80)
<223> n is a, c, g, or t

<220>
<221> misc_feature
<222> (494)..(494)
<223> n is a, c, g, or t

<220>
<221> misc_feature
<222> (578)..(578)
<223> n is a, c, g, or t

<220>
<221> misc_feature
<222> (602)..(602)
<223> n is a, c, g, or t

<220>
<221> misc_feature
<222> (606)..(607)
<223> n is a, c, g, or t

<220>
<221> misc_feature
<222> (609)..(609)
<223> n is a, c, g, or t

<220>
<221> misc_feature
<222> (674)..(676)
<223> n is a, c, g, or t

<220>
<221> misc_feature
<222> (691)..(691)
<223> n is a, c, g, or t

<220>
<221> misc_feature
<222> (703)..(703)
<223> n is a, c, g, or t

<220>
<221> misc_feature
<222> (711)..(712)
<223> n is a, c, g, or t

<220>
<221> misc_feature
<222> (719)..(719)
<223> n is a, c, g, or t

<400> 42
nnnnnnnnnn ngnnnnnnnt anannnnttt gtacaaaaaa gcaggctggt accggtccgg 60

aattcccggg atcgcagcgn ctaacgcgtg gccacgccat ccctccgcgt gcctaattaa 120

ttggaagcac ccagcgcaca tcatcagtcg aagtgagcgt cagcgagcga ccgcaccggc 180

acggcgccca tccatccatc gccatcctct agcccagtcc aaaggaaccc tagccatgag 240

cgccgcctcc aagaagctct tccaggccgc caggtccctc gtcctctccg cttctcaccg 300

ctccgtcctc gccgccgagg gccgcaccgc cgcgctcgcc acgctcacca actttggcag 360

gaagaccctc cccaccgcct acttatccta ccacaagcag ggatcccacc atgccgcgtc 420

ggggtgggga gccatcgccg ccgcagtccc agctgcagtt tacatgctcc aggaccagga 480

ggctcatgct gcanagatgg agcgcacctt cattgccatc aagcctgatg gcgtccaaag 540

aggcctgatt tctgagatta tgagccgatt tgagaganaa ggctataagc ttgttgccat 600

cnagcnnant gttccatcca aagaatttgc tgagaagcac taccatgatc tcaaggaaag 660

gcctttcttc agtnnnttgt gtgattttct nggctctggc ccngtgcttg nnatggttn 719

<210> 43
<211> 161
<212> PRT
<213> Zea mays

<220>
<221> misc_feature
<222> (87)..(87)
<223> Xaa can be any naturally occurring amino acid

<220>
<221> misc_feature
<222> (115)..(115)
<223> Xaa can be any naturally occurring amino acid

<220>
<221> misc_feature
<222> (123)..(125)
<223> Xaa can be any naturally occurring amino acid

<220>
<221> misc_feature
<222> (147)..(147)
<223> Xaa can be any naturally occurring amino acid

<220>
<221> misc_feature
<222> (159)..(159)
<223> Xaa can be any naturally occurring amino acid

<400> 43

Met Ser Ala Ala Ser Lys Lys Leu Phe Gln Ala Ala Arg Ser Leu Val
1               5                   10                  15

Leu Ser Ala Ser His Arg Ser Val Leu Ala Ala Glu Gly Arg Thr Ala
            20                  25                  30

Ala Leu Ala Thr Leu Thr Asn Phe Gly Arg Lys Thr Leu Pro Thr Ala
        35                  40                  45

Tyr Leu Ser Tyr His Lys Gln Gly Ser His His Ala Ala Ser Gly Trp
    50                  55                  60

Gly Ala Ile Ala Ala Ala Val Pro Ala Ala Val Tyr Met Leu Gln Asp
65                  70                  75                  80

Gln Glu Ala His Ala Ala Xaa Met Glu Arg Thr Phe Ile Ala Ile Lys
                85                  90                  95

Pro Asp Gly Val Gln Arg Gly Leu Ile Ser Glu Ile Met Ser Arg Phe
            100                 105                 110

Glu Arg Xaa Gly Tyr Lys Leu Val Ala Ile Xaa Xaa Xaa Val Pro Ser
        115                 120                 125

Lys Glu Phe Ala Glu Lys His Tyr His Asp Leu Lys Glu Arg Pro Phe
    130                 135                 140

Phe Ser Xaa Leu Cys Asp Phe Leu Gly Ser Gly Pro Val Leu Xaa Met
145                 150                 155                 160

Val

<210> 44
<211> 239
<212> PRT
<213> Oryza sativa

<400> 44

Met Ser Lys Leu Cys Gln Ser Ala Cys Lys Ala Ala Lys Ser Leu Leu
1               5                   10                  15

Ser Ala Thr Ala Ala Ala Ser Ser Pro Arg Thr Ser Leu Leu Ala Glu
            20                  25                  30

Gly Arg Asn Ala Ala Leu Ala Thr Leu Thr Asn Leu Gly Arg Lys Thr
        35                  40                  45

Leu Pro Thr Ala Tyr Ala Tyr Ser Tyr His His Asn Ser Ser Ala Ala
    50                  55                  60

```
Ala Ala Gly Trp Leu Ala Ala Ile Pro Ala Ala Val Tyr Met Leu Gln
65                  70                  75                  80

Asp Gln Glu Ala His Ala Ala Glu Met Glu Arg Thr Phe Ile Ala Ile
                85                  90                  95

Lys Pro Asp Gly Val Gln Arg Gly Leu Ile Ser Glu Ile Leu Ser Arg
            100                 105                 110

Phe Glu Arg Lys Gly Phe Lys Leu Val Ala Ile Lys Leu Val Val Pro
        115                 120                 125

Ser Lys Glu Phe Ala Gln Lys His Tyr His Asp Leu Lys Asp Arg Pro
    130                 135                 140

Phe Phe Asn Gly Leu Cys Asp Phe Leu Ser Ser Gly Pro Val Leu Ala
145                 150                 155                 160

Met Val Trp Glu Gly Glu Gly Val Ile Lys Tyr Gly Arg Lys Leu Ile
                165                 170                 175

Gly Ala Thr Asp Pro Gln Lys Ser Glu Pro Gly Thr Ile Arg Gly Asp
            180                 185                 190

Leu Ala Val Val Val Gly Arg Asn Ile Ile His Gly Ser Asp Gly Pro
        195                 200                 205

Glu Thr Ala Lys Ala Glu Ile Gly Leu Trp Phe Glu Pro Arg Glu Leu
    210                 215                 220

Val Ser Tyr Thr Ser Asn Glu Glu Lys Trp Ile Tyr Gly Val Asn
225                 230                 235
```

```
<210>  45
<211>  233
<212>  PRT
<213>  pea

<400>  45
```

```
Met Ala Ser His Leu Cys Lys Ser Ala Ser Arg Ala Ala Arg Ser Leu
1               5                   10                  15

Leu Ser Ala Ser Phe His Ser Gln Gly Arg Ala Val Ala Ala Ala Ala
            20                  25                  30

Ala Val Ala Ser Ile Arg Lys Val Pro Val Phe Ala Pro Asn Tyr Arg
        35                  40                  45
```

```
Arg Thr Gly Ser Gly Asn Gly Pro Ser Ser Trp Ile Ala Gly Ala Leu
    50                  55                  60

Ala Leu Pro Ala Ala Ala Tyr Met Leu Gln Asp Gln Glu Val His Ala
65                  70                  75                  80

Ala Glu Leu Glu Arg Thr Phe Ile Ala Ile Lys Pro Asp Gly Val Gln
                85                  90                  95

Arg Gly Leu Ile Ser Glu Ile Ile Ser Arg Phe Glu Arg Lys Gly Phe
            100                 105                 110

Lys Leu Val Gly Ile Lys Val Leu Ile Pro Thr Lys Gln Phe Ala Gln
        115                 120                 125

Gln His Tyr His Asp Leu Lys Glu Arg Pro Phe Phe Asn Gly Leu Cys
    130                 135                 140

Asp Phe Leu Ser Ser Gly Pro Val Ile Ala Met Val Trp Glu Gly Glu
145                 150                 155                 160

Gly Val Ile Thr Tyr Gly Arg Lys Leu Ile Gly Ala Thr Asp Pro Gln
                165                 170                 175

Lys Ser Ala Pro Gly Thr Ile Arg Gly Asp Leu Ala Val Val Val Gly
            180                 185                 190

Arg Asn Ile Ile His Gly Ser Asp Gly Pro Glu Thr Ala Lys Asp Glu
        195                 200                 205

Ile Lys Leu Trp Phe Lys Pro Glu Glu Leu Val Ser Phe Thr Ser Asn
    210                 215                 220

Ser Glu Lys Trp Ile Tyr Gly Asp Asn
225                 230

<210>  46
<211>  238
<212>  PRT
<213>  Arabidospsis thaliana

<400>  46

Met Ser Ser Gln Ile Cys Arg Ser Ala Ser Lys Ala Ala Lys Ser Leu
1               5                   10                  15

Leu Ser Ser Ala Lys Asn Ala Arg Phe Phe Ser Glu Gly Arg Ala Ile
            20                  25                  30

Gly Ala Ala Ala Ala Val Ser Ala Ser Gly Lys Ile Pro Leu Tyr Ala
        35                  40                  45
```

Ser Asn Phe Ala Arg Ser Ser Gly Ser Gly Val Ala Ser Lys Ser Trp
    50                  55                  60

Ile Thr Gly Leu Leu Ala Leu Pro Ala Ala Ala Tyr Met Ile Gln Asp
65                  70                  75                  80

Gln Glu Val Leu Ala Ala Glu Met Glu Arg Thr Phe Ile Ala Ile Lys
                85                  90                  95

Pro Asp Gly Val Gln Arg Gly Leu Ile Ser Glu Ile Ile Ser Arg Phe
            100                 105                 110

Glu Arg Lys Gly Phe Lys Leu Val Gly Ile Lys Val Ile Val Pro Ser
        115                 120                 125

Lys Asp Phe Ala Gln Lys His Tyr His Asp Leu Lys Glu Arg Pro Phe
    130                 135                 140

Phe Asn Gly Leu Cys Asp Phe Leu Ser Ser Gly Pro Val Ile Ala Met
145                 150                 155                 160

Val Trp Glu Gly Asp Gly Val Ile Arg Tyr Gly Arg Lys Leu Ile Gly
                165                 170                 175

Ala Thr Asp Pro Gln Lys Ser Glu Pro Gly Thr Ile Arg Gly Asp Leu
            180                 185                 190

Ala Val Thr Val Gly Arg Asn Ile Ile His Gly Ser Asp Gly Pro Glu
        195                 200                 205

Thr Ala Lys Asp Glu Ile Ser Leu Trp Phe Lys Pro Gln Glu Leu Val
    210                 215                 220

Ser Tyr Thr Ser Asn Ser Glu Lys Trp Leu Tyr Gly Asp Asn
225                 230                 235

<210> 47
<211> 235
<212> PRT
<213> Grapevine

<400> 47

Met Ser Ser Gln Ile Cys Arg Ser Ala Ser Arg Ala Ala Arg Ser Leu
1               5                   10                  15

Leu Ser Ala Ser Lys Ser Ser Asn Leu Leu Ala Glu Gly Arg Ala Val
            20                  25                  30

Ala Ala Val Ala Ala Leu Ser Ser Arg Gly Lys Pro Phe Leu Ser Ser
35 40 45

Phe Gly Asn Ala Gly Ser Gly Asn Ala Tyr Arg Gly Trp Leu Ser Ser
50 55 60

Val Leu Ala Leu Pro Ala Ala Ala Tyr Met Met Gln Glu Gln Glu Leu
65 70 75 80

His Ala Thr Glu Met Glu Arg Thr Phe Ile Ala Ile Lys Pro Asp Gly
85 90 95

Val Gln Arg Gly Leu Ile Ala Glu Ile Leu Ser Arg Phe Glu Arg Lys
100 105 110

Gly Phe Lys Leu Val Ala Ile Lys Ile Val Val Pro Ser Lys Asp Phe
115 120 125

Ala Gln Lys His Tyr His Asp Leu Lys Glu Arg Pro Phe Phe Asn Gly
130 135 140

Leu Cys Asp Phe Leu Ser Ser Gly Pro Val Val Ala Met Val Trp Glu
145 150 155 160

Gly Glu Gly Val Ile Lys Tyr Gly Arg Lys Leu Ile Gly Ala Thr Asp
165 170 175

Pro Gln Lys Ser Glu Pro Gly Thr Ile Arg Gly Asp Leu Ala Val Val
180 185 190

Val Gly Arg Asn Ile Ile His Gly Ser Asp Gly Pro Glu Thr Ala Lys
195 200 205

Asp Glu Ile Asn Leu Trp Phe Lys Pro Glu Glu Leu Val Asn Tyr Ser
210 215 220

Ser Asn Ala Glu Lys Trp Ile Tyr Gly Val Asn
225 230 235

<210> 48
<211> 238
<212> PRT
<213> Pennycress

<400> 48

Met Ser Ser Gln Ile Cys Arg Ser Ala Ser Lys Ala Ala Arg Ser Leu
1 5 10 15

Leu Ser Ser Ala Arg Asn Val Arg Phe Phe Ser Glu Gly Arg Ala Ile
20 25 30

```
Gly Ala Ala Ala Ala Val Ser Ala Ser Gly Lys Ile Pro Leu Tyr Ala
        35                  40                  45

Ser Asn Phe Ala Arg Ser Ser Gly Ser Gly Ala Ala Ser Lys Asn Trp
    50                  55                  60

Leu Thr Gly Leu Ile Ala Leu Pro Ala Ala Ala Leu Met Leu Gln Asp
65                  70                  75                  80

Gln Glu Val Leu Ala Ala Glu Met Glu Arg Thr Phe Ile Ala Ile Lys
                85                  90                  95

Pro Asp Gly Val Gln Arg Gly Leu Ile Ser Glu Ile Val Ser Arg Phe
            100                 105                 110

Glu Arg Lys Gly Phe Lys Leu Val Gly Ile Lys Val Val Val Pro Ser
        115                 120                 125

Lys Asp Phe Ala Gln Lys His Tyr His Asp Leu Lys Glu Arg Thr Phe
    130                 135                 140

Phe Asn Gly Leu Cys Asp Phe Leu Ser Ser Gly Pro Val Ile Ala Met
145                 150                 155                 160

Val Trp Glu Gly Glu Gly Val Ile Arg Tyr Gly Arg Lys Leu Ile Gly
                165                 170                 175

Ala Thr Asp Pro Gln Lys Ser Glu Pro Gly Thr Ile Arg Gly Asp Leu
            180                 185                 190

Ala Val Val Val Gly Arg Asn Ile Ile His Gly Ser Asp Gly Pro Glu
        195                 200                 205

Thr Ala Lys Asp Glu Ile Asn Leu Trp Phe Lys Pro Gln Glu Leu Val
    210                 215                 220

Ser Tyr Thr Asn Asn Ala Glu Lys Trp Ile Tyr Gly Asp Asn
225                 230                 235
```

<210> 49
<211> 195
<212> PRT
<213> Oryza sativa

<400> 49

```
Met Ser Lys Leu Cys Gln Ser Ala Cys Lys Ala Ala Lys Ser Leu Leu
1               5                   10                  15
```

Ser Ala Thr Ala Ala Ala Ser Ser Gln Arg Thr Ser Leu Leu Ala Val
20 25 30

Tyr Met Leu Gln Asp Gln Glu Ala His Ala Ala Glu Met Glu Arg Thr
35 40 45

Phe Ile Ala Ile Lys Pro Asp Gly Val Gln Arg Gly Leu Ile Ser Glu
50 55 60

Ile Leu Ser Arg Phe Glu Arg Lys Gly Phe Lys Leu Val Ala Ile Lys
65 70 75 80

Leu Val Val Pro Ser Lys Glu Phe Ala Gln Lys His Tyr His Asp Leu
85 90 95

Lys Asp Arg Pro Phe Phe Asn Gly Leu Cys Asp Phe Leu Ser Ser Gly
100 105 110

Pro Val Leu Ala Met Val Trp Glu Gly Glu Gly Val Ile Lys Tyr Gly
115 120 125

Arg Lys Leu Ile Gly Ala Thr Asp Pro Gln Lys Ser Glu Pro Gly Thr
130 135 140

Ile Arg Gly Asp Leu Ala Val Val Val Gly Arg Asn Ile Ile His Gly
145 150 155 160

Ser Asp Gly Pro Glu Thr Ala Lys Ala Glu Ile Gly Leu Trp Phe Glu
165 170 175

Pro Arg Glu Leu Val Ser Tyr Thr Ser Asn Glu Glu Lys Trp Ile Tyr
180 185 190

Gly Val Asn
195

<210> 50
<211> 972
<212> DNA
<213> Arabidopsis thaliana

<400> 50
ccctgtttgc acatcactct tctcagtcct cacgcatcgt atcagagaaa atgagctccc 60

aaatctgcag atctgcttct agagcagcca ggtctcttct ttcttcagcc aagaatgctc 120

gtttcttctc cgaaggccga gccattggtg cagcaagtgt ggtgcatgcg actggaaaag 180

tccctcagta tgcatccaac tttggaaaat cgggttctgg cttcgtctct aatagctgga 240

tcaccggact ccttgctctt cctgctgcag ccttcatgct ccaagatcag gaagcacttg 300

ctgcagagat ggaacgcact ttcatcgcta tcaaacctga tggagtgcag cgaggactga 360

tatcagaaat cattacacgg ttcgaacgca aaggatacaa gcttgttggc attaaagtca 420

tggttccttc aaagggtttc gcgcagaagc attaccatga tctaaaggag agacctttct 480

tcaacggctt gtgtaacttc cttagctcag gccctgttgt tgccatggta tgggaaggtg 540

aaggagtgat tagatacgga cgtaaactga ttggagccac tgatcctcag aaatcagaac 600

ctggaactat ccgaggcgat ctcgctgttg ttgttggaag gaacattata catggaagcg 660

atggaccaga gacagctaaa gacgagatca gcttgtggtt taagcctgaa gaactcgttt 720

cttacactag taacgctgag aagtggatct acggccagaa ctgaactatt ccctctttct 780

cccttatttt caaccgttag atcagacata aataattact tcttttgttt tttcttttac 840

ccaaacactg aaaacgtacc gggaataaca aatttactgc atttgttatc aagtttgttc 900

taatagaccc aatgaaatta ggccacgtgt gcgatcatgt aaccggttta ccgtaatgtg 960

gacttgaaat cc 972

<210> 51
<211> 237
<212> PRT
<213> Arabidopsis thaliana

<400> 51

Met Ser Ser Gln Ile Cys Arg Ser Ala Ser Arg Ala Ala Arg Ser Leu
1 5 10 15

Leu Ser Ser Ala Lys Asn Ala Arg Phe Phe Ser Glu Gly Arg Ala Ile
20 25 30

Gly Ala Ala Ser Val Val His Ala Thr Gly Lys Val Pro Gln Tyr Ala
35 40 45

Ser Asn Phe Gly Lys Ser Gly Ser Gly Phe Val Ser Asn Ser Trp Ile
50 55 60

Thr Gly Leu Leu Ala Leu Pro Ala Ala Ala Phe Met Leu Gln Asp Gln
65 70 75 80

Glu Ala Leu Ala Ala Glu Met Glu Arg Thr Phe Ile Ala Ile Lys Pro
85 90 95

Asp Gly Val Gln Arg Gly Leu Ile Ser Glu Ile Ile Thr Arg Phe Glu
100 105 110

Arg Lys Gly Tyr Lys Leu Val Gly Ile Lys Val Met Val Pro Ser Lys
115 120 125

Gly Phe Ala Gln Lys His Tyr His Asp Leu Lys Glu Arg Pro Phe Phe
130 135 140

Asn Gly Leu Cys Asn Phe Leu Ser Ser Gly Pro Val Val Ala Met Val
145                 150                 155                 160

Trp Glu Gly Glu Gly Val Ile Arg Tyr Gly Arg Lys Leu Ile Gly Ala
                165                 170                 175

Thr Asp Pro Gln Lys Ser Glu Pro Gly Thr Ile Arg Gly Asp Leu Ala
            180                 185                 190

Val Val Val Gly Arg Asn Ile Ile His Gly Ser Asp Gly Pro Glu Thr
        195                 200                 205

Ala Lys Asp Glu Ile Ser Leu Trp Phe Lys Pro Glu Glu Leu Val Ser
    210                 215                 220

Tyr Thr Ser Asn Ala Glu Lys Trp Ile Tyr Gly Gln Asn
225                 230                 235

<210> 52
<211> 29
<212> DNA
<213> artificial sequences

<220>
<223> primer

<400> 52
ggggacaagt ttgtacaaaa aagcaggct 29

<210> 53
<211> 29
<212> DNA
<213> artificial sequence

<220>
<223> primer

<400> 53
ggggaccact ttgtacaaga aagctgggt 29

<210> 54
<211> 54
<212> DNA
<213> artificial sequence

<220>
<223> primer

<400> 54
ttaaacaagt ttgtacaaaa aagcaggctg caattaaccc tcactaaagg gaac 54

<210> 55
<211> 53
<212> DNA

<213> artificial sequence

<220>
<223> primer

<400> 55
ttaaaccact ttgtacaaga aagctgggtg cgtaatacga ctcactatag ggc 53

<210> 56
<211> 12856
<212> DNA
<213> artificial sequence

<220>
<223> vector

<400> 56
cgccttggcg cgccgatcat ccacaagttt gtacaaaaaa gctgaacgag aaacgtaaaa 60

tgatataaat atcaatatat taaattagat tttgcataaa aaacagacta cataatactg 120

taaaacacaa catatccagt cactatggcg gccgcattag gcaccccagg ctttacactt 180

tatgcttccg gctcgtataa tgtgtggatt ttgagttagg atttaaatac gcgttgatcc 240

ggcttactaa aagccagata acagtatgcg tatttgcgcg ctgatttttg cggtataaga 300

atatatactg atatgtatac ccgaagtatg tcaaaaagag gtatgctatg aagcagcgta 360

ttacagtgac agttgacagc gacagctatc agttgctcaa ggcatatatg atgtcaatat 420

ctccggtctg gtaagcacaa ccatgcagaa tgaagcccgt cgtctgcgtg ccgaacgctg 480

gaaagcggaa aatcaggaag ggatggctga ggtcgcccgg tttattgaaa tgaacggctc 540

ttttgctgac gagaacaggg gctggtgaaa tgcagtttaa ggtttacacc tataaaagag 600

agagccgtta tcgtctgttt gtggatgtac agagtgatat cattgacacg cccggtcgac 660

ggatggtgat ccccctggcc agtgcacgtc tgctgtcaga taaagtctcc cgtgaacttt 720

acccggtggt gcatatcggg gatgaaagct ggcgcatgat gaccaccgat atggccagtg 780

tgccggtctc cgttatcggg gaagaagtgg ctgatctcag ccaccgcgaa aatgacatca 840

aaaacgccat taacctgatg ttctggggaa tataaatgtc aggctccctt atacacagcc 900

agtctgcagg tcgaccatag tgactggata tgttgtgttt tacagtatta tgtagtctgt 960

tttttatgca aaatctaatt taatatattg atatttatat cattttacgt ttctcgttca 1020

gctttcttgt acaaagtggt gttaacctag acttgtccat cttctggatt ggccaactta 1080

attaatgtat gaaataaaag gatgcacaca tagtgacatg ctaatcacta taatgtgggc 1140

atcaaagttg tgtgttatgt gtaattacta gttatctgaa taaaagagaa agagatcatc 1200

catatttctt atcctaaatg aatgtcacgt gtctttataa ttctttgatg aaccagatgc 1260

atttcattaa ccaaatccat atacatataa atattaatca tatataatta atatcaattg 1320

ggttagcaaa acaaatctag tctaggtgtg ttttgcgaat tgcggccgcc accgcggtgg 1380

agctcgaatt ccggtccggg tcacctttgt ccaccaagat ggaactgcgg ccgctcatta 1440

attaagtcag gcgcgcctct agttgaagac acgttcatgt cttcatcgta agaagacact 1500
cagtagtctt cggccagaat ggccatctgg attcagcagg cctagaaggc catttaaatc 1560
ctgaggatct ggtcttccta aggacccggg atatcggacc gattaaactt taattcggtc 1620
cgaagcttga agttcctatt ccgaagttcc tattctccag aaagtatagg aacttcgcat 1680
gcctgcagtg cagcgtgacc cggtcgtgcc cctctctaga gataatgagc attgcatgtc 1740
taagttataa aaaattacca catatttttt ttgtcacact tgtttgaagt gcagtttatc 1800
tatctttata catatattta aactttactc tacgaataat ataatctata gtactacaat 1860
aatatcagtg ttttagagaa tcatataaat gaacagttag acatggtcta aaggacaatt 1920
gagtattttg acaacaggac tctacagttt tatcttttta gtgtgcatgt gttctccttt 1980
ttttttgcaa atagcttcac ctatataata cttcatccat tttattagta catccattta 2040
gggtttaggg ttaatggttt ttatagacta atttttttag tacatctatt ttattctatt 2100
ttagcctcta aattaagaaa actaaaactc tattttagtt tttttattta ataatttaga 2160
tataaaatag aataaaataa agtgactaaa aattaaacaa ataccctttta agaaattaaa 2220
aaaactaagg aaacattttt cttgtttcga gtagataatg ccagcctgtt aaacgccgtc 2280
gacgagtcta acggacacca accagcgaac cagcagcgtc gcgtcgggcc aagcgaagca 2340
gacggcacgg catctctgtc gctgcctctg gacccctctc gagagttccg ctccaccgtt 2400
ggacttgctc cgctgtcggc atccagaaat tgcgtggcgg agcggcagac gtgagccggc 2460
acggcaggcg gcctcctcct cctctcacgg caccggcagc tacgggggat tcctttccca 2520
ccgctccttc gctttccctt cctcgcccgc cgtaataaat agacaccccc tccacaccct 2580
ctttccccaa cctcgtgttg ttcggagcgc acacacacac aaccagatct cccccaaatc 2640
cacccgtcgg cacctccgct tcaaggtacg ccgctcgtcc tccccccccc ccctctctac 2700
cttctctaga tcggcgttcc ggtccatgca tggttagggc ccggtagttc tacttctgtt 2760
catgtttgtg ttagatccgt gtttgtgtta gatccgtgct gctagcgttc gtacacggat 2820
gcgacctgta cgtcagacac gttctgattg ctaacttgcc agtgtttctc tttggggaat 2880
cctgggatgg ctctagccgt tccgcagacg ggatcgattt catgattttt tttgtttcgt 2940
tgcatagggt ttggtttgcc cttttccttt atttcaatat atgccgtgca cttgtttgtc 3000
gggtcatctt ttcatgcttt tttttgtctt ggttgtgatg atgtggtctg gttgggcggt 3060
cgttctagat cggagtagaa ttctgtttca aactacctgg tggatttatt aattttggat 3120
ctgtatgtgt gtgccataca tattcatagt tacgaattga agatgatgga tggaaatatc 3180
gatctaggat aggtatacat gttgatgcgg gttttactga tgcatataca gagatgcttt 3240
ttgttcgctt ggttgtgatg atgtggtgtg gttgggcggt cgttcattcg ttctagatcg 3300
gagtagaata ctgtttcaaa ctacctggtg tatttattaa ttttggaact gtatgtgtgt 3360

gtcatacatc ttcatagtta cgagtttaag atggatggaa atatcgatct aggataggta 3420
tacatgttga tgtgggtttt actgatgcat atacatgatg gcatatgcag catctattca 3480
tatgctctaa ccttgagtac ctatctatta taataaacaa gtatgtttta taattatttt 3540
gatcttgata tacttggatg atggcatatg cagcagctat atgtggattt ttttagccct 3600
gccttcatac gctatttatt tgcttggtac tgtttctttt gtcgatgctc accctgttgt 3660
ttggtgttac ttctgcaggt cgactttaac ttagcctagg atccacacga caccatgata 3720
gaggtgaaac cgattaacgc agaggatacc tatgaactaa ggcatagaat actcagacca 3780
aaccagccga tagaagcgtg tatgtttgaa agcgatttac ttcgtggtgc atttcactta 3840
ggcggctatt acgggggcaa actgatttcc atagcttcat tccaccaggc cgagcactca 3900
gaactccaag gccagaaaca gtaccagctc cgaggtatgg ctaccttgga aggttatcgt 3960
gagcagaagg cgggatcgag tctaattaaa cacgctgaag aaattcttcg taagaggggg 4020
gcggacttgc tttggtgtaa tgcgcggaca tccgcctcag gctactacaa aaagttaggc 4080
ttcagcgagc agggagaggt attcgacacg ccgccagtag gacctcacat cctgatgtat 4140
aaaaggatca cataactagc tagtcagtta acctagactt gtccatcttc tggattggcc 4200
aacttaatta atgtatgaaa taaaaggatg cacacatagt gacatgctaa tcactataat 4260
gtgggcatca aagttgtgtg ttatgtgtaa ttactagtta tctgaataaa agagaaagag 4320
atcatccata tttcttatcc taaatgaatg tcacgtgtct ttataattct ttgatgaacc 4380
agatgcattt cattaaccaa atccatatac atataaatat taatcatata taattaatat 4440
caattgggtt agcaaaacaa atctagtcta ggtgtgtttt gcgaattcag agctcgaatt 4500
cattccgatt aatcgtggcc tcttgctctt caggatgaag agctatgttt aaacgtgcaa 4560
gcgctactag acaattcagt acattaaaaa cgtccgcaat gtgttattaa gttgtctaag 4620
cgtcaatttg tttacaccac aatatatcct gccaccagcc agccaacagc tccccgaccg 4680
gcagctcggc acaaaatcac cactcgatac aggcagccca tcagtccggg acggcgtcag 4740
cgggagagcc gttgtaaggc ggcagacttt gctcatgtta ccgatgctat tcggaagaac 4800
ggcaactaag ctgccgggtt tgaaacacgg atgatctcgc ggagggtagc atgttgattg 4860
taacgatgac agagcgttgc tgcctgtgat caaatatcat ctccctcgca gagatccgaa 4920
ttatcagcct tcttattcat ttctcgctta accgtgacag gctgtcgatc ttgagaacta 4980
tgccgacata ataggaaatc gctggataaa gccgctgagg aagctgagtg gcgctatttc 5040
tttagaagtg aacgttgacg atcgtcgacc gtaccccgat gaattaattc ggacgtacgt 5100
tctgaacaca gctggatact tacttgggcg attgtcatac atgacatcaa caatgtaccc 5160
gtttgtgtaa ccgtctcttg gaggttcgta tgacactagt ggttcccctc agcttgcgac 5220
tagatgttga ggcctaacat tttattagag agcaggctag ttgcttagat acatgatctt 5280
caggccgtta tctgtcaggg caagcgaaaa ttggccattt atgacgacca atgccccgca 5340

gaagctccca tctttgccgc catagacgcc gcgcccccct tttggggtgt agaacatcct 5400

tttgccagat gtggaaaaga agttcgttgt cccattgttg gcaatgacgt agtagccggc 5460

gaaagtgcga gacccatttg cgctatatat aagcctacga tttccgttgc gactattgtc 5520

gtaattggat gaactattat cgtagttgct ctcagagttg tcgtaatttg atggactatt 5580

gtcgtaattg cttatggagt tgtcgtagtt gcttggagaa atgtcgtagt tggatgggga 5640

gtagtcatag ggaagacgag cttcatccac taaaacaatt ggcaggtcag caagtgcctg 5700

ccccgatgcc atcgcaagta cgaggcttag aaccaccttc aacagatcgc gcatagtctt 5760

ccccagctct ctaacgcttg agttaagccg cgccgcgaag cggcgtcggc ttgaacgaat 5820

tgttagacat tatttgccga ctaccttggt gatctcgcct ttcacgtagt gaacaaattc 5880

ttccaactga tctgcgcgcg aggccaagcg atcttcttgt ccaagataag cctgcctagc 5940

ttcaagtatg acgggctgat actgggccgg caggcgctcc attgcccagt cggcagcgac 6000

atccttcggc gcgattttgc cggttactgc gctgtaccaa atgcgggaca acgtaagcac 6060

tacatttcgc tcatcgccag cccagtcggg cggcgagttc catagcgtta aggtttcatt 6120

tagcgcctca aatagatcct gttcaggaac cggatcaaag agttcctccg ccgctggacc 6180

taccaaggca acgctatgtt ctcttgcttt tgtcagcaag atagccagat caatgtcgat 6240

cgtggctggc tcgaagatac ctgcaagaat gtcattgcgc tgccattctc caaattgcag 6300

ttcgcgctta gctggataac gccacggaat gatgtcgtcg tgcacaacaa tggtgacttc 6360

tacagcgcgg agaatctcgc tctctccagg ggaagccgaa gtttccaaaa ggtcgttgat 6420

caaagctcgc cgcgttgttt catcaagcct tacagtcacc gtaaccagca aatcaatatc 6480

actgtgtggc ttcaggccgc catccactgc ggagccgtac aaatgtacgg ccagcaacgt 6540

cggttcgaga tggcgctcga tgacgccaac tacctctgat agttgagtcg atacttcggc 6600

gatcaccgct tccctcatga tgtttaactc ctgaattaag ccgcgccgcg aagcggtgtc 6660

ggcttgaatg aattgttagg cgtcatcctg tgctcccgag aaccagtacc agtacatcgc 6720

tgtttcgttc gagacttgag gtctagtttt atacgtgaac aggtcaatgc cgccgagagt 6780

aaagccacat tttgcgtaca aattgcaggc aggtacattg ttcgtttgtg tctctaatcg 6840

tatgccaagg agctgtctgc ttagtgccca ctttttcgca aattcgatga gactgtgcgc 6900

gactcctttg cctcggtgcg tgtgcgacac aacaatgtgt tcgatagagg ctagatcgtt 6960

ccatgttgag ttgagttcaa tcttcccgac aagctcttgg tcgatgaatg cgccatagca 7020

agcagagtct tcatcagagt catcatccga gatgtaatcc ttccggtagg ggctcacact 7080

tctggtagat agttcaaagc cttggtcgga taggtgcaca tcgaacactt cacgaacaat 7140

gaaatggttc tcagcatcca atgtttccgc cacctgctca gggatcaccg aaatcttcat 7200

atgacgccta acgcctggca cagcggatcg caaacctggc gcggcttttg gcacaaaagg 7260

cgtgacaggt ttgcgaatcc gttgctgcca cttgttaacc cttttgccag atttggtaac 7320

tataatttat gttagaggcg aagtcttggg taaaaactgg cctaaaattg ctggggattt 7380

caggaaagta aacatcacct tccggctcga tgtctattgt agatatatgt agtgtatcta 7440

cttgatcggg ggatctgctg cctcgcgcgt ttcggtgatg acggtgaaaa cctctgacac 7500

atgcagctcc cggagacggt cacagcttgt ctgtaagcgg atgccgggag cagacaagcc 7560

cgtcagggcg cgtcagcggg tgttggcggg tgtcggggcg cagccatgac ccagtcacgt 7620

agcgatagcg gagtgtatac tggcttaact atgcggcatc agagcagatt gtactgagag 7680

tgcaccatat gcggtgtgaa ataccgcaca gatgcgtaag gagaaaatac cgcatcaggc 7740

gctcttccgc ttcctcgctc actgactcgc tgcgctcggt cgttcggctg cggcgagcgg 7800

tatcagctca ctcaaaggcg gtaatacggt tatccacaga atcaggggat aacgcaggaa 7860

agaacatgtg agcaaaaggc cagcaaaagg ccaggaaccg taaaaaggcc gcgttgctgg 7920

cgtttttcca taggctccgc ccccctgacg agcatcacaa aaatcgacgc tcaagtcaga 7980

ggtggcgaaa cccgacagga ctataaagat accaggcgtt tccccctgga agctccctcg 8040

tgcgctctcc tgttccgacc ctgccgctta ccggatacct gtccgccttt ctcccttcgg 8100

gaagcgtggc gctttctcat agctcacgct gtaggtatct cagttcggtg taggtcgttc 8160

gctccaagct gggctgtgtg cacgaacccc ccgttcagcc cgaccgctgc gccttatccg 8220

gtaactatcg tcttgagtcc aacccggtaa gacacgactt atcgccactg gcagcagcca 8280

ctggtaacag gattagcaga gcgaggtatg taggcggtgc tacagagttc ttgaagtggt 8340

ggcctaacta cggctacact agaaggacag tatttggtat ctgcgctctg ctgaagccag 8400

ttaccttcgg aaaaagagtt ggtagctctt gatccggcaa acaaaccacc gctggtagcg 8460

gtggtttttt tgtttgcaag cagcagatta cgcgcagaaa aaaaggatct caagaagatc 8520

ctttgatctt ttctacgggg tctgacgctc agtggaacga aaactcacgt taagggattt 8580

tggtcatgag attatcaaaa aggatcttca cctagatcct tttaaattaa aaatgaagtt 8640

ttaaatcaat ctaaagtata tatgagtaaa cttggtctga cagttaccaa tgcttaatca 8700

gtgaggcacc tatctcagcg atctgtctat ttcgttcatc catagttgcc tgactccccg 8760

tcgtgtagat aactacgata cgggagggct taccatctgg ccccagtgct gcaatgatac 8820

cgcgagaccc acgctcaccg gctccagatt tatcagcaat aaaccagcca gccggaaggg 8880

ccgagcgcag aagtggtcct gcaactttat ccgcctccat ccagtctatt aattgttgcc 8940

gggaagctag agtaagtagt tcgccagtta atagtttgcg caacgttgtt gccattgctg 9000

caggggggggg gggggggggg gacttccatt gttcattcca cggacaaaaa cagagaaagg 9060

aaacgacaga ggccaaaaag cctcgctttc agcacctgtc gtttcctttc ttttcagagg 9120

gtattttaaa taaaaacatt aagttatgac gaagaagaac ggaaacgcct taaaccggaa 9180

aattttcata aatagcgaaa acccgcgagg tcgccgcccc gtaacctgtc ggatcaccgg 9240

aaaggacccg taaagtgata atgattatca tctacatatc acaacgtgcg tggaggccat 9300
caaaccacgt caaataatca attatgacgc aggtatcgta ttaattgatc tgcatcaact 9360
taacgtaaaa acaacttcag acaatacaaa tcagcgacac tgaatacggg gcaacctcat 9420
gtcccccccc cccccccccc tgcaggcatc gtggtgtcac gctcgtcgtt tggtatggct 9480
tcattcagct ccggttccca acgatcaagg cgagttacat gatcccccat gttgtgcaaa 9540
aaagcggtta gctccttcgg tcctccgatc gttgtcagaa gtaagttggc cgcagtgtta 9600
tcactcatgg ttatggcagc actgcataat tctcttactg tcatgccatc cgtaagatgc 9660
ttttctgtga ctggtgagta ctcaaccaag tcattctgag aatagtgtat gcggcgaccg 9720
agttgctctt gcccggcgtc aacacgggat aataccgcgc cacatagcag aactttaaaa 9780
gtgctcatca ttggaaaacg ttcttcgggg cgaaaactct caaggatctt accgctgttg 9840
agatccagtt cgatgtaacc cactcgtgca cccaactgat cttcagcatc ttttactttc 9900
accagcgttt ctgggtgagc aaaaacagga aggcaaaatg ccgcaaaaaa gggaataagg 9960
gcgacacgga aatgttgaat actcatactc ttcctttttc aatattattg aagcatttat 10020
cagggttatt gtctcatgag cggatacata tttgaatgta tttagaaaaa taaacaaata 10080
ggggttccgc gcacatttcc ccgaaaagtg ccacctgacg tctaagaaac cattattatc 10140
atgacattaa cctataaaaa taggcgtatc acgaggccct ttcgtcttca agaattggtc 10200
gacgatcttg ctgcgttcgg atattttcgt ggagttcccg ccacagaccc ggattgaagg 10260
cgagatccag caactcgcgc cagatcatcc tgtgacggaa ctttggcgcg tgatgactgg 10320
ccaggacgtc ggccgaaaga gcgacaagca gatcacgctt ttcgacagcg tcggatttgc 10380
gatcgaggat ttttcggcgc tgcgctacgt ccgcgaccgc gttgagggat caagccacag 10440
cagcccactc gaccttctag ccgacccaga cgagccaagg gatctttttg gaatgctgct 10500
ccgtcgtcag gctttccgac gtttgggtgg ttgaacagaa gtcattatcg tacggaatgc 10560
caagcactcc cgaggggaac cctgtggttg gcatgcacat acaaatggac gaacggataa 10620
accttttcac gccctttaa atatccgtta ttctaataaa cgctcttttc tcttaggttt 10680
acccgccaat atatcctgtc aaacactgat agtttaaact gaaggcggga aacgacaatc 10740
tgatcatgag cggagaatta agggagtcac gttatgaccc ccgccgatga cgcgggacaa 10800
gccgttttac gtttggaact gacagaaccg caacgttgaa ggagccactc agcaagctgg 10860
tacgattgta atacgactca ctatagggcg aattgagcgc tgtttaaacg ctcttcaact 10920
ggaagagcgg ttacccggac cgaagcttga agttcctatt ccgaagttcc tattctctag 10980
aaagtatagg aacttcagat ctcgatgctc accctgttgt ttggtgttac ttctgcaggt 11040
cgactctaga ggatccacca tgagcccaga acgacgcccg gccgacatcc gccgtgccac 11100
cgaggcggac atgccggcgg tctgcaccat cgtcaaccac tacatcgaga caagcacggt 11160

caacttccgt accgagccgc aggaaccgca ggactggacg gacgacctcg tccgtctgcg 11220

ggagcgctat ccctggctcg tcgccgaggt ggacggcgag gtcgccggca tcgcctacgc 11280

gggcccctgg aaggcacgca acgcctacga ctggacggcc gagtcgaccg tgtacgtctc 11340

cccccgccac cagcggacgg gactgggctc cacgctctac acccacctgc tgaagtccct 11400

ggaggcacag ggcttcaaga gcgtggtcgc tgtcatcggg ctgcccaacg acccgagcgt 11460

gcgcatgcac gaggcgctcg gatatgcccc ccgcggcatg ctgcgggcgg ccggcttcaa 11520

gcacgggaac tggcatgacg tgggtttctg gcagctggac ttcagcctgc cggtaccgcc 11580

ccgtccggtc ctgcccgtca ccgagatctg atccgtcgac caacctagac ttgtccatct 11640

tctggattgg ccaacttaat taatgtatga aataaaagga tgcacacata gtgacatgct 11700

aatcactata atgtgggcat caaagttgtg tgttatgtgt aattactagt tatctgaata 11760

aaagagaaag agatcatcca tatttcttat cctaaatgaa tgtcacgtgt ctttataatt 11820

ctttgatgaa ccagatgcat ttcattaacc aaatccatat acatataaat attaatcata 11880

tataattaat atcaattggg ttagcaaaac aaatctagtc taggtgtgtt ttgcgaattg 11940

cggccgcgat ctggggaatt cccatggaca ccggtaattc ccatgatctt ctctccttca 12000

tcaatggatg ccatgtttca taacaataac accaaatgtt tgatgagcta ccaacaattg 12060

cgcaaagact atggctaagc tcgagctcgc tcgctacaag ttgttgactt tcaaatacaa 12120

gtttgttttt ggaacaccaa atattctaca tgatctttca ctaagttgcg caccactatc 12180

aaaagattat ctaggccatt attcaagtaa agagtgaaca cgtctaagac ccacaaccac 12240

accaaataga atacgcatac atgcaacata ttgtgcaaga agtatccaac tggactccca 12300

tgtattctaa aactattttc gtagagttaa agttatgaca aacttatcaa ataaaaattt 12360

gaacgctgga ccaaaacttt catctttcaa atccaccatc gtctatcctc ataaattgtt 12420

ttgattataa cacatctacg taaatcattt gttttgaaca atactaattt aattttatta 12480

agtcaaataa cctgcttaga aaataatccc tccacctcat ttaacaattt cttgtcaaac 12540

acacaccaag aaaaaaatta atgaaagaga aaagaaatga aaaggacatg gagttgaata 12600

ctagcaaaat tgattgaagg aagattcaca attgaaattg aaaccattta atttattttc 12660

gggtccataa taataaattg gtaagaataa aaacccgatc aagtccggta cagtacaatt 12720

ccactccacc aactccttac ttaaacccct atttataccc actctcatcc tcactcttcc 12780

ttcacctctc acactctctt ctctctctca aaccctcac acaaacgctg cgtttagtgt 12840

aagaaattca atccgg 12856

<210> 57
<211> 825
<212> DNA
<213> Zea mays

<400> 57

aaatccttac agaattgctg tagtttcata gtgctagatg tggacagcaa agcgccgctg 60

tatgcttctg cttttctttt ttggtgtgtg tagccacatc ctttgttcct gcccggcgcc 120

atcccacttg gttgtttttt tttatgattg aaagccttca tgcttcctcg gtcaatcacc 180

ggtgcgcact gggagcatcg ccggaaaaaa aattcttcgg ctaagagtaa cttctttctc 240

cttttcttct ctgatctcgc gagcagtgct gataacgtgt tgtaatctac ttagcggtaa 300

cgagattgag agagacaaaa tgacagaact attgtcttta ttgcagagtg tcatgtattt 360

atacagggga tacaaagtct cccaaggggt gtgtcccttg ggagtaactg ccagttgatc 420

acaggacaat attttgtaac aaaacgtaca catcgtcaaa atagcgaggc atgaaactgg 480

ccttggccat ggacgcgtga agcgcgccat gcgttggata tgtggtcaat aagtatatac 540

aatacaatgt ttaacagagc tgatagtact gctttggcac atttttgtcc acgcttcatg 600

agagataaaa cacctgcacg taaattcaca tgctgcactg aaggcccgat cactgaggag 660

cgaactgccg taactccctt ctatatatac ccccagtccc tgtttcagtt ttcgtcaagc 720

tagcagcacc aagttgtcga tcacttgcct gctcttgagc tcgattaagc tatcatcagc 780

tacagcatcc gatcccaaac tgcaactgta gcagcgacaa ctgcc 825

<210> 58
<211> 860
<212> DNA
<213> Zea mays

<400> 58
ctggtaatta ttggctgtag gattctaaac agagcctaaa tagctggaat agctctagcc 60

ctcaatccaa actaatgata tctatactta tgcaactcta aatttttatt ctaaaagtaa 120

tatttcattt ttgtcaacga gattctctac tctattccac aatcttttga agcaatattt 180

accttaaatc tgtactctat accaataatc atatattcta ttatttattt ttatctctct 240

cctaaggagc atccccctat gtctgcatgg cccccgcctc gggtcccaat ctcttgctct 300

gctagtagca cagaagaaaa cactagaaat gacttgcttg acttagagta tcagataaac 360

atcatgttta cttaacttta atttgtatcg gtttctacta tttttataat atttttgtct 420

ctatagatac tacgtgcaac agtataatca acctagttta atccagagcg aaggattttt 480

tactaagtac gtgactccat atgcacagcg ttccttttat ggttcctcac tgggcacagc 540

ataaacgaac cctgtccaat gttttcagcg cgaacaaaca gaaattccat cagcgaacaa 600

acaacataca tgcgagatga aaataaataa taaaaaaagc tccgtctcga taggccggca 660

cgaatcgaga gcctccatag ccagtttttt ccatcggaac ggcggttcgc gcacctaatt 720

atatgcacca cacgcctata aagccaacca acccgtcgga ggggcgcaag ccagacagaa 780

gacagcccgt cagcccctct cgtttttcat ccgccttcgc ctccaaccgc gtgcgctcca 840

cgcctcctcc aggaaagcga 860

<210> 59
<211> 899
<212> DNA
<213> Zea mays

<400> 59
gtgcagcgtg acccggtcgt gcccctctct agagataatg agcattgcat gtctaagtta 60

taaaaaatta ccacatattt tttttgtcac acttgtttga agtgcagttt atctatcttt 120

atacatatat ttaaacttta ctctacgaat aatataatct atagtactac aataatatca 180

gtgttttaga gaatcatata aatgaacagt tagacatggt ctaaaggaca attgagtatt 240

ttgacaacag gactctacag ttttatcttt ttagtgtgca tgtgttctcc tttttttttg 300

caaatagctt cacctatata atacttcatc cattttatta gtacatccat ttagggttta 360

gggttaatgg tttttataga ctaatttttt tagtacatct attttattct attttagcct 420

ctaaattaag aaaactaaaa ctctatttta gtttttttat ttaataattt agatataaaa 480

tagaataaaa taaagtgact aaaaattaaa caaataccct ttaagaaatt aaaaaaacta 540

aggaaacatt tttcttgttt cgagtagata atgccagcct gttaaacgcc gtcgacgagt 600

ctaacggaca ccaaccagcg aaccagcagc gtcgcgtcgg gccaagcgaa gcagacggca 660

cggcatctct gtcgctgcct ctggacccct ctcgagagtt ccgctccacc gttggacttg 720

ctccgctgtc ggcatccaga aattgcgtgg cggagcggca gacgtgagcc ggcacggcag 780

gcggcctcct cctcctctca cggcacggca gctacggggg attcctttcc caccgctcct 840

tcgctttccc ttcctcgccc gccgtaataa atagacaccc cctccacacc ctctttccc 899

<210> 60
<211> 879
<212> DNA
<213> Medicago sativa

<400> 60
aattcccatg atcttctctc cttcatcaat ggatgccatg tttcataaca ataacaccaa 60

atgtttgatg agctaccaac aattgcgcaa agactatggc taagctcgag ctcgctcgct 120

acaagttgtt gactttcaaa tacaagtttg tttttggaac accaaatatt ctacatgatc 180

tttcactaag ttgcgcacca ctatcaaaag attatctagg ccattattca agtaaagagt 240

gaacacgtct aagacccaca accacaccaa atagaatacg catacatgca acatattgtg 300

caagaagtat ccaactggac tcccatgtat tctaaaacta ttttcgtaga gttaaagtta 360

tgacaaactt atcaaataaa aatttgaacg ctggaccaaa actttcatct ttcaaatcca 420

ccatcgtcta tcctcataaa ttgttttgat tataacacat ctacgtaaat catttgtttt 480

gaacaatact aatttaattt tattaagtca aataacctgc ttagaaaata atccctccac 540

ctcatttaac aatttcttgt caaacacaca ccaagaaaaa aattaatgaa agagaaaaga 600

aatgaaaagg acatggagtt gaatactagc aaaattgatt gaaggaagat tcacaattga 660

aattgaaacc atttaattta ttttcgggtc cataataata aattggtaag aataaaaacc 720

cgatcaagtc cggtacagta caattccact ccaccaactc cttacttaaa cccctattta 780

tacccactct catcctcact cttccttcac ctctcacact ctcttctctc tctcaaaacc 840

ctcacacaaa cgctgcgttt agtgtaagaa attcaatcc 879

<210> 61
<211> 318
<212> DNA
<213> Solanum tuberosum

<400> 61
agacttgtcc atcttctgga ttggccaact taattaatgt atgaaataaa aggatgcaca 60

catagtgaca tgctaatcac tataatgtgg gcatcaaagt tgtgtgttat gtgtaattac 120

tagttatctg aataaaagag aaagagatca tccatatttc ttatcctaaa tgaatgtcac 180

gtgtctttat aattctttga tgaaccagat gcatttcatt aaccaaatcc atatacatat 240

aaatattaat catatataat taatatcaat tgggttagca aaacaaatct agtctaggtg 300

tgttttgcga attgcggc 318

**Description**

This application claims the benefit of U.S. Provisional Application No. 60/968754 filed August 29, 2007, the entire contents of which are hereby incorporated by reference.

## FIELD OF THE INVENTION

The field of invention relates to plant breeding and genetics and, in particular, relates to recombinant DNA constructs useful in plants for altering root architecture.

## BACKGROUND OF THE INVENTION

Water and nutrient availability limit plant growth in all but a very few natural ecosystems. They limit yield in most agricultural ecosystems. Plant roots serve important functions such as water and nutrient uptake, anchorage of the plants in the soil and the establishment of biotic interactions at the rhizosphere. Elucidation of the genetic regulation of plant root development and function is therefore the subject of considerable interest in agriculture and ecology.

The root system originates from a primary root that develops during embryogenesis. The primary root produces secondary roots, which in turn produce tertiary roots. All secondary, tertiary, quaternary and further roots are referred to as lateral roots. Many plants, including maize, can also produce shoot borne roots, from consecutive under-ground nodes (crown roots) or above-ground nodes (brace roots). Three major processes affect the overall architecture of the root system. First, cell division at the primary root meristem enables indeterminate growth by adding new cells to the root. Second, lateral root formation increases the exploratory capacity of the root system. Third, root-hair formation increases the total surface of primary and lateral roots (Lopez-Bucio et al., Current Opinion in Plant Biology (2003) 6: 280-287). In maize mutants have been isolated that are missing only a subset of root types. In *Arabidopsis,* mutations in root patterning genes such as *SHORTROOT* and *SCARECROW ,* which show developmental defects in primary and lateral roots, have been identified (J.E. Malamy, Plant, Cell and Environment (2005) 28: 67-77).

A number of maize mutants affected specifically in root development have been identified (Hochholdinger et al 2004, Annals of Botany 93:359-368). The recessive mutants *rtcs* and *rt1* forms no, or fewer, crown and brace roots, while the primary and lateral roots are not affected. In the recessive mutants *des21*, lateral seminal roots and root hairs are absent. Root hairs are lacking in the recessive mutant *rthl-3.* The mutants *lrt1* and *rum1* are affected before lateral root initiation and mutants *slr1* and *slr2* are impaired in lateral root elongation. Intrinsic response pathways that determine root system architecture include hormones, cell cycle regulators and regulatory genes. Water stress and nutrient availability belong to the environmental response pathways that determine root system architecture.

U.S. Application No. 2005-57473 filed February 14, 2005 (U.S.Patent Publication No. 2005/223429 A1 published October 6, 2005) concerns the use of *Arabidopsis* cytokinin oxidase genes to alter cytokinin levels in plants and stimulate root growth.

U.S. Patent No. 6,344,601 (issued February 5, 2002) concerns the under- or overexpression of profilin in a plant cell to alter plant growth habit, e.g. a reduced root and root hair system, delay in the onset of flowering.

WO2004/US16432 (filed May 21, 2004 (WO2004/106531 published December 9, 2004) concerns the use of methods to manipulate the growth rate and/or yield and/or architecture by over expression of cis-prenyltransferase.

U.S. Application No. 2004/489500 filed September 30, 2004 (U.S.Patent Publication No. 2005/059154 A1 published March 13, 2005) concerns methods to modify cell number, architecture and yield using over expression of the transcription factor E2F in plants.

Activation tagging can be utilized to identify genes with the ability to affect a trait. This approach has been used in the model plant species *Arabidopsis thaliana* (Weigel et al., 2000, Plant Physiol. 122:1003-1013).

Insertions of transcriptional enhancer elements can dominantly activate and/or elevate the expression of nearby endogenous genes.

## SUMMARY OF THE INVENTION

The present invention includes:

In one embodiment, an isolated polynucleotide comprising a nucleic acid sequence encoding a NDK or NDK-like polypeptide having an amino acid sequence of at least 80% sequence identity, when compared to SEQ ID NO:25, or of at least 85% sequence identity, when compared to SEQ ID NO:23, or of at least 90%, when compared to SEQ ID NO:21, of at least 95%, when compared to SEQ ID NO:33 based on the Clustal V method of alignment, or a full complement of said nucleic acid sequence.

In a second embodiment, an isolated polynucleotide comprising a nucleic acid sequence encoding a NDK or NDK-like polypeptide having an amino acid sequence of at least 85% sequence identity, when compared to SEQ ID NO: 25, or of at least 90% sequence identity, when compared to SEQ ID NO:23, or of at least 95% sequence identity, when compared to SEQ ID NO:21 based on the Clustal V method of alignment, or a full complement of said nucleic acid sequence.

In a third embodiment, an isolated polynucleotide comprising a nucleic acid sequence encoding a NDK or NDK-like polypeptide having an amino acid sequence of at least 90% sequence identity, when compared to SEQ ID NO:25, or of at least 95% sequence identity, when compared to SEQ ID NO:23, based on the Clustal V method of alignment, or a full complement of said nucleic acid sequence.

In a fourth embodiment, an isolated polynucleotide comprising a nucleic acid sequence encoding a NDK or NDK-like polypeptide having an amino acid sequence of at least 95% sequence identity, when compared to SEQ ID NO: 25, based on the Clustal V method of alignment, or a full complement of said nucleic acid sequence.

In a fifth embodiment, an isolated polynucleotide comprising a nucleic acid sequence encoding a NDK or NDK-like polypeptide, wherein the amino acid sequence of the polypeptide comprises SEQ ID NO: 21, 23, 25, or 33.

In a sixth embodiment, an isolated polynucleotide comprising a nucleic acid sequence encoding a NDK or NDK-like polypeptide, wherein the nucleic acid sequence comprises SEQ ID NO:20, 22, 24, or 32.

In further embodiments, vectors and recombinant constructs comprising any of the foregoing polynucleotides and cells comprising the recombinant constructs.

In additional embodiments, methods for transforming a cell with any of the foregoing the polynucleotides and for producing and regenerating a transformed plant comprising any of the foregoing polynucleotides.

In another embodiment, a plant comprising in its genome a recombinant DNA construct comprising a polynucleotide operably linked to at least one regulatory element, wherein said polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51 and wherein said plant exhibits altered root architecture when compared to a control plant not comprising said recombinant DNA construct.

In another embodiment, a plant comprising in its genome a recombinant DNA construct comprising:

(a) a polynucleotide operably linked to at least one regulatory element,
wherein said polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51 or
(b) a suppression DNA construct comprising at least one regulatory element operably linked to: (i) all or part of: (A) a nucleic acid sequence encoding a polypeptide having an amino acid sequence of at least 50% sequence identity, based on the Clustal V method of alignment, when compared to SEQ ID NO:15, 17, 19, 21, 23, 25, 27, 29, 31, 33, 35, 37, 39, 41, 43, or 51 or (B) a full complement of the nucleic acid sequence of (b)(i)(A); or (ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide, and wherein said plant exhibits an alteration of at least one agronomic characteristic when compared to a control plant not comprising said recombinant DNA construct.

In another embodiment, a method of altering root architecture in a plant, comprising (a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO:15, 17, 19, 21, 23, 25, 27, 29, 31, 33, 35, 37, 39, 41, 43, or 51; and (b) regenerating a transgenic plant from the regenerable plant cell after step (a), wherein the transgenic plant comprises in its genome the recombinant DNA construct and exhibits altered root architecture when compared to a control plant not comprising the recombinant DNA construct; and optionally, (c) obtaining a progeny plant derived from the transgenic plant, wherein said progeny plant comprises in its genome the recombinant DNA construct and exhibits altered root architecture when compared to a control plant not comprising the recombinant DNA construct.

In another embodiment, a method of evaluating root architecture in a plant, comprising (a) introducing into a regenerable plant cell a recombinant DNA construct comprising a polynucleotides operably linked to at least one regulatory sequence, wherein the polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51 (b) regenerating a transgenic plant from the regenerable plant cell after step (a), wherein the transgenic plant comprises in its genome the recombinant DNA construct; and (c) evaluating root architecture of the transgenic plant compared to a control plant not comprising the recombinant DNA construct;

and optionally, (d) obtaining a progeny plant derived from the transgenic plant, wherein the progeny plant comprises in its genome the recombinant DNA construct; and optionally, (e) evaluating root architecture of the progeny plant compared to a control plant not comprising the recombinant DNA construct.

In another embodiment, a method of evaluating root architecture in a plant, comprising (a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51 (b) regenerating a transgenic plant from the regenerable plant cell after step (a), wherein the transgenic plant comprises in its genome the recombinant DNA construct; (c) obtaining a progeny plant derived from the transgenic plant, wherein the progeny plant comprises in its genome the recombinant DNA construct; and (d) evaluating root architecture of the progeny plant compared to a control plant not comprising the recombinant DNA construct.

In another embodiment, a method of determining an alteration of an agronomic characteristic in a plant, comprising (a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO:15,17, 19, 21, 23, 25, 27, 29, 31, 33, 35, 37, 39, 41, 43, or 51 (b) regenerating a transgenic plant from the regenerable plant cell after step (a), wherein the transgenic plant comprises in its genome the recombinant DNA construct; and (c) determining whether the transgenic plant exhibits an alteration of at least one agronomic characteristic when compared to a control plant not comprising the recombinant DNA construct; and optionally, (d) obtaining a progeny plant derived from the transgenic plant, wherein the progeny plant comprises in its genome the recombinant DNA construct; and optionally, (e) determining whether the progeny plant exhibits an alteration of at least one agronomic characteristic when compared to a control plant not comprising the recombinant DNA construct.

In another embodiment, a method of determining an alteration of an agronomic characteristic in a plant, comprising (a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51 (b) regenerating a transgenic plant from the regenerable plant cell after step (a), wherein the transgenic plant comprises in its genome the recombinant DNA construct; (c) obtaining a progeny plant derived from the transgenic plant, wherein the progeny plant comprises in its genome the recombinant DNA construct; and (d) determining whether the progeny plant exhibits an alteration of at least one agronomic characteristic when compared to a control plant not comprising the recombinant DNA construct.

In another embodiment, a method of determining an alteration of an agronomic characteristic in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51, or (B) a full complement of the nucleic acid sequence of (b)(i)(A); or

(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide;

(b) regenerating a transgenic plant from the regenerable plant cell after step (a), wherein the transgenic plant comprises in its genome the suppression DNA construct; and

(c) determining whether the transgenic plant exhibits an alteration of at least one agronomic characteristic when compared to a control plant not comprising the suppression DNA construct;

and optionally, (d) obtaining a progeny plant derived from the transgenic plant, wherein the progeny plant comprises in its genome the suppression DNA construct; and optionally, (e) determining whether the progeny plant exhibits an alteration of at least one agronomic characteristic when compared to a control plant not comprising the suppression DNA construct.

In another embodiment, a method of determining an alteration of an agronomic characteristic in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51, or (B) a full complement of the nucleic acid sequence of (b)(i)(A); or
(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide;

(b) regenerating a transgenic plant from the regenerable plant cell after step (a), wherein the transgenic plant comprises in its genome the suppression DNA construct and exhibits altered root architecture when compared to a control plant not comprising the suppression DNA construct;
(c) obtaining a progeny plant derived from the transgenic plant, wherein the progeny plant comprises in its genome the suppression DNA construct; and
(d) determining whether the progeny plant exhibits an alteration of at least one agronomic characteristic when compared to a control plant not comprising the suppression DNA construct.

In another embodiment, a method of altering root architecture in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51; or (B) a full complement of the nucleic acid sequence of (b)(i)(A); or
(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide; and

(b) regenerating a transgenic plant from the regenerable plant cell after step (a), wherein the transgenic plant comprises in its genome the suppression DNA construct and wherein the transgenic plant exhibits altered root architecture when compared to a control plant not comprising the suppression DNA construct; and

optionally, (c) obtaining a progeny plant derived from the transgenic plant, wherein said progeny plant comprises in its genome the recombinant DNA construct and wherein the progeny plant exhibits altered root architecture when compared to a control plant not comprising the suppression DNA construct.
In another embodiment, a method of evaluating root architecture in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51, or (B) a full complement of the nucleic acid sequence of (b)(i)(A); or
(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide;

(b) regenerating a transgenic plant from the regenerable plant cell after step (a), wherein the transgenic plant comprises in its genome the suppression DNA construct; and
(c) evaluating root architecture of the transgenic plant compared to a control plant not comprising the suppression DNA construct;

and optionally, (d) obtaining a progeny plant derived from the transgenic plant, wherein the progeny plant comprises in its genome the suppression DNA construct; and optionally, (e) evaluating root architecture of the progeny plant compared to a control plant not comprising the suppression DNA construct.

In another embodiment, a method of evaluating root architecture in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43, or 51, or (B) a full complement of the nucleic acid sequence of (b)(i)(A); or

(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide;

(b) regenerating a transgenic plant from the regenerable plant cell after step (a), wherein the transgenic plant comprises in its genome the suppression DNA construct;

(c) obtaining a progeny plant derived from the transgenic plant, wherein the progeny plant comprises in its genome the suppression DNA construct; and

(d) evaluating root architecture of the progeny plant compared to a control plant not comprising the suppression DNA construct.

Also included in the present invention is any progeny of the above plants, any seeds of the above plants, and cells from any of the above plants and progeny.

A method of producing seed that can be sold as a product offering with altered root architecture comprising any of the preceding preferred methods, and further comprising obtaining seeds from said progeny plant, wherein said seeds comprise in their genome said recombinant DNA construct.

## BRIEF DESCRIPTION OF THE FIGURES AND SEQUENCE LISTINGS

The invention can be more fully understood from the following detailed description and the accompanying drawings and Sequence Listing which form a part of this application.

Figure 1 shows a map of the pHSbarENDs activation tagging construct (SEQ ID N0:1) used to make the *Arabidopsis* populations.

Figure 2 shows a map of the vector pDONR™/Zeo (SEQ ID NO:2). The attP1 site is at nucleotides 570-801; the attP2 site is at nucleotides 2754-2985 (complementary strand).

Figure 3 shows a map of the vector pDONR™221 (SEQ ID NO:3). The attP1 site is at nucleotides 570-801; the attP2 site is at nucleotides 2754-2985 (complementary strand).

Figure 4 shows a map of the vector pBC-yellow (SEQ ID NO:4), a destination vector for use in construction of expression vectors for *Arabidopsis.* The attR1 site is at nucleotides 11276-11399 (complementary strand); the attR2 site is at nucleotides 9695-9819 (complementary strand).

Figure 5 shows a map of PHP27840 (SEQ ID NO:5), a destination vector for use in construction of expression vectors for soybean. The attR1 site is at nucleotides 7310-7434; the attR2 site is at nucleotides 8890-9014.

Figure 6 shows a map of PHP23236 (SEQ ID NO:6), a destination vector for use in construction of expression vectors for Gaspe Bay Flint derived maize lines. The attR1 site is at nucleotides 2006-2130; the attR2 site is at nucleotides 2899-3023.

Figure 7 shows a map of PHP10523 (SEQ ID NO:7), a plasmid DNA present in *Agrobacterium* strain LBA4404.

Figure 8 shows a map of PHP23235 (SEQ ID NO:8), a vector used to construct the destination vector PHP23236.

Figure 9 shows a map of the entry clone PHP20234 (SEQ ID NO:9), a vector carrying the PINII terminator. The attR2 site is at nucleotides 599-747; the attL3 site is at nucleotides 1100-1195.

Figure 10 shows a map of PHP28529 (SEQ ID NO:10), a destination vector for use in construction of expression vectors for maize lines. The attR3 site is at nucleotides 3613-3737; the attR4 site is at nucleotides 2035-2159.

Figure 11 shows a map of the entry clone PHP28408 (SEQ ID NO:11), a vector carrying the constitutive maize GOS2 promoter. The attL4 site is at nucleotides 160-255; the attR1 site is at nucleotides 2301-2447.
Figure 12 shows a map of the entry clone PHP22020 (SEQ ID NO:12), a vector carrying the root maize NAS2 promoter. The attR1 site is at nucleotides 31-187; the attL4 site is at nucleotides 2578-2673.
Figure 13 shows a map of PHP29635 (SEQ ID NO:13), a destination vector for use in construction of expression vectors for Gaspe Bay Flint derived maize lines. The attR1 site is at nucleotides 40786-40910; the attR2 site is at nucleotides 41679-41803.
Figure 14 shows a map of PIIOXS2a-FRT87(ni)m (SEQ ID NO:56), a vector used to construct the destination vector PHP29635.
Figs.15A -15K show the multiple alignment of the full length amino acid sequences of SEQ ID NOs: 15, 17, 19, 21, 23, 25, 27, 29, 31, 33, 35, and 37, and SEQ ID NOs:44, 45, 46, 47, 48, 49 and 51. Residues that match the Consensus sequence exactly are shaded. The consensus sequence is shown above each alignment. The consensus residues are determined by a straight majority.
Figure 16 shows a chart of the percent sequence identity and the divergence values for each pair of amino acid sequences of the NDK homologs displayed in Figures 15A-15K.
Figure 17 is the growth medium used for semi-hydroponics maize growth in Example 17.
Figure 18 is a chart setting forth data relating to the effect of different nitrate concentrations on the growth and development of Gaspe Bay Flint derived maize lines in Example 17.

The sequence descriptions and Sequence Listing attached hereto comply with the rules governing nucleotide and/or amino acid sequence disclosures in patent applications as set forth in 37 C.F.R. §1.821-1.825.
The Sequence Listing contains the one letter code for nucleotide sequence characters and the three letter codes for amino acids as defined in conformity with the IUPAC-IUBMB standards described in Nucleic Acids Res. 13: 3021-3030 (1985) and in the Biochemical J. 219 (No. 2):345-373 (1984) which are herein incorporated by reference. The symbols and format used for nucleotide and amino acid sequence data comply with the rules set forth in 37 C.F.R. §1.822.

SEQ ID NO:1 pHSbarENDs
SEQ ID NO:2 pDONR™/Zeo
SEQ ID NO:3 pDONR™221
SEQ ID NO:4 pBC-yellow
SEQ ID NO:5 PHP27840
SEQ ID NO:6 PHP23236
SEQ ID NO:7 PHP10523
SEQ ID NO:8 PHP23235
SEQ ID NO:9 PHP20234
SEQ ID NO:10 PHP28529
SEQ ID NO:11 PHP28408
SEQ ID NO:12 PHP22020
SEQ ID NO:13 PHP29635

Table 1 lists the polypeptides that are described herein, the designation of the cDNA clones that comprise the nucleic acid fragments encoding polypeptides representing all or a substantial portion of these polypeptides, and the corresponding identifier (SEQ ID NO:) as used in the attached Sequence listing.

**TABLE 1**

| Nucleoside Diphosphatase Kinase (NDK) | | | |
|---|---|---|---|
| Protein | Clone Designation | SEQ ID NO: (Nucleotide) | SEQ ID NO: (Amino Acid) |
| NDK | cest1s.pk013.p8:fis | 14 | 15 |
| NDK | cfp2n.pk070.b22:fiS | 16 | 17 |
| NDK | cdr1fi.pk002.a3.f:fis or contig of: cfp2n.pk069.c16 cfp2n.pk069.c16.f | 18 | 19 |
| NDK | lds1c.pk004.f12:fis | 20 | 21 |
| NDK | ep2c.pk002.f17.f:fis | 22 | 23 |
| NDK | ort1f.pk020.a13:fis | 24 | 25 |

(continued)

| Nucleoside Diphosphatase Kinase (NDK) | | | |
|---|---|---|---|
| Protein | Clone Designation | SEQ ID NO: (Nucleotide) | SEQ ID NO: (Amino Acid) |
| NDK | hss1c.pk019.a16:fis | 26 | 27 |
| NDK | sfl1.pk134.d19:fis | 28 | 29 |
| NDK | egh1c.pk002.114:fis | 30 | 31 |
| NDK | sfl1.pk133.14:fis | 32 | 33 |
| NDK | my.p0031.ccmbo48 | 34 | 35 |
| NDK | p0095.cwsab57ra | 36 | 37 |
| NDK | sfl1.pk126.p10 | 38 | 39 |
| NDK | rlr24.pk0071.e7 | 40 | 41 |
| NDK | cfp2n.pk069.c16 | 42 | 43 |

SEQ ID NO:44 corresponds to NCBI GI NO:115465831 (Rice)
SEQ ID NO:45 corresponds to NCBI GI No: 6435320 (Pea)
SEQ ID NO:46 corresponds to NCBI GI No:15237018 (Arabidopsis)
SEQ ID NO:47 corresponds to NCBI GI No:147864944 (Grapevine)
SEQ ID NO:48 corresponds to NCBI GI No: 62870979 (Pennycress)
SEQ ID NO:49 corresponds to NCBI GI No: 125595441 (Rice)
SEQ ID NO:50 is the nucleotide sequence of the *Arabidopsis thaliana*

Nucleoside diphosphatase kinase (NDK) (AT4G23900), wherein nucleotides 51-764 (Stop) code for the amino acid sequence represented in SEQ ID NO:51, NCBI General Identifier No. 11990430).

SEQ ID NO:51 corresponds to NCBI GI NO: 11990430 (AT4G23900)
SEQ ID NO:52 is the attB1 sequence.
SEQ ID NO:53 is the attB2 sequence.
SEQ ID NO:54 is the forward primer VC062 in Example 9.
SEQ ID NO:55 is the reverse primer VC063 in Example 9.
SEQ ID NO:56 PIIOXS2a-FRT87(ni)m.
SEQ ID NO:57 is the maize NAS2 promoter.
SEQ ID NO:58 is the GOS2 promoter.
SEQ ID NO:59 is the ubiquitin promoter.
SEQ ID NO:60 is the S2A promoter.
SEQ ID NO:61 is the PINII terminator.

## DETAILED DESCRIPTION OF PREFERRED EMBODIMENTS

The disclosure of each reference set forth herein is hereby incorporated by reference in its entirety.

As used herein and in the appended claims, the singular forms "a", "an", and "the" include plural reference unless the context clearly dictates otherwise. Thus, for example, reference to "a plant" includes a plurality of such plants, reference to "a cell" includes one or more cells and equivalents thereof known to those skilled in the art, and so forth.

The term "root architecture" refers to the arrangement of the different parts that comprise the root. The terms "root architecture", "root structure", "root system" or "root system architecture" are used interchangeably herewithin.

In general, the first root of a plant that develops from the embryo is called the primary root. In most dicots, the primary root is called the taproot. This main root grows downward and gives rise to branch (lateral) roots. In monocots the primary root of the plant branches, giving rise to a fibrous root system.

The term "altered root architecture" refers to aspects of alterations of the different parts that make up the root system at different stages of its development compared to a reference or control plant. It is understood that altered root architecture encompasses alterations in one or more measurable parameters, including but not limited to, the diameter, length, number, angle or surface of one or more of the root system parts, including but not limited to, the primary root, lateral or branch root, adventitious root, and root hairs, all of which fall within the scope of this invention. These changes can lead to an overall alteration in the area or volume occupied by the root. The reference or control plant does not comprise in its genome the recombinant DNA construct or heterologous construct.

"Agronomic characteristics" is a measurable parameter including but not limited to greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen stress tolerance, nitrogen uptake, root lodging, stalk lodging, plant height, ear length, and harvest index.

"Nitrogen stress tolerance" is a trait of a plant and refers to the ability of the plant to survive under nitrogen limiting conditions.

"Increased nitrogen stress tolerance" of a plant is measured relative to a reference or control plant, and means that the nitrogen stress tolerance of the plant is increased by any amount or measure when compared to the nitrogen stress tolerance of the reference or control plant.

A "nitrogen stress tolerant plant" is a plant that exhibits nitrogen stress tolerance. A nitrogen stress tolerant plant is preferably a plant that exhibits an increase in at least one agronomic characteristic relative to a control plant under nitrogen limiting conditions.

The term "V" stage refers to the leaf stages of a corn plant; e.g. V4 = four, V5=five leaves with visible leaf collars. The leaf collar is the light-colored collar-like "band" located at the base of an exposed leaf blade, near the spot where the leaf blade comes in contact with the stem of the plant. The leaves are counted beginning with the lowermost, short, rounded-tip true leaf and ending with the uppermost leaf with a visible leaf collar.

"ndk", "at-ndk, are used interchangeably herewithin and refer to the *Arabidopsis thaliana* locus, AT4G23900 (SEQ ID NO:50).

NDK refers to the protein (SEQ ID NO:51) encoded by AT4G23900 (SEQ ID N0:50).

"ndk-like" refers to nucleotide homologs from different species, such as corn and soybean, of the *Arabidopsis thaliana* "NDK" locus, AT4G23900 (SEQ ID NO:50) and includes without limitation any of the nucleotide sequences of SEQ ID NOs:14, 16, 18, 20, 22, 24, 26, 28, 30, 32, 34, 36, 38, 40, and 42.

"NDK-like" refers to protein homologs from different species, such as corn and soybean, of the *Arabidopsis thaliana* "NDK" (SEQ ID NO:51) and includes without limitation any of the amino acid sequences of SEQ ID NOs:15, 17, 19, 21, 23, 25, 27, 29, 31, 33, 35, 37, 39, 41, and 43

In the public database the Arabidopsis sequence (AT4G23900) is identified as NDK4 and the proteins from rice, Arabidopsis and Alpine Pennycress, and grapevine (NCBI Gi No's:115465831, 15237018, 62870979, and 1477864944, respectively) as NDKIII and the proteins from pea, and rice (6435320 and 125595441, respectively) as NDKI. It is not clear what determines the numbering of the different members of the NDK family and therefore the sequences of this disclosure are referred to commonly as NDK or NDK-like. All said public NDK's, the full length NDK-like protein homologs (SEQ ID NO's: 15, 17, 19, 21, 23, 25, 27, 29, 31, 33, 35, and 37 and the Arabidopsis NDK (SEQ ID NO:51) contain a conserved Histidine with the pattern NXXHGSDXX.

"Environmental conditions" refer to conditions under which the plant is grown, such as the availability of water, availability of nutrients (for example nitrogen), or the presence of disease.

"Transgenic" refers to any cell, cell line, callus, tissue, plant part or plant, the genome of which has been altered by the presence of a heterologous nucleic acid, such as a recombinant DNA construct, including those initial transgenic events as well as those created by sexual crosses or asexual propagation from the initial transgenic event. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

"Genome" as it applies to plant cells encompasses not only chromosomal DNA found within the nucleus, but organelle DNA found within subcellular components (e.g., mitochondrial, plastid) of the cell.

"Plant" includes reference to whole plants, plant organs, plant tissues, seeds and plant cells and progeny of same. Plant cells include, without limitation, cells from seeds, suspension cultures, embryos, meristematic regions, callus tissue, leaves, roots, shoots, gametophytes, sporophytes, pollen, and microspores.

"Progeny" comprises any subsequent generation of a plant.

"Transgenic" refers to any cell, cell line, callus, tissue, plant part or plant, the genome of which has been altered by the presence of a heterologous nucleic acid, such as a recombinant DNA construct, including those initial transgenic events as well as those created by sexual crosses or asexual propagation from the initial transgenic event. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

"Transgenic plant" includes reference to a plant which comprises within its genome a heterologous polynucleotide. Preferably, the heterologous polynucleotide is stably integrated within the genome such that the polynucleotide is passed on to successive generations. The heterologous polynucleotide may be integrated into the genome alone or as part of a recombinant DNA construct.

"Heterologous" with respect to sequence means a sequence that originates from a foreign species, or, if from the same species, is substantially modified from its native form in composition and/or genomic locus by deliberate human intervention.

"Polynucleotide", "nucleic acid sequence", "nucleotide sequence", or "nucleic acid fragment" are used interchangeably and is a polymer of RNA or DNA that is single- or double-stranded, optionally containing synthetic, non-natural or altered nucleotide bases. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

"Polypeptide", "peptide", "amino acid sequence" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The terms "polypeptide", "peptide", "amino acid sequence", and "protein" are also inclusive of modifications including, but not limited to, glycosylation, lipid attachment, sulfation, gamma-carboxylation of glutamic acid residues, hydroxylation and ADP-ribosylation.

"Messenger RNA (mRNA)" refers to the RNA that is without introns and that can be translated into protein by the cell.

"cDNA" refers to a DNA that is complementary to and synthesized from a mRNA template using the enzyme reverse transcriptase. The cDNA can be single-stranded or converted into the double-stranded form using the Klenow fragment of DNA polymerase I.

"Mature" protein refers to a post-translationally processed polypeptide; i.e., one from which any pre- or propeptides present in the primary translation product have been removed.

"Precursor" protein refers to the primary product of translation of mRNA; i.e., with pre- and pro-peptides still present. Pre- and pro-peptides may be and are not limited to intracellular localization signals.

"Isolated" refers to materials, such as nucleic acid molecules and/or proteins, which are substantially free or otherwise removed from components that normally accompany or interact with the materials in a naturally occurring environment. Isolated polynucleotides may be purified from a host cell in which they naturally occur. Conventional nucleic acid purification methods known to skilled artisans may be used to obtain isolated polynucleotides. The term also embraces recombinant polynucleotides and chemically synthesized polynucleotides.

"Recombinant" refers to an artificial combination of two otherwise separated segments of sequence, e.g., by chemical synthesis or by the manipulation of isolated segments of nucleic acids by genetic engineering techniques. "Recombinant" also includes reference to a cell or vector, that has been modified by the introduction of a heterologous nucleic acid or a cell derived from a cell so modified, but does not encompass the alteration of the cell or vector by naturally occurring events (e.g., spontaneous mutation, natural transformation/transduction/transposition) such as those occurring without deliberate human intervention.

"Recombinant DNA construct" refers to a combination of nucleic acid fragments that are not normally found together in nature. Accordingly, a recombinant DNA construct may comprise regulatory sequences and coding sequences that are derived from different sources, or regulatory sequences and coding sequences derived from the same source, but arranged in a manner different than that normally found in nature.

The terms "entry clone" and "entry vector" are used interchangeably herein.

"Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a nucleic acid fragment capable of controlling transcription of another nucleic acid fragment.

"Promoter functional in a plant" is a promoter capable of controlling transcription in plant cells whether or not its origin is from a plant cell.

"Tissue-specific promoter" and "tissue-preferred promoter" are used interchangeably, and refer to a promoter that is expressed predominantly but not necessarily exclusively in one tissue or organ, but that may also be expressed in one specific cell.

"Developmentally regulated promoter" refers to a promoter whose activity is determined by developmental events.

"Operably linked" refers to the association of nucleic acid fragments in a single fragment so that the function of one is regulated by the other. For example, a promoter is operably linked with a nucleic acid fragment when it is capable of regulating the transcription of that nucleic acid fragment.

"Expression" refers to the production of a functional product. For example, expression of a nucleic acid fragment may refer to transcription of the nucleic acid fragment (e.g., transcription resulting in mRNA or functional RNA) and/or translation of mRNA into a precursor or mature protein.

"Phenotype" means the detectable characteristics of a cell or organism.

"Introduced" in the context of inserting a nucleic acid fragment (e.g., a recombinant DNA construct) into a cell, means "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid or mitochondrial DNA), converted into an autonomous replicon, or transiently expressed (e.g., transfected mRNA).

A "transformed cell" is any cell into which a nucleic acid fragment (e.g., a recombinant DNA construct) has been introduced.

"Transformation" as used herein refers to both stable transformation and transient transformation.

"Stable transformation" refers to the introduction of a nucleic acid fragment into a genome of a host organism resulting in genetically stable inheritance. Once stably transformed, the nucleic acid fragment is stably integrated in the genome of the host organism and any subsequent generation.

"Transient transformation" refers to the introduction of a nucleic acid fragment into the nucleus, or DNA-containing organelle, of a host organism resulting in gene expression without genetically stable inheritance.

"Allele" is one of several alternative forms of a gene occupying a given locus on a chromosome. When the alleles present at a given locus on a pair of homologous chromosomes in a diploid plant are the same that plant is homozygous at that locus. If the alleles present at a given locus on a pair of homologous chromosomes in a diploid plant differ that plant is heterozygous at that locus. If a transgene is present on one of a pair of homologous chromosomes in a diploid plant that plant is hemizygous at that locus.

Sequence alignments and percent identity calculations may be determined using a variety of comparison methods designed to detect homologous sequences including, but not limited to, the Megalign® program of the LASERGENE® bioinformatics computing suite (DNASTAR® Inc., Madison, WI). Unless stated otherwise, multiple alignment of the sequences provided herein were performed using the Clustal V method of alignment (Higgins and Sharp (1989) CABIOS. 5:151-153) with the default parameters (GAP PENALTY=10, GAP LENGTH PENALTY=10). Default parameters for pairwise alignments and calculation of percent identity of protein sequences using the Clustal V method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences, using the Clustal V program, it is possible to obtain "percent identity" and "divergence" values by viewing the "sequence distances" table on the same program; unless stated otherwise, percent identities and divergences provided and claimed herein were calculated in this manner.

Standard recombinant DNA and molecular cloning techniques used herein are well known in the art and are described more fully in Sambrook, J., Fritsch, E.F. and Maniatis, T. Molecular Cloning: A Laboratory Manual; Cold Spring Harbor Laboratory Press: Cold Spring Harbor, 1989 (hereinafter "Sambrook").

Turning now to preferred embodiments:

Preferred embodiments include isolated polynucleotides and polypeptides, recombinant DNA constructs, compositions (such as plants or seeds) comprising these recombinant DNA constructs, and methods utilizing these recombinant DNA constructs.

Preferred Isolated Polynucleotides and Polypeptides

The present invention includes the following preferred isolated polynucleotides and polypeptides:

An isolated polynucleotide comprising: (i) a nucleic acid sequence encoding a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51; or (ii) a full complement of the nucleic acid sequence of (i). Any of the foregoing isolated polynucleotides may be utilized in any recombinant DNA constructs (including suppression DNA constructs) of the present invention. The polypeptide is preferably a NDK or NDK-like protein.

An isolated polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51. The polypeptide is preferably a NDK or NDK-like protein.

An isolated polynucleotide comprising (i) a nucleic acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO:14, 16, 18, 20, 22, 24, 26, 28, 30, 32, 34, 36, 38, 40, 42 or 50, or (ii) a full complement of the nucleic acid sequence of (i). Any of the foregoing isolated polynucleotides may be utilized in any recombinant DNA constructs (including suppression DNA constructs) of the present invention. The isolated polynucleotide encodes a NDK or NDK-like protein.

Preferred Recombinant DNA Constructs and Suppression DNA Constructs.

In one aspect, the present invention includes recombinant DNA constructs (including suppression DNA constructs).

In one preferred embodiment, a recombinant DNA construct comprises a polynucleotide operably linked to at least one regulatory sequence (e.g., a promoter functional in a plant), wherein the polynucleotide comprises (i) a nucleic acid sequence encoding an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO:15, 17,19, 21, 23, 25, 27, 29, 31, 33, 35, 37, 39, 41, 43 or 51, or (ii) a full complement of the nucleic acid sequence of (i).

In another preferred embodiment, a recombinant DNA construct comprises a polynucleotide operably linked to at least one regulatory sequence (e.g., a promoter functional in a plant), wherein said polynucleotide comprises (i) a nucleic acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO:14, 16, 18, 20, 22, 24, 26, 28, 30, 32, 34, 36, 38, 40, 42 or 50, or (ii) a full complement of the nucleic acid sequence of (i).

Figs.15A -15K show the multiple alignment of the full length amino acid sequences of SEQ ID NOs:15, 17, 19, 21, 23, 25, 27, 29, 31, 33, 35, and 37, and SEQ ID NOs:44, 45, 40, 47, 48, 49 and 51. The multiple alignment of the sequences was performed using the Megalign® program of the LASERGENE® bioinformatics computing suite (DNASTAR® Inc., Madison, WI); in particular, using the Clustal V method of alignment (Higgins and Sharp (1989) CABIOS. 5: 151-153) with the multiple alignment default parameters of GAP PENALTY=10 and GAP LENGTH PENALTY=10, and the pairwise alignment default parameters of KTUPLE=1, GAP PENALTY=3, WINDOW=5 and DIAGONALS SAVED=5.

Fig.16 shows the percent sequence identity and the divergence values for each pair of amino acids sequences displayed in Figs. 15A-15K.

In another preferred embodiment, a recombinant DNA construct comprises a polynucleotide operably linked to at least one regulatory sequence (e.g., a promoter functional in a plant), wherein said polynucleotide encodes a NDK or NDK-like protein.

In another aspect, the present invention includes suppression DNA constructs.

A suppression DNA construct preferably comprises at least one regulatory sequence (preferably a promoter functional in a plant) operably linked to (a) all or part of (i) a nucleic acid sequence encoding a polypeptide having an amino acid sequence of at least 50%, 51 %, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO:15, 17, 19, 21, 23, 25, 27, 29, 31, 33, 35, 37, 39, 41, 43 or 51, or (ii) a full complement of the nucleic acid sequence of (a)(i); or (b) a region derived from all or part of a sense strand or antisense strand of a target gene of interest, said region having a nucleic acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to said all or part of a sense strand or antisense strand from which said region is derived, and wherein said target gene of interest encodes a NDK or NDK-like protein; or (c) all or part of (i) a nucleic acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO:14, 16, 18, 20, 22, 24, 26, 28, 30, 32, 34, 36, 38, 40, 42 or 50, or (ii) a full complement of the nucleic acid sequence of (c)(i). The suppression DNA construct preferably comprises a cosuppression construct, antisense construct, viral-suppression construct, hairpin suppression construct, stem-loop suppression construct, double-stranded RNA-producing construct, RNAi construct, or small RNA construct (e.g., an siRNA construct or an miRNA construct).

It is understood, as those skilled in the art will appreciate, that the invention encompasses more than the specific exemplary sequences. Alterations in a nucleic acid fragment which result in the production of a chemically equivalent amino acid at a given site, but do not affect the functional properties of the encoded polypeptide, are well known in the art. For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the polypeptide molecule would also not be expected to alter the activity of the polypeptide. Each of the proposed modifications is well within the routine skill in the art, as is determination of retention of biological activity of the encoded products.

"Suppression DNA construct" is a recombinant DNA construct which when transformed or stably integrated into the genome of the plant, results in "silencing" of a target gene in the plant. The target gene may be endogenous or transgenic to the plant. "Silencing," as used herein with respect to the target gene, refers generally to the suppression of levels of mRNA or protein/enzyme expressed by the target gene, and/or the level of the enzyme activity or protein functionality. The term "suppression" includes lower, reduce, decline, decrease, inhibit, eliminate or prevent. "Silencing" or "gene silencing" does not specify mechanism and is inclusive, and not limited to, anti-sense, cosuppression, viral-suppression, hairpin suppression, stem-loop suppression, RNAi-based approaches, and small RNA-based approaches.

A suppression DNA construct may comprise a region derived from a target gene of interest and may comprise all or part of the nucleic acid sequence of the sense strand (or antisense strand) of the target gene of interest. Depending upon the approach to be utilized, the region may be 100% identical or less than 100% identical (e.g., at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identical) to all or part of the sense strand (or antisense strand) of the gene of interest.

Suppression DNA constructs are well-known in the art, are readily constructed once the target gene of interest is selected, and include, without limitation, cosuppression constructs, antisense constructs, viral-suppression constructs, hairpin suppression constructs, stem-loop suppression constructs, double-stranded RNA-producing constructs, and more generally, RNAi (RNA interference) constructs and small RNA constructs such as siRNA (short interfering RNA) constructs and miRNA (microRNA) constructs.

"Antisense inhibition" refers to the production of antisense RNA transcripts capable of suppressing the expression of the target protein. "Antisense RNA" refers to an RNA transcript that is complementary to all or part of a target primary transcript or mRNA and that blocks the expression of a target isolated nucleic acid fragment (U.S. Patent No. 5,107,065). The complementarity of an antisense RNA may be with any part of the specific gene transcript, i.e., at the 5' non-coding sequence, 3' non-coding sequence, introns, or the coding sequence.

"Cosuppression" refers to the production of sense RNA transcripts capable of suppressing the expression of the target protein. "Sense" RNA refers to RNA transcript that includes the mRNA and can be translated into protein within a cell or *in vitro.* Cosuppression constructs in plants have been previously designed by focusing on overexpression of a nucleic acid sequence having homology to a native mRNA, in the sense orientation, which results in the reduction of all RNA having homology to the overexpressed sequence (see Vaucheret et al. (1998) Plant J. 16:651-659; and Gura (2000) Nature 404:804-808).

Another variation describes the use of plant viral sequences to direct the suppression of proximal mRNA encoding sequences (PCT Publication WO 98/36083 published on August 20, 1998).

Previously described is the use of "hairpin" structures that incorporate all, or part, of an mRNA encoding sequence in a complementary orientation that results in a potential "stem-loop" structure for the expressed RNA (PCT Publication WO 99/53050 published on October 21, 1999). In this case the stem is formed by polynucleotides corresponding to the gene of interest inserted in either sense or anti-sense orientation with respect to the promoter and the loop is formed by some polynucleotides of the gene of interest, which do not have a complement in the construct. This increases the frequency of cosuppression or silencing in the recovered transgenic plants. For review of hairpin suppression see Wesley, S.V. et al. (2003) Methods in Molecular Biology, Plant Functional Genomics: Methods and Protocols 236:273-286.

A construct where the stem is formed by at least 30 nucleotides from a gene to be suppressed and the loop is formed by a random nucleotide sequence has also effectively been used for suppression (PCT Publication No. WO 99/61632 published on December 2, 1999).

The use of poly-T and poly-A sequences to generate the stem in the stem-loop structure has also been described (PCT Publication No. WO 02/00894 published January 3, 2002).

Yet another variation includes using synthetic repeats to promote formation of a stem in the stem-loop structure.

Transgenic organisms prepared with such recombinant DNA fragments have been shown to have reduced levels of the protein encoded by the nucleotide fragment forming the loop as described in PCT Publication No. WO 02/00904, published 03 January 2002.

RNA interference refers to the process of sequence-specific post-transcriptional gene silencing in animals mediated by short interfering RNAs (siRNAs) (Fire et al., Nature 391:806 1998). The corresponding process in plants is commonly referred to as post-transcriptional gene silencing (PTGS) or RNA silencing and is also referred to as quelling in fungi. The process of post-transcriptional gene silencing is thought to be an evolutionarily-conserved cellular defense mechanism used to prevent the expression of foreign genes and is commonly shared by diverse flora and phyla (Fire et al., Trends Genet. 15:358 1999). Such protection from foreign gene expression may have evolved in response to the production of double-stranded RNAs (dsRNAs) derived from viral infection or from the random integration of transposon elements into a host genome via a cellular response that specifically destroys homologous single-stranded RNA of viral genomic RNA. The presence of dsRNA in cells triggers the RNAi response through a mechanism that has yet to be fully characterized.

The presence of long dsRNAs in cells stimulates the activity of a ribonuclease III enzyme referred to as dicer. Dicer is involved in the processing of the dsRNA into short pieces of dsRNA known as short interfering RNAs (siRNAs) (Berstein et al., Nature 409:363, 2001). Short interfering RNAs derived from dicer activity are typically about 21 to about 23 nucleotides in length and comprise about 19 base pair duplexes (Elbashir et al., Genes Dev. 15:188,2001). Dicer has also been implicated in the excision of 21- and 22-nucleotide small temporal RNAs (stRNAs) from precursor RNA of conserved structure that are implicated in translational control (Hutvagner et al., Science 293:834, 2001). The RNAi response also features an endonuclease complex, commonly referred to as an RNA-induced silencing complex (RISC), which mediates cleavage of single-stranded RNA having sequence complementarity to the antisense strand of the siRNA duplex. Cleavage of the target RNA takes place in the middle of the region complementary to the antisense strand of the siRNA duplex (Elbashir et al., Genes Dev. 15:188,2001). In addition, RNA interference can also involve small RNA (e.g., miRNA) mediated gene silencing, presumably through cellular mechanisms that regulate chromatin structure and thereby prevent transcription of target gene sequences (see, e.g., Allshire, Science 297:1818-1819, 2002; Volpe et al., Science 297:1833-1837, 2002; Jenuwein, Science 297:2215-2218, 2002; and Hall et al., Science 297:2232-2237, 2002). As such, miRNA molecules of the invention can be used to mediate gene silencing via interaction with RNA transcripts or alternately by interaction with particular gene sequences, wherein such interaction results in gene silencing either at the transcriptional or post-transcriptional level.

RNAi has been studied in a variety of systems. Fire et al. (Nature 391:806, 1998) were the first to observe RNAi in C. elegans. Wianny and Goetz (Nature Cell Biol. 2:70, 1999) describe RNAi mediated by dsRNA in mouse embryos. Hammond et al. (Nature 404:293, 2000) describe RNAi in Drosophila cells transfected with dsRNA. Elbashir et al., (Nature 411:494, 2001) describe RNAi induced by introduction of duplexes of synthetic 21-nucleotide RNAs in cultured mammalian cells including human embryonic kidney and HeLa cells.

Small RNAs play an important role in controlling gene expression. Regulation of many developmental processes, including flowering, is controlled by small RNAs. It is now possible to engineer changes in gene expression of plant genes by using transgenic constructs which produce small RNAs in the plant.

Small RNAs appear to function by base-pairing to complementary RNA or DNA target sequences. When bound to RNA, small RNAs trigger either RNA cleavage or translational inhibition of the target sequence. When bound to DNA target sequences, it is thought that small RNAs can mediate DNA methylation of the target sequence. The consequence of these events, regardless of the specific mechanism, is that gene expression is inhibited.

It is thought that sequence complementarity between small RNAs and their RNA targets helps to determine which mechanism, RNA cleavage or translational inhibition, is employed. It is believed that siRNAs, which are perfectly complementary with their targets, work by RNA cleavage. Some miRNAs have perfect or near-perfect complementarity with their targets, and RNA cleavage has been demonstrated for at least a few of these miRNAs. Other miRNAs have several mismatches with their targets, and apparently inhibit their targets at the translational level. Again, without being held to a particular theory on the mechanism of action, a general rule is emerging that perfect or near-perfect complementarity causes RNA cleavage, whereas translational inhibition is favored when the miRNA/target duplex contains many mismatches. The apparent exception to this is microRNA 172 (miR172) in plants. One of the targets of miR172 is APETALA2 (AP2), and although miR172 shares near-perfect complementarity with AP2 it appears to cause translational inhibition of AP2 rather than RNA cleavage.

MicroRNAs (miRNAs) are noncoding RNAs of about 19 to about 24 nucleotides (nt) in length that have been identified in both animals and plants (Lagos-Quintana et al., Science 294:853-858 2001, Lagas-Quintana et al., Curr. Biol. 12:735-739, 2002; Lau et al., Science 294:858-862, 2001; Lee and Ambros, Science 294:862-864, 2001; Llave et al., Plant Cell 14:1605-1619, 2002; Mourelatos et al., Genes. Dev. 16:720-728, 2002; Park et al., Curr. Biol. 12:1484-1495, 2002; Reinhart et al., Genes. Dev. 16:1616-1626, 2002). They are processed from longer precursor transcripts that range in size from approximately 70 to 200 nt, and these precursor transcripts have the ability to form stable hairpin structures. In animals, the enzyme involved in processing miRNA precursors is called Dicer, an RNAse III-like protein (Grishok et al., Cell 106:23-34, 2001; Hutvagner et al., Science 293:834-838, 2001; Ketting et al., Genes. Dev. 15: 2654-2659, 2001). Plants also have a Dicer-like enzyme, DCL1 (previously named CARPEL FACTORY/SHORT INTEGUMENTS1/ SUSPENSOR1), and recent evidence indicates that it, like Dicer, is involved in processing the hairpin precursors to generate mature miRNAs (Park et al., Curr. Biol. 12:1484-1495, 2002; Reinhart et al., Genes. Dev. 16: 1616-1626, 2002). Furthermore, it is becoming clear from recent work that at least some miRNA hairpin precursors originate as longer polyadenylated transcripts, and several different miRNAs and associated hairpins can be present in a single transcript (Lagos-Quintana et al., Science 294:853-858, 2001; Lee et al., EMBO J 21:4663-4670, 2002). Recent work has also examined the selection of the miRNA strand from the dsRNA product arising from processing of the hairpin by DICER (Schwartz, et al., Cell 115:199-208, 2003). It appears that the stability (i.e. G:C vs. A:U content, and/or mismatches) of the two ends of the processed dsRNA affects the strand selection, with the low stability end being easier to unwind by a helicase activity. The 5' end strand at the low stability end is incorporated into the RISC complex, while the other strand is degraded.

MicroRNAs appear to regulate target genes by binding to complementary sequences located in the transcripts produced by these genes. In the case of lin-4 and let-7, the target sites are located in the 3' UTRs of the target mRNAs (Lee et al., Cell 75:843-854, 1993; Wightman et al., Cell 75:855-862, 1993; Reinhart et al., Nature 403:901-906, 2000; Slack et al., Mol. Cell 5:659-669, 2000), and there are several mismatches between the lin-4 and let-7 miRNAs and their target sites. Binding of the lin-4 or let-7 miRNA appears to cause downregulation of steady-state levels of the protein encoded by the target mRNA without affecting the transcript itself (Olsen and Ambros, Dev. Biol. 216:671-680, 1999). On the other hand, recent evidence suggests that miRNAs can in some cases cause specific RNA cleavage of the target transcript within the target site, and this cleavage step appears to require 100% complementarity between the miRNA and the target transcript (Hutvagner and Zamore, Science 297:2056-2060, 2002; Llave et al., Plant Cell 14:1605-1619, 2002). It seems likely that miRNAs can enter at least two pathways of target gene regulation: Protein downregulation when target complementarity is <100%, and RNA cleavage when target complementarity is 100%. MicroRNAs entering the RNA cleavage pathway are analogous to the 21-25 nt short interfering RNAs (siRNAs) generated during RNA interference (RNAi) in animals and posttranscriptional gene silencing (PTGS) in plants (Hamilton and Baulcombe 1999; Hammond et al., 2000; Zamore et al., 2000; Elbashir et al., 2001), and likely are incorporated into an RNA-induced silencing complex (RISC) that is similar or identical to that seen for RNAi.

Identifying the targets of miRNAs with bioinformatics has not been successful in animals, and this is probably due to the fact that animal miRNAs have a low degree of complementarity with their targets. On the other hand, bioinformatic approaches have been successfully used to predict targets for plant miRNAs (Llave et al., Plant Cell 14: 1605-1619 2002; Park et al., Curr. Biol. 12:1484-1495 2002; Rhoades et al., Cell 110:513-520 2002), and thus it appears that plant miRNAs have higher overall complementarity with their putative targets than do animal miRNAs. Most of these predicted target transcripts of plant miRNAs encode members of transcription factor families implicated in plant developmental patterning or cell differentiation.

A recombinant DNA construct (including a suppression DNA construct) of the present invention preferably comprises at least one regulatory sequence.

A preferred regulatory sequence is a promoter.

A number of promoters can be used in recombinant DNA constructs (and suppression DNA constructs) of the present invention. The promoters can be selected based on the desired outcome, and may include constitutive, tissue-specific, cell specific, inducible, or other promoters for expression in the host organism.

High level, constitutive expression of the candidate gene under control of the 35S or UBI promoter may have pleiotropic effects, although Candidate gene efficacy may be estimated when driven by a constitutive promoter.

Use of tissue-specific and/or stress-specific expression may eliminate undesirable effects but retain the ability to alter root architecture. This effect has been observed in *Arabidopsis* (Kasuga et al. (1999) Nature Biotechnol. 17: 287-291).

Suitable constitutive promoters for use in a plant host cell include, for example, the core promoter of the Rsyn7 promoter and other constitutive promoters disclosed in WO 99/43838 and U.S. Patent No. 6,072,050; the core CaMV 35S promoter (Odell et al., Nature 313:810-812 (1985)); rice actin (McElroy et al., Plant Cell 2:163-171 (1990)); ubiquitin (UBI) (Christensen metal, Plant Mol. Biol. 12:619-632 (1989) and Christensen et al., Plant Mol. Biol. 18:675-689 (1992)); pEMU (Last et al., Theor. Appl. Genet. 81:581-588 (1991)); MAS (Velten et al., EMBO J. 3:2723-2730 (1984)); ALS promoter (U.S. Patent No. 5,659,026), the maize GOS2 promoter (WO0020571 A2, published April 1, 2000) and the like. Other constitutive promoters include, for example, those discussed in U.S. Patent Nos. 5,608,149; 5,508,144; 5,604,121; 5,589,597; 5,466,785; 5,399,680; 5,268,463; 5,608,142; and 6,177,611.

In choosing a promoter to use in the methods of the invention, it may be desirable to use a tissue-specific or developmentally regulated promoter.

A preferred tissue-specific or developmentally regulated promoter is a DNA sequence which regulates the expression of a DNA sequence selectively in the cells/tissues of a plant critical to tassel development, seed set, or both, and limits the expression of such a DNA sequence to the period of tassel development or seed maturation in the plant.

Any identifiable promoter may be used in the methods of the present invention which causes the desired temporal and spatial expression.

Promoters which are seed or embryo specific and may be useful in the invention include soybean Kunitz trysin inhibitor (Kti3, Jofuku and Goldberg, Plant Cell 1:1079-1093 (1989)), patatin (potato tubers) (Rocha-Sosa, M., et al. (1989) EMBO J. 8:23-29), convicilin, vicilin, and legumin (pea cotyledons) (Rerie, W.G., et al. (1991) Mol. Gen. Genet. 259:149-157; Newbigin, E.J., et al. (1990) Planta 180:481-470; Higgins, T.J.V., et al. (1988) Plant. Mol. Biol. 11:683-695), zein (maize endosperm) (Schemthaner, J.P., et al. (1988) EMBO J. 7:1249-1255), phaseolin (bean cotyledon) (Segupta-Gopalan, C., et al. (1985) Proc. Natl. Acad. Sci. U.S.A. 82:3320-3324), phytohemagglutinin (bean cotyledon) (Voelker, T. et al. (1987) EMBO J. 6:3571-3577), B-conglycinin and glycinin (soybean cotyledon) (Chen, Z-L, et al. (1988) EMBO J. 7:297- 302), glutelin (rice endosperm), hordein (barley endosperm) (Marris, C., et al. (1988) Plant Mol. Biol. 10: 359-366), glutenin and gliadin (wheat endosperm) (Colot, V., et al. (1987) EMBO J. 6:3559-3554), and sporamin (sweet potato tuberous root) (Hattori, T., et al. (1990) Plant Mol. Biol. 14:595-604). Promoters of seed-specific genes operably linked to heterologous coding regions in chimeric gene constructions maintain their temporal and spatial expression pattern in transgenic plants. Such examples include *Arabidopsis thaliana* 2S seed storage protein gene promoter to express enkephalin peptides in *Arabidopsis* and *Brassica napus* seeds (Vanderkerckhove et al., Bio/Technology 7: L929-932 (1989)), bean lectin and bean beta-phaseolin promoters to express luciferase (Riggs et al., Plant Sci. 63:47-57 (1989)), and wheat glutenin promoters to express chloramphenicol acetyl transferase (Colot et al., EMBO J 6:3559-3564 (1987)).

Inducible promoters selectively express an operably linked DNA sequence in response to the presence of an endogenous or exogenous stimulus, for example by chemical compounds (chemical inducers) or in response to environmental, hormonal, chemical, and/or developmental signals. Inducible or regulated promoters include, for example, promoters regulated by light, heat, stress, flooding or drought, phytohormones, wounding, or chemicals such as ethanol, jasmonate, salicylic acid, or safeners.

Preferred promoters include the following: 1) the stress-inducible RD29A promoter (Kasuga et al. (1999) Nature Biotechnol. 17:287-91); 2) the barley promoter, B22E; expression of B22E is specific to the pedicel in developing maize kernels ("Primary Structure of a Novel Barley Gene Differentially Expressed in Immature Aleurone Layers". Klemsdal, S.S. et al., Mol. Gen. Genet. 228(1/2):9-16 (19991)); and 3) maize promoter, Zag2 ("Identification and molecular characterization of ZAG1, the maize homolog of the Arabidopsis floral homeotic gene AGAMOUS", Schmidt, R.J. et al., Plant Cell 5(7):729-737 (1993))."Structural characterization, chromosomal localization and phylogenetic evaluation of two pairs of AGAMOUS-like MADS-box genes from maize", Theissen et al., Gene 156(2): 155-168 (1995); NCBI GenBank Accession No. X80206)). Zag2 transcripts can be detected 5 days prior to pollination to 7 to 8 days after pollination (DAP), and directs expression in the carpel of developing female inflorescences and Ciml which is specific to the nucleus of developing maize kernels. Ciml transcript is detected 4 to 5 days before pollination to 6 to 8 DAP. Other useful promoters include any promoter which can be derived from a gene whose expression is maternally associated with developing female florets.

Additional preferred promoters for regulating the expression of the nucleotide sequences of the present invention in plants are vascular element specific or stalk-preferrred promoters. Such stalk-preferred promoters include the alfalfa S2A promoter (GenBank Accession No. EF030816; Abrahams et al., Plant Mol. Biol. 27:513-528 (1995)) and S2B promoter (GenBank Accession No. EF030817) and the like, herein incorporated by reference.

Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters". New promoters of various types useful in plant cells are constantly being discovered; numerous examples may be found in the compilation by Okamuro, J. K., and Goldberg, R. B., Biochemistry of Plants 15:1-82 (1989). (Put this with the other constitutive promoter description.)

Preferred promoters may include: RIP2, mLIP15, ZmCOR1, Rab17, CaMV 35S, RD29A, B22E, Zag2, SAM synthetase, ubiquitin (SEQ ID NO:61), CaMV 19S, nos, Adh, sucrose synthase, R-allele, root cell promoter, the vascular tissue specific promoters S2A (Genbank accession number EF030816; SEQ ID NO:62) and S2B (Genbank accession number EF030817) and the constitutive promoter GOS2 (SEQ ID NO:60) from *Zea mays.* Other preferred promoters include root preferred promoters, such as the maize NAS2 promoter (SEQ ID NO:59), the maize Cyclo promoter (US 2006/0156439, published July 13, 2006), the maize ROOTMET2 promoter (WO05063998, published July 14, 2005), the CR1BIO promoter (WO06055487, published May 26, 2006), the CRWAQ81 (WO05035770, published April 21, 2005) and the maize ZRP2.47 promoter (NCBI accession number: U38790, gi: 1063664).

A "substantial portion" of a nucleotide sequence comprises a nucleotide sequence that is sufficient to afford putative identification of the promoter that the nucleotide sequence comprises. Nucleotide sequences can be evaluated either manually, by one skilled in the art, or using computer-based sequence comparison and identification tools that employ algorithms such as BLAST (Basic Local Alignment Search Tool; Altschul et al. (1993) J. Mol. Siol. 215:403-410). In general, a sequence of thirty or more contiguous nucleotides is necessary in order to putatively identify a promoter nucleic acid sequence as homologous to a known promoter. The skilled artisan, having the benefit of the sequences as reported herein, may now use all or a substantial portion of the disclosed sequences for purposes known to those skilled in this art. Accordingly, the instant invention comprises the complete sequences as reported in the accompanying Sequence Listing, as well as substantial portions of those sequences as defined above.

Recombinant DNA constructs (and suppression DNA constructs) of the present invention may also include other regulatory sequences, including but not limited to, translation leader sequences, introns, and polyadenylation recognition sequences. In another preferred embodiment of the present invention, a recombinant DNA construct of the present invention further comprises an enhancer or silencer.

An intron sequence can be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold. Buchman and Berg, Mol. Cell Biol. 8:4395-4405 (1988); Callis et al., Genes Dev. 1:1183-1200 (1987). Such intron enhancement of gene expression is typically greatest when placed near the 5' end of the transcription unit. Use of maize introns Adh1-S intron 1, 2, and 6, the Bronze-1 intron are known in the art. See generally, The Maize Handbook, Chapter 116, Freeling and Walbot, Eds., Springer, New York (1994).

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added can be derived from, for example, the nopaline synthase or octopine synthase genes, or alternatively from another plant gene, or less preferably from any other eukaryotic gene.

A translation leader sequence is a DNA sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner, R. and Foster, G. D. Molecular Biotechnology 3:225 (1995)).

In another preferred embodiment of the present invention, a recombinant DNA construct of the present invention further comprises an enhancer or silencer.

Any plant can be selected for the identification of regulatory sequences and genes to be used in creating recombinant DNA constructs and suppression DNA constructs of the present invention. Examples of suitable plant targets for the isolation of genes and regulatory sequences would include but are not limited to alfalfa, apple, apricot, *Arabidopsis,* artichoke, arugula, asparagus, avocado, banana, barley, beans, beet, blackberry, blueberry, broccoli, brussels sprouts, cabbage, canola, cantaloupe, carrot, cassava, castorbean, cauliflower, celery, cherry, chicory, cilantro, citrus, clementines, clover, coconut, coffee, corn, cotton, cranberry, cucumber, Douglas fir, eggplant, endive, escarole, eucalyptus, fennel, figs, garlic, gourd, grape, grapefruit, honey dew, jicama, kiwifruit, lettuce, leeks, lemon, lime, Loblolly pine, linseed, mango, melon, mushroom, nectarine, nut, oat, oil palm, oil seed rape, okra, olive, onion, orange, an ornamental plant, palm, papaya, parsley, parsnip, pea, peach, peanut, pear, pepper, persimmon, pine, pineapple, plantain, plum, pomegranate, poplar, potato, pumpkin, quince, radiata pine, radicchio, radish, rapeseed, raspberry, rice, rye, sorghum, Southern pine, soybean, spinach, squash, strawberry, sugarbeet, sugarcane, sunflower, sweet potato, sweetgum, tangerine, tea, tobacco, tomato, triticale, turf, turnip, a vine, watermelon, wheat, yarns, and zucchini. Particularly preferred plants for the identification of regulatory sequences are *Arabidopsis,* corn, wheat, soybean, and cotton.

## Preferred Compositions

A preferred composition of the present invention is a plant comprising in its genome any of the recombinant DNA constructs (including any of the suppression DNA constructs) of the present invention (such as those preferred constructs discussed above). Preferred compositions also include any progeny of the plant, and any seed obtained from the plant or its progeny, wherein the progeny or seed comprises within its genome the recombinant DNA construct (or suppression DNA construct). Progeny includes subsequent generations obtained by self-pollination or out-crossing of a plant. Progeny also includes hybrids and inbreds.

Preferably, in hybrid seed propagated crops, mature transgenic plants can be self-pollinated to produce a homozygous inbred plant. The inbred plant produces seed containing the newly introduced recombinant DNA construct (or suppression DNA construct). These seeds can be grown to produce plants that would exhibit altered root (or plant) architecture, or used in a breeding program to produce hybrid seed, which can be grown to produce plants that would exhibit altered root (or plant) architecture. Preferably, the seeds are maize.

Preferably, the plant is a monocotyledonous or dicotyledonous plant, more preferably, a maize or soybean plant, even more preferably a maize plant, such as a maize hybrid plant or a maize inbred plant. The plant may also be sunflower, sorghum, castor bean, grape, canola, wheat, alfalfa, cotton, rice, barley or millet.

Preferably, the recombinant DNA construct is stably integrated into the genome of the plant.

Particularly preferred embodiments include but are not limited to the following preferred embodiments:

1. A plant (preferably a maize or soybean plant) comprising in its genome a recombinant DNA construct comprising a polynucleotide operably linked to at least one regulatory sequence, wherein said polynucleotide encodes a polypeptide having an amino acid sequence of at least 50%, 51 %, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51, and wherein said plant exhibits an altered root architecture when compared to a control plant not comprising said recombinant DNA construct. Preferably, the plant further exhibits an alteration of at least one agronomic characteristic when compared to the control plant.

2. A plant (preferably a maize or soybean plant) comprising in its genome:

   a recombinant DNA construct comprising:

      (a) a polynucleotide operably linked to at least one regulatory element, wherein said polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51, or

      (b) a suppression DNA construct comprising at least one regulatory element operably linked to:

         (i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51, or (B) a full complement of the nucleic acid sequence of (b)(i)(A); or

         (ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide, and wherein said plant exhibits an alteration of at least one agronomic characteristic when compared to a control plant not comprising said recombinant DNA construct.

3. A plant (preferably a maize or soybean plant) comprising in its genome a recombinant DNA construct comprising a polynucleotide operably linked to at least one regulatory sequence, wherein said polynucleotide encodes a NDK or NDK-like protein, and wherein said plant exhibits an altered root architecture when compared to a control plant not comprising said recombinant DNA construct. Preferably, the plant further exhibits an alteration of at least one agronomic characteristic.
Preferably, the NDK protein is from *Arabidopsis thaliana, Zea mays, Glycine max, Glycine tabacina, Glycine soja* or *Glycine tomentella.*

4. A plant (preferably a maize or soybean plant) comprising in its genome a suppression DNA construct comprising at least one regulatory element operably linked to a region derived from all or part of a sense strand or antisense strand of a target gene of interest, said region having a nucleic acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to said all or part of a sense strand or antisense strand from which said region is derived, and wherein said target gene of interest encodes a NDK or NDK-like protein, and wherein said plant exhibits an alteration of at least one agronomic characteristic when compared to a control plant not comprising said recombinant DNA construct.

5. A plant (preferably a maize or soybean plant) comprising in its genome a suppression DNA construct comprising at least one regulatory element operably linked to all or part of (a) a nucleic acid sequence encoding a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51, or (b) a full complement of the nucleic acid sequence of (a), and wherein said plant exhibits an alteration of at least one agronomic characteristic when compared to a control plant not comprising said recombinant DNA construct.

6. Any progeny of the above plants in preferred embodiments 1-5, any seeds of the above plants in preferred embodiments 1-5, any seeds of progeny of the above plants in preferred embodiments 1-5, and cells from any of the above plants in preferred embodiments 1-5 and progeny thereof.

In any of the foregoing preferred embodiments 1-6 or any other embodiments of the present invention, the recombinant DNA construct (or suppression DNA construct) preferably comprises at least a promoter that is functional in a plant as a preferred regulatory sequence.

In any of the foregoing preferred embodiments 1-6 or any other embodiments of the present invention, the alteration of at least one agronomic characteristic is either an increase or decrease, preferably an increase.

In any of the foregoing preferred embodiments 1-6 or any other embodiments of the present invention, the at least one agronomic characteristic is preferably selected from the group consisting of greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen uptake, nitrogen stress tolerance, root lodging, stalk lodging, plant height, ear length and harvest index. Yield, greenness, biomass and root lodging are particularly preferred agronomic characteristics for alteration (preferably an increase).

In any of the foregoing preferred embodiments 1-6 or any other embodiments of the present invention, the plant preferably exhibits the alteration of at least one agronomic characteristic irrespective of the environmental conditions, for example, water and nutrient availability, when compared to a control plant.

One of ordinary skill in the art is familiar with protocols for determining alteration in plant root architecture. For example, transgenic maize plants can be assayed for changes in root architecture at seedling stage, flowering time or maturity. Alterations in root architecture can be determined by counting the nodal root numbers of the top 3 or 4 nodes of the greenhouse grown plants or the width of the root band. "Root band" refers to the width of the mat of roots at the bottom of a pot at plant maturity. Other measures of alterations in root architecture include, but are not limited to, the number of lateral roots, average root diameter of nodal roots, average root diameter of lateral roots, number and length of root hairs. The extent of lateral root branching (e.g. lateral root number, lateral root length) can be determined by sub-sampling a complete root system, imaging with a flat-bed scanner or a digital camera and analyzing with WinRHIZO™ software (Regent Instruments Inc.).

Data taken on root phenotype are subjected to statistical analysis, normally a t-test to compare the transgenic roots with that of non-transgenic sibling plants. One-way ANOVA may also be used in cases where multiple events and/or constructs are involved in the analysis.

The Examples below describe some representative protocols and techniques for detecting alterations in root architecture.

One can also evaluate alterations in root architecture by the ability of the plant to increase yield in field testing when compared, under the same conditions, to a control or reference plant.

One can also evaluate alterations in root architecture by the ability of the plant to maintain substantial yield (preferably at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% yield) in field testing under stress conditions (e.g., nutrient over-abundance or limitation, water over-abundance or limitation, presence of disease), when compared to the yield of a control or reference plant under non-stressed conditions.

Alterations in root architecture can also be measured by determining the resistance to root lodging of the transgenic plants compared to reference or control plants.

One of ordinary skill in the art would readily recognize a suitable control or reference plant to be utilized when assessing or measuring an agronomic characteristic or phenotype of a transgenic plant in any embodiment of the present invention in which a control or reference plant is utilized (e.g., compositions or methods as described herein). For example, by way of non-limiting illustrations:

1. Progeny of a transformed plant which is hemizygous with respect to a recombinant DNA construct (or suppression DNA construct), such that the progeny are segregating into plants either comprising or not comprising the recombinant DNA construct (or suppression DNA construct): the progeny comprising the recombinant DNA construct (or suppression DNA construct) would be typically measured relative to the progeny not comprising the recombinant DNA construct (or suppression DNA construct) (i.e., the progeny not comprising the recombinant DNA construct (or suppression DNA construct) is the control or reference plant).

2. Introgression of a recombinant DNA construct (or suppression DNA construct) into an inbred line, such as in maize, or into a variety, such as in soybean: the introgressed line would typically be measured relative to the parent inbred or variety line (i.e., the parent inbred or variety line is the control or reference plant).

3. Two hybrid lines, where the first hybrid line is produced from two parent inbred lines, and the second hybrid line is produced from the same two parent inbred lines except that one of the parent inbred lines contains a recombinant DNA construct (or suppression DNA construct): the second hybrid line would typically be measured relative to the first hybrid line (i.e., the parent inbred or variety line is the control or reference plant).

4. A plant comprising a recombinant DNA construct (or suppression DNA construct): the plant may be assessed or measured relative to a control plant not comprising the recombinant DNA construct (or suppression DNA construct) but otherwise having a comparable genetic background to the plant (e.g., sharing at least 90%, 91%, 92%, 93%, 94%, 95%, 96%, 97%, 98%, 99%, or 100% sequence identity of nuclear genetic material compared to the plant comprising the recombinant DNA construct (or suppression DNA construct). There are many laboratory-based techniques available for the analysis, comparison and characterization of plant genetic backgrounds; among these are Isozyme Electrophoresis, Restriction Fragment Length Polymorphisms (RFLPs), Randomly Amplified Polymorphic DNAs (RAPDs), Arbitrarily Primed Polymerase Chain Reaction (AP-PCR), DNA Amplification Fingerprinting (DAF), Sequence Characterized Amplified Regions (SCARs), Amplified Fragment Length Polymorphisms (AFLP®s), and Simple Sequence Repeats (SSRs) which are also referred to as Microsatellites.

Furthermore, one of ordinary skill in the art would readily recognize that a suitable control or reference plant to be utilized when assessing or measuring an agronomic characteristic or phenotype of a transgenic plant would not include a plant that had been previously selected, via mutagenesis or transformation, for the desired agronomic characteristic or phenotype.

Preferred Methods

Preferred methods include but are not limited to methods for altering root architecture in a plant, methods for evaluating alteration of root architecture in a plant, methods for altering an agronomic characteristic in a plant, methods for determining an alteration of an agronomic characteristic in a plant, and methods for producing seed. Preferably, the plant is a monocotyledonous or dicotyledonous plant, more preferably, a maize or soybean plant, even more preferably a maize plant. The plant may also be sunflower, sorghum, castor bean, canola, wheat, alfalfa, cotton, rice, barley or millet. The seed is preferably a maize or soybean seed, more preferably a maize seed, and even more preferably, a maize hybrid seed or maize inbred seed.

Particularly preferred methods include but are not limited to the following:

A method of altering root architecture of a plant, comprising: (a) introducing into a regenerable plant cell a recombinant DNA construct comprising a polynucleotide operably linked to at least one regulatory sequence (preferably a promoter functional in a plant), wherein the polynucleotide encodes a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 5, 17, 19, 21, 23, 25, 27, 29, 31, 33, 35, 37, 39, 41, 43 or 51; and (b) regenerating a transgenic plant from the regenerable plant cell after step (a), wherein the transgenic plant comprises in its genome the recombinant DNA construct and exhibits altered root architecture when compared to a control plant not comprising the recombinant DNA construct. The method may further comprise (c) obtaining a progeny plant derived from the transgenic plant, wherein said progeny plant comprises in its genome the recombinant DNA construct and exhibits altered root architecture when compared to a control plant not comprising the recombinant DNA construct.

A method of altering root architecture in a plant, comprising: (a) introducing into a regenerable plant cell a suppression DNA construct comprising at least one regulatory sequence (preferably a promoter functional in a plant) operably linked to:

(i) all or part of: (A) a nucleic acid sequence encoding a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51, or (B) a full complement of the nucleic acid sequence of (a)(i)(A); or

(ii) a region derived from all or part of a sense strand or antisense strand of a target gene of interest, said region having a nucleic acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91 %, 92%, 93%, 94%, 95%, 96%, 97%, 98%, 99%, or 100% sequence identity, based on the Clustal V method of alignment, when compared to said all or part of a sense strand or antisense strand from which said region is derived, and wherein said target gene of interest encodes a NDK or NDK-like polypeptide; and

(b) regenerating a transgenic plant from the regenerable plant cell after step (a), wherein the transgenic plant comprises in its genome the recombinant DNA construct and exhibits an altered root architecture when compared to a control plant not comprising the suppression DNA construct. The method may further comprise (c) obtaining a progeny plant derived from the transgenic plant, wherein said progeny plant comprises in its genome the recombinant DNA construct and exhibits altered root architrecture when compared to a control plant not comprising the suppression DNA construct.

A method of evaluating altered root architecture in a plant, comprising (a) introducing into a regenerable plant cell a recombinant DNA construct comprising a polynucleotide operably linked to at least on regulatory sequence (preferably a promoter functional in a plant), wherein the polynucleotide encodes a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51, or (b) regenerating a transgenic plant from the regenerable plant cell after step (a), wherein the transgenic plant comprises in its genome the recombinant DNA construct; and (c) evaluating root architecture of the transgenic plant compared to a control plant not comprising the recombinant DNA construct. The method may further comprise (d) obtaining a progeny plant derived from the transgenic plant, wherein the progeny plant comprises in its genome the recombinant DNA construct; and (e) evaluating root architecture of the progeny plant compared to a control plant not comprising the recombinant DNA construct.

A method of evaluating altered root architecture in a plant, comprising (a) introducing into a regenerable plant cell a suppression DNA construct comprising at least one regulatory sequence (preferably a promoter functional in a plant) operably linked to:

(i) all or part of: (A) a nucleic acid sequence encoding a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15,17, 19, 21, 23, 25, 27, 29, 31, 33, 35, 37, 39, 41, 43 or 51, or (B) a full complement of the nucleic acid sequence of (a)(i)(A); or (ii) a region derived from all or part of a sense strand or antisense strand of a target gene of interest, said region having a nucleic acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to said all or part of a sense strand or antisense strand from which said region is derived, and wherein said target gene of interest encodes a NDK or NDK-like polypeptide; and

(b) regenerating a transgenic plant from the regenerable plant cell after step (a), wherein the transgenic plant comprises in its genome the suppression DNA construct; and (c) evaluating the transgenic plant for altered root architecture compared to a control plant not comprising the suppression DNA construct. The method may further comprise (d) obtaining a progeny plant derived from the transgenic plant, wherein the progeny plant comprises in its genome the suppression DNA construct; and (e) evaluating the progeny plant for altered root architecture compared to a control plant not comprising the suppression DNA construct.

A method of evaluating altered root architecture in a plant, comprising (a) introducing into a regenerable plant cell a recombinant DNA construct comprising a polynucleotide operably linked to at least one regulatory sequence (preferably a promoter functional in a plant), wherein said polynucleotide encodes a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51 (b) regenerating a transgenic plant from the regenerable plant cell after step (a), wherein the transgenic plant comprises in its genome the recombinant DNA construct; (c) obtaining a progeny plant derived from said transgenic plant, wherein the progeny plant comprises in its genome the recombinant DNA construct; and (d) evaluating the progeny plant for altered root architecture compared to a control plant not comprising the recombinant DNA construct.

A method of evaluating root architecture in a plant, comprising:

(a) introducing into a regenerable plant cell a suppression DNA construct comprising at least one regulatory element operably linked to: (i) all or part of: (A) a nucleic acid sequence encoding a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51, or (B) a full complement of the nucleic acid sequence of (a)(i)(A); or (ii) a region derived from all or part of a sense strand or antisense strand of a target gene of interest, said region having a nucleic acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to said all or part of a sense strand or antisense strand from which said region is derived, and wherein said target gene of interest encodes a NDK or NDK-like polypeptide; (b) regenerating a transgenic plant from the regenerable plant cell after step (a), wherein the transgenic plant comprises in its genome the suppression DNA construct;
(c) obtaining a progeny plant derived from the transgenic plant, wherein the progeny plant comprises in its genome the suppression DNA construct; and (d) evaluating root architecture of the progeny plant compared to a control plant not comprising the suppression DNA construct.

A method of determining an alteration of an agronomic characteristic in a plant, comprising (a) introducing into a regenerable plant cell a recombinant DNA construct comprising a polynucleotide operably linked to at least on regulatory sequence (preferably a promoter functional in a plant), wherein said polynucleotide encodes a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51 (b) regenerating a transgenic plant from the regenerable plant cell after step (a), wherein the transgenic plant comprises in its genome said recombinant DNA construct; and (c) determining whether the transgenic plant exhibits an alteration of at least one agronomic characteristic when compared to a control plant not comprising the recombinant DNA construct. The method may further comprise (d) obtaining a progeny plant derived from the transgenic plant, wherein the progeny plant comprises in its genome the recombinant DNA construct; and (e) determining whether the progeny plant exhibits an alteration of at least one agronomic characteristic when compared to a control plant not comprising the recombinant DNA construct.

A method of determining an alteration of an agronomic characteristic in a plant, comprising (a) introducing into a regenerable plant cell a suppression DNA construct comprising at least one regulatory sequence (preferably a promoter functional in a plant) operably linked to all or part of (i) a nucleic acid sequence encoding a polypeptide having an amino acid sequence of at least 50%, 51 %, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51, or (ii) a full complement of the nucleic acid sequence of (i); (b) regenerating a transgenic plant from the regenerable plant cell after step (a), wherein the transgenic plant comprises in its genome the suppression DNA construct; and (c) determining whether the transgenic plant exhibits an alteration in at least one agronomic characteristic when compared to a control plant not comprising the suppression DNA construct. The method may further comprise (d) obtaining a progeny plant derived from the transgenic plant, wherein the progeny plant comprises in its genome the suppression DNA construct; and (e) determining whether the progeny plant exhibits an alteration in at least one agronomic characteristic when compared to a control plant not comprising the suppression DNA construct.

A method of determining an alteration of an agronomic characteristic in a plant, comprising (a) introducing into a regenerable plant cell a recombinant DNA construct comprising a polynucleotide operably linked to at least one regulatory sequence (preferably a promoter functional in a plant), wherein said polynucleotide encodes a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51 (b) regenerating a transgenic plant from the regenerable plant cell after step (a), wherein the transgenic plant comprises in its genome said recombinant DNA construct; (c) obtaining a progeny plant derived from said transgenic plant, wherein the progeny plant comprises in its genome the recombinant DNA construct; and (d) determining whether the progeny plant exhibits an alteration of at least one agronomic characteristic when compared to a control plant not comprising the recombinant DNA construct. The method of determining an alteration of an agronomic characteristic in a plant may further comprise determining whether the transgenic plant exhibits an alteration of at least one agronomic characteristic when compared, under varying environmental conditions, to a control plant not comprising the recombinant DNA construct.

A method of determining an alteration of an agronomic characteristic in a plant, comprising (a) introducing into a regenerable plant cell a suppression DNA construct comprising at least one regulatory sequence (preferably a promoter functional in a plant) operably linked to all or part of (i) a nucleic acid sequence encoding a polypeptide having an amino acid sequence of at least 50%, 51%, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to SEQ ID NO: 15, 17, 19, 21, 23, 25, 27, 29, 31, 33, 35, 37, 39, 41, 43 or 51, or (ii) a full complement of the nucleic acid sequence of (i);
(b) regenerating a transgenic plant from the regenerable plant cell after step (a), wherein the transgenic plant comprises in its genome the suppression DNA construct; (c) obtaining a progeny plant derived from said transgenic plant, wherein the progeny plant comprises in its genome the suppression DNA construct; and (d) determining whether the progeny plant exhibits an alteration in at least one

agronomic characteristic when compared to a control plant not comprising the recombinant DNA construct.

A method of determining an alteration of an agronomic characteristic in a plant, comprising: (a) introducing into a regenerable plant cell a suppression DNA construct comprising at least one regulatory element operably linked to a region derived from all or part of a sense strand or antisense strand of a target gene of interest, said region having a nucleic acid sequence of at least 50%, 51 %, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to said all or part of a sense strand or antisense strand from which said region is derived, and wherein said target gene of interest encodes a NDK or NDK-like polypeptide; (b) regenerating a transgenic plant from the regenerable plant cell after step (a), wherein the transgenic plant comprises in its genome the suppression DNA construct; and (c) determining whether the transgenic plant exhibits an alteration of at least one agronomic characteristic when compared to a control plant not comprising the suppression DNA construct. The method may further comprise: (d) obtaining a progeny plant derived from the transgenic plant, wherein the progeny plant comprises in its genome the suppression DNA construct; and (e) determining whether the progeny plant exhibits an alteration of at least one agronomic characteristic when compared to a control plant not comprising the suppression DNA construct.

A method of determining an alteration of an agronomic characteristic in a plant, comprising: (a) introducing into a regenerable plant cell a suppression DNA construct comprising at least one regulatory element operably linked to a region derived from all or part of a sense strand or antisense strand of a target gene of interest, said region having a nucleic acid sequence of at least 50%, 51 %, 52%, 53%, 54%, 55%, 56%, 57%, 58%, 59%, 60%, 56%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity, based on the Clustal V method of alignment, when compared to said all or part of a sense strand or antisense strand from which said region is derived, and wherein said target gene of interest encodes a NDK or NDK-like polypeptide; (b) regenerating a transgenic plant from the regenerable plant cell after step (a), wherein the transgenic plant comprises in its genome the suppression DNA construct; (c) obtaining a progeny plant derived from the transgenic plant, wherein the progeny plant comprises in its genome the suppression DNA construct; and (d) determining whether the progeny plant exhibits an alteration of at least one agronomic characteristic when compared to a control plant not comprising the suppression DNA construct.

A method of producing seed (preferably seed that can be sold as a product offering with altered root architecture) comprising any of the preceding preferred methods, and further comprising obtaining seeds from said progeny plant, wherein said seeds comprise in their genome said recombinant DNA construct (or suppression DNA construct).

In any of the foregoing preferred methods or any other embodiments of methods of the present invention, the step of determining an alteration of an agronomic characteristic in a transgenic plant, if applicable, may preferably comprise determining whether the transgenic plant exhibits an alteration of at least one agronomic characteristic when compared, under varying environmental conditions, to a control plant not comprising the recombinant DNA construct.

In any of the foregoing preferred methods or any other embodiments of methods of the present invention, the step of determining an alteration of an agronomic characteristic in a progeny plant, if applicable, may preferably comprise determining whether the progeny plant exhibits an alteration of at least one agronomic characteristic when compared, under varying environmental conditions, to a control plant not comprising the recombinant DNA construct.

In any of the preceding preferred methods or any other embodiments of methods of the present invention, in said introducing step said regenerable plant cell preferably comprises a callus cell (preferably embryogenic), a gametic cell, a meristematic cell, or a cell of an immature embryo. The regenerable plant cells are preferably from an inbred maize plant.

In any of the preceding preferred methods or any other embodiments of methods of the present invention, said regenerating step preferably comprises: (i) culturing said transformed plant cells in a media comprising an embryogenic promoting hormone until callus organization is observed; (ii) transferring said transformed plant cells of step (i) to a first media which includes a tissue organization promoting hormone; and (iii) subculturing said transformed plant cells after step (ii) onto a second media, to allow for shoot elongation, root development or both.

In any of the preceding preferred methods or any other embodiments of methods of the present invention, alternatives exist for introducing into a regenerable plant cell a recombinant DNA construct comprising a polynucleotide operably linked to at least one regulatory sequence. For example, one may introduce into a regenerable plant cell a regulatory sequence (such as one or more enhancers, preferably as part of a transposable element), and then screen for an event in which the regulatory sequence is operably linked to an endogenous gene encoding a polypeptide of the instant invention.

The introduction of recombinant DNA constructs of the present invention into plants may be carried out by any suitable technique, including but not limited to direct DNA uptake, chemical treatment, electroporation, microinjection, cell fusion, infection, vector mediated DNA transfer, bombardment, or *Agrobacterium* mediated transformation.

In any of the preceding preferred methods or any other embodiments of methods of the present invention, the at least one agronomic characteristic is preferably selected from the group consisting of greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen uptake, nitrogen stress tolerance, root lodging, stalk lodging, plant height, ear length, stalk lodging and harvest index. Yield, greenness, biomass and root lodging are particularly preferred agronomic characteristics for alteration (preferably an increase).

In any of the preceding preferred methods or any other embodiments of methods of the present invention, the plant preferably exhibits the alteration of at least one agronomic characteristic irrespective of the environmental conditions when compared to a control.

The introduction of recombinant DNA constructs of the present invention into plants may be carried out by any suitable technique, including but not limited to direct DNA uptake, chemical treatment, electroporation, microinjection, cell fusion, infection, vector mediated DNA transfer, bombardment, or *Agrobacterium* mediated transformation.

Preferred techniques are set forth below in the Examples below for transformation of maize plant cells and soybean plant cells.

Other preferred methods for transforming dicots, primarily by use of *Agrobacterium* tumefaciens, and obtaining transgenic plants include those published for cotton (U.S. Patent No. 5,004,863, U.S. Patent No. 5,159,135, U.S. Patent No. 5,518, 908); soybean (U.S. Patent No. 5,569,834, U.S. Patent No. 5,416,011, McCabe et. al., Bio/Technology 6: 923 (1988), Christou et al., Plant Physiol. 87:671 674 (1988)); Brassica (U.S. Patent No. 5,463,174); peanut (Cheng et al., Plant Cell Rep. 15:653 657 (1996), McKently et al., Plant Cell Rep. 14:699 703 (1995)); papaya; and pea (Grant et al., Plant Cell Rep. 15:254 258, (1995)).

Transformation of monocotyledons using electroporation, particle bombardment, and *Agrobacterium* have also been reported and are included as preferred methods, for example, transformation and plant regeneration as achieved in asparagus (Bytebier et al., Proc. Natl. Acad. Sci. U.S.A. 84:5354, (1987)); barley (Wan and Lemaux, Plant Physiol. 104:37 (1994)); Zea mays (Rhodes et al., Science 240:204 (1988), Gordon-Kamm et al., Plant Cell 2:503 618 (1990), Fromm et al., BiolTechnology 8:833 (1990), Koziel et al., Bio/Technology 11:194, (1993), Armstrong et al., Crop Science 35:550-557 (1995)); oat (Somers et al., Bio/Technology 10:1589 (1992)); orchard grass (Horn et al., Plant Cell Rep. 7: 469 (1988)); rice (Toriyama et al., Theor. Appl. Genet. 205:34, (1986); Part et al., Plant Mol. Biol. 32:1135 1148, (1996); Abedinia et al., Aust. J. Plant Physiol. 24:133 141 (1997); Zhang and Wu, Theor. Appl. Genet. 78:835 (1988); Zhang et al., Plant Cell Rep. 7:379, (1988); Battraw and Hall, Plant Sci. 86:191 202 (1992); Christou et al., BiolTechnology 9:957 (1991)); rye (De la Pena et al., Nature 325:274 (1987)); sugarcane (Bower and Birch, Plant J. 2:409 (1992)); tall fescue (Wang et al., BiolTechnology 10:691 (1992)), and wheat (Vasil et al., BiolTechnology 10:667 (1992); U.S. Patent No. 5,631,152).

There are a variety of methods for the regeneration of plants from plant tissue. The particular method of regeneration will depend on the starting plant tissue and the particular plant species to be regenerated.

The regeneration, development, and cultivation of plants from single plant protoplast transformants or from various transformed explants is well known in the art (Weissbach and Weissbach, In: Methods for Plant Molecular Biology, (Eds.), Academic Press, Inc. San Diego, CA, (1988)). This regeneration and growth process typically includes the steps of selection of transformed cells, culturing those individualized cells through the usual stages of embryonic development through the rooted plantlet stage. Transgenic embryos and seeds are similarly regenerated. The resulting transgenic rooted shoots are thereafter planted in an appropriate plant growth medium such as soil.

The development or regeneration of plants containing the foreign, exogenous isolated nucleic acid fragment that encodes a protein of interest is well known in the art. Preferably, the regenerated plants are self-pollinated to provide homozygous transgenic plants. Otherwise, pollen obtained from the regenerated plants is crossed to seed-grown plants of agronomically important lines. Conversely, pollen from plants of these important lines is used to pollinate regenerated plants. A transgenic plant of the present invention containing a desired polypeptide is cultivated using methods well known to one skilled in the art.

## EXAMPLES

The present invention is further illustrated in the following Examples, in which parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions. Thus, various modifications of the invention in addition to those shown and described herein will be apparent to those skilled in the art from the foregoing description. Such modifications are also intended to fall within the scope of the appended claims.

## EXAMPLE 1

### Creation of an *Arabidopsis* Population with Activation-Tagged Genes

A 18.4kb T-DNA based binary construct was created, pHSbarENDs (Fig.1; SEQ ID NO:1;) containing four multimerized enhancer elements derived from the Cauliflower Mosaic Virus 35S promoter, corresponding to sequences -341 to -64, as defined by Odell et al. (1985) Nature 313:810-812. The construct also contains vector sequences (pUC9) to allow plasmid rescue, transposon sequences (Ds) to remobilize the T-DNA, and the bar gene to allow for glufosinate selection of transgenic plants. Only the 10.8kb segment from the right border (RB) to left border (LB) inclusive will be transferred into the host plant genome. Since the enhancer elements are located near the RB, they can induce cis-activation of genomic loci following T-DNA integration.

The pHSbarENDs construct was transformed into *Agrobacterium tumefaciens* strain C58, grown in LB at 25°C to OD600 ~1.0. Cells were then pelleted by centrifugation and resuspended in an equal volume of 5% sucrose/0.05% Silwet L-77 (OSI Specialties, Inc). At early bolting, soil grown *Arabidopsis thaliana* ecotype Col-0 were top watered with the *Agrobacterium* suspension. A week later, the same plants were top watered again with the same *Agrobacterium* strain in sucrose/Silwet. The plants were then allowed to set seed as normal. The resulting $T_1$ seed were sown on soil, and transgenic seedlings were selected by spraying with glufosinate (Finale®; AgrEvo; Bayer Environmental Science). $T_2$ seed was collected from approximately 35,000 individual glufosinate resistant $T_1$ plants. $T_2$ plants were grown and equal volumes of $T_3$ seed from 96 separate $T_2$ lines were pooled. This constituted 360 sub-populations.
The *Agrobacterium* strain and whole plant transformation was performed as described above.
A total of 100,000 glufosinate resistant $T_1$ seedlings were selected. $T_2$ seed from each line was kept separate.

## EXAMPLE 2A

### Screens to Identify Lines with Altered Root Architecture (non-limiting Nitrogen conditions)

Activation-tagged *Arabidopsis* seedlings, grown under non-limiting nitrogen conditions, can be analyzed for altered root system architecture when compared to control seedlings during early development from the population described in Example 1.

From each of 96,000 separate T1 activation-tagged lines, ten T2 seeds can be sterilized with chlorine gas and planted on petri plates containing the following medium: 0.5x N-Free Hoagland's, 60 mM $KNO_3$, 0.1% sucrose, 1 mM MES and 1% Phytagel™. Typically 10 plates are placed in a rack. Plates are kept for three days at 4°C to stratify seeds and then held vertically for 11 days at 22° C light and 20° C dark. Photoperiod is 10 h; 8 h dark, average light intensity was ~180 $\mu$mol/m$^2$/s. Racks (typically holding 10 plates each) are rotated daily within each shelf. At day 14, plates are evaluated for seedling status, whole plate digital images were taken, and analyzed for root area. Plates are arbitrarily divided in 10 horizontal areas. The root area in each of 10 horizontal zones on the plate is expressed as a percentage of the total area. Only areas in zones 3 to 9 are used to calculate the total root area of the line. Rootbot image analysis tool (proprietary) developed by ICORIA can be used to assess root area. Total root area is expressed in mm$^2$.

Lines with enhanced root growth characteristics are expected to lie at the upper extreme of the root area distributions. A sliding window approach can be used to estimate the variance in root area for a given rack with the assumption that there could be up to two outliers in the rack. Environmental variations in various factors including growth media, temperature, and humidity can cause significant variation in root growth, especially between sow dates. Therefore the lines are grouped by sow date and shelf for the data analysis. The racks in a particular sow date/shelf group are then sorted by mean root area. Root area distributions for sliding windows is performed by combining data for a rack, $r_i$, with data from the rack with the next lowest, ($r_{i-1}$, and the next highest mean root area, $r_{i+1}$. The variance of the combined distribution is then analyzed to identify outliers in $r_i$ using a Grubbs-type approach (Barnett et al., Outliers in Statistical Data, John Wiley & Sons, 3rd edition (1994).

Lines with significant enhanced root growth as determined by the method outlined above, are designated as Phase 1 hits. Phase 1 hits are re-screened in duplicate under the same assay conditions. When either or both of the Phase 2 replicates shows a significant difference from the mean, the line is then considered a validated root architecture line.

Those lines that were again found to be outliers in at least one plate in Phase 2 were subjected to a Phase 3 screening performed *in house,* to validate the results obtained in Phase 1 and Phase 2. The results were validated in Phase 3 using both the Rootboot image analysis (as described above) and WinRHIZO® as described below. The confirmation was performed in the same fashion as in the first round of screening. T2 seeds were sterilized using 50% household bleach .01% triton X-100 solution and plated onto the same plate medium as described in the first round of screening at a density of 10 seeds/plate. Plates were kept for three days at 4°C to stratify seeds, and grown in the same temperature and photoperiod as the first experiment with the light intensity ~160 $\mu mol/m^2/s$. Plates were placed vertically into the eight center positions of a 10 plate rack with the first and last position holding blank plates. The racks and the plates within a rack were rotated every other day. Two sets of pictures were taken for each plate. The first set taking place at day 14 - 16 when the primary roots for most lines had reached the bottom of the plate, the second set of pictures two days later after more lateral roots had developed. The latter set of picture was usually used for data analysis. These seedlings grown on vertical plates were analyzed for root growth with the software WinRHIZO® (Regent Instruments Inc), an image analysis system specifically designed for root measurement. WinRHIZO® uses the contrast in pixels to distinguish the light root from the darker background. To identify the maximum amount of roots without picking up background, the pixel classification was 150 - 170 and the filter feature was used to remove objects that have a length/ width ratio less then 10.0. The area on the plates analyzed was from the edge of the plant's leaves to about 1 cm from the bottom of the plate. The exact same WinRHIZO® settings and area of analysis were used to analyze all plates within a batch. The total root length score given by WinRHIZO® for a plate was divided by the number of plants that had germinated and had grown halfway down the plate. Three plates for every line were grown and their scores were averaged. This average was then compared to the average of three plates containing wild type seeds that were grown at the same time.

*Arabidopsis* activation tagged lines re-confirmed by having a higher value of root growth compared to wild type were then used for the molecular identification of the DNA flanking the T-DNA insertion.

Example 2B

Identification of Mutant Lines with an Altered Root Phenotype in a Mutant Population (limiting Nitrogen conditions)

A two-step screening procedure can be used, comprising:

(1) Identification of an altered root growth phenotype in a vertical plate assay;
(2) Confirm herbicide resistance and root phenotype in rescued mutant lines;

The primary screen is based on vertical plates containing Nitrogen-free Hoagland salts, 0.3% sucrose and 1 mM $KNO_3$. The media also contains 0.8% - 1.0% PhytaGel as a gelling agent. Media with Phytagel at 1.0% is sometimes difficult to pour as it solidifies quickly, however, at below 0.8% the media will slide off plates when placed vertically. Mutants from an activation-tagged population where pools of 100 lines each are available for a total of 36000 lines are being screened. On each plate, 12 mutant and 2 wild type Columbia seeds are seeded. Plates are placed in a growth room with a constant temperature of 26°C, 16hr-day cycle with an average of 110 $\mu E/m^2s$ light intensity at the top of the plates. These plates are photographed 3 - 4 times in a 2.5 week time frame. Individual seedlings are rescued when a clear root phenotype is observed. Rescued seedlings are grown to maturity in a growth chamber (24°c, 16 hr day, 250-300 $\mu E/m^2s$) for seed collection.

For the secondary screening, seeds from putative hits identified in the primary screen are sowed on plates containing the same media as above plus 6 mg/L bialaphos. Wild type Columbia seeds are sown at the same time on the same media but without bialaphos. Each plate has 10 seeds. There are 3 plates for each mutant line, and 2 plates for wild type Columbia, as replication. These plates are placed under the same growth conditions as described above in a growth room. Those lines that do not have herbicide resistance or no obvious root phenotype are discarded as false positives. Lines validated by the second screen are saved for further study.

EXAMPLE 3

Identification of Activation-Tagged Genes

Genes flanking the T-DNA insert in lines with altered root architecture are identified using one, or both, of the following two standard procedures: (1) thermal asymmetric interlaced (TAIL) PCR (Liu et al., (1995), Plant J. 8:457-63); and (2) SAIFF PCR (Siebert et al., (1995) Nucleic Acids Res. 23:1087-1088). In lines with complex multimerized T-DNA inserts, TAIL PCR and SAIFF PCR may both prove insufficient to identify candidate genes. In these cases, other procedures, including inverse PCR, plasmid rescue and/or genomic library construction, can be employed.
A successful result is one where a single TAIL or SAIFF PCR fragment contains a T-DNA border sequence and *Arabidopsis* genomic sequence.
Once a tag of genomic sequence flanking a T-DNA insert is obtained, candidate genes are identified by alignment to publicly available *Arabidopsis* genome sequence.
Specifically, the annotated gene nearest the 35S enhancer elements/T-DNA RB are candidates for genes that are activated.
To verify that an identified gene is truly near a T-DNA and to rule out the possibility that the TAIL/SAIFF fragment is a chimeric cloning artifact, a diagnostic PCR on genomic DNA is done with one oligo in the T-DNA and one oligo specific for the candidate gene. Genomic DNA samples that give a PCR product are interpreted as representing a T-DNA insertion. This analysis also verifies a situation in which more than one insertion event occurs in the same line, e.g., if multiple differing genomic fragments are identified in TAIL and/or SAIFF PCR analyses.

EXAMPLE 4

Identification of Activation -Tagged ndk Gene

The ndk gene was obtained by the screening procedure as described in Example 2B and subsequently subjected to a phase 3 (in house) screening as described in Example 2A. Identification of the activation-tagged gene was performed as described in Example 3.
One line (1-6) displaying altered root architecture was further analyzed. DNA from the line was extracted and the T-DNA insertion was found by ligation mediated PCR (Siebert et al., (1995) Nucleic Acids Res. 23:1087-1088) using primers within the LeftBorder of the T-DNA. Once a tag of genomic sequence flanking a T-DNA insert was obtained, the candidate gene was identified by sequence alignment to the completed *Arabidopsis* genome. One of the insertion sites identified was identified as a chimeric insertion; Left Border T-DNA sequence was determined to be at both ends of the T-DNA insertion. It is still possible that the enhancer elements located near the Right Border of the T-DNA are close enough to have an effect on the nearby candidate gene. In this case the location of the Right Border was assumed to be present at the insertion site, and the two genes that flank the insertion site were chosen as candidates. One of the genes nearest the 35S enhancers of the chimeric insertion was AT4G23900 (SEQ ID NO:50; NCBI GI NO:11990430; *Arabidopsis thaliana* nucleoside diphosphate kinase 4), encoding the NDK4 protein (SEQ ID NO.51), referred herein as nucleoside diphosphate kinase or NDK.

**EXAMPLE 5A**

**Validation of a Candidate *Arabidopsis* Gene (AT4G23900) for its ability to enhance root architecture in plants via Transformation into *Arabidopsis***

Candidate genes can be transformed into *Arabidopsis* and overexpressed under the 35S promoter. If the same or similar phenotype is observed in the transgenic line as in the parent activation-tagged line, then the candidate gene is considered to be a validated "lead gene" in *Arabidopsis.*
The *Arabidopsis* AT4G23900 Gene can be directly tested for its ability to enhance Root Architecture in *Arabidopsis.*
The *Arabidopsis* AT4G23900 cDNA was PCR amplified with oligos that introduce the attB1 (SEQ ID NO:51) sequence, a consensus start sequence (CAACA) upstream of the ATG start codon and the first 23 nucleotides of the protein coding-region of the AT4G23900 cDNA (nucleotides 51-764 (Stop) of SEQ ID NO:50) and the attB2 (SEQ ID NO:53) sequence and the last 21 nucleotides of the protein-coding region including the stop codon of said cDNA. Using Invitrogen™ Gateway® technology a MultiSite Gateway® BP Recombination Reaction was performed with pDONR™/Zeo (Invitrogen™, Fig. 2; SEQ ID NO:2). This process removes the bacteria lethal ccdB gene, as well as the chloramphenicol resistance gene (CAM) from pDONR™/Zeo and directionally clones the PCR product with flanking attB1 (SEQ ID NO:52) and attB2 (SEQ ID NO:53) sites creating entry clone PHP28731.
A 16.8-kb T-DNA based binary vector, called pBC-yellow (Fig. 4, SEQ ID NO:4), was constructed with the 1.3-kb 35S promoter immediately upstream of the Invitrogen™ Gateway® C1 conversion insert containing the ccdB gene and the chloramphenicol resistance gene (CAM) flanked by attR1 and attR2 sequences. The vector also contains a YFP marker under the control of the Rd29a promoter for the selection of transformed seeds.

Using Invitrogen ™ Gateway® technology a MultiSite Gateway® LR Recombination Reaction was performed on the entry clone containing the directionally cloned PCR product and pBC-yellow. This allowed rapid and directional cloning of the AT4G23900 gene behind the 35S promoter in pBC-yellow.

The 35S- AT4G23900 gene construct was introduced into wild-type *Arabidopsis* ecotype Col-0, using the same Agrobacterium-mediated transformation procedure described in Example 1.

Transgenic T1 seeds were selected by the presence of the fluorescent YFP marker. Fluorescent seeds were subjected to the Root Architecture Assay following the procedure described in Example 2A. Transgenic T1 seeds were re-screened using 6 plates per construct. Two plates per rack containing non-transformed Columbia seed discarded from fluorescent seed sorting served as a control.

Six plates per construct were analyzed statistically and a trend was detected between the number of plants growing on a plate and their average WinRHIZO® score. WinRHIZO® scores were normalized for this trend and the root score corresponding to the construct was divided by the wild-type root score.

## EXAMPLE 5B

### Screen of Candidate Genes under Nitrogen Limiting Conditions

Transgenic T1 seed selected by the presence of the fluorescent marker YFP as described above in Example 5A can also be screened for their tolerance to grow under nitrogen limiting conditions. For this purpose 32 transgenic individuals can be grown next to 32 wild-type individuals on one plate with either 0.4mM $KNO_3$ or 60mM $KNO_3$. If a line shows a statistically significant difference from the controls, the line is considered a validated nitrogen-deficiency tolerant line. After masking the plate image to remove background color, two different measurements are collected for each individual: total rosetta area, and the percentage of color that falls into a green color bin. Using hue, saturation and intensity data (HIS), the green color bin consists of hues 50-66. Total rosetta area is used as a measure of plant biomass, whereas the green color bin has been shown by dose-response studies to be an indicator of nitrogen assimilation.

## EXAMPLE 5C

### Validation of a Candidate *Arabidopsis* Gene AT4G23900 for its ability to improve nitrogen utilization in plants via Transformation into Arabidopsis

Transgenic seeds were screened for their ability to grow under nitrogen limiting conditions as described in Example 5B.

Plants were evaluated at 10, 11, 12 and 13 days. Transgenic individuals expressing the Arabidopsis Candidate gene (AT4G23900) scored better under nitrogen limiting conditions compared to the wild type, however, they did not validate as nitrogen-deficient tolerant compared to the wild type plants, when grown on media containing limiting concentrations of nitrogen (0.4 mM $KNO_3$). No difference was observed between the transgenic and wild type plants under non-limiting nitrogen conditions (60mM $KNO_3$).

## EXAMPLE 5D

### Screen to Identify Lines with Improved Nitrate Uptake

For each overexpressor line, twelve T2 plants are sown on 96 well micro titer plates containing 2 mM $MgSO_4$, 0.5 mM $KH_2PO_4$, 1 mM $CaCl_2$, 2.5 mM KCl, 0.15 mM Sprint 330, 0.06 mM $FeSO_4$, 1 $\mu$M $MnCl_2$ $4H_2O$, 1 $\mu$M $ZnSO_4$ $7H_2O$, 3 $\mu$M $H_3BO_3$, 0.1 $\mu$M $NaMoO_4$, 0.1 $\mu$M $CuSO_4$ $5H_2O$, 0.8 mM potassium nitrate, 0.1 % sucrose, 1 mM MES, 200 $\mu$M bromophenol red and 0.40 % Phytagel™ (pH assay medium). The pH of the medium is so that the color of bromophenol is red, the pH indicator dye, is yellow.

Four lines are plated per plate, and the inclusion of 12 wild-type individuals and 12 individuals from a line that has shown an improvement in nitrate uptake (positive control) on each plate makes for a total of 72 individuals on each 96 well micro titer plate A web-based random sequence generator can be used to determine the order of the lines on each plate. Seeds are not plated in Row A or Row H on the 96 well micro titer plate. Four plates are plated for each experiment, resulting in a maximum of 48 plants per line analyzed. Plates are kept for three days in the dark at 4 °C to stratify seeds, and then placed horizontally for six days at 22 °C light and dark. Photoperiod is sixteen hours light; eight hours dark, with an average light intensity of ~200 mmol/$m^2$/s. Plates are rotated and shuffled within each shelf. At day eight or nine (five or six days of growth), seedling status is evaluated by recording the color of the medium as pink, peach, yellow or no germination. Then the plants and/or seeds are removed from each well. Each medium plug is transferred to 1.2 ml micro titer tubes and placed in the corresponding well in a 96 well deep micro titer plate. An equal volume of water containing 2 μM flourescein is added to each 1.2 ml micro titer tube. The plate is covered with foil and autoclaved on liquid cycle. Each tube is mixed well, and an aliquot is removed from each tube and analyzed for amount of nitrate remaining in the medium. If t-test shows that a line is significantly different ($p<0.05$) from wild-type control, the line is then considered a validated improved nitrate uptake line.

**EXAMPLE 5E**

**Validation of increased nitrate uptake by transgenic lines containing the Candidate *Arabidopsis* Gene (AT4G23900).**

Transgenic seeds were screened for increased nitrate uptake as described in Example 5D.

Transgenic individuals overexpressing the Arabidopsis Candidate gene (AT4G23900) validated as an improved nitrate uptake line compared to wild type plants not overexpressing the Arabidopsis candidate gene.

**EXAMPLE 6**

**Composition of cDNA Libraries: Isolation and Sequencing of cDNA Clones**

cDNA libraries representing mRNAs from various tissues of *Canna edulis* (Canna), *Momordica charantia* (balsam pear), *Brassica* (mustard), *Cyamopsis tetragonoloba* (guar), *Zea mays* (maize), *Oryza sativa* (rice), *Glycine max* (soybean), *Helianthus annuus* (sunflower) and *Triticum aestivum* (wheat) were prepared. The characteristics of the libraries are described below.

**TABLE 2**

| cDNA Libraries from Canna, Balsam Pear, Mustard, Guar, Maize, Rice, Soybean, Sunflower and Wheat | | |
|---|---|---|
| Library | Tissue | Clone |
| cur1f | Corn (Zea mays, B73) developing root (full length) | cdr1f.pk002.a3.f:fis |
| cest1s | Maize, stalk, elongation zone. | cest1s.pk013.p8:fis |
| cfp2n | Maize Silk pollinated and unpollinated, pooled, full-length enriched, normalized. | cfpn.pk070.b22;fis<br>cfpn.pk069.c16 |
| sfl1 | Soybean immature flower. | sft1.pk126.p10<br>sfl1.pk134.d19:fis<br>sfl1.pk133.14:fis |
| p0095 | Ear leaf sheath, screened 1 Growth conditions: field; control or untreated tissues Growth stage: 2-3 weeks after pollen shed; plants were allowed to pollinate naturally | p0095.cwsab57ra |
| rlr24 | Rice leaf 15 days after germination, 24 hrs after infection of strain *Magnaporthe grisea* 4360-R-62 (AVR2-YAMO); resistant. | rlr24.pk0071.e7 |
| hss1c | Sclerotinia infected sunflower plants. | hss1c.pk019.a16:fis |
| Ids1c | Guar (*Cyamopsis tetragonoloba*) seeds harvested at 15 DAF. | Ids1c.pk004.f12 |
| epc2c | Frac, 10 and 11 Psyllium seed coats containing 70% water soluble arabinoxylans, major cell wall constituent in maize. | epc2c.pk003.I14:fis |
| egh1c | Upland cotton *(Gossypium hirsutum)* germinating seeds. | egh1c.pk002.I114 |

(continued)

| cDNA Libraries from Canna, Balsam Pear, Mustard, Guar, Maize, Rice, Soybean, Sunflower and Wheat | | |
|---|---|---|
| Library | Tissue | Clone |
| ort1f | Oat *(Avena strigosa)* full length oat root tip. | ort1f.pk020.a13:fis |

cDNA libraries may be prepared by any one of many methods available. For example, the cDNAs may be introduced into plasmid vectors by first preparing the cDNA libraries in Uni-ZAP™ XR vectors according to the manufacturer's protocol (Stratagene Cloning Systems, La Jolla, CA). The Uni-ZAP™ XR libraries are converted into plasmid libraries according to the protocol provided by Stratagene. Upon conversion, cDNA inserts will be contained in the plasmid vector pBluescript. In addition, the cDNAs may be introduced directly into precut Bluescript II SK(+) vectors (Stratagene) using T4 DNA ligase (New England Biolabs), followed by transfection into DH10B cells according to the manufacturer's protocol (GIBCO BRL Products). Once the cDNA inserts are in plasmid vectors, plasmid DNAs are prepared from randomly picked bacterial colonies containing recombinant pBluescript plasmids, or the insert cDNA sequences are amplified via polymerase chain reaction using primers specific for vector sequences flanking the inserted cDNA sequences. Amplified insert DNAs or plasmid DNAs are sequenced in dye-primer sequencing reactions to generate partial cDNA sequences (expressed sequence tags or "ESTs"; see Adams et al., (1991) Science 252:1651-1656). The resulting ESTs are analyzed using a Perkin Elmer Model 377 fluorescent sequencer.

Full-insert sequence (FIS) data is generated utilizing a modified transposition protocol. Clones identified for FIS are recovered from archived glycerol stocks as single colonies, and plasmid DNAs are isolated via alkaline lysis. Isolated DNA templates are reacted with vector primed M13 forward and reverse oligonucleotides in a PCR-based sequencing reaction and loaded onto automated sequencers. Confirmation of clone identification is performed by sequence alignment to the original EST sequence from which the FIS request is made.

Confirmed templates are transposed via the Primer Island transposition kit (PE Applied Biosystems, Foster City, CA) which is based upon the *Saccharomyces cerevisiae* Ty1 transposable element (Devine and Boeke (1994) Nucleic Acids Res. 22:3765-3772). The *in vitro* transposition system places unique binding sites randomly throughout a population of large DNA molecules. The transposed DNA is then used to transform DH10B electro-competent cells (Gibco BRL/Life Technologies, Rockville, MD) via electroporation. The transposable element contains an additional selectable marker (named DHFR; Fling and Richards (1983) Nucleic Acids Res. 11:5147-5158), allowing for dual selection on agar plates of only those subclones containing the integrated transpason. Multiple subclones are randomly selected from each transposition reaction, plasmid DNAs are prepared via alkaline lysis, and templates are sequenced (ABI Prism dye-terminator ReadyReaction mix) outward from the transposition event site, utilizing unique primers specific to the binding sites within the transposon.

Sequence data is collected (ABI Prism Collections) and assembled using Phred and Phrap (Ewing et al. (1998) Genome Res. 8:175-185; Ewing and Green (1998) Genome Res. 8:186-194). Phred is a public domain software program which re-reads the ABI sequence data, re-calls the bases, assigns quality values, and writes the base calls and quality values into editable output files. The Phrap sequence assembly program uses these quality values to increase the accuracy of the assembled sequence contigs. Assemblies are viewed by the Consed sequence editor (Gordon et al. (1998) Genome Res. 8:195-202).

In some of the clones the cDNA fragment corresponds to a portion of the 3'-terminus of the gene and does not cover the entire open reading frame. In order to obtain the upstream information one of two different protocols are used. The first of these methods results in the production of a fragment of DNA containing a portion of the desired gene sequence while the second method results in the production of a fragment containing the entire open reading frame. Both of these methods use two rounds of PCR amplification to obtain fragments from one or more libraries. The libraries some times are chosen based on previous knowledge that the specific gene should be found in a certain tissue and some times are randomly-chosen. Reactions to obtain the same gene may be performed on several libraries in parallel or on a pool of libraries. Library pools are normally prepared using from 3 to 5 different libraries and normalized to a uniform dilution. In the first round of amplification both methods use a vector-specific (forward) primer corresponding to a portion of the vector located at the 5'-terminus of the clone coupled with a gene-specific (reverse) primer. The first method uses a sequence that is complementary to a portion of the already known gene sequence while the second method uses a gene-specific primer complementary to a portion of the 3'-untranslated region (also referred to as UTR). In the second round of amplification a nested set of primers is used for both methods. The resulting DNA fragment is ligated into a pBluescript vector using a commercial kit and following the manufacturer's protocol. This kit is selected from many available from several vendors including Invitrogen™ (Carlsbad, CA), Promega Biotech (Madison, WI), and Gibco-BRL (Gaithersburg, MD). The plasmid DNA is isolated by alkaline lysis method and submitted for sequencing and assembly using Phred/Phrap, as above.

## EXAMPLE 7

### Identification of cDNA Clones

cDNA clones encoding NDK-like polypeptides were identified by conducting BLAST (Basic Local Alignment Search Tool; Altschul et al. (1993) J. Mol. Biol. 215:403-410; see also the explanation of the BLAST algorithm on the world wide web site for the National Center for Biotechnology Information at the National Library of Medicine of the National Institutes of Health) searches for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences obtained as described in Example 6 were analyzed for similarity to all publicly available DNA sequences contained in the "nr" database using the BLASTN algorithm provided by the National Center for Biotechnology Information (NCBI). The DNA sequences were translated in all reading frames and compared for similarity to all publicly available protein sequences contained in the "nr" database using the BLASTX algorithm (Gish and States (1993) Nat. Genet. 3: 266-272) provided by the NCBI. For convenience, the P-value (probability) of observing a match of a cDNA sequence to a sequence contained in the searched databases merely by chance as calculated by BLAST are reported herein as "pLog" values, which represent the negative of the logarithm of the reported P-value. Accordingly, the greater the pLog value, the greater the likelihood that the cDNA sequence and the BLAST "hit" represent homologous proteins.

ESTs submitted for analysis are compared to the Genbank database as described above. ESTs that contain sequences more 5- or 3-prime can be found by using the BLASTn algorithm (Altschul et al (1997) Nucleic Acids Res. 25:3389-3402.) against the Du Pont proprietary database comparing nucleotide sequences that share common or overlapping regions of sequence homology. Where common or overlapping sequences exist between two or more nucleic acid fragments, the sequences can be assembled into a single contiguous nucleotide sequence, thus extending the original fragment in either the 5 or 3 prime direction. Once the most 5-prime EST is identified, its complete sequence can be determined by Full Insert Sequencing as described in Example 6. Homologous genes belonging to different species can be found by comparing the amino acid sequence of a known gene (from either a proprietary source or a public database) against an EST database using the tBLASTn algorithm. The tBLASTn algorithm searches an amino acid query against a nucleotide database that is translated in all 6 reading frames. This search allows for differences in nucleotide codon usage between different species, and for codon degeneracy.

## EXAMPLE 8

### Characterization of cDNA Clones Encoding NDK-like Polypeptides

The BLASTX search using the EST sequences from clones listed in Table 1 revealed similarity of the polypeptides encoded by the cDNAs to NDK-like polypeptides from *rice* (GI No. 115465831, and 125595441 corresponding to SEQ ID NO's :44, and 49, respectively), *Arabidopsis a* (GI No. 15237018 corresponding to SEQ ID NO:46), pea (GI No. 6435320 corresponding to SEQ ID NO:45), grapevine (GI No. 147884944 corresponding to SEQ ID NO:47), and pennycress (GI No. 62870979 corresponding to SEQ ID NO:48) Shown in Table 3 are the BLAST results for individual ESTs ("EST"), the sequences of the entire cDNA inserts comprising the indicated cDNA clones ("FIS"), the sequences of contigs assembled from two or more EST, FIS or PCR sequences ("Contig"), or sequences encoding an entire or functional protein derived from an FIS or a contig ("CGS"):

**TABLE 3**

| BLAST Results and Percent Identity for Sequences Encoding Polypeptides Homologous to NDK-like Polypeptides | | | | |
|---|---|---|---|---|
| Sequence | Status | NCBI GI No. | BLAST pLog Score | % identity |
| cest1s.pk013.p8:fis SEQ ID NO:14 | CGS | 115465831 (Rice) SEQ ID NO:44 | 90 | 73.9 |
| cfp2n.pk070.b22;fis SEQ ID NO:16 | CGS | 115465831 (Rice) SEQ ID NO:44 | 90 | 73.9 |
| cdr1f.pk002.a3.f:fis SEQ ID NO:18 | CGS | 115465831 (Rice) SEQ ID NO:44 | 90 | 78.2 |
| Ids1c.pk004.f12:fis SEQ ID NO:20 | CGS | 6435320(Pea) SEQ ID NO:45 | 94 | 83.3 |

(continued)

| BLAST Results and Percent Identity for Sequences Encoding Polypeptides Homologous to NDK-like Polypeptides | | | | |
|---|---|---|---|---|
| Sequence | Status | NCBI GI No. | BLAST pLog Score | % identity |
| ep2c.pk002.f17.f:fis SEQ ID NO:22 | CGS | 15237018 (Arabidopsis) SEQ ID NO:46 | 89 | 76.8 |
| ort1f.pk020.a13:fis SEQ ID NO:24 | CGS | 115465831 (Rice) SEQ ID NO:44 | 100 | 79.8 |
| hss1c.pk019.a16:fis SEQ ID NO:26 | CGS | 147864944 (Grapevine) SEQ ID NO:47 | 91 | 77.9 |
| sfl1.pk134.d19:fis SEQ ID NO:28 | CGS | 6435320 (Pea) SECT ID NO:45 | 89 | 83.3 |
| egh1c.pk002.I14:fis SEQ ID NO:30 | CGS | 62870979 (Alpine Pennycress) SEQ ID NO: 48 | 93 | 79.8 |
| sfl1.pk133.14:fis SEQ ID NO:32 | CGS | 15237018 (Arabidopsis) SEQ ID NO:46 | 91 | 77.1 |
| p0095.cwsab57ra | CGS | 125595441 | 78 | 90.8 |
| SEQ ID NO:36 | | (Rice) SEQ ID NO:49 | | |
| sfl1.pk126.p10 SEQ ID NO:38 | EST | 15237018 (Arabidopsis) SEQ ID NO:46 | 47 | 66.3 |
| rlr24.pk0071.e7 SEQ ID NO:40 | EST | 115465831 (Rice) SEQ ID NO:44 | <10 | 64.5 |
| cfp2n.pk069.c16 SEQ ID NO:42 | EST | 115465831 (Rice) SEQ ID NO:44 | 46 | 67.1 |

Figs.15A -15K show the multiple alignment of the full length amino acid sequences of SEQ ID NOs: 15, 17, 19, 21, 23, 25, 27, 29, 31, 33, 35, and 37, and SEQ ID NOs:44, 45, 46, 47, 48, 49 and 51 Figure 16 presents the percent sequence identities and divergence values for each sequence pair presented in Figures 15A-15K.

Sequence alignments and percent identity calculations were performed using the Megalign program of the LASERGENE bioinformatics computing suite (DNASTAR Inc., Madison, WI). Multiple alignment of the sequences was performed using the Clustal method of alignment (Higgins and Sharp (1989) CABIOS. 5:151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5.

Sequence alignments and BLAST scores and probabilities indicate that the nucleic acid fragments comprising the instant cDNA clones encode NDK-like polypeptides.

**TABLE 4**

| BLAST Results for Sequences Encoding Polypeptides Homologous to NDK and NDK-like polypeptides | | | | |
|---|---|---|---|---|
| Sequence | Status | Reference | Blast pLog Score | % identity |
| cest1s.pk013.p8:fis SEQ ID NO:14 | CGS | ·SEQ ID NO: 42932 in EP1033405-A2 | 121 | 99.6 |
| cfp2n.pk070.b22;fis SEQ ID NO:16 | CGS | SEQ ID NO: 42932 in EP1033405-A2 | 121 | 99.6 |
| cdr1f.pk002.a3.f:fis SEQ ID NO:18 | CGS | SEQ ID NO: 43288 in EP1033405-A2 | 121 | 99.2 |
| Ids1c.pk004.f12:fis SEQ ID NO:20 | CGS | SEQ ID NO: 13361 in US2005108791 | 95 | 89.1 |

(continued)

| BLAST Results for Sequences Encoding Polypeptides Homologous to NDK and NDK-like polypeptides | | | | |
|---|---|---|---|---|
| Sequence | Status | Reference | Blast pLog Score | % identity |
| ep2c.pk002.f17.f:fis SEQ ID NO:22 | CGS | SEQ ID NO: 2692 in WO2004035798 | 90 | 82.6 |
| ort1f.pk020.a13:fis SEQ ID NO:24 | CGS | SEQ ID NO:137093 in US2007044171 | 105 | 75.6 |
| hss1c.pk019.a16:fis SEQ ID NO:26 | CGS | SEQ ID NO:64801 in US2007061916 | 93 | 97.4 |
| sfl1.pk134.d19:fis SEQ ID NO:28 | CGS | SEQ ID NO: 13364 in US2005108791 | 101 | 98.7 |
| egh1c.pk002.I14:fis SEQ ID NO:30 | CGS | SEQ ID NO: 64801 in US200700196 | 115 | 100 |
| sfl1.pk133.14:fis SEQ ID NO: 32 | CGS | SEQ ID NO:13364 in US2005108791 | 101 | 90.7 |
| my.p0031.ccmbo48 SEQ ID NO:34 | CGS | SEQ ID NO:43288 in EP1033405 | 122 | 99.6 |
| p0095.cwsab57ra SEQ ID NO:36 | EST | SEQ ID NO:42932 in EP1033405 | 85 | 99.3 |
| sfl1.pk126.p10 SEQ ID NO: 38 | EST | SEQ ID NO:13364 in US2005108791 | 54 | 80.2 |
| rlr24.pk0071.e7 SEQ ID NO: 40 | EST | SEQ ID NO:13369 in US2005108791 | 24 | 53.9 |
| cfp2n.pk069.c16 SEQ ID NO: 42 | EST | SEQ ID NO:43288 in EP1033405 | 68 | 94.4 |

## EXAMPLE 9

### Preparation of a Plant Expression Vector Containing a Homolog of the *Arabidopsis* Lead Gene (AT4G23900)

Sequences homologous to the lead ndk gene can be identified using sequence comparison algorithms such as BLAST (Basic Local Alignment Search Tool; Altschul et al., J. Mol. Biol. 215:403-410 (1993); see also the explanation of the BLAST algorithm on the world wide web site for the National Center for Biotechnology Information at the National Library of Medicine of the National Institutes of Health). Homologous ndk-like sequences, such as the ones described in Example 8, can be PCR-amplified by either of the following methods.

Method 1 (RNA-based): If the 5' and 3' sequence information for the protein-coding region of a ndk homolog is available, gene-specific primers can be designed as outlined in Example 5A. RT-PCR can be used with plant RNA to obtain a nucleic acid fragment containing the ndk protein-coding region flanked by attB1 (SEQ ID NO:52) and attB2 (SEQ ID NO:53) sequences. The primer may contain a consensus Kozak sequence (CAACA) upstream of the start codon.

Method 2 (DNA-based): Alternatively, if a cDNA clone is available for a gene encoding a NDK polypeptide homolog, the entire cDNA insert (containing 5' and 3' non-coding regions) can be PCR amplified. Forward and reverse primers can be designed that contain either the attB1 sequence and vector-specific sequence that precedes the cDNA insert or the attB2 sequence and vector-specific sequence that follows the cDNA insert, respectively. For a cDNA insert cloned into the vector pBluescript SK+, the forward primer VC062 (SEQ ID NO:54) and the reverse primer VC063 (SEQ ID NO:55) can be used.

Methods 1 and 2 can be modified according to procedures known by one skilled in the art. For example, the primers of method 1 may contain restriction sites instead of attB1 and attB2 sites, for subsequent cloning of the PCR product into a vector containing attB1 and attB2 sites. Additionally, method 2 can involve amplification from a cDNA clone, a lambda clone, a BAC clone or genomic DNA.

A PCR product obtained by either method above can be combined with the Gateway® donor vector, such as pDONR™/Zeo (Invitrogen™, Fig. 2; SEQ ID NO:2) or pDONR™221 (Invitrogen™, Fig. 3; SEQ ID NO:3) using a BP Recombination Reaction. This process removes the bacteria lethal ccdB gene, as well as the chloramphenicol resistance gene (CAM) from pDONR™221 and directionally clones the PCR product with flanking attB1 and attB2 sites to create an entry clone. Using the Invitrogen™ Gateway® Clonase™ technology, the homologous ndk-like gene from the entry clone can then be transferred to a suitable destination vector to obtain a plant expression vector for use with *Arabidopsis,* corn and soy, such as pBC-Yellow (Fig.4; SEQ ID NO:4), PHP27840 (Fig.5; SEQ ID NO:5) or PHP23236 (Fig.6; SEQ ID NO:6), to obtain a plant expression vector for use with *Arabidopsis,* soybean and corn, respectively.

Alternatively a MultiSite Gateway® LR recombination reaction between multiple entry clones and a suitable destination vector can be performed to create an expression vector. An Example of this procedure is outlined in Example 14A, describing the construction of maize expression vectors for transformation of maize lines.

## EXAMPLE 10

### Preparation of Soybean Expression Vectors and Transformation of Soybean with Validated *Arabidopsis* Lead Genes and homologs thereof

Soybean plants can be transformed to overexpress the validated *Arabidopsis* gene (AT4G23900) and the corresponding homologs from various species in order to examine the resulting phenotype.

The entry clones described in Example 5A and 9 can be used to directionally clone each gene into PHP27840 vector (Fig. 5, SEQ ID NO:5) such that expression of the gene is under control of the SCP1 promoter.

Soybean embryos may then be transformed with the expression vector comprising sequences encoding the instant polypeptides.

To induce somatic embryos, cotyledons, 3-5 mm in length dissected from surface sterilized, immature seeds of the soybean cultivar A2872, can be cultured in the light or dark at 26°C on an appropriate agar medium for 6-10 weeks. Somatic embryos, which produce secondary embryos, are then excised and placed into a suitable liquid medium. After repeated selection for clusters of somatic embryos which multiply as early, globular staged embryos, the suspensions are maintained as described below. Soybean embryogenic suspension cultures can be maintained in 35mL liquid media on a rotary shaker, 150 rpm, at 26 °C with florescent lights on a 16:8 hour day/night schedule. Cultures are subcultured every two weeks by inoculating approximately 35 mg of tissue into 35 mL of liquid medium.

Soybean embryogenic suspension cultures may then be transformed by the method of particle gun bombardment (Klein et al. (1987) Nature (London) 327:70-73, U.S. Patent No. 4,945,050). A DuPont Biolistic™ PDS1000/HE instrument (helium retrofit) can be used for these transformations.

A selectable marker gene which can be used to facilitate soybean transformation is a chimeric gene composed of the 35S promoter from cauliflower mosaic virus (Odell et al. (1985) Nature 373:810-812), the hygromycin phosphotransferase gene from plasmid pJR225 (from *E. coli;* Gritz et al. (1983) Gene 25:179-188) and the 3' region of the nopaline synthase gene from the T-DNA of the Ti plasmid of *Agrobacterium tumefaciens.* Another selectable marker gene which can be used to facilitate soybean transformation is an herbicide-resistant acetolactate synthase (ALS) gene from soybean or *Arabidopsis.* ALS is the first common enzyme in the biosynthesis of the branched-chain amino acids valine, leucine and isoleucine. Mutations in ALS have been identified that convey resistance to some or all of three classes of inhibitors of ALS (US Patent No. 5,013,659; the entire contents of which are herein incorporated by reference). Expression of the herbicide-resistant ALS gene can be under the control of a SAM synthetase promoter (U.S. Patent Application No. US-2003-0226166-A1; the entire contents of which are herein incorporated by reference).

To 50 μL of a 60 mg/mL 1 μm gold particle suspension is added (in order): 5 μL DNA (1 μg/μL), 20 μL spermidine (0.1 M), and 50 μL $CaCl_2$ (2.5 M). The particle preparation is then agitated for three minutes, spun in a microfuge for 10 seconds and the supernatant removed. The DNA-coated particles are then washed once in 400 μL 70% ethanol and resuspended in 40 μL of anhydrous ethanol. The DNA/particle suspension can be sonicated three times for one second each. Five μL of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post bombardment with fresh media containing 50 mg/mL hygromycin. This selective media can be refreshed weekly. Seven to eight weeks post bombardment, green, transformed tissue may be observed growing from untransformed, necrotic embryogenic clusters. Isolated green tissue is removed and inoculated into individual flasks to generate new, clonally propagated, transformed embryogenic suspension cultures. Each new line may be treated as an independent transformation event. These suspensions can then be subcultured and maintained as clusters of immature embryos or regenerated into whole plants by maturation and germination of individual somatic embryos.

Enhanced root architecture can be measured in soybean by growing the plants in soil and wash the roots before analysis of the total root mass with WinRHIZO®.

Soybean plants transformed with validated genes can then be assayed to study agronomic characteristics relative to control or reference plants. For example, nitrogen utilization efficacy, yield enhancement and/or stability under various environmental conditions (e.g. nitrogen limiting conditions, drought etc.).

## EXAMPLE 11

### Transformation of Maize with validated *Arabidopsis* Lead Genes Using Particle Bombardment

Maize plants can be transformed to overexpress a validated *Arabidopsis* lead gene or the corresponding homologs from various species in order to examine the resulting phenotype.

The Gateway® entry clones described in Example 5A can be used to directionally clone each gene into a maize transformation vector. Expression of the gene in maize can be under control of a constitutive promoter such as the maize ubiquitin promoter (Christensen et al., Plant Mol. Biol. 12:619-632 (1989) and Christensen et al., Plant Mol. Biol. 18: 675-689 (1992))

The recombinant DNA construct described above can then be introduced into maize cells by the following procedure. Immature maize embryos can be dissected from developing caryopses derived from crosses of the inbred maize lines H99 and LH132. The embryos are isolated ten to eleven days after pollination when they are 1.0 to 1.5 mm long. The embryos are then placed with the axis-side facing down and in contact with agarose-solidified N6 medium (Chu et al., Sci. Sin. Peking 18:659-668 (1975)). The embryos are kept in the dark at 27°C. Friable embryogenic callus consisting of undifferentiated masses of cells with somatic proembryoids and embryoids borne on suspensor structures proliferates from the scutellum of these immature embryos. The embryogenic callus isolated from the primary explant can be cultured on N6 medium and sub-cultured on this medium every two to three weeks.

The plasmid, p35S/Ac (obtained from Dr. Peter Eckes, Hoechst Ag, Frankfurt, Germany) may be used in transformation experiments in order to provide for a selectable marker. This plasmid contains the pat gene (see European Patent Publication 0 242 236) which encodes phosphinothricin acetyl transferase (PAT). The enzyme PAT confers resistance to herbicidal glutamine synthetase inhibitors such as phosphinothricin, The *pat* gene in p35S/Ac is under the control of the 35S promoter from cauliflower mosaic virus (Odell et al., Nature 313:810-S12 (1985)) and the 3' region of the nopaline synthase gene from the T-DNA of the Ti plasmid of *Agrobacterium tumefaciens.*

The particle bombardment method (Klein et al., Nature 327:70-73 (1987)) may be used to transfer genes to the callus culture cells. According to this method, gold particles (1 μm in diameter) are coated with DNA using the following technique. Ten μg of plasmid DNAs are added to 50 μL of a suspension of gold particles (60 mg per mL). Calcium chloride (50 μL of a 2.5 M solution) and spermidine free base (20 μL of a 1.0 M solution) are added to the particles. The suspension is vortexed during the addition of these solutions. After ten minutes, the tubes are briefly centrifuged (5 sec at 15,000 rpm) and the supernatant removed. The particles are resuspended in 200 μL of absolute ethanol, centrifuged again and the supernatant removed. The ethanol rinse is performed again and the particles resuspended in a final volume of 30 μL of ethanol. An aliquot (5 μL) of the DNA-coated gold particles can be placed in the center of a Kapton™ flying disc (Bio-Rad Labs). The particles are then accelerated into the maize tissue with a Biolistic® PDS-1000/He (Bio-Rad Instruments, Hercules CA), using a helium pressure of 1000 psi, a gap distance of 0.5 cm and a flying distance of 1.0 cm.

For bombardment, the embryogenic tissue is placed on filter paper over agarose-solidified N6 medium. The tissue is arranged as a thin lawn and covered a circular area of about 5 cm in diameter. The petri dish containing the tissue can be placed in the chamber of the PDS-1000/He approximately 8 cm from the stopping screen. The air in the chamber is then evacuated to a vacuum of 28 inches of Hg. The macrocarrier is accelerated with a helium shock wave using a rupture membrane that bursts when the He pressure in the shock tube reaches 1000 psi.

Seven days after bombardment the tissue can be transferred to N6 medium that contains bialaphos (5 mg per liter) and lacks casein or proline. The tissue continues to grow slowly on this medium. After an additional two weeks the tissue can be transferred to fresh N6 medium containing bialaphos. After six weeks, areas of about 1 cm in diameter of actively growing callus can be identified on some of the plates containing the bialaphos-supplemented medium. These calli may continue to grow when sub-cultured on the selective medium.

Plants can be regenerated from the transgenic callus by first transferring clusters of tissue to N6 medium supplemented with 0.2 mg per liter of 2,4-D. After two weeks the tissue can be transferred to regeneration medium (Fromm et al., Bio/Technology 8:833-839 (1990)). Transgenic T0 plants can be regenerated and their phenotype determined following HTP procedures. T1 seed can be collected.

T1 plants can be grown and analyzed for phenotypic changes. The following parameters can be quantified using image analysis: plant area, volume, growth rate and color analysis can be collected and quantified. Expression constructs that result in an alteration of root architecture or any one of the agronomic characteristics listed above compared to suitable control plants, can be considered evidence that the *Arabidopsis* lead gene functions in maize to alter root architecture or plant architecture.

Furthermore, a recombinant DNA construct containing a validated *Arabidopsis* gene can be introduced into an maize line either by direct transformation or introgression from a separately transformed line.

Transgenic plants, either inbred or hybrid, can undergo more vigorous field-based experiments to study root or plant architecture, yield enhancement and/or resistance to root lodging under various environmental conditions (e.g. variations in nutrient and water availability).

Subsequent yield analysis can also be done to determine whether plants that contain the validated *Arabidopsis* lead gene have an improvement in yield performance, when compared to the control (or reference) plants that do not contain the validated *Arabidopsis* lead gene. Plants containing the validated *Arabidopsis* lead gene would improved yield relative to the control plants, preferably 50% less yield loss under adverse environmental conditions or would have increased yield relative to the control plants under varying environmental conditions.

## EXAMPLE 12

### Electroporation of *Agrobacterium tumefaciens* LBA4404

Electroporation competent cells (40 μl), such as *Agrobacterium tumefaciens* LBA4404 (containing PHP10523), are thawn on ice (20-30 min). PHP10523 contains VIR genes for T-DNA transfer, an *Agrobacterium* low copy number plasmid origin of replication, a tetracycline resistance gene, and a cos site for in vivo DNA biomolecular recombination. Meanwhile the electroporation cuvette is chilled on ice. The electroporator settings are adjusted to 2.1 kV.

A DNA aliquot (0.5 μL JT (US 7,087,812) parental DNA at a concentration of 0.2 μg -1.0 μg in low salt buffer or twice distilled $H_2O$ is mixed with the thawn *Agrobacterium* cells while still on ice. The mix is transferred to the bottom of electroporation cuvette and kept at rest on ice for 1-2 min. The cells are electroporated (Eppendorf electroporator 2510) by pushing "Pulse" button twice (ideally achieving a 4.0 msec pulse). Subsequently 0.5 ml 2xYT medium (or SOCmedium) are added to cuvette and transferred to a 15 ml Falcon tube. The cells are incubated at 28-30° C, 200-250 rpm for 3 h.

Aliquots of 250 μl are spread onto #30B (YM + 50μg/mL Spectinomycin) plates and incubated 3 days at 28-30° C. To increase the number of transformants one of two optional steps can be performed:

*Option 1*: overlay plates with 30 μl of 15 mg/ml Rifampicin. LBA4404 has a chromosomal resistance gene for Rifampicin. This additional selection eliminates some contaminating colonies observed when using poorer preparations of LBA4404 competent cells.

*Option* 2: Perform two replicates of the electroporation to compensate for poorer electrocompetent cells.

Identification of transformants:

Four independent colonies are picked and streaked on AB minimal medium plus 50mg/mL Spectinomycin plates (#12S medium) for isolation of single colonies. The plated are incubate at 28° C for 2-3 days.

A single colony for each putative co-integrate is picked and inoculated with 4 ml #60A with 50 mg/l Spectinomycin. The mix is incubated for 24 h at 28° C with shaking. Plasmid DNA from 4 ml of culture is isolated using Qiagen Miniprep + optional PB wash. The DNA is eluted in 30 μl . Aliquots of 2 μl are used to electroporate 20 μl of DH10b + 20 μl of $ddH_2O$ as per above.

Optionally a 15 μl aliquot can be used to transform 75-100 μl of Invitrogen™-Library Efficiency DH5α. The cells are spread on LB medium plus 50mg/mL Spectinomycin plates (#34T medium) and incubated at 37° C overnight.

Three to four independent colonies are picked for each putative co-integrate and inoculated 4 ml of 2xYT (#60A) with 50 μg/ml Spectinomycin. The cells are incubated at 37° C overnight with shaking.

The plasmid DNA is isolated from 4 ml of culture using QIAprep® Miniprep with optional PB wash (elute in 50 μl) and 8 μl are used for digestion with SalI (using JT parent and PHP10523 as controls).

Three more digestions using restriction enzymes BamHI, EcoRI, and HindIII are performed for 4 plasmids that represent 2 putative co-integrates with correct SalI digestion pattern (using parental DNA and PHP10523 as controls). Electronic gels are recommended for comparison.

Alternatively, for high throughput applications, such as described for Gaspe Bay Flint Derived Maize Lines (Examples 15-17), instead of evaluating the resulting co-integrate vectors by restriction analysis, three colonies can be simultaneously used for the infection step as described in Example 13.

## EXAMPLE 13

*Agrobacterium* mediated transformation into maize

Maize plants can be transformed to overexpress a validated *Arabidopsis* lead gene or the corresponding homologs from various species in order to examine the resulting phenotype.

Agrobacterium-mediated transformation of maize is performed essentially as described by Zhao et al., in Meth. Mol. Biol. 318:315-323 (2006) (see also Zhao et al., Mol. Breed. 8:323-333 (2001) and U.S. Patent No. 5,981,840 issued November 9, 1999, incorporated herein by reference). The transformation process involves bacterium innoculation, co-cultivation, resting, selection and plant regeneration.

*1.Immature Embryo Preparation*

Immature embryos are dissected from caryopses and placed in a 2mL microtube containing 2 mL PHI-A medium.

*2.Agrobacterium Infection and Co-Cultivation of Embryos*

*2.1 Infection Step*

PHI-A medium is removed with 1 mL micropipettor and 1 mL *Agrobacterium* suspension is added. Tube is gently inverted to mix. The mixture is incubated for 5 min at room temperature.

*2.2 Co-Culture Step*

The *Agrobacterium* suspension is removed from the infection step with a 1 mL micropipettor. Using a sterile spatula the embryos are scraped from the tube and transferred to a plate of PHI-B medium in a 100x15 mm Petri dish. The embryos are oriented with the embryonic axis down on the surface of the medium. Plates with the embryos are cultured at 20°C, in darkness, for 3 days. L-Cysteine can be used in the co-cultivation phase. With the standard binary vector, the co-cultivation medium supplied with 100-400 mg/L L-cysteine is critical for recovering stable transgenic events.

*3. Selection of Putative Transgenic Events*

To each plate of PHI-D medium in a 100x15 mm Petri dish, 10 embryos are transferred, maintaining orientation and the dishes are sealed with Parafilm. The plates are incubated in darkness at 28 °C. Actively growing putative events, as pale yellow embryonic tissue are expected to be visible in 6-8 weeks. Embryos that produce no events may be brown and necrotic, and little friable tissue growth is evident. Putative transgenic embryonic tissue is subcultured to fresh PHI-D plates at 2-3 week intervals, depending on growth rate. The events are recorded.

*4. Regeneration* of *T0 plants*

Embryonic tissue propagated on PHI-D medium is subcultured to PHI-E medium (somatic embryo maturation medium); in 100x25 mm Petri dishes and incubated at 28 °C, in darkness, until somatic embryos mature, for about 10-18 days. Individual, matured somatic embryos with well-defined scutellum and coleoptile are transferred to PHI-F embryo germination medium and incubated at 28 °C in the light (about 80 μE from cool white or equivalent fluorescent lamps). In 7-10 days, regenerated plants, about 10 cm tall, are potted in horticultural mix and hardened-off using standard horticultural methods.

Media for Plant Transformation

1. PHI-A: 4g/L CHU basal salts, 1.0 mL/L 1000X Eriksson's vitamin mix, 0.5mg/L thiamin HCL, 1.5 mg/L 2,4-D, 0.69 g/L L-proline, 68.5 g/L sucrose, 36g/L glucose, pH 5.2. Add 100μM acetosyringone, filter-sterilized before using.
2. PHI-B: PHI-A without glucose, increased 2,4-D to 2mg/L, reduced sucrose to 30 g/L and supplemented with 0.85 mg/L silver nitrate (filter-sterilized), 3.0 g/L gelrite, 100μM acetosyringone (filter-sterilized), 5.8.
3. PHI-C: PHI-B without gelrite and acetosyringonee, reduced 2,4-D to 1.5 mg/L and supplemented with 8.0 g/L agar, 0.5 g/L Ms-morpholino ethane sulfonic acid (MES) buffer, 100mg/L carbenicillin (filter-sterilized).
4. PHI-D: PHI-C supplemented with 3mg/L bialaphos (filter-sterilized).

5. PHI-E: 4.3 g/L of Murashige and Skoog (MS) salts, (Gibco, BRL 11117-074), 0.5 mg/L nicotinic acid, 0.1 mg/L thiamine HCl, 0.5mg/L pyridoxine HCl, 2.0 mg/L glycine, 0.1 g/L myo-inositol, 0.5 mg/L zeatin (Sigma, cat.no. Z-0164), 1 mg/L indole acetic acid (IAA), 26.4 μg/L abscisic acid (ABA), 60 g/L sucrose, 3 mg/L bialaphos (filter-sterilized), 100 mg/L carbenicillin (fileter-sterilized), 8g/L agar, pH 5.6.
6. PHI-F: PHI-E without zeatin, IAA, ABA; sucrose reduced to 40 g/L; replacing agar with 1.5 g/L gelrite; pH 5.6.

Plants can be regenerated from the transgenic callus by first transferring clusters of tissue to N6 medium supplemented with 0.2 mg per liter of 2,4-D. After two weeks the tissue can be transferred to regeneration medium (Fromm et al. (1990) Bio/Technology 8:833-839).
Phenotypic analysis of transgenic T0 plants and T1 plants can be performed.

T1 plants can be analyzed for phenotypic changes. Using image analysis T1 plants can be analyzed for phenotypical changes in plant area, volume, growth rate and color analysis can be taken at multiple times during growth of the plants. Alteration in root architecture can be assayed as described in Example 20.

Subsequent analysis of alterations in agronomic characteristics can be done to determine whether plants containing the validated *Arabidopsis* lead gene have an improvement of at least one agronomic characteristic, when compared to the control (or reference) plants that do not contain the validated *Arabidopsis* lead gene. The alterations may also be studied under various environmental conditions.

Expression constructs that result in a significant alteration in root architecture will be considered evidence that the *Arabidopsis* gene functions in maize to alter root architecture.

## EXAMPLE 14A

### Construction of Maize expression vectors with the *Arabidopsis* Lead Gene (AT4G23900) using *Agrobacterium* mediated Transformation

Maize expression vectors were prepared with the *Arabidopsis* ndk gene (AT4G23900) under the control of the NAS2 (SEQ ID NO:57 and GOS2 (SEQ ID NO:58) promoter. PINII was the terminator (SEQ ID NO:61)
Using Invitrogen ™ Gateway® technology the entry clone, created as described in Example 5A, PHP 28731, containing the *Arabidopsis* ndk gene (AT4G23900) was used in separate Gateway® LR reactions with:

1) the constitutive maize GOS2 promoter entry clone (PHP28408, Fig.11, SEQ ID NO:11) and the PinII Terminator entry clone (PHP20234, Fig.9, SEQ ID NO:9) into the destination vector PHP28529 (Fig.10 , SEQ ID NO:10). The resulting vector was named PHP28911.
2) the root maize NAS2 promoter entry clone (PHP22020, Fig.12 ,SEQ ID NO:12) and the PinII Terminator entry clone (PHP20234, Fig.9, SEQ ID NO:9) into the destination vector PHP28529 (Fig.10 , SEQ ID NO:10). The resulting vector was named PHP28912.

The destination vector PHP28529 added to each of the final vectors (PHP28911 and PHP28912) also an:

1) RD29A promoter::yellow fluorescent protein:PinII terminator cassette for Arabidospis seed sorting
2) a Ubiquitin promoter:moPAT/red fluorescent protein fusion:PinII terminator cassette for transformation selection and Z.mays seed sorting.

## EXAMPLE 14B

### Preparation of Maize expression constructs containing the *Arabidopsis* ndk gene and Homologs thereof

The *Arabidopsis* ndk gene and the corresponding homologs from maize and other species (Table 1) can be transformed into maize lines using the procedures outlined in Examples 5A and 14A. Maize expression vectors with *Arabidopsis* ndk gene and the corresponding homologs from maize and other species (Table 1) can be prepared as outlined in Examples 5A and 14A. In addition to the GOS2 or NAS2 promoter, other promoters such as, but not limited to the ubiquitin promoter, the S2A and S2B promoter, the maize ROOTMET2 promoter, the maize Cyclo, the CR1BIO, the CRWAQ81 and the maize ZRP2.4447 are useful for directing expression of ndk and ndk-like genes in maize. Furthermore, a variety of terminators, such as, but not limited to the PINII terminator, could be used to achieve expression of the gene of interest in maize.

**EXAMPLE 14C**

Transformation of Maize Lines with the *Arabidopsis* Lead Gene (AT4G23900) and corresponding homologs from other species using *Agrobacterium* mediated Transformation

The final vectors (vectors for expression in Maize, Example 14A, and B) can be then electroporated separately into LBA4404 *Agrobacterium* containing PHP10523 (Fig.7; SEQ ID NO:7, Komari et al. Plant J 10:165-174 (1996), NCBI GI: 59797027) to create the co-integrate vectors for maize transformation. The co-integrate vectors are formed by recombination of the final vectors (maize expression vectors) with PHP10523, through the COS recombination sites contained on each vector. The co-integrate vectors contain in addition to the expression cassettes described in Examples 14A-B, also genes needed for the *Agrobacterium* strain and the *Agrobacterium* mediated transformation,(TET, TET, TRFA, ORI terminator, CTL, ORI V, VIR C1, VIR C2, VIR G, VIR B). Transformation into a maize line can be performed as described in Example 13.

**EXAMPLE15**

Preparation of the destination vectors PHP23236 and PHP29635 for Transformation of Gaspe Bay Flint derived Maize Lines

Destination vector PHP23236 (Fig.6, SEQ ID NO:6) was obtained by transformation of *Agrobacterium* strain LBA4404 containing plasmid PHP10523 (Fig.7, SEQ ID NO:7) with plasmid PHP23235 (Fig.8, SEQ ID NO:8) and isolation of the resulting co-integration product. Destination vector PHP23236, can be used in a recombination reaction with an entry clone as described in Example 16 to create a maize expression vector for transformation of Gaspe Bay Flint derived maize lines. Expression of the gene of interest is under control of the ubiquitin promoter (SEQ ID NO:59). PHP29635 (Fig.13, SEQ ID NO:13) was obtained by transformation of *Agrobacterium* strain LBA4404 containing plasmid PHP10523 with plasmid PIIOXS2a-FRT87(ni)m (Fig.14, SEQ ID NO:56) and isolation of the resulting co-integration product. Destination vector PHP29635 can be used in a recombination reaction with an entry clone as described in Example 16 to create a maize expression vector for transformation of Gaspe Bay Flint derived maize lines. Expression of the gene of interest is under control of the S2A promoter (SEQ ID NO:60).

**EXAMPLE 16**

Preparation of Plasmids for Transformation of Gaspe Bay Flint Derived Maize Lines

Using Invitrogen™ Gateway® Recombination technology, entry clones containing the *Arabidopsis* ndk gene (AT4G23900) or a maize ndk-like homolog can be created, as described in Examples 5A and 9 and used to directionally clone each gene into destination vector PHP23236 (Example 15) for expression under the ubiquitin promoter or into destination vector PHP29635 (Example 15) for expression under the S2A promoter. Each of the expression vectors are T-DNA binary vectors for Agrobacterium-mediated transformation into corn._

Gaspe Bay Flint Derived Maize Lines can be transformed with the expression constructs as described in Example 17.

**EXAMPLE 17**

Transformation of Gaspe Bay Flint Derived Maize Lines with Validated *Arabidopsis* Lead Genes and corresponding homologs from other species

Maize plants can be transformed as described in Example 16 to overexpress the *Arabidopsis* AT4G23900 gene and the corresponding homologs from other species, such as the ones listed in Table 1, in order to examine the resulting phenotype. In addition to the promoters decribed in Example 16 other promoters such the S2B promoter, the maize ROOTMET2 promoter, the maize Cyclo, the CR1BIO, the CRWAQ81 and the maize ZRP2.4447 are useful for directing expression of ndk and ndk-like genes in maize. Furthermore, a variety of terminators, such as, but not limited to the PINII terminator, can be used to achieve expression of the gene of interest in Gaspe Bay Flint Derived Maize Lines.

Recipient Plants

Recipient plant cells can be from a uniform maize line having a short life cycle ("fast cycling"), a reduced size, and high transformation potential. Typical of these plant cells for maize are plant cells from any of the publicly available Gaspe Bay Flint (GBF) line varieties. One possible candidate plant line variety is the F1 hybrid of GBF x QTM (Quick Turnaround Maize, a publicly available form of Gaspe Bay Flint selected for growth under greenhouse conditions) disclosed in Tomes et al. U.S. Patent Application Publication No. 2003/0221212. Transgenic plants obtained from this line are of such a reduced size that they can be grown in four inch pots (1/4 the space needed for a normal sized maize plant) and mature in less than 2.5 months. (Traditionally 3.5 months is required to obtain transgenic T0 seed once the transgenic plants are acclimated to the greenhouse.) Another suitable line is a double haploid line of GS3 (a highly transformable line) X Gaspe Flint. Yet another suitable line is a transformable elite inbred line carrying a transgene which causes early flowering, reduced stature, or both.

Transformation Protocol

Any suitable method may be used to introduce the transgenes into the maize cells, including but not limited to inoculation type procedures using *Agrobacterium* based vectors as described in Example 9. Transformation may be performed on immature embryos of the recipient (target) plant.

Precision Growth and Plant Tracking

The event population of transgenic (T0) plants resulting from the transformed maize embryos is grown in a controlled greenhouse environment using a modified randomized block design to reduce or eliminate environmental error. A randomized block design is a plant layout in which the experimental plants are divided into groups (e.g., thirty plants per group), referred to as blocks, and each plant is randomly assigned a location with the block.

For a group of thirty plants, twenty-four transformed, experimental plants and six control plants (plants with a set phenotype) (collectively, a "replicate group") are placed in pots which are arranged in an array (a.k.a. a replicate group or block) on a table located inside a greenhouse. Each plant, control or experimental, is randomly assigned to a location with the block which is mapped to a unique, physical greenhouse location as well as to the replicate group. Multiple replicate groups of thirty plants each may be grown in the same greenhouse in a single experiment. The layout (arrangement) of the replicate groups should be determined to minimize space requirements as well as environmental effects within the greenhouse. Such a layout may be referred to as a compressed greenhouse layout.

An alternative to the addition of a specific control group is to identify those transgenic plants that do not express the gene of interest. A variety of techniques such as RT-PCR can be applied to quantitatively assess the expression level of the introduced gene. T0 plants that do not express the transgene can be compared to those which do.

Each plant in the event population is identified and tracked throughout the evaluation process, and the data gathered from that plant is automatically associated with that plant so that the gathered data can be associated with the transgene carried by the plant. For example, each plant container can have a machine readable label (such as a Universal Product Code (UPC) bar code) which includes information about the plant identity, which in turn is correlated to a greenhouse location so that data obtained from the plant can be automatically associated with that plant.

Alternatively any efficient, machine readable, plant identification system can be used, such as two-dimensional matrix codes or even radio frequency identification tags (RFID) in which the data is received and interpreted by a radio frequency receiver/processor. See U.S. Published Patent Application No. 2004/0122592, incorporated herein by reference.

Phenotypic Analysis Using Three-Dimensional Imaging

Each greenhouse plant in the T0 event population, including any control plants, is analyzed for agronomic characteristics of interest, and the agronomic data for each plant is recorded or stored in a manner so that it is associated with the identifying data (see above) for that plant. Confirmation of a phenotype (gene effect) can be accomplished in the T1 generation with a similar experimental design to that described above.

The T0 plants are analyzed at the phenotypic level using quantitative, nondestructive imaging technology throughout the plant's entire greenhouse life cycle to assess the traits of interest. Preferably, a digital imaging analyzer is used for automatic multi-dimensional analyzing of total plants. The imaging may be done inside the greenhouse. Two camera systems, located at the top and side, and an apparatus to rotate the plant, are used to view and image plants from all sides. Images are acquired from the top, front and side of each plant. All three images together provide sufficient information to evaluate the biomass, size and morphology of each plant.

Due to the change in size of the plants from the time the first leaf appears from the soil to the time the plants are at the end of their development, the early stages of plant development are best documented with a higher magnification from the top. This may be accomplished by using a motorized zoom lens system that is fully controlled by the imaging software.

In a single imaging analysis operation, the following events occur: (1) the plant is conveyed inside the analyzer area, rotated 360 degrees so its machine readable label can be read, and left at rest until its leaves stop moving; (2) the side image is taken and entered into a database; (3) the plant is rotated 90 degrees and again left at rest until its leaves stop moving, and (4) the plant is transported out of the analyzer.

Plants are allowed at least six hours of darkness per twenty four hour period in order to have a normal day/ night cycle.

Imaging Instrumentation

Any suitable imaging instrumentation may be used, including but not limited to light spectrum digital imaging instrumentation commercially available from LemnaTec GmbH of Wurselen, Germany. The images are taken and analyzed with a LemnaTec Scanalyzer HTS LT-0001-2 having a 1/2" IT Progressive Scan IEE CCD imaging device. The imaging cameras may be equipped with a motor zoom, motor aperture and motor focus. All camera settings may be made using LemnaTec software. Preferably, the instrumental variance of the imaging analyzer is less than about 5% for major components and less than about 10% for minor components.

Softwa re

The imaging analysis system comprises a LemnaTec HTS Bonit software program for color and architecture analysis and a server database for storing data from about 500,000 analyses, including the analysis dates. The original images and the analyzed images are stored together to allow the user to do as much reanalyzing as desired. The database can be connected to the imaging hardware for automatic data collection and storage. A variety of commercially available software systems (e.g. Matlab, others) can be used for quantitative interpretation of the imaging data, and any of these software systems can be applied to the image data set.

Conveyor System

A conveyor system with a plant rotating device may be used to transport the plants to the imaging area and rotate them during imaging. For example, up to four plants, each with a maximum height of 1.5 m, are loaded onto cars that travel over the circulating conveyor system and through the imaging measurement area. In this case the total footprint of the unit (imaging analyzer and conveyor loop) is about 5 m x 5 m.

The conveyor system can be enlarged to accommodate more plants at a time. The plants are transported along the conveyor loop to the imaging area and are analyzed for up to 50 seconds per plant. Three views of the plant are taken. The conveyor system, as well as the imaging equipment, should be capable of being used in greenhouse environmental conditions.

Illumination

Any suitable mode of illumination may be used for the image acquisition. For example, a top light above a black background can be used. Alternatively, a combination of top- and backlight using a white background can be used. The illuminated area should be housed to ensure constant illumination conditions. The housing should be longer than the measurement area so that constant light conditions prevail without requiring the opening and closing or doors. Alternalvely, the illumination can be varied to cause excitation of either transgene (e.g., green fluorescent protein (GFP), red fluorescent protein (RFP)) or endogenous (e.g. Chlorophyll) fluorophores.

Biomass Estimation Based on Three-Dimensional Imaging

For best estimation of biomass the plant images should be taken from at least three axes, preferably the top and two side (sides 1 and 2) views. These images are then analyzed to separate the plant from the background, pot and pollen control bag (if applicable). The volume of the plant can be estimated by the calculation:

$$Volume(voxels) = \sqrt{TopArea(pixels)} \times \sqrt{Side1Area(pixels)} \times \sqrt{Side2Area(pixels)}$$

In the equation above the units of volume and area are "arbitrary units". Arbitrary units are entirely sufficient to detect gene effects on plant size and growth in this system because what is desired is to detect differences (both positive-larger and negative-smaller) from the experimental mean, or control mean. The arbitrary units of size (e.g. area) may be trivially converted to physical measurements by the addition of a physical reference to the imaging process. For instance, a physical reference of known area can be included in both top and side imaging processes. Based on the area of these physical references a conversion factor can be determined to allow conversion from pixels to a unit of area such as square centimeters ($cm^2$). The physical reference may or may not be an independent sample. For instance, the pot, with a known diameter and height, could serve as an adequate physical reference.

Color Classification

The imaging technology may also be used to determine plant color and to assign plant colors to various color classes. The assignment of image colors to color classes is an inherent feature of the LemnaTec software. With other image analysis software systems color classification may be determined by a variety of computational approaches.

For the determination of plant size and growth parameters, a useful classification scheme is to define a simple color scheme including two or three shades of green and, in addition, a color class for chlorosis, necrosis and bleaching, should these conditions occur. A background color class which includes non plant colors in the image (for example pot and soil colors) is also used and these pixels are specifically excluded from the determination of size. The plants are analyzed under controlled constant illumination so that any change within one plant over time, or between plants or different batches of plants (e.g. seasonal differences) can be quantified.

In addition to its usefulness in determining plant size growth, color classification can be used to assess other yield component traits. For these other yield component traits additional color classification schemes may be used. For instance, the trait known as "staygreen", which has been associated with improvements in yield, may be assessed by a color classification that separates shades of green from shades of yellow and brown (which are indicative of senescing tissues). By applying this color classification to images taken toward the end of the T0 or T1 plants' life cycle, plants that have increased amounts of green colors relative to yellow and brown colors (expressed, for instance, as Green/Yellow Ratio) may be identified. Plants with a significant difference in this Green/Yellow ratio can be identified as carrying transgenes which impact this important agronomic trait.

The skilled plant biologist will recognize that other plant colors arise which can indicate plant health or stress response (for instance anthocyanins), and that other color classification schemes can provide further measures of gene action in traits related to these responses.

Plant Architecture Analysis

Transgenes which modify plant architecture parameters may also be identified using the present invention, including such parameters as maximum height and width, internodal distances, angle between leaves and stem, number of leaves starting at nodes and leaf length. The LemnaTec system software may be used to determine plant architecture as follows. The plant is reduced to its main geometric architecture in a first imaging step and then, based on this image, parameterized identification of the different architecture parameters can be performed. Transgenes that modify any of these architecture parameters either singly or in combination can be identified by applying the statistical approaches previously described.

Pollen Shed Date

Pollen shed date is an important parameter to be analyzed in a transformed plant, and may be determined by the first appearance on the plant of an active male flower. To find the male flower object, the upper end of the stem is classified by color to detect yellow or violet anthers. This color classification analysis is then used to define an active flower, which in turn can be used to calculate pollen shed date.

Alternatively, pollen shed date and other easily visually detected plant attributes (e.g. pollination date, first silk date) can be recorded by the personnel responsible for performing plant care. To maximize data integrity and process efficiency this data is tracked by utilizing the same barcodes utilized by the LemnaTec light spectrum digital analyzing device. A computer with a barcode reader, a palm device, or a notebook PC may be used for ease of data capture recording time of observation, plant identifier, and the operator who captured the data.

Orientation of the Plants

Mature maize plants grown at densities approximating commercial planting often have a planar architecture. That is, the plant has a clearly discernable broad side, and a narrow side. The image of the plant from the broadside is determined. To each plant a well defined basic orientation is assigned to obtain the maximum difference between the broadside and edgewise images. The top image is used to determine the main axis of the plant, and an additional rotating device is used to turn the plant to the appropriate orientation prior to starting the main image acquisition.

**EXAMPLE 18**

Screening of Gaspe Bay Flint Derived Maize Lines and Hybrids Under Nitrogen Limiting Conditions

Some transgenic plants will contain two or three doses of Gaspe Flint-3 with one dose of GS3 (GS3/(Gaspe-3)2X or GS3/(Gaspe-3)3X) and will segregate 1:1 for a dominant transgene. Other transgenic plants will be regular inbreds and will be used in top crosses to generate test hybrids. Plants will be planted in Turface, a commercial potting medium, and watered four times each day with 1 mM $KNO_3$ growth medium and with 2 mM $KNO_3$, or higher, growth medium (see Fig.17). Control plants grown in 1 mM $KNO_3$ medium will be less green, produce less biomass and have a smaller ear at anthesis. Gaspe-derived lines will be grown to flowering stage whereas regular inbreds and hybrids will be grown to V4 to V5 stages.

Statistics are used to decide if differences seen between treatments are significantly different. Fig.18 illustrates one method which places letters after the values. Those values in the same column that have the same letter (not group of letters) following them are not significantly different. Using this method, if there are no letters following the values in a column, then there are no significant differences between any of the values in that column or, in other words, all the values in that column are equal. Expression of a transgene will result in plants with improved plant growth in 1 mM $KNO_3$ when compared to a transgenic null. Thus biomass and greenness data (as described in Example 17) will be collected at time of sampling (anthesis for Gaspe and V4-V5 for others) and compared to a transgenic null. In addition, total nitrogen in the plants will be analyzed in ground tissues. Improvements in growth, greenness, nitrogen accumulation and ear size at anthesis will be indications of increased nitrogen use efficiency.

**EXAMPLE 19**

Yield Analysis of Maize Lines with Validated *Arabidopsis* Lead Gene (AT4G23900)

A recombinant DNA construct containing a validated *Arabidopsis* gene can be introduced into a maize line either by direct transformation or introgression from a separately transformed line.

Transgenic plants, either inbreds or top cross hybrids, can undergo more vigorous field-based experiments to study yield enhancement and/or stability under various environmental conditions, such as variations in water and nutrient availability. A standardized yield trial will typically include 4 to 6 replications, and at least 4 locations. Combined harvest yield data will be collected.

Subsequent yield analysis can be done to determine whether plants that contain the validated *Arabidopsis* lead gene have an improvement in yield performance under various environmental conditions, when compared to the control plants, either a construct null or wildtype, that do not contain the validated *Arabidopsis* lead gene. Reduction in yield can be measured for both in the case of nitrogen or water stress environments. Plants containing the validated *Arabidopsis* lead gene have less yield loss relative to the control plants, preferably 50% less yield loss.

**EXAMPLE 20**

Assays to Determine Alterations of Root Architecture in Maize

Transgenic maize plants are assayed for changes in root architecture at seedling stage, flowering time or maturity. Assays to measure alterations of root architecture of maize plants include, but are not limited to the methods outlined below. To facilitate manual or automated assays of root architecture alterations, corn plants can be grown in clear pots.

1) Root mass (dry weights). Plants are grown in Turface, a growth media that allows easy separation of roots. Oven-dried shoot and root tissues are weighed and a root/shoot ratio calculated.

2) Levels of lateral root branching. The extent of lateral root branching (e.g. lateral root number, lateral root length) is determined by sub-sampling a complete root system, imaging with a flat-bed scanner or a digital camera and analyzing with WinRHIZO™ software (Regent Instruments Inc.).

3) Root band width measurements. The root band is the band or mass of roots that forms at the bottom of greenhouse pots as the plants mature. The thickness of the root band is measured in mm at maturity as a rough estimate of root mass.

4) Nodal root count. The number of crown roots coming off the upper nodes can be determined after separating the root from the support medium (e.g. potting mix). In addition the angle of crown roots and/or brace roots can be measured. Digital analysis of the nodal roots and amount of branching of nodal roots form another extension to the aforementioned manual method.

All data taken on root phenotype are subjected to statistical analysis, normally a t-test to compare the transgenic roots with that of non-transgenic sibling plants. One-way ANOVA may also be used in cases where multiple events and/or constructs are involved in the analysis.

## EXAMPLE 21

Analysis of Roots of Maize Seedlings containing the Arabidopsis ndk Gene compared to Roots from Seedlings not containing the ndk Gene

A maize expression vector, containing the maize GOS2 promoter and the Arabidopsis ndk gene was prepared as described in Example 14A. Transformation of maize was achieved via Agrobacterium mediated transformation as described in example 14C by creating a cointegrate vector (PHP29007) and roots were assayed using a seedling assay as described in Example 20. All 10 events from construct PHP29007 (ZM-GOS2::AT-NDK4) were assayed in a greenhouse experiment, where 9 plants per each event were grown in Turface media to V4 stage. Seeds were from the T1 generation (from ears collected from T0 plants). The control in the experiment were 15 plants of the same hybrid maize line, not containing the recombinant construct, grown to the same stage. Seeds were planted using a complete random block design. Plants were harvested 19 days after planting, when they reached V4 stage. Roots were washed and collected separately from shoots. All samples were oven-dried before dry weights were taken on an analytical balance.

As can be seen from Table 5 a total of 4 events were found to have significant changes in shoot dry weights, and 2 events with significant changes in root dry weights, when compared to the control.

T-test analysis was performed to show significant differences between each transgenic event and the control. The p-values are shown for each trait: root dry weights, shoot dry weights, and root-to-shoot ratios. Bold face fonts indicate the transgenic had a higher value than the control. Those that had a p-value of less than 0.1 are indicated with an asterisk (*).

**TABLE 5**

| Comparison of transgenic and control seedlings | | | |
|---|---|---|---|
| **EVENT** | **Root Dry Weight** | **Shoot Dry Weight** | **Root/Shoot Ratio** |
| 1 | **0.330** | **0.070** | 0.305 |
| 2 | 0.405 | 0.411 | **0.500** |
| 3 | 0.002 * | 0.000 * | **0.008 *** |
| 4 | 0.000 * | 0.000 * | **0.016 *** |
| 5 | **0.225** | **0.082 *** | 0.436 |
| 6 | 0.573 | 0.752 | 0.809 |
| 7 | **0.955** | 0.576 | **0.181** |
| 8 | **0.261** | **0.074 *** | 0.407 |
| 9 | 0.683 | 0.463 | **0.608** |
| 10 | 0.682 | 0.663 | **0.368** |

## EXAMPLE 22

Yield testing of transgenic hybrids grown under normal and under nitrogen depleted conditions in the field.

A field experiment was carried out on a farm in Johnston, Iowa in the 2007 season. Ten (10) transgenic events expressing the Arabidopsis NDK4 gene driven by the maize GOS2 promoter, and two controls were included in the experiment. One control consisted of a non-transgenic null with nulls bulked across all 10 events. The other control consisted of the wild type (same hybrid maize line not containing the recombinant construct) used in transformation. All of the plants were hybrid maize lines generated from a common inbred tester.

Two treatments were applied, consisting of conditions wherein the plants were either grown under "normal" nitrogen or under nitrogen "depleted" conditions. The "normal" treatment included application of a nitrogen fertilizer at a rate of 250lb per acre. Nitrogen "depleted" conditions were achieved by growing the transgenic and non-transgenic control maize lines in a field wherein soil nitrogen levels had been withdrawn by crops grown in previous years in the absence of fertilizer.

Nitrogen depletion was controlled at the level that caused 30% yield reduction, compared to the normal nitrogen treatment, and required a 100lb per acre nitrogen fertilization rate.. The experiments were set up as 2-row plots with a density of 32000 plants per acre. Four (4) and six (6) replications were included in the normal and the depleted nitrogen treatment,respectively. Plants were planted on May 21, 2007 and harvested combined on September 26 and 27, 2007. Yield was measured as bushels per acre.

The yield data from the experiment are summarized in the Table 6 below. Overall, one (1) event under low nitrogen and four (4) events under normal nitrogen conditions showed significant increase in yield over the bulked null control. One event under low nitrogen had a significant reduction in yield compared to control. The majority of the events tested, showed a positive trend in yield increase over nulls.

**TABLE 6**

| Yield tests of transgenic versus control plants under low and normal nitrogen conditions. | | | |
|---|---|---|---|
| Event | Yield increase over null | Significance | Treatment |
| 1 | 6.74% | | Low nitrogen |
| 2 | 7.05% | | Low nitrogen |
| 3 | -1.84% | | Low nitrogen |
| 4 | 0.33% | | Low nitrogen |
| 5 | 1.66% | | Low nitrogen |
| 6 | 4.34% | | Low nitrogen |
| 7 | -9.10% | P = 0.1 | Low nitrogen |
| **8** | **7.89%** | **P = 0.1** | **Low nitrogen** |
| 9 | 4.13% | | Low nitrogen |
| 10 | 1.05% | | Low nitrogen |
| 1 | 3.25% | | Normal nitrogen |
| 2 | 2.40% | | Normal nitrogen |
| 3 | -1.71% | | Normal nitrogen |
| **4** | **9.16%** | **P = 0.1** | **Normal nitrogen** |
| 5 | 2.70% | | Normal nitrogen |
| **6** | **5.26%** | **P = 0.1** | **Normal nitrogen** |
| **7** | **6.66%** | **P = 0.1** | **Normal nitrogen** |
| 8 | -0.04% | | Normal nitrogen |
| 9 | -2.57% | | Normal nitrogen |
| **10** | **7.27%** | **P = 0.1** | **Normal nitrogen** |

Embodiments of the invention are also described in the claims of the International application as filed:

1. A plant comprising in its genome a recombinant DNA construct comprising a polynucleotide operably linked to at least one regulatory element, wherein said polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51 and wherein said plant exhibits altered root architecture when compared to a control plant not comprising said recombinant DNA construct.
2. The plant of claim 1, wherein the plant is a maize plant or a soybean plant.
3. A plant comprising in its genome:

a recombinant DNA construct comprising:

(a) a polynucleotide operably linked to at least one regulatory element,
wherein said polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51, or
(b) a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51, or (B) a full complement of the nucleic acid sequence of (b)(i)(A); or
(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide,
and wherein said plant exhibits an alteration of at least one agronomic characteristic when compared to a control plant not comprising said recombinant DNA construct.

4. The plant of claim 3, wherein the plant is a maize plant or a soybean plant.
5. The plant of claim 3, wherein said plant exhibits said alteration of said at least one agronomic characteristic when compared, under varying environmental conditions, to said control plant not comprising said recombinant DNA construct.
6. The plant of claim 5, wherein said varying environmental condition is at least one selected from drought, nitrogen, or disease.
7. The plant of claim 5, wherein the plant is a maize plant or a soybean plant.
8. The plant of claim 7, wherein the plant is a maize plant or a soybean plant.
9. The plant of claim 3, wherein said at least one agronomic characteristic is selected from the group consisting of greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen uptake, nitrogen stress tolerance, root lodging, stalk lodging, plant height, ear length and harvest index.
10. The plant of claim 9, wherein the plant is a maize plant or a soybean plant.
11. The plant of claim 3, wherein said plant exhibits an increase of said at least one agronomic characteristic when compared to said control plant.
12. The plant of claim 11, wherein the plant is a maize plant or a soybean plant.
13. A method of altering root architecture in a plant, comprising:

(a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51; and
(b) regenerating a transgenic plant from the regenerable plant cell after step (a), wherein the transgenic plant comprises in its genome the recombinant DNA construct and exhibits altered root architecture when compared to a control plant not comprising the recombinant DNA construct.

14. The method of claim 13, further comprising:

(c) obtaining a progeny plant derived from the transgenic plant, wherein said progeny plant comprises in its genome the recombinant DNA construct and exhibits altered root architecture when compared to a control plant not comprising the recombinant DNA construct.

15. A method of evaluating root architecture in a plant, comprising:

(a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51;
(b) regenerating a transgenic plant from the regenerable plant cell after step (a), wherein the transgenic plant comprises in its genome the recombinant DNA construct; and
(c) evaluating root architecture of the transgenic plant compared to a control plant not comprising the recombinant DNA construct.

16. The method of claim 15, further comprising:

(d) obtaining a progeny plant derived from the transgenic plant, wherein the progeny plant comprises in its genome the recombinant DNA construct; and
(e) evaluating root architecture of the progeny plant compared to a control plant not comprising the recombinant DNA construct.

17. A method of evaluating root architecture in a plant, comprising:

(a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15,17,19, 21, 23, 25, 27, 29, 31, 33, 35, 37, 39, 41, 43 or 51;
(b) regenerating a transgenic plant from the regenerable plant cell after step (a), wherein the transgenic plant comprises in its genome the recombinant DNA construct;
(c) obtaining a progeny plant derived from the transgenic plant, wherein the progeny plant comprises in its genome the recombinant DNA construct; and
(d) evaluating root architecture of the progeny plant compared to a control plant not comprising the recombinant DNA construct.

18. A method of determining an alteration of an agronomic characteristic in a plant, comprising:

(a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15,17,19, 21, 23, 25, 27, 29, 31, 33, 35, 37, 39, 41, 43 or 51;
(b) regenerating a transgenic plant from the regenerable plant cell after step (a), wherein the transgenic plant comprises in its genome the recombinant DNA construct; and
(c) determining whether the transgenic plant exhibits an alteration of at least one agronomic characteristic when compared to a control plant not comprising the recombinant DNA construct.

19. The method of claim 18, further comprising:

(d) obtaining a progeny plant derived from the transgenic plant, wherein the progeny plant comprises in its genome the recombinant DNA construct; and
(e) determining whether the progeny plant exhibits an alteration of at least one agronomic characteristic when compared to a control plant not comprising the recombinant DNA construct.

20. The method of claim 19, wherein said determining step comprises determining whether the transgenic plant exhibits an alteration of at least one agronomic characteristic when compared, under varying environmental conditions, to a control plant not comprising the recombinant DNA construct.
21. The method of claim 19, wherein said determining step (e) comprises determining whether the progeny plant exhibits an alteration of at least one agronomic characteristic when compared, under varying environmental conditions, to a control plant not comprising the recombinant DNA construct.
22. A method of determining an alteration of an agronomic characteristic in a plant, comprising:

(a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51;
(b) regenerating a transgenic plant from the regenerable plant cell after step (a), wherein the transgenic plant comprises in its genome the recombinant DNA construct;

(c) obtaining a progeny plant derived from the transgenic plant, wherein the progeny plant comprises in its genome the recombinant DNA construct; and
(d) determining whether the progeny plant exhibits an alteration of at least one agronomic characteristic when compared to a control plant not comprising the recombinant DNA construct.

23. The method of claim 22, wherein said determining step comprises determining whether the transgenic plant exhibits an alteration of at least one agronomic characteristic when compared, under varying environmental conditions, to a control plant not comprising the recombinant DNA construct.
24. A method of determining an alteration of an agronomic characteristic in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51, or (B) a full complement of the nucleic acid sequence of (a)(i)(A); or
(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide;

(b) regenerating a transgenic plant from the regenerable plant cell after step (a), wherein the transgenic plant comprises in its genome the suppression DNA construct; and
(c) determining whether the transgenic plant exhibits an alteration of at least one agronomic characteristic when compared to a control plant not comprising the suppression DNA construct.

25. The method of claim 24, wherein said determining step comprises determining whether the transgenic plant exhibits an alteration of at least one agronomic characteristic when compared, under varying environmental conditions, to a control plant not comprising the suppression DNA construct.
26. The method of claim 24, further comprising:

(d) obtaining a progeny plant derived from the transgenic plant, wherein the progeny plant comprises in its genome the suppression DNA construct; and
(e) determining whether the progeny plant exhibits an alteration of at least one agronomic characteristic when compared to a control plant not comprising the suppression DNA construct.

27. The method of claim 26, wherein said determining step (e) comprises determining whether the progeny plant exhibits an alteration of at least one agronomic characteristic when compared, under varying environmental conditions, to a control plant not comprising the suppression DNA construct.
28. A method of determining an alteration of an agronomic characteristic in a plant, comprising:

(a)introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15,17, 19, 21, 23, 25, 27, 29, 31, 33, 35, 37, 39, 41, 43 or 51, or (B) a full complement of the nucleic acid sequence of (a)(i)(A); or
(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide;

(b) regenerating a transgenic plant from the regenerable plant cell after step (a), wherein the transgenic plant comprises in its genome the suppression DNA construct and exhibits altered root architecture when compared to a control plant not comprising the suppression DNA construct;
(c) obtaining a progeny plant derived from the transgenic plant, wherein the progeny plant comprises in its genome the suppression DNA construct; and

(d) determining whether the progeny plant exhibits an alteration of at least one agronomic characteristic when compared to a control plant not comprising the suppression DNA construct.

29. The method of claim 28, wherein said determining step comprises determining whether the transgenic plant exhibits an alteration of at least one agronomic characteristic when compared, under varying environmental conditions, to a control plant not comprising the recombinant DNA construct.

30. A method of altering root architecture in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51, or (B) a full complement of the nucleic acid sequence of (a)(i)(A); or

(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide; and

(b) regenerating a transgenic plant from the regenerable plant cell after step (a), wherein the transgenic plant comprises in its genome the suppression DNA construct and wherein the transgenic plant exhibits altered root architecture when compared to a control plant not comprising the suppression DNA construct.

31. The method of claim 30, further comprising:

(c) obtaining a progeny plant derived from the transgenic plant, wherein said progeny plant comprises in its genome the recombinant DNA construct and wherein the progeny plant exhibits altered root architecture when compared to a control plant not comprising the suppression DNA construct.

32. A method of evaluating root architecture in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15, 17, 19, 21, 23, 25, 27, 29, 31, 33, 35, 37, 39, 41, 43 or 51, or (B) a full complement of the nucleic acid sequence of (a)(i)(A); or

(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide;

(b) regenerating a transgenic plant from the regenerable plant cell after step (a), wherein the transgenic plant comprises in its genome the suppression DNA construct; and

(c) evaluating root architecture of the transgenic plant compared to a control plant not comprising the suppression DNA construct.

33. The method of claim 32, further comprising:

(d) obtaining a progeny plant derived from the transgenic plant, wherein the progeny plant comprises in its genome the suppression DNA construct; and

(e) evaluating root architecture of the progeny plant compared to a control plant not comprising the suppression DNA construct.

34. A method of evaluating root architecture in a plant, comprising:

(a) introducing into a regenerable plant cell a suppression DNA construct comprising at least one regulatory element operably linked to:

(i) all or part of: (A) a nucleic acid sequence encoding a polypeptide having an amino acid sequence of at least 50% sequence identity, based on the Clustal V method of alignment, when compared to SEQ ID NO: 15,17, 19, 21, 23, 25, 27, 29, 31, 33, 35, 37, 39, 41, 43 or 51, or (B) a full complement of the nucleic acid sequence of (a)(i)(A); or
(ii) a region derived from all or part of a sense strand or antisense strand of a target gene of interest, said region having a nucleic acid sequence of at least 50% sequence identity, based on the Clustal V method of alignment, when compared to said all or part of a sense strand or antisense strand from which said region is derived, and wherein said target gene of interest encodes a NDK or NDK-like polypeptide;

(b) regenerating a transgenic plant from the regenerable plant cell after step (a), wherein the transgenic plant comprises in its genome the suppression DNA construct;
(c)obtaining a progeny plant derived from the transgenic plant, wherein the progeny plant comprises in its genome the suppression DNA construct; and
(d) evaluating root architecture of the progeny plant compared to a control plant not comprising the suppression DNA construct.

35. An isolated polynucleotide comprising a nucleic acid sequence encoding a NDK or NDK-like polypeptide having an amino acid sequence of at least 80% sequence identity, when compared to SEQ ID NO:25, or of at least 85% sequence identity, when compared to SEQ ID NO:23, or of at least 90%, when compared to SEQ ID NO: 21, or of at least 95%, when compared to SEQ ID NO:33, based on the Clustal V method of alignment, or a full complement of said nucleic acid sequence.
36. The polynucleotide of Claim 35, wherein the amino acid sequence of the polypeptide and the amino acid sequence of SEQ ID NO:25 have at least 85% sequence identity, or the amino acid sequence of SEQ ID NO:23 have at least 90% identity, or the amino acid sequence of SEQ ID NO:21 have at least 95% sequence identity, based on the Clustal alignment method.
37. The polynucleotide of Claim 35, wherein the amino acid sequence of the polypeptide and the amino acid sequence of SEQ ID NO:25 have at least 90% sequence identity, or the amino acid sequence of SEQ ID NO:23 have at least 95% identity, based on the Clustal alignment method.
38. The polynucleotide of Claim 35, wherein the amino acid sequence of the polypeptide and the amino acid sequence of SEQ ID NO:25 have at least 95% sequence identity, based on the Clustal alignment method.
39. The polynucleptide of Claim 35, wherein the amino acid sequence of the polypeptide comprises SEQ ID NO: 21, 23, 25, or 33.
40. The polynucleotide of Claim 35, wherein the nucleic acid sequences comprises SEQ ID NO: 20, 22, 24, or 32.
41. A vector comprising the polynucleotide of Claim 35.
42. A recombinant DNA construct comprising the polynucleotide of Claim 35 operably linked to at least one regulatory sequence.
43. A method for transforming a cell, comprising transforming a cell with the polynucleotide of Claim 35.
44. A cell comprising the recombinant DNA construct of Claim 42.
45. A method for producing a plant comprising transforming a plant cell with the polynucleotide of Claim 35 and regenerating a plant from the transformed plant cell.In further embodiments, vectors and recombinant constructs comprising any of the foregoing polynucleotides and cells comprising the recombinant constructs.

## Claims

1. A method of altering root architecture in a plant, comprising:

   (a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15; and
   (b) regenerating a transgenic plant from the regenerable plant cell after step (a), wherein the transgenic plant comprises in its genome the recombinant DNA construct and exhibits altered root architecture when compared to a control plant not comprising the recombinant DNA construct.

2. The method of claim 1, further comprising:

   (c) obtaining a progeny plant derived from the transgenic plant, wherein said progeny plant comprises in its genome the recombinant DNA construct and exhibits altered root architecture when compared to a control plant not comprising the recombinant DNA construct.

3. The method of claim 1, further comprising:

   (c) obtaining a progeny plant derived from the transgenic plant, wherein the progeny plant comprises in its genome the recombinant DNA construct; and
   (d) evaluating root architecture of the progeny plant compared to a control plant not comprising the recombinant DNA construct.

4. The method of claim 1, further comprising:

   (c) determining whether the transgenic plant exhibits an alteration of at least one agronomic characteristic when compared to a control plant not comprising the recombinant DNA construct; and optionally further comprising:
   (d) obtaining a progeny plant derived from the transgenic plant, wherein the progeny plant comprises in its genome the recombinant DNA construct; and

(e) determining whether the progeny plant exhibits an alteration of at least one agronomic characteristic when compared to a control plant not comprising the recombinant DNA construct.

**5.** A method of evaluating root architecture in a plant, comprising:

(a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15;
(b) regenerating a transgenic plant from the regenerable plant cell after step (a), wherein the transgenic plant comprises in its genome the recombinant DNA construct;
(c) obtaining a progeny plant derived from the transgenic plant, wherein the progeny plant comprises in its genome the recombinant DNA construct; and
(d) evaluating root architecture of the progeny plant compared to a control plant not comprising the recombinant DNA construct.

**6.** A method of determining an alteration of an agronomic characteristic in a plant, comprising:

(a) introducing into a regenerable plant cell a recombinant DNA construct comprising a polynucleotide operably linked to at least one regulatory sequence, wherein the polynucleotide encodes a polypeptide having an amino acid sequence of at least 50% sequence identity, based on the Clustal V method of alignment, when compared to SEQ ID NO: 15;
(b) regenerating a transgenic plant from the regenerable plant cell after step (a), wherein the transgenic plant comprises in its genome the recombinant DNA construct; and
(c) determining whether the transgenic plant exhibits an alteration of at least one agronomic characteristic when compared to a control plant not comprising the recombinant DNA construct.

**7.** The method of claim 6, further comprising:

(d) obtaining a progeny plant derived from the transgenic plant, wherein the progeny plant comprises in its genome the recombinant DNA construct; and
(e) determining whether the progeny plant exhibits an alteration of at least one agronomic characteristic when compared to a control plant not comprising the recombinant DNA construct.

**8.** The method of claim 7, wherein said determining step comprises determining whether the transgenic plant exhibits an alteration of at least one agronomic characteristic when compared, under varying environmental conditions, to a control plant not comprising the recombinant DNA construct.

**9.** The method of claim 8, wherein the varying environmental condition is at least one selected from drought, nitrogen or disease.

**10.** The method of any one of claims 1-9, wherein said polynucleotide encodes a polypeptide having an amino acid sequence of at least 95% sequence identity, based on the Clustal V method of alignment, when compared to SEQ ID NO: 15.

**11.** The method of any one of claims 1-10, wherein said plant is selected from the group consisting of: maize, soybean, sunflower, sorghum, canola, wheat, alfalfa, cotton, rice, barley and millet.

Fig.1

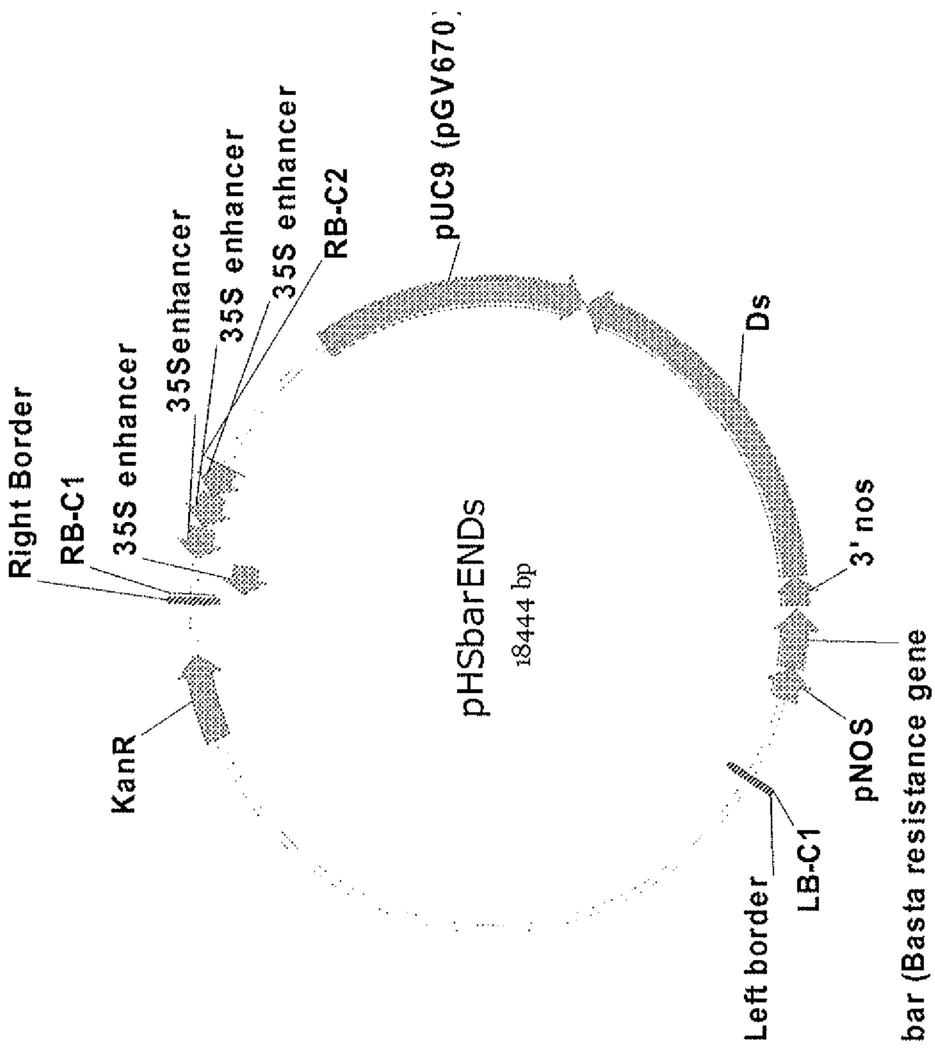
pHSbarENDs
18444 bp
Right Border
RB-C1
35S enhancer
35Senhancer
35S enhancer
35S enhancer
RB-C2
pUC9 (pGV670)
Ds
3' nos
bar (Basta resistance gene
pNOS
LB-C1
Left border
KanR

Fig.2

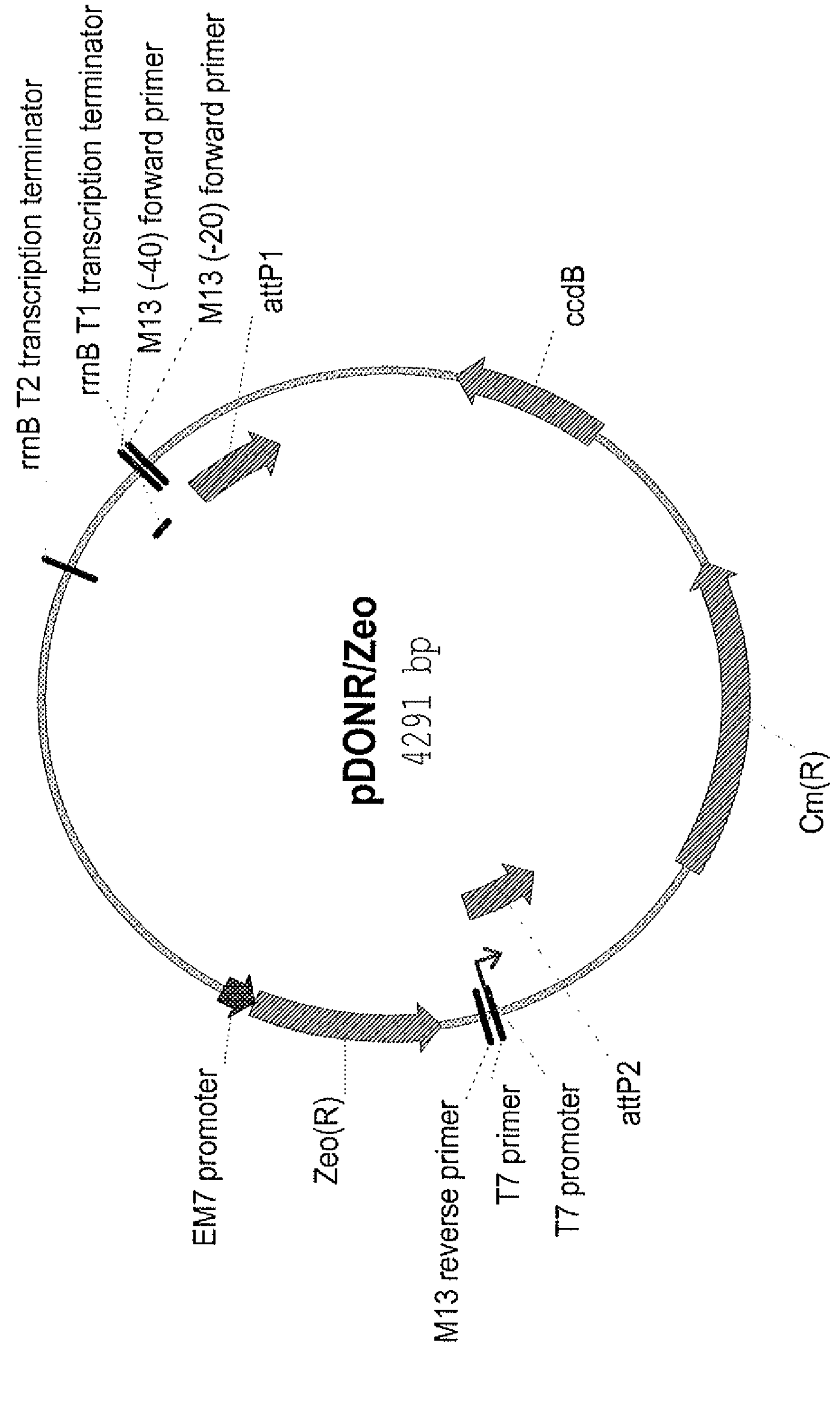
pDONR/Zeo
4291 bp
rrnB T2 transcription terminator
rrnB T1 transcription terminator
M13 (-40) forward primer
M13 (-20) forward primer
attP1
ccdB
Cm(R)
attP2
T7 promoter
T7 primer
M13 reverse primer
Zeo(R)
EM7 promoter

Fig.3

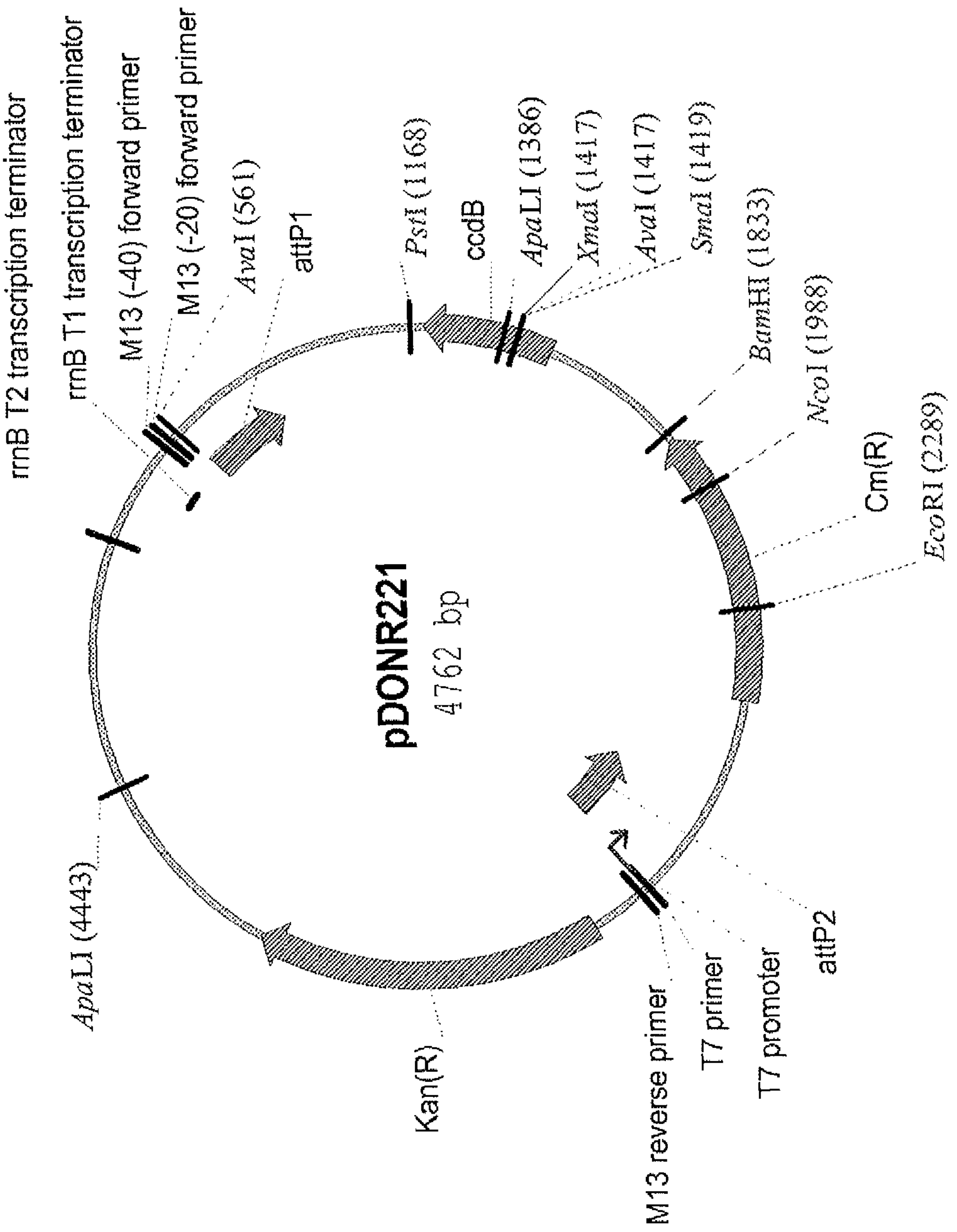
pDONR221
4762 bp
rrnB T2 transcription terminator
rrnB T1 transcription terminator
M13 (-40) forward primer
M13 (-20) forward primer
AvaI (561)
attP1
PstI (1168)
ccdB
ApaLI (1386)
XmaI (1417)
AvaI (1417)
SmaI (1419)
BamHI (1833)
NcoI (1988)
Cm(R)
EcoRI (2289)
attP2
T7 promoter
T7 primer
M13 reverse primer
Kan(R)
ApaLI (4443)

Fig.4

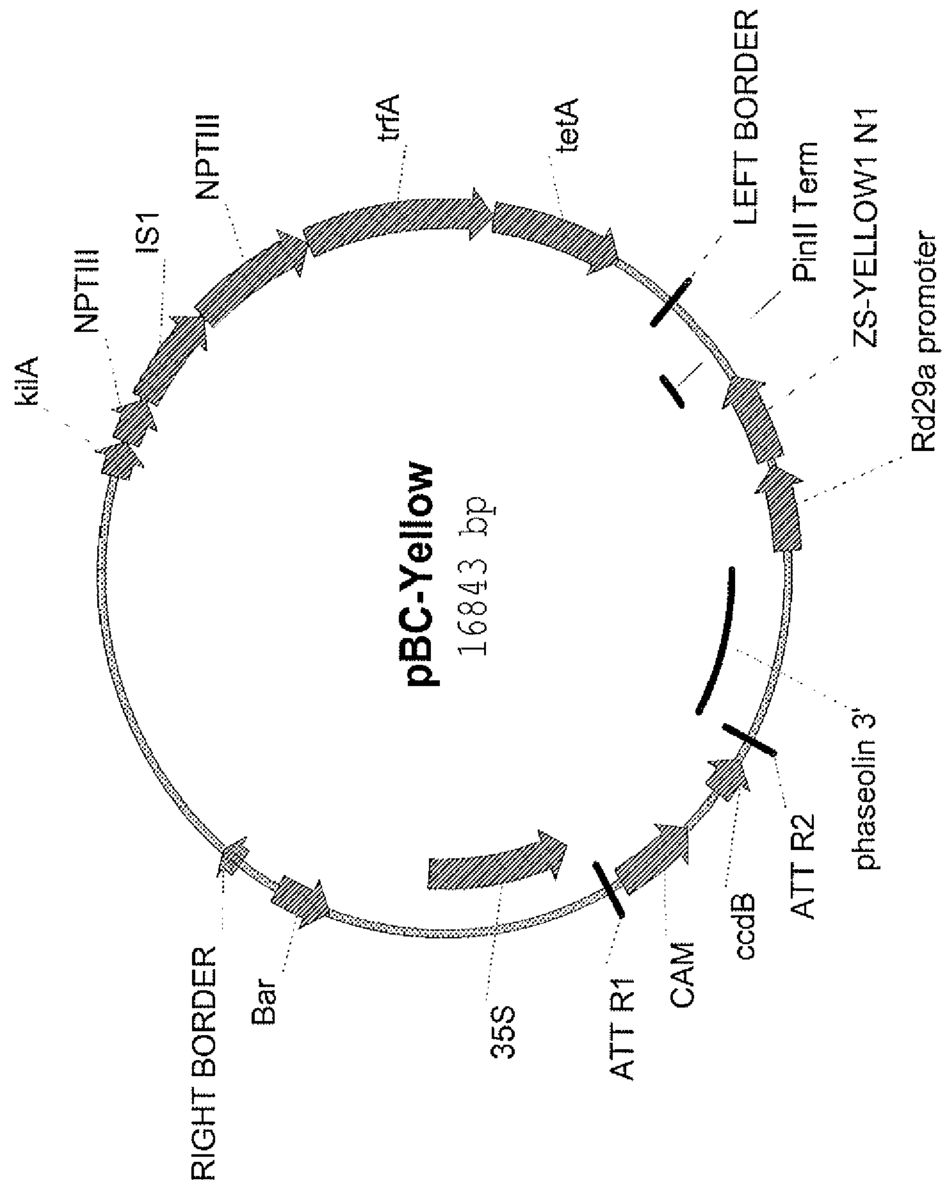
pBC-Yellow
16843 bp
RIGHT BORDER
Bar
35S
ATT R1
CAM
ccdB
ATT R2
phaseolin 3'
Rd29a promoter
ZS-YELLOW1 N1
PinII Term
LEFT BORDER
tetA
trfA
NPTIII
IS1
NPTIII
kilA

FIG. 5

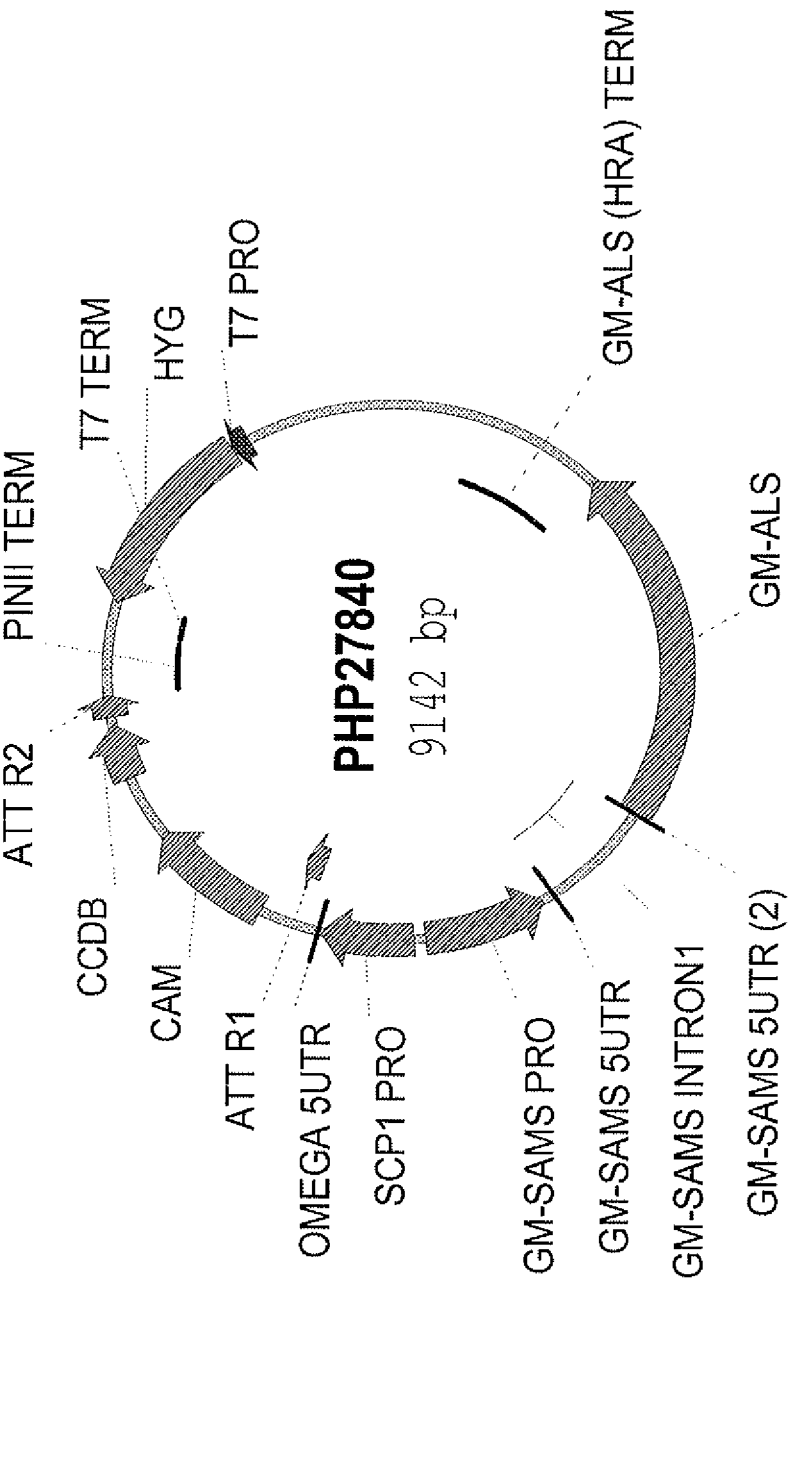
PHP27840
9142 bp
T7 PRO
HYG
T7 TERM
PINII TERM
ATT R2
CCDB
CAM
ATT R1
OMEGA 5UTR
SCP1 PRO
GM-SAMS PRO
GM-SAMS 5UTR
GM-SAMS INTRON1
GM-SAMS 5UTR (2)
GM-ALS
GM-ALS (HRA) TERM

Fig.6

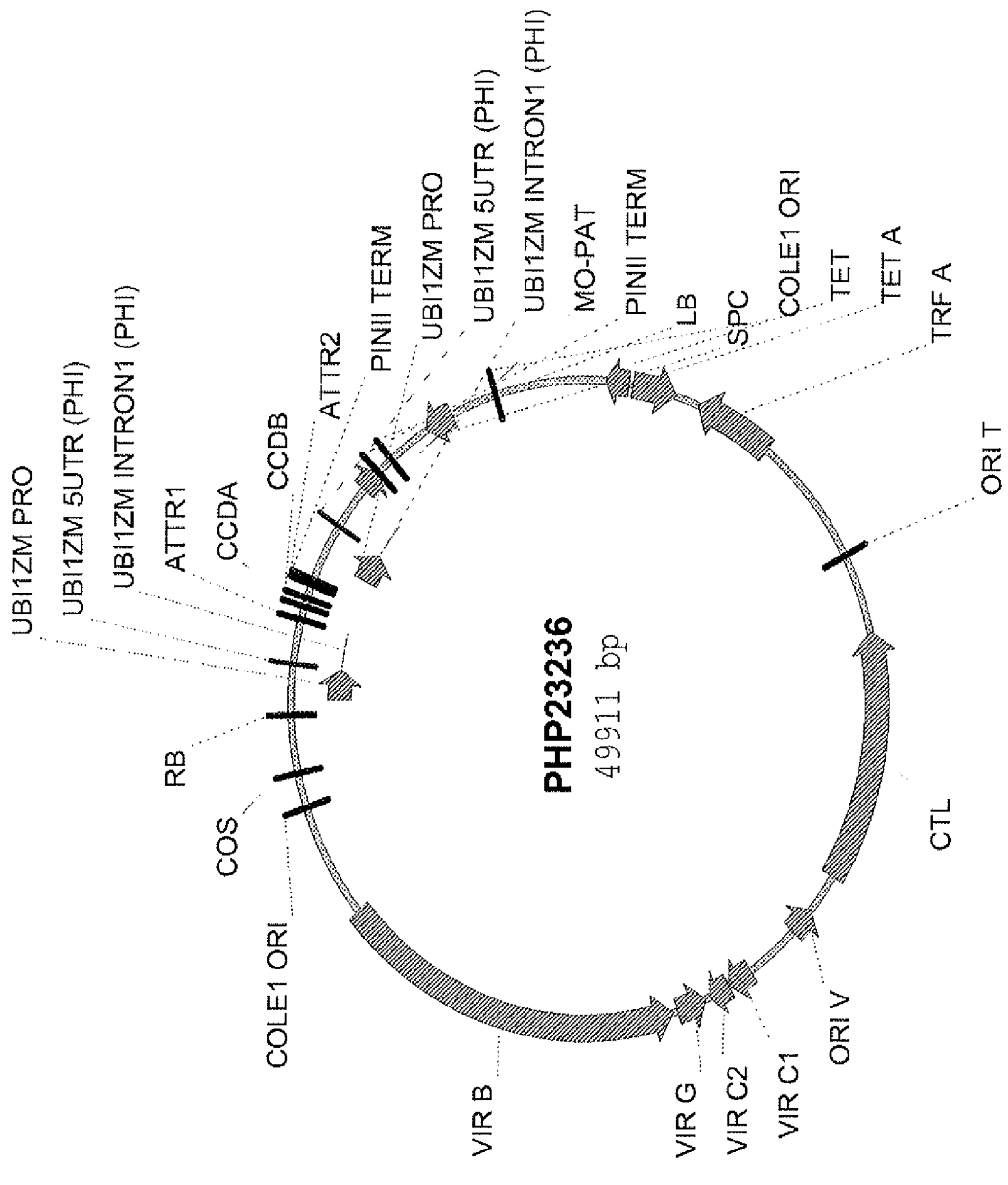
PHP23236
49911 bp
UBI1ZM PRO
UBI1ZM 5UTR (PHI)
UBI1ZM INTRON1 (PHI)
ATTR1
CCDA
CCDB
ATTR2
PINII TERM
UBI1ZM PRO
UBI1ZM 5UTR (PHI)
UBI1ZM INTRON1 (PHI)
MO-PAT
PINII TERM
LB
SPC
COLE1 ORI
TET
TET A
TRF A
ORI T
CTL
ORI V
VIR C1
VIR C2
VIR G
VIR B
COLE1 ORI
COS
RB

Fig.7

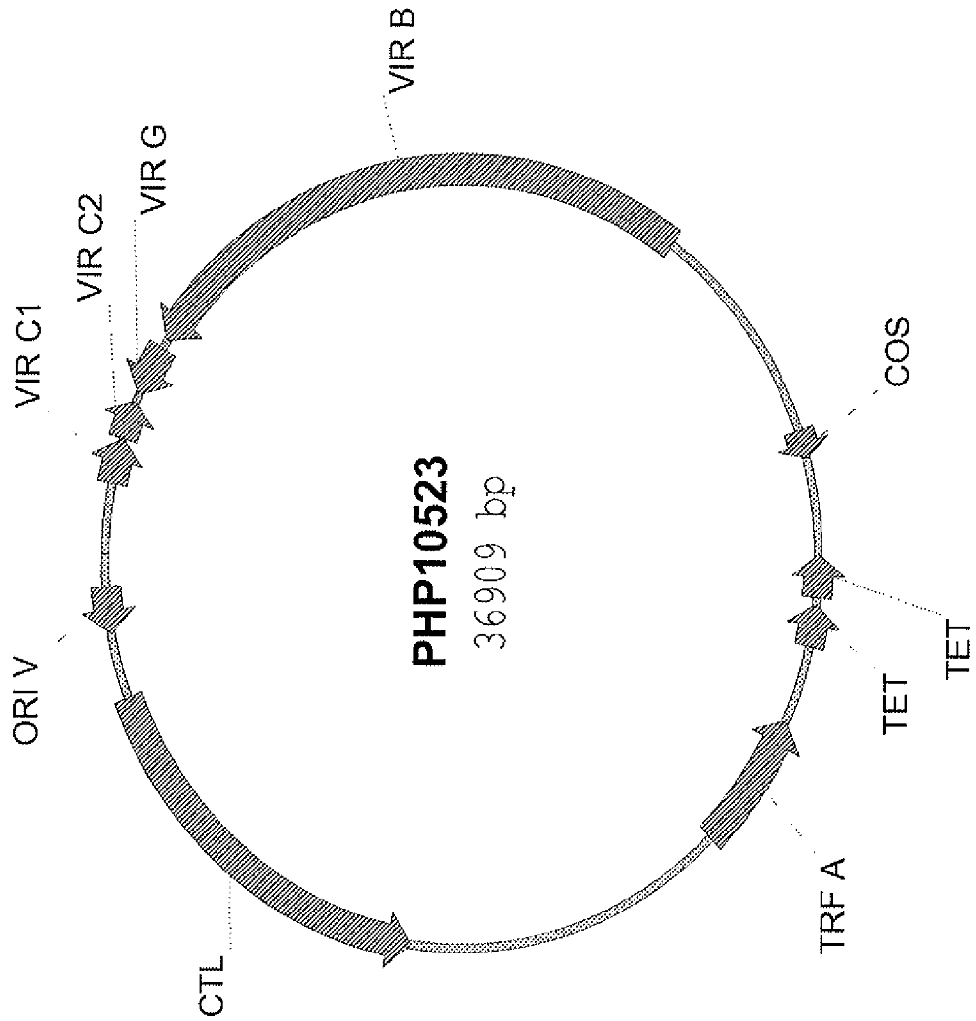
PHP10523
36909 bp
VIR B
VIR G
VIR C2
VIR C1
ORI V
CTL
TRF A
TET
TET
COS

Fig.8

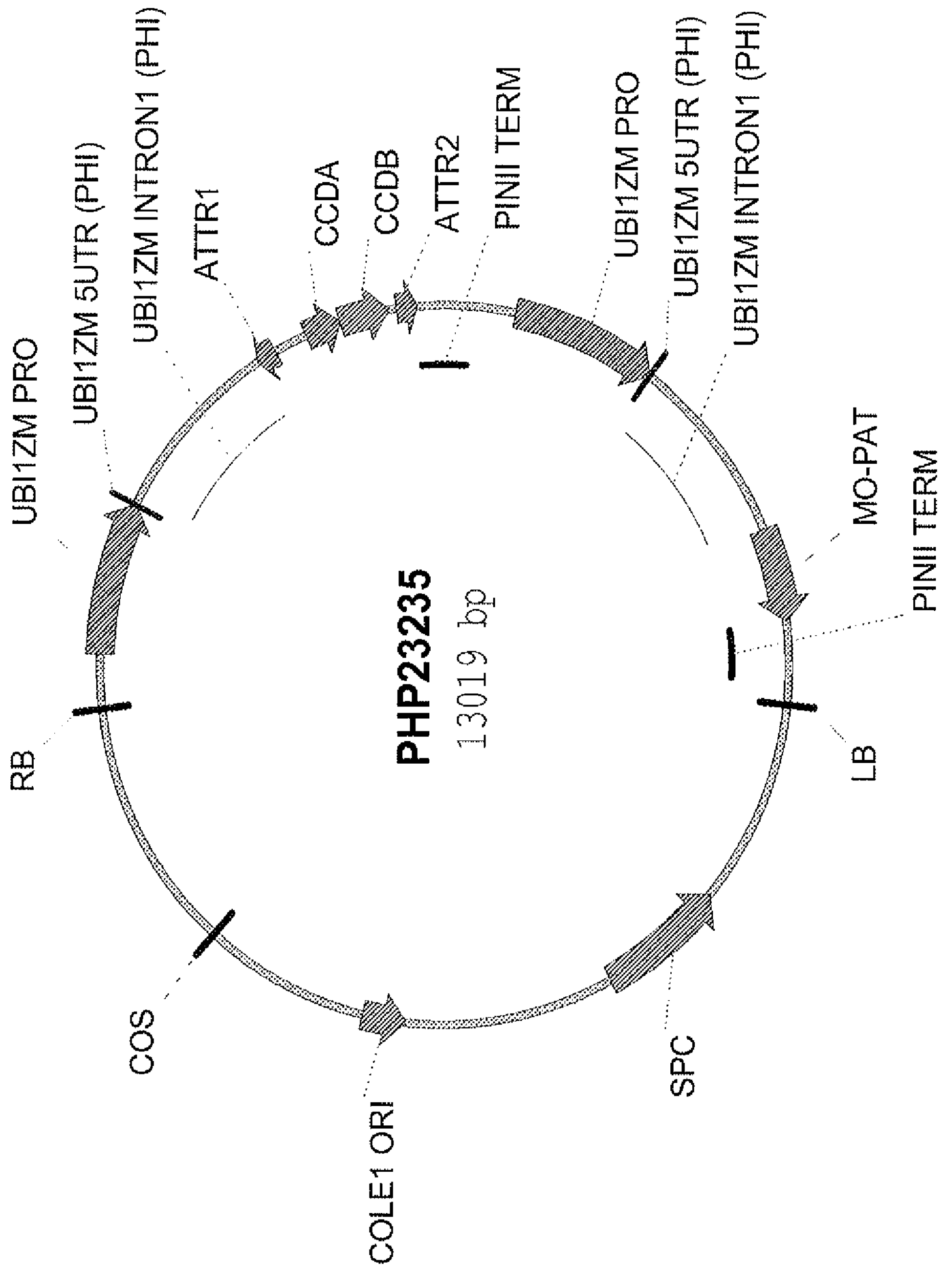
PHP23235
13019 bp
RB
UBI1ZM PRO
UBI1ZM 5UTR (PHI)
UBI1ZM INTRON1 (PHI)
ATTR1
CCDA
CCDB
ATTR2
PINII TERM
UBI1ZM PRO
UBI1ZM 5UTR (PHI)
UBI1ZM INTRON1 (PHI)
MO-PAT
PINII TERM
LB
SPC
COLE1 ORI
COS

Fig.9

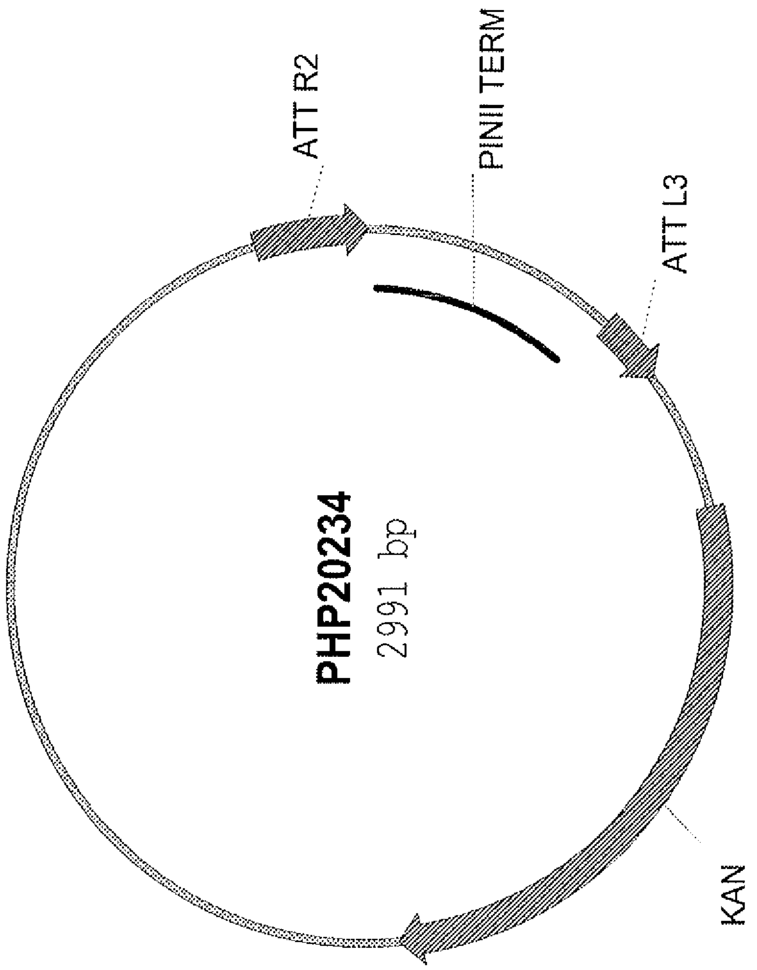

Fig.10

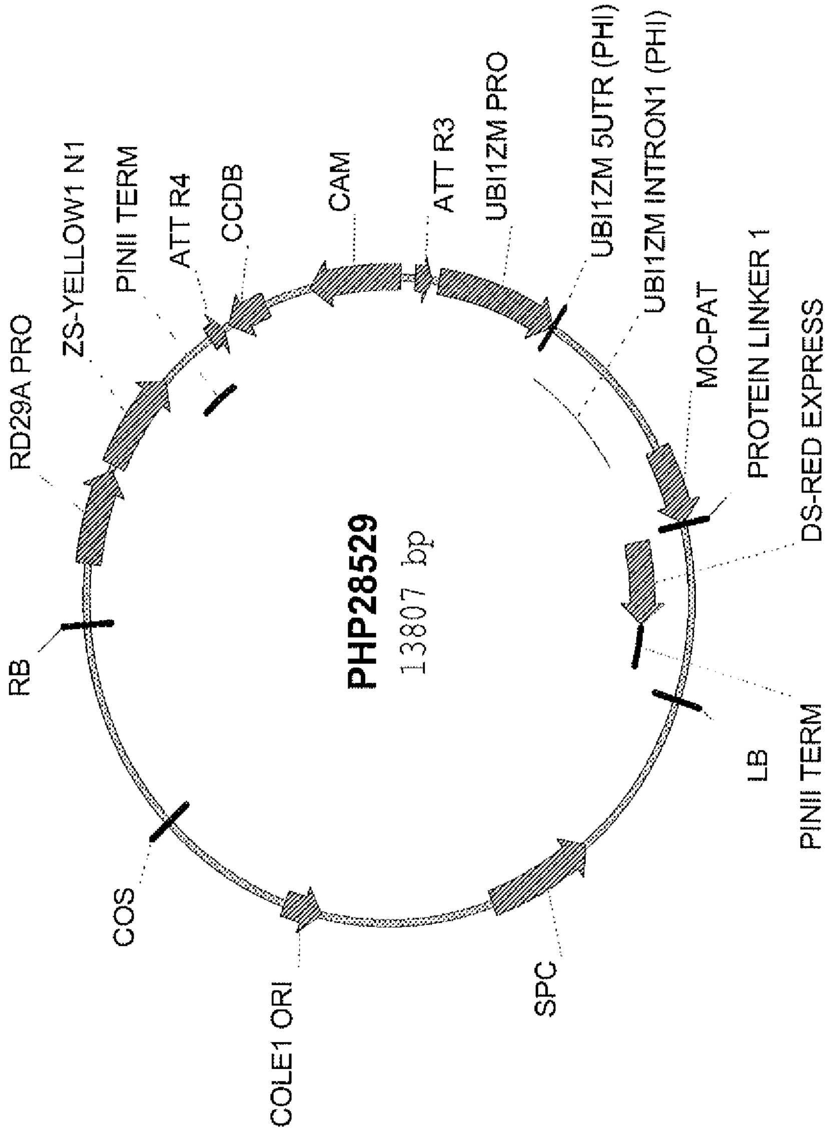
PHP28529
13807 bp
RB
RD29A PRO
ZS-YELLOW1 N1
PINII TERM
ATT R4
CCDB
CAM
ATT R3
UBI1ZM PRO
UBI1ZM 5UTR (PHI)
UBI1ZM INTRON1 (PHI)
MO-PAT
PROTEIN LINKER 1
DS-RED EXPRESS
PINII TERM
LB
SPC
COLE1 ORI
COS

Fig.11

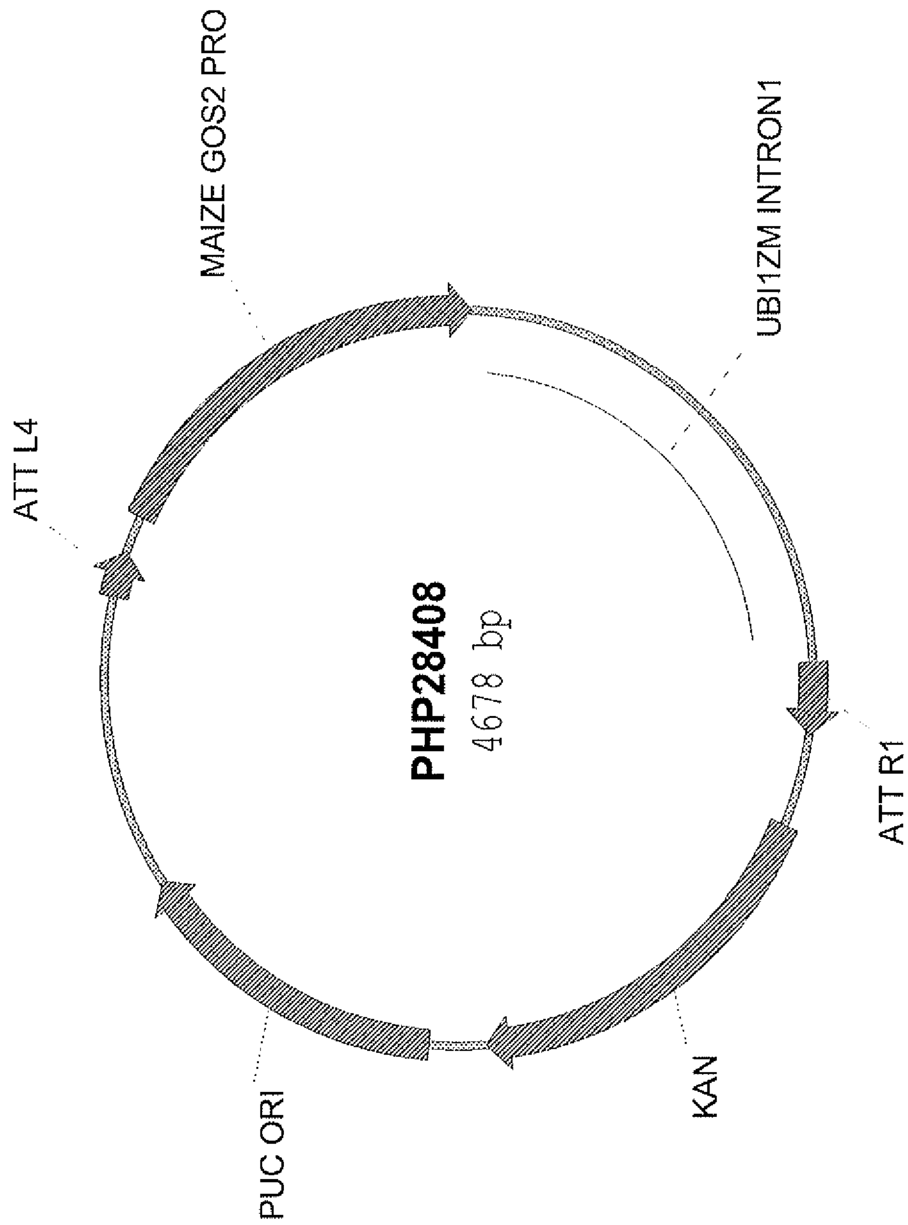
PHP28408
4678 bp
MAIZE GOS2 PRO
UBI1ZM INTRON1
ATT L4
ATT R1
KAN
PUC ORI

Fig.12

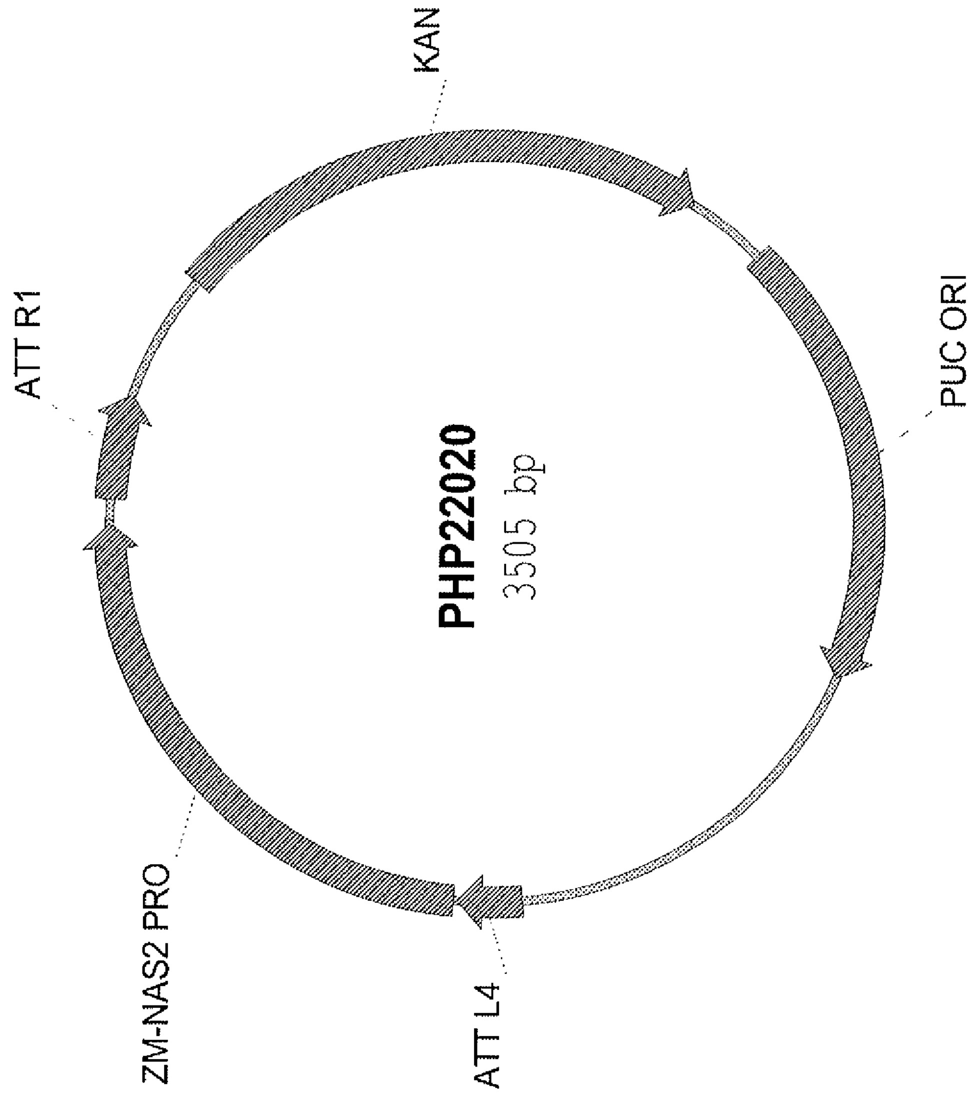

Fig.13

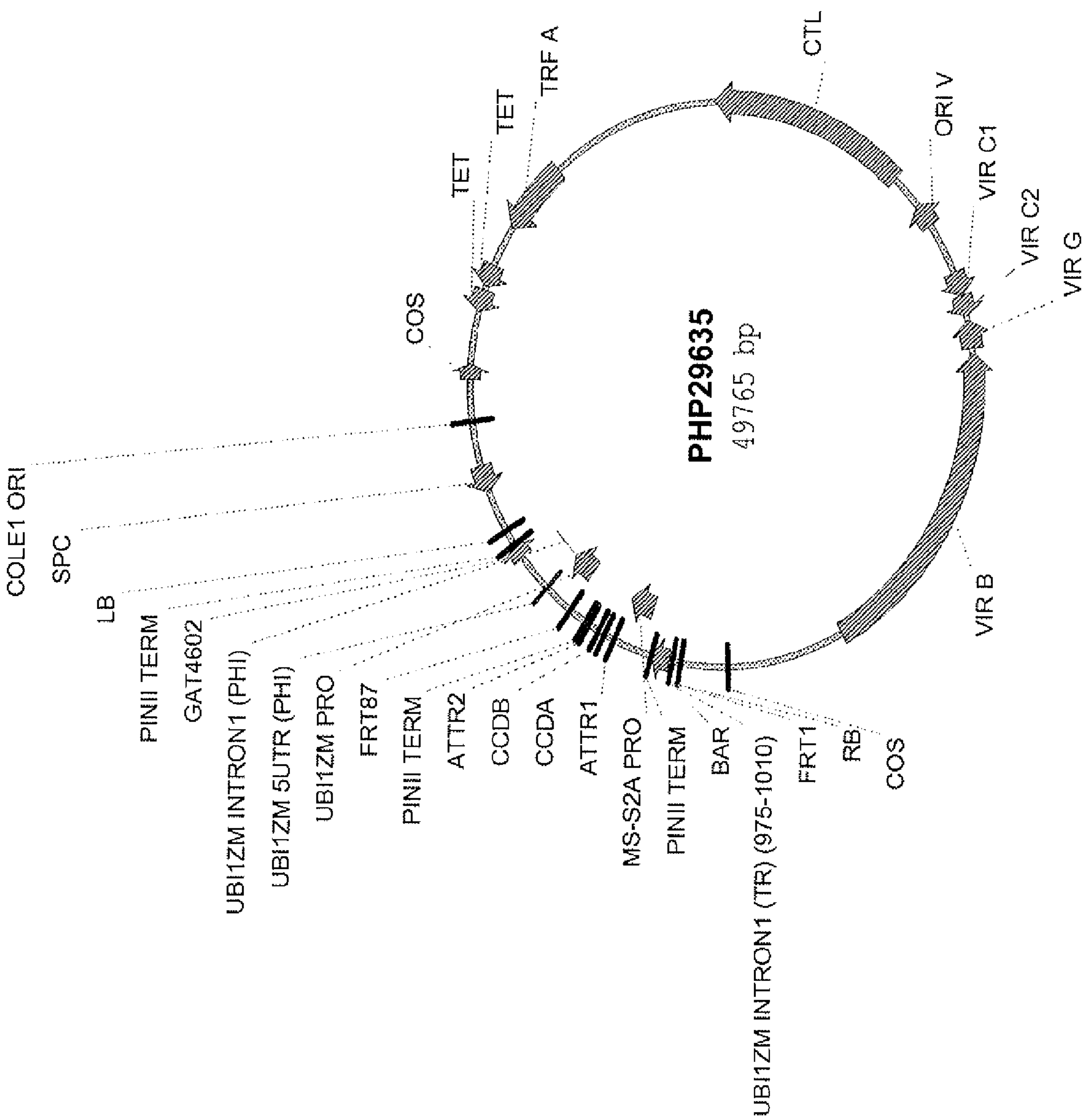
PHP29635
49765 bp
CTL
TRF A
TET
TET
COS
ORI V
VIR C1
VIR C2
VIR G
VIR B
COLE1 ORI
SPC
LB
PINII TERM
GAT4602
UBI1ZM INTRON1 (PHI)
UBI1ZM 5UTR (PHI)
UBI1ZM PRO
FRT87
PINII TERM
ATTR2
CCDB
CCDA
ATTR1
MS-S2A PRO
PINII TERM
BAR
UBI1ZM INTRON1 (TR) (975-1010)
FRT1
RB
COS

Fig.14

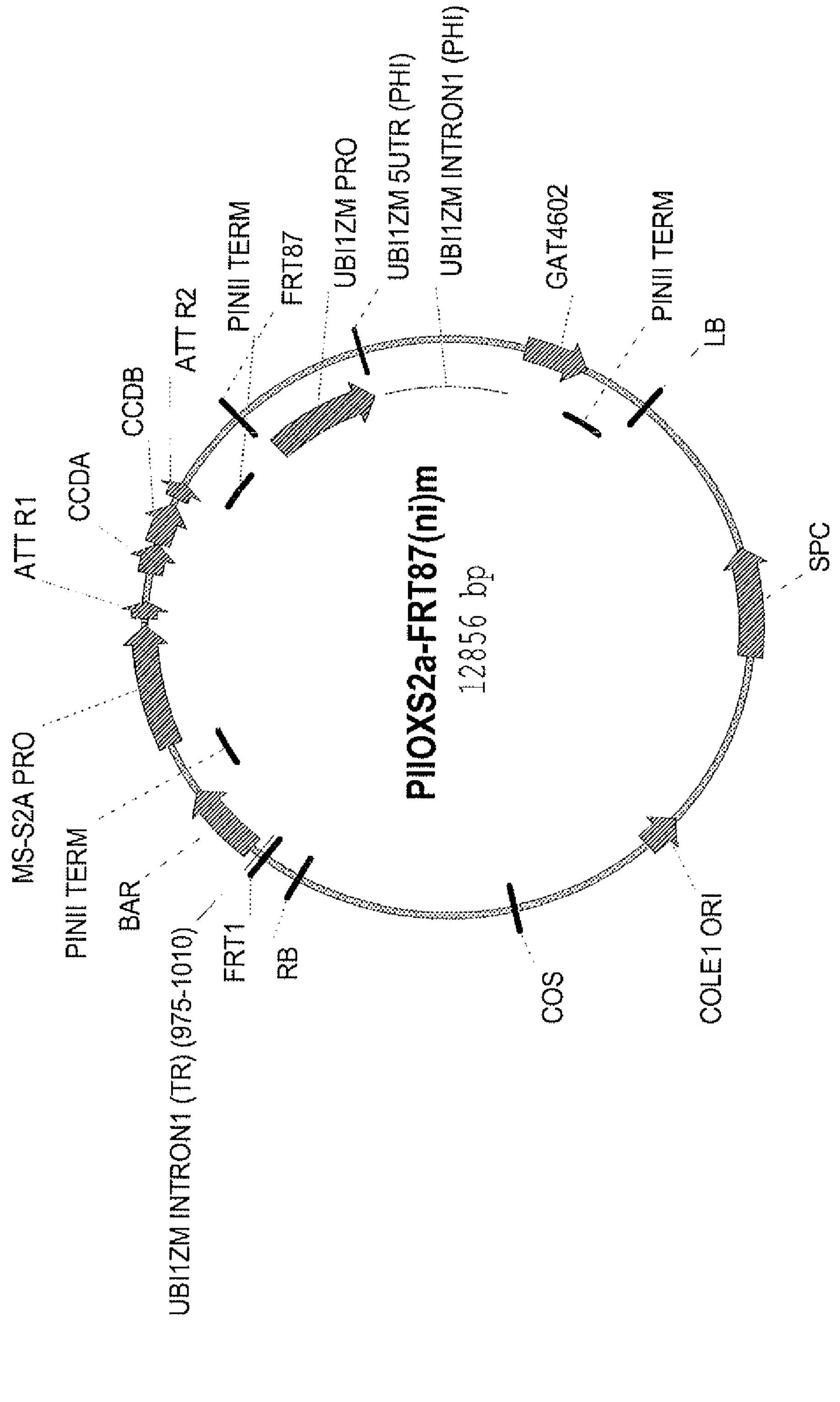
PIIOXS2a-FRT87(ni)m
12856 bp
ATT R1
CCDA
CCDB
ATT R2
PINII TERM
FRT87
UBI1ZM PRO
UBI1ZM 5UTR (PHI)
UBI1ZM INTRON1 (PHI)
GAT4602
PINII TERM
LB
SPC
COLE1 ORI
COS
RB
FRT1
UBI1ZM INTRON1 (TR) (975-1010)
BAR
PINII TERM
MS-S2A PRO

# Fig.15A

| | | | | | | | | | | | | | | | | | | | | | | | | | | |
|---|---|---|---|---|---|---|---|---|---|---|---|---|---|---|---|---|---|---|---|---|---|---|---|---|---|---|
| | M | S | S | - | - | - | - | Q | L | C | Q | S | A | S | R | A | A | R | S | L | L | S | A | A | - | Majority |
| | | | | | | | | | | 10 | | | | | | | | | | 20 | | | | | | |
| 1 | M | S | G | S | A | S | R | K | L | F | Q | - | - | - | - | A | A | R | S | I | V | - | L | S | A | SEQ ID NO 15 |
| 1 | M | S | G | S | A | S | R | K | L | F | Q | - | - | - | - | A | A | R | S | T | V | - | L | S | A | SEQ ID NO 17 |
| 1 | M | S | A | - | A | S | K | K | L | F | Q | - | - | - | - | A | A | R | S | L | V | - | L | S | A | SEQ ID NO 19 |
| 1 | M | A | S | - | - | - | - | Q | V | C | K | S | A | S | R | A | A | R | S | L | L | S | A | - | S | SEQ ID NO 21 |
| 1 | M | R | S | - | - | - | - | Q | I | C | R | S | A | - | - | - | - | R | S | L | L | S | A | A | S | SEQ ID NO 23 |
| 1 | M | S | S | S | - | - | - | K | M | Y | Q | S | A | C | K | A | A | R | S | L | I | - | - | - | - | SEQ ID NO 25 |
| 1 | M | S | S | - | - | - | - | Q | I | F | R | S | A | S | R | A | A | R | S | I | V | S | S | A | S | SEQ ID NO 27 |
| 1 | - | A | S | - | - | - | - | Q | V | C | K | S | A | S | R | A | A | R | S | L | L | S | S | - | - | SEQ ID NO 29 |
| 1 | M | S | S | - | - | - | - | Q | I | F | R | S | A | S | R | A | A | R | S | L | L | S | - | A | S | SEQ ID NO 31 |
| 1 | M | A | S | - | - | - | - | Q | V | C | K | A | A | S | R | A | A | R | S | L | L | S | A | - | - | SEQ ID NO 33 |
| 1 | M | S | A | - | A | S | K | K | L | F | Q | - | - | - | - | A | A | R | S | L | V | - | L | S | A | SEQ ID NO 35 |
| 1 | M | - | - | - | - | - | - | - | - | - | - | - | - | - | - | - | - | - | - | - | - | - | - | - | - | SEQ ID NO 37 |
| 1 | M | - | - | S | - | - | - | K | L | C | Q | S | A | C | K | A | A | K | S | L | L | S | A | T | A | SEQ ID NO 44 |
| 1 | M | A | S | - | - | - | - | H | L | C | K | S | A | S | R | A | A | R | S | L | L | S | A | - | S | SEQ ID NO 45 |
| 1 | M | S | S | - | - | - | - | Q | I | C | R | S | A | S | K | A | A | K | S | L | L | S | S | A | - | SEQ ID NO 46 |
| 1 | M | S | S | - | - | - | - | Q | I | C | R | S | A | S | R | A | A | R | S | L | L | S | - | A | S | SEQ ID NO 47 |
| 1 | M | S | S | - | - | - | - | Q | I | C | R | S | A | S | K | A | A | R | S | L | L | S | S | A | - | SEQ ID NO 48 |
| 1 | M | - | - | S | - | - | - | K | L | C | Q | S | A | C | K | A | A | K | S | L | L | S | A | T | A | SEQ ID NO 49 |
| 1 | M | S | S | - | - | - | - | Q | I | C | R | S | A | S | R | A | A | R | S | L | L | S | S | A | - | SEQ ID NO 51 |

# Fig.15B

| | | | | | 30 | | | | | | | | | | 40 | | | | | | | | | | 50 | |
|---|---|---|---|---|---|---|---|---|---|---|---|---|---|---|---|---|---|---|---|---|---|---|---|---|---|---|
| | K | A | S | - | R | F | - | - | - | A | S | E | G | R | A | A | A | A | A | A | A | V | S | L | G | Majority |
| 21 | S | Q | R | S | S | F | S | V | L | A | A | E | G | R | T | A | A | L | A | - | - | - | N | F | G | SEQ ID NO 1 |
| 21 | S | Q | R | S | S | F | S | V | L | A | A | E | G | R | T | A | A | L | A | - | - | - | N | F | G | SEQ ID NO 1 |
| 20 | S | H | R | S | - | - | - | V | L | A | A | E | G | R | T | A | A | L | A | T | L | T | N | F | G | SEQ ID NO 1 |
| 21 | K | A | S | - | R | F | - | - | - | Y | S | Q | G | - | - | - | S | A | A | A | A | V | T | L | G | SEQ ID NO 2 |
| 18 | K | Q | S | S | R | S | - | - | - | F | S | S | G | G | R | A | A | A | A | A | A | V | S | G | R | SEQ ID NO 2 |
| 19 | A | S | S | S | A | R | S | A | V | L | A | D | G | R | N | A | A | L | A | T | L | T | N | L | G | SEQ ID NO 2 |
| 22 | K | Q | K | - | R | L | - | - | - | F | S | E | G | R | S | V | A | A | A | T | A | V | S | V | K | SEQ ID NO 2 |
| 19 | - | - | - | - | R | F | - | - | - | H | S | Q | G | R | A | L | G | A | A | A | A | V | S | L | S | SEQ ID NO 2 |
| 21 | K | A | S | - | R | F | - | - | - | Y | S | E | G | R | A | V | A | A | A | A | A | V | S | L | G | SEQ ID NO 3 |
| 20 | - | - | - | - | S | R | - | - | - | A | S | H | G | R | A | V | G | A | A | T | A | V | S | L | S | SEQ ID NO 3 |
| 20 | S | N | R | S | - | - | - | V | L | A | A | E | G | R | T | A | A | L | A | T | L | T | N | F | G | SEQ ID NO 3 |
| 2 | - | - | - | - | - | - | - | - | - | - | - | - | - | - | - | - | - | - | - | - | - | - | - | - | - | SEQ ID NO 3 |
| 21 | A | A | S | S | P | R | T | S | L | L | A | E | G | R | N | A | A | L | A | T | L | T | N | L | G | SEQ ID NO 4 |
| 21 | - | - | - | - | - | F | - | - | - | H | S | Q | G | R | A | V | A | A | A | A | A | V | A | S | I | SEQ ID NO 4 |
| 21 | K | N | A | - | R | F | - | - | - | F | S | E | G | R | A | I | G | A | A | A | A | V | S | A | S | SEQ ID NO 4 |
| 21 | K | S | S | - | N | L | - | - | - | L | A | E | G | R | A | V | A | A | V | A | A | L | S | S | R | SEQ ID NO 4 |
| 21 | R | N | V | - | R | F | - | - | - | F | S | E | G | R | A | I | G | A | A | A | A | V | S | A | S | SEQ ID NO 4 |
| 21 | A | A | S | S | Q | R | T | S | L | L | A | - | - | - | - | - | - | - | - | - | - | - | - | - | - | SEQ ID NO 4 |
| 21 | K | N | A | - | R | F | - | - | - | F | S | E | G | R | A | I | G | A | A | S | V | V | H | A | T | SEQ ID NO 5 |

# Fig.15C

| | | | | | | | | | | | | | | | | | | | | | | | | | | |
|---|---|---|---|---|---|---|---|---|---|---|---|---|---|---|---|---|---|---|---|---|---|---|---|---|---|---|
| | R | K | V | - | - | P | A | Y | A | S | - | Y | G | R | S | - | S | - | - | - | - | - | S | - | - | Majority |
| | | | | | | | | | | 60 | | | | | | | | | | 70 | | | | | | |
| 43 | R | K | I | L | P | S | A | C | - | - | S | Y | H | R | Q | G | S | H | - | - | - | - | - | - | - | SEQ ID NO 15 |
| 43 | R | K | I | L | P | S | A | C | - | - | S | Y | H | R | Q | G | S | H | - | - | - | - | - | - | - | SEQ ID NO 17 |
| 42 | R | K | T | L | P | T | A | Y | - | L | S | Y | H | K | Q | G | S | H | H | - | - | - | - | - | - | SEQ ID NO 19 |
| 39 | R | K | V | - | - | P | F | Y | A | S | N | Y | G | R | S | G | S | R | S | G | S | A | A | - | - | SEQ ID NO 21 |
| 40 | G | R | W | - | - | S | S | L | A | S | - | Y | A | - | - | - | - | - | - | - | - | A | S | G | N | SEQ ID NO 23 |
| 44 | R | T | R | I | P | A | A | Y | S | - | S | Y | H | T | N | A | G | A | A | - | - | - | - | - | - | SEQ ID NO 25 |
| 43 | G | V | L | - | - | P | A | L | A | S | - | F | G | R | D | - | - | - | - | - | - | H | S | G | N | SEQ ID NO 27 |
| 37 | S | K | V | - | - | P | L | F | - | - | - | Y | G | K | N | G | S | G | N | G | S | A | S | S | S | SEQ ID NO 29 |
| 42 | G | K | V | - | - | P | L | L | A | S | A | Y | G | S | T | - | - | - | - | - | - | A | S | A | N | SEQ ID NO 31 |
| 38 | C | K | V | - | - | P | L | F | - | - | - | Y | G | K | N | G | S | G | S | G | S | A | S | S | S | SEQ ID NO 33 |
| 42 | R | K | T | L | P | T | A | Y | - | L | S | Y | H | K | Q | G | S | H | H | - | - | - | - | - | - | SEQ ID NO 35 |
| 2 | - | - | - | - | - | - | - | - | - | - | - | - | - | - | - | - | - | - | - | - | - | - | - | - | - | SEQ ID NO 37 |
| 46 | R | K | T | L | P | T | A | Y | A | Y | S | Y | H | H | N | S | S | A | A | - | - | - | - | - | - | SEQ ID NO 44 |
| 38 | R | K | V | - | - | P | V | F | A | P | N | Y | R | R | T | G | S | G | N | G | P | S | S | - | - | SEQ ID NO 45 |
| 42 | G | K | I | - | - | P | L | Y | A | S | N | F | A | R | S | - | - | - | - | - | S | G | S | G | V | SEQ ID NO 46 |
| 42 | G | K | - | - | - | P | F | L | S | S | - | F | G | N | A | - | - | - | - | - | - | G | S | G | N | SEQ ID NO 47 |
| 42 | G | K | I | - | - | P | L | Y | A | S | N | F | A | R | S | - | - | - | - | - | S | G | S | G | A | SEQ ID NO 48 |
| 32 | - | - | - | - | - | - | - | - | - | - | - | - | - | - | - | - | - | - | - | - | - | - | - | - | - | SEQ ID NO 49 |
| 42 | G | K | V | - | - | P | Q | Y | A | S | N | F | G | K | S | - | - | - | - | - | - | G | S | G | F | SEQ ID NO 51 |

# Fig.15D

| | | | | | | | | | | | | | | | | | | | | | | | | | | |
|---|---|---|---|---|---|---|---|---|---|---|---|---|---|---|---|---|---|---|---|---|---|---|---|---|---|---|
| | A | S | S | G | W | I | A | G | A | L | A | L | P | A | A | A | Y | M | L | Q | D | Q | E | A | H | Majority |
| | | | | | 80 | | | | | | | | | | 90 | | | | | | | | | | 100 | |
| 59 | A | A | S | G | W | G | A | I | A | A | A | V | P | A | A | V | Y | M | L | Q | D | Q | E | A | H | SEQ ID NO 15 |
| 59 | A | A | S | G | W | G | A | I | A | A | A | V | P | A | A | V | Y | M | L | Q | D | Q | E | A | H | SEQ ID NO 17 |
| 60 | A | A | S | G | W | G | A | I | A | A | A | V | P | A | A | V | Y | M | L | Q | D | Q | E | A | H | SEQ ID NO 19 |
| 60 | - | - | - | - | W | I | P | G | A | L | A | L | P | A | A | A | Y | M | F | Q | D | Q | E | V | H | SEQ ID NO 21 |
| 54 | A | N | S | G | W | M | T | G | L | L | A | L | P | T | A | A | F | M | L | Q | E | Q | E | A | H | SEQ ID NO 23 |
| 62 | R | G | Y | G | W | - | - | - | I | A | G | I | P | A | A | A | Y | M | L | Q | D | Q | E | V | H | SEQ ID NO 25 |
| 59 | S | S | S | T | W | I | A | G | A | L | A | L | P | A | A | A | Y | M | L | Q | D | Q | E | A | H | SEQ ID NO 27 |
| 57 | S | S | S | S | W | I | S | G | A | L | A | L | P | A | A | A | Y | M | F | Q | D | Q | E | V | Q | SEQ ID NO 29 |
| 59 | A | S | R | A | W | L | S | G | V | F | A | L | P | V | A | A | Y | M | L | Q | E | Q | E | V | H | SEQ ID NO 31 |
| 58 | S | S | S | - | W | I | S | G | A | L | A | L | P | A | A | A | Y | M | F | Q | D | Q | E | V | R | SEQ ID NO 33 |
| 60 | A | A | S | G | W | G | A | I | A | A | A | V | P | A | A | V | Y | M | L | Q | D | Q | E | A | H | SEQ ID NO 35 |
| 2 | - | - | - | - | - | - | - | - | - | - | - | - | - | - | - | - | - | - | - | - | - | - | - | - | - | SEQ ID NO 37 |
| 65 | A | A | - | G | W | - | - | - | L | A | A | I | P | A | A | V | Y | M | L | Q | D | Q | E | A | H | SEQ ID NO 44 |
| 59 | - | - | - | - | W | I | A | G | A | L | A | L | P | A | A | A | Y | M | L | Q | D | Q | E | V | H | SEQ ID NO 45 |
| 60 | A | S | K | S | W | I | T | G | L | L | A | L | P | A | A | A | Y | M | I | Q | D | Q | E | V | L | SEQ ID NO 46 |
| 57 | A | Y | R | G | W | L | S | S | V | L | A | L | P | A | A | A | Y | M | M | Q | E | Q | E | L | H | SEQ ID NO 47 |
| 60 | A | S | K | N | W | L | T | G | L | I | A | L | P | A | A | A | L | M | L | Q | D | Q | E | V | L | SEQ ID NO 48 |
| 32 | - | - | - | - | - | - | - | - | - | - | - | - | - | - | - | V | Y | M | L | Q | D | Q | E | A | H | SEQ ID NO 49 |
| 59 | V | S | N | S | W | I | T | G | L | L | A | L | P | A | A | A | F | M | L | Q | D | Q | E | A | L | SEQ ID NO 51 |

Fig.15E

| | | | | | | | | | | | | | | | | | | | | | | | | | |
|---|---|---|---|---|---|---|---|---|---|---|---|---|---|---|---|---|---|---|---|---|---|---|---|---|---|
| | A | A | E | M | E | R | T | F | I | A | I | K | P | D | G | V | Q | R | G | L | I | S | E | I | I | Majority |
| | | | | | | | | | | 110 | | | | | | | | | | 120 | | | | | | |
| 84 | A | A | E | M | E | R | T | F | I | A | I | K | P | D | G | V | Q | R | G | L | I | S | E | I | V | SEQ ID NO 1 |
| 84 | A | A | E | M | E | R | T | F | I | A | I | K | P | D | G | V | Q | R | G | L | I | S | E | I | V | SEQ ID NO 1 |
| 85 | A | A | E | M | E | R | T | F | I | A | I | K | P | D | G | V | Q | R | G | L | I | S | E | I | M | SEQ ID NO 1 |
| 81 | A | A | E | L | E | R | T | F | I | A | I | K | P | D | G | V | Q | R | G | L | I | A | E | I | L | SEQ ID NO 2 |
| 79 | A | A | E | M | E | R | T | F | I | A | I | K | P | D | G | V | Q | R | G | L | I | S | E | I | I | SEQ ID NO 2 |
| 84 | A | A | E | L | E | R | T | F | I | A | I | K | P | D | G | V | Q | R | G | L | I | S | E | I | V | SEQ ID NO 2 |
| 84 | A | A | Q | M | E | R | T | F | I | A | I | K | P | D | G | V | Q | R | G | L | I | S | E | I | I | SEQ ID NO 2 |
| 82 | A | A | E | L | E | R | T | F | I | A | I | K | P | D | G | V | Q | R | G | L | I | S | E | I | I | SEQ ID NO 2 |
| 84 | A | A | E | M | E | R | T | F | I | A | I | K | P | D | G | V | Q | R | G | L | I | S | E | I | I | SEQ ID NO 3 |
| 82 | A | A | E | L | E | R | T | F | I | A | I | K | P | D | G | V | Q | R | G | L | I | S | E | I | I | SEQ ID NO 3 |
| 85 | A | A | E | M | E | R | T | F | I | A | I | K | P | D | G | V | Q | R | G | L | I | S | E | I | M | SEQ ID NO 3 |
| 2 | - | - | - | - | E | R | T | F | I | A | I | K | P | D | G | V | Q | R | G | L | I | S | E | I | V | SEQ ID NO 3 |
| 86 | A | A | E | M | E | R | T | F | I | A | I | K | P | D | G | V | Q | R | G | L | I | S | E | I | L | SEQ ID NO 4 |
| 80 | A | A | E | L | E | R | T | F | I | A | I | K | P | D | G | V | Q | R | G | L | I | S | E | I | I | SEQ ID NO 4 |
| 85 | A | A | E | M | E | R | T | F | I | A | I | K | P | D | G | V | Q | R | G | L | I | S | E | I | I | SEQ ID NO 4 |
| 82 | A | T | E | M | E | R | T | F | I | A | I | K | P | D | G | V | Q | R | G | L | I | A | E | I | L | SEQ ID NO 4 |
| 85 | A | A | E | M | E | R | T | F | I | A | I | K | P | D | G | V | Q | R | G | L | I | S | E | I | V | SEQ ID NO 4 |
| 42 | A | A | E | M | E | R | T | F | I | A | I | K | P | D | G | V | Q | R | G | L | I | S | E | I | L | SEQ ID NO 4 |
| 84 | A | A | E | M | E | R | T | F | I | A | I | K | P | D | G | V | Q | R | G | L | I | S | E | I | I | SEQ ID NO 5 |

# Fig.15F

| | | | | | | | | | | | | | | | | | | | | | | | | | |
|---|---|---|---|---|---|---|---|---|---|---|---|---|---|---|---|---|---|---|---|---|---|---|---|---|---|
| | S | R | F | E | R | K | G | F | K | L | V | A | I | K | L | V | V | P | S | K | E | F | A | Q | K | Majority |
| | | | | | 130 | | | | | | | | | | 140 | | | | | | | | | | 150 | |
| 109 | N | R | F | E | R | K | G | Y | K | L | V | A | I | K | L | I | V | P | S | K | G | F | A | E | K | SEQ ID NO 1 |
| 109 | N | R | F | E | R | K | G | Y | K | L | V | A | I | K | L | I | V | P | S | K | G | F | A | E | K | SEQ ID NO 1 |
| 110 | S | R | F | E | R | K | G | Y | K | L | V | A | I | K | L | I | V | P | S | K | E | F | A | E | K | SEQ ID NO 1 |
| 106 | S | R | F | E | R | K | G | Y | K | L | V | G | I | K | V | V | I | P | T | K | E | F | A | Q | Q | SEQ ID NO 2 |
| 104 | S | R | F | E | R | K | G | F | K | L | V | G | I | K | V | V | I | P | S | K | D | F | A | Q | R | SEQ ID NO 2 |
| 109 | S | R | F | E | R | K | G | F | K | L | V | A | I | K | L | V | V | P | S | K | E | F | A | Q | K | SEQ ID NO 2 |
| 109 | A | R | F | E | R | K | G | F | K | L | V | A | I | K | L | V | T | P | T | K | S | F | A | Q | K | SEQ ID NO 2 |
| 107 | S | R | F | E | R | K | G | Y | K | L | V | G | I | K | V | V | I | P | K | K | E | F | A | Q | R | SEQ ID NO 2 |
| 109 | S | R | F | E | R | K | G | F | K | L | V | A | I | K | L | V | V | P | S | K | E | F | A | Q | K | SEQ ID NO 3 |
| 107 | S | R | F | E | R | K | G | Y | K | L | V | G | I | K | V | V | I | P | S | K | E | F | A | Q | K | SEQ ID NO 3 |
| 110 | S | R | F | E | R | K | G | Y | K | L | V | A | I | K | L | I | V | P | S | K | E | F | A | E | K | SEQ ID NO 3 |
| 23 | N | R | F | E | R | K | G | Y | K | L | V | A | I | K | L | I | V | P | S | K | G | F | A | E | K | SEQ ID NO 3 |
| 111 | S | R | F | E | R | K | G | F | K | L | V | A | I | K | L | V | V | P | S | K | E | F | A | Q | K | SEQ ID NO 4 |
| 105 | S | R | F | E | R | K | G | F | K | L | V | G | I | K | V | L | I | P | T | K | Q | F | A | Q | Q | SEQ ID NO 4 |
| 110 | S | R | F | E | R | K | G | F | K | L | V | G | I | K | V | I | V | P | S | K | D | F | A | Q | K | SEQ ID NO 4 |
| 107 | S | R | F | E | R | K | G | F | K | L | V | A | I | K | I | V | V | P | S | K | D | F | A | Q | K | SEQ ID NO 4 |
| 110 | S | R | F | E | R | K | G | F | K | L | V | G | I | K | V | V | V | P | S | K | D | F | A | Q | K | SEQ ID NO 4 |
| 67 | S | R | F | E | R | K | G | F | K | L | V | A | I | K | L | V | V | P | S | K | E | F | A | Q | K | SEQ ID NO 4 |
| 109 | T | R | F | E | R | K | G | Y | K | L | V | G | I | K | V | M | V | P | S | K | G | F | A | Q | K | SEQ ID NO 5 |

# Fig.15G

| | | | | | | | | | | | | | | | | | | | | | | | | | |
|---|---|---|---|---|---|---|---|---|---|---|---|---|---|---|---|---|---|---|---|---|---|---|---|---|---|
| | H | Y | H | D | L | K | E | R | P | F | F | N | G | L | C | D | F | L | S | S | G | P | V | L | A | Majority |
| | | | | | | | | | | 160 | | | | | | | | | | 170 | | | | | | |
| 134 | H | Y | H | D | L | K | E | R | P | F | F | N | G | L | C | D | F | L | S | S | G | P | V | L | A | SEQ ID NO 15 |
| 134 | H | Y | H | D | L | K | E | R | P | F | F | N | G | L | C | D | F | L | S | S | G | P | V | L | A | SEQ ID NO 17 |
| 135 | H | Y | H | D | L | K | E | R | P | F | F | S | G | L | C | D | F | L | S | S | G | P | V | L | A | SEQ ID NO 19 |
| 131 | H | Y | H | D | L | K | E | R | P | F | F | N | G | L | C | E | F | L | S | S | G | P | V | I | A | SEQ ID NO 21 |
| 129 | H | Y | H | D | L | K | E | R | P | F | F | N | G | L | C | N | F | L | S | S | G | P | V | I | A | SEQ ID NO 23 |
| 134 | H | Y | H | D | L | K | E | R | P | F | F | S | G | L | C | D | F | L | S | S | G | P | V | L | A | SEQ ID NO 25 |
| 134 | H | Y | H | D | L | K | E | R | P | F | F | D | G | L | C | D | F | L | S | S | G | P | V | L | A | SEQ ID NO 27 |
| 132 | H | Y | H | D | L | K | E | R | P | F | F | D | G | L | C | D | F | L | S | S | G | P | V | I | A | SEQ ID NO 29 |
| 134 | H | Y | D | D | L | K | E | R | P | F | F | N | G | L | C | E | F | L | S | S | G | P | V | L | A | SEQ ID NO 31 |
| 132 | H | Y | H | D | L | K | E | R | P | F | F | D | G | L | C | D | F | L | S | S | G | P | V | I | A | SEQ ID NO 33 |
| 135 | H | Y | H | D | L | K | E | R | P | F | F | S | G | L | C | D | F | L | S | S | G | P | V | L | A | SEQ ID NO 35 |
| 48 | H | Y | H | D | L | K | E | R | P | F | F | N | G | L | C | D | F | L | S | S | G | P | V | L | A | SEQ ID NO 37 |
| 136 | H | Y | H | D | L | K | D | R | P | F | F | N | G | L | C | D | F | L | S | S | G | P | V | L | A | SEQ ID NO 44 |
| 130 | H | Y | H | D | L | K | E | R | P | F | F | N | G | L | C | D | F | L | S | S | G | P | V | I | A | SEQ ID NO 45 |
| 135 | H | Y | H | D | L | K | E | R | P | F | F | N | G | L | C | D | F | L | S | S | G | P | V | I | A | SEQ ID NO 46 |
| 132 | H | Y | H | D | L | K | E | R | P | F | F | N | G | L | C | D | F | L | S | S | G | P | V | V | A | SEQ ID NO 47 |
| 135 | H | Y | H | D | L | K | E | R | T | F | F | N | G | L | C | D | F | L | S | S | G | P | V | I | A | SEQ ID NO 48 |
| 92 | H | Y | H | D | L | K | D | R | P | F | F | N | G | L | C | D | F | L | S | S | G | P | V | L | A | SEQ ID NO 49 |
| 134 | H | Y | H | D | L | K | E | R | P | F | F | N | G | L | C | N | F | L | S | S | G | P | V | V | A | SEQ ID NO 51 |

# Fig.15H

| | Sequence (positions 176–200; ticks at 180, 190, 200) | |
|---|---|---|
| | M V W E G E G V I K Y G R K L I G A T D P Q K S E | Majority |
| 159 | M V W E G E G V I K Y G R K L I G A T D P Q K S E | SEQ ID NO 15 |
| 159 | M V W E G E G V I K Y G R K L I G A T D P Q K S E | SEQ ID NO 17 |
| 160 | M V W E G E G V I K Y G R K L I G A T D P Q K S E | SEQ ID NO 19 |
| 156 | M V W E G Q G V I T Y G R K L I G A T D P Q K S E | SEQ ID NO 21 |
| 154 | M V W E G K G V I K Y G H K L I G A T D P Q K S E | SEQ ID NO 23 |
| 159 | M V W E G E G V I K Y G R K M I G A T D P A K S E | SEQ ID NO 25 |
| 159 | M V W E G E G V I K Y G R K L I G A T D P Q K S E | SEQ ID NO 27 |
| 157 | M V W E G Q G V I S Y G R K L I G A T D P Q K S E | SEQ ID NO 29 |
| 159 | M V W E G E G V I K Y G R K L I G A T D P Q K S E | SEQ ID NO 31 |
| 157 | M V W E G Q G V I S Y G R K L I G A T D P Q K S E | SEQ ID NO 33 |
| 160 | M V W E G E G V I K Y G R K L I G A T D P Q K S E | SEQ ID NO 35 |
| 73 | M V W E G E G V I K Y G R K L I G A T D P Q K S E | SEQ ID NO 37 |
| 161 | M V W E G E G V I K Y G R K L I G A T D P Q K S E | SEQ ID NO 44 |
| 155 | M V W E G E G V I T Y G R K L I G A T D P Q K S A | SEQ ID NO 45 |
| 160 | M V W E G D G V I R Y G R K L I G A T D P Q K S E | SEQ ID NO 46 |
| 157 | M V W E G E G V I K Y G R K L I G A T D P Q K S E | SEQ ID NO 47 |
| 160 | M V W E G E G V I R Y G R K L I G A T D P Q K S E | SEQ ID NO 48 |
| 117 | M V W E G E G V I K Y G R K L I G A T D P Q K S E | SEQ ID NO 49 |
| 159 | M V W E G E G V I R Y G R K L I G A T D P Q K S E | SEQ ID NO 51 |

Fig.15I

| | Sequence (210, 220) | |
|---|---|---|
| | P G T I R G D L A V V V G R N I I H G S D G P E T | Majority |
| 184 | P G T I R G D L G V V V G R N I I H G S D G P E T | SEQ ID NO 15 |
| 184 | P G T I R G D L G V V V G R N I I H G S D G P E T | SEQ ID NO 17 |
| 185 | P G T I R G D L A I V V G R N I I H G S D G P E T | SEQ ID NO 19 |
| 181 | P G T I R G D L A V V V G R N I I H G S D G P E T | SEQ ID NO 21 |
| 179 | P G T I R G D L A V V V G R N I I H G S D G P E T | SEQ ID NO 23 |
| 184 | P G T I R G D L A V V V G R N I I H G S D G P E T | SEQ ID NO 25 |
| 184 | P G T I R G D L A V V V G R N I I H G S D G P E T | SEQ ID NO 27 |
| 182 | P G T I R G D L A V V V G R N I I H G S D G P E T | SEQ ID NO 29 |
| 184 | P G T I R G D L A V V V G R N I I H G S D G P E T | SEQ ID NO 31 |
| 182 | P G T I R G D L A V V V G R N I I H G S D G P E T | SEQ ID NO 33 |
| 185 | P G T I R G D L A I V V G R N I I H G S D G P E T | SEQ ID NO 35 |
| 98 | P G T I R G D L G V V V G R N I I H G S D G P E T | SEQ ID NO 37 |
| 186 | P G T I R G D L A V V V G R N I I H G S D G P E T | SEQ ID NO 44 |
| 180 | P G T I R G D L A V V V G R N I I H G S D G P E T | SEQ ID NO 45 |
| 185 | P G T I R G D L A V T V G R N I I H G S D G P E T | SEQ ID NO 46 |
| 182 | P G T I R G D L A V V V G R N I I H G S D G P E T | SEQ ID NO 47 |
| 185 | P G T I R G D L A V V V G R N I I H G S D G P E T | SEQ ID NO 48 |
| 142 | P G T I R G D L A V V V G R N I I H G S D G P E T | SEQ ID NO 49 |
| 184 | P G T I R G D L A V V V G R N I I H G S D G P E T | SEQ ID NO 51 |

Fig.15J

```
      A K D E I A L W F K P E E L V S Y T S N A E K W I   Majority
              230                 240                 250
209   A K D E I A L W F E P K E L V S Y T S N A E K W V   SEQ ID NO 15.
209   A K D E I A L W F E P K E L V S Y T S N A E K W V   SEQ ID NO 17.
210   A K D E I A L W F E P K E L V S Y T S N A E K W I   SEQ ID NO 19.
206   A K E E I K L W F K P E E L V S F T S N A E K W I   SEQ ID NO 21.
204   A K D E I S L W F K P E E L V S Y T S N T E K W L   SEQ ID NO 23.
209   A K D E I A L W F T P K E L V S Y T S N E E K W V   SEQ ID NO 25.
209   A K D E I N L W F K P E E L T N Y T S N Q E K W V   SEQ ID NO 27.
207   A K D E I K L W F K P E E L V S F T S N A E K W I   SEQ ID NO 29.
209   A K H E I N L W F K P Q E L V N Y T S N A E K W V   SEQ ID NO 31.
207   A K D E I K L W F K P E E L V S F T S N A E K W V   SEQ ID NO 33.
210   A K D E I A L W F E P K E L V S Y T S N A E K W I   SEQ ID NO 35.
123   A K D E I A L W F E P K E L V S Y T S N A E K W V   SEQ ID NO 37.
211   A K A E I G L W F E P R E L V S Y T S N E E K W I   SEQ ID NO 44.
205   A K D E I K L W F K P E E L V S F T S N S E K W I   SEQ ID NO 45.
210   A K D E I S L W F K P Q E L V S Y T S N S E K W L   SEQ ID NO 46.
207   A K D E I N L W F K P E E L V N Y S S N A E K W I   SEQ ID NO 47.
210   A K D E I N L W F K P Q E L V S Y T N N A E K W I   SEQ ID NO 48.
167   A K A E I G L W F E P R E L V S Y T S N E E K W I   SEQ ID NO 49.
209   A K D E I S L W F K P E E L V S Y T S N A E K W I   SEQ ID NO 51.
```

Fig.15K

| | | | | | | |
|---|---|---|---|---|---|---|
| | Y | G | V | N | - | Majority |
| 234 | Y | G | V | N | . | SEQ ID NO 15. |
| 234 | Y | G | V | N | . | SEQ ID NO 17. |
| 235 | Y | G | V | N | . | SEQ ID NO 19. |
| 231 | Y | G | E | N | . | SEQ ID NO 21. |
| 229 | Y | G | D | N | . | SEQ ID NO 23. |
| 234 | Y | G | V | N | . | SEQ ID NO 25. |
| 234 | Y | G | V | N | . | SEQ ID NO 27. |
| 232 | Y | G | A | - | N | SEQ ID NO 29. |
| 234 | Y | G | N | N | . | SEQ ID NO 31. |
| 232 | Y | G | V | N | . | SEQ ID NO 33. |
| 235 | Y | G | V | N | . | SEQ ID NO 35. |
| 148 | Y | G | V | N | . | SEQ ID NO 37. |
| 236 | Y | G | V | - | N | SEQ ID NO 44. |
| 230 | Y | G | D | - | N | SEQ ID NO 45. |
| 235 | Y | G | - | D | N | SEQ ID NO 46. |
| 232 | Y | G | V | - | N | SEQ ID NO 47. |
| 235 | Y | G | - | D | N | SEQ ID NO 48. |
| 192 | Y | G | V | - | N | SEQ ID NO 49. |
| 234 | Y | G | - | Q | N | SEQ ID NO 51. |

# Fig.16

Percent Identity

| | 1 | 2 | 3 | 4 | 5 | 6 | 7 | 8 | 9 | 10 | 11 | 12 | 13 | 14 | 15 | 16 | 17 | 18 | 19 | | |
|---|---|---|---|---|---|---|---|---|---|---|---|---|---|---|---|---|---|---|---|---|---|
| 1 | | 99.6 | 85.7 | 67.2 | 69.1 | 72.7 | 71.0 | 65.5 | 69.7 | 66.9 | 85.7 | 99.3 | 73.9 | 68.7 | 68.9 | 68.9 | 70.2 | 80.0 | 69.6 | 1 | SEQ ID NO 15.pro |
| 2 | 0.0 | | 85.7 | 67.2 | 69.1 | 72.7 | 71.0 | 65.5 | 69.7 | 66.9 | 85.7 | 99.3 | 73.9 | 68.7 | 68.9 | 68.9 | 70.2 | 80.0 | 69.6 | 2 | SEQ ID NO 17.pro |
| 3 | 6.7 | 6.7 | | 68.1 | 67.4 | 76.9 | 71.4 | 68.1 | 69.7 | 68.2 | 99.2 | 94.7 | 78.2 | 69.5 | 67.6 | 71.1 | 68.1 | 82.1 | 67.9 | 3 | SEQ ID NO 19.pro |
| 4 | 34.8 | 34.8 | 34.9 | | 70.8 | 67.2 | 71.5 | 81.7 | 79.1 | 81.7 | 68.1 | 83.6 | 69.4 | 83.3 | 76.2 | 74.5 | 75.7 | 77.4 | 78.3 | 4 | SEQ ID NO 21.pro |
| 5 | 35.3 | 35.3 | 37.1 | 23.2 | | 67.4 | 74.2 | 72.1 | 74.7 | 70.8 | 67.4 | 85.5 | 70.0 | 73.4 | 76.8 | 74.2 | 76.0 | 75.9 | 75.1 | 5 | SEQ ID NO 23.pro |
| 6 | 23.6 | 23.6 | 21.2 | 34.7 | 34.6 | | 69.3 | 66.8 | 70.2 | 67.8 | 76.9 | 91.4 | 79.8 | 68.2 | 67.6 | 68.9 | 69.3 | 83.1 | 66.7 | 6 | SEQ ID NO 25.pro |
| 7 | 28.0 | 28.0 | 30.9 | 28.0 | 25.0 | 32.3 | | 73.2 | 79.4 | 74.2 | 71.4 | 88.8 | 69.3 | 75.1 | 74.8 | 77.9 | 74.4 | 78.5 | 76.8 | 7 | SEQ ID NO 27.pro |
| 8 | 36.3 | 36.3 | 34.4 | 11.5 | 25.6 | 36.2 | 26.6 | | 75.7 | 91.9 | 67.7 | 84.9 | 66.4 | 83.3 | 77.4 | 71.5 | 76.6 | 76.9 | 77.0 | 8 | SEQ ID NO 29.pro |
| 9 | 30.6 | 30.6 | 31.6 | 20.7 | 22.1 | 30.1 | 19.8 | 23.1 | | 75.8 | 69.7 | 89.5 | 71.8 | 77.3 | 78.6 | 82.6 | 79.8 | 81.5 | 78.9 | 9 | SEQ ID NO 31.pro |
| 10 | 31.8 | 31.8 | 30.8 | 13.5 | 26.1 | 31.8 | 24.6 | 6.7 | 22.4 | | 68.2 | 87.5 | 67.4 | 82.0 | 77.1 | 73.2 | 75.8 | 77.9 | 75.8 | 10 | SEQ ID NO 33.pro |
| 11 | 6.7 | 6.7 | 0.4 | 34.9 | 37.1 | 21.2 | 30.9 | 34.4 | 31.6 | 30.8 | | 94.7 | 78.2 | 69.5 | 67.6 | 71.1 | 68.5 | 82.1 | 68.4 | 11 | SEQ ID NO 35.pro |
| 12 | 0.0 | 0.0 | 4.8 | 17.1 | 15.4 | 7.7 | 11.5 | 15.7 | 10.7 | 12.2 | 4.8 | | 90.8 | 84.2 | 87.5 | 88.8 | 88.2 | 90.8 | 89.5 | 12 | SEQ ID NO 37.pro |
| 13 | 19.0 | 19.0 | 15.0 | 30.4 | 30.3 | 16.8 | 32.8 | 34.0 | 27.5 | 30.8 | 15.0 | 9.2 | | 69.5 | 68.9 | 71.9 | 69.7 | 97.9 | 68.4 | 13 | SEQ ID NO 44.pro |
| 14 | 31.0 | 31.0 | 33.0 | 14.4 | 22.8 | 33.7 | 24.5 | 14.0 | 23.3 | 16.7 | 33.0 | 16.4 | 30.4 | | 78.5 | 74.7 | 76.8 | 77.9 | 78.5 | 14 | SEQ ID NO 45.pro |
| 15 | 34.9 | 34.9 | 36.7 | 23.1 | 21.1 | 36.5 | 25.1 | 20.5 | 22.7 | 22.8 | 36.0 | 13.1 | 30.8 | 21.4 | | 76.6 | 92.4 | 79.5 | 86.9 | 15 | SEQ ID NO 46.pro |
| 16 | 33.2 | 33.2 | 32.8 | 25.9 | 24.2 | 31.3 | 22.9 | 28.7 | 17.8 | 27.5 | 32.8 | 11.5 | 28.3 | 25.9 | 24.1 | | 77.9 | 81.0 | 76.6 | 16 | SEQ ID NO 47.pro |
| 17 | 33.5 | 33.5 | 35.2 | 23.7 | 22.8 | 34.4 | 25.7 | 21.7 | 21.0 | 24.0 | 34.5 | 12.3 | 30.1 | 22.6 | 8.0 | 22.4 | | 80.0 | 85.7 | 17 | SEQ ID NO 48.pro |
| 18 | 15.9 | 15.9 | 12.3 | 21.5 | 20.3 | 12.6 | 22.0 | 22.0 | 17.3 | 19.8 | 12.3 | 9.2 | 0.5 | 19.0 | 20.1 | 17.2 | 19.4 | | 79.0 | 18 | SEQ ID NO 49.pro |
| 19 | 33.5 | 33.5 | 33.8 | 22.0 | 22.8 | 37.9 | 23.9 | 20.6 | 23.9 | 23.0 | 33.2 | 10.8 | 32.1 | 22.1 | 12.9 | 24.1 | 14.4 | 20.8 | | 19 | SEQ ID NO 51.pro |
| | 1 | 2 | 3 | 4 | 5 | 6 | 7 | 8 | 9 | 10 | 11 | 12 | 13 | 14 | 15 | 16 | 17 | 18 | 19 | | |

Percent Divergence

Fig. 17

**Modified Hoagland's solutions -**
**16X concentrations for semi-hydroponics maize growth.**

| Nutrient | 1 mM $KNO_3$ | 2 mM $KNO_3$ | 3 mM $KNO_3$ | 4 mM $KNO_3$ |
|---|---|---|---|---|
| $KNO_3$ | 16 mM | 32 mM | 48 mM | 64 mM |
| KCl | 48 mM | 32 mM | 16 mM | ------- |
| $KH_2PO_4$ | 11 mM | 11 mM | 11 mM | 11 mM |
| $MgSO_4$ | 16 mM | 16 mM | 16 mM | 16 mM |
| $CaCl_2 \cdot 2H_2O$ | 16 mM | 16 mM | 16 mM | 16 mM |
| Sprint 330 | 1.6 g/L | 1.6 g/L | 1.6 g/L | 1.6 g/L |
| $H_3BO_3$ | 24 μM | 24 μM | 24 μM | 24 μM |
| 5 mM $MnCl_2 \cdot 4H_2O$ | 8 μM | 8 μM | 8 μM | 8 μM |
| 5 mM $ZnSO_4 \cdot 7\ H_2O$ | 8 M | 8 μM | 8 μM | 8 μM |
| 0.5 mM $CuSO_4 \cdot 5\ H_2O$ | 800 nM | 800 nM | 800 nM | 800 nM |
| 0.5 mM $H_2MoO_4 \cdot H_2O$ | 800 nM | 800 nM | 800 nM | 800 nM |

Dilute 16X with tap water and determine the pH of the final mixture.

Add 3-12 mL $H_2SO_4$ if the pH is above 6.5.

Optimum pH is 5.0 - 5.5

# Fig. 18

The effect of different nitrate concentrations on the growth and development of Gaspe Bay Flint derived maize lines (see Example 10C).

| [nitrate] | root (g dwt) | shoot (g dwt) | total vegetative (g dwt) | ear & husk (g dwt) | tassel (g dwt) | tiller # | tiller (g dwt) |
|---|---|---|---|---|---|---|---|
| 1 week after emergence | | | | | | | |
| 1 mM | 0.070a | 0.105b | 0.175b | | | | |
| 2 mM | 0.073a | 0.137ab | 0.209ab | | | | |
| 3 mM | 0.056a | 0.120ab | 0.176ab | | | | |
| 4 mM | 0.074a | 0.157a | 0.231a | | | | |
| 2 weeks after emergence | | | | | | | |
| 1 mM | 0.331ab | 0.544c | 0.875c | | | | |
| 2 mM | 0.266b | 0.951b | 1.217b | | | | |
| 3 mM | 0.352a | 1.171a | 1.523a | | | | |
| 4 mM | 0.303ab | 1.209a | 1.512a | | | | |
| 3 weeks after emergence | | | | | | | |
| 1 mM | 0.757a | 1.283b | 2.040b | 0.379c | 0.239c | 0.8c | 0.080b |
| 2 mM | 0.785a | 2.033a | 2.819a | 0.718a | 0.363bc | 2.3 | 0.506a |
| 3 mM | 0.664a | 1.911a | 2.574a | 0.451bc | 0.403ab | 2.8ab | 0.441a |
| 4 mM | 0.845a | 2.129a | 2.974a | 0.650ab | 0.506a | 3.3a | 0.688a |
| 4 weeks after emergence | | | | | | | |
| 1 mM | 0.842b | 2.010b | 2.852b | 1.318b | 0.677b | * | * |
| 2 mM | 1.493a | 3.772a | 5.265a | 3.130a | 1.018a | * | * |
| 3 mM | 1.232ab | 3.563a | 4.795a | 3.060a | 0.875ab | * | * |
| 4 mM | 1.010b | 2.943a | 3.952a | 2.787a | 0.891ab | * | * |

* Tillers removed 3 weeks after emergence
Means with similar letters are not different by protected Least Significant Difference (LSD) (0.05)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 96875407 P
- US 200557473 A
- US 2005223429 A1
- US 6344601 B
- WO 2004US16432 A
- WO 2004106531 A
- US 2004489500 A
- US 2005059154 A1
- US 5107065 A
- WO 9836083 A
- WO 9953050 A
- WO 9961632 A
- WO 0200894 A
- WO 0200904 A
- WO 9943838 A
- US 6072050 A
- US 5659026 A
- WO 0020571 A2
- US 5608149 A
- US 5508144 A
- US 5604121 A
- US 5589597 A
- US 5466785 A
- US 5399680 A
- US 5268463 A
- US 5608142 A
- US 6177611 A
- US 20060156439 A
- WO 05063998 A
- WO 06055487 A
- WO 05035770 A
- US 5004863 A
- US 5159135 A
- US 5518908 A
- US 5569834 A
- US 5416011 A
- US 5463174 A
- US 5631152 A
- EP 1033405 A2
- US 2005108791 A
- WO 2004035798 A
- US 2007044171 A
- US 2007061916 A
- US 200700196 B
- EP 1033405 A
- US 4945050 A
- US 5013659 A
- US 20030226166 A1
- EP 0242236 A
- US 7087812 B
- US 5981840 A
- US 20030221212 A, Tomes
- US 20040122592 A


### Non-patent literature cited in the description


- **LOPEZ-BUCIO et al.** *Current Opinion in Plant Biology,* 2003, vol. 6, 280-287
- **J.E. MALAMY.** *Plant, Cell and Environment,* 2005, vol. 28, 67-77
- **HOCHHOLDINGER et al.** Annals of Botany. 2004, vol. 93, 359-368
- **WEIGEL et al.** *Plant Physiol.,* 2000, vol. 122, 1003-1013
- *Nucleic Acids Res.,* 1985, vol. 13, 3021-3030
- *Biochemical J.,* 1984, vol. 219 (2), 345-373
- **HIGGINS ; SHARP.** *CABIOS,* 1989, vol. 5, 151-153
- **SAMBROOK, J. ; FRITSCH, E.F. ; MANIATIS, T.** Molecular Cloning: A Laboratory Manual. Cold Spring Harbor Laboratory Press, 1989
- **VAUCHERET et al.** *Plant J.,* 1998, vol. 16, 651-659
- **GURA.** *Nature,* 2000, vol. 404, 804-808
- **WESLEY, S.V. et al.** *Methods in Molecular Biology, Plant Functional Genomics: Methods and Protocols,* 2003, vol. 236, 273-286
- **FIRE et al.** *Nature,* 1998, vol. 391, 806
- **FIRE et al.** *Trends Genet.,* 1999, vol. 15, 358
- **BERSTEIN et al.** *Nature,* 2001, vol. 409, 363
- **ELBASHIR et al.** *Genes Dev.,* 2001, vol. 15, 188
- **HUTVAGNER et al.** *Science,* 2001, vol. 293, 834
- **ALLSHIRE.** *Science,* 2002, vol. 297, 1818-1819
- **VOLPE et al.** *Science,* vol. 297, 1833-1837
- **JENUWEIN.** *Science,* 2002, vol. 297, 2215-2218
- **HALL et al.** *Science,* 2002, vol. 297, 2232-2237

- **WIANNY ; GOETZ.** *Nature Cell Biol.,* 1999, vol. 2, 70
- **HAMMOND et al.** *Nature,* 2000, vol. 404, 293
- **ELBASHIR et al.** *Nature,* 2001, vol. 411, 494
- **LAGOS-QUINTANA et al.** *Science,* 2001, vol. 294, 853-858
- **LAGAS-QUINTANA et al.** *Curr. Biol.,* 2002, vol. 12, 735-739
- **LAU et al.** *Science,* 2001, vol. 294, 858-862
- **LEE ; AMBROS.** *Science,* 2001, vol. 294, 862-864
- **LLAVE et al.** *Plant Cell,* 2002, vol. 14, 1605-1619
- **MOURELATOS et al.** *Genes. Dev.,* 2002, vol. 16, 720-728
- **PARK et al.** *Curr. Biol.,* 2002, vol. 12, 1484-1495
- **REINHART et al.** *Genes. Dev.,* 2002, vol. 16, 1616-1626
- **GRISHOK et al.** *Cell,* 2001, vol. 106, 23-34
- **HUTVAGNER et al.** *Science,* 2001, vol. 293, 834-838
- **KETTING et al.** *Genes. Dev.,* 2001, vol. 15, 2654-2659
- **LEE et al.** *EMBO J,* 2002, vol. 21, 4663-4670
- **SCHWARTZ et al.** *Cell,* 2003, vol. 115, 199-208
- **LEE et al.** *Cell,* 1993, vol. 75, 843-854
- **WIGHTMAN et al.** *Cell,* 1993, vol. 75, 855-862
- **REINHART et al.** *Nature,* 2000, vol. 403, 901-906
- **SLACK et al.** *Mol. Cell,* 2000, vol. 5, 659-669
- **OLSEN ; AMBROS.** *Dev. Biol.,* 1999, vol. 216, 671-680
- **HUTVAGNER ; ZAMORE.** *Science,* 2002, vol. 297, 2056-2060
- **PARK et al.** *Curr. Biol.,* 2003, vol. 12, 1484-1495
- **RHOADES et al.** *Cell,* 2002, vol. 110, 513-520
- **KASUGA et al.** *Nature Biotechnol.,* 1999, vol. 17, 287-291
- **ODELL et al.** *Nature,* 1985, vol. 313, 810-812
- **MCELROY et al.** *Plant Cell,* 1990, vol. 2, 163-171
- **CHRISTENSEN.** *Plant Mol. Biol.,* 1989, vol. 12, 619-632
- **CHRISTENSEN et al.** *Plant Mol. Biol.,* 1992, vol. 18, 675-689
- **LAST et al.** *Theor. Appl. Genet.,* 1991, vol. 81, 581-588
- **VELTEN et al.** *EMBO J.,* 1984, vol. 3, 2723-2730
- **JOFUKU ; GOLDBERG.** *Plant Cell,* 1989, vol. 1, 1079-1093
- **ROCHA-SOSA, M. et al.** *EMBO J.,* 1989, vol. 8, 23-29
- **RERIE, W.G. et al.** *Mol. Gen. Genet.,* 1991, vol. 259, 149-157
- **NEWBIGIN, E.J. et al.** *Planta,* 1990, vol. 180, 481-470
- **HIGGINS, T.J.V. et al.** *Plant. Mol. Biol.,* 1988, vol. 11, 683-695
- **SCHEMTHANER, J.P. et al.** *EMBO J.,* 1988, vol. 7, 1249-1255
- **SEGUPTA-GOPALAN, C. et al.** *Proc. Natl. Acad. Sci. U.S.A.,* 1985, vol. 82, 3320-3324
- **VOELKER, T. et al.** *EMBO J.,* 1987, vol. 6, 3571-3577
- **CHEN, Z-L et al.** *EMBO J.,* 1988, vol. 7, 297-302
- **MARRIS, C. et al.** *Plant Mol. Biol.,* 1988, vol. 10, 359-366
- **COLOT, V. et al.** *EMBO J.,* 1987, vol. 6, 3559-3554
- **HATTORI, T. et al.** *Plant Mol. Biol.,* 1990, vol. 14, 595-604
- **VANDERKERCKHOVE et al.** *Bio/Technology,* 1989, vol. 7, L929-932
- **RIGGS et al.** *Plant Sci.,* 1989, vol. 63, 47-57
- **COLOT et al.** *EMBO J,* 1987, vol. 6, 3559-3564
- **KASUGA et al.** *Nature Biotechnol.,* 1999, vol. 17, 287-91
- **KLEMSDAL, S.S. et al.** Primary Structure of a Novel Barley Gene Differentially Expressed in Immature Aleurone Layers. *Mol. Gen. Genet.,* vol. 228 (1/2), 9-16
- **SCHMIDT, R.J. et al.** Identification and molecular characterization of ZAG1, the maize homolog of the Arabidopsis floral homeotic gene AGAMOUS. *Plant Cell,* 1993, vol. 5 (7), 729-737
- **THEISSEN et al.** Structural characterization, chromosomal localization and phylogenetic evaluation of two pairs of AGAMOUS-like MADS-box genes from maize. *Gene,* 1995, vol. 156 (2), 155-168
- **ABRAHAMS et al.** *Plant Mol. Biol.,* 1995, vol. 27, 513-528
- **OKAMURO, J. K. ; GOLDBERG, R. B.** *Biochemistry of Plants,* 1989, vol. 15, 1-82
- **ALTSCHUL et al.** *J. Mol. Siol.,* 1993, vol. 215, 403-410
- **BUCHMAN ; BERG.** *Mol. Cell Biol.,* 1988, vol. 8, 4395-4405
- **CALLIS et al.** *Genes Dev.,* 1987, vol. 1, 1183-1200
- The Maize Handbook. Springer, 1994
- **TURNER, R. ; FOSTER, G. D.** *Molecular Biotechnology,* 1995, vol. 3, 225
- **MCCABE.** *Bio/Technology,* 1988, vol. 6, 923
- **CHRISTOU et al.** *Plant Physiol.,* 1988, vol. 87, 671-674
- **CHENG et al.** *Plant Cell Rep.,* 1996, vol. 15, 653-657

- **MCKENTLY et al.** *Plant Cell Rep.,* 1995, vol. 14, 699-703
- **GRANT et al.** *Plant Cell Rep.,* 1995, vol. 15, 254-258
- **BYTEBIER et al.** *Proc. Natl. Acad. Sci. U.S.A.,* 1987, vol. 84, 5354
- **WAN ; LEMAUX.** *Plant Physiol.,* 1994, vol. 104, 37
- **RHODES et al.** *Science,* 1988, vol. 240, 204
- **GORDON-KAMM et al.** *Plant Cell,* 1990, vol. 2, 503-618
- **FROMM et al.** *BiolTechnology,* 1990, vol. 8, 833
- **KOZIEL et al.** *Bio/Technology,* 1993, vol. 11, 194
- **ARMSTRONG et al.** *Crop Science,* 1995, vol. 35, 550-557
- **SOMERS et al.** *Bio/Technology,* 1992, vol. 10, 1589
- **HORN et al.** *Plant Cell Rep.,* 1988, vol. 7, 469
- **TORIYAMA et al.** *Theor. Appl. Genet.,* 1986, vol. 205, 34
- **PART et al.** *Plant Mol. Biol.,* 1996, vol. 32, 1135-1148
- **ABEDINIA et al.** *Aust. J. Plant Physiol.,* 1997, vol. 24, 133-141
- **ZHANG ; WU.** *Theor. Appl. Genet.,* 1988, vol. 78, 835
- **ZHANG et al.** *Plant Cell Rep.,* 1988, vol. 7, 379
- **BATTRAW ; HALL.** *Plant Sci.,* 1992, vol. 86, 191-202
- **CHRISTOU et al.** *BiolTechnology,* 1991, vol. 9, 957
- **PENA et al.** *Nature,* 1987, vol. 325, 274
- **BOWER ; BIRCH.** *Plant J.,* 1992, vol. 2, 409
- **WANG et al.** *BiolTechnology,* 1992, vol. 10, 691
- **VASIL et al.** *BiolTechnology,* 1992, vol. 10, 667
- **WEISSBACH ; WEISSBACH.** Methods for Plant Molecular Biology. Academic Press, Inc. San Diego, 1988
- **LIU et al.** *Plant J.,* 1995, vol. 8, 457-63
- **SIEBERT et al.** *Nucleic Acids Res.,* 1995, vol. 23, 1087-1088
- **ADAMS et al.** *Science,* 1991, vol. 252, 1651-1656
- **DEVINE ; BOEKE.** *Nucleic Acids Res.,* 1994, vol. 22, 3765-3772
- **FLING ; RICHARDS.** *Nucleic Acids Res.,* 1983, vol. 11, 5147-5158
- **EWING et al.** *Genome Res.,* 1998, vol. 8, 175-185
- **EWING ; GREEN.** *Genome Res.,* 1998, vol. 8, 186-194
- **GORDON et al.** *Genome Res.,* 1998, vol. 8, 195-202
- **ALTSCHUL et al.** *J. Mol. Biol.,* 1993, vol. 215, 403-410
- **GISH ; STATES.** *Nat. Genet.,* 1993, vol. 3, 266-272
- **ALTSCHUL et al.** *Nucleic Acids Res.,* 1997, vol. 25, 3389-3402
- **KLEIN et al.** *Nature (London,* 1987, vol. 327, 70-73
- **ODELL et al.** *Nature,* 1985, vol. 373, 810-812
- **GRITZ et al.** *Gene,* 1983, vol. 25, 179-188
- **CHRISTENSEN et al.** *Plant Mol. Biol.,* 1989, vol. 12, 619-632
- **CHU et al.** *Sci. Sin. Peking,* 1975, vol. 18, 659-668
- **ODELL et al.** *Nature,* 1985, vol. 313, 810-S12
- **KLEIN et al.** *Nature,* 1987, vol. 327, 70-73
- **FROMM et al.** *Bio/Technology,* 1990, vol. 8, 833-839
- **ZHAO et al.** *Meth. Mol. Biol.,* 2006, vol. 318, 315-323
- **ZHAO et al.** *Mol. Breed.,* 2001, vol. 8, 323-333
- **KOMARI et al.** *Plant J,* 1996, vol. 10, 165-174